# EUROPEAN PATENT SPECIFICATION

(11) **EP 1 606 268 B1**
(45) Date of publication and mention of the grant of the patent: **20.05.2015**
(21) Application number: 04715097.4
(22) Date of filing: 26.02.2004
(51) Int. Cl.: C07D 243/14, A61K 31/5513, A61P 35/00, C07D 403/12, C07D 401/04, C07D 401/06, C07D 401/12, C07D 403/04, C07D 405/06, C07D 405/12, C07D 409/12, C07D 413/06, C07D 417/04

(54) **5,10-DIHYDRO-11H-DIBENZO[B,E][1,4]DIAZEPIN-11-ONE AS KINASE INHIBITORS**
5,10-DIHYDRO-11H-DIBENZO[B,E][1,4]DIAZEPIN-11-ON VERBINDUNGEN ALS KINASE INHIBITOREN
COMPOSÉS DE 5,10-DIHYDRO-11H-DIBENZO[B,E][1,4]DIAZEPIN-11-ONE COMME INHIBITEURS DE KINASE

(30) Priority: 27.02.2003 US 375412; 25.02.2004 US 785120
(43) Date of publication of application: 21.12.2005
(73) Proprietor: AbbVie Inc., North Chicago, IL 60064 (US)
(72) Inventor: HASVOLD, Lisa, A., Grayslake, IL 60030 (US); HEXAMER, Laura, Grayslake, IL 60030 (US); LI, Gaoquan, Park City, IL 60085 (US); LIN, Nan-Horng, Vernon Hills, IL, 60061 (US); SHAM, Hing, Vernon Hills, IL 60061 (US); SOWIN, Tom, Wadsworth, IL 60083 (US); SULLIVAN, Gerard, M., Lake Villa, IL 60046 (US); WANG, Le, Vernon Hills, IL 60061 (US); XIA, Ping Xia, St. Louis, MO 63144 (US)
(74) Representative: Modiano, Micaela Nadia
(86) International application number: PCT/US2004/005728
(87) International publication number: WO 2004/076424

(56) References cited:
- EP-A- 0 679 641
- WO-A-00/59901
- WO-A-00/64900
- WO-A-95/17400
- REBA R C ET AL: "Synthesis of Some Substituted Dibenzodiazepinones and Pyridobenzodiazepinones" JOURNAL OF HETEROCYCLIC CHEMISTRY, vol. 35, 1998, pages 675-686, XP002289300
- MURUGESAN N ET AL: "Design and synthesis of nonpeptidal endothelin receptor antagonists based on the structure of a cyclic pentapeptide" BIOORGANIC & MEDICINAL CHEMISTRY LETTERS, OXFORD, GB, vol. 5, no. 3, 2 February 1995 (1995-02-02), pages 253-258, XP004135769 ISSN: 0960-894X
- DATABASE BEILSTEIN BEILSTEIN INSTITUTE FOR ORGANIC CHEMISTRY, FRANKFURT-MAIN, DE; XP002289301 Database accession no. BRN: 173563 & JOURNAL OF MEDICINAL CHEMISTRY, vol. 6, 1963, pages 255-261,
- DATABASE BEILSTEIN BEILSTEIN INSTITUTE FOR ORGANIC CHEMISTRY, FRANKFURT-MAIN, DE; XP002289302 Database accession no. BRN: 657947 & ARZNEIM. FORSCH., vol. 13, 1963, pages 324-328,
- DATABASE BEILSTEIN BEILSTEIN INSTITUTE FOR ORGANIC CHEMISTRY, FRANKFURT-MAIN, DE; XP002289303 Database accession no. BRN996479 & J. CHEM. SOC. PERKIN TRANS. 1, 1976, pages 1286-1290,

## Description

### Technical Field

The present invention relates to substituted tricyclic heterocycles which are useful for inhibiting protein kinases, methods of making the compounds, and compositions containing the compounds.

### Background of the Invention

Protein kinases have been clearly shown to be important in the progression of many disease states that are induced by the inappropriate proliferation of cells. These kinases are often found to be up-regulated in many hyperproliferative states such as cancer. These kinases may be important in cell signaling, where their inappropriate activation induces cells to proliferate (e.g., EGFR, ERBB2, VEGFR, FGFR, PDGFR, c-Met, IGF-1R, RET, TIE2). Alternatively, they may be involved in signal transduction within cells (e.g., c-Src, PKC, Akt, PKA, c-Abl, PDK-1). Often these signal transduction genes are recognized proto-oncogenes. Many of these kinases control cell cycle progression near the G1-S transition (e.g., Cdk2, Cdk4), at the G2-M transition (e.g., Weel, Mytl, Chk1, Cdc2) or at the spindle checkpoint (Plk, Auroral or 2, Bubl or 3). Furthermore, kinases are intimately linked to the DNA damage response (e.g., ATM, ATR, Chkl, Chk2). Deregulation of these cellular functions: cell signaling, signal transduction, cell cycle control, and DNA repair, are all hallmarks of hyperproliferative diseases, particularly cancer. It is therefore likely that pharmacological modulation of one or more kinases would be useful in slowing or stopping disease progression in these diseases.

As background art, Cohen, V.I. et al, J. Hetercyclic Chem. Vol. 35, 675-686 (1998), which relates to the synthesis of some substituted dibenzodiazepinones and pyridobenzodiazepinones, discloses the compound 3-fluoro-11-oxo-10,11-dihydro-5H-dibenzo[b,e][1,4] diazepine. WO 95/17400 discloses 3-chloro-11-oxo-10,11-dihydro-5H-dibenzo[b,e][1,4] diazepine as an intermediate. WO 00/59901 relates to substituted 1,4-dihydroindeno[1,2-c]pyrazoles as inhibitors of tyrosine kinase which may be used in the treatment of cancer. WO 00/64900 discloses pyrazolobenzodiazepines as inhibitors of cyclin-dependent kinases which may be used in the treatment of cancer. EP-A-9 679 641 discloses tricyclic heterocyclic sulfonamide and sulfonic ester derivatives having antitumor activity.

### Summary of the Invention

The scope of the invention as well as further embodiments are defined by the appended claims. In its principle embodiment the present invention provides a compound of formula (I) 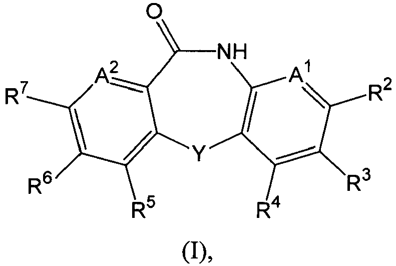 or a therapeutically acceptable salt thereof, wherein
(a)
   A¹ is CH;
   A² is CH;
   R² is selected from the group consisting of hydrogen, alkenyl, alkoxy, alkoxyalkoxy, alkoxyalkoxyalkoxy, alkoxyalkyl, alkoxycarbonyl, alkoxycarbonylalkyl, alkyl, alkylcarbonyl, alkylcarbonylalkyl, amino, aminoalkoxy, aminoalkyl, aminocarbonyl, aminocarbonylalkyl, aminosulfonyl, aryl, arylalkoxy, arylalkyl, aryloxyalkyl, carboxy, carboxyalkyl, cyano, cyanoalkyl, cycloalkyl, cycloalkylalkyl, halo, haloalkoxy, haloalkyl, heterocyclyl, heterocyclylalkoxy, heterocyclylalkyl, heterocyclylcarbonylalkyl, heterocyclyloxyalkyl, hydroxy, hydroxyalkoxy, hydroxyalkyl, nitro, nitroalkyl, and -(CR⁹R¹⁰)ₘC(O)NR¹¹R¹²;
   R³, R⁴ and R⁵ are each hydrogen;
   R⁶ is selected from the group consisting of aryl, cycloalkyl, halo, heterocyclyl, and -XR¹³;
   R⁷ is hydrogen;
   R⁹ and R¹⁰ are independently selected from the group consisting of hydrogen, alkenyl, alkyl, aminoalkyl, and hydroxyalkyl; or
   R⁹ and R¹⁰, together with the carbon atom to which they are attached, form a cycloalkyl group;
   R¹¹ and R¹² are each independently selected from the group consisting of hydrogen, alkenyl, alkoxyalkyl, alkyl, amino, aminoalkyl, aryl, arylalkyl, cyanoalkyl, cycloalkyl, cycloalkylalkyl, haloalkyl, heterocyclyl, heterocyclylalkyl, hydroxyalkyl, -NR^{c}R^{d}, (NR^{c}R^{d})alkyl, and (NR^{c}R^{d})carbonylalkyl; or
   R¹¹ and R¹², together with the nitrogen atom to which they are attached, form a heterocyclyl ring selected from the group consisting of azetidinyl, diazepanyl, imidazolidinyl, morpholinyl, piperazinyl, piperidinyl, pyrrolidinyl and thiomorpholinyl, which can each be optionally substituted with one, two, or three substituents independently selected from the group consisting of alkoxy, alkoxycarbonyl, alkenyl, alkyl, alkylcarbonyl, aryl, carboxy, carboxyalkyl, heterocyclyl, heterocyclylalkyl, hydroxy, hydroxyalkyl, -NR^{c}R^{d}, (NR^{c}R^{d})alkyl, and (NR^{c}R^{d})carbonyl;
   R¹³ is selected from the group consisting of aryl, cycloalkyl, and heterocyclyl;
   X is selected from the group consisting of -O-, -NR¹⁴-, -C(O)-, -S-, -SO₂-, -(CH₂)ₙ-, -C(O)NR¹⁴-, -NR¹⁴C(O)-, -SO₂NR¹⁴-, -NR¹⁴SO₂, -O(CH₂)ₘ-, -(CH₂)ₘO-, -CH=CH-, and -C≡C-; wherein R¹⁴ is selected from the group consisting of hydrogen, alkenyl, alkyl, aminoalkyl, and hydroxyalkyl;
   Y is NH;
   m is 0-3; and
   n is 1-3;
   or wherein
(b)
   A¹ is CH;
   A² is CH;
   Y is NH;
   R⁴, R⁵, and R⁷ are each hydrogen;
   R⁶ is phenyl substituted in the three position with methoxy and substituted in the four position with alkoxycarbonyl, alkoxycarbonylalkenyl, alkylcarbonyl, alkylsulfonyl, cyano, halo, haloalkenyl, hydroxy, nitro, NH₂SO₂-, or NH₂CO-; and either
      (i) R² is hydrogen and R³ is -(CR⁹R¹⁰)C(O)NHR¹² where R⁹ and R¹⁰ are independently selected from the group consisting of hydrogen, alkenyl, alkyl, aminoalkyl, and hydroxyalkyl, or R⁹ and R¹⁰, together with the carbon atom to which they are attached, form a cycloalkyl group, and R¹² is phenyl optionally substituted with 1 morpholinyl group;
      (ii) R² is hydrogen and R³ is hydroxyalkyl;
      (iii) R² is hydrogen and R³ is alkoxy;
      (iv) R² and R³ are independently -(CR⁹R¹⁰)C(O)NHR¹² where R⁹ and R¹⁰ are independently selected from the group consisting of hydrogen, alkenyl, alkyl, aminoalkyl, and hydroxyalkyl, or R⁹ and R¹⁰, together with the carbon atom to which they are attached, form a cycloalkyl group, and R¹² is phenyl optionally substituted with 1 morpholinyl group;
      (v) R² and R³ are independently hydroxalkyl; or
      (vi) R² and R³ are independently alkoxy;
      wherein
      alkyl is a C₁-C₁₀ alkyl;
      alkenyl is a C₂-C₆ alkenyl;
      alkoxy is a C₁-C₁₀ alkoxy;
      aryl is phenyl group, or a bicyclic or tricyclic fused ring system wherein one or more of the fused rings is a phenyl group, wherein aryl can be optionally substituted with one, two, three, four, or five substituents independently selected from the group consisting of alkoxy, alkoxyalkyl, alkoxycarbonyl, alkoxycarbonylalkenyl, alkoxycarbonylalkyl, alkoxysulfonyl, alkyl, alkylcarbonyl, alkylsulfonyl, amino, aminocarbonyl, aminosulfonyl, a second aryl group, arylalkyl, arylsulfonyl, carboxy, cyano, formyl, halo, haloalkoxy, haloalkenyl, haloalkyl, heterocyclyl, heterocyclylalkyl, hydroxy, hydroxyalkyl, nitro, (NR^{c}R^{d})alkyl, and oxo, wherein the second aryl group, the aryl part of the arylalkyl and the arylsulfonyl, the heterocyclyl, and the heterocyclyl part of the heterocyclylalkyl can be optionally substituted with one, two, three, four, or five substituents independently selected from the group consisting of alkoxy, alkoxyalkyl, alkoxycarbonyl, alkyl, alkylcarbonyl, carboxy, cyano, halo, haloalkoxy, haloalkyl, hydroxy, nitro, and oxo;
      heterocyclyl is selected from a five-, six-, or seven-membered ring containing one, two, or three heteroatoms independently selected from the group consisting of nitrogen, oxygen, and sulfur, wherein the five-membered ring has zero to two double bonds and the six- and seven-membered rings have zero to three double bonds; bicyclic groups in which the heterocyclyl ring is fused to a phenyl group, a monocyclic cycloalkenyl group, a monocyclic cycloalkyl group, or another monocyclic heterocyclyl group; and tricyclic groups in which a bicyclic system is fused to a phenyl group, a monocyclic cycloalkenyl group, a monocyclic cycloalkyl group, or another monocyclic heterocyclyl group; wherein heterocyclyl can be optionally substituted with one, two, three, four, or five substituents independently selected from the group consisting of alkoxy, alkoxyalkyl, alkoxycarbonyl, alkyl, alkylcarbonyl, alkylsulfonyl, amino, aminocarbonyl, aminosulfonyl, aryl, arylalkyl, arylsulfonyl, carboxy, cyano, halo, a second heterocyclyl group, heterocyclylalkyl, hydroxy, hydroxyalkyl, nitro, -NR^{c}R^{d}, (NR^{c}R^{d})alkyl, and oxo, wherein the aryl, the aryl part of the arylalkyl and the arylsulfonyl, the second heterocyclyl group, and the heterocyclyl part of the heterocyclylalkyl can be optionally substituted with one, two, three, four, or five substituents independently selected from the group consisting of alkoxy, alkoxyalkyl, alkoxycarbonyl, alkyl, alkylcarbonyl, carboxy, cyano, halo, haloalkoxy, haloalkyl, hydroxy, methylenedioxy, nitro, and oxo;
      cycloalkyl is a saturated monocyclic, bicyclic, or tricyclic hydrocarbon ring system having three to twelve carbon atoms, wherein cycloalkyl can be optionally substituted with one, two, three, or four substituents independently selected from the group consisting of alkoxy, alkoxyalkyl, alkoxycarbonyl, alkyl, alkylcarbonyl, alkylsulfonyl, -NR^{c}R^{d}, (NR^{c}R^{d})alkyl, (NR^{c}R^{d})carbonyl, a second aryl group, arylalkyl, arylsulfonyl, carboxy, cyano, halo, heterocyclyl, heterocyclylalkyl, hydroxy, hydroxyalkyl, nitro, and oxo, wherein the second aryl group, the aryl part of the arylalkyl and the arylsulfonyl, the heterocyclyl, and the heterocyclyl part of the heterocyclylalkyl can be optionally substituted with one, two, three, four, or five substituents independently selected from the group consisting of alkoxy, alkoxyalkyl, alkoxycarbonyl, alkyl, alkylcarbonyl, carboxy, cyano, halo, haloalkoxy, haloalkyl, hydroxy, nitro, and oxo;
      cycloalkenyl is a non-aromatic cyclic or bicyclic ring system having three to ten carbon atoms and one to three rings, wherein each five-membered ring has one double bond, each six-membered ring has one or two double bonds, each seven-and eight-membered ring has one to three double bonds, and each nine-to ten-membered ring has one to four double bonds;
      amino refers to -NR^{a}R^{b}, wherein R^{a} and R^{b} are independently selected from the group consisting of hydrogen, alkoxyalkylcarbonyl, alkoxycarbonyl, alkyl, alkylcarbonyl, alkylsulfonyl, aryl, arylalkyl, arylalkylcarbonyl, arylalkylsulfonyl, arylsulfonyl, arylsulfonylalkyl, arylsulfonylalkylcarbonyl, cycloalkyl, cycloalkylalkyl, cycloalkylcarbonyl, haloalkylsulfonyl, heterocyclyl, heterocyclylalkylcarbonyl, heterocyclylalkylsulfonyl, heterocyclylcarbonyl, heterocyclylsulfonyl, -NR^{c}R^{d}, (NR^{c}R^{d})alkyl, (NR^{c}R^{d})alkylcarbonyl, (NR^{c}R^{d})alkylsulfonyl, (NR^{c}R^{d})carbonyl, (NR^{c}R^{d})carbonylalkyl, and (NR^{c}R^{d})carbonylalkylcarbonyl, wherein aryl can be optionally substituted with one, two, three, four, or five substituents independently selected from the group consisting of alkoxy, alkoxyalkyl, alkoxycarbonyl, alkyl, alkylcarbonyl, alkylsulfonyl, a second aryl group, arylalkyl, arylsulfonyl, carboxy, cyano, halo, heterocyclyl, heterocyclylalkyl, hydroxy, nitro, and oxo, wherein the second aryl group, the aryl part of the arylalkyl and the arylsulfonyl, the heterocyclyl, and the heterocyclyl part of the heterocyclylalkyl can be optionally substituted with one, two, three, four, or five substituents independently selected from the group consisting of alkoxy, alkoxyalkyl, alkoxycarbonyl, alkyl, alkylcarbonyl, carboxy, cyano, halo, haloalkoxy, haloalkyl, hydroxy, nitro, and oxo; and
      R^{c} and R^{d} are each independently hydrogen, alkoxycarbonyl, alkyl, alkylcarbonyl, aryl, arylsulfonyl, heterocyclcarbonyl, or formyl;
      provided that 3-chloro-11-oxo-10,11-dihydro-5H-dibenzo[b,e][1,4]diazepine and 3-fluoro-11-oxo-10,11-dihydro-5H-dibenzo[b,e][1,4]diazepine are excluded.

In another embodiment, the present invention provides a compound of formula (I) wherein
A¹ is CR¹;
A² is CR⁸;
Y is NR¹⁵;
R¹, R², R³, R⁴, R⁵, R⁷, R⁸, and R¹⁵ are hydrogen; and
R⁶ is as defined in formula (I).

In another embodiment, the present invention provides a compound of formula (I) wherein
A¹ is CR¹;
A² is CR⁸;
Y is NR¹⁵;
R¹, R³, R⁴, R⁵ R⁷, R⁸, and R¹⁵ are hydrogen;
R² is selected from the group consisting of alkoxycarbonyl and alkoxycarbonylalkyl; and
R⁶ is as defined in formula (I).

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is selected from the group consisting of amino and aryl; and
R⁶ is as defined in formula (I).

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is selected from the group consisting of carboxy, carboxyalkyl, cyano, nitro, and heterocyclyl; and
R⁶ is as defined in formula (I).

In another embodiment, the present invention provides a compound of formula (I) wherein
A¹ is CR¹;
A² is CR⁸;
Y is NR¹⁵; and
R¹, R³, R⁴, R⁵, R⁷, R⁸, and R¹⁵ are hydrogen;
R² is -(CR⁹R¹⁰)ₘC(O)NR¹¹R¹²;
m is 0;
one of R¹¹ and R¹² is hydrogen and the other is heterocyclylalkyl; and
R⁶ is as defined in formula (I).

In another embodiment, the present invention provides a compound of formula (I) wherein
A¹ is CR¹;
A² is CR⁸;
Y is NR¹⁵; and
R¹, R³, R⁴, R⁵, R⁷, R⁸, and R¹⁵ are hydrogen;
R² is -(CR⁹R¹⁰)ₘC(O)NR¹¹R¹²;
m is 0;
one of R¹¹ and R¹² is hydrogen and the other is selected from the group consisting of amino, aminoalkyl, arylalkyl, and hydroxyalkyl; and
R⁶ is as defined in formula (I).

In another embodiment, the present invention provides a compound of formula (I) wherein
A¹ is CR¹;
A² is CR⁸;
Y is NR¹⁵; and
R¹, R³, R⁴, R⁵, R⁷, R⁸, and R¹⁵ are hydrogen;
R² is -(CR⁹R¹⁰)ₘC(O)NR¹¹R¹²;
m is 0;
R¹¹ and R¹², together with the nitrogen atom to which they are attached, form a heterocyclyl ring selected from the group consisting of diazepanyl, imidazolidinyl, morpholinyl, piperazinyl, piperidinyl, and pyrrolidinyl, which can each be optionally substituted with one, two, or three substituents independently selected from the group consisting of alkoxy, alkoxycarbonyl, alkenyl, alkyl, alkylcarbonyl, aryl, carboxy, carboxy, heterocyclyl, heterocyclylalkyl, hydroxy, and hydroxyalkyl; and
R⁶ is as defined in formula (I).

In another embodiment, the present invention provides a compound of formula (I) wherein
A¹ is CR¹;
A² is CR⁸;
Y is NR¹⁵; and
R¹, R³, R⁴, R⁵, R⁷, R⁸, and R¹⁵ are hydrogen;
R² is -(CR⁹R¹⁰)ₘC(O)NR¹¹R¹²;
mis 1;
one of R¹¹ and R¹² is selected from the group consisting of hydrogen and alkyl and the other is heterocyclylalkyl; and
R⁶ is as defined in formula (I).

In another embodiment, the present invention provides a compound of formula (I) wherein
A¹ is CR¹;
A² is CR⁸;
Y is NR¹⁵; and
R¹, R³, R⁴, R⁵ R⁷, R⁸, and R¹⁵ are hydrogen;
R² is -(CR⁹R¹⁰)ₘC(O)NR¹¹R¹²;
m is 1;
one of R¹¹ and R¹² is selected from the group consisting of hydrogen and alkyl and the other is selected from the group consisting of alkoxyalkyl and hydroxyalkyl; and
R⁶ is as defined in formula (I).

In another embodiment, the present invention provides a compound of formula (I) wherein
A¹ is CR¹;
A² is CR⁸;
Y is NR¹⁵; and
R¹, R³, R⁴, R⁵, R⁷, R⁸, and R¹⁵ are hydrogen;
R² is -(CR⁹R¹⁰)ₘC(O)NR¹¹R¹²;
mis 1;
one of R¹¹ and R¹² is selected from the group consisting of hydrogen and alkyl and the other is selected from the group consisting of alkyl, aminoalkyl, aryl, aryalkyl, and heterocyclyl; and
R⁶ is as defined in formula (I).

In another embodiment, the present invention provides a compound of formula (I) wherein
A¹ is CR¹;
A² is CR⁸;
Y is NR¹⁵; and
R¹, R³, R⁴, R⁵, R⁷, R⁸, and R¹⁵ are hydrogen;
R² is -(CR⁹R¹⁰)ₘC(O)NR¹¹R¹²;
m is 1;
R¹¹ and R¹², together with the nitrogen atom to which they are attached, form a heterocyclyl ring selected from the group consisting of diazepanyl, imidazolidinyl, morpholinyl, piperazinyl, piperidinyl, and pyrrolidinyl, which can each be optionally substituted with one, two, or three substituents independently selected from the group consisting of alkoxy, alkoxycarbonyl, alkenyl, alkyl, alkylcarbonyl, aryl, carboxy, carboxy, heterocyclyl, heterocyclylalkyl, hydroxy, and hydroxyalkyl; and
R⁶ is as defined in formula (I).

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is -(CR⁹R¹⁰)ₘC(O)NR¹¹R¹²;
m is 1; and
R¹¹ is hydrogen;
R¹² is aryl wherein the aryl is phenyl optionally substituted with 1 morpholinyl group; and
R⁶ is aryl wherein the aryl is phenyl substituted in the three position with methoxy and substituted in the four position with alkoxycarbonyl, alkoxycarbonylalkenyl, alkylcarbonyl, alkylsulfonyl, cyano, halo, haloalkenyl, hydroxy, nitro, NH₂SO₂-, or NH₂CO-.

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is -(CR⁹R¹⁰)ₘC(O)NR¹¹R¹²;
m is 1; and
R¹¹ is hydrogen;
R¹² is aryl wherein the aryl is phenyl optionally substituted with 1 morpholinyl group; and
R⁶ is aryl wherein the aryl is phenyl substituted in the three position with methoxy and substituted in the four position with a heterocyclyl group.

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is -(CR⁹R¹⁰)ₘC(O)NR¹¹R¹²;
m is 1; and
R¹¹ is hydrogen;
R¹² is aryl wherein the aryl is phenyl optionally substituted with 1 morpholinyl group; and
R⁶ is heterocyclyl.

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is -(CR⁹R¹⁰)ₘC(O)NR¹¹R¹²;
m is 1; and
R¹¹ is hydrogen;
R¹² is aryl wherein the aryl is phenyl optionally substituted with 1 morpholinyl group; and
R⁶ is heterocyclyl wherein the heterocyclyl is pyrazinyl, pyridazinyl, pyridinyl, or pyrimidinyl wherein the pyrazinyl, pyridazinyl, pyridinyl, or pyrimidinyl is optionally substituted with 1 substitutent selected from alkyl, -NH₂, halo, methoxy or hydroxy.

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is hydroxyalkyl; and
R⁶ is aryl wherein the aryl is phenyl substituted in the three position with methoxy and substituted in the four position with alkoxycarbonyl, alkoxycarbonylalkenyl, alkylcarbonyl, alkylsulfonyl, cyano, halo, haloalkenyl, hydroxy, nitro, NH₂SO₂-, or NH₂CO-.

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is hydroxyalkyl; and
R⁶ is aryl wherein the aryl is phenyl substituted in the three position with methoxy and substituted in the four position with a heterocyclyl group.

In another embodiment, the present invention provides a compound of formula (I) wherein
A¹ is CR¹;
A² is CR⁸;
Y is NR¹⁵; and
R¹, R³, R⁴, R⁵, R⁷, R⁸, and R¹⁵ are hydrogen;
R² is hydroxyalkyl; and
R⁶ is heterocyclyl.

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is hydroxyalkyl; and
R⁶ is heterocyclyl wherein the heterocyclyl is pyrazinyl, pyridazinyl, pyridinyl, or pyrimidinyl wherein the pyrazinyl, pyridazinyl, pyridinyl, or pyrimidinyl is optionally substituted with 1 substituent selected from alkyl, -NH₂, halo, methoxy or hydroxy.

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is alkoxy; and
R⁶ is aryl wherein the aryl is phenyl substituted in the three position with methoxy and substituted in the four position with alkoxycarbonyl, alkoxycarbonylalkenyl, alkylcarbonyl, alkylsulfonyl, cyano, halo, haloalkenyl, hydroxy, nitro, NH₂SO₂-, or NH₂CO-.

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is alkoxy; and
R⁶ is aryl wherein the aryl is phenyl substituted in the three position with methoxy and substituted in the four position with a heterocyclyl group.

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is alkoxy; and
R⁶ is heterocyclyl.

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is alkoxy; and
R⁶ is heterocyclyl wherein the heterocyclyl is pyrazinyl, pyridazinyl, pyridinyl, or pyrimidinyl wherein the pyrazinyl, pyridazinyl, pyridinyl, or pyrimidinyl is optionally substituted with 1 substituent selected from alkyl, -NH₂, halo, methoxy or hydroxy.

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is arylalkoxy; and
R⁶ is aryl wherein the aryl is phenyl substituted in the three position with methoxy and substituted in the four position with alkoxycarbonyl, alkoxycarbonylalkenyl, alkylcarbonyl, alkylsulfonyl, cyano, halo, haloalkenyl, hydroxy, nitro, NH₂SO₂-, or NH₂CO-.

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is arylalkoxy; and
R⁶ is aryl wherein the aryl is phenyl substituted in the three position with methoxy and substituted in the four position with a heterocyclyl group.

In another embodiment, the present invention provides a compound of formula (I) wherein
A¹ is CR¹;
A² is CR⁸;
Y is NR¹⁵; and
R¹, R³, R⁴, R⁵, R⁷, R⁸, and R¹⁵ are hydrogen;
R² is arylalkoxy; and
R⁶ is heterocyclyl.

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is arylalkoxy; and
R⁶ is heterocyclyl wherein the heterocyclyl is pyrazinyl, pyridazinyl, pyridinyl, or pyrimidinyl wherein the pyrazinyl, pyridazinyl, pyridinyl, or pyrimidinyl is optionally substituted with 1 substituent selected from alkyl, -NH₂, halo, methoxy or hydroxy.

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is aryloxyalkyl wherein the aryl is phenyl substituted with 1 morpholinyl group; and
R⁶ is aryl wherein the aryl is phenyl substituted in the three position with methoxy and substituted in the four position with alkoxycarbonyl, alkoxycarbonylalkenyl, alkylcarbonyl, alkylsulfonyl, cyano, halo, haloalkenyl, hydroxy, nitro, NH₂SO₂-, or NH₂CO-.

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is aryloxyalkyl wherein the aryl is phenyl substituted with 1 morpholinyl group; and
R⁶ is aryl wherein the aryl is phenyl substituted in the three position with methoxy and substituted in the four position with a heterocyclyl group.

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is aryloxyalkyl wherein the aryl is phenyl substituted with 1 morpholinyl group; and
R⁶ is heterocyclyl.

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is aryloxyalkyl wherein the aryl is phenyl substituted with 1 morpholinyl group; and
R⁶ is heterocyclyl wherein the heterocyclyl is pyrazinyl, pyridazinyl, pyridinyl, or pyrimidinyl wherein the pyrazinyl, pyridazinyl, pyridinyl, or pyrimidinyl is optionally substituted with 1 substituent selected from alkyl, -NH₂, halo, methoxy or hydroxy.

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is heterocyclyloxyalkyl; and
R⁶ is aryl wherein the aryl is phenyl substituted in the three position with methoxy and substituted in the four position with alkoxycarbonyl, alkoxycarbonylalkenyl, alkylcarbonyl, alkylsulfonyl, cyano, halo, haloalkenyl, hydroxy, nitro, NH₂SO₂-, or NH₂CO-.

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is heterocyclyloxyalkyl; and
R⁶ is aryl wherein the aryl is phenyl substituted in the three position with methoxy and substituted in the four position with a heterocyclyl group.

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is heterocyclyloxyalkyl; and
R⁶ is heterocyclyl.

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is heterocyclyloxyalkyl; and
R⁶ is heterocyclyl wherein the heterocyclyl is pyrazinyl, pyridazinyl, pyridinyl, or pyrimidinyl wherein the pyrazinyl, pyridazinyl, pyridinyl, or pyrimidinyl is optionally substituted with 1 substituent selected from alkyl, -NH₂, halo, methoxy or hydroxy.

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is heterocyclyloxyalkyl wherein the heterocyclyl is pyridinyl optionally substituted with 1 substituent selected from alkyl, halo, or hydroxy; and
R⁶ is aryl wherein the aryl is phenyl substituted in the three position with methoxy and substituted in the four position with alkoxycarbonyl, alkoxycarbonylalkenyl, alkylcarbonyl, alkylsulfonyl, cyano, halo, haloalkenyl, hydroxy, nitro, NH₂SO₂-, or NH₂CO-.

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is heterocyclyloxyalkyl wherein the heterocyclyl is pyridinyl optionally substituted with 1 substituent selected from alkyl, halo, or hydroxy; and
R⁶ is aryl wherein the aryl is phenyl substituted in the three position with methoxy and substituted in the four position with a heterocyclyl group.

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is heterocyclyloxyalkyl wherein the heterocyclyl is pyridinyl optionally substituted with 1 substituent selected from alkyl, halo, or hydroxy; and
R⁶ is heterocyclyl.

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is heterocyclyloxyalkyl wherein the heterocyclyl is pyridinyl optionally substituted with 1 substituent selected from alkyl, halo, or hydroxy; and
R⁶ is heterocyclyl wherein the heterocyclyl is pyrazinyl, pyridazinyl, pyridinyl, or pyrimidinyl wherein the pyrazinyl, pyridazinyl, pyridinyl, or pyrimidinyl is optionally substituted 1 substituent selected from with alkyl, -NH₂, halo, methoxy or hydroxy.

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is heterocyclylalkyl; and
R⁶ is aryl wherein the aryl is phenyl substituted in the three position with methoxy and substituted in the four position with alkoxycarbonyl, alkoxycarbonylalkenyl, alkylcarbonyl, alkylsulfonyl, cyano, halo, haloalkenyl, hydroxy, nitro, NH₂SO₂-, or NH₂CO-.

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is heterocyclylalkyl; and
R⁶ is aryl wherein the aryl is phenyl substituted in the three position with methoxy and substituted in the four position with a heterocyclyl group.

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is heterocyclylalkyl; and
R⁶ is heterocyclyl.

In another embodiment, the present invention provides a compound of formula (I) wherein
A¹ is CR¹;
A² is CR⁸;
Y is NR¹⁵;
R¹, R³, R⁴, R⁵, R⁷, R⁸, and R¹⁵ are hydrogen;
R² is heterocyclylalkyl; and
R⁶ is heterocyclyl wherein the heterocyclyl is pyrazinyl, pyridazinyl, pyridinyl, or pyrimidinyl wherein the pyrazinyl, pyridazinyl, pyridinyl, or pyrimidinyl is optionally substituted with 1 substituent selected from alkyl, -NH₂, halo, methoxy or hydroxy.

In another embodiment, the present invention provides a compound of formula (I) wherein
A¹ is CR¹;
A² is CR⁸;
Y is NR¹⁵;
R¹, R², R⁴, R⁵, R⁷, R⁸, and R¹⁵ are hydrogen;
R³ is -(CR⁹R¹⁰)ₘC(O)NR¹¹R¹²;
m is 1; and
R¹¹ is hydrogen;
R¹² is aryl wherein the aryl is phenyl optionally substituted with 1 morpholinyl group; and
R⁶ is aryl wherein the aryl is phenyl substituted in the three position with methoxy and substituted in the four position with alkoxycarbonyl, alkoxycarbonylalkenyl, alkylcarbonyl, alkylsulfonyl, cyano, halo, haloalkenyl, hydroxy, nitro, NH₂SO₂-, or NH₂CO-.

In another embodiment, the present invention provides a compound of formula (I) wherein
A¹ is CR¹;
A² is CR⁸;
Y is NR¹⁵;
R¹, R², R⁴, R⁵, R⁷, R⁸ and R¹⁵ are hydrogen;
R³ is hydroxyalkyl; and
R⁶ is aryl wherein the aryl is phenyl substituted in the three position with methoxy and substituted in the four position with alkoxycarbonyl, alkoxycarbonylalkenyl, alkylcarbonyl, alkylsulfonyl, cyano, halo, haloalkenyl, hydroxy, nitro, NH₂SO₂-, or NH₂CO-.

In another embodiment, the present invention provides a compound of formula (I) wherein
A¹ is CR¹;
A² is CR⁸;
Y is NR¹⁵; and
R¹, R², R⁴, R⁵, R⁷, R⁸, and R¹⁵ are hydrogen;
R³ is alkoxy;
R⁶ is aryl wherein the aryl is phenyl substituted in the three position with methoxy and substituted in the four position with alkoxycarbonyl, alkoxycarbonylalkenyl, alkylcarbonyl, alkylsulfonyl, cyano, halo, haloalkenyl, hydroxy, nitro, NH₂SO₂-, or NH₂CO-.

In another embodiment, the present invention provides a compound of formula (I) wherein
A¹ is CR¹;
A² is CR⁸;
Y is NR¹⁵;
R¹, R⁴, R⁵, R⁷, R⁸, and R¹⁵ are hydrogen;
R² is -(CR⁹R¹⁰)ₘC(O)NR¹¹R¹²;
R³ is -(CR⁹R¹⁰)ₘC(O)NR¹¹R¹²;
m is 1; and
R¹¹ is hydrogen;
R¹² is aryl wherein the aryl is phenyl optionally substituted with 1 morpholinyl group; and
R⁶ is aryl wherein the aryl is phenyl substituted in the three position with methoxy and substituted in the four position with alkoxycarbonyl, alkoxycarbonylalkenyl, alkylcarbonyl, alkylsulfonyl, cyano, halo, haloalkenyl, hydroxy, nitro, NH₂SO₂-, or NH₂CO-.

In another embodiment, the present invention provides a compound of formula (I) wherein
A¹ is CR¹;
A² is CR⁸;
Y is NR¹⁵;
R¹, R⁴, R⁵, R⁷, R⁸, and R¹⁵ are hydrogen;
R² is hydroxyalkyl;
R³ is hydroxyalkyl; and
R⁶ is aryl wherein the aryl is phenyl substituted in the three position with methoxy and substituted in the four position with alkoxycarbonyl, alkoxycarbonylalkenyl, alkylcarbonyl, alkylsulfonyl, cyano, halo, haloalkenyl, hydroxy, nitro, NH₂SO₂-, or NH₂CO-.

In another embodiment, the present invention provides a compound of formula (I) wherein
A¹ is CR¹_{;}
A² is CR⁸_{;}
Y is NR¹⁵;
R¹, R⁴, R⁵, R⁷, R⁸, and R¹⁵ are hydrogen;
R² is alkoxy;
R³ is alkoxy; and
R⁶ is aryl wherein the aryl is phenyl substituted in the three position with methoxy and substituted in the four position with alkoxycarbonyl, alkoxycarbonylalkenyl, alkylcarbonyl, alkylsulfonyl, cyano, halo, haloalkenyl, hydroxy, nitro, NH₂SO₂-, or NH₂CO-.

In another embodiment, the present invention provides a pharmaceutical composition comprising a compound of formula (I) 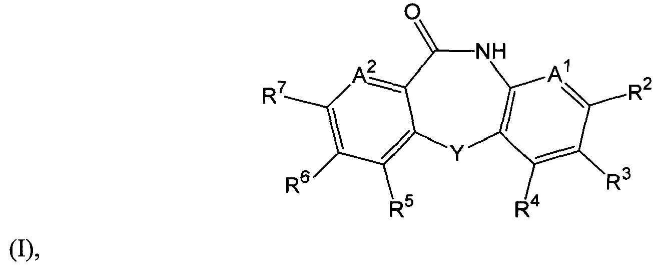 or a therapeutically acceptable salt thereof, in combination with a therapeutically acceptable carrier,wherein
(a)
   A¹ is CH;
   A² is CH;
   R² is selected from the group consisting of hydrogen, alkenyl, alkoxy, alkoxyalkoxy, alkoxyalkoxyalkoxy, alkoxyalkyl, alkoxycarbonyl, alkoxycarbonylalkyl, alkyl, alkylcarbonyl, alkylcarbonylalkyl, amino, aminoalkoxy, aminoalkyl, aminocarbonyl, aminocarbonylalkyl, aminosulfonyl, aryl, arylalkoxy, arylalkyl, aryloxyalkyl, carboxy, carboxyalkyl, cyano, cyanoalkyl, cycloalkyl, cycloalkylalkyl, halo, haloalkoxy, haloalkyl, heterocyclyl, heterocyclylalkoxy, heterocyclylalkyl, heterocyclylcarbonylalkyl, heterocyclyloxyalkyl, hydroxy, hydroxyalkoxy, hydroxyalkyl, nitro, nitroalkyl, and -(CR⁹R¹⁰)ₘC(O)NR¹¹R¹²;
   R³, R⁴ and R⁵ are each hydrogen;
   R⁶ is selected from the group consisting of aryl, cycloalkyl, halo, heterocyclyl, and -XR¹³;
   R⁷ is hydrogen;
   R⁹ and R¹⁰ are independently selected from the group consisting of hydrogen, alkenyl, alkyl, aminoalkyl, and hydroxyalkyl; or
   R⁹ and R¹⁰, together with the carbon atom to which they are attached, form a cycloalkyl group;
   R¹¹ and R¹² are each independently selected from the group consisting of hydrogen, alkenyl, alkoxyalkyl, alkyl, amino, aminoalkyl, aryl, arylalkyl, cyanoalkyl, cycloalkyl, cycloalkylalkyl, haloalkyl, heterocyclyl, heterocyclylalkyl, hydroxyalkyl, -NR^{c}R^{d}, (NR^{c}R^{d})alkyl, and (NR^{c}R^{d})carbonylalkyl; or
   R¹¹ and R¹², together with the nitrogen atom to which they are attached, form a heterocyclyl ring selected from the group consisting of azetidinyl, diazepanyl, imidazolidinyl, morpholinyl, piperazinyl, piperidinyl, pyrrolidinyl and thiomorpholinyl, which can each be optionally substituted with one, two, or three substituents independently selected from the group consisting of alkoxy, alkoxycarbonyl, alkenyl, alkyl, alkylcarbonyl, aryl, carboxy, carboxyalkyl, heterocyclyl, heterocyclylalkyl, hydroxy, hydroxyalkyl, -NR^{c}R^{d}, (NR^{c}R^{d})alkyl, and (NR^{c}R^{d})carbonyl;
   R¹³ is selected from the group consisting of aryl, cycloalkyl, and heterocyclyl;
   X is selected from the group consisting of -O-, -NR¹⁴-, -C(O)-, -S-, -SO₂-, -(CH₂)ₙ-, -C(O)NR¹⁴-, -NR¹⁴C(O)-, -SO₂NR¹⁴-, -NR¹⁴SO₂, -O(CH₂)ₘ-, -(CH₂)ₘO-, -CH=CH-, and -C≡C-; wherein R¹⁴ is selected from the group consisting of hydrogen, alkenyl, alkyl, aminoalkyl, and hydroxyalkyl;
   Y is NH;
   m is 0-3; and
   n is 1-3;
   or wherein
(b)
   A¹ is CH;
   A² is CH;
   Y is NH;
   R⁴, R⁵, and R⁷ are each hydrogen;
   R⁶ is phenyl substituted in the three position with methoxy and substituted in the four position with alkoxycarbonyl, alkoxycarbonylalkenyl, alkylcarbonyl, alkylsulfonyl, cyano, halo, haloalkenyl, hydroxy, nitro, NH₂SO₂-, or NH₂CO-; and either
      (i) R² is hydrogen and R³ is -(CR⁹R¹⁰)C(O)NHR¹² where R⁹ and R¹⁰ are independently selected from the group consisting of hydrogen, alkenyl, alkyl, aminoalkyl, and hydroxyalkyl, or R⁹ and R¹⁰, together with the carbon atom to which they are attached, form a cycloalkyl group, and R¹² is phenyl optionally substituted with 1 morpholinyl group;
      (ii) R² is hydrogen and R³ is hydroxyalkyl;
      (iii) R² is hydrogen and R³ is alkoxy;
      (iv) R² and R³ are independently -(CR⁹R¹⁰)C(O)NHR¹² where R⁹ and R¹⁰ are independently selected from the group consisting of hydrogen, alkenyl, alkyl, aminoalkyl, and hydroxyalkyl, or R⁹ and R¹⁰, together with the carbon atom to which they are attached, form a cycloalkyl group, and R¹² is phenyl optionally substituted with 1 morpholinyl group;
      (v) R² and R³ are independently hydroxalkyl; or
      (vi) R² and R³ are independently alkoxy;
   wherein
   alkyl is a C₁-C₁₀ alkyl;
   alkenyl is a C₂-C₆ alkenyl;
   alkoxy is a C₁-C₁₀ alkoxy;
   aryl is phenyl group, or a bicyclic or tricyclic fused ring system wherein one or more of the fused rings is a phenyl group, wherein aryl can be optionally substituted with one, two, three, four, or five substituents independently selected from the group consisting of alkoxy, alkoxyalkyl, alkoxycarbonyl, alkoxycarbonylalkenyl, alkoxycarbonylalkyl, alkoxysulfonyl, alkyl, alkylcarbonyl, alkylsulfonyl, amino, aminocarbonyl, aminosulfonyl, a second aryl group, arylalkyl, arylsulfonyl, carboxy, cyano, formyl, halo, haloalkoxy, haloalkenyl, haloalkyl, heterocyclyl, heterocyclylalkyl, hydroxy, hydroxyalkyl, nitro, (NR^{c}R^{d})alkyl, and oxo, wherein the second aryl group, the aryl part of the arylalkyl and the arylsulfonyl, the heterocyclyl, and the heterocyclyl part of the heterocyclylalkyl can be optionally substituted with one, two, three, four, or five substituents independently selected from the group consisting of alkoxy, alkoxyalkyl, alkoxycarbonyl, alkyl, alkylcarbonyl, carboxy, cyano, halo, haloalkoxy, haloalkyl, hydroxy, nitro, and oxo;
   heterocyclyl is selected from a five-, six-, or seven-membered ring containing one, two, or three heteroatoms independently selected from the group consisting of nitrogen, oxygen, and sulfur, wherein the five-membered ring has zero to two double bonds and the six- and seven-membered rings have zero to three double bonds; bicyclic groups in which the heterocyclyl ring is fused to a phenyl group, a monocyclic cycloalkenyl group, a monocyclic cycloalkyl group, or another monocyclic heterocyclyl group; and tricyclic groups in which a bicyclic system is fused to a phenyl group, a monocyclic cycloalkenyl group, a monocyclic cycloalkyl group, or another monocyclic heterocyclyl group; wherein heterocyclyl can be optionally substituted with one, two, three, four, or five substituents independently selected from the group consisting of alkoxy, alkoxyalkyl, alkoxycarbonyl, alkyl, alkylcarbonyl, alkylsulfonyl, amino, aminocarbonyl, aminosulfonyl, aryl, arylalkyl, arylsulfonyl, carboxy, cyano, halo, a second heterocyclyl group, heterocyclylalkyl, hydroxy, hydroxyalkyl, nitro, -NR^{c}R^{d}, (NR^{c}R^{d})alkyl, and oxo, wherein the aryl, the aryl part of the arylalkyl and the arylsulfonyl, the second heterocyclyl group, and the heterocyclyl part of the heterocyclylalkyl can be optionally substituted with one, two, three, four, or five substituents independently selected from the group consisting of alkoxy, alkoxyalkyl, alkoxycarbonyl, alkyl, alkylcarbonyl, carboxy, cyano, halo, haloalkoxy, haloalkyl, hydroxy, methylenedioxy, nitro, and oxo;
   cycloalkyl is a saturated monocyclic, bicyclic, or tricyclic hydrocarbon ring system having three to twelve carbon atoms, wherein cycloalkyl can be optionally substituted with one, two, three, or four substituents independently selected from the group consisting of alkoxy, alkoxyalkyl, alkoxycarbonyl, alkyl, alkylcarbonyl, alkylsulfonyl, -NR^{c}R^{d}, (NR^{c}R^{d})alkyl, (NR^{c}R^{d})carbonyl, a second aryl group, arylalkyl, arylsulfonyl, carboxy, cyano, halo, heterocyclyl, heterocyclylalkyl, hydroxy, hydroxyalkyl, nitro, and oxo, wherein the second aryl group, the aryl part of the arylalkyl and the arylsulfonyl, the heterocyclyl, and the heterocyclyl part of the heterocyclylalkyl can be optionally substituted with one, two, three, four, or five substituents independently selected from the group consisting of alkoxy, alkoxyalkyl, alkoxycarbonyl, alkyl, alkylcarbonyl, carboxy, cyano, halo, haloalkoxy, haloalkyl, hydroxy, nitro, and oxo;
   cycloalkenyl is a non-aromatic cyclic or bicyclic ring system having three to ten carbon atoms and one to three rings, wherein each five-membered ring has one double bond, each six-membered ring has one or two double bonds, each seven-and eight-membered ring has one to three double bonds, and each nine-to ten-membered ring has one to four double bonds;
   amino refers to -NR^{a}R^{b}, wherein R^{a} and R^{b} are independently selected from the group consisting of hydrogen, alkoxyalkylcarbonyl, alkoxycarbonyl, alkyl, alkylcarbonyl, alkylsulfonyl, aryl, arylalkyl, arylalkylcarbonyl, arylalkylsulfonyl, arylsulfonyl, arylsulfonylalkyl, arylsulfonylalkylcarbonyl, cycloalkyl, cycloalkylalkyl, cycloalkylcarbonyl, haloalkylsulfonyl, heterocyclyl, heterocyclylalkylcarbonyl, heterocyclylalkylsulfonyl, heterocyclylcarbonyl, heterocyclylsulfonyl, -NR^{c}R^{d}, (NR^{c}R^{d})alkyl, (NR^{c}R^{d})alkylcarbonyl, (NR^{c}R^{d})alkylsulfonyl, (NR^{c}R^{d})carbonyl, (NR^{c}R^{d})carbonylalkyl, and (NR^{c}R^{d})carbonylalkylcarbonyl, wherein aryl can be optionally substituted with one, two, three, four, or five substituents independently selected from the group consisting of alkoxy, alkoxyalkyl, alkoxycarbonyl, alkyl, alkylcarbonyl, alkylsulfonyl, a second aryl group, arylalkyl, arylsulfonyl, carboxy, cyano, halo, heterocyclyl, heterocyclylalkyl, hydroxy, nitro, and oxo, wherein the second aryl group, the aryl part of the arylalkyl and the arylsulfonyl, the heterocyclyl, and the heterocyclyl part of the heterocyclylalkyl can be optionally substituted with one, two, three, four, or five substituents independently selected from the group consisting of alkoxy, alkoxyalkyl, alkoxycarbonyl, alkyl, alkylcarbonyl, carboxy, cyano, halo, haloalkoxy, haloalkyl, hydroxy, nitro, and oxo; and
   R^{c} and R^{d} are each independently hydrogen, alkoxycarbonyl, alkyl, alkylcarbonyl, aryl, arylsulfonyl, heterocyclcarbonyl, or formyl.

Also disclosed herein is a method for inhibiting protein kinases in a patient in recognized need of such treatment comprising administering to the patient a therapeutically acceptable amount of a compound of formula (I), or a therapeutically acceptable salt thereof.

In addition, disclosed herein is a method for treating cancer in a patient in recognized need of such treatment comprising administering to the patient a therapeutically acceptable amount of a compound of formula (I), or a therapeutically acceptable salt thereof.

### Detailed Description of the Invention

As used in the present specification the following terms have the meanings indicated:
As used herein, the singular forms "a", "an", and "the" include plural reference unless the context clearly dictates otherwise.

The term "alkenyl," as used herein, refers to a straight or branched chain group of two to six carbon atoms containing at least one carbon-carbon double bond.

The term "alkoxy," as used herein, refers to an alkyl group attached to the parent molecular moiety through an oxygen atom.

The term "alkoxyalkoxy," as used herein, refers to an alkoxy group attached to the parent molecular moiety through an alkoxy group.

The term "alkoxyalkoxyalkoxy," as used herein, refers to an alkoxyalkoxy group attached to the parent molecular moiety through an alkoxy group.

The term "alkoxyalkyl," as used herein, refers to an alkoxy group attached to the parent molecular moiety through an alkyl group.

The term "alkoxycarbonyl," as used herein, refers to an alkoxy group attached to the parent molecular moiety through a carbonyl group.

The term "alkoxycarbonylalkenyl," as used herein, refers to an alkoxycarbonyl group attached to the parent molecular moiety through an alkenyl group.

The term "alkoxycarbonylalkyl," as used herein, refers to an alkoxycarbonyl group attached to the parent molecular moiety through an alkyl group.

The term "alkyl," as used herein, refers to a group derived from a straight or branched chain saturated hydrocarbon containing from one to ten carbon atoms.

The term "alkylcarbonyl," as used herein, refers to an alkyl group attached to the parent molecular moiety through a carbonyl group.

The term "alkylcarbonylalkyl," as used herein, refers to an alkylcarbonyl group attached to the parent molecular moiety through an alkyl group.

The term "alkylsulfonyl," as used herein, refers to an alkyl group attached to the parent molecular moiety through a sulfonyl group.

The term "amino," as used herein, refers to -NR^{a}R^{b}, wherein R^{a} and R^{b} are independently selected from the group consisting of hydrogen, alkoxyalkylcarbonyl, alkoxycarbonyl, alkyl, alkylcarbonyl, alkylsulfonyl, aryl, arylalkyl, arylalkylcarbonyl, arylalkylsulfonyl, arylsulfonyl, arylsulfonylalkyl, arylsulfonylalkylcarbonyl, cycloalkyl, cycloalkylalkyl, cycloalkylcarbonyl, haloalkylsulfonyl, heterocyclyl, heterocyclylalkylcarbonyl, heterocyclylalkylsulfonyl, heterocyclylcarbonyl, heterocyclylsulfonyl, -NR^{c}R^{d}, (NR^{c}R^{d})alkyl, (NR^{c}R^{d})alkylcarbonyl, (NR^{c}R^{d})alkylsulfonyl, (NR^{c}R^{d})carbonyl, (NR^{c}R^{d})carbonylalkyl, and (NR^{c}R^{d})carbonylalkylcarbonyl, wherein the aryl groups of the present invention can be optionally substituted with one, two, three, four, or five substituents independently selected from the group consisting of alkoxy, alkoxyalkyl, alkoxycarbonyl, alkyl, alkylcarbonyl, alkylsulfonyl, a second aryl group, arylalkyl, arylsulfonyl, carboxy, cyano, halo, heterocyclyl, heterocyclylalkyl, hydroxy, nitro, and oxo, wherein the second aryl group, the aryl part of the arylalkyl and the arylsulfonyl, the heterocyclyl, and the heterocyclyl part of the heterocyclylalkyl can be optionally substituted with one, two, three, four, or five substituents independently selected from the group consisting of alkoxy, alkoxyalkyl, alkoxycarbonyl, alkyl, alkylcarbonyl, carboxy, cyano, halo, haloalkoxy, haloalkyl, hydroxy, nitro, and oxo. Representative examples of amino include, but are not limited to, -NH₂, methylamino, dimethylamino, diethylamino, ethylmethylamino, (4-aminobutanoyl)amino, (3-aminopropanoyl)amino, [(2S)-2-amino-4-methylpentanoyl]amino, [(2R)-2-amino-4-methylpentanoyl]amino, (2-amino-4-methylpentanoyl)amino, [(dimethylamino)acetyl]amino, [(1-methyl-1H-imidazol-5-yl)acetyl]amino, (1H-imidazol-4-ylacetyl)amino, thien-3-ylcarbonylamino, thien-2-ylcarbonylamino, (1H-pyrrol-3-ylcarbonyl)amino, (1H-pyrrol-2-ylcarbonyl)amino, [(2,5-dimethyl-1H-pyrrol-3-yl)carbonyl]amino, (1,3-thiazol-4-ylcarbonyl)amino, (1H-pyrazol-5-ylcarbonyl)amino, (1H-pyrazol-4-ylcarbonyl)amino, isonicotinoylamino, (3-pyrrolidin-1-ylpropanoyl)amino, (3-piperidin-1-ylpropanoyl)amino, (3-morpholin-4-ylpropanoyl)amino, [3-(phenylsulfonyl)propanoyl]amino, ({[(4-methylphenyl)sulfonyl]amino}acetyl)amino, (pyridin-2-ylcarbonyl)amino, (pyridin-3-ylcarbonyl)amino, (pyridin-3-ylacetyl)amino, [(4-methylpiperazin-1-yl)acetyl]amino, {[(2S)-5-oxopyrrolidin-2-yl]carbonyl}amino, {[(2R)-5-oxopyrrolidin-2-yl)carbonyl}amino, [(2-furoylamino)acetyl]amino, [(2S)-2-hydroxy-2-phenylethanoyl]amino, [(2R)-2-hydroxy-2-phenylethanoyl]amino, [(2-hydroxy-2-phenylethanoyl]amino, (3-piperidin-1-ylpropanoyl)amino, [(3-chloropropyl)sulfonyl]amino, (benzylsulfonyl)amino, [(2,2,2-trifluoroethyl)sulfonyl] amino, [(1-methyl-1H-imidazol-4-yl)sulfonyl]amino, [(3-morpholin-4-ylpropyl)sulfonyl]amino, [(3-piperidin-1-ylpropyl)sulfonyl]amino, ([3-(diethylamino)propyl]sulfonyl)amino, {[3-(dimethylamino)propyl]sulfonyl} amino, [(chloromethyl)sulfonyl]amino, (4-morpholin-4-ylbenzoyl)amino, (tetrahydro-2H-pyran-4-ylcarbonyl)amino, cis [(4-hydroxycyclohexyl)carbonyl]amino, and [2-(dimethylamino)ethyl](methyl)amino.

The term "aminoalkoxy," as used herein, refers to an amino group attached to the parent molecular moiety through an alkoxy group.

The term "aminoalkyl," as used herein, refers to an amino group attached to the parent molecular moiety through an alkyl group.

The term "aminocarbonyl," as used herein, refers to an amino group attached to the parent molecular moiety through a carbonyl group.

The term "aminocarbonylalkyl," as used herein, refers to an aminocarbonyl group attached to the parent molecular moiety through an alkyl group.

The term "aminosulfonyl," as used herein, refers to an amino group attached to the parent molecular moiety through a sulfonyl group.

The term "aryl," as used herein, refers to a phenyl group, or a bicyclic or tricyclic fused ring system wherein one or more of the fused rings is a phenyl group. Bicyclic fused ring systems are exemplified by a phenyl group fused to a monocyclic cycloalkenyl group, as defined herein, a monocyclic cycloalkyl group, as defined herein, or another phenyl group. Tricyclic fused ring systems are exemplified by a bicyclic fused ring system fused to a monocyclic cycloalkenyl group, as defined herein, a monocyclic cycloalkyl group, as defined herein, or another phenyl group. Representative examples of aryl include, but are not limited to, anthracenyl, azulenyl, fluorenyl, indanyl, indenyl, naphthyl, phenyl, and tetrahydronaphthyl. The aryl groups of the present invention can be optionally substituted with one, two, three, four, or five substituents independently selected from the group consisting of alkoxy, alkoxyalkyl, alkoxycarbonyl, alkoxycarbonylalkenyl, alkoxycarbonylalkyl, alkoxysulfonyl, alkyl, alkylcarbonyl, alkylsulfonyl, amino, aminocarbonyl, aminosulfonyl, a second aryl group, arylalkyl, arylsulfonyl, carboxy, cyano, formyl, halo, haloalkoxy, haloalkenyl, haloalkyl, heterocyclyl, heterocyclylalkyl, hydroxy, hydroxyalkyl, nitro, (NR^{c}R^{d})alkyl, and oxo, wherein the second aryl group, the aryl part of the arylalkyl and the arylsulfonyl, the heterocyclyl, and the heterocyclyl part of the heterocyclylalkyl can be optionally substituted with one, two, three, four, or five substituents independently selected from the group consisting of alkoxy, alkoxyalkyl, alkoxycarbonyl, alkyl, alkylcarbonyl, carboxy, cyano, halo, haloalkoxy, haloalkyl, hydroxy, nitro, and oxo.

The term "arylalkoxy," as used herein, refers to an aryl group attached to the parent molecular moiety through an alkoxy group.

The term "arylalkyl," as used herein, refers to an aryl group attached to the parent molecular moiety through an alkyl group. The alkyl portion of the arylalkyl group can be substituted with at least one substituent selected from the group consisting of alkoxy and hydroxy. Representative examples of arylalkyl include, but are not limited to, benzyl, 2-phenylethyl, 2-hydroxy-4-phenylbutyl, (2R)-2-hydroxy-4-phenylbutyl, and (2S)-2-hydroxy-4-phenylbutyl.

The term "arylalkylsulfonyl," as used herein, refers to an arylalkyl group attached to the parent molecular moiety through a sulfonyl group.

The term "aryloxy," as used herein, refers to an aryl group attached to the parent molecular moiety through an oxygen atom.

The term "aryloxyalkyl," as used herein, refers to an aryloxy group attached to the parent molecular moiety through an alkyl group.

The term "arylsulfonyl," as used herein, refers to an aryl group attached to the parent molecular moiety through a sulfonyl group.

The term "arylsulfonylalkyl," as used herein, refers to an arylsulfonyl group attached to the parent molecular moiety through an alkyl group.

The term "arylsulfonylalkylcarbonyl," as used herein, refers to an arylsulfonylalkyl group attached to the parent molecular moiety through a carbonyl group.

The term "carbonyl," as used herein, refers to -C(O)-.

The term "carboxy," as used herein, refers to -CO₂H.

The term "carboxyalkyl," as used herein, refers to a carboxy group attached to the parent molecular moiety through an alkyl group.

The term "cyano," as used herein, refers to -CN.

The term "cyanoalkyl," as used herein, refers to a cyano group attached to the parent molecular moiety through an alkyl group.

The term "cycloalkenyl," as used herein, refers to a non-aromatic cyclic or bicyclic ring system having three to ten carbon atoms and one to three rings, wherein each five-membered ring has one double bond, each six-membered ring has one or two double bonds, each seven- and eight-membered ring has one to three double bonds, and each nine-to ten-membered ring has one to four double bonds. Examples of cycloalkenyl groups include, but are not limited to, cyclohexenyl, octahydronaphthalenyl, and norbornylenyl.

The term "cycloalkyl," as used herein, refers to a saturated monocyclic, bicyclic, or tricyclic hydrocarbon ring system having three to twelve carbon atoms. Examples of cycloalkyl groups include, but are not limited to, cyclopropyl, cyclopentyl, bicyclo[3.1.1]heptyl, and adamantyl. The cycloalkyl groups of the present invention can be optionally substituted with one, two, three, or four substituents independently selected from the group consisting of alkoxy, alkoxyalkyl, alkoxycarbonyl, alkyl, alkylcarbonyl, alkylsulfonyl, -NR^{c}R^{d}, (NR^{c}R^{d})alkyl, (NR^{c}R^{d})carbonyl, a second aryl group, arylalkyl, arylsulfonyl, carboxy, cyano, halo, heterocyclyl, heterocyclylalkyl, hydroxy, hydroxyalkyl, nitro, and oxo, wherein the second aryl group, the aryl part of the arylalkyl and the arylsulfonyl, the heterocyclyl, and the heterocyclyl part of the heterocyclylalkyl can be optionally substituted with one, two, three, four, or five substituents independently selected from the group consisting of alkoxy, alkoxyalkyl, alkoxycarbonyl, alkyl, alkylcarbonyl, carboxy, cyano, halo, haloalkoxy, haloalkyl, hydroxy, nitro, and oxo.

The term "cycloalkylalkyl," as used herein, refers to a cycloalkyl group attached to the parent molecular moiety through an alkyl group.

The term "cycloalkylcarbonyl," as used herein, refers to a cycloalkyl group attached to the parent molecular moiety through a carbonyl group.

The term "formyl," as used herein, means a -CHO group.

The terms "halo" and "halogen," as used herein, refer to F, Cl, Br, or I.

The term "haloalkoxy," as used herein, refers to a haloalkyl group attached to the parent molecular moiety through an oxygen atom.

The term "haloalkyl," as used herein, refers to an alkyl group substituted by one, two, three, or four halogen atoms.

The term "haloalkenyl," as used herein, refers to an alkenyl group substituted by one, two, three, or four halogen atoms.

The term "haloalkylsulfonyl," as used herein, refers to a haloalkyl group attached to the parent molecular moiety through a sulfonyl group.

The term "heterocyclyl," as used herein, refers to a five-, six-, or seven-membered ring containing one, two, or three heteroatoms independently selected from the group consisting of nitrogen, oxygen, and sulfur. The five-membered ring has zero to two double bonds and the six- and seven-membered rings have zero to three double bonds. The term "heterocyclyl" also includes bicyclic groups in which the heterocyclyl ring is fused to a phenyl group, a monocyclic cycloalkenyl group, as defined herein, a monocyclic cycloalkyl group, as defined herein, or another monocyclic heterocyclyl group, as defined herein; and tricyclic groups in which a bicyclic system is fused to a phenyl group, a monocyclic cycloalkenyl group, as defined herein, a monocyclic cycloalkyl group, as defined herein, or another monocyclic heterocyclyl group. The heterocyclyl groups of the present invention can be attached to the parent molecular moiety through a carbon atom or a nitrogen atom in the group. Examples of heterocyclyl groups include, but are not limited to, 1H-benzimidazolyl, benzofuranyl, 1,3-benzodioxole benzothienyl, 1,3-dioxolane, furyl, imidazolyl, indolinyl, indolyl, isoquinolinyl, isothiazolyl, isoxazolyl, morpholinyl, 1,2,4-oxadiazole, 1,3,4-oxadiazole, oxazolyl, 1,3-oxazolidinyl, piperazinyl, piperidinyl, pyrazolyl, pyridinyl, pyrimidinyl, pyrrolidinyl, pyrrolopyridinyl, pyrrolyl, quinolinyl, 1,2,4-thiadiazole, 1,3,4-thiadiazole, thiazolyl, thienyl, thiomorpholinyl, and the like. The heterocyclyl groups of the present invention can be optionally substituted with one, two, three, four, or five substituents independently selected from the group consisting of alkoxy, alkoxyalkyl, alkoxycarbonyl, alkyl, alkylcarbonyl, alkylsulfonyl, amino, aminocarbonyl, aminosulfonyl, aryl, arylalkyl, arylsulfonyl, carboxy, cyano, halo, a second heterocyclyl group, heterocyclylalkyl, hydroxy, hydroxyalkyl, nitro, -NR^{c}R^{d}, (NR^{c}R^{d})alkyl, and oxo, wherein the aryl, the aryl part of the arylalkyl and the arylsulfonyl, the second heterocyclyl group, and the heterocyclyl part of the heterocyclylalkyl can be optionally substituted with one, two, three, four, or five substituents independently selected from the group consisting of alkoxy, alkoxyalkyl, alkoxycarbonyl, alkyl, alkylcarbonyl, carboxy, cyano, halo, haloalkoxy, haloalkyl, hydroxy, methylenedioxy, nitro, and oxo. Representative examples of a substituted heterocyclyl include, but are not limited to, isothiazolidine 1,1-dioxide, 2-oxopyridin-1(2H)-yl, 5-fluoro-2-oxopyridin-1(2H)-yl, 6-oxopyridazin-1(6H)-yl, 3-methyl-2-oxopyridin-1(2H)-yl, 4-methyl-2-oxopyridin-1(2H)-yl, 5-chloro-2-oxopyridin-1(2H)-yl, 1-oxoisoquinolin-2(1H)-yl, 2-oxo-5-(trifluoromethyl)pyridin-1(2H)-yl, 3-methoxy-2-oxopyridin-1(2H)-yl, 1,4-dioxa-8-azaspiro[4.5]decane, 2-oxopymolidin-1-yl, 2-oxo-1,3-oxazolidin-5-yl, and 2,2-dimethyl-1,3-dioxolan-4-yl.

The term "heterocyclylalkoxy," as used herein, refers to a heterocyclyl group attached to the parent molecular group through an alkoxy group.

The term "heterocyclylalkyl," as used herein, refers to a heterocyclyl group attached to the parent molecular group through an alkyl group.

The term "heterocyclylalkylcarbonyl," as used herein, refers to a heterocyclylalkyl group attached to the parent molecular group through a carbonyl group.

The term "heterocyclylcarbonylalkyl," as used herein, refers to a heterocyclylcarbonyl group attached to the parent molecular group through an alkyl group.

The term "heterocyclylalkylsulfonyl," as used herein, refers to a heterocyclylalkyl group attached to the parent molecular group through a sulfonyl group.

The term "heterocyclylcarbonyl," as used herein, refers to a heterocyclyl group attached to the parent molecular group through a carbonyl group.

The term "heterocyclyloxy," as used herein, refers to a heterocyclyl group attached to the parent molecular group through an oxygen atom.

The term "heterocyclyloxyalkyl," as used herein, refers to a heterocyclyloxy group attached to the parent molecular group through an alkyl group.

The term "heterocyclylsulfonyl," as used herein, refers to a heterocyclyl group attached to the parent molecular group through a sulfonyl group.

The term "hydroxy," as used herein, refers to -OH.

The term "hydroxyalkyl," as used herein, refers to an alkyl group substituted by at least one hydroxy group. The alkyl part of the hydroxyalkyl group can be optionally substituted with an aryl group.

The term "hydroxyalkoxy," as used herein, refers to an alkoxy group substituted by at least one hydroxy group. The hydroxyalkoxy group can be optionally substituted with a -SO₃H group.

The term "methylenedioxy" as used herein, refers to a -OCH₂O- group wherein the oxygen atoms of the methylenedioxy are attached to the parent molecular moiety through two adjacent carbon atoms on a phenyl group or the oxygen atoms of the methylenedioxy are attached to the same single carbon atom on a heterocycle including, but not limited to, azetidinyl, piperindinyl, pyrrolidinyl, and azepanyl.

The term "-NR^{c}R^{d}" as used herein, refers to two groups, R^{c} and R^{d}, which are appended to the parent molecular moiety through a nitrogen atom. R^{c} and R^{d} are each independently hydrogen, alkoxycarbonyl, alkyl, alkylcarbonyl, aryl, arylsulfonyl, heterocyclcarbonyl, or formyl. Representative examples of -NR^{c}R^{d} include, but are not limited to, -NH₂, methylamino, acetylamino, dimethylamino, and acetylmethylamino.

The term "(NR^{c}R^{d})alkyl" as used herein, refers to a -NR^{c}R^{d} group, as defined herein, appended to the parent molecular moiety through an alkyl group, as defined herein. Representative examples of (NR^{c}R^{d})alkyl include, but are not limited to, aminomethyl, (methylamino)methyl, 2-(dimethylamino)ethyl, and (ethylmethylamino)carbonyl.

The term "(NR^{c}R^{d})alkylcarbonyl" as used herein, refers to a (NR^{c}R^{d})alkyl group, as defined herein, appended to the parent molecular moiety through a carbonyl group, as defined herein. Representative examples of (NR^{c}R^{d})alkylcarbonyl include, but are not limited to, (4-aminobutanoyl)amino, (3-aminopropanoyl)amino, [(2S)-2-amino-4-methylpentanoyl]amino, and [(dimethyl amino)acetyl] amino.

The term "(NR^{c}R^{d})carbonyl" as used herein, refers to a -NR^{c}R^{d} group, as defined herein, appended to the parent molecular moiety through a carbonyl group, as defined herein. Representative examples of (NR^{c}R^{d})carbonyl include, but are not limited to, aminocarbonyl, (methylamino)carbonyl, (dimethylamino)carbonyl, and (ethylmethylamino)carbonyl.

The term "(NR^{c}R^{d})carbonylalkyl" as used herein, refers to a (NR^{c}R^{d})carbonyl group, as defined herein, appended to the parent molecular moiety through an alkyl group, as defined herein.

The term "(NR^{c}R^{d})carbonylalkylcarbonyl" as used herein, refers to a (NR^{c}R^{d})carbonylalkyl group, as defined herein, appended to the parent molecular moiety through a carbonyl group, as defined herein.

The term "nitro," as used herein, refers to -NO₂.

The term "nitroalkyl," as used herein, refers to a nitro group attached to the parent molecular moiety through an alkyl group.

The term "oxo," as used herein, refers to (=O).

The term "sulfonyl," as used herein, refers to -SO₂-.

The compounds of the present invention can exist as therapeutically acceptable salts. The term "therapeutically acceptable salt," as used herein, represents salts or zwitterionic forms of the compounds of the present invention which are water or oil-soluble or dispersible, which are suitable for treatment of diseases without undue toxicity, irritation, and allergic response; which are commensurate with a reasonable benefit/risk ratio, and which are effective for their intended use. The salts can be prepared during the final isolation and purification of the compounds or separately by reacting an amino group with a suitable acid. Representative acid addition salts include acetate, adipate, alginate, citrate, aspartate, benzoate, benzenesulfonate, bisulfate, butyrate, camphorate, camphorsulfonate, digluconate, glycerophosphate, hemisulfate, heptanoate, hexanoate, formate, fumarate, hydrochloride, hydrobromide, hydroiodide, 2-hydroxyethansulfonate, lactate, maleate, mesitylenesulfonate, methanesulfonate, naphthylenesulfonate, nicotinate, 2-naphthalenesulfonate, oxalate, pamoate, pectinate, persulfate, 3-phenylproprionate, picrate, pivalate, propionate, succinate, tartrate, trichloroacetate, trifluoroacetate, phosphate, glutamate, bicarbonate, para-toluenesulfonate, and undecanoate. Also, amino groups in the compounds of the present invention can be quaternized with methyl, ethyl, propyl, and butyl chlorides, bromides, and iodides; dimethyl, diethyl, dibutyl, and diamyl sulfates; decyl, lauryl, myristyl, and steryl chlorides, bromides, and iodides; and benzyl and phenethyl bromides. Examples of acids which can be employed to form therapeutically acceptable addition salts include inorganic acids such as hydrochloric, hydrobromic, sulfuric, and phosphoric, and organic acids such as oxalic, maleic, succinic, and citric.

Basic addition salts can be prepared during the final isolation and purification of the compounds by reacting a carboxy group with a suitable base such as the hydroxide, carbonate, or bicarbonate of a metal cation or with ammonia or an organic primary, secondary, or tertiary amine. The cations of therapeutically acceptable salts include lithium, sodium, potassium, calcium, magnesium, and aluminum, as well as nontoxic quaternary amine cations such as ammonium, tetramethylammonium, tetraethylammonium, methylamine, dimethylamine, trimethylamine, triethylamine, diethylamine, ethylamine, tributylamine, pyridine, N,N-dimethylaniline, N-methylpiperidine, N-methylmorpholine, dicyclohexylamine, procaine, dibenzylamine, N,N-dibenzylphenethylamine, 1-ephenamine, and N,N'-dibenzylethylenediamine. Other representative organic amines useful for the formation of base addition salts include ethylenediamine, ethanolamine, diethanolamine, piperidine, and piperazine.

The present compounds can also exist as therapeutically acceptable prodrugs. The term "therapeutically acceptable prodrug," refers to those prodrugs which are suitable for use in contact with the tissues of patients without undue toxicity, irritation, and allergic response, are commensurate with a reasonable benefit/risk ratio, and are effective for their intended use. The term "prodrug," refers to compounds which are rapidly transformed in vivo to parent compounds of formula (I) for example, by hydrolysis in blood.

Asymmetric centers exist in the compounds of the present invention. These centers are designated by the symbols "R" or "S," depending on the configuration of substituents around the chiral carbon atom. It should be understood that the invention encompasses all stereochemical isomeric forms, or mixtures thereof, which possess the ability to inhibit protein kinases. Individual stereoisomers of compounds can be prepared synthetically from commercially available starting materials which contain chiral centers or by preparation of mixtures of enantiomeric products followed by separation such as conversion to a mixture of diastereomers followed by separation or recrystallization, chromatographic techniques, or direct separation of enantiomers on chiral chromatographic columns. Starting compounds of particular stereochemistry are either commercially available or can be made and resolved by techniques known in the art.

In accordance with methods of treatment and pharmaceutical compositions disclosed herein, the compounds can be administered alone or in combination with other anticancer agents. When using the compounds, the specific therapeutically effective dose level for any particular patient will depend upon factors such as the disorder being treated and the severity of the disorder; the activity of the particular compound used; the specific composition employed; the age, body weight, general health, sex, and diet of the patient; the time of administration; the route of administration; the rate of excretion of the compound employed; the duration of treatment; and drugs used in combination with or coincidently with the compound used. The compounds can be administered orally, parenterally, osmotically (nasal sprays), rectally, vaginally, or topically in unit dosage formulations containing carriers, adjuvants, diluents, vehicles, or combinations thereof. The term "parenteral" includes infusion as well as subcutaneous, intravenous, intramuscular, and intrasternal injection.

Parenterally administered aqueous or oleaginous suspensions of the compounds can be formulated with dispersing, wetting, or suspending agents. The injectable preparation can also be an injectable solution or suspension in a diluent or solvent. Among the acceptable diluents or solvents employed are water, saline, Ringer's solution, buffers, monoglycerides, diglycerides, fatty acids such as oleic acid, and fixed oils such as monoglycerides or diglycerides.

The anticancer effect of parenterally administered compounds can be prolonged by slowing their absorption. One way to slow the absorption of a particular compound is administering injectable depot forms comprising suspensions of crystalline, amorphous, or otherwise water-insoluble forms of the compound. The rate of absorption of the compound is dependent on its rate of dissolution which is, in turn, dependent on its physical state. Another way to slow absorption of a particular compound is administering injectable depot forms comprising the compound as an oleaginous solution or suspension. Yet another way to slow absorption of a particular compound is administering injectable depot forms comprising microcapsule matrices of the compound trapped within liposomes, microemulsions, or biodegradable polymers such as polylactide-polyglycolide, polyorthoesters or polyanhydrides. Depending on the ratio of drug to polymer and the composition of the polymer, the rate of drug release can be controlled.

Transdermal patches can also provide controlled delivery of the compounds. The rate of absorption can be slowed by using rate controlling membranes or by trapping the compound within a polymer matrix or gel. Conversely, absorption enhancers can be used to increase absorption.

Solid dosage forms for oral administration include capsules, tablets, pills, powders, and granules. In these solid dosage forms, the active compound can optionally comprise diluents such as sucrose, lactose, starch, talc, silicic acid, aluminum hydroxide, calcium silicates, polyamide powder, tableting lubricants, and tableting aids such as magnesium stearate or microcrystalline cellulose. Capsules, tablets and pills can also comprise buffering agents, and tablets and pills can be prepared with enteric coatings or other release-controlling coatings. Powders and sprays can also contain excipients such as talc, silicic acid, aluminum hydroxide, calcium silicate, polyamide powder, or mixtures thereof. Sprays can additionally contain customary propellants such as chlorofluorohydrocarbons or substitutes thereof.

Liquid dosage forms for oral administration include emulsions, microemulsions, solutions, suspensions, syrups, and elixirs comprising inert diluents such as water. These compositions can also comprise adjuvants such as wetting, emulsifying, suspending, sweetening, flavoring, and perfuming agents.

Topical dosage forms include ointments, pastes, creams, lotions, gels, powders, solutions, sprays, inhalants, and transdermal patches. The compound is mixed under sterile conditions with a carrier and any needed preservatives or buffers. These dosage forms can also include excipients such as animal and vegetable fats, oils, waxes, paraffins, starch, tragacanth, cellulose derivatives, polyethylene glycols, silicones, bentonites, silicic acid, talc and zinc oxide, or mixtures thereof. Suppositories for rectal or vaginal administration can be prepared by mixing the compounds with a suitable non-irritating excipient such as cocoa butter or polyethylene glycol, each of which is solid at ordinary temperature but fluid in the rectum or vagina. Ophthalmic formulations comprising eye drops, eye ointments, powders, and solutions are also contemplated as being within the scope of this invention.

The total daily dose of the compounds administered to a host in single or divided doses can be in amounts from about 0.1 to about 200 mg/kg body weight or preferably from about 0.25 to about 100 mg/kg body weight. Single dose compositions can contain these amounts or submultiples thereof to make up the daily dose.

### Determination of Biological Activity

The Chkl enzymatic assay was carried out using recombinant Chkl kinase domain protein covering amino acids from residue 1 to 289 and a polyhistidine tag at the C-terminal end. Human cdc25c peptide substrate contained a sequence from amino acid residue 204 to 225. The reaction mixture contained 25 mM of HEPES at pH 7.4, 10 mM MgCl₂, 0.08 mM Triton X-100, 0.5 mM DTT, 5 µM ATP, 4 nM 33P ATP, 5 µM cdc25c peptide substrate, and 6.3 nM of the recombinant Chk1 protein. Compound vehicle DMSO was maintained at 2% in the final reaction. After 30 minutes at room temperature, the reaction was stopped by addition of equal volume of 4M NaCl and 0.1M EDTA, pH 8. A 40 µL aliquot of the reaction was added to a well in a Flash Plate (NEN Life Science Products, Boston, MA) containing 160 µL of phosphate-buffered saline (PBS) without calcium chloride and magnesium chloride and incubated at room temperature for 10 minutes. The plate was then washed 3 times in PBS with 0.05% of Tween-20 and counted in a Packard TopCount counter (Packard BioScience Company, Meriden, CT).

Compounds of the present invention inhibited Chkl at IC₅₀ values between about 0.2 nM and about 280 µM. Preferred compounds inhibited Chk1 at IC₅₀ values between about 0.2 nM and about 80 nM. Most preferred compounds inhibited Chkl at IC₅₀ values between about 0.2 nM and about 30 nM. Thus, the compounds of the invention are useful in treating disorders which are caused or exacerbated by increased protein kinase levels.

The compounds of the invention, including but not limited to those specified in the examples, possess the ability to inhibit protein kinases. As protein kinase inhibitors, such compounds are useful in the treatment of both primary and metastatic solid tumors, including carcinomas of breast, colon, rectum, lung, oropharynx, hypopharynx, esophagus, stomach, pancreas, liver, gallbladder and bile ducts, small intestine, urinary tract (including kidney, bladder and urothelium), female genital tract (including cervix, uterus, and ovaries as well as choriocarcinoma and gestational trophoblastic disease), male genital tract (including prostate, seminal vesicles, testes and germ cell tumors), endocrine glands (including the thyroid, adrenal, and pituitary glands), and skin, as well as hemangiomas, melanomas, sarcomas (including those arising from bone and soft tissues as well as Kaposi's sarcoma) and tumors of the brain, nerves, eyes, and meninges (including astrocytomas, gliomas, glioblastomas, retinoblastomas, neuromas, neuroblastomas, Schwannomas, and meningiomas). Such compounds may also be useful in treating solid tumors arising from hematopoietic malignancies such as leukemias (i.e., chloromas, plasmacytomas and the plaques and tumors of mycosis fungicides and cutaneous T-cell lymphoma/leukemia) as well as in the treatment of lymphomas (both Hodgkin's and non-Hodgkin's lymphomas). In addition, these compounds may be useful in the prevention of metastases from the tumors described above either when used alone or in combination with radiotherapy and/or other chemotherapeutic agents.

### Synthetic Methods

Abbreviations which have been used in the descriptions of the scheme and the examples that follow are: Me for methyl, Et for ethyl, LHMDS for lithium bis(trimethylsilyl)amide, HATU for O-(-7-Azabenzotriazol-1-yl)-N,N,N',N',-tetramethyluronium hexafluorophosphate, PPh₃ for triphenylphosphine, PCy₃ for tricyclohexylphosphine, dba for dibenzylideneacetone, CyMAP for 2-dicyclohexyl phosphino-2'-(N, N-dimethylsmino)biphenyl, EDC for 1-(3-dimethylaminopropyl)-3-ethylcarbodiimide hydrochloride, 1,3-dicyclohexylcarbodiimide, HOBt t for 1-hydroxybenzotriazole, DMSO for dimethylsulfoxide, DME for 1,2-dimethoxyethane, THF for tetrahydrofuran, DMF for N,N-dimethylformamide, TFA for trifluoracetic acid, DPPF for diphenylphosphinoferrocene, OAc for acetate, TMS for trimethylsilyl, DMA for dimethylacetamide, and DIEA for diisopropylethylamine.

The compounds and processes of the present invention will be better understood in connection with the following synthetic schemes which illustrate the methods by which the compounds of the invention may be prepared. Starting materials can be obtained from commercial sources or prepared by well-established literature methods known to those of ordinary skill in the art. The groups A¹, A², X, Y, Z, and R¹-R⁷ are as defined above unless otherwise noted below.

This invention is intended to encompass compounds having formula (I) when prepared by synthetic processes or by metabolic processes. Preparation of the compounds of the invention by metabolic processes include those occurring in the human or animal body (in vivo) or processes occurring in vitro.

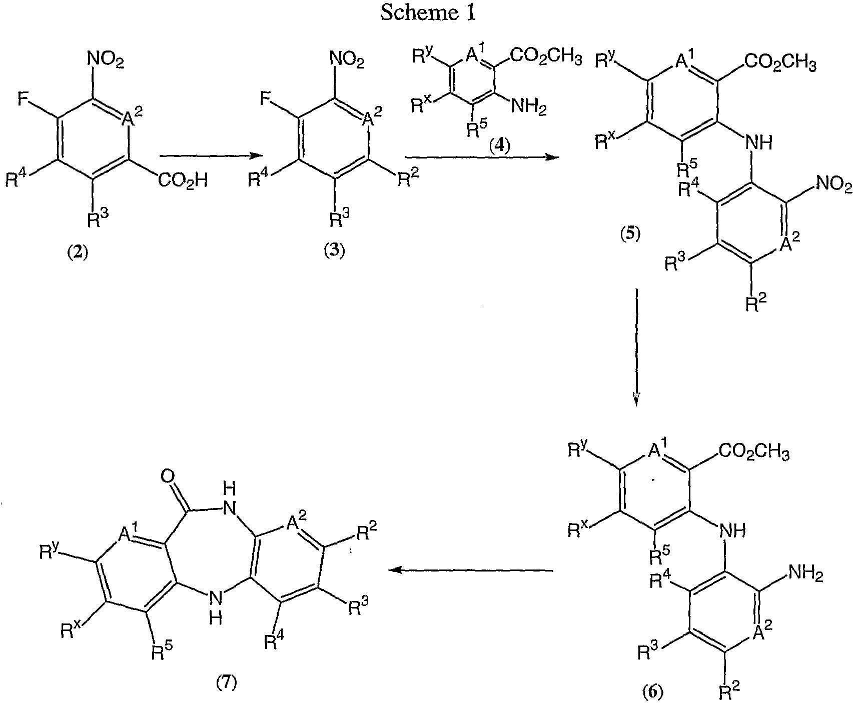

Scheme 1 shows the synthesis of compounds of formula (7) (compounds of formula (I) wherein Y is NR¹⁵ wherein R¹⁵ is H). Compounds of formula (2) can be esterified using conditions known to those of ordinary skill in the art to provide compounds of formula (3) where R² is alkoxycarbonyl. Alternatively, compounds of formula (2) can be treated sequentially with thionyl chloride, trimethylsilyldiazomethane, and silver (I) benzoate in triethylamine to provide compounds of formula (3) where R² is alkoxycarbonylalkyl. Compounds of formula (3) can be treated with compounds of formula (4) (where one of R^{x} and R^{y} is halogen and the other is hydrogen) in the presence of a base such as K₂CO₃, Na₂CO₃, or Cs₂CO₃ in a polar solvent such as N,N-dimethylacetamide to provide compounds of formula (5). Reduction of the nitro group using conditions known to those of ordinary skill in the art (for example, Pt/C in the presence of hydrogen in an alcoholic solvent system such as ethanol and ethyl acetate) provides compounds of formula (6) which can be cyclized in the presence of a strong acid such as concentrated HCl to provide compounds of formula (7).

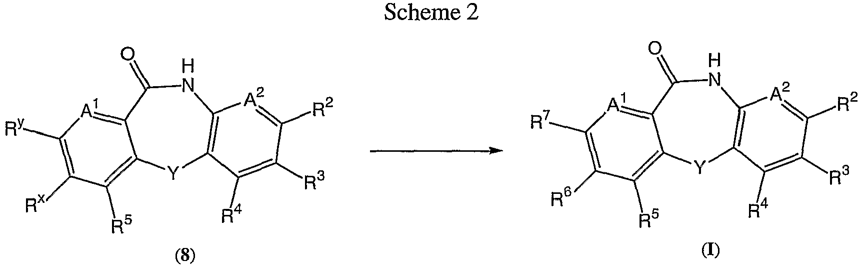

The conversion of compounds of formula (8) to compounds of formula (I) is shown in Scheme 2. Compounds of formula (I) where one of R⁶ and R⁷ is hydrogen and the other is aryl or heterocyclyl can be prepared by coupling compounds of formula (8) where one of R^{x} and R^{y} is Cl, Br, or I to an appropriately substituted organometallic reagent (such as an organoborane or an organostannane) in the presence of a palladium catalyst (such as Pd(PPh₃)₄, Pd(PCy₃)₂Cl₂, or Pd₂(dba)₃ with CyMAP) and optionally in the presence of a base (when the organometallic reagent is an organoborane) such as Cs₂CO₃ or CsF.

Alternatively, compounds of formula (I) where one of R⁶ and R⁷ is hydrogen and the other is -XR¹³ (where X is -O- or -NR¹⁴- and R¹³ is aryl or heterocyclyl) can be prepared by treating compounds of formula (8) where one of R^{x} and R^{y} is Cl, Br, or I with the appropriately substituted alcohol or amine in the presence of a base such as Cs₂CO₃ or CsF and a palladium catalyst such as Pd₂(dba)₃ with CyMAP or Pd(PCy₃)₂Cl₂.

Compounds of formula (I) where R² is alkoxycarbonyl or alkoxycarbonylalkyl can be saponified using conditions known to those of ordinary skill in the art, then reacted with an appropriately substituted amine in the presence of a coupling agent such as EDC, DCC, HOBt, and mixtures thereof, to provide compounds of formula (I) where R² is aminocarbonyl or aminocarbonylalkyl.

Compounds of formula (I) where R² is Br or Cl can be coupled to an appropriately substituted organometallic reagent as described above to provide compounds of formula (I) where R² is aryl or heterocyclyl.

The present invention will now be described in connection with certain preferred embodiments which are not intended to limit its scope. On the contrary, the present invention covers all alternatives, modifications, and equivalents as can be included within the scope of the claims. Thus, the following examples, which include preferred embodiments, will illustrate the preferred practice of the present invention, it being understood that the examples are for the purposes of illustration of certain preferred embodiments and are presented to provide what is believed to be the most useful and readily understood description of its procedures and conceptual aspects.

Compounds of the invention were named by ACD/ChemSketch version 5.0 (developed by Advanced Chemistry Development, Inc., Toronto, ON, Canada) or were given names consistent with ACD nomenclature.

Methyl 4-chloro-2-iodobenzoate was prepared from the corresponding carboxylic acid by methods known to those of ordinary skill in the art.

### Example 1

### methyl 3-chloro-11-oxo-10,11-dihydro-5H-dibenzo[b,e][1,4]diazepine-8-carboxylate

### Example 1A

### methyl 2-{[2-amino-4-(methoxycarbonyl)phenyl]amino}-4-chlorobenzoate

A mixture of methyl 4-chloro-2-iodobenzoate (0.593g, 2 mmol), methyl 3,4-diaminobenzoate (0.332g, 2 mmol), copper (0.126g, 2 mmol), and K₂CO₃ (0.276g, 2 mmol) in chlorobenzene (40 mL) was heated to reflux for 20 hours, cooled to room temperature, diluted with ethyl acetate, and filtered through diatomaceous earth (Celite^{®}). The solution was washed with water and brine, dried (MgSO₄), filtered, and concentrated under vacuum. The residue was purified by flash column chromatography on silica gel with 7:3 hexanes/ethyl acetate to provide 0.453g (68 %) of the desired product. MS (DCI) m/e 334 (M+H)⁺, 352 (M+NH₄)⁺; ¹H NMR (300 MHz, DMSO-d₆) δ 8.99 (s, 1H), 7.89 (d, J = 8.5 Hz, 1 H), 7.47 (d, J = 1.0 Hz, 1H), 7.21 (m, 2H), 6.80 (dd, J = 8.5, 2.0 Hz, 1H), 6.65 (d, J = 2.1 Hz, 1H), 5.30 (s, 2H), 3.86 (s, 3H), 3.82 (s, 3H).

### Example 1B

### methyl 3-chloro-11-oxo-10,11-dihydro-5H-dibenzo[b,e][1,4]diazepine-8-carboxylate

A solution of Example 1A (0.43g, 1.28 mmol) in 37% HCl (20 mL) and methanol (50 mL) was heated to reflux for 20 hours, cooled to room temperature, and filtered. The filter cake was washed with 1:1 H₂O/methanol and dried in a vacuum oven at 65° C to provide 0.34g (87%) of the desired product. MS (DCI) m/e 303 (M+H)⁺, 320 (M+NH₄)⁺; ¹H NMR (300 MHz, DMSO-d₆) δ 10.04 (s, 1H), 8.50 (s, 1H), 7.72 (d, J = 8.5 Hz, 1H), 7.55-7.60 (m, 2H), 7.08 (d, J = 2 Hz, 1H), 7.03 (d, J = 8.1 Hz, 1H), 6.96 (dd, J = 8.5, 2 Hz, 1H), 3.80 (s, 3H).

### Example 2

### 8-bromo-3-chloro-5,10-dihydro-11H-dibenzo[b,e][1,4]diazepin-11-one

### Example 2A

### methyl 2-[(4-bromo-2-nitrophenyl)amino]-4-chlorobenzoate

A mixture of methyl 4-chloro-2-iodobenzoate (5.93g, 20 mmol), 4-bromo-2-nitroaniline (4.34g, 20 mmol), copper (1.26g, 20 mmol), and K₂CO₃ (2.76g, 20 mmol) in chlorobenzene (300 mL) was heated to reflux for 2 days, cooled to room temperature, diluted with ethyl acetate, and filtered through diatomaceous earth (Celite^{®}). The solution was washed with water and brine, dried (MgSO₄), filtered, and concentrated under vacuum. The residue was purified by flash column chromatography on silica gel with 9:1 hexanes/ethyl acetate to provide 6.86g (89 %) of the desired product. MS (DCI) m/e 386 (M+H)⁺, 403 (M+NH₄)⁺; ¹H NMR (300 MHz, DMSO-d₆) δ 10.83 (s, 1H), 8.28 (d, J = 2.4 Hz, 1H), 7.97 (d, J = 8.5 Hz, 1H), 7.82 (dd, J = 9.1, 2.4 Hz, 1H), 7.63 (d, J = 9.1 Hz, 1H), 7.52 (d, J = 2.1 Hz, 1H), 7.17 (dd, J = 8.5, 2 Hz, 1H), 3.87 (s, 3H).

### Example 2B

### 8-bromo-3-chloro-5 ,10-dihydro-11H-dibenzo[b,e][1,4]diazepin-11-one

A mixture of Example 2A (6.0g, 15.6 mmol) and SnCl₂-2H₂O (10.54g, 46.8 mmol) in 37% HCl (200 mL) and methanol (300 mL) was heated to reflux for 20 hours, cooled to room temperature, and filtered. The filter cake was washed with 1:1 H₂O/methanol and dried in a vacuum oven at 65° C to provide 4.6g (91%) of the desired product. MS (DCI) m/e 324 (M+H)⁺, 341 (M+NH₄)⁺; ¹H NMR (300 MHz, DMSO-d₆) δ 9.98 (s, 1H), 8.18 (s, 1H), 7.69 (d, J = 8.5 Hz, 1H), 7.12-7.16 (m, 2H), 7.05 (d, J = 2 Hz, 1H), 6.95 (dd, J = 8.4, 2 Hz, 1H), 6.91 (d, J = 9.1 Hz, 1H).

### Example 3

### 3-chloro-8-nitro-5,10-dihydro-11H-dibenzo[b,e][1,4]diazepin-11-one

### Example 3A

### methyl 2-[(2-amino-4-nitrophenyl)amino]-4-chlorobenzoate

The desired product was prepared by substituting 2-amino-4-nitrophenylamine for methyl 3,4-diaminobenzoate in Example 1A. MS (DCI) m/e 322 (M+H)⁺, 339 (M+NH₄)⁺; ¹H NMR (300 MHz, DMSO-d₆) δ 9.08 (s, 1H), 7.92 (d, J = 8.5 Hz, 1H), 7.67 (d, J = 2.8 Hz, 1H), 7.47 (dd, J = 8.5, 2.4 Hz, 1H), 7.33 (d, J = 8.8 Hz, 1H), 6.90 (dd, J = 8.5, 2.0 Hz, 1H), 6.86 (d, J = 2.0 Hz, 1H), 5.65 (s, 2H), 3.87 (s, 3H).

### Example 3B

### 3-chloro-8-nitro-5,10-dihydro-11H-dibenzo[b,e][1,4]diazepin-11-one

The desired product was prepared by substituting Example 3A for Example 1A in Example 1B. MS (DCI) m/e 290 (M+H)⁺, 307 (M+NH₄)⁺; ¹H NMR (400 MHz, DMSO-d₆) δ 10.16 (s, 1H), 8.84 (s, 1H), 7.84-7.87 (m, 2H), 7.76 (d, J = 8.6 Hz, 1H), 7.08-7.11 (m, 2H), 6.99 (dd, J = 8.3, 1.7 Hz, 1H).

### Example 4

### 3-chloro-11-oxo-10,11-dihydro-5H-dibenzo[b,e][1,4]diazepine-8-carbonitrile

### Example 4A

### methyl 2-[(2-amino-4-cyanophenyl)amino]-4-chlorobenzoate

The desired product was prepared by substituting 3,4-diaminobenzonitrile for methyl 3,4-diaminobenzoate in Example 1A. MS (DCI) m/e 302 (M+H)⁺, 319 (M+NH₄)⁺; ¹H NMR (500 MHz, DMSO-d₆) δ 8.99 (s, 1H), 7.89 (d, J = 8.8 Hz, 1H), 7.26 (d, J = 8.1 Hz, 1H), 7.13 (d, J = 1.9 Hz, 1H), 7.00 (dd, J = 7.8, 1.9 Hz, 1H), 6.83 (dd, J = 8.7, 2.0 Hz, 1H), 6.67 (d, J = 1.9 Hz, 1H), 5.47 (s, 2H), 3.86 (s, 3H).

### Example 4B

### 3-chloro-11-oxo-10,11-dihydro-5H-dibenzo[b,e][1,4]diazepine-8-carbonitrile

The desired product was prepared by substituting Example 4A for Example 1A in Example 1B. MS (DCI) m/e 290 (M+H)⁺, 307 (M+NH₄)⁺; ¹H NMR (400 MHz, DMSO-d₆) δ 10.06 (s, 1H), 8.61 (s, 1H), 7.73 (d, J = 8.6 Hz, 1H), 7.40 (dd, J = 8.6, 1.8 Hz, 1H), 7.30 (d, J = 1.8 Hz, 1H), 7.07 (d, J = 2.4 Hz, 1H), 7.06 (d, J = 3.6 Hz, 1H), 6.98 (dd, J = 8.3,1.9 Hz, 1H).

### Example 5

### 3-chloro-8-(trifluoromethyl)-5,10-dihydro-11H-dibenzo[b,e][1,4]diazepin-11-one

### Example 5A

### methyl 4-chloro-2-1 [2-nitro-4-(trifluoromethyl)phenyl]amino}benzoate

The desired product was prepared by substituting 2-nitro-4-(trifluoromethyl)aniline for methyl 3,4-diaminobenzoate in Example 1A. MS (DCI) m/e 375 (M+H)⁺, 392 (M+NH₄)⁺; ¹H NMR (300 MHz, DMSO-d₆) δ 11.05 (s, 1H), 8.42 (d, J = 2.0 Hz, 1H), 8.00 (d, J = 8.8 Hz, 1H), 7.93 (dd, J = 9.1, 2.3 Hz, 1H), 7.75 (d, J = 8.8 Hz, 1H), 7.67 (d, J = 2.0 Hz, 1H), 7.30 (dd, J = 8.5, 2.1 Hz, 1H), 5.47 (s, 2H), 3.88 (s, 3H).

### Example 5B

### 2-{[2-amino-4-(trifluoromethyl)phenyl]amino}-4-chlorobenzoic acid

The desired product was prepared by substituting Example 5A for Example 2A in Example 2B. MS (DCI) m/e 331 (M+H)⁺; ¹H NMR (500 MHz, DMSO-d₆) δ 9.29 (s, 1H), 7.89 (d, J = 8.4 Hz, 1H), 7.32 (d, J = 8.1 Hz, 1H), 7.24 (d, J = 1.6 Hz, 1H), 7.00 (dd, J = 8.1, 1.3 Hz, 1H), 6.78 (dd, J = 8.7, 2.1 Hz, 1H), 6.62 (d, J = 1.9 Hz, 1H).

### Example 5C

### 3-chloro-8-(trifluoromethyl)-5,10-dihydro-11H-dibenzo[b,e][1,4]diazepin-11-one

A mixture of Example 5B (0.3g, 0.91 mmol) and TsOH·H₂O (0.35g, 1.82 mmol) in toluene (50 mL) was heated to reflux for 20 hours using a Dean-Stark trap to remove water. The reaction was cooled to room temperature, concentrated under vacuum, diluted with ethyl acetate, washed with saturated NaHCO₃ and brine, dried (MgSO₄) filtered, and concentrated under vacuum. The residue was purified by flash column chromatography on silica gel with 7:3 hexanes/ethyl acetate to provide 0.21g (81%) of the desired product. MS (DCI) m/e 313 (M+H)⁺, 330 (M+NH₄)⁺; ¹H NMR (300 MHz, DMSO-d₆) δ 10.08 (s, 1H), 8.48 (s, 1H), 7.72 (d, J = 8.4 Hz, 1H), 7.29-7.35 (m, 2H), 7.12 (d, J = 8.1 Hz, 1H), 7.08 (d, J = 2.0 Hz, 1H), 6.97 (dd, J = 8.5, 2.1 Hz, 1H).

### Example 6

### methyl (3-chloro-11-oxo-10,11-dihydro-5H-dibenzo[b,e][1,4]diazepin-8-yl)acetate

### Example 6A

### methyl (4-amino-3-nitrophenyl)acetate

Concentrated nitric acid (10 mL, >69% pure) was added to acetic anhydride (100 mL) cooled to -10 °C. The solution was treated portionwise with N-[4-(cyanomethyl)phenyl]acetamide (5.0g, 28.7 mmol) at a rate which maintained an internal temperature below -5 °C. The solution was stirred for 1 hour while warming to room temperature. The solution was poured into an ice/water mixture and extracted several times with ethyl acetate. The combined extracts were washed with 10% Na₂CO₃ and brine, dried (MgSO₄) filtered, and concentrated under vacuum. The residue was purified by flash column chromatography on silica gel with 1:1 hexanes/ethyl acetate to provide 4.82g (77%) of N-[4-(cyanomethyl)-2-nitrophenyl]acetamide.

A mixture of N-[4-(cyanomethyl)-2-nitrophenyl]acetamide (4.8 g), concentrated HCl (100 mL), and water (300 mL) was heated to reflux for two days, cooled to room temperature, and concentrated to near dryness under vacuum. The concentrate was treated with methanol (300 mL) and concentrated H₂SO₄ (30 mL), heated to reflux overnight, and concentrated under vacuum to remove the methanol. The residue was partitioned between ethyl acetate and water and the organic layers were combined, washed with saturated NaHCO₃ and brine, dried (MgSO₄), and concentrated under vacuum. The residue was purified by flash column chromatography on silica gel with 7:3 hexanes/ethyl acetate to provide 4.1g (89%) of the desired product. MS (DCI) m/e 211 (M+H)⁺, 228 (M+NH₄)⁺; ¹H NMR (300 MHz, DMSO-d₆) δ 7.88 (d, J = 2 Hz, 1H), 7.39 (s, 2H), 7.30 (dd, J = 8.5, 2 Hz, 1H), 6.97 (d, J = 8.4 Hz, 1H), 3.61 (s, 3H).

### Example 6B

### methyl 4-chloro-2-{[4-(2-methoxy-2-oxoethyl)-2-nitrophenyl]amino}benzoate

The desired product was prepared by substituting methyl (4-amino-3-nitrophenyl)acetate for 4-bromo-2-nitroaniline in Example 2A. MS (DCI) m/e 379 (M+H)⁺, 396 (M+NH₄)⁺; ¹H NMR (300 MHz, DMSO-d₆) δ 10.84 (s, 1H), 8.10 (d, J = 2.1 Hz, 1H), 7.96 (d, J = 8.5, Hz, 1H), 7.67 (d, J = 8.5 Hz, 1H), 7.60 (dd, J = 8.8, 2.0 Hz, 1H), 7.46 (d, J = 2.1 Hz, 1H), 7.11 (dd, J = 8.5, 2 Hz, 1H), 3.69 (s, 3H), 3.81 (s, 2H), 3.65 (s, 3H).

### Example 6C

### methyl 2- {2-amino-4-(2-methoxy-2-oxoethyl)phenyl]amino}-4-chlorobenzoate

A mixture of Example 6B (0.73g, 1.93 mmol), 5% Pt/C, methanol (15 mL) and ethyl acetate (15 mL) was equipped with a balloon of hydrogen gas and stirred at room temperature. After uptake of the hydrogen was complete, the solution was filtered through diatomaceous earth (Celite^{®}). The filtrate was concentrated under vacuum and the residue was purified by flash column chromatography on silica gel with 7:3 hexanes/ethyl acetate to provide 0.64g (95%) of the desired product. MS (DCI) m/e 349 (M+H)⁺, 366 (M+NH₄)⁺; ¹H NMR (500 MHz, DMSO-d₆) δ 8.81 (s, 1H), 7.85 (d, J = 8.5 Hz, 1H), 6.96 (d, J = 8.1 Hz, 1H), 6.69-6.72 (m, 2H), 6.50 (dd, J =7.8, 1.8 Hz, 1H), 6.40 (d, J = 2.1 Hz, 1H), 5.00 (s, 2H), 3.86 (s, 3H), 3.63 (s, 3H), 3.55 (s, 2H).

### Example 6D

### methyl (3-chloro-11-oxo-10,11-dihydro-5H-dibenzo[b,e][1,4]diazepin-8-yl)acetate

The desired product was prepared by substituting Example 6C for Example 5B in Example 5C. MS (DCI) m/e 317 (M+H)⁺, 334 (M+NH₄)⁺; ¹H NMR (500 MHz, DMSO-d₆) δ 9.88 (s, 1H), 8.03 (s, 1H), 7.68 (d, J = 8.7 Hz, 1H), 7.06 (d, J = 2.2 Hz, 1H), 6.91-6.93 (m, 2H), 6.86-6.87 (m, 2H), 3.60 (s, 3H), 3.54 (s, 2H).

### Example 7

### 8-amino-3-chloro-5,10-dihydro-11H-dibenzo[b,e][1,4]diazepin-11-one

### Example 7A

### methyl 2-[(2-amino-4-nitrophenyl)amino]-4-chlorobenzoate

The desired product was prepared by substituting 4-nitro-1,2-benzenediamine for methyl 3,4-diaminobenzoate in Example 1A.

### Example 7B

### methyl 4-chloro-2-[(2,4-diaminophenyl)amino]benzoate

The desired product was prepared by substituting Example 7A for Example 6B in Example 6C. MS (DCI) m/e 292 (M+H)⁺; ¹H NMR (300 MHz, DMSO-d₆) δ 8.58 (s, 1H), 7.80 (d, J = 8.8 Hz, 1H), 6.63 (d, J = 2.1 Hz, 1H), 6.60 (d, J = 2.4 Hz, 1H), 6.34 (d, J = 2.1 Hz, 1H), 6.03 (d, J = 2.2 Hz, 1H), 5.89 (dd, J = 8.1, 2.5 Hz, 1H), 4.84 (s, 2H), 4.63 (s, 2H), 3.84 (s, 3H).

### Example 7C

### 8-amino-3-chloro-5,10-dihydro-11H-dibenzo[b,e][1,4]diazepin-11-one

The desired product was prepared by substituting Example 7B for Example 5B in Example 5C. MS (DCI) m/e 259 (M)⁺, 277 (M+NH₄)⁺; ¹H NMR (500 MHz, DMSO-d₆) δ 9.69 (s, 1H), 7.64 (d, J = 8.4 Hz, 1H), 7.56 (s, 1H), 7.00 (d, J = 1.9 Hz, 1H), 6.85 (dd, J = 8.4, 1.9 Hz, 1H), 6.65 (d, J = 8.1 Hz, 1H), 6.20-6.21 (m, 2H), 4.81 (s, 2H).

### Example 8

### 3-chloro-8-hydroxy-5,10-dihydro-11H-dibenzo[b,e][1,4]diazepin-11-one

### Example 8A

### meth 2-{[4-(benzyloxy)-2-nitrophenyl]amino}-4-chlorobenzoate

The desired product was prepared by substituting 4-(benzyloxy)-2-nitroaniline for 4-bromo-2-nitroaniline in Example 2A. MS (DCI) m/e 413 (M+H)⁺, 430 (M+NH₄)⁺; ¹H NMR (300 MHz, DMSO-d₆) δ 10.53 (s, 1H), 7.93 (d, J = 8.4 Hz, 1H), 7.73 (d, J = 3.1 Hz, 1H), 7.67 (d, J = 9.2 Hz, 1H), 7.36-7.51 (m, 6H), 7.23 (d, J = 2.0 Hz, 1H), 7.00 (dd, J = 8.5, 2.0 Hz, 1H), 5.21 (s, 2H), 3.89 (s, 3H).

### Example 8B

### methyl 2-{[2-amino-4-(benzyloxy)phenyl]amino}-4-chlorobenzoate

The desired product was prepared by substituting Example 8A for Example 6B in Example 6C. MS (DCI) m/e 383 (M+H)⁺; ¹H NMR (500 MHz, DMSO-d₆) δ 8.70 (s, 1H), 7.83 (d, J = 8.8 Hz, 1H), 7.33-7.46 (m, 5H), 6.90 (d, J = 8.4 Hz, 1H), 6.67 (dd, J = 8.8, 2.2 Hz, 1H), 6.47 (d, J = 2.8 Hz, 1H), 6.33 (d, J = 2.2 Hz, 1H), 6.27 (dd, J = 8.5, 2.8 Hz, 1H), 5.04 (s, 2H), 5.01 (s, 2H), 3.85 (s, 3H).

### Example 8C

### 3-chloro-8-hydroxy-5,10-dihydro-11H-dibenzo[b,e][1,4]diazepin-11-one

The desired product was prepared by substituting Example 8B for Example 5B in Example 5C. MS (DCI) m/e 260 (M)⁺, 278 (M+NH₄)⁺; ¹H NMR (500 MHz, DMSO-d₆) δ 9.79 (s, 1H), 9.15(s, 1H), 7.56 (s, 1H), 7.71 (s, 1H), 7.65 (d, J = 8.4 Hz, 1H), 7.03 (d, J = 2.2 Hz, 1H), 6.87 (dd, J = 8.4, 2.2 Hz, 1H), 6.77 (d, J = 8.7 Hz, 1H), 6.44 (d, J = 2.5 Hz, 1H), 6.38 (dd, J = 8.4,2.5 Hz, 1H).

### Example 9

### 3-bromo-5,10-dihydro-11H-dibenzo[b,e][1,4]diazepin-11-one

A mixture of 4-bromo-2-chlorobenzoic acid (2.35g, 10 mmol), 1,2-benzenediamine (1.08g, 10 mmol) and copper (0.63g, 10 mmol) in chlorobenzene (150 mL) was heated to reflux for 48 hours and filtered. The filter cake was washed with ethyl acetate several times. The combined filtrates were concentrated under vacuum and the residue was purified by flash column chromatography on silica gel with 8:2 hexanes/ethyl acetate to provide 1.20g (22%) of the desired product. MS (DCI) m/e 289 (M+H)⁺, 307 (M+NH₄)⁺; ¹H NMR (500 MHz, DMSO-d₆) δ 9.90 (s, 1H), 8.02 (s, 1H), 7.62 (d, J = 8.4 Hz, 1H), 7.24 (d, J = 1.8 Hz, 1H), 7.06 (dd, J = 8.4, 1.8 Hz, 1H), 6.92-6.95 (m, 5H).

### Example 10

### 3-(4-hydroxy-3-methoxyphenyl)-5,10-dihydro-11H-dibenzo[b,e][1,4]diazepin-11-one

A mixture of Example 9 (116mg, 0.4 mmol), 2-methoxy-4-(4,4,5,5-tetramethyl-1,3,2-dioxaborolan-2-yl)phenol (150mg, 0.6 mmol), Pd(PPh₃)₄ (23mg, 0.02 mmol), and CsF (121mg, 0.8 mmol) in DME (20 mL) and methanol (10 mL) was heated to reflux for 16 hours, cooled to room temperature, and partitioned between ethyl acetate and water. The organic layer was washed with brine, dried (MgSO₄), filtered, and concentrated under vacuum. The residue was purified by flash column chromatography on silica gel with 1:1 hexanes/ethyl acetate to provide 0.108g (81%) of the desired product. MS (DCI) m/e 333 (M+H)⁺, 350 (M+NH₄)⁺; ¹H NMR (500 MHz, DMSO-d₆) δ 9.90 (s, 1H), 8.02 (s, 1H), 7.62 (d, J = 8.4 Hz, 1H), 7.24 (d, J = 1.8 Hz, 1H), 7.15-7.18 (m, 2H), 7.08 (dd, J = 8.1, 1.5 Hz, 1H), 6.97-7.02 (m, 5H), 3.86 (s, 3H).

### Example 11

### 3-(11-oxo-10,11-dihydro-5H-dibenzo[b,e][1,4]diazepin-3-yl)benzonitrile

The desired product was prepared by substituting 3-cyanophenylboronic acid for 2-methoxy-4-(4,4,5,5-tetramethyl-1,3,2-dioxaborolan-2-yl)phenol in Example 10. MS (DCI) m/e 312 (M+H)⁺, 329 (M+NH₄)⁺; ¹H NMR (500 MHz, DMSO-d₆) δ 9.86 (s, 1H), 8.10 (s, 1H), 7.98 (d, J = 8.4 Hz, 1H), 7.94 (s, 1H), 7.88 (d, J = 7.8 Hz, 1H), 7.79 (d, J = 8.1 Hz, 1H), 7.71 (t, J = 7.8 Hz, 1H), 7.34 (d, J = 1.9 Hz, 1H), 7.26 (dd, J = 8.1, 1.9 Hz, 1H), 6.90-7.02 (m, 4H).

### Example 12

### methyl-3-(4-hydroxy-3-methoxyphenyl)-11-oxo-10,11-dihydro-5H-dibenzo[b,e][1,4]diazepine-8-carboxylate

A mixture of Example 1B (92mg, 0.3 mmol), 2-methoxy-4-(4,4,5,5-tetramethyl-1,3,2-dioxaborolan-2-yl)phenol (112mg, 0.45 mmol), Pd(PCy₃)₂Cl₂ (llmg, 0.015 mmol), and CsF (144mg, 0.9 mmol) in DME (20 mL) and methanol (10 mL) was heated to reflux for 16 hours, cooled to room temperature, and partitioned between ethyl acetate and water. The organic layer was washed with brine, dried (MgSO₄), filtered, and concentrated under vacuum. The residue was purified by flash column chromatography on silica gel with 1:1 hexanes/ethyl acetate to provide 95mg (81%) of the desired product. MS (DCI) m/e 391 (M+H)⁺; ¹H NMR (500 MHz, DMSO-d₆) δ 9.88 (s, 1H), 9.26 (s, 1H), 8.36 (s, 1H), 7.75 (d, J = 8.1 Hz, 1H), 7.60 (d, J = 2.2 Hz, 1H), 7.55 (dd, J = 8.1, 1.9 Hz, 1H), 7.24 (d, J = 2.1 Hz, 1H), 7.17-7.19 (m, 2H), 7.06-7.09 (m, 2H), 6.87 (d, J = 8.1 Hz, 1H), 3.86 (s, 3H), 3.80 (s, 3H).

### Example 13

### 3-(4-hydroxy-3-methoxyphenyl)-11-oxo-10,11-dihydro-5H-dibenzo[b,e][1,4]diazepine-8-carboxylic acid

A mixture of Example 12 (80mg, 0.2 mmol) and LiOH (24 mL, 1 mmol) in methanol (10 mL), THF (10 mL), and water (10 mL) was heated to reflux overnight, cooled to room temperature, and adjusted to pH <5 with concentrated HCl. The solid was collected by filtration to provide 55mg of the desired product. MS (DCI) m/e 377 (M+H)⁺; ¹H NMR (300 MHz, DMSO-d₆) δ 12.65 (s, 1H), 9.89 (s, 1H), 9.28 (s, 1H), 8.33 (s, 1H), 7.75 (d, J = 8.1 Hz, 1H), 7.57 (d, J = 1.7 Hz, 1H), 7.52 (dd, J = 8.1, 1.6 Hz, 1H), 7.24 (d, J = 1.7 Hz, 1H), 7.17-7.20 (m, 2H), 7.04-7.10 (m, 2H), 6.88 (d, J = 8.1 Hz, 1H), 3.86 (s, 3H).

### Example 14

### 3-(4-hydroxy-3-methoxyphenyl)-11-oxoN[3-(1-pyrrolidinyl)propyl]-10,11-dihydro-5H-dibenzo[b,e][1,4]diazepine-8-carboxamide

A mixture of Example 13 (55mg, 0.15 mmol), 3-pyrrolidin-1-ylpropylamine (58mg, 0.45 mmol), EDC (143mg, 0.75 mmol), HOBt (101mg, 75 mmol), and triethlyamine hydrochloride (103mg, 0.75 mmol) in DMF (5 mL) was heated to 65 °C for 16 hours, cooled to room temperature, poured into water, and extracted with 3:1 dichloromethane/isopropyl alcohol. The combined extracts were washed with brine, dried (MgSO₄), filtered, and concentrated under vacuum. The residue was purified by HPLC to provide 45mg of the desired product as the trifluoroacetate salt. MS (DCI) m/e 487 (M+H)⁺; ¹H NMR (500 MHz, DMSO-d₆) δ 9.82 (s, 1H), 8.36 (t, J = 5.6 Hz, 1H), 8.21 (s, 1H), 7.74 (d, J = 8.1 Hz, 1H), 7.46 (d, J = 1.8 Hz, 1H), 7.41 (dd, J = 8.1, 1.9 Hz, 1H), 7.26 (d, J = 1.6 Hz, 1H), 7.18 (d, J = 2.2 Hz, 1H), 7.16 (d, J = 1.6 Hz, 1H), 7.09 (dd, J = 8.4, 2.2 Hz, 1H), 7.04 (d, J = 8.4 Hz, 1H), 6.88 (d, J = 8.1 Hz, 1H), 3.86 (s, 3H), 3.25-3.29 (m, 2H), 2.55 (br m, 6H), 1.68-1.72 (m, 6H).

### Example 15

### N-[3-(dimethylamino propyl]-3-(4-hydroxy-3-methoxyphenyl)-11-oxo-10,11-dihydro-5H-dibenzo[b,e][1,4]diazepine-8-carboxamide

The desired product was prepared by substituting N,N-dimethyl-1,3-propanediamine for 3-pyrrolidin-1-ylpropylamine in Example 14. MS (DCI) m/e 461 (M+H)⁺; ¹H NMR (500 MHz, DMSO-d₆) (TFA salt) δ 9.82 (s, 1H), 9.24 (br s, 1H), 8.26 (t, J = 5.6 Hz, 1H), 8.15 (s, 1H), 7.73 (d, J = 8.2 Hz, 1H), 7.45 (d, J = 1.9 Hz, 1H), 7.41 (dd, J = 8.1, 1.9 Hz, 1H), 7.23 (d, J = 1.6 Hz, 1H), 7.18 (d, J = 2.1 Hz, 1H), 7.16 (d, J = 1.9 Hz, 1H), 7.07 (dd, J = 8.1, 2.1 Hz, 1H), 7.02 (d, J = 8.1 Hz, 1H), 6.87 (d, J = 8.5 Hz, 1H), 3.86 (s, 3H), 3.21-3.25 (m, 2H), 2.25 (t, J = 7.2 Hz, 2H), 2.13 (s, 6H), 1.62 (m, 2H).

### Example 16

### 3-(4-hydroxy-3-methoxyphenyl)N[3-(4-morpholinyl)propyl]-11-oxo-10,11-dihydro-5H-dibenzo[b,e][1,4]diazepine-8-carboxamide

The desired product was prepared by substituting 3-(4-morpholinyl)propylamine for 3-pyrrolidin-1-ylpropylamine in Example 14. MS (DCI) m/e 502 (M+H)⁺; ¹H NMR (500 MHz, DMSO-d₆) (TFA salt) δ 9.84 (s, 1H), 9.65 (br s, 1H), 9.25 (br s, 1H), 8.41 (t, J = 5.7 Hz, 1H), 8.21 (s, 1H), 7.73 (d, J = 8.1 Hz, 1H), 7.47 (d, J = 2.1 Hz, 1H), 7.44 (dd, J = 8.1, 2.1 Hz, 1H), 7.24 (d, J = 1.6 Hz, 1H), 7.17-7.19 (m, 2H), 7.08 (dd, J = 8.1, 1.9 Hz, 1H), 7.04 (d, J = 8.1 Hz, 1H), 6.87 (d, J = 8.1 Hz, 1H), 3.97-4.00 (m, 2H), 3.90 (s, 3H), 3.61-3.66 (m, 2H), 3.42-3.48 (m, 4H), 3.05-3.14 (m, 4H), 1.86-1.92 (m, 2H).

### Example 17

### 3-(4-hydroxy-3-methoxyphenyl)-11-oxoN[3-(2-oxo-1-pyrrolidinyl)propyl)-10,11-dihydro-5H-dibenzo[b,e][1,4]diazepine-8-carboxamide

The desired product was prepared by substituting 1-(3-aminopropyl)-2-pyrrolidinone for 3-pyrrolidin-1-ylpropylamine in Example 14. MS (DCI) m/e 501 (M+H)⁺; ¹H NMR (500 MHz, DMSO-d₆) (TFA salt) δ 9.84 (s, 1H), 9.65 (br s, 1H), 9.29 (br s, 1H), 8.24 (t, J = 5.2 Hz, 1H), 8.18 (s, 1H), 7.74 (d, J = 8.2 Hz, 1H), 7.42-7.47 (m, 2H), 7.24 (s, 1H), 7.17 (m, 1H), 7.09 (dd, J = 8.3, 1.8 Hz, 1H), 7.43 (d, J = 8.0 Hz, 1H), 6.80 (d, J = 7.9 Hz, 1H), 3.86 (s, 3H), 3.17-3.23 (m, 6H), 2.20-2.24 (m, 2H), 1.89-1.94 (m, 2H), 1.63-1.71 (m, 2H).

### Example 18

### N-(2-hydroxyethyl)-3-(4-hydroxy-3-methoxyphenyl)-11-oxo-10,11-dihydro-5H-dibenzo[b,e][1,4]diazepine-8-carboxamide

The desired product was prepared by substituting 2-aminoethanol for 3-pyrrolidin-1-ylpropylamine in Example 14. MS (DCI) m/e 420 (M+H)⁺; ¹H NMR (500 MHz, DMSO-d₆) δ 9.82 (s, 1H), 9.25 (s, 1H), 8.16-8.18 (m, 2H), 7.43 (d, J = 8.4 Hz, 1H), 7.47 (d, J = 1.9 Hz, 1H), 7.45 (dd, J = 8.4, 2.1 Hz, 1H), 7.23 (d, J = 1.5 Hz, 1H), 7.16-7.18 (m, 2H), 7.08 (dd, J = 8.3, 1.8 Hz, 1H), 7.01 (d, J = 8.1 Hz, 1H), 6.87 (d, J = 8.5 Hz, 1H), 4.67 (t, J = 5.6 Hz, 1H), 3.86 (s, 3H), 3.47-3.50 (m, 2H), 3.27-3.30 (m, 2H).

### Example 19

### N-(2,3-dihydroxypropyl)-3-(4-hydroxy-3-methoxyphenyl)-11-oxo-10,11-dihydro-5H-dibenzo[b,e][1,4]diazepine-8-carboxamide

The desired product was prepared by substituting 3-amino-1,2-propanediol for 3-pyrrolidin-1-ylpropylamine in Example 14. MS (DCI) m/e 450 (M+H)⁺; ¹H NMR (500 MHz, DMSO-d₆) δ 9.82 (s, 1H), 9.25 (s, 1H), 8.17 (s, 1H), 8.12 (t, J = 5.6 Hz, 1H), 7.43 (d, J = 8.1 Hz, 1H), 7.44-7.48 (m, 2H), 7.24 (d, J = 1.6 Hz, 1H), 7.16-7.18 (m, 2H), 7.08 (dd, J = 8.1, 1.9 Hz, 1H), 7.02 (d, J = 8.1 Hz, 1H), 6.87 (d, J = 8.1 Hz, 1H), 4.75 (t, J = 2.5 Hz, 1H), 4.53 (t, J = 5.6 Hz, 1H), 3.86 (s, 3H), 3.60 (m, 1H), 3.32-3.34 (m, 2H), 3.14-3.20 (m, 2H).

### Example 20

### N-[2-(acetylamino)ethyl]-3-(4-hydroxy-3-methoxyphenyl)-11-oxo-10,11-dihydro-5H-dibenzo[b,e][1,4]diazepine-8-carboxamide

The desired product was prepared by substituting N-(2-aminoethyl)acetamide for 3-pyrrolidin-1-ylpropylamine in Example 14. MS (DCI) m/e 461 (M+H)⁺; ¹H NMR (500 MHz, DMSO-d₆) δ 9.84 (s, 1H), 9.25 (br s, 1H), 8.28 (t, J = 5.5 Hz, 1H), 8.17 (s, 1H), 7.92 (t, J = 5.2 Hz, 1H), 7.23 (d, J = 8.3 Hz, 1H), 7.47 (d, J = 1.8 Hz, 1H), 7.42, (dd, J = 8.3, 1.9 Hz, 1H), 7.24 (d, J = 1.9 Hz, 1H), 7.16-7.18 (m, 2H), 7.08 (dd, J = 8.3, 2.1 Hz, 1H), 7.02 (d, J = 8.3 Hz, 1H), 6.87 (d, J = 8.0 Hz, 1H), 3.86 (s, 3H), 3.24-3.27 (m, 2H), 3.15-3.20 (m, 2H), 1.80 (s, 3H).

### Example 21

### 3-(4-hydroxy-3-methoxyphenyl)-8-[(3-hydroxy-1-pyrrolidinyl)carbonyl]-5,10-dihydro-11H-dibenzo[b,e][1,4]diazepin-11-one

The desired product was prepared by substituting 3-pyrrolidinol for 3-pyrrolidin-1-ylpropylamine in Example 14. MS (DCI) m/e 446 (M+H)⁺; ¹H NMR (500 MHz, DMSO-d₆) δ 9.83 (s, 1H), 9.25 (br s, 1H), 8.12 (s, 1H), 7.74 (d, J = 8.3 Hz, 1H), 7.24 (d, J = 1.5 Hz, 1H), 7.13-7.18 (m, 4H), 7.08 (dd, J = 8.3, 2.2 Hz, 1H), 7.01 (d, J = 8.3 Hz, 1H), 6.87 (d, J = 8.3 Hz, 1H), 4.27 (m, 1H), 3.86 (s, 3H), 3.40-3.61-3.27 (m, 4H), 1.79 (m, 2H).

### Example 22

### 3-(4-hydroxy-3-methoxyphenyl)-8-{[(2S)-2-(hydroxymethyl)-1-pyrrolidinyl]carbonyl}-5,10-dihydro-11H-dibenzo[b,e][1,4]diazepin-11-one

The desired product was prepared by substituting (2S)-2-pyrrolidinylmethanol for 3-pyrrolidin-1-ylpropylamine in Example 14. MS (DCI) m/e 460 (M+H)⁺; ¹H NMR (500 MHz, DMSO-d₆) δ 9.80 (s, 1H), 9.25 (br s, 1H), 8.10 (s, 1H), 7.73 (d, J = 8.3 Hz, 1H), 7.24 (d, J = 1.6 Hz, 1H), 7.18 (d, J = 2.1 Hz, 1H), 7.07 7.16 (m, 3H), 7.01 (d, J = 8.3 Hz, 1H), 6.87 (d, J = 8.0 Hz, 1H), 3.34-3.44(m, 5H), 3.86 (s, 3H), 1.84-1.92 (m, 4H).

### Example 23

### 3-(4-hydroxy-3-methoxyphenyl)-8-{[2-(hydroxymethyl)-1-piperidinyl]carbonyl}-5,10-dihydro-11H-dibenzo[b,e][1,4]diazepin-11-one

The desired product was prepared by substituting 2-piperidinylmethanol for 3-pyrrolidin-1-ylpropylamine in Example 14. MS (DCI) m/e 474 (M+H)⁺; ¹H NMR (500 MHz, DMSO-d₆) δ 9.84 (s, 1H), 9.29 (br s, 1H), 8.08 (s, 1H), 7.72 (d, J = 8.3 Hz, 1H), 7.25 (d, J = 1.5 Hz, 1H), 7.16-7.21 (m, 2H), 7.09 (m, 1H), 6.98-7.03 (m, 2H), 6.88 (d, J = 8.0 Hz, 1H), 4.35 (m, 1H), 3.86 (s, 3H), 3.28-3.34 (m, 4H), 1.48-1.80 (m, 6H).

### Example 24

### ethyl 1-{[3-(4-hydroxy-3-methoxyphenyl)-11-oxo-10,11-dihydro-5H-dibenzo[b,e][1,4]diazepin-8-yl]carbonyl}-2-piperidinecarboxylate

The desired product was prepared by substituting ethyl 2-piperidinecarboxylate for 3-pyrrolidin-1-ylpropylamine in Example 14. MS (DCI) m/e 516 (M+H)⁺; ¹H NMR (500 MHz, DMSO-d₆) δ 9.89 (s, 1H), 9.28 (s, 1H), 8.14 (s, 1H), 7.73-7.74 (d, J = 8.2 Hz, 1H), 7.24 (d, J = 1.2 Hz, 1H), 7.17-7.19 (m, 2H), 7.07-7.09 (dd, J = 8.2, 2.1 Hz, 1H), 6.95-7.05 (m, 3H), 6.88 (d, J = 8.2 Hz, 1H), 4.16 (br s, 2H), 3.86 (s, 3H), 3.61 (m, 1H), 1.29-1.79 (m, 11H).

### Example 25

### ethyl 1-{(3-(4-hydroxy-3-methoxyphenyl)-11-oxo-10,11-dihydro-5H-dibenzo[b,e][1,4]diazepin-8-yl]carbonyl}-3-piperidinecarboxylate

The desired product was prepared by substituting ethyl 3-piperidinecarboxylate for 3-pyrrolidin-1-ylpropylamine in Example 14. MS (DCI) m/e 516 (M+H)⁺; ¹H NMR (500 MHz, DMSO-d₆,) δ 9.88 (s, 1H), 9.28 (s, 1H), 8.12 (s, 1H), 7.73-7.74 (d, J = 8.2 Hz, 1H), 7.24 (d, J = 1.5 Hz, 1H), 7.16-7.18 (m, 2H), 7.08-7.09 (dd, J = 8.2, 2.1 Hz, 1H), 6.99-7.04 (b, 3H), 6.88 (d, J = 8.2 Hz, 1H), 4.06 (b, 2H), 3.86 (s, 3H), 3.06-3.11 (m, 1H), 2.50-2.54 (m, 2H), 1.06-1.96 (m, 11H).

### Example 26

### ethyl 1-{[3-(4-hydroxy-3-methoxyphenyl)-11-oxo-10,11-dihydro-5H-dibenz[b,e][1,4]diazepin-8-yl]carbonyl}-4-piperidinecarboxylate

The desired product was prepared by substituting ethyl 4-piperidinecarboxylate for 3-pyrrolidin-1-ylpropylamine in Example 14. MS (DCI) m/e 516 (M+H)⁺; ¹H NMR (DMSO-d₆, 500 NMz) δ 9.86 (s, 1H), 9.28 (br s, 1H), 8.13 (s, 1H), 7.74 (d, J = 8.2 Hz, 1H), 7.24 (d, J = 1.5 Hz, 1H), 7.17-7.19 (m, 2H), 7.08 (dd, J = 8.2, 1.8 Hz, 1H), 6.99-7.04 (m, 3H), 6.88 (d, J = 8.2 Hz, 1H), 4.08 (q, J = 7.0 Hz, 2H), 3.71 (m, 2H), 3.86 (s, 3H), 2.98 (m, 2H), 2.63 (m, 1H), 1.85 (m, 2H), 1.47-1.55 (m, 2H), 1.19 (t, J = 7.0 Hz, 3H).

### Example 27

### 3-(4-hydroxy-3-methoxyphenyl)-8-[(3-hydroxy-1-peridinyl)carbonyl]-5,10-dihydro-11H-dibenzo[b,e][1,4]diazepin-11-one

The desired product was prepared by substituting 3-piperidinol for 3-pyrrolidine-1-ylpropylamine in Example 14. MS (DCI) m/e 460 (M+H)⁺; ¹H NMR (500 MHz, DMSO-d₆) δ 9.85 (s, 1H), 9.28 (s, 1H), 8.11 (s, 1H), 7.73 (d, J = 8.2 Hz, 1H), 7.24 (d, J = 1.5 Hz, 1H), 7.17-7.19 (m, 2H), 7.08 (dd, J = 8.2, 1.5 Hz, 1H), 6.98-7.04 (m, 3H), 6.88 (d, J = 8.2 Hz, 1H), 3.86 (s, 3H), 3.48 (m, 1H), 2.98 (m, 2H), 1.38-1.84 (m, 6H).

### Example 28

### 3-(4-hydroxy-3-methoxyphenyl)-11-oxoN(3-pyridinylmethyl)-10,11-dihydro-5H-dibenzo[b,e][1,4]diazepine-8-carboxamide

The desired product was prepared by substituting 3-pyridinylmethylamine for 3-pyrrolidin-1-ylpropylamine in Example 14. MS (DCI) m/e 467 (M+H)⁺; ¹H NMR (500 MHz, DMSO-d₆) (TFA salt) δ 9.88 (s, 1H), 8.99 (t, J = 5.8 Hz, 1H), 8.73 (s, 1H), 8.67 (d, J = 4.9 Hz, 1H), 8.25 (s, 1H), 8.15 (d, J = 7.9 Hz, 1H), 7.73-7.75 (m, 2H), 7.51 (s, 1H), 7.49 (d, J = 1.8 Hz, 1H), 7.25 (d, J = 1.5 Hz, 1H), 7.17-7.19 (m, 2H), 7.09 (dd, J = 8.2, 2.1 Hz, 1H), 7.07 (d, J = 8.5 Hz, 1H), 6.88 (d, J = 7.9 Hz, 1H), 4.55 (d, J = 5.5 Hz, 2H), 3.86 (s, 3H).

### Example 29

### 3-(4-hydroxy-3-methoxyphenyl)N[4-(methylsulfonyl)benzyl]-11-oxo-10,11-dihydro-5H-dibenzo[b,e][1,4]diazepine-8-carboxamide

The desired product was prepared by substituting 1-[4-(methylsulfonyl)phenyl]methanamine for 3-pyrrolidin-1-ylpropylamine in Example 14. MS (DCI) m/e 545 (M+H)⁺; ¹H NMR (500 MHz, DMSO-d₆) δ 9.84 (s, 1H), 9.25 (s, 1H), 8.94 (t, J = 5.9 Hz, 1H), 8.20 (s, 1H), 7.88 (d, J = 8.1 Hz, 1H), 7.74 (d, J = 8.4 Hz, 1H), 7.55 (d, J = 8.4 Hz, 1H), 7.50-7.52 (m, 2H), 7.24 (d, J = 1.6 Hz, 1H), 7.18 (m, 2H), 7.09 (dd, J = 8.1, 2.2 Hz, 1H), 7.05 (d, J = 8.1 Hz, 1H), 6.88 (d, J = 8.1 Hz, 1H), 4.53 (d, J = 5.9 Hz, 2H), 3.86 (s, 3H), 3.18 (s, 3H).

### Example 30

### N-(2-fluorobenzyl)-3-(4-hydroxy-3-methoxyphenyl)-11-oxo-10,11-dihydro-5H-dibenzo[b,e][1,4]diazepine-8-carboxamide

The desired product was prepared by substituting 2-fluorobenzylamine for 3-pyrrolidin-1-ylpropylamine in Example 14. MS (DCI) m/e 484 (M+H)⁺; ¹H NMR (500 MHz, DMSO-d₆) δ 9.83 (s, 1H), 9.25 (s, 1H), 8.78 (t, J = 5.8 Hz, 1H), 8.19 (s, 1H), 7.74 (d, J = 8.1 Hz, 1H), 7.49-7.51 (m, 2H), 7.28-7.36 (m, 2H), 7.24 (s, 1H), 7.14-7.18 (m, 3H), 7.08 (dd, J = 8.3, 2.0 Hz, 1H), 7.04 (d, J = 8.1 Hz, 1H), 6.88 (d, J = 8.1 Hz, 1H), 4.45 (d, J = 5.9 Hz, 2H), 3.86 (s, 3H).

### Example 31

### 3-(4-hydroxy-3-methoxyphenyl)N(2-methoxybenzyl)-11-oxo-10,11-dihydro-5H-dibenzo[b,e][1,4] diazepine-8-carboxamide

The desired product was prepared by substituting 2-methoxybenzylamine for 3-pyrrolidin-1-ylpropylamine in Example 14. MS (DCI) m/e 496 (M+H)⁺; ¹H NMR (500 MHz, DMSO-d₆) δ 9.84 (s, 1H), 9.25 (s, 1H), 8.90 (t, J = 5.9 Hz, 1H), 8.20 (s, 1H), 7.93 (s, 1H), 7.91 (s, 1H), 7.74 (d, J = 8.1 Hz, 1H), 7.50-7.52 (m, 2H), 7.44 (s, 1H), 7.42 (s, 1H), 7.24 (d, J = 1.6 Hz, 1H), 7.14-7.18 (m, 2H), 7.08 (dd, J = 8.3, 2.0 Hz, 1H), 7.05 (d, J = 8.1 Hz, 1H), 6.88 (d, J = 8.1 Hz, 1H), 4.51 (d, J = 5.9 Hz, 2H), 3.86 (s, 3H), 3.84 (s, 3H).

### Example 32

### 3-(4-hydroxy-3-methoxyphenyl)-11-oxoN(2-pyridinylmethyl)-10,11-dihydro-5H-dibenzor[b,e][1,4]diazepine-8-carboxamide

The desired product was prepared by substituting 2-pyridinylmethylamine for 3-pyrrolidin-1-ylpropylamine in Example 14. MS (DCI) m/e 467 (M+H)⁺; ¹H NMR (500 MHz, DMSO-d₆) (TFA salt) δ 9.86 (s, 1H), 8.98 (t, J = 5.8 Hz, 1H), 8.64 (d, J = 4.7 Hz, 1H), 8.23 (s, 1H), 8.06 (m, 1H), 7.75 (d, J = 8.4 Hz, 1H), 7.52-7.56 (m, 3H), 7.25 (d, J = 1.6 Hz, 1H), 7.17-7.19 (m, 2H), 7.06-7.10 (m, 2H), 6.88 (d, J = 8.1 Hz, 1H), 4.62 (d, J = 5.6 Hz, 2H), 3.86 (s, 3H).

### Example 33

### 3-(4-hydroxy-3-methoxyphenyl)-11-oxoN(4-pyridinylmethyl)-10,11-dihydro-5H-dibenzo[b,e][1,4]diazepine-8-carboxamide

The desired product was prepared by substituting 4-pyridinylmethylamine for 3-pyrrolidin-1-ylpropylamine in Example 14. MS (DCI) m/e 467 (M+H)⁺; ¹H NMR (500 MHz, DMSO-d₆) (TFA salt) δ 9.86 (s, 1H), 9.05 (t, J = 5.9 Hz, 1H), 8.73 (d, J = 6.2 Hz, 1H), 8.24 (s, 1H), 7.71-7.76 (m, 2H), 7.54 (d, J = 1.9 Hz, 1H), 7.53 (s, 1H), 7.25 (d, J = 1.6 Hz, 1H), 7.17-7.19 (m, 2H), 7.06-7.10 (m, 2H), 6.88 (d, J = 8.1 Hz, 1H), 4.62 (d, J = 5.6 Hz, 2H), 3.86 (s, 3H).

### Example 34

### 3-(4-hydroxy-3-methoxyphenyl)N[2-(4-methoxyphenyl)ethyl]-11-oxo-10,11-dihydro-5H-dibenzo[b,e][1,4]diazepine-8-carboxamide

The desired product was prepared by substituting 2-(2-methoxyphenyl)ethylamine for 3-pyrrolidin-1-ylpropylamine in Example 14. MS (DCI) m/e 510 (M+H)⁺;¹H NMR (500 MHz, DMSO-d₆) δ 9.84 (s, 1H), 9.25 (t, J = 5.9 Hz, 1H), 8.29 (d, J = 5.6 Hz, 1H), 8.16 (s, 1H), 7.74 (d, J = 8.1 Hz, 1H), 7.46 (d, J = 1.9 Hz, 1H), 7.41 (dd, J = 8.4, 1.9 Hz, 1H), 7.24 (d, J = 1.6 Hz, 1H), 7.17-7.18 (m, 2H), 7.12-7.15 (m, 2H), 7.08 (dd, J = 8.3, 2.0 Hz, 1H), 7.02 (d, J = 8.2 Hz, 1H), 6.88 (d, J = 8.1 Hz, 1H), 6.83-6.86 (m, 2H), 3.86 (s, 3H), 3.71 (s, 3H), 3.38-3.42 (m, 2H), 2.75 (t, J = 7.3 Hz, 2H).

### Example 35

### 1-{[3-(4-hydroxy-3-methoxyphenyl)-11-oxo-10,11-dihydro-5H-dibenzo[b,e][1,4]diazepin-8-yl]carbonyl}-2-piperidinecarboxylic acid

The desired product was prepared by substituting Example 24 for Example 12 in Example 13. MS (ESI) m/e 486 (M-H)⁻; ¹H NMR (500 MHz, DMSO-d₆,) δ 12.90 (br s, 1H), 9.85 (s, 1H), 9.24 (s, 1H), 8.10 (s, 1H), 7.73 (d, J = 8.1 Hz, 1H), 7.24 (s, 1H), 7.16-7.18 (m, 1H), 7.08 (dd, J = 8.3, 2.0 Hz, 1H), 6.92-7.05 (m, 3H), 6.87 (d, J = 8.1 Hz, 1H), 5.11 (m, 1H), 3.86 (s, 3H), 3.59 (m, 1H), 3.16 (m, 1H), 1.30-2.16 (m, 6H).

### Example 36

### 1-{[3-(4-hydroxy-3-methoxyphenyl)-11-oxo-10,11-dihydro-5H-dibenzo[b,e][1,4]diazepin-8-yl]carbonyl}-3-piperidinecarboxylic acid

The desired product was prepared by substituting Example 25 for Example 12 in Example 13. MS (ESI) m/e 486 (M-H)⁻; ¹H NMR (500 MHz, DMSO-d₆) δ12.36 (s, 1H), 9.84 (s, 1H), 9.24 (s, 1H), 8.10 (s, 1H), 7.73 (d, J = 8.4 Hz, 1H), 7.24 (d, J = 1.6 Hz, 1H), 7.16-7.18 (m, 2H), 7.08 (dd, J = 8.3, 2.0 Hz, 1H), 6.98-7.04 (m, 3H), 6.88 (d, J = 8.4 Hz, 1H), 3.86 (s, 3H), 2.90-3.02 (m, 2H), 2.43 (m, 1H), 1.98 (m, 1H), 1.41-1.63 (m, 3H).

### Example 37

### 1-{[3-(4-hydroxy-3-methoxyphenyl)-11-oxo-10,11 -dihydro-5H-dibenzo[b,e][1,4]diazepin-8-yl]carbonyl}-4-piperidinecarboxylic acid

The desired product was prepared by substituting Example 26 for Example 12 in Example 13. MS (ESI) m/e 486 (M-H)⁻; ¹H NMR (500 MHz, DMSO-d₆) δ 12.25 (s, 1H), 9.83 (s, 1H), 9.24 (s, 1H), 8.10 (s, 1H), 7.73 (d, J = 8.1 Hz, 1H), 7.24 (d, J = 1.6 Hz, 1H), 7.16-7.18 (m, 2H), 7.08 (dd, J = 8.1, 2.2 Hz, 1H), 6.98-7.04 (m, 3H), 6.88 (d, J = 8.1 Hz, 1H), 3.86 (s, 3H), 2.90-3.02 (m, 2H), 2.45-2.54 (m, 3H), 1.80-1.86 (m, 2H), 1.46-1.34 (m, 2H).

### Example 38

### tert-butyl 2-{[3-(4-hydroxy-3-methoxyphenyl)-11-oxo-10,11-dihydro-5H-dibenzo[b,e][1,4]diazepin-8-yl]carbonyl}hydrazinecarboxylate

The desired product was prepared by substituting tert-butyl hydrazinecarboxylate for 3-pyrrolidin-1-ylpropylamine in Example 14. MS (DCI) m/e 491 (M+H)⁺; ¹H NMR (500 MHz, DMSO-d₆) δ 9.75 (br s, 1H), 9.64 (s, 1H), 8.55 (s, 1H), 7.99 (s, 1H), 7.50 (d, J = 8.3 Hz, 1H), 7.19-7.23 (m, 2H), 7.00 (d, J = 1.53 Hz, 1H), 6.98-6.95 (m, 2H), 6.84 (dd, J = 8.3, 2.2 Hz, 1H), 6.80 (d, J = 8.3 Hz, 1H), 6.64 (d, J = 8.3 Hz, 1H), 3.82 (s, 3H), 1.18 (s, 9H).

### Example 39

### 3-(4-hydroxy-3-methoxyphenyl)-11-oxo-10,11-dihydro-5H-dibenzo[b,e][1,4]diazepine-8-carbonitrile

The desired product was prepared by substituting Example 4 for Example 1B in Example 12. MS (DCI) m/e 357 (M+H)⁺, 375 (M+NH₄)⁺; ¹H NMR (500 MHz, DMSO-d₆) δ 9.92 (s, 1H), 9.27 (s, 1H), 8.50 (s, 1H), 7.77 (d, J = 8.1 Hz, 1H), 7.39 (dd, J = 8.4, 1.9 Hz, 1H), 7.30 (d, J = 1.9 Hz, 1H), 7.18-7.23 (m, 3H), 7.08-7.11 (m, 2H), 6.84 (d, J = 8.1 Hz, 1H), 3.86 (s, 3H).

### Example 40

### 3-(4-hydroxy-3-methoxyphenyl)-8-nitro-5,10-dihydro-11H-dibenzo[b,e][1,4]diazepin-11-one

The desired product was prepared by substituting Example 3 for Example 1B in Example 12. MS (DCI) m/e 378 (M+H)⁺, 395 (M+NH₄)⁺; ¹H NMR (500 MHz, DMSO-d₆) δ 10.03 (s, 1H), 9.29 (s, 1H), 8.77 (s, 1H), 7.83-7.87 (m, 2H), 7.80 (d, J = 8.3 Hz, 1H), 7.19-7.25 (m, 3H), 7.08-7.13 (m, 2H), 6.89 (d, J = 8.3 Hz, 1H), 3.86 (s, 3H).

### Example 41

### 8-amino-3-(4-hydroxy-3-methoxyphenyl)-5,10-dihydro-11H-dibenzo[b,e][1,4]diazepin-11-one

The desired product was prepared by substituting Example 7C for Example 1B in Example 12. MS (DCI) m/e 347 (M+H)⁺; ¹H NMR (500 MHz, DMSO-d₆) (HCl salt) δ 9.99 (s, 1H), 9.26 (br s, 1H), 8.07 (s, 1H), 7.73 (d, J = 8.1 Hz, 1H), 7.26 (d, J = 1.6 Hz, 1H), 7.16-7.18 (m, 2H), 7.07-7.08 (m, 2H), 6.87-6.93 (m, 3H), 3.86 (s, 3H).

### Example 42

### N-[3-(4-hydroxy-3-methoxyphenyl)-11-oxo-10,11-dihydro-5H-dibenzo[b,e][1,4]diazepin-8-yl]methanesulfonamide

### Example 42A

### N-(3-chloro-11-oxo-10,11-dihydro-5H-dibenzo[b,e][1,4]diazepin-8-yl)methanesulfonamide

A mixture of Example 7C (30mg, 0.11 mmol) and CH₃SO₂Cl (13mg, 0.112 mmol) in pyridine (4 mL) was stirred overnight at room temperature. The excess pyridine was removed under reduced pressure and the residue was washed with hexanes and filtered. The filter cake was dried in a vacuum oven to provide the desired product. MS (DCI) m/e 338 (M+H)⁺, 355 (M+NH₄)⁺.

### Example 42B

### N-[3-(4-hydroxy-3-methoxyphenyl)-11-oxo-10,11-dihydro-5H-dibenzo[b,e][1,4]diazepin-8-yl]methanesulfonamide

The desired product was prepared by substituting Example 42A for Example 1B in Example 12. MS (DCI) m/e 426 (M+H)⁺; ¹H NMR (500 MHz, DMSO-d₆) δ 9.81 (s, 1H), 9.46 (s, 1H), 9.22 (br s, 1H), 7.84 (s, 1H), 7.71 (d, J = 8.1 Hz, 1H), 7.22 (d, J = 1.6 Hz, 1H), 7.14-7.17 (m, 2H), 7.07 (dd, J = 8.3, 2.0 Hz, 1H), 6.97 (d, J = 8.4 Hz, 1H), 6.91 (d, J = 2.5 Hz, 1H), 6.87 (d, J= 8.1 Hz, 1H), 6.80 (dd, J = 8.4, 2.2 Hz, 1H), 3.86 (s, 3H), 2.92 (s, 3H).

### Example 43

### N-[3-(3-methoxy-4-nitrophenyl)-11-oxo-10,11-dihydro-5H-dibenzo[b,e][1,4]diazepin-8-yl]methanesulfonamide

The desired product was prepared by substituting Example 43A and 2-(3-methoxy-4-nitrophenyl)-4,4,5,5-tetramethyl-1,3,2-dioxaborolane for Example 1B and 2-methoxy-4-(4,4,5,5-tetramethyl-1,3,2-dioxaborolan-2-yl)phenol, respectively, in Example 12. MS (DCI) m/e 454 (M+H)⁺, 472 (M+NH₄)⁺; ¹H NMR (500 MHz, DMSO-d₆) δ 9.93 (s, 1H), 9.48 (s, 1H), 8.00 (d, J = 8.4 Hz, 1H), 7.97 (s, 1H), 7.79 (d, J = 8.1 Hz, 1H), 7.50 (d, J = 1.3 Hz, 1H), 7.32-7.34 (m, 2H), 7.29 (dd, J = 8.1, 1.6 Hz, 1H), 6.97 (d, J = 8.4 Hz, 1H), 6.92 (d, J = 2.2 Hz, 1H), 6.81 (dd, J = 8.4, 2.2 Hz, 1H), 4.02 (s, 3H), 2.91 (s, 3H).

### Example 44

### N-[3-(4-hydroxy-3-methoxyphenyl)-11-oxo-10,11-dihydro-5H-dibenzo[b,e][1,4]diazepin-8-yl]acetamide

### Example 44A

### N-(3-chloro-11-oxo-10,11-dihydro-5H-dibenzo[b,e][1,4]diazepin-8-yl)acetamide

A solution of Example 7C (100mg, 0.38 mmol) in pyridine (10 mL) at 0 °C was treated with acetyl chloride (32mg, 0.40 mmol), stirred overnight, and concentrated. The residue was purified by flash column chromatography on silica gel with ethyl acetate to provide 64mg (56%) of the desired product. MS (DCI) m/e 302 (M+H)⁺, 319 (M+NH₄)⁺; ¹H NMR (500 MHz, DMSO-d₆) δ 9.93 (s, 1H), 9.81 (s, 1H), 7.93 (s, 1H), 7.67 (d, J = 8.4 Hz, 1H), 7.25 (d, J = 1.9 Hz, 1H), 7.16 (dd, J = 8.4, 2.2 Hz, 1H), 7.05 (d, J = 1.9 Hz, 1H), 6.90 (dd, J = 8.4, 1.9 Hz, 1H), 6.88 (d, J = 8.7 Hz, 1H), 1.99 (s, 3H).

### Example 44B

### N-[3-(4-hydroxy-3-methoxyphenyl)-11-oxo-10,11-dihydro-5H-dibenzo[b,e][1,4]diazepin-8-yl]acetamide

The desired product was prepared by substituting Example 44A for Example 1B in Example 12. MS (DCI) m/e 390 (M+H)⁺; ¹H NMR (500 MHz, DMSO-d₆) δ 9.80 (s, 1H), 9.78 (s, 1H), 9.22 (s, 1H), 7.75 (s, 1H), 7.70 (d, J = 8.1 Hz, 1H), 7.21-7.24 (m, 2H), 7.12-7.17 (m, 3H), 7.07 (m, 1H), 6.91 (d, J = 8.7 Hz, 1H), 6.87 (d, J = 8.1 Hz, 1H), 3.85 (s, 3H), 1.99 (s, 2H).

### Example 45

### N-[3-(3-methoxy-4-nitrophenyl)-11-oxo-10,11-dihydro-5H-dibenzo[b,e][1,4]diazepin-8-yl]acetamide

The desired product was prepared by substituting Example 44A and 2-(3-methoxy-4-nitrophenyl)-4,4,5,5-tetramethyl-1,3,2-dioxaborolane for Example 1B and 2-methoxy-4-(4,4,5,5-tetramethyl-1,3,2-dioxaborolan-2-yl)phenol, respectively, in Example 12. MS (DCI) m/e 419 (M+H)⁺; ¹H NMR (500 MHz, DMSO-d₆) δ 9.93 (s, 1H), 9.81 (s, 1H), 8.00 (d, J = 8.4 Hz, 1H), 7.89 (s, 1H), 7.79 (d, J = 8.1 Hz, 1H), 7.52 (d, J = 1.6 Hz, 1H), 7.35-7.37 (m, 2H), 7.26-7.30 (m, 2H), 7.16 (dd, J = 8.4, 2.2 Hz, 1H), 6.93 (d, J = 8.4 Hz, 1H), 4.03 (s, 3H), 2.00 (s, 3H).

### Example 46

### 8-(3-aminophenyl)-3-(4-hydroxy-3-methoxyphenyl)-5,10-dihydro-11H-dibenzo[b,e][1,4]diazepin-11-one

### Example 46A

### 8-(3-aminophenyl)-3-chloro-5,10-dihydro-11H-dibenzo[b,e][1,4]diazepin-11-one

The desired product was prepared by substituting Example 2 and 3-(4,4,5,5-tetramethyl-1,3,2-dioxaborolan-2-yl)phenylamine for Example 1B and 2-methoxy-4-(4,4,5,5-tetramethyl-1,3,2-dioxaborolan-2-yl)phenol, respectively, in Example 12. MS (DCI) m/e 335 (M+H)⁺, 353 (M+NH₄)⁺; ¹H NMR (500 MHz, DMSO-d₆) δ 9.94 (s, 1H), 8.13 (s, 1H), 7.72 (d, J = 8.4 Hz, 1H), 7.17-7.19 (m, 2H), 7.06-7.09 (m, 2H), 7.01 (d, J = 8.11 Hz, 1H), 6.93 (dd, J = 8.6,2.0 Hz, 1H), 6.73 (d, J = 1.9 Hz, 1H), 6.66 (d, J = 7.8 Hz, 1H), 6.52 (dd, J = 7.5, 1.9 Hz, 1H), 5.11 (s, 2H).

### Example 46B

### 8-(3-aminophenyl)-3-(4-hydroxy-3-methoxyphenyl)-5,10-dihydro-11H-dibenzo[b,e][1,4]diazepin-11-one

The desired product was prepared by substituting Example 46A for Example 1B in Example 12. MS (DCI) m/e 424 (M+H)⁺; ¹H NMR (500 MHz, DMSO-d₆,) δ 9.91 (s, 1H), 9.24 (s, 1H), 7.97 (s, 1H), 7.74 (d, J = 8.1 Hz, 1H), 7.25 (d, J = 1.3 Hz, 1H), 7.03-7.19 (m, 7H), 6.88 (d, J = 8.11 Hz, 1H), 6.73 (s, 1H), 6.67, (d, J = 7.8 Hz, 1H), 6.62 (dd, J = 8.0, 1.4 Hz, 1H), 5.10 (s, 2H), 3.86 (s, 3H).

### Example 47

### 3-(4-hydroxy-3-methoxyphenyl)-8-(3-hydroxyphenyl)-5,10-dihydro-11H-dibenzo[b,e][1,4]diazepin-11-one

### Example 47A

### 3-chloro-8-(3-hydroxyphenyl)-5.10-dihydro-11H-dibenzo[b,e][1,4]diazepin-11-one

The desired product was prepared by substituting Example 2 and 3-(4,4,5,5-tetramethyl-1,3,2-dioxaborolan-2-yl)phenol for Example 1B and 2-methoxy-4-(4,4,5,5-tetramethyl-1,3,2-dioxaborolan-2-yl)phenol, respectively, in Example 12. MS (DCI) m/e 336 (M+H)⁺, 354 (M+NH₄)⁺; ¹H NMR (500 MHz, DMSO-d₆) δ 9.95 (s, 1H), 9.49 (s, 1H), 8.17 (s, 1H), 7.72 (d, J = 8.3 Hz, 1H), 7.20-7.24 (m, 3H), 7.09 (d, J = 2.2 Hz, 1H), 7.03 (d, J = 8.11 Hz, 1H), 6.91-6.97 (m, 3H), 6.73, (dd, J = 8.0, 1.5 Hz, 1H).

### Example 47B

### 3-(4-hydroxy-3-methoxyphenyl)-8-(3-hydroxyphenyl)-5,10-dihydro-11H-dibenzo[b,e][1,4]diazepin-11-one

The desired product was prepared by substituting Example 47A for Example 1B in Example 12. MS (DCI) m/e 425 (M+H)⁺; ¹H NMR (500 MHz, DMSO-d₆) δ 9.81 (s, 1H), 9.46 (s, 1H), 9.24 (s, 1H), 8.00 (s, 1H), 7.75 (d, J = 8.1 Hz, 1H), 7.15-7.25 (m, 7H), 7.09 (dd, J = 8.3, 2.0 Hz, 1H), 7.06 (d, J = 8.11 Hz, 1H), 6.96 (d, J = 7.8 Hz, 1H), 6.92 (s, 1H), 6.88 (d, J = 8.1 Hz, 1H), 6.72 (dd, J = 8.0, 2.0 Hz, 1H), 3.87 (s, 3H).

### Example 48

### 3-(4-hydroxy-3-methoxyphenyl)-8-(3-pyridinyl)-5,10-dihydro-11H-dibenzo[b,e][1,4]diazepin-11-one

### Example 48A

### 3-chloro-8-(3-pyridinyl)-5,10-dihydro-11H-dibenzo[b,e][1,4]diazepin-11-one

The desired product was prepared by substituting Example 2 and diethyl 3-pyridinylboronate for Example 1B and 2-methoxy-4-(4,4,5,5-tetramethyl-1,3,2-dioxaborolan-2-yl)phenol, respectively, in Example 12. MS (DCI) m/e 322 (M+H)⁺; ¹H NMR (500 MHz, DMSO-d₆) δ 9.95 (s, 1H), 8.78 (d, J = 2.2 Hz, 1H), 8.53 (dd, J = 4.7, 1.3 Hz, 1H), 8.24 (s, 1H), 7.94 (m, 1H), 7.72 (d, J = 8.6 Hz, 1H), 7.46 (dd, J = 7.5,4.8 Hz, 1H), 7.31-7.36 (m, 2H), 7.09 (m, 1H), 6.95 (dd, J = 8.1, 2.0 Hz, 1H).

### Example 48B

### 3-(4-hydroxy-3-methoxyphenyl)-8-(3-pyridinyl)-5,10-dihydro-11H-dibenzo[b,e][1,4]diazepin-11-one

The desired product was prepared by substituting Example 48A for Example 1B in Example 12. MS (DCI) m/e 410 (M+H)⁺; ¹H NMR (500 MHz, DMSO-d₆) δ 9.81 (s, 1H), 9.25 (s, 1H), 8.79 (d, J = 1.9 Hz, 1H), 8.25 (d, J = 3.4 Hz, 1H), 8.09 (s, 1H), 7.95 (dd, J = 6.2, 2.2 Hz, 1H), 7.76 (d, J = 8.4 Hz, 1H), 7.46 (dd, J = 7.9, 4.9 Hz, 1H), 7.32-7.35 (m, 2H), 7.26 (d, J = 1.6 Hz, 1H), 7.16-7.19 (m, 2H), 7.12 (d, J = 8.1 Hz, 1H), 7.10 (dd, J = 8.3, 2.0 Hz, 1H), 6.88 (d, J = 8.1 Hz, 1H), 3.87 (s, 3H).

### Example 49

### 3-(4-hydroxy-3-methoxyphenyl)-8-(1H-pyrrol-2-yl)-5,10-dihydro-11H-dibenzo[b,e][1,4]diazepin-11-one

### Example 49A

### 3-chloro-8-(1H-pyrrol-2-yl)-5,10-dihydro-11H-dibenzo[b,e][1,4]diazepin-11-one

The desired product was prepared by substituting Example 2 and 1-(tert-butoxycarbonyl)-1H-pyrrol-2-ylboronic acid for Example 9 and 2-methoxy-4-(4,4,5,5-tetramethyl-1,3,2-dioxaborolan-2-yl)phenol, respectively, in Example 10 MS (DCI) m/e 310 (M+H)⁺, 327 (M+NH₄)⁺; ¹H NMR (500 MHz, DMSO-d₆) δ 11.10 (s, 1H), 9.87 (s, 1H), 8.05 (s, 1H), 7.70 (d, J = 8.3 Hz, 1H), 7.18-7.22 (m, 2H), 7.07 (d, J = 2.2 Hz, 1H), 6.91-6.96 (m, 2H), 6.79 (m, 1H), 6.29 (t, J = 3.7 Hz, 1H), 6.08 (m, 1H).

### Example 49B

### 3-(4-hydroxy-3-methoxyphenyl)-8-(1H-pyrrol-2-yl)-5,10-dihydro-11H-dibenzo[b,e][1,4]diazepin-11-one

The desired product was prepared by substituting Example 49A for Example 1B in Example 12. MS (DCI) m/e 398 (M+H)⁺; ¹H NMR (500 MHz, DMSO-d₆) δ 11.09 (s, 1H), 9.74 (s, 1H), 9.24 (br s, 1H), 7.89 (s, 1H), 7.73 (d, J = 6.9 Hz, 1H), 7.08-7.24 (m, 6H), 6.99 (d, J = 6.6 Hz, 1H) 6.88 (d, J = 6.9 Hz, 1H), 6.78 (s, 1H), 6.29 (s, 1H), 6.08 (s, 1H), 3.86 (s, 3H).

### Example 50

### 3-(3-methoxy-4-nitrophenyl)-8-(1H-pyrrol-2-yl)-5,10-dihydro-11H-dibenzo[b,e][1,4]diazepin-11-one

The desired product was prepared by substituting Example 49A and 2-(3-methoxy-4-nitrophenyl)-4,4,5,5-tetramethyl-1,3,2-dioxaborolane for Example 1B and 2-methoxy-4-(4,4,5,5-tetramethyl-1,3,2-dioxaborolan-2-yl)phenol, respectively, in Example 12. MS (DCI) m/e 427 (M+H)⁺, 444 (M+NH₄)⁺; ¹H NMR (500 MHz, DMSO-d₆) δ 11.10 (s, 1H), 9.87 (s, 1H), 8.00-8.02 (m, 2H), 7.82 (d, J = 8.3 Hz, 1H), 7.53 (s, 1H), 7.34-7.36 (m, 2H), 7.30 (dd, J = 8.3, 1.5 Hz, 1H), 7.20-7.22 (m, 2H), 7.00 (d, J = 8.9 Hz, 1H), 6.79 (d, J = 1.2 Hz, 1H), 6.29 (s, 1H), 6.08 (d, J = 2.8 Hz, 1H), 4.04 (s, 3H).

### Example 51

### methyl [3-(3-methoxy-4-nitrophenyl)-11-oxo-10,11-dihydro-5H-dibenzo[b,e][1,4]diazepin-8-yl]acetate

The desired product was prepared by substituting Example 6D and 2-(3-methoxy-4-nitrophenyl)-4,4,5,5-tetramethyl-1,3,2-dioxaborolane for Example 1B and 2-methoxy-4-(4,4,5,5-tetramethyl-1,3,2-dioxaborolan-2-yl)phenol, respectively, in Example 12. MS (ESI) m/e 432 (M-H)⁻; ¹H NMR (500 MHz, DMSO-d₆) δ 9.87 (s, 1H), 7.98-8.01 (m, 2H), 7.78 (d, J = 8.4 Hz, 1H), 7.51 (d, J = 1.6 Hz, 1H), 7.30-7.34 (m, 2H), 7.29 (dd, J = 8.3, 1.7 Hz, 1H), 6.96 (d, J = 7.8 Hz, 1H), 6.85-6.87 (m, 2H), 4.02 (s, 3H), 3.59 (s, 3H), 3.53 (s, 2H).

### Example 52

### methyl [3-(4-hydroxy-3-methoxyphenyl)-11-oxo-10,11-dihydro-5H-dibenzo[b,e][1,4]diazepin-8-yl]acetate

The desired product was prepared by substituting Example 6D for Example 1B in Example 12. MS (DCI) m/e 404 (M+H)⁺; ¹H NMR (500 MHz, DMSO-d₆) δ 9.74 (s, 1H), 9.23 (s, 1H), 7.86 (s, 2H), 7.71 (d, J = 8.1 Hz, 1H), 7.23 (d, J = 1.6 Hz, 1H), 7.17 (d, J = 1.9 Hz, 1H), 7.15 (dd, J = 8.4, 1.6 Hz, 1H), 7.07 (dd, J = 8.3, 2.0 Hz, 1H), 6.95 (d, J = 7.8 Hz, 1H), 6.84-6.88 (m, 3H), 3.86 (s, 3H), 3.60 (s, 3H), 3.53 (s, 2H).

### Example 53

### methyl [3-(3-methoxyphenyl)-11-oxo-10,11-dihydro-5H-dibenzo[b,e][1,4]diazepin-8-yl]acetate

The desired product was prepared by substituting Example 6D and 3-methoxyphenylboronic acid for Example 1B and 2-methoxy-4-(4,4,5,5-tetramethyl-1,3,2-dioxaborolan-2-yl)phenol, respectively, in Example 12. MS (ESI) m/e 387 (M-H)⁻; ¹H NMR (300 MHz, DMSO-d₆) δ 9.83 (s, 1H), 7.92 (s, 1H), 7.75 (d, J = 8.14 Hz, 1H), 7.39 (d, J = 7.80 Hz, 1H), 7.29 (d, J = 1.70 Hz, 1H), 7.15-7.22 (m, 3H), 6.94-7.01 (m, 2H), 6.84-6.87 (m, 2H), 3.83 (s, 3H), 3.60 (s, 3H), 3.54 (s, 2H).

### Example 54

### methyl [3-(4-chloro-3-methoxyphenyl)-11-oxo-10,11-dihydro-5H-dibenzo[b,e][1,4]diazepin-8-yl]acetate

### Example 54A

### methyl [11-oxo-3-(4,4,5,5-tetramethyl-1,3,2-dioxaborolan-2-yl)-10,11-dihydro-5H-dibenzo[b,e][1,4]diazepin-8-yl]acetate

A mixture of CyMAP ligand (23mg, 0.058 mmol) and Pd₂(dba)₃ (11mg, 0.012 mmol) in dioxane (2.0 mL) purged with nitrogen was stirred at room temperature under nitrogen for 30 minutes, treated with Example 6D (93mg, 0.29 mmol), bis(pinacolato)diboron (85mg, 0.33 mmol), and potassium acetate (47mg, 0.47 mmol), stirred at 85 °C under nitrogen overnight, cooled to room temperature, diluted with ethyl acetate, filtered through diatomaceous earth (Celite^{®}), and concentrated under vacuum. The residue was dissolved in dichloromethane (2.5 mL), treated with hexanes (10.0 mL), concentrated to a final volume of about 5-6 mL, and filtered. The filter cake provided 99mg (83%) of the desired product. MS (DCI/NH₃) m/e 409 (M+H)⁺.

### Example 54B

### methyl[3-(4-chloro-3-methoxyphenyl)-11-oxo-10,11-dihydro-5H-dibenzo[b,e][1,4]diazepin-8-yl]acetate

The desired product was prepared by substituting Example 54A and 2-chloro-5-iodoanisole for 2-methoxy-4-(4,4,5,5-tetramethyl-1,3,2-dioxaborolan-2-yl)phenol and Example 1B, respectively, in Example 10. MS (DCI) m/e 423 (M+H)⁺, 440 (M+NH₄)⁺; ¹H NMR (500 MHz, DMSO-d₆) δ 9.83 (s, 1H), 7.94 (s, 1H), 7.77 (d, J = 8.0 Hz, 1H), 7.53 (d, J = 8.3 Hz, 1H), 7.29 (d, J = 1.7 Hz, 1H), 7.34 (d, J = 1.8 Hz, 1H), 7.30 (d, J = 1.8 Hz, 1H), 7.19-7.24 (m, 2H), 6.96 (d, J = 8.0 Hz, 1H), 6.84-6.87 (m, 2H), 3.96 (s, 3H), 3.60 (s, 3H), 3.54 (s, 2H).

### Example 55

### 3-(3-methoxy-4-nitrophenyl)-5,10-dihydro-11H-dibenzo[b,e][1,4]diazepin-11-one

The desired product was prepared by substituting 2-(3-methoxy-4-nitrophenyl)-4,4,5,5-tetramethyl-1,3,2-dioxaborolane for 2-methoxy-4-(4,4,5,5-tetramethyl-1,3,2-dioxaborolan-2-yl)phenol in Example 10. MS (DCI) m/e 362 (M+H)⁺, 379 (M+NH₄)⁺; ¹H NMR (500 MHz, DMSO-d₆) δ 9.88 (s, 1H), 8.00-8.02 (m, 2H), 7.80 (d, J = 8.0 Hz, 1H), 7.53 (d, J = 1.8 Hz, 1H), 7.33-7.36 (m, 2H), 7.30 (dd, J = 8.1, 1.7 Hz, 1H), 6.90-7.03 (m, 4H), 4.03 (s, 3H).

### Example 56

### 3-(4-chloro-3-methoxyphenyl)-5,10-dihydro-11H-dibenzo[b,e][1,4]diazepin-11-one

### Example 56A

### 3-(4,4,5,5-tetramethyl-1,3,2-dioxaborolan-2-yl)-5,10-dihydro-11H-dibenzo[b,e][1,4]diazepin-11-one

A mixture of tri(cyclohexyl)phosphine (336mg, 1.2 mmol) and Pd₂(dba)₃ (230mg, 0.25 mmol) in dioxane (60 mL) purged with nitrogen was stirred at room temperature under nitrogen for 30 minutes, treated with Example 9 (2.89g, 10 mmol), bis(pinacolato)diboron (2.8g, 11 mmol), and potassium acetate (1.47g, 15 mmol) stirred at 85 °C under nitrogen overnight, cooled to room temperature, diluted with ethyl acetate, and poured into water. The organic layer was washed with brine, dried (MgSO₄), filtered, and concentrated under vacuum. The residue was purified by flash column chromatography on silica gel with 3:2 hexanes/ethyl acetate to provide 3.1g (92%) of the desired product. MS (DCI) m/e 337 (M+H)⁺, 354 (M+NH₄)⁺; ¹H NMR (500 MHz, DMSO-d₆) δ 9.86 (s, 1H), 7.82 (s, 1H), 7.68 (d, J = 7.8 Hz, 1H), 7.42 (s, 1H), 7.16 (dd, J = 7.8, 0.9 Hz, 1H), 7.02 (dd, J = 7.8, 1.6 Hz, 1H), 6.88-6.98 (m, 3H), 1.30 (s, 12H).

### Example 56B

### 3-(4-chloro-3-methoxyphenyl)-5,10-dihydro-11H-dibenzo[b,e][1,4]diazepin-11-one

The desired product was prepared by substituting Example 56A and 2-chloro-5-iodoanisole for 2-methoxy-4-(4,4,5,5-tetramethyl-1,3,2-dioxaborolan-2-yl)phenol and Example 9, respectively, in Example 10. MS (DCI) m/e 362 (M+H)⁺, 379 (M+NH₄)⁺; ¹H NMR (500 MHz, DMSO-d₆) δ 9.84 (s, 1H), 7.95 (s, 1H), 7.78 (d, J = 8.0 Hz, 1H), 7.53 (d, J = 8.0 Hz, 1H), 7.34 (d, J = 1.8 Hz, 1H), 7.31 (m, 1H), 7.20-7.25 (m, 2H), 6.89-7.03 (m, 2H), 3.96 (s, 3H).

### Example 57

### 3-(4-bromo-3-methoxyphenyl)-5,10-dihydro-11H-dibenzo[b,e][1,4]diazepin-11-one

### Example 57A

### 4-bromo-3-methoxyaniline

The desired product was prepared by substituting 2-bromo-5-nitroanisole for Example 6B in Example 6C. MS (ESI) m/e 203 (M+H)⁺; ¹H NMR (300 MHz, DMSO-d₆) δ 7.09 (d, J = 8.48 Hz, 1H), 6.30 (d, J = 2.37 Hz, 1H), 6.09 (dd, J = 8.48, 2.37 Hz, 1H), 5.28 (s, 2H), 3.72 (s, 3H).

### Example 57B

### 1-bromo-4-iodo-2-methoxybenzene

Example 57A (102mg, 0.50 mmol) was treated with concentrated HCl (10 mL), cooled to 0 °C, treated with a solution of NaNO₂ (45mg, 0.65 mmol) in H₂O (5 mL), stirred at 0 °C for 1 hour, treated with a solution of KI (249mg, 1.5 mmol) in H₂O (5 mL), stirred overnight while warming to room temperature, and extracted with ethyl acetate. The extract was washed with H₂O and 10% Na₂S₂O₃, dried (MgSO₄) filtered, and concentrated to provide 147mg (94%) of the desired product. MS (ESI) m/e 332 (M+20)⁺; ¹H NMR (300 MHz, DMSO-d₆) δ 7.39 (d, J = 1.87 Hz, 1H), 7.34 (d, J = 8.11 Hz, 1H), 7.24 (dd, J = 8.11, 1.87 Hz, 1H), 3.86 (s, 3H).

### Example 57C

### 3-(4-bromo-3-methoxyphenyl)-5,10-dihydro-11H-dibenzo[b,e][1,4]diazepin-11-one

The desired product was prepared by substituting Example 56A and Example 57B for 2-methoxy-4-(4,4,5,5-tetramethyl-1,3,2-dioxaborolan-2-yl)phenol and Example 9, respectively, in Example 10. MS (ESI) m/e 393 (M-H)⁻; ¹H NMR (500 MHz, DMSO-d₆) δ 9.83 (s, 1H), 7.95 (s, 1H), 7.77 (d, J = 8.11 Hz, 1H), 7.68 (d, J = 8.11 Hz, 1H), 7.31 (d, J = 1.56 Hz, 1H), 7.30 (d, J = 1.87 Hz, 1H), 7.23 (dd, J = 8.27, 1.72 Hz, 1H), 7.14 (dd, J = 8.11, 1.87 Hz, 1H), 6.89-7.02 (m, 4H), 3.95 (s, 3H).

### Example 58

### methyl [3-(4-bromo-3-methoxyphenyl)-11-oxo-10,11-dihydro-5H-dibenzo[b,e][1,4]diazepin-8-yl]acetate

The desired product was prepared by substituting Example 54A and Example 57B for 2-methoxy-4-(4,4,5,5-tetramethyl-1,3,2-dioxaborolan-2-yl)phenol and Example 9, respectively, in Example 10. MS (ESI) m/e 465 (M-H)⁻; ¹H NMR (300 MHz, DMSO-d₆) δ 9.85 (s, 1H), 7.95 (s, 1H), 7.76 (d, J = 8.11 Hz, 1H), 7.68 (d, J = 8.5 Hz, 1H), 7.29-7.30 (m, 2H), 7.30 (d, J = 1.87 Hz, 1H), 7.23 (dd, J = 8.4, 1.7 Hz, 1H), 7.14 (dd, J = 8.1, 2.0 Hz, 1H), 6.95 (m, 1H), 6.83-6.87 (m, 2H), 3.95 (s, 3H), 3.59 (s, 3H), 3.54 (s, 2H).

### Example 59

### 3-(4-acetyl-3-methoxyphenyl)-5,10-dihydro-11H-dibenzo[b,e][1,4]diazepin-11-one

### Example 59A

### 4-chloro-1-iodo-2-methoxybenzene

The desired product was prepared by substituting 2-amino-5-chloroanisole for Example 57A in Example 57B. MS (ESI) m/e 288 (M+20)⁺; ¹H NMR (300 MHz, DMSO-d₆) δ 7.76 (d, J = 8.48 Hz, 1H), 7.09 (d, J = 2.03 Hz, 1H), 6.84 (dd, J = 8.31, 2.20 Hz, 1H), 3.85 (s, 3H).

### Example 59B

### 1-(4-chloro-2-methoxyphenyl)ethanone

A solution of Example 59A (2.68g, 10 mmol) in DMF (40 mL) was treated with triethylamine (1.53 mL, 11 mmol), n-butyl vinyl ether (6.5 mL, 50 mmol), DPPF (554mg, 1 mmol) and Pd(OAc)₂ (112mg, 0.5 mmol), purged with nitrogen, heated to 80 °C for 6 hours, cooled to room temperature, and partitioned between ethyl acetate and water. The aqueous layer was extracted with ethyl acetate, and the combined organic layers were washed with water and brine, dried (MgSO₄), filtered, and concentrated under vacuum. The residue was purified by flash column chromatography on silica gel with 4:1 hexanes/ethyl acetate to provide 1.20g (65 %) of the desired product. MS (ESI) m/e 185 (M+H)⁺; ¹H NMR (300 MHz, DMSO-d₆) δ 7.60 (d, J = 8.14 Hz, 1H), 7.27 (d, J = 1.70 Hz, 1H), 7.09 (dd, J = 8.31, 1.86 Hz, 1H), 3.92 (s, 3H).

### Example 59C

### 3-(4-acetyl-3-methoxyphenyl)-5,10-dihydro-11H-dibenzo[b,e][1,4]diazepin-11-one

A mixture of Example 56A (67mg, 0.2 mmol), Example 59B (37mg, 0.2 mmol), CyMAP ligand (11.8mg, 0.03 mmol), Pd(OAc)₂ (4.9mg, 0.002 mmol), and CsF (91mg, 0.6 mmol) in DME (8 mL) and methanol (4 mL) was heated to reflux overnight, cooled to room temperature, and partitioned between water and ethyl acetate. The aqueous layer was extracted with ethyl acetate and the combined organic layers were washed with brine, dried (MgSO₄), filtered, and concentrated under vacuum. The residue was purified by flash column chromatography on silica gel with 1:1 hexanes/ethyl acetate to provide 0.036g (50%) of the desired product. MS (ESI) m/e 359 (M+H)⁺; ¹H NMR (300 MHz, DMSO-d₆) δ 9.88 (s, 1H), 7.99 (s, 1H), 7.79 (d, J = 8.14 Hz, 1H), 7.70 (d, J = 8.14 Hz, 1H), 7.27-7.36 (m, 3H), 6.91-7.01 (m, 5H), 4.00 (s, 3H), 2.56 (s, 3H).

### Example 60

### 2-methoxy-4-(11-oxo-10,11-dihydro-5H-dibenzo[b,e][1,4]diazepin-3-yl)benzonitrile

### Example 60A

### 4-chloro-2-methoxybenzonitrile

A mixture of Example 59A (2.68g, 10 mmol), Zn(CN)₂ (0.654g, 5.5 mmol), and Pd(PPh₃)₄ (0.577g, 0.5 mmol) in DMF (15 mL) was stirred at 90 °C for 6 hours and cooled to room temperature. The reaction mixture was poured into water (500 mL) and extracted with ethyl acetate several times. The combined extracts were washed with water and brine, dried (MgSO₄), filtered, and concentrated under vacuum. The residue was purified by flash column chromatography on silica gel with 9:1 hexanes/ethyl acetate to provide 1.34g (90%) of the desired product. MS (DCI) m/e 168 (M+H)⁺; ¹H NMR (300 MHz, CDCl₃) δ 7.49 (d, J = 8.1 Hz, 1H), 6.97-7.03 (m, 2H).

### Example 60B

### 2-methoxy-4-(11-oxo-10,11-dihydro-5H-dibenzo[b,e][1,4]diazepin-3-yl)benzonitrile

The desired product was prepared by substituting Example 60A for Example 59B in Example 59C. MS (DCI) m/e 342 (M+H)⁺, 359 (M+NH₄)⁺; ¹H NMR (500 MHz, DMSO-d₆) δ 9.88 (s, 1H), 7.98 (s, 1H), 7.84 (d, J = 7.8 Hz, 1H), 7.80 (d, J = 8.1 Hz, 1H), 7.42 (s, 1H), 7.33-7.35 (m, 2H), 7.29 (dd, J = 8.1, 1.6 Hz, 1H), 6.90-7.02 (m, 4H), 4.02 (s, 3H).

### Example 61

### methyl [3-(4-cyano-3-methoxyphenyl)-11-oxo-10,11-dihydro-5H-dibenzo[b,e][1,4]diazepin-8-yl]acetate

The desired product was prepared by substituting Example 54A and Example 60A for Example 56A and Example 59B, respectively, in Example 59C. MS (DCI) m/e 414 (M+H)⁺, 431 (M+NH₄)⁺; ¹H NMR (500 MHz, DMSO-d₆) δ 9.87 (s, 1H), 7.97 (s, 1H), 7.83 (d, J = 7.8 Hz, 1H), 7.79 (d, J = 8.1 Hz, 1H), 7.42 (s, 1H), 7.32-7.34 (m, 2H), 7.29 (dd, J = 8.3, 1.7 Hz, 1H), 6.96 (d, J = 8.1 Hz, 1H), 6.95-6.97 (m, 2H), 4.02 (s, 3H), 3.60 (s, 3H), 3.54 (s, 2H).

### Example 62

### methyl 2-methoxy-4-(11-oxo-10,11-dihydro-5H-dibenzo[b,e][1,4]diazepin-3-yl)benzoate

The desired product was prepared by substituting methyl 4-chloro-2-methoxybenzoate for Example 59B in Example 59C. MS (DCI) m/e 375 (M+H)⁺, 392 (M+NH₄)⁺; ¹H NMR (500 MHz, DMSO-d₆) δ 9.85 (s, 1H), 7.97 (s, 1H), 7.76-7.80 (m, 2H), 7.34-7.36 (m, 2H), 7.28 (d, J = 1.6 Hz, 1H), 7.27 (d, J = 1.6 Hz, 1H), 6.91-7.03 (m, 4H), 3.93 (s, 3H), 3.81 (s, 3H).

### Example 63

### 2-methoxy-4-(11-oxo-10,11-dihydro-5H-dibenzo[b,e][1,4]diazepin-3-yl)benzoic acid

The desired product was prepared by substituting Example 62 for Example 12 in Example 13. MS (DCI) m/e 361 (M+H)⁺, 378 (M+NH₄)⁺; ¹H NMR (500 MHz, DMSO-d₆) δ 9.85 (s, 1H), 7.97 (s, 1H), 7.79 (d, J = 8.1 Hz, 1H), 7.75 (d, J = 7.8 Hz, 1H), 7.24-7.28 (m, 2H), 6.91-7.03 (m, 4H), 3.92 (s, 3H).

### Example 64

### N-(3,4-dihydroxybenzyl)-2-methoxy-4-(11-oxo-10,11-dihydro-5H-dibenzo[b,e][1,4]diazepin-3-yl)benzamide

The desired product was prepared by substituting Example 63 and 4-(aminomethyl)-1,2-benzenediol for Example 13 and 3-pyrrolidin-1-ylpropylamine, respectively, in Example 14. MS (ESI) m/e 483 (M+H)⁺; ¹H NMR (400 MHz, DMSO-d₆) δ 9.84 (s, 1H), 8.79 (s, 1H), 8.54 (t, J = 5.98 Hz, 1H), 7.97 (s, 1H), 7.87 (d, J = 7.98 Hz, 1H), 7.79 (d, J = 7.98 Hz, 1H), 7.35 (d, J = 1.53 Hz, 1H), 7.34 (d, J = 1.23 Hz, 1H), 7.30 (dd, J = 7.98, 1.53 Hz, 1H), 7.27 (dd, J = 8.29, 1.53 Hz, 1H), 6.89-7.03 (m, 4H), 6.75 (d, J = 1.84 Hz, 1H), 6.67 (m, 1H), 6.59 (dd, J = 7.98, 1.84 Hz, 1H), 4.34 (d, J = 5.83 Hz, 2H), 3.99 (s, 3H).

### Example 65

### 2-methoxy-4-(11-oxo-10,11-dihydro-5H-dibenzo[b,e][1,4]diazepin-3-yl)benzamide

The desired product was prepared by substituting 4-chloro-2-methoxybenzamide for Example 59B in Example 59C. MS (DCI) m/e 360 (M+H)⁺, 377 (M+NH₄)⁺; ¹H NMR (500 MHz, DMSO-d₆) δ 9.89 (s, 1H), 8.00 (s, 1H), 7.91 (d, J = 8.1 Hz, 1H), 7.79 (d, J = 8.0 Hz, 1H), 7.70 (s, 1H), 7.60 (d, J = 1.2 Hz, 1H), 7.36 (d, J = 1.8 Hz, 1H), 7.33 (s, 1H), 6.89-7.04 (m, 4H), 4.00 (s, 3H).

### Example 66

### methyl [3-(5-methoxy-2-methyl-4-nitrophenyl)-11-oxo-10,11-dihydro-5H-dibenzo[b,e][1,4]diazepin-8-yl]acetate

### Example 66A

### 1-chloro-5-methoxy-2-methyl-4-nitrobenzene

Acetic anhydride (10 mL) at -10 °C was treated with concentrated nitric acid (>69% pure, 1 mL) and then treated portionwise with 2-chloro-4-methoxy-1-methylbenzene (0.78g, 5 mmol) at a rate that maintained the internal temperature lower than -5 °C. The solution was stirred for additional one hour while warming to room temperature, poured into an ice and water mixture, and extracted with ethyl acetate several times. The organic layers were combined, washed with 10% Na₂CO₃ and brine, dried (MgSO₄), and concentrated under vacuum. The residue was purified by flash column chromatography on silica gel eluting with 9:1 hexanes/ethyl acetate to provide 0.75g (71%) of the desired product. MS (DCI) m/e 219 (M+NH₄)⁺; ¹H NMR (CDCl₃, 300 MHz) δ 7.78 (s, 1H), 7.09 (s, 1H), 3.94 (s, 3H), 2.35 (s, 3H).

### Example 66B

### methyl [3-(5-methoxy-2-methyl-4-nitrophenyl)-11-oxo-10,11-dihydro-5H-dibenzo[b,e][1,4]diazepin-8-yl]acetate

The desired product was prepared by substituting Example 66A and Example 54A for Example 59B and Example 56A, respectively, in Example 59C. MS (DCI) m/e 447 (M+H)⁺; ¹H NMR (500 MHz, DMSO-d₆) δ 9.87 (s, 1H), 7.95 (s, 1H), 7.86 (s, 1H), 7.77 (d, J = 8.0 Hz, 1H), 7.16 (s, 1H), 7.00 (d, J = 1.5 Hz, 1H), 6.92-6.95 (m, 2H), 6.85-6.88 (m, 2H), 3.91 (s, 3H), 3.60 (s, 3H), 3.54 (s, 2H), 2.21 (s, 3H).

### Example 67

### methyl[3-(4-cyano-5-methoxy-2-methylphenyl)-11-oxo-10,11-dihydro-5H-dibenzo[b,e][1,4]diazepin-8-yl]acetate

### Example 67A

### 1-chloro-4-iodo-5-methoxy-2-methylbenzene

The desired product was prepared by substituting 4-chloro-2-methoxy-5-methylaniline for Example 57A in Example 57B. MS (DCI) m/e 283 (M+H)⁺; ¹H NMR (300 MHz, CDCl₃) δ 7.62 (s, 1H), 6.80 (s, 1H), 3.85 (s, 3H), 2.27 (s, 3H).

### Example 67B

### 4-chloro-2-methoxy-5-methylbenzonitrile

The desired product was prepared by substituting Example 67A for Example 59A in Example 60A. MS (DCI) m/e 199 (M+NH₄)⁺; ¹H NMR (300 MHz, CDCl₃) δ 7.40 (s, 1H), 6.98 (s, 1H), 3.91 (s, 3H), 2.31 (s, 3H).

### Example 67C

### methyl [3-(4-cyano-5-methoxy-2-methylphenyl)-11-oxo-10,11-dihydro-5H-dibenzo[b,e][1,4]diazepin-8-yl]acetate

The desired product was prepared by substituting Example 54A and Example 67B for Example 56A and Example 59B, respectively, in Example 59C. MS (DCI) m/e 428 (M+H)⁺; ¹H NMR (500 MHz, DMSO-d₆) δ 9.86 (s, 1H), 7.93 (s, 1H), 7.76 (d, J = 8.1 Hz, 1H), 7.68 (s, 1H), 7.05 (s, 1H), 6.99 (d, J = 1.6 Hz, 1H), 6.96 (d, J = 8.1 Hz, 1H), 6.90-6.94 (m, 2H), 6.85-6.87 (m, 2H), 3.90 (s, 3H), 3.60 (s, 3H), 3.54 (s, 2H), 2.17 (s, 3H).

### Example 68

### 3-(2-methoxy-4-pyridinyl)-5,10-dihydro-11H-dibenzo[b,e][1,4]diazepin-11-one

### Example 68A

### 2-fluoro-3-iodopyridine

A -40 °C solution of diethylamine (12.4g, 125 mmol) in THF (100 mL) was treated dropwise with 1.6 M n-butyllithiumi in hexane (79 mL, 125 mmol), stirred at 0 °C briefly, cooled to -78 °C, treated with a solution of 2-fluororpyridine (9.71 g, 100 mmol) in THF (80 mL), stirred at -78 °C for 2 hours, treated with a solution of iodine (34.48g, 120 mmol) in THF (100 mL), and stirred overnight while gradually warming to room temperature. The reaction mixture was poured into water (1 L) and extracted with diethyl ether several times. The combined extracts were washed with water, Na₂S₂O₃, and brine, dried (MgSO₄), filtered, and concentrated under vacuum. The residue was purified by flash column chromatography on silica gel eluting with 25:1 hexanes/ethyl acetate to provide 15.9g (71%) of the desired product.

### Example 68B

### 2-fluoro-4-iodopyridine

A -40 °C solution of diethylamine (5.51g, 55.5 mmol) in THF (80 mL) was treated dropwise with 2.5 M n-butyllithium in hexane (22.2 mL, 55.5 mmol), stirred at 0 °C briefly, cooled to -78 °C, treated with a solution of Example 68A (9.9g, 44.4 mmol) in THF (80 mL), stirred at -78 °C for 2 hours, treated with water (3.6g, 200 mmol), stirred for 5 minutes, then poured into water (500 mL), and extracted with diethyl ether several times. The combined extracts were washed water and brine, dried (MgSO₄), filtered and concentrated under vacuum. The residue was purified by flash column chromatography on silica gel eluting with 25:1 hexanes/ethyl acetate to provide 9.5g (96%) of the desired product.

### Example 68C

### 3-(2-fluoro-4-pyridinyl)-5,10-dihydro-11H-dibenzo[b,e][1,4]diazepin-11-one

The desired product was prepared by substituting Example 68B and Example 56A for Example 9 and 2-methoxy-4-(4,4,5,5-tetramethyl-1,3,2-dioxaborolan-2-yl)phenol, respectively, in Example 10. MS (DCI) m/e 428 (M+H)⁺; ¹H NMR (500 MHz, DMSO-d₆) δ 9.92 (s, 1H), 8.35 (d, J = 5.3 Hz, 1H), 8.00 (s, 1H), 7.82 (d, J = 8.1 Hz, 1H), 7.60-7.62 (m, 1H), 7.44 (s, 1H), 7.42 (s, 1H), 7.34 (dd, J = 8.1, 1.9 Hz, 1H), 6.91-7.03 (m, 4H).

### Example 68D

### 3-(2-methoxy-4-pyridinyl)-5,10-dihydro-11H-dibenzo[b,e][1,4]diazepin-11-one

A mixture of sodium (14mg, 0.6 mmol) in methanol (5 mL) was treated with Example 68C (46mg, 0.15 mmol), heated to reflux until a homogeneous solution formed, and concentrated. The residue was diluted with water (10 mL), adjusted to pH 5 with 10% HCl, and extracted with ethyl acetate several times. The combined extracts were washed with brine, dried (MgSO₄), filtered, and concentrated under vacuum. The residue was purified by flash column chromatography on silica gel eluting with 3:2 hexanes/ethyl acetate to provide 35mg (74%) of the desired product. MS (DCI) m/e 318 (M+H)⁺; ¹H NMR (500 MHz, DMSO-d₆) δ 9.89 (s, 1H), 8.26 (d, J = 5.5 Hz, 1H), 7.97 (s, 1H), 7.79 (d, J = 8.3 Hz, 1H), 7.38 (d, J = 1.8 Hz, 1H), 7.27 (dd, J = 8.3, 1.7 Hz, 1H), 7.24 (dd, J = 5.5, 1.5 Hz, 1H), 6.90-7.03 (m, 5H).

### Example 69

### 3-(2-methoxy-4-pyridinyl)-11-oxoN[3-(1-pyrrolidinyl)propyl]-10,11-dihydro-5H-dibenzo[b,e][1,4]diazepine-8-carboxamide

### Example 69A

### 2-fluoro-4-(4,4,5,5-tetramethyl-1,3,2-dioxaborolan-2-yl)pyridine

A -78 °C solution of Example 68B (3.35g, 15 mmol) diethyl ether (100 mL) was treated dropwise with 2.5 M n-butyllithium (7.2 mL, 18 mmol), stirred for 2 hours at -78 °C, treated with tributyl borate (4.14g, 18 mmol), stirred at -78 °C for one hour and warmed to room temperature over 2 hours. The solution was treated with pinacol (2.30g, 19.5 mmol) and acetic acid (0.9g, 15 mmol), stirred overnight, and filtered through diatomaceous earth (Celite^{®}). The pad was washed with diethyl ether several times and the filtrate was concentrated to a volume of 50 mL. The mixture was diluted with ethyl acetate, washed with water and brine, dried (MgSO₄), filtered, and concentrated under vacuum. The residue was purified by flash column chromatography on silica gel eluting with 25:1 hexanes/ethyl acetate to provide 2.34g (70%) of the desired product. MS (DCI) m/e 224 (M+H)⁺; ¹H NMR (300 MHz, CDCl₃,) δ 8.24 (d, J = 5.1 Hz, 1H), 7.50 (dd, J = 4.8, 2.7 Hz, 1H), 7.23 (d, J = 2.4 Hz, 1H), 1.36 (s, 12H).

### Example 69B

### methyl 3-(2-fluoro-4-pyridinyl)-11-oxo-10,11-dihydro-5H-dibenzo[b,e][1,4]diazepine-8-carboxylate

The desired product was prepared by substituting Example 69A and Example 1B for Example 56A and Example 59B, respectively, in Example 59C. MS (DCI) m/e 364 (M+H)⁺, 381 (M+NH₄)⁺; ¹H NMR (500 MHz, DMSO-d₆) δ 10.05 (s, 1H), 8.48 (s, 1H), 8.36 (d, J = 5.3 Hz, 1H), 7.85 (d, J = 8.1 Hz, 1H), 7.62 (m, 2H), 7.57 (dd, J = 8.3, 2.0 Hz, 1H), 7.45 (s, 1H), 7.24 (t, J = 2.3 Hz, 1H), 7.37 (dd, J = 8.1, 1.9 Hz, 1H), 7.08 (d, J = 8.4 Hz, 1H), 3.61 (s, 3H).

### Example 69C

### 3-(2-fluoro-4-pyridinyl)-11-oxo-10,11-dihydro-5H-dibenzo[b,e][1,4]diazepine-8-carboxylic acid

A mixture of Example 69B (60mg, 0.17 mmol) and LiOH (20mg, 0.85 mmol) in water (5 mL) and THF (5 mL) was heated to reflux until a homogeneous solution formed, adjusted to pH 5 with 10 % HCl, and extracted with ethyl acetate several times. The combined extracts were washed with brine, dried (CaCl₂), filtered, and concentrated under vacuum to provide 47mg (80%) of the desired product. MS (DCI) m/e 349 (M)⁺; ¹H NMR (500 MHz, DMSO-d₆) δ 10.03 (s, 1H), 8.42 (s, 1H), 8.36 (d, J = 5.3 Hz, 1H), 7.85 (d, J = 8.4 Hz, 1H), 7.62 (d, J = 5.3 Hz, 1H), 7.60 (d, J = 1.6 Hz, 1H), 7.55 (dd, J = 8.4, 1.9 Hz, 1H), 7.45 (s, 1H), 7.24 (d, J = 1.6 Hz, 1H), 7.37 (dd, J = 8.1, 1.9 Hz, 1H), 7.05 (d, J = 8.4 Hz, 1H).

### Example 69D

### 3-(2-fluoro-4-pyridinyl)-11-oxoN[3-(1-pyrrolidinyl)propyl]-10,11-dihydro-5H-dibenzo[b,e] [1,4]diazepine-8-carboxamide

The desired product was prepared by substituting Example 69C for Example 13 in Example 14. MS (DCI) m/e 460 (M+H)⁺; ¹H NMR (500 MHz, DMSO-d₆, TFA salt) δ 10.01 (s, 1H), 9.47 (s, 1H), 8.41 (t, J = 5.6 Hz, 1H), 8.36 (d, J = 5.3 Hz, 1H), 8.33 (s, 1H), 7.83 (d, J = 7.3 Hz, 1H), 7.62 (d, J = 5.3 Hz, 1H), 7.42-7.50 (m, 4H), 7.37 (d, J = 8.3, 1.7 Hz, 1H), 7.04 (d, J = 8.4 Hz, 1H), 3.53-3.56 (m, 2H), 3.28-3.31 (m, 2H), 3.14-3.17 (m, 2H), 2.96-3.00 (m, 2H), 1.99-2.02 (m, 2H), 1.84-1.88 (m, 4H).

### Example 69E

### 3-(2-methoxy-4-pyridinyl)-11-oxoN[3-(1-pyrrolidinyl)propyl]-10,11-dihydro-5H-dibenzo[b,e][1,4]diazepine-8-carboxamide

The desired product was prepared by substituting Example 69D for Example 68C in Example 68D. MS (DCI) m/e 472 (M+H)⁺; ¹H NMR (500 MHz, DMSO-d₆) δ 9.95 (s, 1H), 8.33 (t, J = 5.5 Hz, 1H), 8.25-8.27 (m, 2H), 7.80 (d, J = 8.1 Hz, 1H), 7.47 (d, J = 1.9 Hz, 1H), 7.42 (dd, J = 8.3, 2.0 Hz, 1H), 7.37 (d, J = 1.9 Hz, 1H), 7.29 (dd, J = 8.3, 1.7 Hz, 1H), 7.24 (dd, J = 5.3, 1.6 Hz, 1H), 7.01-7.04 (m, 2H), 3.91 (s, 3H), 3.24-3.28 (m, 2H), 2.41-2.44 (m, 6H), 1.65-1.68 (m, 6H).

### Example 70

### 11-oxo-3-(2-oxo-1,2-dihydro-4-pyridinyl)N[3-(1-pyrrolidinyl)propyl]-10,11-dihydro-5H-dibenzo[b,e][1,4]diazepine-8-carboxamide

A solution of Example 69D (TFA salt, 150mg) in acetic acid (25 mL) and water (5 mL) was heated to 100 °C for 16 hours, cooled to room temperature, and concentrated under vacuum. The residue was purified by preparative HPLC to provide 120mg of the desired product as the TFA salt. MS (DCI) m/e 458 (M+H)⁺; ¹H NMR (500 MHz, DMSO-d₆) δ 11.12 (br s, 1H), 9.98 (s, 1H), 9.56 (s, 1H), 8.42 (s, 1H), 8.28 (d, J = 2.8 Hz, 1H), 7.79 (dd, J = 8.1, 2.9 Hz, 1H), 7.45-7.50 (m, 3H), 7.31 (s, 1H), 7.21 (dd, J = 8.3, 1.9 Hz, 1H), 7.04 (dd, J = 8.3, 2.8 Hz, 1H), 6.54 (s, 1H), 6.41-6.43 (m, 1H), 3.54-3.58 (m, 2H), 3.30-3.32 (m, 2H), 3.15-3.17 (m, 2H), 2.97-3.04 (m, 2H), 2.01 (m, 2H), 1.86-1.87 (m, 4H).

### Example 71

### methyl [3-(2-fluoro-5-methyl-4-pyridinyl)-11-oxo-10,11-dihydro-5H-dibenzo[b,e][1,4]diazepin-8-yl]acetate

### Example 71A

### 2-fluoro-3-iodo-5-methylpyridine

The desired product was prepared by substituting 2-fluoro-5-methylpyridine for 2-fluoropyridine in Example 68A. MS (DCI) m/e 238 (M+H)⁺.

### Example 71B

### 2-fluoro-4-iodo-5-methylpyridine

The desired product was prepared by substituting Example 71A for Example 69A in Example 69B. MS (DCI) m/e 238 (M+H)⁺.

### Example 71C

### 2-fluoro-5-methyl-4-(4,4,5,5-tetramethyl-1,3,2-dioxaborolan-2-yl)pyridine

The desired product was prepared by substituting Example 71B for Example 68B in Example 69A. MS (DCI) m/e 237 (M)⁺; ¹H NMR (300 MHz, CDCl₃) 8 8.00 (s, 1H), 7.22 (d, J = 2.2 Hz, 1H), 2.44 (s, 3H), 1.36 (s, 12H).

### Example 71D

### methyl [3-(2-fluoro-5-methyl-4-pyridinyl)-11-oxo-10,11-dihydro-5H-dibenzo[b,e][1,4]diazepin-8-yl]acetate

The desired product was prepared by substituting Example 71C and Example 6D for Example 56A and Example 59B, respectively, in Example 59C. MS (DCI) m/e 392 (M+H)⁺, 409 (M+NH₄)⁺; ¹H NMR (500 MHz, DMSO-d₆) δ 9.90 (s, 1H), 8.18 (s, 1H), 7.96 (s, 1H), 7.78 (d, J = 8.0 Hz, 1H), 7.08 (d, J = 1.8 Hz, 1H), 7.03 (d, J = 1.2 Hz, 1H), 6.92-6.97 (m, 2H), 6.85-6.88 (m, 2H), 3.60 (s, 3H), 3.54 (s, 2H), 2.22 (s, 3H).

### Example 72

### methyl [3-(2-methoxy-5-methyl-4-pyridinyl)-11-oxo-10,11-dihydro-5H-dibenzo[b,e][1.4]diazepin-8-yl]acetate

A mixture of sodium (18mug, 0.8 mmol) in methanol (5 mL) was treated with Example 71D (30mg, 0.077 mmol), heated to reflux for 24 hours, and concentrated under vacuum. The residue was diluted with water (10 mL), adjusted to pH 5 with 10% HCl, and extracted with ethyl acetate several times. The combined extracts were washed with brine, dried (MgS0₄), filtered, and concentrated under vacuum. The residue was treated with excess 2.0M TMSCHN₂ in hexanes and concentrated to provide the desired product. MS (DCI) m/e 404 (M+H)⁺; ¹H NMR (500 MHz, DMSO-d₆) δ 9.86 (s, 1H), 8.08 (d, J = 5.5 Hz, 1H), 7.92 (s, 1H), 7.74 (d, J = 8.0 Hz, 1H), 6.98 (s, 1H), 6.84-6.93 (m, 4H), 6.64 (s, 1H), 3.84 (s, 3H), 3.59 (s, 3H), 3.53 (s, 2H), 2.13 (s, 3H).

### Example 73

### [3-(3-methoxy-4-nitropheny)-11-oxo-10,11-dihydro-5H-dibenzo[b.e][1,4]diazepin-8-yl]acetic acid

The desired product was prepared by substituting Example 51 for Example 12 in Example 13. MS (ESI) m/e 418 (M-H)⁻; ¹H NMR (300 MHz, DMSO-d₆) δ 12.28 (s, 1H), 9.90 (s, 1H), 8.00-8.03 (m, 2H), 7.80 (d, J = 8.1 Hz, 1H), 7.52 (d, J = 1.7 Hz, 1H), 7.29-7.36 (m, 3H), 6.96 (m, 1H), 6.84-6.87 (m, 2H), 4.03 (s, 3H), 3.43 (s, 2H).

### Example 74

### 2-[3-(3-methoxy-4-nitrophenyl)-11-oxo-10,11-dihydro-5H-dibenzo[b,e][1,4]diazepin-8-yl]N[3-(1-pyrrolidinyl)propyl]acetamide

The desired product was prepared by substituting Example 73 for Example 13 in Example 14. MS (ESI) m/e 528 (M-H)⁻; ¹H NMR (400 MHz, DMSO-d₆) δ 9.93 (s, 1H), 8.12 (t, 1H), 8.02 (d, 1H), 8.00 (s, 1H), 7.80 (d, 1H), 7.52 (d, 1H), 7.35 (t, 1H), 7.33 (t, 1H), 7.30 (d, 1H), 6.95 (d, 1H), 6.86-6.88 (m, 2H), 4.03 (s, 3H), 3.48 (b, 2H), 3.29 (s, 2h), 3.07-3.12 (q, 2H), 3.00-3.06 (m, 2H), 2.86-2.95 (m, 2H), 1.91-1.99 (m, 2H), 1.70-1.84 (m, 4H).

### Example 75

### N-[2-(dimethylamino)ethyl]-2-[3-(3-methoxy-4-nitrophenyl)-11-oxo10,11-dihydro-5H-dibenzo[b,e][1,4]diazepin-8-yl]Nmethylacetamide

The desired product was prepared by substituting Example 73 and N-[2-(dimethylamino)ethyl]Nmethylamine for Example 13 and 3-pyrrolidin-1-ylpropylamine, respectively, in Example 14. MS (ESI) m/e 502 (M-H)⁻; ¹H NMR (500 MHz, DMSO-d₆) δ 9.86 (s, 1H), 8.01 (d, J = 8.11 Hz, 2H), 7.95 (s, 1H), 7.79 (d, J = 8.11 Hz, 1H), 7.52 (d, J = 1.56 Hz, 1H), 7.33-7.35 (m, 2H), 7.30 (dd, J = 8.11, 1.56 Hz, 1H), 6.95 (d, J = 8.11 Hz, 1H), 6.84 (d, J = 2.18 Hz, 1H), 6.82 (d, J = 8.11 Hz, 1H), 4.03 (s, 3H), 3.56 (s, 1H), 3.54 (s, 1H), 3.35 (t, J = 6.86 Hz, 2H), 2.96 (s, 2H), 2.81 (s, 1H), 2.32 (t, J = 6.86 Hz, 2H), 2.14 (d, J = 2.50 Hz, 6H).

### Example 76

### 8-[2-(3-hydroxy-1-piperidinyl)-2-oxoethyl]-3-(3-methoxy-4-nitrophenyl)-5,10-dihydro-11H-dibenzo[b,e][1,4]diazepin-11-one

The desired product was prepared by substituting Example 73 and 3-piperidinol for Example 13 and 3-pyrrolidin-1-ylpropylamine, respectively, in Example 14. MS (ESI) m/e 501 (M-H)⁻; ¹H NMR (500 MHz, DMSO-d₆) 8 9.87 (d, J = 2.81 Hz, 1H), 8.01 (d, J = 8.42 Hz, 2H), 7.96 (s, 1H), 7.80 (d, J = 8.42 Hz, 1H), 7.52 (d, J = 1.56 Hz, 1H), 7.33-7.35 (m, 2H), 7.29 (dd, J = 8.11, 1.56 Hz, 1H), 6.95 (d, J = 7.80 Hz, 1H), 6.80-6.85 (m, 2H), 4.79-4.84 (m, 2H), 4.03 (s, 3H), 3.42 (m, 4H), 2.93-3.07 (m, 2H), 1.80 (m, 1H), 1.62 (m, 1H), 1.39 (m, 1H), 1.23 (m, 1H).

### Example 77

### 3-(3-methoxy-4-nitrophenyl)-8-[2-(4-methyl-1,4-diazepan-1-yl)-2-oxoethyl]-5,10-dihydro-11H-dibenzo[b,e][1,4]diazepin-11-one

The desired product was prepared by substituting Example 73 and 1-methyl-1,4-diazepane for Example 13 and 3-pyrrolidin-1-ylpropylamine, respectively, in Example 14. MS (ESI) m/e 514 (M-H)⁻; ¹H NMR (500 MHz, DMSO-d₆) δ 9.89 (s, 1H), 8.01 (d, J = 8.14 Hz, 1H), 7.98 (s, 1H), 7.79 (d, J = 8.14 Hz, 1H), 7.52 (d, J = 1.36 Hz, 1H), 7.28-7.36 (m, 3H), 6.95 (d, J = 8.14 Hz, 1H), 6.81-6.85 (m, 2H), 4.03 (s, 3H), 3.56 (s, 2H), 3.38-3.53 (m, 6H), 2.54 (m, 1H), 2.47 (m, 1H), 2.25 (d, J = 4.41 Hz, 3H); 1.71-1.80 (m, 2H).

### Example 78

### 2-[3-(3-methoxy-4-nitrophenyl)-11-oxo-10,11-dihydro-5H-dibenzo[b,e][1,4]diazepin-8-yl]-N,N-dimethylacetamide

The desired product was prepared by substituting Example 73 and N,N-dimethylamine for Example 13 and 3-pyrrolidin-1-ylpropylamine, respectively, in Example 14. MS (ESI) m/e 445 (M-H)⁻; ¹H NMR (300 MHz, DMSO-d₆) δ 9.88 (s, 1H), 8.01 (d, J = 8.48 Hz, 1H), 7.97 (s, 1H), 7.79 (d, J = 8.14 Hz, 1H), 7.52 (d, J = 1.70 Hz, 1H), 7.32-7.36 (m, 2H), 7.28-7.32 (dd, J = 8.14, 1.70 Hz, 1H) 6.95 (d, J = 7.80 Hz, 1H), 6.80-6.85 (m, 2H), 4.03 (s, 3H), 3.55 (s, 2H), 2.97 (s, 3H), 2.81 (s, 3H).

### Example 79

### 8-[2-(4-hydroxy-1-piperidinyl)-2-oxoethyl]-3-(3-methoxy-4-nitrophenyl)-5,10-dihydro-11H-dibenzo[b,e][1,4]diazepin-11-one

The desired product was prepared by substituting Example 73 and 4-piperidinol for Example 13 and 3-pyrrolidin-1-ylpropylamine, respectively, in Example 14. MS (ESI) m/e 501 (M-H)⁻; ¹H NMR (500 MHz, DMSO-d₆) δ 9.87 (s, 1H), 8.01 (d, J = 8.42 Hz, 1H), 7.96 (s, 1H), 7.80 (d, J = 8.11 Hz, 1H), 7.52 (d, J = 1.56 Hz, 1H), 7.34-7.35 (m, 2H), 7.29 (dd, J = 8.27, 1.72 Hz, 1H), 6.94 (d, J = 7.80 Hz, 1H), 6.81-6.84 (m, 2H), 4.67 (s, 1H), 4.03 (s, 3H), 3.88-3.91 (m, 1H), 3.63-3.69 (m, 2H), 3.56 (s, 2H), 3.10-3.15 (m, 1H), 2.96-3.01 (m, 1H), 1.61-1.67 (m, 2H), 1.15-1.24 (m, 2H).
Example 80 to Example 118 were prepared using the following procedure:
Syntheses were performed using a PE Biosystems (Applied Biosystems) Solaris 530 organic synthesizer. Each of the round bottom flasks was charged with 81mg of polymer-supported (PS)-DCC resin (loading 1.24 mmol/g) supplied by Argonaut Technologies. The reaction block was then assembled and placed on the Solaris 530. The amine monomers (0.6 mmol) were each dissolved in 3 mL of DMA. Example 73 (MW 419.11) was dissolved in 40 mL of DMA (747mg, 1.78 mmol). Solutions of HOBt (409g, 3.0 mmol) in 68 mL of DMA and DIEA (1.580 mL in 68 mL DMA) were placed on the instrument. The Solaris was primed with DMA then into each of the 48 vials containing PS-DCC resin was added 0.75 mL of the Example 73 solution (0.033 mmol) followed by 0.75 mL of HOBt solution (1 equivalent), 0.209 mL of each amine solution (1.25 equivalents) and 0.75 mL of DIEA solution (3 equivalents). The reactions were heated to 55 °C overnight and transferred with methanol to 20 mL vials containing 39mg MP-Carbonate resin (2.55mmol/g, 3 equivalents). The MP-Carbonate resin was filtered and the reactions were concentrated to dryness. The residues were dissolved in 1:1
DMSO/methanol and purified by reverse phase HPLC using 10:100 acetonitrile/ 0.1% aqueous TFA.

### Example 80

### 2-[3-(3-methoxy-4-nitrophenyl)-11-oxo-10,11-dihydro-5H-dibenzo[b,e][1,4]diazepin-8-yl]N[2-(2-pyridinyl)ethyl]acetamide

The desired product was prepared using 2-(2-pyridinyl)ethylamine. MS (ESI) 524 (M+H)⁺; ¹H NMR (DMSO-d₆) δ 9.86 (s, 1H), 8.61 (d, J = 4.7 Hz, 1H), 8.02 (m, 3H), 7.95 (s, 1H), 7.81 (d, J = 8.1 Hz, 1H), 7.51 (m, 3H), 7.34 (m, 2H), 7.30 (dd, J = 8.3,1.7 Hz, 1H), 6.92 (d, J = 8.1 Hz, 1H), 6.80 (m, 2H), 4.03 (s, 3H), 3.43 (m, 2H), 3.23 (s, 2H), 2.98 (m, 2H).

### Example 812-[3-(3-methoxy-4-nitrophenyl)-11-oxo-10,11-dihydro-5H-dibenzo[b,e][1,4]diazepin-8-yl]N[2-(3-pyridinyl)ethyl]acetamide

The desired product was prepared using 2-(3-pyridinyl)ethylamine. MS (ESI) 524 (M+H)⁺; ¹H NMR (DMSO-d₆) δ 9.86 (s, 1H), 8.63 (s, 1H), 8.58 (d, J = 5.0 Hz, 1H), 8.03 (m, 3H), 7.95 (s, 1H), 7.81 (d, J = 8.1 Hz, 1H), 7.62 (m, 1H), 7.52 (s, 1H), 7.34 (m, 2H), 7.30 (dd, J = 8.3, 1.7 Hz, 1H), 6.93 (d, J = 8.1 Hz, 1H), 6.80 (m, 2H), 4.03 (s, 3H), 3.34 (m, 2H), 3.24 (s, 2H), 2.83 (m, 2H).

### Example 82

### 2-[3-(3-Methoxy-4-nitro-phenyl)-11-oxo-10,11-dihydro-5H-dibenzo[b,e][1,4]diazepin-8-yl]N(4-pyridin-2-yl-ethyl)-acetamide

The desired product was prepared using 2-(4-pyridinyl)ethylamine. MS (ESI) 524 (M+H)⁺; ¹H NMR (DMSO-d₆) δ 9.87 (s, 1H), 8.65 (d, J = 5.3 Hz, 2H), 8.05 (br t, J = 5.6 Hz, 1H), 8.01 (d, J = 8.4 Hz, 1H), 7.95 (s, 1H), 7.81 (d, J = 8.4 Hz, 1H), 7.64 (m, 2H), 7.52 (s, 1H), 7.34 (m, 2H), 7.30 (dd, J = 8.1, 1.9 Hz, 1H), 6.93 (d, J = 8.1 Hz, 1H), 6.80 (m, 2H), 4.03 (s, 3H), 3.34 (m, 2H), 3.24 (s, 2H), 2.83 (m, 2H).

### Example 83

### 2-[3-(3-methoxy-4-nitrophenyl)-11-oxo-10,11-dihydro-5H-dibenzo[b,e][1,4]diazepin-8-yl]N3-quinolinylacetamide

The desired product was prepared using 3-quinolinamine. MS (ESI) 546 (M+H)⁺; ¹H NMR (DMSO-d₆) δ 10.60 (s, 1H), 9.91 (s, 1H), 8.93 (d, J = 2.5 Hz, 1H), 8.68 (d, J = 2.2 Hz, 1H), 8.01 (m, 2H), 7.96 (d, J = 8.1 Hz, 1H), 7.90 (d, J = 7.8 Hz, 1H), 7.80 (d, J = 8.1 Hz, 1H), 7.64 (m, 1H), 7.57 (m, 1H), 7.53 (d, J = 1.6 Hz, 1H), 7.35 (m, 2H), 7.30 (dd, J = 8.1, 1.6 Hz, 1H), 6.99 (m, 3H), 4.03 (s, 3H), 3.62 (s, 2H).

### Example 84

### 2-[3-(3-methoxy-4-nitrophenyl)-11-oxo-10,11-dihydro-5H-dibenzo[b,e][1,4]diazepin-8-yl]N6-quinolinylacetamide

The desired product was prepared using 6-quinolinamine. MS (ESI) 544 (M-H)⁻; ¹H NMR (DMSO-d₆) δ 10.53 (s, 1H), 9.92 (s, 1H), 8.87 (d, J = 3.1 Hz, 1H), 8.45 (m, 2H), 8.02 (m, 3H), 7.89 (dd, J = 9.4, 2.2 Hz, 1H), 7.80 (d, J = 8.1 Hz, 1H), 7.61 (dd, J = 8.4, 4.4 Hz, 1H), 7.52 (d, J = 1.6 Hz, 1H), 7.35 (m, 2H), 7.30 (dd, J = 8.3, 1.7 Hz, 1H), 6.98 (m, 3H), 4.03 (s, 3H), 3.61 (s, 2H).

### Example 85

### 2-[3-(3-methoxy-4-nitrophenyl)-11-oxo-10,11-dihydro-5H-dibenzo[b,e][1,4]diazepin-8-yl]N[4-(4-morpholinyl)phenyl]acetamide

The desired product was prepared using 4-(4-morpholinyl)aniline. MS (ESI) 580 (M+H)⁺; ¹H NMR (DMSO-d₆) δ 9.90 (s, 1H), 9.87 (s, 1H), 8.01 (d, J = 8.4 Hz, 1H), 7.96 (s, 1H), 7.80 (d, J = 8.1 Hz, 1H), 7.52 (d, J = 1.2 Hz, 1H), 7.44 (d, J = 9.0 Hz, 2H), 7.35 (m, 2H), 7.30 (dd, J = 8.3, 1.7 Hz, 1H), 6.95 (m, 3H), 6.88 (d, J = 9.0 Hz, 2H), 4.03 (s, 3H), 3.72 (m, 4H), 3.48 (s, 2H), 3.03 (m, 4H).

### Example 86

### N-[(1S)-1-(hydroxymethyl)-2-methylpropyl]-2-[3-(3-methoxy-4-nitrophenyl)-11-oxo-10,11-dihydro-5H-dibenzo[b,e][1,4]diazepin-8-yl]acetamide

The desired product was prepared using (2S)-2-amino-3-methyl-1-butanol. MS (ESI) 505 (M+H)⁺; ¹H NMR (DMSO-d₆) δ 9.88 (s, 1H), 8.01 (d, J = 8.4 Hz, 1H), 7.93 (s, 1H), 7.81 (d, J = 8.1 Hz, 1H), 7.60 (d, J = 8.7 Hz, 1H), 7.52 (d, J = 1.2 Hz, 1H), 7.34 (m, 2H), 7.30 (dd, J = 8.3, 1.7 Hz, 1H), 6.93 (m, 1H), 6.87 (m, 2H), 4.50 (br s, 1H), 4.03 (s, 3H), 3.53 (m, 1H), 3.35 (m, 2H), 3.25 (s, 2H), 1.81 (m, 1H), 0.83 (d, J = 7.5 Hz, 3H), 0.79 (d, J = 7.5 Hz, 3H).

### Example 87

### N-[(1R)-1-(hydroxymethyl)-2-methylpropyl]-2-[3-(3-methoxy-4-nitrophenyl)-11-oxo-10,11-dihydro-5H-dibenzo[b,e][1,4]diazepin-8-yl]acetamide

The desired product was prepared using (2R)-2-amino-3-methyl-1-butanol. MS (ESI) 505 (M+H)⁺; ¹H NMR (DMSO-d₆) δ 9.88 (s, 1H), 8.01 (d, J = 8.4 Hz, 1H), 7.93 (s, 1H), 7.81 (d, J = 8.1 Hz, 1H), 7.60 (d, J = 8.7 Hz, 1H), 7.52 (d, J = 1.2 Hz, 1H), 7.34 (m, 2H), 7.30 (dd, J = 8.3, 1.7 Hz, 1H), 6.93 (m, 1H), 6.87 (m, 2H), 4.50 (br s, 1H), 4.03 (s, 3H), 3.53 (m, 1H), 3.35 (m, 2H), 3.25 (s, 2H), 1.81 (m, 1H), 0.83 (d, J = 7.5 Hz, 3H), 0.79 (d, J = 7.5 Hz, 3H).

### Example 88

### N-(3-ethoxypropyl)-2-[3-(3-methoxy-4-nitrophenyl)-11-oxo-10,11-dihydro-5H-dibenzo[b,e][1,4]diazepin-8-yl]acetamide

The desired product was prepared using 3-ethoxypropylamine. MS (ESI) 505 (M+H)⁺; ¹H NMR (DMSO-d₆) δ 9.88 (s, 1H), 8.01 (d, J = 8.4 Hz, 1H), 7.93 (s, 1H), 7.88 (br t, J = 5.3 Hz, 1H), 7.80 (d, J = 8.1 Hz, 1H), 7.52 (d, J = 1.6 Hz, 1H), 7.34 (m, 2H), 7.30 (dd, J = 8.3, 1.7 Hz, 1H), 6.93 (d, J = 8.1 Hz, 1H), 6.85 (m, 2H), 4.03 (s, 3H), 3.35 (m, 4H), 3.25 (s, 2H), 3.01 (q, J = 7.5 Hz, 2H), 1.60 (m, 2H), 1.06 (t, J = 7.5 Hz, 3H).

### Example 89

### N-[2-(diethylamino)ethyl]-2-[3-(3-methoxy-4-nitrophenyl)-11-oxo-10,11-dihydro-5H-dibenzo[b,e][1,4]diazepin-8-yl]acetamide

The desired product was prepared using N-(2-aminoethyl)-N,N-diethylamine. MS (ESI) 518 (M+H)⁺; ¹H NMR (DMSO-d₆) δ 9.88 (s, 1H), 9.12 (v br s, 1H), 8.26 (br t, J = 5.6 Hz, 1H), 8.01 (d, J = 8.4 Hz, 1H), 7.98 (s, 1H), 7.80 (d, J = 8.1 Hz, 1H), 7.52 (d, J = 1.6 Hz, 1H), 7.34 (m, 2H), 7.30 (dd, J = 8.3, 1.7 Hz, 1H), 6.95 (d, J = 8.1 Hz, 1H), 6.87 (m, 2H), 4.03 (s, 3H), 3.32 (s, 2H), 3.10 (m, 6H), 1.15 (t, J = 7.3 Hz, 6H).

### Example 90

### N-[(1R)-1-(hydroxymethyl)-3-methylbutyl]-2-[3-(3-methoxy-4-nitrophenyl)-11-oxo-10,11-dihydro-5H-dibenzo[b,e][1,4]diazepin-8-yl]acetamide

The desired product was prepared using (2R)-2-amino-4-methyl-1-pentanol. MS (ESI) 519 (M+H)⁺; ¹H NMR (DMSO-d₆) δ 9.88 (s, 1H), 8.01 (d, J = 8.4 Hz, 1H), 7.93 (s, 1H), 7.81 (d, J = 8.1 Hz, 1H), 7.63 (d, J = 8.4 Hz, 1H), 7.52 (d, J = 1.6 Hz, 1H), 7.34 (m, 2H), 7.30 (dd, J = 8.3, 1.7 Hz, 1H), 6.93 (d, J = 8.1 Hz 1H), 6.87 (m, 2H), 4.57 (br s, 1H), 4.03 (s, 3H), 3.74 (m, 1H), 3.22 (m, 2H), 3.26 (s, 2H), 1.55 (m, 1H), 1.27 (m, 2), 0.84 (d, J = 6.9 Hz, 3H), 0.79 (d, J = 6.9 Hz, 3H).

### Example 91

### N-[(1S)-1-(hydroxymethyl)-3-methylbutyl]-2-[3-(3-methoxy-4-nitrophenyl)-11-oxo-10,11-dihydro-5H-dibenzo[b,e][1,4]diazepin-8-yl]acetamide

The desired product was prepared using (2S)-2-amino-4-methyl-1-pentanol. MS (ESI) 519 (M+H)⁺; ¹H NMR (DMSO-d₆) δ 9.88 (s, 1H), 8.01 (d, J = 8.4 Hz, 1H), 7.93 (s, 1H), 7.81 (d, J = 8.1 Hz, 1H), 7.63 (d, J = 8.4 Hz, 1H), 7.52 (d, J = 1.6 Hz, 1H), 7.34 (m, 2H), 7.30 (dd, J = 8.3, 1.7 Hz, 1H), 6.93 (d, J = 8.1 Hz 1H), 6.87 (m, 2H), 4.57 (br s, 1H), 4.03 (s, 3H), 3.74 (m, 1H), 3.22 (m, 2H), 3.26 (s, 2H), 1.55 (m, 1H), 1.27 (m, 2), 0.84 (d, J = 6.9 Hz, 3H), 0.79 (d, J = 6.9 Hz, 3H).

### Example 92

### N-[3-(1H-imidazol-1-yl)propyl]-2-[3-(3-methoxy-4-nitrophenyl)-11-oxo-10,11-dihydro-5H-dibenzo[b,e][1,4]diazepin-8-yl]acetamide

The desired product was prepared using 3-(1H-imidazol-1-yl)propylamine. MS (ESI) 527 (M+H)⁺; ¹H NMR (DMSO-d₆) δ 14.35 (v br s, 1H), 9.88 (s, 1H), 9.05 (s, 1H), 8.07 (br t, J = 5.8 Hz, 1H), 8.01 (d, J = 8.4 Hz, 1H), 7.96 (s, 1H), 7.80 (d, J = 8.1 Hz, 1H), 7.75 (s, 1H), 7.67 (s, 1H), 7.52 (d, J = 1.6 Hz, 1H), 7.34 (m, 2H), 7.30 (dd, J = 8.3, 1.7 Hz, 1H), 6.93 (d, J = 7.8 Hz, 1H), 6.85 (m, 2H), 4.17 (m, 2H), 4.03 (s, 3H), 3.29 (s, 2H), 3.03 (m, 2H), 1.94 (m, 2H).

### Example 93

### N-(3-hydroxypropyl)-2-[3-(3-methoxy-4-nitrophenyl)-11-oxo-10,11-dihydro-5H-dibenzo[b,e][1,4]diazepin-8-yl]acetamide

The desired product was prepared using 3-amino-1-propanol. MS (ESI) 477 (M+H)⁺; ¹H NMR (DMSO-d₆) δ 9.88 (s, 1H), 8.01 (d, J = 8.4 Hz, 1H), 7.94 (s, 1H), 7.90 (br t, J = 5.5 Hz, 1H), 7.80 (d, J = 8.4 Hz, 1H), 7.52 (d, J = 1.6 Hz, 1H), 7.34 (m, 2H), 7.30 (dd, J = 8.3, 1.7 Hz, 1H), 6.93 (d, J = 7.8 Hz, 1H), 6.85 (m, 2H), 4.37 (br s, 1H), 4.03 (s, 3H), 3.39 (m, 2H), 3.25 (s, 2H), 3.08 (m, 2H), 1.54 (m, 2H).

### Example 94

### N-[3-(diethylamino)propyl]-2-[3-(3-methoxy-4-nitrophenyl)-11-oxo-10,11-dihydro-5H-dibenzo[b,e][1,4]diazepin-8-yl]acetamide

The desired product was prepared using N-(3-aminopropyl)-N,N-diethylamine. MS (ESI) 532 (M+H)⁺; ¹H NMR (DMSO-d₆) δ 9.88 (s, 1H), 9.00 (v br s, 1H), 8.12 (br t, J = 5.8 Hz, 1H), 8.01 (d, J = 8.4 Hz, 1H), 7.96 (s, 1H), 7.80 (d, J = 8.1 Hz, 1H), 7.52 (d, J = 1.6 Hz, 1H), 7.34 (m, 2H), 7.30 (dd, J = 8.3, 1.7 Hz, 1H), 6.95 (d, J = 7.8 Hz, 1H), 6.87 (m, 2H), 4.03 (s, 3H), 3.28 (s, 2H), 3.12 (m, 2H), 3.05 (m, 4H), 2.95 (m, 2H), 1.72 (m, 2H), 1.10 (t, J = 7.3 Hz, 6H).

### Example 95

### N-[(1S)-2-hydroxy-1-phenylethyl]-2-[3-(3-methoxy-4-nitrophenyl)-11-oxo-10,11-dihydro-5H-dibenzo[b,e][1,4]diazepin-8-yl]acetamide

The desired product was prepared using (2S)-2-amino-2-phenylethanol. MS (ESI) 539 (M+H)⁺; ¹H NMR (DMSO-d₆) δ 9.87 (s, 1H), 8.35 (d, J = 8.1 Hz, 1H), 8.01 (d, J = 8.4 Hz, 1H), 7.94 (s, 1H), 7.80 (d, J = 8.1 Hz, 1H), 7.52 (d, J = 1.2 Hz, 1H), 7.34 (m, 2H), 7.27 (m, 5H), 7.20 (m, 1H), 6.93 (d, J = 7.8 Hz, 1H), 6.87 (m, 2H), 4.80 (m, 2H), 4.03 (s, 3H), 3.55 (d, J = 6.2 Hz, 2H), 3.38 (s, 2H).

### Example 96

### N-[(1R)-2-hydroxy-1-phenylethyl]-2-[3-(3-methoxy-4-nitrophenyl)-11-oxo-10,11-dihydro-5H-dibenzo[b,e][1,4]diazepin-8-yl]acetamide

The desired product was prepared using (2R)-2-amino-2-phenylethanol. MS (ESI) 539 (M+H)⁺; ¹H NMR (DMSO-d₆) δ 9.87 (s, 1H), 8.35 (d, J = 8.1 Hz, 1H), 8.01 (d, J = 8.4 Hz, 1H), 7.94 (s, 1H), 7.80 (d, J = 8.1 Hz, 1H), 7.52 (d, J = 1.2 Hz, 1H), 7.34 (m, 2H), 7.27 (m, 5H), 7.20 (m, 1H), 6.93 (d, J = 7.8 Hz, 1H), 6.87 (m, 2H), 4.80 (m, 2H), 4.03 (s, 3H), 3.55 (d, J = 6.2 Hz, 2H), 3.38 (s, 2H).

### Example 97

### N-(3,4-difluorobenzyl)-2-[3-(3-methoxy-4-nitrophenyl)-11-oxo-10,11-dihydro-5H-dibenzo[b,e][1,4]diazepin-8-yl]acetamide

The desired product was prepared using 3,4-difluorobenzylamine. MS (ESI) 545 (M+H)⁺; ¹H NMR (DMSO-d₆) δ 9.88 (s, 1H), 8.48 (br t, J = 5.9 Hz, 1H), 8.01 (d, J = 8.4 Hz, 1H), 7.96 (s, 1H), 7.80 (d, J = 8.1 Hz, 1H), 7.52 (d, J = 1.6 Hz, 1H), 7.35 (m, 3H), 7.30 (dd, J = 8.3, 1.7 Hz, 1H), 7.24 (m, 1H), 7.06 (br m, 1H), 6.94 (d, J = 7.8 Hz, 1H), 6.88 (m, 2H), 4.23 (d, J = 5.9 Hz, 2H), 4.03 (s, 3H), 3.36 (s, 2H).

### Example 98

### N-(4-hydroxybutyl)-2-[3-(3-methoxy-4-nitrophenyl)-11-oxo-10,11-dihydro-5H-dibenzo[b,e][1,4]diazepin-8-yl]acetamide

The desired product was prepared using 4-amino-1-butanol. MS (ESI) 491 (M+H)⁺; ¹H NMR (DMSO-d₆) δ 9.88 (s, 1H), 8.01 (d, J = 8.4 Hz, 1H), 7.94 (s, 1H), 7.90 (br t, J = 5.8 Hz, 1H), 7.80 (d, J = 8.1 Hz, 1H), 7.52 (d, J =1.6 Hz, 1H), 7.34 (m, 2H), 7.30 (dd, J = 8.3, 1.7 Hz, 1H), 6.93 (d, J = 7.8 Hz, 1H), 6.85 (m, 2H), 4.37 (br t, J = 6.2 Hz, 1H), 4.03 (s, 3H), 3.37 (m, 2H), 3.26 (s, 2H), 3.02 (m, 2H), 1.40 (m, 4H).

### Example 99

### N-(1-benzyl-4-piperidinyl)-2-[3-(3-methoxy-4-nitrophenyl)-11-oxo-10,11-dihydro-5H-dibenzo[b,e][1,4]diazepin-8-yl]acetamide

The desired product was prepared using 1-benzyl-4-piperidinamine. MS (ESI) 592 (M+H)⁺; ¹H NMR (DMSO-d₆) δ 9.88 (s, 1H), 9.46 (br s, 1H), 8.05 (d, J = 7.5 Hz, 1H), 8.01 (d, J = 8.4 Hz, 1H), 7.95 (s, 1H), 7.80 (d, J = 8.1 Hz, 1H), 7.50 (m, 6H), 7.34 (m, 2H), 7.30 (dd, J = 8.3, 1.7 Hz, 1H), 6.93 (d, J = 7.8 Hz, 1H), 6.85 (m, 2H), 4.27 (d, J = 5.0 Hz, 2H), 4.03 (s, 3H), 3.72 (m, 1H), 3.27 (s, 2H), 3.04 (m, 2H), 1.90 (m, 2H), 1.60 (m, 2H).

### Example 100

### N-[4-(dimethylamino)butyl]-2-[3-(3-methoxy-4-nitrophenyl)-11-oxo-10,11-dihydro-5H-dibenzo[b,e][1,4]diazepin-8-yl]acetamide

The desired product was prepared using N-(4-aminobutyl)-N,N-dimethylamine. MS (ESI) 518 (M+H)⁺; ¹H NMR (DMSO-d₆) δ 9.88 (s, 1H), 9.28 (v br s, 1H), 8.01 (m, 2H), 7.96 (s, 1H), 7.80 (d, J = 8.1 Hz, 1H), 7.52 (d, J = 1.6 Hz, 1H), 7.34 (m, 2H), 7.30 (dd, J = 8.3, 1.7 Hz, 1H), 6.95 (d, J = 7.8 Hz, 1H), 6.87 (m, 2H), 4.03 (s, 3H), 3.27 (s, 2H), 3.05 (m, 4H), 2.72 (s, 3H), 2.70 (s, 3H), 1.54 (m, 2H), 1.40 (m, 2H).

### Example 101

### N-{2-[4-(aminosulfonyl)phenyl]ethyl}-2-[3-(3-methoxy-4-nitrophenyl)-11-oxo-10,11-dihydro-5H-dibenzo[b,e][1,4]diazepin-8-yl]acetamide

The desired product was prepared using 4-(2-aminoethyl)benzenesulfonamide. MS (ESI) 602 (M+H)⁺; ¹H NMR (DMSO-d₆) δ 9.88 (s, 1H), 8.05 (br t, J = 5.5 Hz, 1H), 8.01 (d, J = 8.4 Hz, 1H), 7.94 (s, 1H), 7.80 (d, J = 8.1 Hz, 1H), 7.73 (d, 2H), 7.52 (d, J = 1.6 Hz, 1H), 7.36 (m, 4H), 7.30 (dd, J = 8.1, 1.6 Hz, 1H), 7.25 (s, 2H), 6.94 (d, J = 8.1 Hz, 1H), 6.87 (s, 1H), 6.82 (dd, J = 8.1, 1.6 Hz, 1H), 4.03 (s, 3H), 3.25 (s, 2H), 2.78 (m, 2H).

### Example 102

### N-(2-hydroxyethyl)-2-[3-(3-methoxy-4-nitrophenyl)-11-oxo-10,11-dihydro-5H-dibenzo[b,e][1,4]diazepin-8-yl]Npropylacetamide

The desired product was prepared using 2-(propylamino)ethanol. MS (ESI) 505 (M+H)⁺; ¹H NMR (DMSO-d₆) δ 9.88 (s, 1H), 8.01 (d, J = 8.4 Hz, 1H), 7.95 and 7.93 (both s, total 1H), 7.80 (d, J = 8.1 Hz, 1H), 7.52 (d, J = 1.2 Hz, 1H), 7.34 (m, 2H), 7.30 (dd, J = 8.1, 1.6 Hz, 1H), 6.93 (d, J = 7.8 Hz, 1H), 6.83 (m, 2H), 4.80 and 4.60 (both v br s, total 1H), 4.03 (s, 3H), 3.59 and 3.53 (both s, total 2H), 3.50 and 3.45 (both m, total 2H), 3.20 (m, 1H), 1.45 (m, 2H), 0.83 and 0.79 (both t, J = 7.3 Hz, total 3H).

### Example 103

### 8-[2-(4-ethyl-1-piperazinyl)-2-oxoethyl]-3-(3-methoxy-4-nitrophenyl)-5,10-dihydro-11H-dibenzo[b,e][1,4]diazepin-11-one

The desired product was prepared using 1-ethylpiperazine. MS (ESI) 516 (M+H)⁺.

### Example 104

### 8-{2-[4-(2-hydroxyethyl)-1-piperazinyl]-2-oxoethyl}-3-(3-methoxy-4-nitrophenyl)-5,10-dihydro-11H-dibenzo[b,e][1,4]diazepin-11-one

The desired product was prepared using 2-(1-piperazinyl)ethanol. MS (ESI) 532 (M+H)⁺.

### Example 105

### 2-[3-(3-methoxy-4-nitrophenyl)-11-oxo-10,11-dihydro-5H-dibenzo[b,e][1,4]diazepin-8-yl]NmethylN[2-(2-pyridinyl)ethyl]acetamide

The desired product was prepared using N-methylN[2-(2-pyridinyl)ethyl]amine. MS (ESI) 538 (M+H)⁺; ¹H NMR (DMSO-d₆) δ 9.85 (s, 1H), 8.65 (m, 1H), 8.09, 8.02, 8.00, 7.95 (m, d, d, m, J (for the d) =2.5 Hz, total 3H), 7.80 (m, 1H), 7.60 (m, 1H), 7.52 (s, 1H), 7.48 (m, 1H), 7.35 (m, 2H), 7.30 (m, 1H), 6.93 (d, J = 8.1 Hz, 1H), 6.76 (m, 1H), 6.72 (m, 1H), 4.04 (s, 3H), 3.67, (m, 2H), 3.49 and 3.42 (both s, total 2H), 3.05 (m, 2H), 2.97 and 2.82 (both s, total 3H).

### Example 106

### 3-(3-methoxy-4-nitrophenyl)-8-[2-oxo-2-(4-phenyl-1-piperazinyl)ethyl]-5,10-dihydro-11H-dibenzo[b,e][1,4]diazepin-11-one

The desired product was prepared using 1-phenylpiperazine. MS (ESI) 564 (M+H)⁺; ¹H NMR (DMSO-d₆) δ 9.88 (s, 1H), 8.01 (d, J = 8.4 Hz, 1H), 7.92 (s, 1H), 7.80 (d, J = 8.4 Hz, 1H), 7.52 (d, J = 1.2 Hz, 1H), 7.35 (m, 2H), 7.30 (dd, J = 8.1, 1.6 Hz, 1H), 7.20 (m, 2H), 6.94 (m, 3H), 6.85 (m, 2H), 6.79 (m, 1H), 4.03 (s, 3H), 3.64 (s, 2H), 3.60 (m, 4H), 3.08 (m, 4H).

### Example 107

### 3-(3-methoxy-4-nitrophenyl)-8-{2-oxo-2-[4-(2-pyridinyl)-1-piperazinyl]ethyl}-5,10-dihydro-11H-dibenzo[b,e][1,4]diazepin-11-one

The desired product was prepared using 1-(2-pyridinyl)piperazine. MS (ESI) 565 (M+H)⁺; ¹H NMR (DMSO-d₆) δ 9.88 (s, 1H), 8.17 (m, 1H), 8.01 (d, J = 8.4 Hz, 1H), 7.97 (s, 1H), 7.80 (d, J = 8.1 Hz, 1H), 7.73 (br m, 1H), 7.52 (s, 1H), 7.35 (m, 2H), 7.30 (dd, J = 8.1, 1.6 Hz, 1H), 7.15 (br m, 1H), 6.96 (d, J = 8.7 Hz, 1H), 6.85 (m 2H), 6.78 (br m, 1H), 4.03 (s, 3H), 3.65 (s, 2H), 3.62 (br s, 4H), 3.54 (br m, 4H).

### Example 108

### 2-[3-(3-methoxy-4-nitrophenyl)-11-oxo-10,11-dihydro-5H-dibenzo[b,e][1,4]diazepin-8-yl]N(3-pyridinylmethyl)acetamide

The desired product was prepared using 3-pyridinylmethylamine. MS (ESI) 510 (M+H)⁺; ¹H NMR (DMSO-d₆) δ 9.88 (s, 1H), 8.58 (m, 3H), 8.01 (d, J = 8.4 Hz, 1H), 7.97 (s, 1H), 7.94 (m, 1H), 7.80 (d, J = 8.1 Hz, 1H), 7.61 (m, 1H), 7.52 (s, 1H), 7.34 (m, 2H), 7.30 (dd, J = 8.1, 1.9 Hz, 1H), 6.93 (d, J = 7.8 Hz, 1H), 6.85 (m, 2H), 4.34 (d, J = 5.9 Hz, 2H), 4.03 (s, 3H), 3.36 (s, 2H).

### Example 109

### 2-[3-(3-methoxy-4-nitrophenyl)-11-oxo-10,11-dihydro-5H-dibenzo[b,e][1,4]diazepin-8-yl]N(4-pyridinylmethyl)acetamide

The desired product was prepared using 4-pyridinylmethylamine. MS (ESI) 510 (M+H)⁺; ¹H NMR (DMSO-d₆) δ 9.89 (s, 1H), 8.66 (m, 3H), 8.01 (d, J = 8.4 Hz, 1H), 7.98 (s, 1H), 7.81 (d, J = 8.1 Hz, 1H), 7.58 (d, J = 5.6 Hz, 2H), 7.52 (s, 1H), 7.34 (m, 2H), 7.30 (dd, J = 8.3, 1.7 Hz, 1H), 6.96 (d, J = 8.1 Hz, 1H), 6.90 (m, 2H), 4.42 (d, J = 5.6 Hz, 2H), 4.03 (s, 3H), 3.43 (s, 2H).

### Example 110

### 2-[3-(3-methoxy-4-nitrophenyl)-11-oxo-10,11-dihydro-5H-dibenzo[b,e][1,4]diazepin-8-yl]N(2-pyridinylmethyl)acetamide

The desired product was prepared using 2-pyridinylmethylamine. MS (ESI) 510 (M+H)⁺; ¹H NMR (DMSO-d₆) δ 9.88 (s, 1H), 8.58 (m, 2H), 8.01 (d, J = 8.4 Hz, 1H), 7.97 (s, 1H), 7.93 (m, 1H), 7.81 (d, J = 8.1 Hz, 1H), 7.52 (s, 1H), 7.41 (m, 2H), 7.34 (m, 2H), 7.30 (dd, J = 8.1, 1.6 Hz, 1H), 6.96 (d, J = 8.1 Hz, 1H), 6.90 (m, 2H), 4.41 (d, J = 5.6 Hz, 2H), 4.03 (s, 3H), 3.41 (s, 2H).

### Example 111

### N-[2-(dimethylamino)ethyl]-2-[3-(3-methoxy-4-nitrophenyl)-11-oxo-10,11-dihydro-5H-dibenzo[b,e][1,4]diazepin-8-yl]acetamide

The desired product was prepared using N-(2-aminoethyl)-N,N-dimethylamine. MS (ESI) 490 (M+H)⁺; ¹H NMR (DMSO-d₆) δ 9.88 (s, 1H), 9.28 (v br s, 1H), 8.18 (br t, J = 5.6 Hz, 1H), 8.01 (d, J = 8.4 Hz, 1H), 7.96 (s, 1H), 7.80 (d, J = 8.1 Hz, 1H), 7.52 (d, J = 1.2 Hz, 1H), 7.34 (m, 2H), 7.30 (dd, J = 8.1, 1.6 Hz, 1H), 6.95 (d, J = 7.8 Hz, 1H), 6.87 (m, 2H), 4.03 (s, 3H), 3.32 (s, 2H), 3.12 (v br m, 2H), 2.78 (s, 6H).

### Example 112

### N-[3-(dimethylamino)propyl]-2-[3-(3-methoxy-4-nitrophenyl)-11-oxo-10,11-dihydro-5H-dibenzo[b,e][1,4]diazepin-8-yl]acetamide

The desired product was prepared using N-(3-aminopropyl)-N,N-dimethylamine. MS (ESI) 504 (M+H)⁺; ¹H NMR (DMSO-d₆) δ 9.88 (s, 1H), 9.30 (v br s, 1H), 8.10 (br t, J = 5.6 Hz, 1H), 8.01 (d, J = 8.4 Hz, 1H), 7.96 (s, 1H), 7.80 (d, J = 8.1 Hz, 1H), 7.52 (d, J = 1.6 Hz, 1H), 7.34 (m, 2H), 7.30 (dd, J = 8.1, 1.6 Hz, 1H), 6.95 (d, J = 7.8 Hz, 1H), 6.87 (m, 2H), 4.03 (s, 3H), 3.28 (s, 2H), 3.09 (m, 2H), 2.98 (m, 2H), 2.74 (s, 3H), 2.73 (s, 3H), 1.74 (m, 2H).

### Example 113

### 2-[3-(3-methoxy-4-nitrophenyl)-11-oxo-10,11-dihydro-5H-dibenzo[b,e][1,4]diazepin-8-yl]N[2-(1-pyrrolidinyl)ethyl]acetamide

The desired product was prepared using 2-(1-pyrrolidinyl)ethylamine. MS (ESI) 516 (M+H)⁺; ¹H NMR (DMSO-d₆) δ 9.88 (s, 1H), 9.50 (v br s, 1H), 8.19 (br t, J = 5.8 Hz, 1H), 8.01 (d, J = 8.4 Hz, 1H), 7.96 (s, 1H), 7.80 (d, J = 8.4 Hz, 1H), 7.52 (d, J = 1.6 Hz, 1H), 7.34 (m, 2H), 7.30 (dd, J = 8.3, 1.7 Hz, 1H), 6.95 (d, J = 7.8 Hz, 1H), 6.87 (m, 2H), 4.03 (s, 3H), 3.55 (m, 2H), 3.32 (s, 2H), 3.18 (m, 2H), 2.97 (m, 2H), 1.97 (m, 2H), 1.83 (m, 2H).

### Example 114

### 2-[3-(3-methoxy-4-nitrophenyl)-11-oxo-10,11-dihydro-5H-dibenzo[b,e][1,4]diazepin-8-yl]N[2-(1-piperidinyl)ethyl]acetamide

The desired product was prepared using 2-(1-piperidinyl)ethylamine. MS (ESI) 530 (M+H)⁺; ¹H NMR (DMSO-d₆) δ 9.88 (s, 1H), 9.08 (v br s, 1H), 8.21 (br t, J = 5.6 Hz, 1H), 8.01 (d, J = 8.4 Hz, 1H), 7.96 (s, 1H), 7.80 (d, J = 8.1 Hz, 1H), 7.52 (d, J = 1.6 Hz, 1H), 7.34 (m, 2H), 7.30 (dd, J = 8.1, 1.9 Hz, 1H), 6.95 (d, J = 7.8 Hz, 1H), 6.87 (m, 2H), 4.03 (s, 3H), 3.32 (s, 2H), 3.08 (m, 2H), 2.85 (m, 2H), 1.76 (m, 2H), 1.60 (m, 3H), 1.33 (m, 1H).

### Example 115

### 2-[3-(3-methoxy-4-nitrophenyl)-11-oxo-10,11-dihydro-5H-dibenzo[b,e][1,4]diazepin-8-yl]N[3-(1-piperidinyl)propyl]acetamide

The desired product was prepared using 3-(1-piperidinyl)propylamine. MS (ESI) 544 (M+H)⁺; ¹H NMR (DMSO-d₆) δ 9.88 (s, 1H), 8.93 (v br s, 1H), 8.11 (br t, J = 5.8 Hz, 1H), 8.01 (d, J = 8.4 Hz, 1H), 7.96 (s, 1H), 7.80 (d, J = 8.1 Hz, 1H), 7.52 (d, J = 1.2 Hz, 1H), 7.34 (m, 2H), 7.30 (dd, J = 8.3, 1.7 Hz, 1H), 6.95 (d, J = 8.1 Hz, 1H), 6.87 (m, 2H), 4.03 (s, 3H), 3.28 (s, 2H), 3.10 (m, 2H), 2.94 (m, 2H), 2.78 (m, 2H), 1.75 (m, 4H), 1.60 (m, 3H), 1.33 (m, 1H).

### Example 116

### 2-[3-(3-methoxy-4-nitrophenyl)-11-oxo-10,11-dihydro-5H-dibenzo[b,e][1,4]diazepin-8-yl]N[2-(4-morpholinyl)ethyl]acetamide

The desired product was prepared using 2-(4-morpholinyl)ethylamine. MS (ESI) 532 (M+H)⁺; ¹H NMR (DMSO-d₆) δ 9.88 (s, 1H), 9.69 (v br s, 1H), 8.20 (v br s, 1H), 8.01 (d, J = 8.4 Hz, 1H), 7.96 (s, 1H), 7.80 (d, J = 8.4 Hz, 1H), 7.52 (d, J = 1.6 Hz, 1H), 7.34 (m, 2H), 7.30 (dd, J = 8.3, 1.7 Hz, 1H), 6.95 (d, J = 8.4 Hz, 1H), 6.87 (m, 2H), 4.03 (s, 3H), 3.95 (br m, 2H), 3.63 (br m, 2H), 3.32 (s, 2H), 3.15 (br m, 3H).

### Example 117

### 2-[3-(3-methoxy-4-nitrophenyl)-11-oxo-10,11-dihydro-5H-dibenzo[b,e][1,4]diazepin-8-yl]N[3-(4-morpholinyl)propyl]acetamide

The desired product was prepared using 3-(4-morpholinyl)propylamine. MS (ESI) 546 (M+H)⁺; ¹H NMR (DMSO-d₆) δ 9.88 (s, 1H), 9.55 (v br s, 1H), 8.12 (br t, J = 5.8 Hz, 1H), 8.01 (d, J = 8.4 Hz, 1H), 7.96 (s, 1H), 7.80 (d, J = 8.1 Hz, 1H), 7.52 (d, J = 1.6 Hz, 1H), 7.34 (m, 2H), 7.30 (dd, J = 8.3, 1.7 Hz, 1H), 6.95 (d, J = 7.8 Hz, 1H), 6.87 (m, 2H), 4.03 (s, 3H), 3.95 (m, 2H), 3.60 (br t, 2H), 3.29 (s, 2H), 3.10 (m, 2H), 3.03 (m, 2H), 1.76 (m, 2H).

### Example 118

### 2-[3-(3-methoxy-4-nitrophenyl)-11-oxo-10,11-dihydro-5H-dibenzo[b,e][1,4]diazepin-8-yl]N[2-(4-methyl-1-piperazinyl)ethyl]acetamide

The desired product was prepared using 2-(4-methyl-1-piperazinyl)ethanamine. MS (ESI) 545 (M+H)⁺.

### Example 119

### 8-{2-[(2S)-2-(hydroxymethyl)-1-pyrrolidinyl]-2-oxoethyl}-3-(3-methoxy-4-nitrophenyl)-5,10-dihydro-11H-dibenzo[b,e][1,4]diazepin-11-one

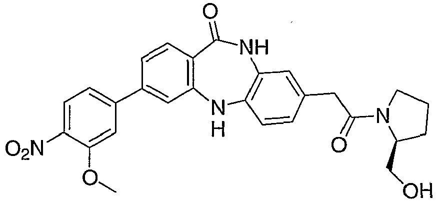

The desired product was prepared by substituting Example 73 and (2S)-2-pyrrolidinylmethanol for Example 13 and 3-pyrrolidin-1-ylpropylamine, respectively, in Example 14. MS (ESI) m/e 503 (M+H)⁺; ¹H NMR (DMSO-d₆, 300 MHz): δ 9.89 (s, 1H), 8.01 (d, J = 8.14 Hz, 1H), 7.97 (s, 1H), 7.79 (d, J = 8.14 Hz, 1H), 7.52 (d, J = 1.36 Hz, 1H), 7.29-7.36 (m, 3H), 6.94 (d, J = 8.14 Hz, 1H), 6.81-6.86 (m, 2H), 4.03 (s, 3H), 3.90-3.98 (m, 1H), 3.48 (s, 2H), 3.21-3.30 (m, 4H), 1.74-1.91 (m, 4H); MS m/e (ESI) 503 (M-H)⁺.

### Example 120

### 8-amino-3-(3-methoxy-4-nitrophenyl)-5,10-dihydro-11H-dibenzo[b,e][1,4]diazepin-11-one

The desired product was prepared by substituting Example 7C and 2-(3-methoxy-4-nitrophenyl)-4,4,5,5-tetramethyl-1,3,2-dioxaborolane for Example 1B and 2-methoxy-4-(4,4,5,5-tetramethyl-1,3,2-dioxaborolan-2-yl)phenol, respectively, in Example 12. MS (DSI) m/e 377 (M+H)⁺; ¹H NMR (500 MHz, DMSO-d₆, TFA salt) δ 9.91 (s, 1H), 7.94 (d, J = 8.3 Hz, 1H), 7.87 (s, 1H), 7.73 (d, J = 8.0 Hz, 1H), 7.45 (s, 1H), 7.27-7.28 (m, 2H), 7.23 (d, J = 8.0 Hz, 1H), 6.88 (d, J = 8.0 Hz, 1H), 6.58-6.61 (m, 2H), 3.96 (s, 3H).

### Example 121

### N-[3-(3-methoxy-4-nitrophenyl)-11-oxo-10,11-dihydro-5H-dibenzo[b,e][1,4]diazepin-8-yl]urea

A mixture Example 120 (56 mg, 0.15 mmol) and NaOCN (17 mg, 0.263 mmol) in 4 mL of acetic acid and 0.8 mL of H₂O was stirred at room temperature for 12 hours. The solvents were removed and the residue was purified by preparative HPLC. MS (DCI) m/e 420 (M+H)⁺; ¹H NMR (500 MHz, DMSO-d₆) δ 9.86 (s, 1H), 8.36 (s, 1H), 8.00 (d, J=8.42 Hz, 1H), 7.78-7.80 (m, 2H), 7.52 (d, J=1.56 Hz, 1H), 7.73-7.75 (m, 2H), 7.28 (dd, J=8.27, 1.72 Hz, 1H), 7.01-7.05 (m, 2H), 6.88 (d, J=8.42 Hz, 1H), 5.74 (s, 2H), 4.03 (s, 3H).

### Example 122

### 2-(dimethylamino)N[3-(3-methoxy-4-nitrophenyl)-11-oxo-10,11-dihydro-5H-dibenzo[b,e][1,4]diazepin-8-yl]acetamide

A mixture of Example 120 (38 mg, 0.10 mmol), dimethylaminoacetic acid (17 mg, 0.12 mmol), HATU (46 mg, 0.12 mmol) and Et₃N (0.1g) in 1 mL of DMF was stirred overnight. The reaction mixture was partitioned between H₂O and ethyl acetate, and the organic layer was separated, washed with brine, dried, filtered, and concentrated under vacuum. The residue was purified by preparative HPLC to give the desired product.. MS (DCI) m/e 462 (M+H)⁺; ¹H NMR (500 MHz, DMSO-d₆) δ 10.68 (s, 1H), 10.03 (s, 1H), 8.10 (s, 1H), 8.01 (d, J=8.59 Hz, 1H), 7.80 (d, J=8.29 Hz, 1H), 7.53 (d, J=1.23 Hz, 1H), 7.43 (s, 1H), 7.21-7.36 (m, 4H), 7.03 (d, J=8.59 Hz, 1H), 4.11 (s, 2H), 4.03 (s, 3H), 2.86 (s, 6H).

### Example 123

### (2S)-2-aminoN[3-(3-methoxy-4-nitrophenyl)-11-oxo-10,11-dihydro-5H-dibenzo[b,e][1,4]diazepin-8-yl]-4-methylpentanamide

A mixture of Example 120 (56 mg, 0.10 mmol), L- 2-tert-butoxycarbonylamino-4-methyl-pentanoic acid (39 mg, 0.17 mmol), HATU (65 mg, 0.17 mmol) and Et₃N (0.1g) in 2 mL of DMF was stirred overnight. The reaction mixture was partitioned between H₂O and ethyl acetate, and the organic layer was separated, washed with brine, dried, filtered, and concentrated under vacuum. The residue was purified by flash column chromatography on silica gel with 1:1 hexanes/ethyl acetate to provide 72mg (81%) of {1-[3-(3-methoxy-4-nitro-phenyl)-11-oxo-10,11-dihydro-5H-dibenzo[b,e][1,4]diazepin-8-ylcarbamoyl]-3-methyl-butyl}-carbamic acid tert-butyl ester, which was treated with 2 mL of TFA and 2 mL of CH₂Cl₂. The solvents were removed under vacuum and the residue was purified with preparative HPLC to give the title product. MS (DCI) m/e 490 (M+H)⁺; ¹H NMR (500 MHz, DMSO-d₆) δ 10.42 (s, 1H), 10.02 (s, 1H), 8.00-8.03 (m, 2H), 7.80 (d, J=8.11 Hz, 1H), 7.53 (s, 1H), 7.30-7.38 (m, 4H), 7.22 (d, J=8.42 Hz, 1H), 7.00 (d, J=8.74, 1H), 4.04 (s, 3H), 3.89 (m, 1H), 1.63-1.67 (m, 3H), 0.93 (t, J=6.24 Hz, 6H).

### Example 124

### 4-AminoN[3-(3-methoxy-4-nitrophenyl)-11-oxo-10,11-dihydro-5H-dibenzo[b,e][1,4]diazepin-8-yl]butyramide

The title compound was prepared by substituting 4-tert-butoxycarbonylamino-butyric acid for (L) 2-tert-butoxycarbonylamino-4-methyl-pentanoic acid in Example 123. MS (DCI) m/e 462 (M+H)⁺; ¹H NMR (500 MHz, DMSO-d₆) δ 9.96 (s, 1H), 9.89 (s, 1H), 8.00 (d, J=8.42 Hz, 1H), 7.94 (s, 1H), 7.75-7.81 (m, 3H), 7.62 (s, 1H), 7.28-7.36 (m, 4H), 7.18 (d, J=8.11 Hz, 1H), 6.95 (d, J=8.73 Hz, 1H), 4.03 (s, 3H), 2.83-2.86 (m, 2H), 2.39 (t, J=6.2 Hz, 2H), 1.84 (m, 2H).

### Example 125

### 3-AminoN[3-(3-methoxy-4-nitrophenyl)-11-oxo-10,11-dihydro-5H-dibenzo[b,e][1,4]diazepin-8-yl]propionamide

The title compound was prepared by substituting 3-tert-butoxycarbonylamino-propionic acid for (L) 2-tert-butoxycarbonylamino-4-methyl-pentanoic acid in Example 123. MS (DCI) m/e 448 (M+H)⁺; ¹H NMR (500 MHz, DMSO-d₆) δ 10.05 (s, 1H), 9.99 (s, 1H), 8.00 (d, J=8.42 Hz, 1H), 7.96 (s, 1H), 7.75-7.81 (m, 3H), 7.52 (s, 1H), 7.34-7.36 (m, 2H), 7.30 (dd, J=8.11, 1.25 Hz, 1H), 7.21-7.24 (m, 2H), 6.96 (d, J=8.73 Hz, 1H), 4.03 (s, 3H), 3.08 (t, J=6.40 Hz, 2H), 2.67 (t, J=6.71 Hz, 2H).

### Example 126

### N-[3-(3-Methoxy-4-nitrophenyl)-11-oxo-10,11-dihydro-5H-dibenzo[b,e][1,4]diazepin-8-yl]-2-(3-methyl-3H-imidazol-4-yl)acetamide

The title compound was prepared by substituting (3-methyl-3H-imidazol-4-yl)-acetic acid for dimethylaminoacetic acid in Example 122. MS (DCI) m/e 499 (M+H)⁺; ¹H NMR (500 MHz, DMSO-d₆) δ 10.17 (s, 1H), 9.99 (s, 1H), 8.87 (s, 1H), 8.00 (d, J=8.42 Hz, 1H), 7.96 (s, 1H), 7.80 (d, J=8.11 Hz, 1H), 7.52 (d, J=1.56 Hz, 1H), 7.49 (s, 1H), 7.20-7.35 (m, 5H), 6.96 (d, J=8.74 Hz, 1H), 4.03 (s, 3H), 3.83 (s, 3H), 3.80 (s, 2H).

### Example 127

### 2-(3H-Imidazol-4-yl)N[3-(3-methoxy-4-nitrophenyl)-11-oxo-10,11-dihydro-5H-dibenzo[b,e][1,4]diazepin-8-yl]acetamide

The title compound was prepared by substituting (3H-imidazol-4-yl)-acetic acid for dimethylaminoacetic acid in Example 122. MS (DCI) m/e 485 (M+H)⁺; ¹H NMR (500 MHz, DMSO-d₆) δ 10.18 (s, 1H), 9.99 (s, 1H), 8.98 (s, 1H), 8.00 (d, J=8.42 Hz, 1H), 7.96 (s, 1H), 7.80 (d, J=8.11 Hz, 1H), 7.52 (d, J=1.56 Hz, 1H), 7.49 (s, 1H), 7.20-7.35 (m, 6H), 6.96 (d, J=8.73 Hz, 1H), 4.03 (s, 3H), 3.83 (s, 2H).

### Example 128

### N-[3-(3-methoxy-4-nitrophenyl)-11-oxo-10,11-dihydro-5H-dibenzo[b,e][1,4]diazepin-8-yl]thiophene-3-carboxamide

The title compound was prepared by substituting thiophene-3-carboxylic acid for dimethylaminoacetic acid in Example 122. MS (DCI) m/e 487 (M+H)⁺; ¹H NMR (300 MHz, DMSO-d₆) δ 9.98 (s, 1H), 9.97 (s, 1H), 8.30 (m, 1H), 8.01 (d, J=8.42 Hz, 1H), 7.81 (d, J=8.11 Hz, 1H), 7.60-7.64 (m, 2H), 7.53 (d, J=1.87 Hz, 1H), 7.44 (d, J=2.18 Hz, 1H), 7.34-7.36 (m, 2H), 7.29-7.31 (m, 2H), 6.99 (d, J=8.42 Hz, 1H), 4.04 (s, 3H).

### Example 129

### N-[3-(3-methoxy-4-nitrophenyl)-11-oxo-10,11-dihydro-5H-dibenzo[b,e][1,4]diazepin-8-yl]-1H-pyrrole-2-carboxamide

The title compound was prepared by substituting 1H-pyrrole-2-carboxylic acid for dimethylaminoacetic acid in Example 122. MS (DCI) m/e 470 (M+H)⁺; ¹H NMR (300 MHz, DMSO-d₆) δ 11.57 (s, 1H), 9.97 (s, 1H), 9.96 (s, 1H), 8.01 (d, J=8.42 Hz, 1H), 7.93 (s, 1H), 7.80 (d, J=8.11 Hz, 1H), 7.53 (d, J=1.56 Hz, 1H), 7.44 (d, J=2.18 Hz, 1H), 7.34-7.36 (m, 2H), 7.26-7.31 (m, 2H), 6.93-7.02 (m, 3H), 6.15 (m, 1H), 4.04 (s, 3H).

### Example 130

### N-[3-(3-methoxy-4-nitrophenyl)-11-oxo-10,11-dihydro-5H-dibenzo[b,e][1,4]diazepin-8-yl]-2,5-dimethyl-1H-pyrrole-3-carboxamide

The title compound was prepared by substituting 2,5-dimethyl-1H-pyrrole-3-carboxylic acid for dimethylaminoacetic acid in Example 122. MS (DCI) m/e 498 (M+H)⁺; ¹H NMR (300 ) MHz, DMSO-d₆) δ 10.77 (s, 1H), 9.88 (s, 1H), 9.09 (s, 1H), 8.00 (d, J=8.42 Hz, 1H), 7.86 (s, 1H), 7.80 (m, 1H), 7.52 (d, J=1.87 Hz, 1H), 7.47 (d, J=2.18 Hz, 1H), 7.34-7.36 (m, 2H), 7.29 (dd, J=8.11, 1.56 Hz, 1H), 7.24 (dd, J=8.58, 2.34 Hz, 1H), 6.92 (d, J=8.73 Hz, 1H), 6.30 (d, J=1.87 Hz, 1H), 4.03 (s, 3H), 2.38 (s, 3H), 2.13 (s, 3H).

### Example 131

### N-[3-(3-methoxy-4-nitrophenyl)-11-oxo-10,11-dihydro-5H-dibenzo[b,e][1,4]diazepin-8-yl]-1,3-thiazole-4-carboxamide

The title compound was prepared by substituting 1,3-thiazole-4-carboxylic acid for dimethylaminoacetic acid in Example 122. MS (DCI) m/e 488 (M+H)⁺; ¹H NMR (300 MHz, DMSO-d₆) δ 10.20 (s, 1H), 9.87 (s, 1H), 9.25 (s, 1H), 8.46 (s, 1H), 7.98-8.02 (m, 2H), 7.81 (d, J=8.11 Hz, 1H), 7.53-7.56 (m, 2H), 7.34-7.38 (m, 3H), 7.30 (dd, J=8.11, 1.87 Hz, 1H), 6.99 (d, J=8.42 Hz, 1H), 4.04 (s, 3H).

### Example 132

### N-[3-(3-methoxy-4-nitrophenyl)-11-oxo-10,11-dihydro-5H-dibenzo[b,e][1,4]diazepin-8-yl]-1H-pyrazole-5-carboxamide

The title compound was prepared by substituting 1H-pyrazole-5-carboxylic acid for dimethylaminoacetic acid in Example 122. MS (DCI) m/e 471 (M+H)⁺; ¹H NMR (300 MHz, DMSO-d₆) δ 13.35 (s, 1H), 9.87 (s, 1H), 9.85 (s, 1H), 8.02 (d, J=8.11 Hz, 1H), 7.81 (d, J=8.11 Hz, 1H), 7.53-7.56 (m, 3H), 7.31-7.36 (m, 5H), 7.30 (dd, J=8.11, 1.87 Hz, 1H), 6.99 (d, J=8.42 Hz, 1H), 4.04 (s, 3H).

### Example 133

### N-[3-(3-methoxy-4-nitrophenyl)-11-oxo-10,11-dihydro-5H-dibenzo[b,e][1,4]diazepin-8-yl]-1H-pyrazole-4-carboxamide

The title compound was prepared by substituting 1H-pyrazole-4-carboxylic acid for dimethylaminoacetic acid in Example 122. MS (DCI) m/e 471 (M+H)⁺; ¹H NMR (300 MHz, DMSO-d₆) δ 9.97 (s, 1H), 9.72 (s, 1H), 8.01 (d, J=8.42 Hz, 1H), 7.93 (s, 1H), 7.80 (d, J=8.11 Hz, 1H), 7.53 (d, J=1.56 Hz, 1H), 7.40 (d, J=2.50 Hz, 1H), 7.34-7.36 (m, 2H), 7.30 (dd, J=8.11, 1.56 Hz, 1H), 7.26 (dd, J=8.42, 2.18 Hz, 1H), 6.99 (d, J=8.73 Hz, 1H), 4.04 (s, 3H).

### Example 134

### N-[3-(3-methoxy-4-nitrophenyl)-11-oxo-10,11-dihydro-5H-dibenzo[b,e] [1,4]diazepin-8-yl]isonicotinamide

The title compound was prepared by substituting isonicotinic acid for dimethylaminoacetic acid in Example 122. MS (DCI) m/e 482 (M+H)⁺; ¹H NMR (300 MHz, DMSO-d₆) δ 10.43 (s, 1H), 10.91 (s, 1H), 8.78 (d, J=5.93 Hz, 1H), 8.01-8.02 (m, 2H), 7.81-7.84 (m, 3H), 7.53 (d, J=1.56 Hz, 1H), 7.48 (d, J=2.18 Hz, 1H), 7.30-7.36 (m, 5H), 7.01 (d, J=8.42 Hz, 1H), 4.04 (s, 3H).

### Example 135

### N-[3-(3-methoxy-4-nitrophenyl)-11-oxo-10,11-dihydro-5H-dibenzo[b,e][1,4]diazepin-8-yl]-3-pyrrolidin-1-ylpropanamide

The title compound was prepared by substituting 3-pyrrolidin-1-ylpropionic acid for dimethylaminoacetic acid in Example 122. MS (DCI) m/e 502 (M+H)⁺; ¹H NMR (300 MHz, DMSO-d₆) δ δ 10.06 (s, 1H), 9.99 (s, 1H), 8.00 (d, J=8.59 Hz, 1H), 7.95 (s, 1H), 7.80 (d, J=8.11 Hz, 1H), 7.52 (d, J=1.23 Hz, 1H), 7.31-7.34 (m, 3H), 7.21-7.23 (m, 2H), 6.96 (d, J=8.42 Hz, 1H), 4.03 (s, 3H), 3.42-3.54 (m, 4H), 3.06 (m, 2H), 2.77 (t, J=7.02 Hz, 2H), 2.01-2.03 (m, 2H), 1.85-1.87 (m, 2H).

### Example 136

### N-[3-(3-methoxy-4-nitrophenyl)-11-oxo-10,11-dihydro-5H-dibenzo[b,e][1,4]diazepin-8-yl]-3-piperidin-1-ylpropanamide

The title compound was prepared by substituting 3-piperidin-1-ylpropionic acid for dimethylaminoacetic acid in Example 122. MS (DCI) m/e 516 (M+H)⁺; ¹H NMR (300 MHz, DMSO-d₆) δ 10.06 (s, 1H), 9.99 (s, 1H), 8.00 (d, J=8.59 Hz, 1H), 7.95 (s, 1H), 7.80 (d, J=8.11 Hz, 1H), 7.52 (d, J=1.56 Hz, 1H), 7.33-7.35 (m, 2H), 7.30 (dd, J=8.27, 1.72 Hz, 1H), 7.24 (d, J=2.18 Hz, 1H), 7.21 (dd, J=8.42, 2.18 Hz, 1H), 6.96 (d, J=8.42 Hz, 1H), 4.03 (s, 3H), 3.42-3.54 (m, 4H), 2.92-2.94 (m, 2H), 2.78 (t, J=7.02 Hz, 2H), 2.61-2.63 (m, 2H), 1.81-1.83 (m, 2H), 1.64-1.66 (m, 2H).

### Example 137

### N-[3-(3-methoxy-4-nitrophenyl)-11-oxo-10,11-dihydro-5H-dibenzo[b,e][1,4]diazepin-8-yl]-3-morpholin-4-ylpropanamide

The title compound was prepared by substituting 3-morpholin-4-ylpropionic acid for dimethylaminoacetic acid in Example 122. MS (DCI) m/e 518 (M+H)⁺; ¹H NMR (300 MHz, DMSO-d₆) δ 10.06 (s, 1H), 9.99 (s, 1H), 8.00 (d, J=8.59 Hz, 1H), 7.95 (s, 1H), 7.80 (d, J=8.11 Hz, 1H), 7.52 (d, J=1.56 Hz, 1H), 7.33-7.35 (m, 2H), 7.30 (dd, J=8.27, 1.72 Hz, 1H), 7.24 (d, J=2.18 Hz, 1H), 7.21 (dd, J=8.42, 2.18 Hz, 1H), 6.96 (d, J=8.42 Hz, 1H), 3.98-4.03 (m, 7H), 3.64-3.67 (m, 2H), 3.42-3.44 (m, 2H), 3.10-3.12 (m, 2H), 2.79-2.81 (m, 2H).

### Example 138

### (2R)-2-hydroxyN[3-(3-methoxy-4-nitrophenyl)-11-oxo-10,11-dihydro-5H-dibenzo[b,e][1,4]diazepin-8-yl]-4-phenylbutanamide

The title compound was prepared by substituting (2R)-2-hydroxy-4-phenylbutanoic acid for dimethylaminoacetic acid in Example 122.. MS (DCI) m/e 539 (M+H)⁺; ¹H NMR (300 MHz, DMSO-d₆) δ 9.91 (s, 1H), 9.56 (s, 1H), 8.01 (d, J=8.42 Hz, 1H), 7.92 (s, 1H), 7.80 (d, J=8.11 Hz, 1H), 7.52 (d, J=1.56 Hz, 1H), 7.42 (d, J=2.18 Hz, 1H), 7.34-7.35 (m, 2H), 7.17-7.34 (m, 6H), 6.94 (d, J=8.42 Hz, 1H), 5.78 (d, =5.78 Hz, 1H), 4.03 (s, 3H), 3.98-4.01 (m, 1H), 3.27-3.29 (m, 2H), 2.69-2.70 (m, 2H).

### Example 139

### N-[3-(3-methoxy-4-nitrophenyl)-11-oxo-10,11-dihydro-5H-dibenzo[b,e][1,4]diazepin-8-yl]-3-(phenylsulfonyl)propanamide

The title compound was prepared by substituting 3-(phenylsulfonyl)propanoic acid for dimethylaminoacetic acid in Example 122. MS (DCI) m/e 573 (M+H)⁺; ¹H NMR (300 MHz, DMSO-d₆) δ 9.93-9.94 (m, 2H), 8.00 (d, J=8.42 Hz, 1H), 7.90-7.92 (m, 3H), 7.74-7.80 (m, 2H), 7.65-7.68 (m, 2H), 7.52 (d, J=1.56 Hz, 1H), 7.33-7.35 (m, 2H), 7.29 (dd, J=8.11, 1.56 Hz, 1H), 7.19 (d, J=2.18 Hz, 1H), 7.11 (dd, J=8.74, 2.18 Hz, 1H), 6.91 (d, J=8.42 Hz, 1H), 4.03 (s, 3H), 3.57-3.60 (m, 2H), 2.64-2.66 (m, 2H).

### Example 140

### N-[3-(3-methoxy-4-nitrophenyl)-11-oxo-10,11-dihydro-5H-dibenzo[b,e][1,4]diazepin-8-yl]-2-{[(4-methylphenyl)sulfonyl]amino}acetamide

The title compound was prepared by substituting {[(4-methylphenyl)sulfonyl]amino}acetic acid for dimethylaminoacetic acid in Example 122. MS (DCI) m/e 588 (M+H)⁺; ¹H NMR (300 MHz, DMSO-d₆) δ 9.95 (s, 1H), 9.77 (s, 1H), 8.00 (d, J=8.42 Hz, 1H), 7.92 (s, 1H), 7.69-7.80 (m, 2H), 7.53 (d, J=1.87 Hz, 1H), 7.33-7.37 (m, 5H), 7.29 (dd, J=8.27, 1.72 Hz, 1H), 7.17 (d, J=2.18 Hz, 1H), 7.08-7.09 (m, 1H), 6.92 (d, J=8.73 Hz, 1H), 4.03 (s, 3H), 3.60 (s, 2H), 2.35 (s, 2H).

### Example 141

### N-[3-(3-methoxy-4-nitrophenyl)-11-oxo-10,11-dihydro-5H-dibenzo[b,e][1,4]diazepin-8-yl]pyrridine-2-carboxamide

The title compound was prepared by substituting pyridine-2-carboxylic acid for dimethylaminoacetic acid in Example 122. MS (DCI) m/e 482 (M+H)⁺; ¹H NMR (300 MHz, DMSO-d₆) δ 10.49 (s, 1H), 9.98 (s, 1H), 8.72 (d, J=4.68 Hz, 1H), 8.14 (d, J=7.80 Hz, 1H), 8.00-8.06 (m, 4H), 7.81 (d, J=8.42 Hz, 1H), 7.67 (m, 1H), 7.62 (d, J=2.18 Hz, 1H), 7.53 (d, J=1.56 Hz, 1H), 7.43 (dd, J=8.58, 2.73 Hz, 1H), 7.35-7.36 (m, 2H), 7.30 (dd, J=8.11, 1.87 Hz, 1H), 7.01 (d, J=8.74 Hz, 1H), 4.04 (s, 3H).

### Example 142

### N-[3-(3-methoxy-4-nitrophenyl)-11-oxo-10,11-dihydro-5H-dibenzo[b,e][1,4]diazepin-8-yl]nicotinamide

The title compound was prepared by substituting nicotinic acid for dimethylaminoacetic acid in Example 122. MS (DCI) m/e 482 (M+H)⁺; ¹H NMR (300 MHz, DMSO-d₆) δ 10.37 (s, 1H), 10.01 (s, 1H), 9.08 (s, 1H), 8.75 (dd, J=4.84, 1.72 Hz, 1H), 8.26-8.28 (m, 1H), 8.00-8.02 (m, 2H), 7.81 (d, J=8.11 Hz, 1H), 7.53-7.57 (m, 2H), 7.48 (d, J=2.18 Hz, 1H), 7.30-7.37 (m, 4H), 7.01 (d, J=8.42 Hz, 1H), 4.04 (s, 3H).

### Example 143

### N-[3-(3-methoxy-4-nitrophenyl)-11-oxo-10,11-dihydro-5H-dibenzo[b,e][1,4]diazepin-8-yl]-2-pyridin-3-ylacetamide

The title compound was prepared by substituting pyridin-3-yl-acetic acid for dimethylaminoacetic acid in Example 122. MS (DCI) m/e 496 (M+H)⁺; ¹H NMR (300 MHz, DMSO-d₆) δ 10.11 (s, 1H), 9.95 (s, 1H), 8.66 (s, 1H), 8.61 (d, J=4.06, Hz, 1H), 8.00-8.04 (m, 2H), 7.93 (s, 1H), 7.79 (d, J=8.11 Hz, 1H), 7.62 (dd, J=7.96, 5.15 Hz, 1H), 7.52 (d, J=1.56 Hz, 1H), 7.33-7.35 (m, 2H), 7.26-7.30 (m, 2H), 7.20 (dd, J=8.74, 2.18 Hz, 1H), 6.95 (d, J=8.74 Hz, 1H), 4.04 (s, 3H), 3.78 (s, 2H).

### Example 144

### N-[3-(3-methoxy-4-nitrophenyl)-11-oxo-10,11-dihydro-5H-dibenzo[b,e][1,4]diazepin-8-yl]-2-(4-methylpiperazin-1-yl)acetamide

The title compound was prepared by substituting (4-methyl-piperazin-1-yl)acetic acid for dimethylaminoacetic acid in Example 122. MS (DCI) m/e 517 (M+H)⁺; ¹H NMR (300 MHz, DMSO-d₆) δ 9.95 (s, 1H), 7.96-8.02 (m, 2H), 7.80 (dd, J=8.11, 2.50 Hz, 1H), 7.25 (d, J=1.56 Hz, 1H), 7.29-7.35 (m, 4H), 7.18 (dd, J=8.58, 2.03 Hz, 1H), 6.96 (m, 1H), 6.71 (m, 1H), 4.03 (s, 3H), 2.89-3.55 (m, 13H).

### Example 145

### 3-ethoxyN[3-(3-methoxy-4-nitrophenyl)-11-oxo-10,11-dihydro-5H-dibenzo[b,e][1,4]diazepin-8-yl]propanamide

The title compound was prepared by substituting 3-ethoxy-propionic acid for dimethylaminoacetic acid in Example 122. MS (DCI) m/e 517 (M+H)⁺; ¹H NMR (500 MHz, DMSO-d₆) δ 9.94 (s, 1H), 9.82 (s, 1H), 8.00 (d, J=8.29 Hz, 1H), 7.91 (s, 1H), 7.80 (d, J=7.98 Hz, 1H), 7.52 (d, J=1.56 Hz, 1H), 7.33-7.35 (m, 2H), 7.28-7.30 (m, 2H), 7.18 (dd, J=8.59, 1.59 Hz, 1H), 6.93 (d, J=8.59 Hz, 1H), 4.03 (s, 3H), 3.63 (t, J=6.29 Hz, 2H), 3.42 (q, J=7.06 Hz, 2H), 2.48-2.52 (m, 2H), 1.09 (t, J=7.06 Hz, 1H).

### Example 146

### (2R)N[3-(3-methoxy-4-nitrophenyl)-11-oxo-10,11-dihydro-5H-dibenzo[b,e][1,4]diazepin-8-yl]-5-oxopyrrolidine-2-carboxamide

The title compound was prepared by substituting (R) 5-oxo-pyrrolidine-2-carboxylic acid for dimethylaminoacetic acid in Example 122. MS (DCI) m/e 488 (M+H)⁺; ¹H NMR (300 MHz, DMSO-d₆) δ 9.97 (s, 1H), 9.93 (s, 1H), 8.01 (d, J=8.42 Hz, 1H), 7.94 (s, 1H), 7.84 (s, 1H), 7.80 (d, J=8.42 Hz, 1H), 7.52 (d, J=1.56 Hz, 1H), 7.34-7.35 (m, 2H), 7.28-7.30 (m, 2H), 7.22 (dd, J=8.73, 2.18 Hz, 1H), 6.95 (d, J=8.74 Hz, 1H), 4.16 (m, 1H), 4.03 (s, 3H), 1.95-2.32 (m, 4H).

### Example 147

### 4-methoxyN[3-(3-methoxy-4-nitrophenyl)-11-oxo-10,11-dihydro-5H-dibenzo[b,e][1,4]diazepin-8-yl]cyclohexanecarboxamide

The title compound was prepared by substituting 4-methoxy-cyclohexanecarboxylic acid for dimethylaminoacetic acid in Example 122. MS (DCI) m/e 517 (M+H)⁺; ¹H NMR (300 MHz, DMSO-d₆) δ 9.90 (s, 1H), 9.74 (s, 1H), 8.00 (d, J=8.42 Hz, 1H), 7.89 (s, 1H), 7.79 (d, J=8.42 Hz, 1H), 7.52 (d, J=1.56 Hz, 1H), 7.33-7.35 (m, 2H), 7.28-7.31 (m, 2H), 7.17 (d, J=8.73 Hz, 1H), 6.91 (d, J=8.73 Hz, 1H), 4.03 (s, 3H), 3.21-3.24 (m, 4H), 0.95-2.26 (m, 8H).

### Example 148

### (2R)-2-methoxyN[3-(3-methoxy-4-nitrophenyl)-11-oxo-10,11-dihydro-5H-dibenzo[b,e][1,4]diazepin-8-yl]-2-phenylacetamide

The title compound was prepared by substituting (R) methoxy-phenyl-acetic acid for dimethylaminoacetic acid in Example 122. MS (DCI) m/e 525 (M+H)⁺; ¹H NMR (300 MHz, DMSO-d₆) δ 9.93 (s, 1H), 9.89 (s, 1H), 8.00 (d, J=8.42 Hz, 1H), 7.93 (s, 1H), 7.79 (d, J=8.11 Hz, 1H), 7.52 (s, 1H), 7.45-7.47 (m, 2H), 7.33-7.39 (m, 6H), 7.29 (dd, J=8.11, 1.56 Hz, 1H), 7.22 (dd, J=8.73, 2.18 Hz, 1H), 6.94 (d, J=8.42 Hz, 1H), 4.81 (s, 1H), 4.03 (s, 3H), 3.55 (s, 3H).

### Example 149

### (2S)-2-methoxyN[3-(3-methoxy-4-nitrophenyl)-11-oxo-10,11-dihydro-5H-dibenzo[b,e][1,4]diazepin-8-yl]-2-phenylacetamide

The title compound was prepared by substituting (R) methoxy-phenyl-acetic acid for dimethylaminoacetic acid in Example 122. MS (DCI) m/e 525 (M+H)⁺; ¹H NMR (300 MHz, DMSO-d₆) δ 9.93 (s, 1H), 9.89 (s, 1H), 8.00 (d, J=8.42 Hz, 1H), 7.93 (s, 1H), 7.79 (d, J=8.11 Hz, 1H), 7.52 (s, 1H), 7.45-7.47 (m, 2H), 7.33-7.39 (m, 6H), 7.29 (dd, J=8.11, 1.56 Hz, 1H), 7.22 (dd, J=8.73, 2.18 Hz, 1H), 6.94 (d, J=8.42 Hz, 1H), 4.81 (s, 1H), 4.03 (s, 3H), 3.55 (s, 3H).

### Example 150

### N-(2-{[3-(3-methoxy-4-nitrophenyl)-11-oxo-10;11-dihydro-5H-dibenzo[b,e][1,4]diazepin-8-yl]amino}-2-oxoethyl)-2-furamide

The title compound was prepared by substituting [(furan-2-carbonyl)-amino]-acetic acid for dimethylaminoacetic acid in Example 122. MS (DCI) m/e 528 (M+H)⁺; ¹H NMR (300 MHz, DMSO-d₆) δ 9.97 (s, 1H), 9.93 (s, 1H), 8.55 (t, J=5.93 Hz, 1H), 8.01 (d, J=8.42 Hz, 1H), 7.93 (s, 1H), 7.79 (d, J=8.11 Hz, 1H), 7.53 (d, J=1.56 Hz, 1H), 7.32-7.35 (m, 2H), 7.26-7.30 (m, 2H), 7.19 (dd, J=8.42, 2.18 Hz, 1H), 7.14 (d, J=2.18 Hz, 1H), 6.95 (d, J=8.73 Hz, 1H), 6.64 (dd, J=3.43, 1.56 Hz, 1H), 4.81 (s, 1H), 4.03 (s, 3H), 3.98 (d, J=5.93 Hz, 2H).

### Example 151

### 1-acetylN[3-(3-methoxy-4-nitrophenyl)-11-oxo-10,11-dihydro-5H-dibenzo[b,e][1,4]diazepin-8-yl]piperidine-4-carboxamide

The title compound was prepared by substituting 1-acetyl-piperidine-4-carboxylic acid for dimethylaminoacetic acid in Example 122. MS (DCI) m/e 530 (M+H)⁺; ¹H NMR (300 MHz, DMSO-d₆) δ 9.92 (s, 1H), 9.81 (s, 1H), 8.01 (d, J=8.42 Hz, 1H), 7.91 (s, 1H), 7.79 (d, J=8.11 Hz, 1H), 7.53 (d, J=1.56 Hz, 1H), 7.33-7.35 (m, 2H), 7.27-7.30 (m, 2H), 7.19 (dd, J=8.73, 2.18 Hz, 1H), 6.93 (d, J=8.42 Hz, 1H), 4.38 (m, 1H), 4.03 (s, 3H), 3.84 (m, 1H), 3.05 (m, 1H), 2.50-2.56 (m, 2H), 2.00 (s, 3H), 2.72.78 (m, 2H), 1.56-1.57 (m, 1H), 1.43 (m, 1H).

### Example 152

### N-[3-(3-methoxy-4-nitrophenyl)-11-oxo-10,11-dihydro-5H-dibenzo[b,e][1,4]diazepin-8-yl]-N'-phenylpentanediamide

The title compound was prepared by substituting 5-anilino-5-oxopentanoic acid for dimethylaminoacetic acid in Example 122. MS (DCI) m/e 566 (M+H)⁺; ¹H NMR (300 MHz, DMSO-d₆) δ 9.93 (s, 1H), 9.86 (s, 1H), 9.80 (s, 1H), 8.01 (d, J=8.42 Hz, 1H), 7.90 (s, 1H), 7.80 (d, J=8.11 Hz, 1H), 7.59 (d, J=7.70 Hz, 2H), 7.52 (d, J=1.56 Hz, 1H), 7.33-7.35 (m, 2H), 7.26-7.30 (m, 4H), 7.18-7.20 (m, 1H), 7.01 (t, J=7.33 Hz, 1H), 6.93 (d, J=8.42 Hz, 1H), 4.03 (s, 3H), 2.32-2.37 (m, 4H), 1.87-1.90 (m, 2H).

### Example 153

### N-[3-(3-methoxy-4-nitrophenyl)-11-oxo-10,11-dihydro-5H-dibenzo[b,e][1,4]diazepin-8-yl]-2-[4-(methylsulfonyl)phenyl]acetamide

The title compound was prepared by substituting (4-methanesulfonyl-phenyl)-acetic acid for dimethylaminoacetic acid in Example 122. MS (DCI) m/e 573 (M+H)⁺; ¹H NMR (300 MHz, DMSO-d₆) δ 10.14 (s, 1H), 9.94 (s, 1H), 8.00 (d, J=8.42 Hz, 1H), 7.93 (s, 1H), 7.88 (d, J=8.42 Hz, 2H), 7.79 (d, J=8.42 Hz, 1H), 7.59 (d, J=8.11 Hz, 2H), 7.52 (d, J=1.56 Hz, 1H), 7.33-7.35 (m, 2H), 7.26-7.30 (m, 2H), 7.19 (dd, J=8.73, 2.18 Hz, 1H), 6.93 (d, J=8.73 Hz, 1H), 4.03 (s, 3H), 3.75 (s, 2H), 3.19 (s, 3H).

### Example 154

### (2S)N[3-(3-methoxy-4-nitrophenyl)-11-oxo-10,11-dihydro-5H-dibenzo[b,e][1,4]diazepin-8-yl]-5-oxopyrrolidine-2-carboxamide

The title compound was prepared by substituting (S) 5-oxo-pyrrolidine-2-carboxylic acid for dimethylaminoacetic acid in Example 122. MS (DCI) m/e 488 (M+H)⁺; ¹H NMR (300 MHz, DMSO-d₆) δ 9.97 (s, 1H), 9.93 (s, 1H), 8.01 (d, J=8.42 Hz, 1H), 7.94 (s, 1H), 7.84 (s, 1H), 7.80 (d, J=8.42 Hz, 1H), 7.52 (d, J=1.56 Hz, 1H), 7.34-7.35 (m, 2H), 7.28-7.30 (m, 2H), 7.22 (dd, J=8.73, 2.18 Hz, 1H), 6.95 (d, J=8.74 Hz, 1H), 4.15 (m, 1H), 4.03 (s, 3H), 1.95-2.32 (m, 4H).

### Example 155

### 4-(8-amino-11-oxo-10,11-dihydro-5H-dibenzo[b,e][1,4]diazepin-3-yl)-2-methoxybenzonitrile

### Example 155A

### 8-amino-3-(4,4,5,5-tetramethyl-1,3,2-dioxaborolan-2-yl)-5,10-dihydro-11H-dibenzo[b,e][1,4]diazepin-11-one

The title compound was prepared by substituting Example 7C for Example 6D in Example 54A. MS (DCI) m/e 352 (M+H)⁺.

### Example 155B

### 4-(8-amino-11-oxo-10,11-dihydro-5H-dibenzo[b,e][1,4]diazepin-3-yl)-2-methoxybenzonitrile

The title compound was prepared by substituting Example 155A and 4-iodo-2-methoxy-benzonitrile for 2-methoxy-4-(4,4,5,5-tetramethyl-1,3,2-dioxaborolan-2-yl)phenol and Example 9, respectively, in Example 10. MS (DCI) m/e 357 (M+H)⁺; ¹H NMR (500 MHz, DMSO-d₆) δ 9.94 (s, 1H), 7.87 (s, 1H), 7.84 (d, J=8.11 Hz, 1H), 7.80 (d, J=8.11 Hz, 1H), 7.42 (s, 1H), 7.32-7.34 (m, 2H), 7.28 (d, J=8.11 Hz, 1H), 6.92 (d, J=8.42 Hz, 1H), 6.60-6.64 (m, 2H), 4.02 (s, 3H).

### Example 156

### (2S)N[3-(4-cyano-3-methoxyphenyl)-11-oxo-10,11-dihydro-5H-dibenzo[b,e][1,4]diazepin-8-yl]-2-methoxy-2-phenylacetamide

The title compound was prepared by substituting Example 155B and (2S)-methoxy(phenyl)acetic acid for Example 120 and dimethylaminoacetic acid, respectively, in Example 122. MS (DCI) m/e 505 (M+H)⁺; ¹H NMR (500 MHz, DMSO-d₆) δ 9.93 (s, 1H), 9.88 (s, 1H), 7.91 (s, 1H), 7.83 (d, J=8.11 Hz, 1H), 7.78 (d, J=8.11 Hz, 1H), 7.47 (d, J=7.18 Hz, 1H), 7.42 (s, 1H), 7.31-7.39 (m, 6H), 7.27 (dd, J=8.27, 1.72 Hz, 1H), 7.22 (dd, J=8.58, 2.34 Hz, 1H), 6.93 (d, J=8.74 Hz, 1H), 4.81 (s, 1H), 4.02(s, 3H), 3.35 (s, 3H).

### Example 157

### N-[3-(4-cyano-3-methoxyphenyl)-11-oxo-10,11-dihydro-5H-dibenzo[b,e][1,4]diazepin-8-yl]-3-piperidin-1-ylpropanamide

The title compound was prepared by substituting Example 155B and 3-piperidine-1-ylpropionic acid for Example 120 and dimethylaminoacetic acid, respectively, in Example 122. MS (DCI) m/e 496 (M+H)⁺; ¹H NMR (500 MHz, DMSO-d₆) δ 10.01 (s, 1H), 9.94 (s, 1H), 7.89 (s, 1H), 7.83 (d, J=8.11 Hz, 1H), 7.79 (d, J=8.11 Hz, 1H), 7.42 (s, 1H), 7.32-7.34 (m, 2H), 7.27 (dd, J=8.27, 1.40 Hz, 1H), 7.24 (d, J=1.87 Hz, 1H), 7.19 (dd, J=8.58, 2.03Hz, 1H), 6.93 (d, J=8.42 Hz, 1H), 4.02 (s, 3H), 2.56-2.58 (m, 2H), 2.35-2.43 (m, 6H), 1.50-1.52 (m, 4H), 1.38-1.40 (m, 2H).

### Example 158

### N-[3-(4-cyano-3-methoxyphenyl)-11-oxo-10,11-dihydro-5H-dibenzo[b,e][1,4]diazepin-8-yl]methanesulfonamide

The title compound was prepared by substituting Example 155B for Example 7C in Example 42A. MS (DCI) m/e 435 (M+H)⁺; ¹H NMR (500 MHz, DMSO-d₆) δ 9.94 (s, 1H), 9.49 (s, 1H), 7.96 (s, 1H), 7.84 (d, J=7.98 Hz, 1H), 7.79 (d, J=7.98 Hz, 1H), 7.42 (s, is), 7.28-7.34 (m, 3H), 6.98 (d, J=8.59 Hz, 1H), 6.93 (d, J=1.84 Hz, 1H), 6.82 (m, 1H), 4.02 (s, 3H), 2.92 (s, 3H).

### Example 159

### 8-amino-3-(4-chloro-3-methoxyphenyl)-5,10-dihydro-11H-dibenzo[b,e][1,4]diazepin-11-one

The title compound was prepared by substituting Example 155A and 1-chloro-4-iodo-2-methoxy-benzene for 2-methoxy-4-(4,4,5,5-tetramethyl-1,3,2-dioxaborolan-2-yl)phenol and Example 9, respectively, in Example 10. MS (DCI) m/e 366 (M+H)⁺; ¹H NMR (500 MHz, DMSO-d₆) δ 9.95 (s, 1H), 7.92 (s, 1H), 7.77 (d, J=7.98 Hz, 1H), 7.53 (d, J=8.24 Hz, 1H), 7.34 (d, J=1.56 Hz, 1H), 7.29 (s, 1H), 7.19-7.24 (m, 2H), 6.96 (d, J=8.29 Hz, 1H), 6.68-6.72 (m, 2H), 3.96 (s, 3H).

### Example 160

### N-[3-(4-chloro-3-methoxyphenyl)-11-oxo-10,11-dihydro-5H-dibenzo[b,e][1,4]diazepin-8-yl]-3-piperidin-1-ylpropanamide

The title compound was prepared by substituting Example 159 and 3-piperidin-1-ylpropionic acid for Example 120 and dimethylaminoacetic acid, respectively, in Example 122. MS (DCI) m/e 506 (M+H)⁺; ¹H NMR (500 MHz, DMSO-d₆) δ 10.10 (s, 1H), 9.89 (s, 1H), 7.85 (s, 1H), 7.76 (d, J=8.29 Hz, 1H), 7.34 (d, J=1.53 Hz, 1H), 7.28 (s, 1H), 7.17-7.23 (m, 5H), 6.92 (d, J=8.29 Hz, 1H), 3.96 (s, 3H), 2.57 (m, 2H), 2.35-2.42 (m, 6H), 1.49-1.51 (m, 4H), 1.38-1.40 (m, 2H).

### Example 161

### 3-(3-methoxy-4-nitrophenyl)-8-(2-oxypyrrolidin-1-yl)-5,10-dihydro-11H-dibenzo[b,e][1,4]diazepin-11-one

### Example 161A

### 4-chloroN(3-chloro-11-oxo-10,11-dihydro-5H-dibenzo[b,e][1,4]diazepin-8-yl)butanamide

The title compound was prepared by substituting 4-chloro-butyryl chloride for CH₃SO₂Cl in Example 42A. MS (DCI) m/e 365 (M+H)⁺; ¹H NMR (500 MHz, DMSO-d₆) δ 9.95 (s, 1H), 9.88 (s, 1H), 7.95 (s, 1H), 7.67 (d, J=8.48 Hz, 1H), 7.27 (d, J=2.03 Hz, 1H), 7.18 (dd, J=8.48, 2.37 Hz, 1H), 7.05 (d, J=2.03 Hz, 1H), 6.87-6.93 (m, 2H), 3.68 (t, J=6.61 Hz, 2H), 2.43 (t, J=7.29 Hz, 2 H), 1.97-2.05 (m, 2H).

### Example 161B

### 3-chloro-8-(2-oxopyrrolidin-1-yl)-5,10-dihydro-11H-dibenzo[b,e][1,4]diazenin-11-one

Sodium (0.138 g, 6 mmol) was dissolved in 15 mL of anhydrous EtOH at 0 °C. To this solution was added Example 161A. The solution was stirred overnight, and the precipitate was collected by filtration to give 0.154 g of the title compound. MS (DCI) m/e 328 (M+H)⁺; ¹H NMR (500 MHz, DMSO-d₆) δ 9.93 (s, 1H), 8.06 (s, 1H), 7.72 (d, J=8.48 Hz, 1H), 7.37 (d, J=2.50 Hz, 1H), 7.28 (dd, J=8.73, 2.50 Hz, 1H), 7.09 (d, J=1.87 Hz, 1H), 6.98 (d, J=8.73 Hz, 1H), 6.95 (dd, J=8.42, 1.87 Hz, 1H), 3.77 (t, J=7.02 Hz, 2H), 2.49 (t, J=7.95 Hz, 2 H), 2.04-2.10 (m, 2H).

### Example 161C

### 3-(3-methoxy-4-nitrophenyl)-8-(2-oxopyrrolidin-1-yl)-5,10-dihydro-11H-dibenzo[b,e][1,4]diazepin-11-one

The title compound was prepared by substituting Example 161B and Example 266G for Example 59B and Example 56A, respectively, in Example 59C. MS (DCI) m/e 445 (M+H)⁺; ¹H NMR (500 MHz, DMSO-d₆) δ 9.90 (s, 1H), 8.00-8.02 (m, 2H), 7.81 (d, J=8.29 Hz, 1H), 7.63 (d, J=1.53 Hz, 1H), 7.32-7.35 (m, 3H), 7.28-7.31 (m, 1H), 7.25 (dd, J=8.75, 2.30 Hz, 1H), 7.00 (d, J=8.90 Hz, 1H), 4.03 (s, 3H), 3.74 (t, J=6.90 Hz, 2H), 2.46 (t, J=8.13 Hz, 2 H), 2.01-2.08 (m, 2H).

### Example 162

### 3-(3-methoxy-4-nitrophenyl)-8-(2-oxopiperidin-1-yl)-5,10-dihydro-11H-dibenzo[b,e][1,4]diazepin-11-one

### Example 162A

### 5-chloroN(3-chloro-11-oxo-10,11-dihydro-5H-dibenzo[b,e][1,4]diazepin-8-yl)pentanamide

The title compound was prepared by substituting 5-chloro-pentanoyl chloride for CH₃SO₂Cl in Example 42A. MS (DCI) m/e 379 (M+H)⁺; ¹H NMR (500 MHz, DMSO-d₆) δ 9.93 (s, 1H), 9.80 (s, 1H), 7.94 (s, 1H), 7.68 (d, J=8.73 Hz, 1H), 7.28 (d, J=1.87 Hz, 1H), 7.18 (dd, J=8.58, 2.03 Hz, 1H), 7.06 (d, J=1.87 Hz, 1H), 6.88-6.92 (m, 2H), 3.62-3.67 (m, 2H), 2.24-2.32 (m, 2 H), 1.61-1.76 (m, 4H).

### Example 162B

### 3-chloro-8-(2-oxopiperidin-1-yl)-5,10-dihydro-11H-dibenzo[b,e][1,4]diazepin-11-one

Sodium (0.200 g, 8.7 mmol) was dissolved in 25 mL of anhydrous EtOH at 0 °C. To this solution was added Example 162A. The solution was heated under reflux for 1 hour. The solution was cooled to room temperature and the precipitate was collected by filtration to give 0.196 g of the title compound. MS (DCI) m/e 342 (M+H)⁺; ¹H NMR (500 MHz, DMSO-d₆) δ 9.88 (s, 1H), 8.07 (s, 1H), 7.67 (d, J=8.42 Hz, 1H), 7.07 (d, J=1.87 Hz, 1H), 6.92-6.96 (m, 2H), 6.85-6.88 (m, 2H), 3.50 (t, J=5.61 Hz, 2H), 2.35 (t, J=6.24 Hz, 2 H), 1.81-1.86 (m, 4H).

### Example 162C

### 3-(3-methoxy-4-nitrophenyl)-8-(2-oxopiperidin-1-yl)-5,10-dihydro-11H-dibenzo[b,e][1,4]diazepin-11-one

The title compound was prepared by substituting Example 162B and Example 266G for Example 59B and Example 56A, respectively, in Example 59C. MS (DCI) m/e 459 (M+H)⁺; ¹H NMR (500 MHz, DMSO-d₆) δ 9.88 (s, 1H), 8.05(s, 1H), 8.01 (d, J=8.29 Hz, 1H), 7.81 (d, J=7.98 Hz, 1H), 7.53 (s, 1H), 7.30-7.36 (m, 3H), 7.00 (m, 1H), 6.86-6.88 (m, 2H), 4.03 (s, 3H), 3.51 (t, J=5.37 Hz, 2H), 2.36 (t, J=6.14 Hz, 2 H), 1.79-1.83 (m, 4H).

### Example 163

### 3-(4-chloro-3-methoxyphenyl)-8-(2-oxopyrrolidin-1-yl)-5,10-dihydro-11H-dibenzo[b,e][1,4]diazepin-11-one

### Example 163A

### 4-chloroN[3-(4-chloro-3-methoxyphenyl)-11-oxo-10,11-dihydro-5H-dibenzo[b,e][1,4]diazepin-8-yl]butanamide

The title compound was prepared by substituting Example 159 and 5-chloro-pentanoyl chloride for Example 7C and CH₃SO₂Cl, respectively, in Example 42A. MS (DCI) m/e 471 (M+H)⁺; ¹H NMR (500 MHz, DMSO-d₆) δ 9.88 (s, 1H), 9.85 (s, 1H), 7.85 (s, 1H), 7.76 (d, J=8.11 Hz, 1H), 7.52 (d, J=8.42 Hz, 1H), 7.34 (s, 1H), 7.28 (d, J=4.37 Hz, 1H), 7.17-7.23 (m, 5H), 6.93 (d, J=8.73 Hz, 1H), 3.62-3.67 (m, 2H), 2.24-2.32 (m, 2 H), 1.61-1.76 (m, 2H).

### Example 163B

### 3-(4-chloro-3-methoxyphenyl)-8-(2-oxopyrrolidin-1-yl)-5,10-dihydro-11H-dibenzo[b,e][1,4]diazepin-11-one

The title compound was prepared by substituting Example 163A for Example 162A in Example 162B. MS (DCI) m/e 434 (M+H)⁺; ¹H NMR (500 MHz, DMSO-d₆) δ 9.84 (s, 1H), 7.94 (s, 1H), 7.77 (d, J=8.29 Hz, 1H), 7.53 (d, J=8.29 Hz, 1H), 7.32-7.35 (m, 2H), 7.29 (d, J=1.84 Hz, 1H), 7.20-7.25 (m, 3H), 6.99 (d, J=8.59 Hz, 1H), 3.96 (s, 3H), 3.74 (t, J=6.90 Hz, 2H), 2.46 (t, J=8.13 Hz, 2 H), 2.00-2.08 (m, 2H).

### Example 164

### 3-chloroN[3-(4-chloro-3-methoxyphenyl)-11-oxo-10,11-dihydro-5H-dibenzo[b,e][1,4]diazepin-8-yl]propane-1-sulfonamide

The title compound was prepared by substituting Example 159 and 3-chloro-propane-1-sulfonyl chloride for Example 7C and CH₃SO₂Cl, respectively, in Example 42A. MS (DCI) m/e 507 (M+H)⁺; ¹H NMR (500 MHz, DMSO-d₆) δ 9.91 (s, 1H), 9.66 (s, 1H), 7.92 (s, 1H), 7.76 (d, J=8.29 Hz, 1H), 7.53 (d, J=8.29 Hz, 1H), 7.33 (d, J=1.84, Hz, 1H), 7.29 (d, J=1.84 Hz, 1H), 7.19-7.24 (m, 2H), 6.97 (d, J=8.29 Hz, 1H), 6.93 (d, J=2.76 Hz, 1H), 6.82 (dd, J=8.59, 2.59 Hz, 1H), 3.96 (s, 3H), 3.71 (t, J=6.60 Hz, 2H), 3.13-3.17 (m, 2 H), 2.08-2.12 (m, 2H).

### Example 165

### N-[3-(4-chloro-3-methoxyphenyl)-11-oxo-10,11-dihydro-5H-dibenzo[b,e][1,4]diazepin-8-yl]-1-phenylmethanesulfonamide

The title compound was prepared by substituting Example 159 and phenylmethanesulfonyl chloride for Example 7C and CH₃SO₂Cl, respectively, in Example 42A. MS (DCI) m/e 521 (M+H)⁺; ¹H NMR (500 MHz, DMSO-d₆) δ 9.93 (s, 1H), 9.64 (s, 1H), 7.92 (s, 1H), 7.78 (d, J=8.29 Hz, 1H), 7.53 (d, J=8.29 Hz, 1H), 7.33-7.37 (m, 4H), 7.20-7.30 (m, 5H), 6.96-6.99 (m, 2H), 6.80 (dd, J=8.59, 2.45 Hz, 1H), 4.38 (s, 2H), 3.96 (s, 3H).

### Example 166

### 3-(4-chloro-3-methoxyphenyl)-8-(1,1-dioxidoisothiazolidin-2-yl)-5,10-dihydro-11H-dibenzo[b,e][1,4]diazepin-11-one

The title compound was prepared by substituting Example 164 for Example 162A in Example 162B. MS (DCI) m/e 470 (M+H)⁺; ¹H NMR (500 MHz, DMSO-d₆) δ 9.91 (s, 1H), 7.97 (s, 1H), 7.77 (d, J=7.93 Hz, 1H), 7.53 (d, J=8.24 Hz, 1H), 7.34 (d, J=1.83, 1H), 7.30 (d, J=1.22 Hz, 1H), 7.20-7.25 (m, 2H), 7.02 (d, J=8.54, Hz, 1H), 6.91 (d, J=2.44 Hz, 1H), 6.87 (dd, J=8.54, 2.44 Hz, 1H), 3.96 (s, 3H), 3.63 (t, J=6.56 Hz, 2H), 3.46 (t, J=7.48 Hz, 2H), 2.35-2.40 (m, 2H).

### Example 167

### 2,2,2-trifluoroN[3-(3-methoxy-4-nitrophenyl)-11-oxo-10,11-dihydro-5H-dibenzo[b,e][1,4]diazepin-8-yl]ethanesulfonamide

The title compound was prepared by substituting Example 120 and 2,2,2-trifluoroethanesulfonyl chloride for Example 7C and CH₃SO₂Cl, respectively, in Example 42A. MS (DCI) m/e 523 (M+H)⁺; ¹H NMR (500 MHz, DMSO-d₆) δ 9.91 (s, 1H), 7.98-8.02 (m, 2H), 7.80 (d, J=8.11 Hz, 1H), 7.52 (s, 1H), 7.29-7.35 (m, 4H), 6.97 (d, J=8.42 Hz, 1H), 6.91 (s, 1H), 6.81 (d, J=8.42 Hz, 1H), 4.31-4.33 (m, 2H), 4.03 (s, 3H).

### Example 168

### N-[3-(3-methoxy-4-nitrophenyl)-11-oxo-10,11-dihydro-5H-dibenzo[b,e][1,4]diazepin-8-yl]-1-methyl-1H-imidazole-4-sulfonamide

The title compound was prepared by substituting Example 120 and 1-methyl-1H-imidazole-4-sulfonyl chloride for Example 7C and CH₃SO₂Cl, respectively, in Example 42A. MS (DCI) m/e 521 (M+H)⁺; ¹H NMR (500 MHz, DMSO-d₆) δ 9.99 (s, 1H), 9.91 (s, 1H), 8.00 (d, J=8.42 Hz, 1H), 7.87 (s, 1H), 7.75-7.78 (m, 2H), 7.71 (s, 1H), 7.51 (J=1.56 Hz, 1H), 7.31-7.35 (m, 2H), 7.28 (dd, J=8.11, 1.87 Hz, 1H), 6.84-6.86 (m, 2H), 6.73 (d, J=8.58, 2.34 Hz, 1H), 4.02 (s, 3H), 3.64 (s, 3H).

### Example 169

### N-[3-(3-methoxy-4-nitrophenyl)-11-oxo-10,11-dihydro-5H-dibenzo[b,e][1,4]diazepin-8-yl]-1-phenylmethanesulfonamide

The title compound was prepared by substituting Example 120 and phenylmethanesulfonyl chloride for Example 7C and CH₃SO₂Cl, respectively, in Example 42A. MS (DCI) m/e 531 (M+H)⁺; ¹H NMR (500 MHz, DMSO-d₆) δ 9.97 (s, 1H), 9.64 (s, 1H), 8.01 (d, J=8.29 Hz, 1H), 7.97 (s, 1H), 7.81 (d, J=8.29 Hz, 1H), 7.53 (d, J=1.84 Hz, 1H), 7.27-7.38 (m, 8H), 6.97-6.99 (m, 2H), 6.80 (dd, J=8.29, 2.46 Hz, 1H), 4.38 (s, 2H), 4.03 (s, 3H).

### Example 170

### 3-chloroN[3-(3-methoxy-4-nitrophenyl)-11-oxo-10,11-dihydro-5H-dibenzo[b,e][1,4]diazepin-8-yl]propane-1-sulfonamide

The title compound was prepared by substituting Example 120 and 3-chloropropane-1-sulfonyl chloride for Example 7C and CH₃SO₂Cl, respectively, in Example 42A. MS (DCI) m/e 517 (M+H)⁺; ¹H NMR (500 MHz, DMSO-d₆) δ 9.98 (s, 1H), 9.70 (s, 1H), 8.01-8.02 (m, 2H), 7.80 (d, J=8.24 Hz, 1H), 7.52 (s, 1H), 7.30-7.35 (m, 3H), 6.98 (d, J=8.24 Hz, 1H), 6.94 (d, J=2.14 Hz, 1H), 6.83 (dd, J=8.54, 2.44 Hz, 1H), 4.03 (s, 3H), 3.71 (t, J=3.71 Hz, 2H), 3.13-3.15 (m, 2H), 2.06-2.13 (m, 2H).

### Example 171

### 8-(1,1-dioxidoisothiazolidin-2-yl)-3-(3-methoxy-4-nitrophenyl)-5,10-dihydro-11H-dibenzo[b,e][1,4]diazepin-11-one

The title compound was prepared by substituting Example 170 for Example 162A in Example 162B. MS (DCI) m/e 481 (M+H)⁺; ¹H NMR (500 MHz, DMSO-d₆) δ 9.93 (s, 1H), 8.00-8.02 (m, 2H), 7.81 (d, J=8.42 Hz, 1H), 7.53 (d, J=1.56 Hz, 1H), 7.34-7.35 (m, 2H), 7.30 (dd, J=8.26, 1.72 Hz, 1H), 7.02 (d, J=8.42, Hz, 1H), 6.92 (d, J=2.18 Hz, 1H), 6.88 (dd, J=8.42, 2.50 Hz, 1H), 4.03 (s, 3H), 3.63 (t, J=6.39 Hz, 2H), 3.46 (t, J=7.49 Hz, 2H), 2.34-2.41 (m, 2H).

### Example172

### N-[3-(3-methoxy-4-nitrophenyl)-11-oxo-10,11-dihydro-5H-dibenzo[b,e][1,4]diazepin-8-yl]-3-morpholin-4-ylpropane-1-sulfonamide

A mixture of Example 170 (10.0 mg, 0.0168 mmol), morpholine (9.0 mmg, 0.112 mmol) in 5 mL of toluene and 1 mL of dioxane was heated under reflux overnight. The solvents were removed under reduced pressure, and residue was purified by preparative HPLC (CH₃CN / 0.1% TFA in H₂O) to give the title compound. MS (DCI) m/e 568 (M+H)⁺; ¹H NMR (500 MHz, DMSO-d₆) δ 9.97 (s, 1H), 9.72 (s, 1H), 8.01-8.03 (m, 2H), 7.81 (d, J=8.42 Hz, 1H), 7.51 (d, J=1.56 Hz, 1H), 7.30-7.38 (m, 3H), 7.00 (d, J=8.42, Hz, 1H), 6.94 (d, J=2.50, Hz, 1H), 6.84 (dd, J=8.58, 2.34 Hz, 1H), 4.03 (s, 3H), 3.88-3.92 (m, 2H), 3.63-3.70 (m, 4H), 3.00-3.20 (m, 6H), 2.03-2.05 (m, 2H).

### Example 173

### N-[3-(3-methoxy-4-nitrophenyl)-11-oxo-10,11-dihydro-5H-dibenzo[b,e][1,4]diazepin-8-yl]-3-piperidin-1-ylpropane-1-sulfonamide

The title compound was prepared by substituting piperidine for morpholine in Example 172. MS (DCI) m/e 566 (M+H)⁺; ¹H NMR (500 MHz, DMSO-d₆) δ 9.97 (s, 1H), 9.71 (s, 1H), 9.07 (br, 1H), 8.01-8.03 (m, 2H), 7.80 (d, J=8.11 Hz, 1H), 7.51 (s, 1H), 7.31-7.38 (m, 3H), 7.00 (d, J=8.42, Hz, 1H), 6.95 (d, J=2.18, Hz, 1H), 6.84 (dd, J=8.73, 2.18 Hz, 1H), 4.03 (s, 3H), 3.12-3.15 (m, 4H), 2.79-2.84 (m, 2H), 1.99-2.05 (m, 2H), 1.75-1.78 (m, 2H), 1.33-1.60 (m, 4H).

### Example 174

### 3-(diethylamino)N[3-(3-methoxy-4-nitrophenyl)-11-oxo-10,11-dihydro-5H-dibenzo[b,e][1,4]diazepin-8-yl]propane-1-sulfonamide

The title compound was prepared by substituting diethylamine for morpholine in Example 172. MS (DCI) m/e 554 (M+H)⁺; ¹H NMR (500 MHz, DMSO-d₆) δ 9.98 (s, 1H), 9.72 (s, 1H), 9.15 (br, 1H), 8.00-8.02 (m, 2H), 7.80 (d, J=7.98 Hz, 1H), 7.52 (d, J=1.84 Hz, 1H), 7.30-7.36 (m, 3H), 7.00 (d, J=8.59, Hz, 1H), 6.95 (d, J=2.45, Hz, 1H), 6.84 (dd, J=8.44, 2.30 Hz, 1H), 4.03 (s, 3H), 3.06-3.17 (m, 8H), 1.96-2.01 (m, 2H), 1.15 (t, J=7.36 Hz, 6H).

### Example 175

### 3-(dimethylamino)N[3-(3-methoxy-4-nitrophenyl)-11-oxo-10,11-dihydro-5H-dibenzo[b,e][1,4]diazepin-8-yl]propane-1-sulfonamide

The title compound was prepared by substituting dimethylamine for morpholine in Example 172. MS (DCI) m/e 526 (M+H)⁺; ¹H NMR (500 MHz, DMSO-d₆) δ 9.97 (s, 1H), 9.72 (s, 1H), 9.43 (br, 1H), 8.00-8.02 (m, 2H), 7.80 (d, J=8.29 Hz, 1H), 7.52 (d, J=1.84 Hz, 1H), 7.30-7.36 (m, 3H), 7.00 (d, J=8.59, Hz, 1H), 6.94 (d, J=2.46, Hz, 1H), 6.84 (dd, J=8.59, 2.46 Hz, 1H), 4.03 (s, 3H), 3.06-3.17 (m, 4H), 1.96-2.03 (m, 2H).

### Example 176

### 1-chloroN[3-(3-methoxy-4-nitrophenyl)-11-oxo-10,11-dihydro-5H-dibenzo[b,e][1,4]diazepin-8-yl]methanesulfonamide

The title compound was prepared by substituting Example 120 and chloro-methanesulfonyl chloride for Example 7C and CH₃SO₂Cl, respectively, in Example 42A. MS (DCI) m/e 489 (M+H)⁺; ¹H NMR (500 MHz, DMSO-d₆) δ 10.18 (s, 1H), 9.94 (s, 1H), 8.00-8.02 (m, 2H), 7.80 (d, J=8.11 Hz, 1H), 7.52 (d, J=1.56 Hz, 1H), 7.30-7.35 (m, 3H), 6.99 (d, J=8.42 Hz, 1H), 6.95 (d, J=2.18 Hz, 1H), 6.85 (dd, J=8.58, 2.34 Hz, 1H), 4.91 (s, 2H), 4.03 (s, 3H).

### Example 177

### N-[3-(3-methoxy-4-nitrophenyl)-11-oxo-10,11-dihydro-5H-dibenzo[b,e][1,4]diazepin-8-yl]-4-morpholin-4-ylbenzamide

The title compound was prepared by substituting 4-morpholin-4-ylbenzoic acid for dimethylaminoacetic acid in Example 122. MS (DCI) m/e 566 (M+H)⁺; ¹H NMR (500 MHz, DMSO-d₆) δ 9.96 (s, 1H), 9.88 (s, 1H), 8.01 (d, J=8.42 Hz, 1H), 7.94 (s, 1H), 7.86 (d, J=8.73 Hz, 2H), 7.81 (d, J=8.11 Hz, 1H), 7.53 (d, J=1.56 Hz, 1H), 7.48 (d, J=2.50 Hz, 1H), 7.34-7.36 (m, 2H), 7.28-7.32 (m 2H), 7.01 (d, J=9.04 Hz, 2H), 6.97 (d, J=8.42 Hz, 1H), 4.04 (s, 3H), 3.74-3.76 (m, 4H), 3.24-3.27 (m, 4H).

### Example 178

### 7-amino-3-(3-methoxy-4-nitrophenyl)-5,10-dihydro-11H-dibenzo[b,e][1,4]diazepin-11-one

### Example 178A

### methyl 2-[(5-amino-2-nitrophenyl)amino]-4-chlorobenzoate

The title compound was prepared by substituting 4-nitro-benzene-1,3-diamine for 4-bromo-2-nitroaniline in Example 2A. MS (DCI) m/e 322 (M+H)⁺; ¹H NMR (300 MHz, DMSO-d₆) δ 11.11 (s, 1H), 7.97 (s, 1H), 7.94 (s, 1H), 7.61 (d, J=2.03 Hz, 1H), 7.16 (dd, J=8.48, 2.03 Hz, 1H), 6.70 (s, 2H), 6.61 (d, J=2.37 Hz, 1H), 6.28 (dd, J=9.32, 2.20 Hz, 1H), 3.87 (s, 3H).

### Example 178B

### methyl 4-chloro-2-[(2,5-diaminophenyl)amino]benzoate

The title compound was prepared by substituting Example 178A for Example 6B in Example 6C. MS (DCI) m/e 292 (M+H)⁺; ¹H NMR (300 MHz, DMSO-d₆) δ 8.83 (s, 1H), 7.84 (d, J=8.48 Hz, 1H), 6.69 (dd, J=8.65, 2.20 Hz, 1H), 6.61 (d, J=8.81 Hz, 1H), 6.57 (d, J=2.03 Hz, 1H), 6.35-6.38 (m, 2H), 4.41 (s, 3H), 4.11 (s, 3H), 3.85 (s, 3H).

### Example 178C

### 7-amino-3-chloro-5,10-dihydro-11H-dibenzo[b,e][1,4]diazepin-11-one

The title compound was prepared by substituting Example 178B for Example 5B in Example 5C. MS (DCI) m/e 260 (M+H)⁺; ¹H NMR (300 MHz, DMSO-d₆) δ 9.52 (s, 1H), 7.75 (s, 1H), 7.63 (d, J=8.48 Hz, 1H), 7.06 (d, J=2.03 Hz, 1H), 6.90 (dd, J=8.31, 2.20 Hz, 1H), 6.63 (d, J=8.48 Hz, 1H), 6.13-6.20 (m, 2H), 4.94 (s, 2H).

### Example 178D

### 7-amino-3-(3-methoxy-4-nitrophenyl)-5,10-dihydro-11H-dibenzo[b,e][1,4]diazepin-11-one

The title compound was prepared by substituting Example 178C and Example 266G for Example 1B and 2-methoxy-4-(4,4,5,5-tetramethyl-1,3,2-dioxaborolan-2-yl)phenol, respectively, in Example 12. MS (DCI) m/e 377 (M+H)⁺; ¹H NMR (300 MHz, DMSO-d₆) δ 9.51 (s, 1H), 8.01 (d, J=8.42 Hz, 1H), 7.77 (d, J=8.11 Hz, 1H), 7.69 (s, 1H), 7.51 (d, J=1.87 Hz, 1H), 7.36 (d, J=1.87 Hz, 1H), 7.33 (dd, J=8.42, 1.87 Hz, 1H), 7.29 (dd, J=8.11, 1.87 Hz, 1H), 6.66 (d, J=8.42 Hz, 1H), 6.25 (d, J=2.18 Hz, 1H), 6.16 (dd, J=8.42, 2.50 Hz, 1H), 4.90 (s, 2H), 4.03 (s, 3H).

### Example 179

### 3-chloroN[3-(3-methoxy-4-nitrophenyl)-11-oxo-10,11-dihydro-5H-dibenzo[b,e][1,4]diazepin-7-yl]propane-1-sulfonamide

The title compound was prepared by substituting Example 178D and 3-chloro-propane-1-sulfonyl chloride for Example 7C and CH₃SO₂Cl, respectively, in Example 42A. MS (DCI) m/e 517 (M+H)⁺; ¹H NMR (500 MHz, DMSO-d₆) δ 9.84 (s, 1H), 9.75 (s, 1H), 8.14 (s, 1H), 8.01 (d, J=8.42 Hz, 1H), 7.81 (d, J=8.11 Hz, 1H), 7.52 (d, J=1.56 Hz, 1H), 7.40 (d, J=1.56 Hz, 1H), 7.34 (dd, J=8.42, 1.87 Hz, 1H), 7.31 (dd, J=8.26, 1.72 Hz, 1H), 6.99 (d, J=2.18 Hz, 1H), 6.93 (d, J=8.73 Hz, 1H), 6.76 (dd, J=8.58, 2.34 Hz, 1H), 4.04 (s, 3H), 3.72 (t, J=6.55 Hz, 2H), 3.14-3.20 (m, 2H), 2.08-2.13 (m, 2H).

### Example 180

### N-[3-(3-methoxy-4-nitrophenyl)-11-oxo-10,11-dihydro-5H-dibenzo[b,e][1,4]diazepin-7-yl]-1-phenylmethanesulfonamide

The title compound was prepared by substituting Example 178D and phenylmethanesulfonyl chloride for Example 7C and CH₃SO₂Cl, respectively, in Example 42A. MS (DCI) m/e 531 (M+H)⁺; ¹H NMR (500 MHz, DMSO-d₆) δ 9.76 (s, 1H), 9.65 (s, 1H), 8.06 (s, 1H), 7.95 (d, J=8.42 Hz, 1H), 7.75 (d, J=8.11 Hz, 1H), 7.46 (s, 1H), 7.35 (d, J=1.56 Hz, 1H), 7.20-7.29 (m, 7H), 6.93 (d, J=2.18, Hz, 1H), 6.86 (d, J=8.42 Hz, 1H), 6.67 (dd, J=8.42, 2.18 Hz, 1H), 4.34 (s, 2H), 3.97 (3, 2H).

### Example 181

### 1-(4-chlorophenyl)N[3-(3-methoxy-4-nitrophenyl)-11-oxo-10,11-dihydro-5H-dibenzo[b,e][1,4]diazepin-7-yl]methanesulfonamide

The title compound was prepared by substituting Example 178D and 4-chlorophenylmethanesulfonyl chloride for Example 7C and CH₃SO₂Cl, respectively, in Example 42A. MS (DCI) m/e 566 (M+H)⁺; ¹H NMR (500 MHz, DMSO-d₆) δ 9.76 (s, 1H), 9.65 (s, 1H), 8.05 (s, 1H), 7.95 (d, J=8.42 Hz, 1H), 7.74 (d, J=8.11 Hz, 1H), 7.46 (s, 1H), 7.21-7.35 (m, 6H), 6.90 (d, J=2.18, Hz, 1H), 6.85 (d, J=8.42 Hz, 1H), 6.56 (dd, J=8.58, 2.34 Hz, 1H), 4.38 (s, 2H), 3.97 (s, 3H).

### Example 182

### N-[3-(3-methoxy-4-nitrophenyl)-11-oxo-10,11-dihydro-5H-dibenzo[b,e][1,4]diazepin-7-yl]-1-methyl-1H-imidazole-4-sulfonamide

The title compound was prepared by substituting Example 178D and 1-methyl-1H-imidazole-4-sulfonyl chloride for Example 7C and CH₃SO₂Cl, respectively, in Example 42A. MS (DCI) m/e 521 (M+H)⁺; ¹H NMR (500 MHz, DMSO-d₆) δ 10.04 (s, 1H), 9.75 (s, 1H), 8.00-8.03 (m, 3H), 7.77-7.80 (m, 1H), 7.71-7.74 (m, 2H), 7.51 (d, J=1.56 Hz, 1H), 7.39 (m, 1H), 7.34 (dd, J=8.42, 1.56 Hz, 1H), 7.30 (dd, J=8.11, 1.87 Hz, 1H), 6.87 (d, J=2.50, Hz, 1H), 6.79 (d, J=8.73 Hz, 1H), 6.68 (dd, J=8.58, 2.34 Hz, 1H), 4.04 (s, 3H), 3.97 (s, 3H).

### Example 183

### N-[3-(3-methoxy-4-nitrophenyl)-11-oxo-10,11-dihydro-5H-dibenzo[b,e][1,4]diazepin-7-yl]methanesulfonamide

The title compound was prepared by substituting Example 178D for Example 7C in Example 42A. MS (DCI) m/e 521 (M+H)⁺; ¹H NMR (500 MHz, DMSO-d₆) δ 9.64 (s, 1H), 9.57 (s, 1H), 8.12 (s, 1H), 8.01 (d, J=8.11 Hz, 1H), 7.80 (d, J=8.42 Hz, 1H), 7.52 (d, J=1.56 Hz, 1H), 7.40 (d, J=1.87 Hz, 1H), 7.35 (dd, J=8.42, 1.87 Hz, 1H), 7.32 (dd, J=8.11, 1.87 Hz, 1H), 6.98 (d, J=2.18, Hz, 1H), 6.93 (d, J=8.73 Hz, 1H), 6.75 (dd, J=8.58, 2.34 Hz, 1H), 4.04 (s, 3H), 2.95 (s, 3H).

### Example 184

### N-[3-(3-methoxy-4-nitrophenyl)-11-oxo-10,11-dihydro-5H-dibenzo[b,e][1,4]diazepin-7-yl]-3-morpholin-4-ylpropane-1-sulfonamide

The title compound was prepared by substituting Example 179 for Example 170 in Example 172. MS (DCI) m/e 568 (M+H)⁺; ¹H NMR (500 MHz, DMSO-d₆) δ 9.86 (s, 1H), 9.77 (s, 1H), 8.12 (s, 1H), 8.01 (d, J=8.42 Hz, 1H), 7.80 (d, J=8.11 Hz, 1H), 7.51 (s, 1H), 7.39 (d, J=1.56 Hz, 1H), 7.31-7.34 (m, 2H), 6.98 (d, J=2.18, Hz, 1H), 6.92 (d, J=8.42, Hz, 1H), 6.75 (dd, J=8.42, 2.18 Hz, 1H), 4.02 (s, 3H), 3.96-3.98(m, 2H), 3.58 (m, 2H), 3.13-3.17 (m, 8H), 3.00-3.02 (m, 2H), 2.02-2.04 (m, 2H).

### Example 185

### N-[3-(3-methoxy-4-nitrophenyl)-11-oxo-10,11-dihydro-5H-dibenzo[b,e][1,4]diazepin-7-yl]-3-piperidin-1-ylpropane-1-sulfonamide

The title compound was prepared by substituting Example 179 and piperidine for Example 170 and morpholine, respectively, in Example 172. MS (DCI) m/e 566 (M+H)⁺; ¹H NMR (500 MHz, DMSO-d₆) δ 9.80 (s, 1H), 9.71 (s, 1H), 9.04 (br, 1H), 8.08 (s, 1H), 7.95 (d, J=8.42 Hz, 1H), 7.74 (d, J=8.11 Hz, 1H), 7.45 (d, J=1.56 Hz, 1H), 7.34 (d, J=1.56 Hz, 1H), 7.23-7.28 (m, 2H), 6.93 (d, J=2.18 Hz, 1H), 6.87 (d, J=8.73 Hz, 1H), 6.70 (dd, J=8.58, 2.34 Hz, 1H), 3.97 (s, 3H), 3.02-3.10 (m, 4H), 2.73-2.78 (m, 2H), 1.95-1.99 (m, 2H), 1.67-1.71 (m, 2H), 1.26-1.55 (m, 4H).

### Example 186

### 3-(diethylamino)N[3-(3-methoxy-4-nitrophenyl)-11-oxo-10,11-dihydro-5H-dibenzo[b,e][1,4]diazepin-7-yl]propane-1-sulfonamide

The title compound was prepared by substituting Example 179 and diethylamine for Example 170 and morpholine, respectively, in Example 172. MS (DCI) m/e 554 (M+H)⁺; ¹H NMR (500 MHz, DMSO-d₆) δ 9.86 (s, 1H), 9.77 (s, 1H), 9.11 (br, 1H), 8.13 (s, 1H), 8.01 (d, J=8.42 Hz, 1H), 7.80 (d, J=8.11 Hz, 1H), 7.51 (d, J=1.56 Hz, 1H), 7.40 (d, J=1.56 Hz, 1H), 7.31-7.34 (m, 2H), 6.99 (d, J=2.50 Hz, 1H), 6.92 (d, J=8.73 Hz, 1H), 6.76 (dd, J=8.58, 2.34 Hz, 1H), 4.02 (s, 3H), 3.05-3.18 (m, 10H), 1.95-2.01 (m, 2H), 1.13 (t, J=71.7 Hz, 6H).

### Example 187

### 3-(dimethylamino)N[3-(3-methoxy-4-nitrophenyl)-11-oxo-10,11-dihydro-5H-dibenzo[b,e][1,4]diazepin-7-yl]propane-1-sulfonamide

The title compound was prepared by substituting Example 179 and dimethylamine for Example 170 and morpholine, respectively, in Example 172. MS (DCI) m/e 526 (M+H)⁺; ¹H NMR (500 MHz, DMSO-d₆) δ 9.82 (s, 1H), 9.53 (s, 1H), 9.12 (br, 1H), 7.88 (s, 1H), 7.77 (d, J=8.42 Hz, 1H), 7.56 (d, J=8.11 Hz, 1H), 7.27 (d, J=1.56 Hz, 1H), 7.15 (d, J=1.56 Hz, 1H), 7.06-7.10 (m, 2H), 6.74 (d, J=2.18 Hz, 1H), 6.68 (d, J=8.73 Hz, 1H), 6.61 (dd, J=8.42, 2.50 Hz, 1H), 3.78 (s, 3H), 2.82-2.92 (m, 4H), 2.49 (s, 6H), 1.74-1.79 (m, 2H).

### Example 188

### N-[3-(3-methoxy-4-nitrophenyl)-11-oxo-10,11-dihydro-5H-dibenzo[b,e][1,4]diazepin-7-yl]tetrahydro-2H-pyran-4-carboxamide

The title compound was prepared by substituting Example 179 and tetrahydro-2H-pyran-4-carboxylic acid for Example 120 and dimethylaminoacetic acid, respectively, in Example 122. MS (DCI) m/e 489 (M+H)⁺; ¹H NMR (500 MHz, DMSO-d₆) δ 9.80 (s, 1H), 9.79 (s, 1H), 8.00-8.02 (m, 2H), 7.79 (d, J=8.11 Hz, 1H), 7.60 (d, J=1.87 Hz, 1H), 7.52 (d, J=1.25 Hz, 1H), 7.35 (dd, J=8.42, 1.56 Hz, 1H), 7.31 (dd, J=8.11, 1.56 Hz, 1H), 6.93-6.95 (m, 1H), 6.88 (d, J=8.42 Hz, 1H), 4.04 (s, 3H), 3.87-3.91 (m, 2H), 3.30-3.36 (m, 2H), 2.26 (m, 1H), 1.63-1.68 (m, 4H).

### Example 189

### 8-(1-hydroxy-1-methylethyl)-3-(3-methoxy-4-nitrophenyl)-5,10-dihydro-11H-dibenzo[b,e][1,4]diazepin-11-one

### Example 189A

### 3-chloro-8-(1-hydroxy-1-methylethyl)-5,10-dihydro-11H-dibenzo[b,e][1,4]diazepin-11-one

Example 1B (0.180g, 0.60 mmol) in 20 mL of THF was treated dropwise with 3.0M MeMgBr (1.6 mL, 4.8 mmol) at room temperature. The resaction mixture was stirred overnight. The reaction was quenched carefully with MeOH, and the mixture was poured into water. To this solution was added 5 mL of concentrated HCl solution, and the mixture was extracted by ethyl acetate several times. The combined organic layers were washed with brine, dried (MgSO4), filtered, and concentrated under vacuum. The residue was purified by flash column chromatography on silica gel eluting with 3:7 hexanes/ethyl acetate to provide 0.108 g (60 %) of the title compound. MS (DCI) m/e 303 (M+H)⁺; ¹H NMR (500 MHz, DMSO-d₆) δ 9.56 (s, 1H), 7.72 (s, 1H), 7.42 (d, J=8.29 Hz, 1H), 6.97 (d, J=1.53 Hz, 1H), 6.82 (d, J=1.84 Hz, 1H), 6.80 (dd, J=8.29, 1.84 Hz, 1H), 6.64-6.68 (m, 2H), 4.65 (s, 1H), 1.12 (s, 6H).

### Example 189B

### 8-(1-hydroxy-1-methylethyl)-3-(3-methoxy-4-nitrophenyl)-5,10-dihydro-11H-dibenzo[b,e][1,4]diazepin-11-one

The desired product was prepared by substituting Example 189A and Example 266G for Example 1B and 2-methoxy-4-(4,4,5,5-tetramethyl-1,3,2-dioxaborolan-2-yl)phenol, respectively, in Example 12. MS (DCI) m/e 420 (M+H)⁺; ¹H NMR (500 MHz, DMSO-d₆) δ 9.80 (s, 1H), 8.00 (d, J=7.98 Hz, 1H), 7.91 (s, 1H), 7.79 (d, J=7.98 Hz, 1H), 7.51 (s, 1H), 7.21-7.34 (m, 2H), 7.12 (s, 1H), 7.03 (d, J=7.98 Hz, 1H), 6.93 (d, J=7.98 Hz, 1H), 4.88 (s, 1H), 4.02 (s, 3H), 1.36 (s, 6H).

### Example 190

### 8-(1-ethyl-1-hydroxypropyl)-3-(3-methoxy-4-nitrophenyl)-5,10-dihydro-11H-dibenzo[b,e][1,4]diazepin-11-one

### Example 190A

### 3-chloro-8-(1-ethyl-1-hydroxypropyl)-5,10-dihydro-11H-dibenzo[b,e][1,4]diazepin-11-one

The title compound was prepared by substituting EtMgBr for MeMgBr in Example 189A. MS (DCI) m/e 331 (M+H)⁺; ¹H NMR (500 MHz, DMSO-d₆) δ 9.80 (s, 1H), 7.96 (s, 1H), 7.68 (d, J=8.48 Hz, 1H), 7.07 (d, J=2.03 Hz, 1H), 7.02 (d, J=1.70 Hz, 1H), 6.92 (d, J=2.03 Hz, 1H), 6.87-6.90 (m, 2H), 4.43 (s, 1H), 1.60-1.70 (m, 4H), 0.61-0.66 (m, 6H).

### Example 190B

### 8-(1-ethyl-1-hydroxypropyl)-3-(3-methoxy-4-nitrophenyl)-5,10-dihydro-11H-dibenzo[b,e][1,4]diazepin-11-one

The desired product was prepared by substituting Example 190A and Example 266G for Example 1B and 2-methoxy-4-(4,4,5,5-tetramethyl-1,3,2-dioxaborolan-2-yl)phenol, respectively, in Example 12. MS (DCI) m/e 448 (M+H)⁺; ¹H NMR (500 MHz, DMSO-d₆) δ 9.78 (s, 1H), 8.00 (d, J=8.29 Hz, 1H), 7.89 (s, 1H), 7.78 (d, J=8.29 Hz, 1H), 7.51 (d, J=1.84 Hz, 1H), 7.32-7.34 (m, 2H), 7.27 (dd, J=8.13. 1.69 Hz, 1H), 7.02 (s, 1H), 6.93 (m, 2H), 4.39 (s, 1H), 4.02 (s, 3H), 1.62-1.65 (m, 4H), 0.63 (t, J=7.36 Hz, 6H).

### Example 191

### 8-(1-hydroxy-1-methylethyl)-3-(pyridin-4-ylamino)-5,10-dihydro-11H-dibenzo[b,e][1,4]diazepin-11-one

A mixture of Example 189A (60 mg, 0.20 mmol), 4-aminopyridine (26 mg, 0.24 mmol), Pd₂(dba)₃ (9.2 mg, 0.01 mmol), CyMAP (11.8 mg, 0.03 mmol), and Cs₂CO₃ (78 mg, 0.24 mmol) in 2 mL of dioxane was heated at 85 °C overnight. After the reaction mixture cooled to room temperature, it was partitioned between EtOAc and water. The aqueous layer was extracted with EtOAc three times. The combined organic layers were washed with brine, dried (MgSO₄), filtered, and concentrated under vacuum. The residue was purified by flash column chromatography on silica gel with 100:5:1 EtOAc/MeOH/NH₄OH to provide 54 mg (75%) of the desired product. MS (DCI) m/e 361 (M+H)⁺; ¹H NMR (500 MHz, DMSO-d₆) δ 9.49 (s, 1H), 9.01 (s, 1H), 8.29 (d, J=4.68 Hz, 1H), 7.79 (s, 1H), 7.64 (d, J=8.73 Hz, 1H), 7.09 (d, J=1.87 Hz, 1H), 6.99-7.03 (m, 3H), 6.86-6.89 (m, 2H), 6.63 (dd, J=8.58, 2.03 Hz, 1H), 4.68 (s, 1H), 1.36 (m, 6H).

### Example 192

### 3-[(2-chloropyridin-4-yl)amino]-8-(1-hydroxy-1-methylethyl)-5,10-dihydro-11H-dibenzo[b,e][1,4]diazepin-11-one

The desired product was prepared by substituting 2-chloro-4-aminopyridine for 4-aminopyridine in Example 191. MS (DCI) m/e 395 (M+H)⁺; ¹H NMR (500 MHz, DMSO-d₆) δ 9.55 (s, 1H), 9.26 (s, 1H), 8.07 (d, J=4.99 Hz, 1H), 7.84 (s, 1H), 7.66 (d, J=8.42 Hz, 1H), 7.09 (s, 1H), 6.98-7.01 (m, 3H), 6.85-6.89 (m, 2H), 6.64 (d, J=8.11 Hz, 1H), 4.86 (s, 1H), 1.35 (m, 6H).

### Example 193

### 4-{[8-(1-hydroxy-1-methylethyl)-11-oxo-10,11-dihydro-5H-dibenzo[b,e][1,4]diazepin-3-yl]amino}pyridine-2-carbonitrile

### Example 193A

### 4-aminopyridine-2-carbonitrile

A mixture of 2-chloro-pyridin-4-ylamine (0.642 g, 5 mmol), Zn(CN)₂ (0.323 g, 2.75 mmol) and Pd(PPh₃)₄ (0.288 g, 0.025 mmol) in 5 mL of DMF was heated at 145 °C for 20 hours. After the reaction mixture cooled to room temperature, it was partitioned between ethyl acetate and H₂O. The aqueous layer was extracted with additional ethyl acetate. The combined organic layers were washed with brine, dried (MgSO₄) filtered, and concentrated under vacuum. The residue was purified by flash column chromatography on silica gel with 9:1 hexanes/ethyl acetate to provide 0.29 g (20%) of the desired product. MS (DCI) m/e 120 (M+H)⁺ ; ¹H NMR (300 MHz, DMSO-d₆) δ 8.08 (d, J=5.76 Hz, 1H), 6.94 (d, J=2.34 Hz, 1H), 6.68 (dd, J=5.76, 2.37 Hz, 1H), 6.59 (s, 2H).

### Example 193B

### 4-{[8-(1-hydroxy-1-methylethyl)-11-oxo-10,11-dihydro-5H-dibenzo[b,e][1,4]diazepin-3-yl]amino}pyridine-2-carbonitrile

The desired product was prepared by substituting Example 193A for 4-aminopyridine in Example 191. MS (DCI) m/e 386 (M+H)⁺; ¹H NMR (500 MHz, DMSO-d₆) δ 9.57 (s, 1H), 9.45 (s, 1H), 8.36 (d, J=5.61 Hz, 1H), 7.83 (s, 1H), 7.67 (d, J=8.42 Hz, 1H), 7.48 (d, J=2.18 Hz, 1H), 7.22 (dd, J=5.77, 2.34 Hz, 1H), 7.09 (d, =1.56 HZ, 1H), 7.00-7.01 (m, 1H), 6.86-6.88 (m, 2H), 6.66 (dd, J=8.73, 1.87 Hz, 1H), 4.86 (s, 1H), 1.35 (s, 6H).

### Example 194

### 8-(1-hydroxy-1-methylethyl)-3-(pyrimidin-4-ylamino)-5,10-dihydro-11H-dibenzo[b,e][1,4]diazepin-11-one

The desired product was prepared by substituting pyrimidin-4-ylamine for 4-aminopyridine in Example 191. MS (DCI) m/e 362 (M+H)⁺; ¹H NMR (500 MHz, DMSO-d₆) δ 9.74 (s, 1H), 9.54 (s, 1H), 8.71 (s, 1H), 8.34 (d, J=4.60 Hz, 1H), 7.85 (s, 1H), 7.65 (d, J=8.29 Hz, 1H), 7.49 (s, 1H), 6.94-7.09 (m, 4H), 6.88 (d, J=4.91 Hz, 1H), 4.87 (s, 1H), 1.37 (s, 6H).

### Example 195

### 8-(1-hydroxy-1-methylethyl)-3-[(2,3,5,6-tetrafluoropyridin-4-yl)amino]-5,10-dihydro-11H-dibenzo[b,e][1,4]diazepin-11-one

The desired product was prepared by substituting 2,3,5,6-tetrafluoro-pyridin-4-ylamine for 4-aminopyridine in Example 191. MS (DCI) m/e 433 (M+H)⁺; ¹H NMR (500 MHz, DMSO-d₆) δ 9.60 (s, 1H), 9.58 (s, 1H), 7.78 (s, 1H), 7.62 (d, J=8.42 Hz, 1H), 7.09 (d, J=1.87 Hz, 1H), 7.00 (dd, J=8.42, 1.87 Hz, 1H), 6.67 (d, J=8.42 Hz, 1H), 6.00-6.-3 (m, 2H), 4.88 (s, 1H), 1.36 (s, 6H).

### Example 196

### 8-(1-ethyl-1-hydroxypropyl)-3-(pyridin-4-ylamino)-5,10-dihydro-11H-dibenzo[b,e][1,4]diazepin-11-one

The desired product was prepared by substituting Example 190A for Example 189A in Example 191. MS (DCI) m/e 389 (M+H)⁺; ¹H NMR (500 MHz, DMSO-d₆) δ 9.47 (s, 1H), 9.01 (s, 1H), 8.28 (s, 1H), 7.77 (s, 1H), 7.62 (d, J=1.52 Hz, 1H), 6.99-7.03 (m, 2H), 6.89 (m, 2H), 6.62 (d, J=7.98 Hz, 1H), 4.38 (s, 1H), 1.61-1.64 (m, 4H), 0.64 (s, 6H).

### Example 197

### 3-[(2-aminopyrimidin-4-yl)amino]-8-(1-hydroxy-1-methylethyl)-5,10-dihydro-11H-dibenzo[b,e][1,4]diazepin-11-one

The desired product was prepared by substituting pyrimidine-2,4-diamine for 4-aminopyridine in Example 191. MS (DCI) m/e 377 (M+H)⁺; ¹H NMR (500 MHz, DMSO-d₆) δ 9.47 (s, 1H), 9.30 (s, 1H), 7.87 (d, J=5.61 Hz, 1H), 7.75 (d, J=1.56 Hz, 1H), 7.58 (d, J=8.42 Hz, 1H), 7.53 (s, 1H), 7.03-7.08 (m, 2H), 7.01 (dd, J=8.26, 1.72 Hz, 1H), 6.89 (d, J=8.11 Hz, 1H), 6.23 (s, 2H), 6.07 (d, J=5.93 Hz, 1H), 4.86 (s, 1H), 1.35 (s, 6H).

### Example 198

### 3-[(2-chloropyridin-4-yl)amino]-8-(1-ethyl-1-hydroxypropyl)-5,10-dihydro-11H-dibenzo[b,e][1,4]diazepin-11-one

The desired product was prepared by substituting Example 190A and 2-chloro-4-aminopyridine for Example 189A and 4-aminopyridine, respectively, in Example 191. MS (DCI) m/e 423 (M+H)⁺; ¹H NMR (500 MHz, DMSO-d₆) δ 9.53 (s, 1H), 9.26 (s, 1H), 8.07 (d, J=5.93 Hz, 1H), 7.83 (s, 1H), 7.66 (d, J=8.42 Hz, 1H), 6.98-7.01 (m, 3H), 6.86-6.89 (m, 3H), 6.63 (dd, J=8.73, 2.18 Hz, 1H), 4.37 (s, 1H), 1.62-1.64 (m, 4H), 0.63 (t, J=7.33Hz, 6H).

### Example 199

### 8-(1-hydroxy-1-methylethyl)-3-[(2,3,6-trifluoropyridin-4-yl)amino]-5,10-dihydro-11H-dibenzo[b,e][1,4]diazepin-11-one

### Example 199A

### 2,3,6-trifluoropyridin-4-amine

A mixture of 3-chloro-2,5,6-trifluoro-pyridin-4-ylamine (1.82 g, 10 mmol), 5% Pd/C (0.5 g), and Et₃N (3.04 g, 30 mmol) in 50 mL of MeOH was equipped with a balloon of hydrogen gas and stirred at room temperature overnight. The solution was filtered through diatomaceous earth (Celite^{®}). The filtrate was concentrated under vacuum and the residue was partitioned between ethyl acetate and H₂O. The aqueous layer was extracted with additional ethyl acetate, and the combined organic layers were washed with brine, dried (MgSO₄), filtered and concentrated under vacuum. The residue was purified by flash column chromatography on silica gel with 7:3 hexanes/ethyl acetate to provide 1.33 g (90%) of the desired product. MS (DCI) m/e 149 (M+H)⁺.

### Example 199B

### 8-(1-hydroxy-1-methylethyl)-3-[(2,3,6-trifluoropyridin-4-yl)amino]-5,10-dihydro-11H-dibenzo[b,e][1,4]diazepin-11-one

The desired product was prepared by substituting Example 199A for 4-aminopyridine in Example 191. MS (DCI) m/e 423 (M+H)⁺; ¹H NMR (500 MHz, DMSO-d₆) δ 9.62 (s, 1H), 9.54 (s, 1H), 7.86 (s, 1H), 7.69 (d, J=8.73 Hz, 1H), 7.10 (d, J=1.87 Hz, 1H), 6.88-6.91 (m, 3H), 6.77-6.80 (m, 2H), 4.99 (s, 1H), 1.36 (s, 6H).

### Example 200

### 3-({2-[(2-chloropyridin-4-yl)amino]pyridin-4-yl}amino)-8-(1-hydroxy-1-methylethyl)-5,10-dihydro-11H-dibenzo[b,e][1,4]diazepin-11-one

The desired product was isolated as a second product in Example 192. MS (DCI) m/e 487 (M+H)⁺; ¹H NMR (500 MHz, DMSO-d₆) δ 9.59 (s, 1H), 9.49 (s, 1H), 8.97 (s, 1H), 8.04 (m, 2H), 7.97 (s, 1H), 7.79 (s, 1H), 7.65 (d, J=8.42 Hz, 1H), 7.41 (s, J=4.99 Hz, 1H), 7.08 (s, 1H), 6.99 (d, J=8.11 Hz, 1H), 6.89 (d, J=8.11 Hz, 1H), 6.82 (s, 1H), 6.61-6.66 (m, 3H), 4.86 (s, 1H), 1.35 (s, 6H).

### Example 201

### methyl 2-methyl-2-{11-oxo-3-[(2,3,6-trifluoropyridin-4-yl)amino]-10,11-dihydro-5H-dibenzo[b,e][1,4]diazepin-8-yl}propanoate

The desired product was prepared by substituting Example 199A and Example 266F for4-aminopyridine and Example 189A, respectively, in Example 191. MS (DCI) m/e 487 (M+H)⁺; ¹H NMR (500 MHz, DMSO-d₆) δ 9.67 (s, 1H), 9.58 (s, 1H), 7.97 (s, 1H), 7.70 (d, J=8.54 Hz, 1H), 6.89-6.95 (m, 4H), 6.82 (d, J=4.27 Hz, 1H), 6.79 (dd, J=8.54, 2.14 Hz, 1H), 3.58 (s, 3H), 1.44 (s, 6H).

### Example 202

### 2-methylN(4-morpholin-4-ylphenyl)-2-{11-oxo-3-[(2,3,6-trifluoropyridin-4-yl)amino]-10,11-dihydro-5H-dibenzo[b,e][1,4]diazepin-8-yl}propanamide

### Example 202A

### 2-(3-chloro-11-oxo-10,11-dihydro-5H-dibenzo[b,e][1,4]diazepin-8-yl)-2-methylpropanoic acid

The desired product was prepared by substituting Example 266F for Example 12 in Example 13. MS m/e (DCI) 331 (M+H)⁺.

### Example 202B

### 2-(3-chloro-11-oxo-10,11-dihydro-5H-dibenzo[b,e][1,4]diazepin-8-yl)-2-methylN(4-morpholin-4-ylphenyl)propanamide

The desired product was prepared by substituting Example 202A and 4-(4-morpholino)aniline for dimethylaminoacetic acid and Example 120, respectively, in Example 122. MS m/e (DCI) 490 (M+H)⁺.

### Example 202C

### 2-methylN(4-morpholin-4-ylphenyl)-2-{11-oxo-3-[(2,3,6-trifluoropyridin-4-yl)amino]-10,11-dihydro-5H-dibenzo[b,e][1,4]diazepin-8-yl}propanamide

The desired product was prepared by substituting Example 202B and Example 199A for Example 189A and 4-aminopyridine, respectively, in Example 191. MS (DCI) m/e 603 (M+H)⁺; ¹H NMR (500 MHz, DMSO-d₆) δ 9.67 (s, 1H), 9.53 (s, 1H), 8.77 (s, 1H), 7.91 (s, 1H), 7.68 (d, J=8.59 Hz, 1H), 7.41 (s, 1H), 7.59 (s, 1H), 7.01 (s, 1H), 6.90-6.92 (m, 3H), 6.45-6.84 (m, 4H), 3.69-3.71 (m, 4H), 2.99-3.01 (m, 4H), 1.47 (s, 6H).

### Example 203

### 2-{3-[(2,6-difluoropyridin-4-yl)amino]-11-oxo-10,11-dihydro-5H-dibenzo[b,e][1,4]diazepin-8-yl}-2-methylN(4-morpholin-4-ylphenyl)propanamide

### Example 203A

### 2,6-Difluoro-pyridin-4-ylamine

The title compound was prepared by substituting 3,5-dichloro-2,6-difluoro-pyridin-4-ylamine for 3-chloro-2,5,6-trifluoro-pyridin-4-ylamine in Example 199A. MS (DCI) m/e 183 (M+H)⁺; ¹H NMR (500 MHz, DMSO-d₆) δ 6.84 (s, 2H), 6.03 (s, 2H).

### Example 203B

### 2-{3-[(2,6-difluoropyridin-4-yl)amino]-11-oxo-10,11-dihydro-5H-dibenzo[b,e][1,4]diazepin-8-yl}-2-methylN(4-morpholin-4-ylphenyl)propanamide

The desired product was prepared by substituting Example 202B and Example 203A for Example 189A and 4-aminopyridine, respectively, in Example 191. MS (DCI) m/e 585 (M+H)⁺; ¹H NMR (500 MHz, DMSO-d₆) δ 9.65 (s, 1H), 9.64 (s, 1H), 8.77 (s, 1H), 7.91 (s, 1H), 7.67 (d, J=8.42 Hz, 1H), 7.40 (d, J=9.04 Hz, 2H), 6.88-6.91 (m, 3H), 6.82 (d, J=9.04 Hz, 2H), 6.66 (dd, J=8.42, 1.87 Hz, 1H), 6.55 (s, 2H), 3.69-3.71 (m, 4H), 2.99-3.01 (m, 4H), 1.47 (s, 6H).

### Example 204

### 3-[(2,6-difluoropyridin-4-yl)amino]-8-(2-hydroxyethyl)-5,10-dihydro-11H-dibenzo[b,e][1,4]diazepin-11-one

### Example 204A

### 3-chloro-8-(2-hydroxyethyl)-5,10-dihydro-11H-dibenzo[b,e][1,4]diazepin-11-one

A mixture of Example 6D (6.32 g, 20 mmol) in 100 mL of THF was treated with 1.0 M LiAlH₄ in THF (35 mL, 35 mmol) at 0 °C. The reaction mixture was stirred for additional 30 min., quenched with MeOH, and concentrated under vacuum. The residue was partitioned between ethyl acetate and pH =3 water. The aqueous layer was extracted with additional ethyl acetate twice. The combined organic layers were washed with brine, dried (MgSO₄), filtered, and concentrated under vacuum. The residue was purified by flash column chromatography on silica gel with 1:9 hexanes/ethyl acetate to provide 5.18 g (90%) of the desired product. MS (DCI) m/e 289 and 291 (M+H)⁺; ¹H NMR (300 MHz, DMSO-d₆) δ 9.83 (s, 1H), 7.96 (s, 1H), 7.67 (d, J=8.5 Hz, 1H), 7.06 (d, J=2.0 Hz, 1H), 6.91 (dd, J=8.5, 2.0 Hz, 1H), 6.81-6.84 (m, 3H), 4.59 (t, J=6.0 Hz, 1H), 3.51-3.53 (m, 2H), 2.58 (t, J=6.9 Hz, 2H).

### Example 204B

### 3-[(2,6-difluoropyridin-4-yl)amino]-8-(2-hydroxyethyl)-5,10-dihydro-11H-dibenzo[b,e][1,4]diazepin-11-one

The desired product was prepared by substituting Example 204A and Example 203A for Example 189A and 4-aminopyridine, respectively, in Example 191. MS (DCI) m/e 383 (M+H)⁺; ¹H NMR (500 MHz, DMSO-d₆) δ 9.57 (s, 1H), 9.51 (s, 1H), 7.75 (s, 1H), 7.59 (d, J=8.73 Hz, 1H), 6.77-6.80 (m, 2H), 6.69-6.71 (m, 2H), 6.59 (d, J=8.42 Hz, 1H), 6.47 (s, 2H), 4.49 (t, J=4.99 Hz, 1H), 3.41-3.45 (m, 2H), 2.49 (t, J=7.02 Hz, 2H).

### Example 205

### 2-{3-[(2,6-difluoropyridin-4-yl)amino]-11-oxo-10,11-dihydro-5H-dibenzo[b,e][1,4]diazepin-8-yl}-2-methylpropanoic acid

### Example 205A

### methyl 2-{3-[(2,6-difluoropyridin-4-yl)amino]-11-oxo-10,11-dihydro-5H-dibenzo[b,e][1,4]diazepin-8-yl}-2-methylpropanoate

The desired product was prepared by substituting Example 266F and Example 203A for Example 189A and 4-aminopyridine, respectively, in Example 191. MS (DCI) m/e 439 (M+H)⁺; ¹H NMR (500 MHz, DMSO-d₆) δ 9.67 (s, 1H), 9.62 (s, 1H), 7.95 (s, 1H), 7.70 (d, J=8.42 Hz, 1H), 6.88-6.95 (m, 4H), 6.68 (dd, J=8.42, 2.18 Hz, 1H), 6.57 (s, 2H), 3.58 (s, 3H), 1.44 (s, 6H).

### Example 205B

### 2-{3-[(2,6-difluoropyridin-4-yl)amino]-11-oxo-10,11-dihydro-5H-dibenzo[b,e][1,4]diazepin-8-yl}-2-methylpropanoic acid

A mixture of Example 205A (0.52 g, 1.18 mmol) and LiOH (0.144 g, 6.0 mmol) in 20 mL of THF and 20 mL of H₂O was heated under reflux overnight. After the solution cooled to room temperature, the solution was neutralized with 10% HCl, and extracted with ethyl acetate. The organic layer was washed with brine, dried (MgSO4), filtrated and concentrated under vacuum to give the title compound. MS (DCI) m/e 425 (M+H)⁺; ¹H NMR (500 MHz, DMSO-d₆) δ 12.27 (br, 1H), 9.72 (s, 1H), 9.68 (s, 1H), 7.98 (s, 1H), 7.75 (d, J=8.73 Hz, 1H), 7.06 (s, 1H), 6.96-6.99 (m, 3H), 6.74 (dd, J=8.73, 2.18 Hz, 1H), 6.62 (s, 2H), 1.46 (s, 6H).

### Example 206

### 3-[(2,6-difluoropyridin-4-yl)amino]-7-morpholin-4-yl-5,10-dihydro-11H-dibenzo[b,e][1,4]diazepin-11-one

The desired product was prepared by substituting Example 450B and Example 203A for Example 189A and 4-aminopyridine, in Example 191. MS (DCI) m/e 439 (M+H)⁺; ¹H NMR (500 MHz, DMSO-d₆) δ 9.65 (s, 1H), 9.49 (s, 1H), 7.79 (s, 1H), 6.83 (d, J=8.73 Hz, 1H), 6.89 (dd, J=8.58, 2.03 Hz, 1H), 6.53-6.57 (m, 4H), 6.57 (s, 2H), 3.71-3.73 (m, 4H), 2.98-3.00 (m, 4H).

### Example 207

### 7-morpholin-4-yl-3-[(2,3,6-trifluoropyridin-4-yl)amino]-5,10-dihydro-11H-dibenzo[b,e][1,4]diazepin-11-one

The desired product was prepared by substituting Example 450B and Example 199A for Example 189A and 4-aminopyridine, in Example 191. MS (DCI) m/e 442 (M+H)⁺; ¹H NMR (500 MHz, DMSO-d₆) δ 9.53 (s, 1H), 9.52 (s, 1H), 7.79 (s, 1H), 7.68 (d, J=8.73 Hz, 1H), 6.90 (d, J=1.87 Hz, 1H), 6.78-6.84 (m, 3H), 6.53-6.55 (m, 2H), 3.71-3.73 (m, 4H), 2.98-3.00 (m, 4H).

### Example 208

### 3-[(2,6-difluoropyridin-4-yl)amino]-8-(2-hydroxy-2-methylpropyl)-5,10-dihydro-11H-dibenzo[b,e][1,4]diazepin-11-one

### Example 208A

### 3-chloro-8-(2-hydroxy-2-methylpropyl)-5,10-dihydro-11H-dibenzo[b,e][1,4]diazepin-11-one

The title compound was prepared by substituting Example 6D for Example 1B in Example 189A. MS (DCI) m/e 317 (M+H)⁺; ¹H NMR (500 MHz, DMSO-d₆) δ 9.79 (s, 1H), 7.92 (s, 1H), 7.66 (d, J=8.42 Hz, 1H), 7.05 (d, J=1.87 Hz, 1H), 6.89 (d, J=8.42, 1.87 Hz, 1H), 6.78-6.85 (m, 3H), 4.21 (s, 1H), 2.49 (s, 2H), 1.01 (s, 6H).

### Example 208B

### 3-[(2,6-difluoropyridin-4-yl)amino]-8-(2-hydroxy-2-methylpropyl)-5,10-dihydro-11H-dibenzo[b,e][1,4]diazepin-11-one

The title compound was prepared by substituting Example 208A and Example 203A for Example 189A and 4-aminopyridine, respectively, in Example 191. MS (DCI) m/e 411 (M+H)⁺; ¹H NMR (500 MHz, DMSO-d₆) δ 9.63 (s, 1H), 9.56 (s, 1H), 7.85 (s, 1H), 7.66 (d, J=8.42 Hz, 1H), 6.89 (s, 1H), 6.80 (d, J=7.17 Hz, 2H), 6.69 (d, J=7.80 Hz, 1H), 6.65 (d, J=8.42 Hz, 1H), 6.54 (s, 2H), 4.24 (s, 1H), 2.49 (s, 2H), 1.02 (s, 6H).

### Example 209

### methyl [11-oxo-3-(pyridin-4-ylamino)-10,11-dihydro-5H-dibenzo[b,e][1,4]diazepin-8-yl]acetate

The title compound was prepared by substituting Example 6D for Example 189A in Example 191. MS (DCI) m/e 375 (M+H)⁺; ¹H NMR (500 MHz, DMSO-d₆) δ 10.50 (s, 1H), 9.74 (s, 1H), 8.31 (s, 1H), 8.29 (s, 1H), 7.98 (s, 1H), 7.70 (d, J=8.42 Hz, 1H), 7.17 (d, J=7.49 Hz, 2H), 6.89 (d, J=2.18 Hz, 1H), 6.86 (d, J=7.80 Hz, 1H), 6.77-6.80 (m, 3H), 3.53 (s, 3H), 3.47 (s, 2H).

### Example 210

### methyl {3-[(2-chloropyridin-4-yl)amino]-11-oxo-10,11-dihydro-5H-dibenzo[b,e][1,4]diazepin-8-yl}acetate

The title compound was prepared by substituting Example 6D and 2-chloro-4-aminopyridine for Example 189A and 4-aminopyridine, respectively, in Example 191. MS (DCI) m/e 409 (M+H)⁺; ¹H NMR (500 MHz, DMSO-d₆) δ 9.63 (s, 1H), 9.31 (s, 1H), 8.09 (d, J=6.14 Hz, 1H), 7.93 (s, 1H), 7.67 (d, J=8.59 Hz, 1H), 7.00-7.01 (m, 2H), 6.82-6.92 (m, 4H), 6.66 (d, J=8.90 Hz, 1H), 3.60 (s, 3H), 3.52 (s, 2H).

### Example 211

### methyl {3-[(2-methylpyridin-4-yl)amino]-11-oxo-10,11-dihydro-5H-dibenzo[b,e][1,4]diazepin-8-yl}acetate

The title compound was prepared by substituting Example 6D and 2-methyl-4-aminopyridine for Example 189A and 4-aminopyridine, respectively, in Example 191. MS (DCI) m/e 389 (M+H)⁺; ¹H NMR (500 MHz, DMSO-d₆) δ 10.81 (s, 1H), 9.80 (s, 1H), 8.26 (d, J=7.37 Hz, 1H), 8.04 (s, 1H), 7.01-7.09 (m, 2H), 6.93-6.94 (m, 2H), 6.82-6.87 (m, 4H), 3.50 (s, 3H), 3.54 (s, 2H).

### Example 212

### 3-[(2-chloropyridin-4-yl)amino]-5,10-dihydro-11H-dibenzo[b,e][1,4]diazepin-11-one

The title compound was prepared by substituting Example 9 and 2-chloro-4-aminopyridine for Example 189A and 4-aminopyridine, respectively, in Example 191. MS (DCI) m/e 334 (M+H)⁺; ¹H NMR (500 MHz, DMSO-d₆) δ 9.64 (s, 1H), 9.29 (s, 1H), 8.09 (d, J=6.14 Hz, 1H), 7.93 (s, 1H), 7.69 (d, J=8.70 Hz, 1H), 6.88-7.01 (m, 7H), 6.67 (dd, J=8.59, 2.15, 1H).

### Example 213

### 8-acetyl-3-[(2-chloropyridin-4-yl)amino]-5,10-dihydro-11H-dibenzo[b,e][1,4]diazepin-11-one

### Example 213A

### 8-acetyl-3-chloro-5,10-dihydro-11H-dibenzo[b,e][1,4]diazepin-11-one

The title compound was isolated as a minor product in Example 189A. MS (DCI) m/e 287 (M+H)⁺; ¹H NMR (500 MHz, DMSO-d₆) δ 9.98 (s, 1H), 8.51 (s, 1H), 7.73 (d, J=8.59 Hz, 1H), 7.57-7.61 (m, 2H), 7.08 (d, J=1.84 Hz, 1H), 7.04 (d, J=8.29 Hz, 2H), 6.96 (dd, J=8.59, 2.15 Hz, 1H), 2.45 (s, 3H).

### Example 213B

### 8-acetyl-3-[(2-chloropyridin-4-yl)amino]-5,10-dihydro-11H-dibenzo[b,e][1,4]diazepin-11-one

The title compound was prepared by substituting Example 213A and 2-chloro-4-pyridine for Example 189A and 4-aminopyridine, respectively, in Example 191. MS (DCI) m/e 378 (M+H)⁺; ¹H NMR (500 MHz, DMSO-d₆) δ 10.00 (s, 1H), 8.59 (s, 2H), 8.49 (s, 1H), 7.86 (d, J=8.11 Hz, 1H), 7.57-7.58 (m, 2H), 7.45 (d, J=5.61 Hz, 2H), 6.94-6.94 (m, 4H), 2.06 (s, 3H).

### Example 214

### {3-[(2-chloropyridin-4-yl)amino]-11-oxo-10,11-dihydro-5H-dibenzo[b,e][1,4]diazepin-8-yl}acetic acid

The title compound was prepared by substituting Example 210 for Example 12 in Example 13. MS (DCI) m/e 395 (M+H)⁺; ¹H NMR (500 MHz, DMSO-d₆) δ 9.69 (s, 1H), 9.65 (s, 1H), 8.11 (d, J=5.93 Hz, 1H), 7.98 (s, 1H), 7.69 (d, J=8.73 Hz, 1H), 7.05-7.06 (m, 2H), 6.91-6.94 (m, 2H), 6.83-6.85 (m, 2H), 6.70 (dd, J=8.58, 2.03 Hz, 1H), 3.42 (s, 2H).

### Example 215

### 3-[(2-chloropyridin-4-yl)amino]-8-isopropenyl-5,10-dihydro-11H-dibenzo[b,e][1,4]diazepin-11-one

The title compound was isolated as a minor product in Example 192. MS (DCI) m/e 377 (M+H)⁺; ¹H NMR (500 MHz, DMSO-d₆) δ 9.77 (s, 1H), 9.50 (s, 1H), 8.12-8.13 (m, 2H), 7.71 (d, J=8.42 Hz, 1H), 7.07-7.13 (m, 3H), 6.91-6.97 (m, 3H), 6.70 (dd, J=8.42, 1.87 Hz, 1H), 5.29 (s, 1H), 4.99 (s, 1H), 2.03 (s, 3H).

### Example 216

### 2-{3-[(2-chloropyridin-4-yl)amino]-11-oxo-10,11-dihydro-5H-dibenzo[b,e][1,4]diazepin-8-yl}N(4-morpholin-4-ylphenyl)acetamide

The title compound was prepared by substituting Example 214 and 4-morpholin-4-ylphenylamine for dimethylaminoacetic acid and Example 120, respectively, in Example 122. MS (DCI) m/e 556 (M+H)⁺; ¹H NMR (500 MHz, DMSO-d₆) δ 9.89 (s, 1H), 9.65 (s, 1H), 9.32 (s, 1H), 8.08 (d, J=6.55 Hz, 1H), 7.89 (s, 1H), 7.67 (d, J=8.42 Hz, 1H), 7.45 (d, J=9.05 Hz, 2H), 6.99-7.01 (m, 2H), 6.86-6.92 (m, 5H), 6.65 (dd, J=8.58, 2.03 Hz, 1H), 3.72-3.74 (m, 4H), 3.45 (s, 2H), 3.04-3.06 (m, 4H).

### Example 217

### 3-[(2-chloropyridin-4-yl)amino]-8-(2-hydroxy-2-methylpropyl)-5,10-dihydro-11H-dibenzo[b,e][1,4]diazepin-11-one

The title compound was prepared by substituting Example 208A and 2-chloro-4-aminopyridine for Example 189A and 4-aminopyridine, respectively, in Example 191. MS (DCI) m/e 409 (M+H)⁺; ¹H NMR (500 MHz, DMSO-d₆) δ 9.59 (s, 1H), 9.30 (s, 1H), 8.09 (d, J=3.05 Hz, 1H), 7.86 (s, 1H), 7.67 (d, J=7.93 Hz, 1H), 7.00 (s, 2H), 6.79-7.87 (m, 4H), 6.65 (d, J=7.63, Hz, 1H), 4.25 (s, 1H), 2.12 (s, 2H), 1.04 (s, 6H).

### Example 218

### 2-{3-[(2-chloropyridin-4-yl)amino]-11-oxo-10,11-dihydro-5H-dibenzo[b,e][1,4]diazepin-8-yl}-2-methylN(4-morpholin-4-ylphenyl)propanamide

The title compound was prepared by substituting Example 202B and 2-chloro-4-aminopyridine for Example 189A and 4-aminopyridine, respectively, in Example 191. MS (DCI) m/e 584 (M+H)⁺; ¹H NMR (500 MHz, DMSO-d₆) δ 9.62 (s, 1H), 9.32 (s, 1H), 8.82 (s, 1H), 8.08 (d, J=6.55 Hz, 1H), 7.92 (s, 1H), 7.67 (d, J=8.59 Hz, 1H), 7.44 (d, J=8.90 Hz, 2H), 6.99-7.02 (m, 3H), 6.86-6.92 (m, 5H), 6.65 (dd, J=8.59, 2.15 Hz, 1H), 3.72-3.74 (m, 4H), 3.04-3.07 (m, 4H), 1.48 (s, 6H).

### Example 219

### 3-[(2-chloropyridin-4-yl)amino]-8-(2-oxopyrrolidin-1-yl)-5,10-dihydro-11H-dibenzo[b,e] [1,4]diazepin-11-one

The title compound was prepared by substituting Example 161B and 2-chloro-4-aminopyridine for Example 189A and 4-aminopyridine, respectively, in Example 191. MS (DCI) m/e 420 (M+H)⁺; ¹H NMR (500 MHz, DMSO-d₆) δ 9.69 (s, 1H), 9.61 (s, 1H), 8.14 (s, 1H), 8.02 (s, 1H), 7.73 (d, J=8.11 Hz, 1H), 7.34 (s, 1H), 7.25 (s, 1H), 6.92-7.08 (m, 4H), 6.72 (d, J=8.11 Hz, 1H), 3.71-3.77 (m, 2H), 3.51-3.53 (m, 2H), 2.05-2.07 (m, 2H).

### Example 220

### methyl {3-[(2-chloropyridin-4-yl)amino]-11-oxo-10,11-dihydro-5H-dibenzo[b,e][1,4]diazepin-7-yl}acetate

The title compound was prepared by substituting Example 388D and 2-chloro-4-aminopyridine for Example 189A and 4-aminopyridine, respectively, in Example 191. MS (DCI) m/e 409 (M+H)⁺; ¹H NMR (500 MHz, DMSO-d₆) δ 9.63 (s, 1H), 9.31 (s, 1H), 8.14 (s, 1H), 8.08-8.10 (m, 1H), 7.95 (s, 1H), 7.67 (d, J=8.59 Hz, 1H), 7.00-7.02 (m, 2H), 6.88-6.89 (m, 3H), 6.78 (dd, J=8.29, 1.84 Hz, 1H), 6.66 (dd, J=8.59, 2.15 Hz, 1H), 3.60 (s, 3H), 3.54 (s, 2H).

### Example 221

### 8-[2-(pyridin-2-yloxy)ethyl]-3-[(2,3,6-trifluoropyridin-4-yl)amino]1-5,10-dihydro-1H-dibenzo[b,e][1,4]diazepin-11-one

### Example 221A

### 3-chloro-8-[2-(pyridin-2-yloxy)ethyl]-5,10-dihydro-11H-dibenzo[b,e][1,4]diazepin-11-one

A mixture of Example 204A (150 mg, 0.52 mmol), pyridin-2(1H)-one (96 mg, 1.0 mmol), PPh₃ (180 mg, 0.68 mmol) and di-tert-butyl azodicarboxylate (160 mg, 0.68 mmol) in 10 mL of THF was stirred overnight. The reaction mixture was purified by flash column chromatography on silica gel with 7:3 hexanes/ethyl acetate to provide 72 mg of the desired product. The column was then washed with 100:3:1 EtOAc/MeOH/NH₄OH to give 79 mg of 3-chloro-8-[2-(2-oxo-2H-pyridin-1-yl)-ethyl]-5,10-dihydro-dibenzo[b,e][1,4]diazepin-11-one. MS (DCI) m/e 366 (M+H)⁺; ¹H NMR (500 MHz, DMSO-d₆) δ 9.83 (s, 1H), 8.13-8.14 (m, 1H), 7.97 (s, 1H), 7.65-7.68 (m, 2H), 7.05 (d, J=1.84 Hz, 1H), 6.89-6.95 (m, 5H), 6.76 (d, J=8.42 Hz, 1H), 4.38 (t, J=6.86 Hz, 2H), 2.99 (t, J=6.86 Hz, 2H).

### Example 221B

### 8-[2-(pyridin-2-yloxy)ethyl]-3-[(2,3,6-trifluoropyridin-4-yl)amino]-5,10-dihydro-11H-dibenzo[b,e][1,4]diazepin-11-one

The title compound was prepared by substituting Example 221A and Example 203A for Example 189A and 4-aminopyridine, respectively, in Example 191. MS (DCI) m/e 478 (M+H)⁺; ¹H NMR (500 MHz, DMSO-d₆) δ 9.67 (s, 1H), 9.55 (s, 1H), 8.14-8.15 (m, 1H), 7.89 (s, 1H), 7.66-7.70 (m, 2H), 6.94-6.97 (m, 1H), 6.87-6.91 (m, 4H), 6.77-6.81 (m, 3H), 4.39 (t, J=6.75 Hz, 2H), 2.89 (t, J=6.90 Hz, 2H).

### Example 222

### 8-(2-hydroxy-2-methylpropyl)-3-[(2,3,5-trifluorophenyl)amino]-5,10-dihydro-11H-dibenzo[b,e][1,4]diazepin-11-one

The title compound was prepared by substituting Example 208A and Example 203A for Example 189A and 4-aminopyridine, respectively, in Example 191. MS (DCI) m/e 428 (M+H)⁺; ¹H NMR (500 MHz, DMSO-d₆) δ 9.68 (s, 1H), 9.59 (s, 1H), 7.87 (s, 1H), 7.74 (d, J=8.42 Hz, 1H), 6.97 (s, 1H), 6.91 (d, J=8.11 Hz, 1H), 6.82-6.86 (m, 4H), 4.27 (s, 1H), 2.65 (s, 2H), 1.08 (s, 6H).

### Example 223

### 3-[(3,5-difluorophenyl)amino]-7-(3-hydroxy-3-methylbutyl)-8-methoxy-5,10-dihydro-11H-dibenzo[b,e][1,4]diazepin-11-one

### Example 223A

### N-(3-bromo-4-methoxyphenyl)acetamide

A mixture of 2-bromo-1-methoxy-4-nitro-benzene (5.7 g, 24.6 mmol) and SnCl₂•2H₂O (16.6 g, 73.7 mmol) in 100 mL of MeOH and 50 mL of concentrated HCl was heated under reflux overnight. After the solution cooled to room temperature, it was extracted with EtOAc several times. The combined organic layers were washed with brine, dried (MgSO₄), filtered, and concentrated under vacuum. The residue was diluted with 100 mL of CH₂Cl₂, and treated with excess Ac₂O and Et₃N at 0 °C. The solution was stirred at room temperature overnight, and washed with brine, dried (MgSO₄), filtered, and concentrated under vacuum to give the title compound. MS (DCI) m/e 245 (M+H)⁺.

### Example 223B

### N-(5-bromo-4-methoxy-2-nitrophenyl)acetamide

Concentrated nitric acid (10 mL, >69% pure) was added to acetic anhydride (50 mL) cooled to -10 °C. The solution was treated portionwise with Example 223A (5.08 g, 20.8 mmol) at a rate which maintained an internal temperature below -5 °C. The solution was stirred for 1 hour while warming to room temperature. The solution was poured into an ice/water mixture and extracted several times with ethyl acetate. The combined extracts were washed with 10% Na₂CO₃ and brine, dried (MgSO₄), filtered, and concentrated under vacuum. The residue was purified by flash column chromatography on silica gel with 3:7 hexanes/ethyl acetate to provide 5.88 g (97%) of the title compound. MS (DCI) m/e 290 (M+H)⁺; ¹H NMR (500 MHz, DMSO-d₆) δ 10.11 (s, 1H), 7.87 (s, 1H), 7.61 (s, 1H), 3.93 (s, 3H), 2.04 (s, 3H).

### Example 223C

### 5-bromo-4-methoxy-2-nitroaniline

Example 223B (5.4 g, 18.7 mmol) in 400 mL of MeOH and 10 mL of concentrated H₂SO₄ was heated under reflux for 4 hours. The solvent was removed under vacuum, and the residue was partitioned between EtOAc and H₂O. The organic layer was separated, washed with brine, dried (MgSO₄), filtrated and concentrated under vacuum to 4.4 g of the desired product. MS (DCI) m/e 248 (M+H)⁺; ¹H NMR (500 MHz, DMSO-d₆) δ 7.48 (s, 1H), 7.39 (s, 1H), 3.81 (s, 3H).

### Example 223D

### methyl 2-[(5-bromo-4-methoxy-2-nitrophenyl)amino]-4-chlorobenzoate

The title compound was prepared by substituting Example 223C for methyl 3,4-diaminobenzoate in Example 1A. MS (DCI) m/e 248 (M+H)⁺; ¹H NMR (500 MHz, DMSO-d₆) δ 10.50 (s, 1H), 7.91-7.95 (m, 2H), 7.74 (s, 1H), 7.32 (d, J=2.03 Hz, 1H), 7.05 (dd, J=8.48, 2.03 Hz, 1H), 3.95 (s, 3H), 3.88 (s, 3H).

### Example 223E

### methyl 2-[(2-amino-5-bromo-4-methoxyphenyl)amino]-4-chlorobenzoate

The title compound was prepared by substituting Example 223D for Example 6B in Example 6C. MS (DCI) m/e 248 (M+H)⁺; ¹H NMR (500 MHz, DMSO-d₆) δ 8.71 (s, 1H), 7.84 (d, J=8.42 Hz, 1H), 7.15 (s, 1H), 6.70 (dd, J=8.58, 2.03 Hz, 1H), 6.56 (s, 1H), 6.28 (d, J=2.18 Hz, 1H), 5.22 (s, 2H), 3.85 (s, 3H), 3.79 (s, 3H).

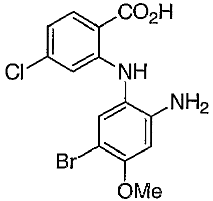

### Example 223F

### 2-[(2-amino-5-bromo-4-methoxyphenyl)amino]-4-chlorobenzoic acid

The title compound was prepared by substituting Example 223E for Example 12 in Example 13. MS (DCI) m/e 372 (M+H)⁺.

### Example 223G

### 7-bromo-3-chloro-8-methoxy-5,10-dihydro-11H-dibenzo[b,e][1,4]diazepin-11-one

A mixture of Example 223F (0.35 g, 0.94 mmol), HATU (0.430 g, 1.13 mmol), and excess Et₃N in 4 mL of DMF was stirred overnight. The reaction mixture was partitioned between EtOAc and H₂O. The organic layer was washed with brine, dried (MgSO₄), filtrated and concentrated to give 0.34 g of the title compound. MS (DCI) m/e 248 (M+H)⁺; ¹H NMR (500 MHz, DMSO-d₆) δ 9.89 (s, 1H), 7.90 (s, 1H), 7.67 (d, J=8.42 Hz, 1H), 7.19 (s, 1H), 7.00 (d, J=1.87 Hz, 1H), 6.93 (dd, J=8.42, 2.18 Hz, 1H), 6.75 (s, 1H), 5.22 (s, 2H), 3.73 (s, 3H).

### Example 223H

### methyl 3-(3-chloro-8-methoxy-11-oxo-10,11-dihydro-5H-dibenzo[b,e][1,4]diazepin-7-yl)acrylate

A mixture of Example 223G (71 mg, 0.2 mmol), methyl acrylate (64 mg, 0.8 mmol), Pd(dppf)Cl₂ (33 mg, 0.04 mmol) and 1mL Et₃N in 3 mL of DMF was heated at 110 °C overnight. The reaction mixture was diluted with EtOAc when it was still warm, and poured onto water. The organic layer was separated, washed with brine, dried (MSO₄), filtered and concentrated. The residue was triturated with 20 mL of 1: 1 EtOH/Aceton to give 60 mg of the title compound. MS (DCI) m/e 359 (M+H)⁺; ¹H NMR (500 MHz, DMSO-d₆) δ 10.04 (s, 1H), 7.91 (s, 1H), 7.86-7.75 (m, 2H), 7.25 (s, 1H), 7.03 (d, J=1.53 Hz, 1H), 6.93 (dd, J=8.59, 1.84 Hz, 1H), 6.75 (s, 1H), 6.35 (d, J=15.96 Hz, 1H), 3.78 (s, 3H), 3.77 (s, 3H).

### Example 223I

### methyl 3-(3-chloro-8-methoxy-11-oxo-10,11-dihydro-5H-dibenzo[b,e][1,4]diazepin-7-yl)propanoate

The title compound was prepared by substituting Example 223H for Example 6B in Example 6C. MS (DCI) m/e 361 (M+H)⁺; ¹H NMR (500 MHz, DMSO-d₆) δ 9.74 (s, 1H), 7.78 (s, 1H), 7.66 (d, J=8.42 Hz, 1H), 7.03 (d, J=1.87 Hz, 1H), 6.89 (dd, J=8.58, 2.03 Hz, 1H), 6.76 (s, 1H), 6.62 (s, 1H), 3.69 (s, 3H), 3.58 (s, 3H), 2.68-2.71 (m, 2H), 2.49-2.52 (m, 2H).

### Example 223J

### 3-chloro-7-(3-hydroxy-3-methylbutyl)-8-methoxy-5,10-dihydro-11H-dibenzo[b,e][1,4]diazepin-11-one

The title compound was prepared by substituting Example 223I for Example 1B in Example 189A. MS (DCI) m/e 361 (M+H)⁺; ¹H NMR (500 MHz, DMSO-d₆) δ 9.74 (s, 1H), 7.76 (s, 1H), 7.66 (d, J=8.24 Hz, 1H), 7.02 (d, J=2.14 Hz, 1H), 6.89 (dd, J=8.39, 1.98 Hz, 1H), 6.76 (s, 1H), 6.59 (s, 1H), 4.20 (s, 1H), 3.68 (s, 3H), 2.45-2.48 (m, 2H), 1.51-1.55 (m, 2H), 1.12 (s, 6H).

### Example 223K

### 3-[(3,5-difluorophenyl)amino]-7-(3-hydroxy-3-methylbutyl)-8-methoxy-5,10-dihydro-11H-dibenzo[b,e][1,4]diazepin-11-one

The title compound was prepared by substituting Example 223J and Example 203A for Example 189A and 4-aminopyridine in Example 191. MS (DCI) m/e 455 (M+H)⁺; ¹H NMR (500 MHz, DMSO-d₆) δ 9.67 (s, 1H), 9.52 (s, 1H), 7.67 (m, 2H), 6.87 (m, 1H), 6.76 (m, 1H), 6.56-6.59 (m, 4H), 4.20 (s, 1H), 3.68 (s, 3H), 2.45-2.48 (m, 2H), 1.51-1.55 (m, 2H), 1.12 (s, 6H).

### Example 224

### 7-(3-hydroxy-3-methylbutyl)-8-methoxy-3-(3-methoxy-4-nitrophenyl)-5,10-dihydro-11H-dibenzo[b,e][1,4]diazepin-11-one

The title compound was prepared by substituting Example 223J and Example 266G for Example 59B and Example 56A, respectively, in Example 59C. MS (DCI) m/e 478 (M+H)⁺; ¹H NMR (500 MHz, DMSO-d₆) δ 9.74 (s, 1H), 7.76 (s, 1H), 7.66 (d, J=8.24 Hz, 1H), 7.02 (d, J=2.14 Hz, 1H), 6.89 (dd, J=8.39, 1.98 Hz, 1H), 6.76 (s, 1H), 6.59 (s, 1H), 4.20 (s, 1H), 3.68 (s, 3H), 2.45-2.48 (m, 2H), 1.51-1.55 (m, 2H), 1.12 (s, 6H).

### Example 225

### 3-[(2_{.}6-difluoropyridin-4-yl)amino]-7-(3-hydroxypropyl)-8-methoxy-5,10-dihydro-11H-dibenzo[b,e][1,4]diazepin-11-one

### Example 225A

### 3-chloro-7-(3-hydroxypropyl)-8-methoxy-5,10-dihydro-11H-dibenzo[b,e][1,4]diazepin-11-one

The title compound was prepared by substituting Example 223I for Example 6D in Example 204A. MS (DCI) m/e 333 (M+H)⁺; ¹H NMR (500 MHz, DMSO-d₆) δ 9.71 (s, 1H), 7.75 (s, 1H), 7.66 (d, J=8.73 Hz, 1H), 7.02 (d, J=1.87 Hz, 1H), 6.89 (dd, J=8.42, 2.18 Hz, 1H), 6.75 (s, 1H), 6.60 (s, 1H), 4.39 (t, J=5.15 Hz, 1H), 3.68 (s, 3H), 2.37-2.41 (m, 2H), 2.44-2.50 (m, 2H), 1.59-1.65 (m, 2H).

### Example 225B

### 3-[(2,6-difluoropyridin-4-yl)amino]-7-(3-hydroxypropyl)-8-methoxy-5,10-dihydro-11H-dibenzo[b,e][1,4]diazepin-11-one

The title compound was prepared by substituting Example 225A and Example 203A for Example 189A and 4-aminopyridine in Example 191A. MS (DCI) m/e 427 (M+H)⁺; ¹H NMR (500 MHz, DMSO-d₆) δ 9.67 (s, 1H), 9.53 (s, 1H), 7.62-7.68 (m, 2H), 6.87 (s, 1H), 6.75 (s, 1H), 6.67 (d, J=7.93 Hz, 1H), 6.60 (s, 1H), 6.57 (s, 2H), 4.42 (t, J=5.15 Hz, 1H), 3.68 (s, 3H), 3.37-3.41 (m, 2H), 2.44-2.50 (m, 2H), 1.59-1.65 (m, 2H).

### Example 226

### 7-(3-hydroxypropyl)-8-methoxy-3-(3-methoxy-4-nitrophenyl)-5,10-dihydro-11H-dibenzo[b,e][1,4]diazepin-11-one

The title compound was prepared by substituting Example 225A and Example 266G for Example 59B and Example 56A, respectively, in Example 59C. MS (DCI) m/e 450 (M+H)⁺; ¹H NMR (500 MHz, DMSO-d₆) δ 9.72 (s, 1H), 8.00 (d, J=8.42 Hz, 1H), 7.78 (d, J=8.42 Hz, 1H), 7.52 (d, J=1.56 Hz, 1H), 7.32-7.35 (m, 2H), 7.27 (dd, J=8.26, 1.72 Hz, 1H), 6.81 (s, 1H), 6.62 (s, 1H), 4.40 (t, J=5.15 Hz, 1H), 3.68 (s, 3H), 3.38-3.42 (m, 2H), 2.44-2.50 (m, 2H), 1.61-1.64 (m, 2H).

### Example 227

### 3-[(2,6-difluoropyridin-4-yl)amino]-8-(3-hydroxy-3-methylbutyl)-5,10-dihydro-11H-dibenzo[b,e][1,4]diazepin-11-one

### Example 227A

### methyl 3-[4-(acetylamino)phenyl]propanoate

3-(4-Aminophenyl)propionic acid (5.0 g, 30 mmol) in 100 mL of CH₂Cl₂ and 5 mL of MeOH was treated with 2.0 M TMSCHN₂ in hexane dropwise (40 mL, 80 mmol). After bubbling ceased, the solvents were removed under vacuum. The residue was diluted with CH₂Cl₂, and treated with excess Ac₂O and Et₃N. The solution was stirred overnight and washed with water, brine, dried (MgSO4), filtered, and concentrated under vacuum to give the title compound. MS (DCI) m/e 222 (M+H)⁺.

### Example 227B

### methyl 3-[4-(acetylamino)-3-nitrophenyl]propanoate

The title compound was prepared by substituting Example 227A for Example 223A in Example 224B. MS (DCI) m/e 267 (M+H)⁺.

### Example 227C

### methyl 3-(4-amino-3-nitrophenyl)propanoate

The title compound was prepared by substituting Example 227B for Example 223B in Example 223C. MS (DCI) m/e 225 (M+H)⁺; ¹H NMR (500 MHz, DMSO-d₆) δ 7.78 (d, J=2.03 Hz, 1H), 7.28-7.32 (m, 2H), 6.94 (d, J=8.11 Hz, 1H), 3.57 (s, 3H), 2.74 (d, J=7.29 Hz, 2H), 2.56-2.61 (t, J=7.29 Hz, 2H).

### Example 227D

### methyl 4-chloro-2-{[4-(3-methoxy-3-oxopropyl)-2-nitrophenyl]amino}benzoate

The title compound was prepared by substituting Example 227C for methyl 3,4-diaminobenzoate in Example 1A. MS (DCI) m/e 393 (M+H)⁺; ¹H NMR (500 MHz, DMSO-d₆) δ 10.77 (s, 1H), 8.01 (d, J=1.69 Hz, 1H), 7.96 (d, J=8.48 Hz, 1H), 7.56-7.65 (m, 2H), 7.41 (d, J=2.03 Hz, 1H), 7.09 (dd, J=8.81, 2.03 Hz, 1H), 3.89 (s, 3H), 3.60 (s, 3H), 2.90 (t, J=7.46 Hz, 2H), 2.59 (t, J=7.29 Hz, 2H).

### Example 227E

### methyl 2-{[2-amino-4-(3-methoxy-3-oxopropyl)phenyl]amino}-4-chlorobenzoate

The title compound was prepared by substituting Example 227D for Example 6B in Example 6C. MS (DCI) m/e 363 (M+H)⁺; ¹H NMR (500 MHz, DMSO-d₆) δ 8.79 (s, 1H), 7.84 (d, J=8.82 Hz, 1H), 6.92 (d, J=7.80 Hz, 1H), 6.69 (dd, J=8.48, 2.03 Hz, 1H), 6.66 (d, J=1.70 Hz, 1H), 6.47 (dd, J=7.97, 1.87 Hz, 1H), 6.37 (d, J=2.03 Hz, 1H), 4.94 (s, 2H), 3.85 (s, 3H), 3.60 (s, 3H), 2.76 (t, J=7.46 Hz, 2H), 2.61 (t, J=7.29 Hz, 2H).

### Example 227F

### methyl 3-(3-chloro-11-oxo-10,11-dihydro-5H-dibenzo[b,e][1,4]diazepin-8-yl)propanoate

The title compound was prepared by substituting Example 227E for Example 6C in Example 6D. MS (DCI) m/e 331 (M+H)⁺; ¹H NMR (500 MHz, DMSO-d₆) δ 9.82 (s, 1H), 7.96 (s, 1H), 7.67 (d, J=8.42 Hz, 1H), 7.05 (d, J=2.18 Hz, 1H), 6.91 (dd, J=8.42, 1.87 Hz, 1H), 6.87-6.88 (m, 1H), 6.81-6.83 (m, 2H), 3.58 (s, 3H), 2.72 (t, J=7.49 Hz, 2H), 2.55 (t, J=7.64 Hz, 2H).

### Example 227G

### 3-chloro-8-(3-hydroxy-3-methylbutyl)-5,10-dihydro-11H-dibenzo[b,e][1,4]diazepin-11-one

The desired product was prepared by substituting Example 227F for Example 1B in Example 189A. MS (DCI) m/e 331 (M+H)⁺; ¹H NMR (500 MHz, DMSO-d₆) δ 9.80 (s, 1H), 7.93 (s, 1H), 7.67 (d, J=8.59 Hz, 1H), 7.05 (d, J=2.15 Hz, 1H), 6.91 (dd, J=8.44, 1.99 Hz, 1H), 6.86-6.89 (m, 1H), 6.78-6.81 (m, 2H), 4.21 (s, 1H), 2.46 (m, 2H), 1.54-1.58 (m, 2H), 1.10 (s, 6H).

### Example 227H

### 3-[(2,6-difluoropyridin-4-yl)amino]-8-(3-hydroxy-3-methylbutyl)-5,10-dihydro-11H-dibenzo[b,e][1,4]diazepin-11-one

The title compound was prepared by substituting Example 227G and Example 203A for Example 189A and 4-aminopyridine in Example 191. MS (DCI) m/e 425 (M+H)⁺; ¹H NMR (500 MHz, DMSO-d₆) δ 9.65 (s, 1H), 9.58 (s, 1H), 7.82 (s, 1H), 7.68 (d, J=8.59 Hz, 1H), 6.84-6.88 (m, 2H), 6.75-6.77 (m, 2H), 6.67 (dd, J=8.59, 1.83 Hz, 1H), 6.55 (s, 2H), 4.20 (s, 1H), 2.44-2.50 (m, 2H), 1.53-1.57 (m, 2H), 1.11 (s, 6H).

### Example 228

### 8-(3-hydroxy-3-methylbutyl)-3-(3-methoxy-4-nitrophenyl)-5,10-dihydro-11H-dibenzo[b,e][1,4]diazepin-11-one

The title compound was prepared by substituting Example 227G and Example 266G for Example 59B and Example 56A, respectively, in Example 59C. MS (DCI) m/e 448 (M+H)⁺; ¹H NMR (500 MHz, DMSO-d₆) δ 9.80 (s, 1H), 8.01 (d, J=8.42 Hz, 1H), 7.90 (s, 1H), 7.80 (d, J=8.11 Hz, 1H), 7.52 (d, J=1.25 Hz, 1H), 7.34-7.35 (m, 2H), 7.29 (dd, J=8.11, 1.56 Hz, 1H), 6.92 (d, J=8.11 Hz, 1H), 6.79-6.81 (m, 2H), 4.21 (s, 1H), 4.03 (s, 3H), 2.48-2.50 (m, 2H), 1.56-1.59 (m, 2H), 1.12 (s, 6H).

### Example 229

### methyl 3-[3-(3-methoxy-4-nitrophenyl)-11-oxo-10,11-dihydro-5H-dibenzo[b,e][1,4]diazepin-8-yl]propanoate

The title compound was prepared by substituting Example 227F and Example 266G for Example 59B and Example 56A, respectively, in Example 59C. MS (DCI) m/e 448 (M+H)⁺; ¹H NMR (500 MHz, DMSO-d₆) δ 9.83 (s, 1H), 8.01 (d, J=8.42 Hz, 1H), 7.93 (s, 1H), 7.79 (d, J=8.42 Hz, 1H), 7.52 (d, J=1.56 Hz, 1H), 7.33-7.35 (m, 2H), 7.29 (dd, J=8.26, 1.72 Hz, 1H), 6.92 (d, J=8.11 Hz, 1H), 6.82-6.83 (m, 2H), 4.03 (s, 3H), 3.58 (s, 3H), 2.73 (t, J=7.49 Hz, 2H), 2.55 (t, J=7.49 Hz, 2H).

### Example 230

### 3-[3-(3-methoxy-4-nitrophenyl)-11-oxo-10,11-dihydro-5H-dibenzo[b,e][1,4]diazepin-8-yl]propanoic acid

The desired product was prepared by substituting Example 229 for Example 12 in Example13. MS (ESI) m/e 434 (M+H)⁺; ¹H NMR (300 MHz, DMSO-d₆) δ 12.04 (br, 1H), 9.81 (s, 1H), 8.01 (d, J=8.14 Hz, 1H), 7.94 (s, 1H), 7.79 (d, J=8.14 Hz, 1H), 7.52 (d, J=1.70 Hz, 1H), 7.30-7.34 (m, 3H), 6.93 (m, 1H), 6.83 (d, J=5.09 Hz, 2H), 4.03 (s, 3H), 2.69 (t, J=7.46 Hz, 2H), 2.45 (t, J=7.46 Hz, 2H).

### Example 231

### 3-[3-(3-methoxy-4-nitrophenyl)-11-oxo-10,11-dihydro-5H-dibenzo[b,e][1,4]diazepin-8-yl]-N,N-dimethylpropanamide

The desired product was prepared by substituting Example 230 and N, N-dimethylamine hydrochloride for dimethylaminoacetic acid and Example 120, respectively, in Example 122. MS (ESI) m/e 461 (M+H)⁺; ¹H NMR (300 MHz, DMSO-d₆) δ 9.83 (s, 1H), 8.01 (d, J=8.14 Hz, 1H)), 7.93 (s, 1H), 7.79 (d, J=8.14 Hz, 1H), 7.5 (d, J=1.7 Hz, 1H), 7.3 (m, 3H), 6.9 (m, 1H), 6.83 (d, J=5.43 Hz, 2H), 4.03 (s, 3H), 2.92 (s, 3H), 2.80 (s, 3H), 2.67 (t, J=7.46, 2H), 2.50-2.52 (m, 2H).

### Example 232

### 3-[3-(3-methoxy-4-nitrophenyl)-11-oxo-10,11-dihydro-5H-dibenzo[b,e][1,4]diazepin-8-yl]N(4-morpholin-4-ylphenyl)propanamide

The desired product was prepared by substituting Example 230 and 4-morpholin-4-ylphenylamine for dimethylaminoacetic acid and Example 120, respectively, in Example 122. MS (ESI) m/e 594 (M+H)⁺; ¹H NMR (300 MHz, DMSO-d₆) δ 9.78 (s, 1H), 9.7 (s, 1H), 7.93 (d, J=8.42 Hz, 1H), 7.85 (s, 1H), 7.72 (d, J=8.11 Hz, 1H), 7.45 (d, J=1.56 Hz, 1H), 7.34 (d, J=9.04 Hz, 2H), 7.27 (m, 2H), 7.22 (dd, J=8.26, 1.72 Hz, 1H), 6.86 (d, J=8.11 Hz, 1H), 6.75-6.79 (m, 4H), 3.96 (s, 3H), 3.64 (br, m, 4H), 2.95 (br, m, 4H), 2.65-2.68 (m, 2H), 2.43-2.45 (m, 2H).

### Example 233

### 8-(3-azetidin-1-yl-3-oxopropyl)-3-(3-methoxy-4-nitrophenyl)-5,10-dihydro-11H-dibenzo[b,e][1,4]diazepin-11-one

The desired product was prepared by substituting Example 230 and azetidine for dimethylaminoacetic acid and Example 120, respectively, in Example 122. MS (ESI) m/e 473 (M+H)⁺; ¹H NMR (300 MHz, DMSO-d₆) δ 9.84 (s, 1H), 8.01 (d, J=8.48 Hz, 1H), 7.94 (s, 1H), 7.79 (d, J=8.14 Hz, 1H), 7.52 (d, J=1.70 Hz, 1H), 7.34 (m, 2H), 7.29 (dd, J=8.14, 1.7 Hz, 1H), 6.90 (m, 1H), 6.82 (m, 2H), 4.03 (s, 3H), 3.99 (d, J=7.46 Hz, 2H), 3.8 (t, J=7.80 Hz, 2H), 2.65 (t, J=7.63 Hz, 2H), 2.30 (t, J=7.80 Hz, 2H), 2.12 (m, 2H).

### Example 234

### 3-(3-methoxy-4-nitrophenyl)-8-(3-oxo-3-pyrrolidin-1-ylpropyl)-5,10-dihydro-11H-dibenzo[b,e][1,4]diazepin-11-one

The desired product was prepared by substituting Example 230 and pyrrolidine for dimethylaminoacetic acid and Example 120, respectively, in Example 122. MS (ESI) m/e 487 (M+H)⁺; ¹H NMR (300 MHz, DMSO-d₆) δ 9.83 (s, 1H), 8.01 (d, J=8.48 Hz, 1H), 7.92 (s, 1H), 7.79 (d, J=8.14 Hz, 1H), 7.52 (d, J=1.7 Hz, 1H), 7.34 (m, 2H), 7.30 (dd, J=8.31, 1.86 Hz, 1H), 6.92 (m, 1H), 6.83 (m, 2H), 4.03 (s, 3H), 3.35-3.24 (br, 4H), 2.69 (t, J=7.63 Hz, 2H), 2.44 (d, J=7.46 Hz, 2H), 1.85-1.70 (br, 4H).

### Example 235

### 3-(3-methoxy-4-nitrophenyl)-8-(3-morpholin-4-yl-3-oxopropyl)-5,10-dihydro-11H-dibenzo[b,e][1,4]diazepin-11-one

The desired product was prepared by substituting Example 230 and morpholine for dimethylaminoacetic acid and Example 120, respectively, in Example 122. MS (ESI) m/e 503 (M+H)⁺; ¹H NMR (300 MHz, DMSO-d₆) & 9.83 (s, 1H), 8.01 (d, J=8.14 Hz, 1H), 7.94 (s, 1H), 7.79 (d, J=8.14 Hz, 1H), 7.52 (d, J=1.70 Hz, 1H), 7.34 (m, 1H), 7.29 (dd, J=8.31, 1.87 Hz, 2H), 6.92 (m, 2H), 6.85 (m, 2H), 4.03 (s, 3H), 3.41 (m, 8H), 2.69 (t, J=7.29 Hz, 2H), 2.55 (d, J=7.46 Hz, 2H).

### Example 236

### N,N-diethyl-3-[3-(3-methoxy-4-nitrophenyl)-11-oxo-10,11-dihydro-5H-dibenzo[b,e][1,4]diazepin-8-yl]propanamide

The desired product was prepared by substituting Example 230 and diethylamine for dimethylaminoacetic acid and Example 120, respectively, in Example 122. MS (ESI) m/e 489 (M+H)⁺; ¹H NMR (300 MHz, DMSO-d₆) & 9.83(s, 1H), 8.01 (d, J=8.48 Hz, 1H), 7.93 (s, 1H), 7.79 (d, J=8.14 Hz, 1H), 7.52 (d, J=1.7 Hz, 1H), 7.33 (m, 2H), 7.29 (dd, J=8.14, 1.70 Hz, 1H), 6.92 (m, 1H), 6.84 (d, J=5.43 Hz, 2H), 4.03 (s, 3H), 3.24 (m, 4H), 2.69 (t, J=7.63 Hz, 2H), 2.5-2.43 (m, 2H), 1.02 (br, 6H).

### Example 237

### 8-[3-(4-hydroxypiperidin-1-yl)-3-oxopropyl]-3-(3-methoxy-4-nitrophenyl)-5,10-dihydro-11H-dibenzo[b,e][1,4]diazepin-11-one

The desired product was prepared by substituting Example 230 and 4-hydroxypiperidine for dimethylaminoacetic acid and Example 120, respectively, in Example 122. MS (ESI) m/e517 (M+H)⁺; ¹H NMR (300 MHz, DMSO-d₆) δ 9.82 (s, 1H), 8.01 (d, J=8.14 Hz, 1H), 7.93 (s, 1H), 7.79 (d, J=8.14 Hz, 1H), 7.52 (d, J=1.36 Hz, 1H), 7.34 (m, 2H), 7.29 (dd, J=8.14, 1.70 Hz, 1H), 6.92 (m, 1H), 6.84 (d, J=5.09 Hz, 2H), 4.69 (s, 1H), 4.03 (s, 3H), 3.65 (s, 2H), 3.2-2.95 (br, 4H), 2.67 (t, J=7.29 Hz, 2H), 1.65 (d, J=9.83 Hz, 2H), 1.24 (m, 2H).

### Example 238

### 3-[3-(3-methoxy-4-nitrophenyl)-11-oxo-10,11-dihydro-5H-dibenzo[b,e][1,4]diazepin-8-yl]N1,3-thiazol-2-ylpropanamide

The desired product was prepared by substituting Example 230 and thiazol-2-ylamine for dimethylaminoacetic acid and Example 120, respectively, in Example 122. MS (ESI) m/e517 (M+H)⁺; ¹H NMR (300 MHz, DMSO-d₆) δ 12.08 (s, 1H), 9.87 (s, 1H), 8.01 (d, J=8.48 Hz, 1H), 7.94 (s, 1H), 7.79 (d, J=8.14 Hz, 1H), 7.52 (d, J=1.7 Hz, 1H), 7.43 (d, J=3.73 Hz, 1H), 7.33 (m, 2H), 7.29 (dd, J=8.14, 1.70 Hz, 1H), 7.18 (d, J=3.73 Hz, 1H), 6.93 (m, 1H), 6.83 (m, 2H), 4.03 (s, 3H), 2.79 (d, J=7.46 Hz, 2H), 2.70 (m, 2H).

### Example 239

### 8-(2-hydroxyethyl)-3-(3-methoxy-4-nitrophenyl)-5,10-dihydro-11H-dibenzo[b,e][1,4]diazepin-11-one

The title compound was prepared by substituting Example 204A and Example 266G for Example 59B and Example 56A, respectively, in Example 59C. MS (DCI) m/e 406 (M+H)⁺; ¹H NMR (500 MHz, DMSO-d₆) δ 9.80 (s, 1H), 7.99 (d, J=8.42 Hz, 1H), 7.89 (s, 1H), 7.78 (d, J=8.11 Hz, 1H), 7.51 (d, J=1.56 Hz, 1H), 7.32-7.34 (m, 2H), 7.28 (dd, J=8.11, 1.56 Hz, 1H), 6.91 (d, J=7.80 Hz, 1H), 6.80-6.82 (m, 2H), 4.57 (t, J=5.15 Hz, 1H), 4.02 (s, 3H), 3.50-3.54 (m, 2H), 2.58 (t, J=7.52 Hz, 2H).

### Example 240

### 8-(3-hydroxypropyl)-3-(3-methoxy-4-nitrophenyl)-5,10-dihydro-11H-dibenzo[b,e][1,4]diazepin-11-one

### Example 240A

### 3-chloro-8-(3-hydroxypropyl)-5,10-dihydro-11H-dibenzo[b,e][1,4]diazpin-11-one

The title compound was prepared by substituting Example 227F for Example 6D in Example 204A. MS (DCI) m/e 289 (M+H)⁺; ¹H NMR (500 MHz, DMSO-d₆) δ 9.81 (s, 1H), 7.94 (s, 1H), 7.68 (d, J=8.42 Hz, 1H), 7.06 (d, J=1.87 Hz, 1H), 6.91 (dd, J=8.42, 1.87 Hz, 1H), 6.88 (m, 1H), 6.79-6.82 (m, 2H), 4.41 (t, J=4.99 Hz, 1H), 3.37-3.41 (m, 2H), 2.46-2.49 (m, 2H), 1.62-1.67 (m, 2H).

### Example 240B

### 8-(3-hydroxypropyl)-3-(3-methoxy-4-nitrophenyl)-5,10-dihydro-11H-dibenzo[b,e][1,4]diazepin-11-one

The title compound was prepared by substituting Example 240A and Example 266G for Example 59B and Example 56A, respectively, in Example 59C. MS (DCI) m/e 420 (M+H)⁺; ¹H NMR (500 MHz, DMSO-d₆) δ 9.81 (s, 1H), 8.01 (d, J=8.42 Hz, 1H), 7.90 (s, 1H), 7.82 (d, J=8.11 Hz, 1H), 7.52 (s, 1H), 7.34-7.35 (m, 2H), 7.29 (dd, J=8.11, 1.56 Hz, 1H), 6.93 (d, J=8.11 Hz, 1H), 6.79-6.82 (m, 2H), 4.42 (t, J=4.83 Hz, 1H), 4.04 (s, 3H), 3.40 (q, J=5.93 Hz, 2H), 2.54-2.50 (m, 2H), 1.63-1.67 (m, 2H).

### Example 241

### methyl 3-[3-(4-chloro-3-methoxyphenyl)-11-oxo-10,11-dihydro-5H-dibenzo[b,e][1,4]diazepin-8-yl]propanoate

### Example 241A

### methyl 3-[11-oxo-3-(4,4,5,5-tetramethyl-1,3,2-dioxaborolan-2-yl)-10,11-dihydro-5H-dibenzo[b,e][1,4]diazoin-8-yl]propanoate

The title compound was prepared by substituting Example 227F for Example 6D in Example 54A. MS (DCI) m/e 420 (M+H)⁺; ¹H NMR (500 MHz, DMSO-d₆) δ 9.79 (s, 1H), 7.74 (s, 1H), 7.65 (d, J=7.80 Hz, 1H), 7.38 (s, 1H), 7.13 (d, J=7.80 Hz, 1H), 6.90 (d, J=8.73 Hz, 1H), 6.79-6.80 (m, 2H), 3.57 (s, 3H), 2.71 (t, J=7.49 Hz, 2H), 2.54 (t, J=7.49 Hz, 2H), 1.92 (s, 12H).

### Example 241B

### methyl 3-[3-(4-chloro-3-methoxyphenyl)-11-oxo-10,11-dihydro-5H-dibenzo[b,e][1,4]diazepin-8-yl]propanoate

The desired product was prepared by substituting Example 241A and 2-chloro-5-iodoanisole for 2-methoxy-4-(4,4,5,5-tetramethyl-1,3,2-dioxaborolan-2-yl)phenol and Example 1B, respectively, in Example 10. MS (DCI) m/e 437 (M+H)⁺; ¹H NMR (500 MHz, DMSO-d₆) δ 9.77 (s, 1H), 7.87 (s, 1H), 7.76 (d, J=8.11 Hz, 1H), 7.53 (d, J=8.11 Hz, 1H), 7.33 (d, J=1.87 Hz, 1H), 7.29 (d, J=1.87 Hz, 1H), 7.19-7.23 (m, 2H), 6.92 (d, J=8.42 Hz, 1H), 6.81-6.82 (m, 2H), 3.96 (s, 3H), 3.58 (s, 3H), 2.72 (t, J=7.49 Hz, 2H), 2.55 (t, J=7.49 Hz, 2H).

### Example 242

### 3-[3-(4-chloro-3-methoxyphenyl)-11-oxo-10,11-dihydro-5H-dibenzo[b,e][1,4]diazepin-8-yl]-N,N-dimethylpropanamide

### Example 242A

### 3-[3-(4-chloro-3-methoxyphenyl)-11-oxo-10,11-dihydro-5H-dibenzo[b,e][1,4]diazepin-8-yl]propanoic acid

The desired product was prepared by substituting Example 241B for Example 12 in Example 13. MS (DCI) m/e 423 (M+H)⁺; ¹H NMR (500 MHz, DMSO-d₆) δ 12.08 (s, 1H), 9.76 (s, 1H), 7.87 (s, 1H), 7.77 (d, J=8.11 Hz, 1H), 7.53 (d, J=8.11 Hz, 1H), 7.34 (d, J=1.87 Hz, 1H), 7.29 (d, J=1.56 Hz, 1H), 7.19-7.23 (m, 2H), 6.93 (d, J=7.80 Hz, 1H), 6.82-6.83 (m, 2H), 3.96 (s, 3H), 2.70 (t, J=7.49 Hz, 2H), 2.46 (t, J=7.64 Hz, 2H).

### Example 242B

### 3-[3-(4-chloro-3-methoxyphenyl)-11-oxo-10,11-dihydro-5H-dibenzo[b,e][1,4]diazepin-8-yl]-N,N-dimethylpropanamide

The title compound was prepared by substituting Example 242B and dimethylamine hydrochloride for Example 120 and dimethylaminoacetic acid, respectively, in Example 122. MS (DCI) m/e 423 (M+H)⁺; ¹H NMR (500 MHz, DMSO-d₆) δ 9.75 (s, 1H), 7.86 (s, 1H), 7.76 (d, J=7.98 Hz, 1H), 7.53 (d, J=7.98 Hz, 1H), 7.33 (d, J=1.84 Hz, 1H), 7.29 (d, J=1.53 Hz, 1H), 7.19-7.23 (m, 2H), 6.91 (d, J=7.80 Hz, 1H), 6.82-6.84 (m, 2H), 3.96 (s, 3H), 2.92 (s, 3H), 2.80 (s, 3H), 2.67 (t, J=7.36 Hz, 2H), 2.50 (t, J=7.64 Hz, 2H).

### Example 243

### 3-(4-chloro-3-methoxyphenyl)-8-(3-hydroxy-3-methylbutyl)-5,10-dihydro-11H-dibenzo[b,e][1,4]diazepin-11-one

The title compound was prepared by substituting Example 241B for Example 1B in Example 189A. MS (DCI) m/e 437 (M+H)⁺; ¹H NMR (500 MHz, DMSO-d₆) δ 9.66(s, 1H), 9.59 (s, 1H), 7.84 (s, 1H), 7.69 (d, J=8.42 Hz, 1H), 6.90 (d, J=1.87 Hz, 1H), 6.84-6.86 (m, 1H), 6.81 (s, 1H), 6.78 (dd, J=7.95, 1.72 Hz, 1H), 6.68 (dd, J=8.42, 2.18 Hz, 1H), 6.57 (m, 2H), 4.22 (s, 1H), 3.96 (s, 3H), 2.48-2.50 (m, 2H), 1.56-1.59 (m, 2H), 1.12 (s, 6H).

### Example 244

### 3-(3-methoxy-4-nitrophenyl)-8-[2-(3-methyl-1,2,4-oxadiazol-5-yl)ethyl]-5,10-dihydro-11H-dibenzo[b,e][1,4]diazepin-11-one

### Example 244A

### N-({3-[3-(3-methoxy-4-nitrophenyl)-11-oxo-10,11-dihydro-5H-dibenzo[b,e][1,4]diazepin-8-yl]propanoyl}oxy)ethanimidamide

A mixture of Example 230 (65 mg, 0.15 mmol), N-hydroxy-acetamidine (14 mg, 0.18 mmol) in 1 mL of DMF was treated with DIC in 1 mL of CH₂Cl₂ at 0 °C. The reaction mixture was stirred overnight and partitioned between EtOAc and water. The organic layer was separated, washed with brine, dried (MgSO4), filtered, and concentrated under vacuum. The residue was purified by flash column chromatography on silica gel with 100:1 ethyl acetate/MeOH to provide the desired product. MS (DCI) m/e 490 (M+H)⁺; ¹H NMR (500 MHz, DMSO-d₆) δ 9.83 (s, 1H), 8.01 (d, J=8.29 Hz, 1H), 7.94 (s, 1H), 7.79 (d, J=8.29 Hz, 1H), 7.52 (d, J=1.23 Hz, 1H), 7.33-7.34 (m, 2H), 7.29 (m, 1H), 6.93 (m, 1H), 6.83-6.85 (m, 2H), 6.27 (s, 2H), 4.03 (s, 3H), 2.73-2.76 (m, 2H), 2.58-2.62 (m, 2H), 1.72 (s, 3H).

### Example 244B

### 3-(3-methoxy-4-nitrophenyl)-8-[2-(3-methyl-1,2,4-oxadiazol-5-yl)ethyl]-5,10-dihydro-11H-dibenzo[b,e][1,4]diazepin-11-one

Example 244A (30 mg, 0.060 mmol) in 2 mL of DMF was heated at 110 °C for 2 hours. After the reaction mixture cooled to room temperature, it was purified by preparative HPLC to give the title compound. MS (DCI) m/e 472 (M+H)⁺; ¹H NMR (500 MHz, DMSO-d₆) δ 9.82 (s, 1H), 7.99 (d, J=8.59 Hz, 1H), 7.94 (s, 1H), 7.78 (d, J=7.98 Hz, 1H), 7.51 (d, J=1.23 Hz, 1H), 7.33-7.34 (m, 2H), 7.29 (m, 1H), 6.92 (m, 1H), 6.82-6.84 (m, 2H), 4.02 (s, 3H), 2.73 (t, J=7.52 Hz, 2H), 2.59 (t, J=7.36 Hz,, 2H), 1.71 (s, 3H).

### Example 245

### methyl 3-[8-methoxy-3-(3-methoxy-4-nitrophenyl)-11-oxo-10,11-dihydro-5H-dibenzo[b,e][1,4]diazepin-7-yl]propanoate

The title compound was prepared by substituting Example 223I and Example 266G for Example 59B and Example 56A, respectively, in Example 59C. MS (DCI) m/e 478 (M+H)⁺; ¹H NMR (500 MHz, DMSO-d₆) δ 9.74 (s, 1H), 8.01 (d, J=8.42 Hz, 1H), 7.78 (d, J=8.11 Hz, 1H), 7.74 (s, 1H), 7.33-7.35 (m, 2H), 7.27 (dd, J=8.26, 1.72 Hz, 1H), 6.81 (s, 1H), 6.64 (s, 1H), 4.03 (s, 3H), 3.70 (s, 3H), 3.58 (s, 3H), 2.70 (t, J=7.49 Hz, 2H), 2.51 (t, J=7.49 Hz, 2H).

### Example 246

### 7-(2-hydroxy-2-methylpropyl)-8-methoxy-3-(3-methoxy-4-nitrophenyl)-5,10-dihydro-11H-dibenzo[b,e][1,4]diazepin-11-one

### Example 246A

### methyl (2-methoxy-5-nitrophenyl)acetate

(2-Hydroxy-phenyl)-acetic acid (6.02 g, 39.6 mmol) was suspended in 17 mL of water at 0 °C. To this solution was added 40% HNO₃ (prepared from 5 mL of concentrated HNO₃ and 3 mL of water). The reaction mixture was stirred for 2 hours at 0 °C, warmed up to room temperature, and stirred for another 30 min. The reaction mixture was poured on the water/ice, extracted with EtOAc several times, dried (MgSO₄), filtered, and concentrated under vacuum. The residue was dissolved in 100 mL of CH₂Cl₂ and 10 mL of MeOH and treated with excess of TMSCHN₂ dropwise. After bubbling ceased, the solvents were removed, and the residue was purified by flash column chromatography on silica gel with 85:15 hexanes/ethyl acetate to provide 2.67g (30%) of the desired product. MS (DCI) m/e 478 (M+H)⁺; ¹H NMR (500 MHz, DMSO-d₆) δ 8.20-8.24 (m, 2H), 7.22 (d, J=9.16 Hz, 1H), 3.91 (s, 3H), 3.76 (s, 2H), 3.61 (s, 3H).

### Example 246B

### methyl [5-(acetylamino)-2-methoxyphenyl]acetate

A mixture of Example 246A (3.8 g, 17 mmol), 5% Pd/C, methanol (50 mL) was equipped with a balloon of hydrogen gas and stirred at room temperature. After uptake of the hydrogen was complete, the solution was filtered through diatomaceous earth (Celite^{®}). The filtrate was concentrated under vacuum and the residue wastreated with excess of Ac₂O and Et₃N to give 3.5 g (88%) of the title compound. MS (DCI) m/e 238 (M+H)⁺; ¹H NMR (500 MHz, DMSO-d₆) δ 9.75 (s, 1H), 7.44 (dd, J=8.82, 2.71 Hz, 1H), 6.90 (d, J=8.82 Hz, 1H), 3.71 (s, 3H), 3.59 (s, 3H), 3.56 (s, 2H), 2.00 (s, 3H).

### Example 246C

### methyl [5-(acetylamino)-2-methoxy-4-nitrophenyl]acetate

The title compound was prepared by substituting Example 246B for Example 223A in Example 223B. MS (DCI) m/e 283 (M+H)⁺; ¹H NMR (500 MHz, DMSO-d₆) δ 10.05 (s, 1H), 7.49 (s, 1H), 7.42 (s, 1H), 3.83 (s, 3H), 3.71 (s, 2H), 3.61 (s, 3H), 2.02 (s, 3H).

### Example 246D

### methyl (5-amino-2-methoxy-4-nitrophenyl)acetate

The title compound was prepared by substituting Example 246C for Example 223B in Example 223C. MS (DCI) m/e 241 (M+H)⁺.

### Example 246E

### methyl 4-chloro-2-{[4-methoxy-5-(2-methoxy-2-oxoethyl)-2-nitrophenyl]amino}benzoate

The title compound was prepared by substituting Example 246D for methyl 3,4-diaminobenzoate in Example 1A. MS (DCI) m/e 409 (M+H)⁺; ¹H NMR (500 MHz, DMSO-d₆) δ 10.58 (s, 1H), 7.94 (d, J=8.82 Hz, 1H), 7.65-7.66 (m, 2H), 7.24 (d, J=2.03 Hz, 1H), 7.02 (dd, J=8.81, 2.03 Hz, 1H), 3.89 (s, 3H), 3.87 (s, 3H), 3.76 (s, 2H), 3.62 (s, 3H).

### Example 246F

### methyl 2- {[2-amino-4-methoxy-5-(2-methoxy-2-oxoethyl)phenyl]amino}-4-chlorobenzoate

The title compound was prepared by substituting Example 246E for Example 6B in Example 6C. MS (DCI) m/e 379 (M+H)⁺; ¹H NMR (500 MHz, DMSO-d₆) δ 8.70 (s, 1H), 7.84 (d, J=8.48 Hz, 1H), 6.82 (s, 1H), 6.67 (dd, J=8.65, 2.20 Hz, 1H), 6.45(s, 1H), 6.33 (d, J=2.03 Hz, 1H), 5.00 (s, 2H), 3.85 (s, 3H), 3.71 (s, 3H), 3.57 (s, 3H), 3.44 (s, 2H).

### Example 246G

### methyl (3-chloro-8-methoxy-11-oxo-10,11-dihydro-5H-dibenzo[b,e][1,4]diazepin-7-yl)acetate

The title compound was prepared by substituting Example 246F for Example 6C in Example 6D. MS (DCI) m/e 347 (M+H)⁺; ¹H NMR (500 MHz, DMSO-d₆) δ 9.81 (s, 1H), 7.84 (s, 1H), 7.66 (d, J=8.48 Hz, 1H), 7.04 (d, J=2.04 Hz, 1H), 6.90 (dd, J=8.48, 2.03 Hz, 1H), 6.81 (s, 1H), 6.84 (s, 1H), 3.66 (s, 3H), 3.58 (s, 3H), 3.50 (s, 2H).

### Example 246H

### 3-chloro-7-(2-hydroxy-2-methylpropyl)-8-methoxy-5,10-dihydro-11H-dibenzo[b,e][1,4]diazepin-11-one

The title compound was prepared by substituting Example 246G for Example 1B in Example 189A. MS (DCI) m/e 347 (M+H)⁺; ¹H NMR (500 MHz, DMSO-d₆) δ 9.75 (s, 1H), 7.82 (s, 1H), 7.66 (d, J=8.24 Hz, 1H), 7.05 (d, J=2.14 Hz, 1H), 6.88 (dd, J=8.39, 1.98 Hz, 1H), 6.85 (s, 1H), 6.60 (s, 1H), 4.25 (s, 1H), 3.65 (s, 3H), 2.56 (s, 2H), 1.03 (s, 6H).

### Example 246I

### 7-(2-hydroxy-2-methylpropyl)-8-methoxy-3-(3-methoxy-4-nitrophenyl)-5,10-dihydro-11H-dibenzo[b,e][1,4]diazepin-11-one

The title compound was prepared by substituting Example 246H and Example 266G for Example 59B and Example 56A, respectively, in Example 59C. MS (DCI) m/e 464 (M+H)⁺; ¹H NMR (500 MHz, DMSO-d₆) δ 9.72 (s, 1H), 8.01 (d, J=8.59 Hz, 1H), 7.78 (m, 1H), 7.51 (s, 1H), 7.32-7.35 (m, 2H), 7.26 (dd, J=7.98, 1.53 Hz, 1H), 6.89 (s, 1H), 6.63 (s, 1H), 4.22 (s, 1H), 4.03 (s, 3H), 3.67 (s, 3H), 3.67 (s, 3H), 3.65 (s, 2H), 1.04 (s, 6H).

### Example 247

### 7-(2-hydroxyethyl)-8-methoxy-3-(3-methoxy-4-nitrophenyl)-5,10-dihydro-11H-dibenzo[b,e][1,4]diazepin-11-one

### Example 247A

### 3-chloro-7-(2-hydroxyethyl)-8-methoxy-5,10-dihydro-11H-dibenzo[b,e][1,4]diazepin-11-one

The title compound was prepared by substituting Example 246G for Example 6D in Example 204A. MS (DCI) m/e 319 (M+H)⁺; ¹H NMR (500 MHz, DMSO-d₆) δ 9.72 (s, 1H), 7.77 (s, 1H), 7.66 (d, J=8.42 Hz, 1H), 7.03 (d, J=2.18 Hz, 1H), 6.88 (dd, J=8.58, 2.03 Hz, 1H), 6.78 (s, 1H), 6.61 (s, 1H), 4.56 (t, J=5.46 Hz, 1H), 3.68 (s, 3H), 3.48-3.52 (m, 2H), 2.59 (t, J=7.18 Hz, 1H).

### Example 247B

### 7-(2-hydroxyethyl)-8-methoxy-3-(3-methoxy-4-nitrobenyl)-5,10-dihydro-11H-dibenzo[b,e][1,4]diazepin-11-one

The title compound was prepared by substituting Example 247A and Example 266G for Example 59B and Example 56A, respectively, in Example 59C. MS (DCI) m/e 436 (M+H)⁺; ¹H NMR (500 MHz, DMSO-d₆) δ 9.76 (s, 1H), 8.01 (d, J=8.54 Hz, 1H), 7.80 (d, J=8.24 Hz, 1H), 7.75 (s, 1H), 7.52 (s, 1H), 7.33-7.35 (m, 2H), 7.27 (dd, J=8.24, 1.83 Hz, 1H), 6.83 (s, 1H), 6.63 (s, 1H), 4.59 (t, J=5.34 Hz, 1H), 4.03 (s, 3H), 3.69 (s, 3H), 3.48-3.52 (m, 2H), 2.60 (t, J=7.02 Hz, 2H).

### Example 248

### 8-methoxy-3-(3-methoxy-4-nitrophenyl)-7-(2-oxopropyl)-5,10-dihydro-11H-dibenzo[b,e][1,4]diazepin-11-one

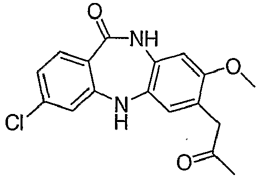

### Example 248A

### 3-chloro-8-methoxy-7-(2-oxopropyl)-5,10-dihydro-11H-dibenzo[b,e][1,4]diazepin-11-one

The title compound was isolated as a minor product in Example 246H. MS (DCI) m/e 331 (M+H)⁺; ¹H NMR (500 MHz, DMSO-d₆) δ 9.81 (s, 1H), 7.82 (s, 1H), 7.67 (d, J=8.54 Hz, 1H), 7.03 (d, J=1.83 Hz, 1H), 6.90 (dd, J=8.39, 1.98 Hz, 1H), 6.73 (s, 1H), 6.64 (s, 1H), 3.65 (s, 3H), 3.56 (s, 3H), 2.08 (s, 2H).

### Example 248B

### 8-methoxy-3-(3-methoxy-4-nitrophenyl)-7-(2-oxopropyl)-5,10-dihydro-11H-dibenzo[b,e][1,4]diazepin-11-one

The title compound was prepared by substituting Example 248A and Example 266G for Example 59B and Example 56A, respectively, in Example 59C. MS (DCI) m/e 448 (M+H)⁺; ¹H NMR (500 MHz, DMSO-d₆) δ 9.79 (s, 1H), 8.01 (d, J=8.59 Hz, 1H), 7.77-7.80 (m, 2H), 7.52 (s, 1H), 7.32-7.35 (m, 2H), 7.28 (d, J=8.29 Hz, 1H), 6.78 (s, 1H), 6.66 (s, 1H), 4.03 (s, 3H), 3.67 (s, 3H), 3.55 (s, 2H), 2.08 (s, 2H).

### Example 249

### 7-(2-hydroxy-1,1-dimethylethyl)-8-methoxy3-(3-methoxy-4-nitrophenyl)-5,10-dihydro-11H-dibenzo[b,e][1,4]diazepin-11-one

### Example 249A

### methyl 2-(2-methoxy-5-nitrophenyl)-2-methylpropanoate

A mixture of Example 246A (1.5 g, 6.66 mmol), MeI (3.78 g, 26.6 mmol), and 18-crown-6 (0.301 g, 1.14 mmol) in 20 mL of anhydrous DMF was cooled to 0 °C. To this solution was added 60% NaH (0.64 g, 16 mmol). The solution was stirred at 0 °C for 30 min and warmed up gradually overnight. The reaction mixture was partitioned between EtOAc and water. The aqueous layer was extracted with additional EtOAc several times. The combined organic layers were washed by brine, dried (MgSO₄), filtered, and concentrated under vacuum. The residue was purified by flash column chromatography on silica gel with 9:1 hexanes/ethyl acetate to provide 1.3 g (77%) of the desired product. MS (DCI) m/e 254 (M+H)⁺; ¹H NMR (500 MHz, DMSO-d₆) δ 8.22 (dd, J=8.99, 2.88 Hz, 1H), 8.12 (d, J=2.71 Hz, 1H), 7.24 (d, J=8.24 Hz, 1H), 3.88 (s, 3H), 3.55 (s, 3H), 1.55 (s, 6H).

### Example 249B

### methyl 2-[5-(acetylamino)-2-methoxyphenyl]-2-methylpropanoate

The title compound was prepared by substituting Example 249A for Example 246A in Example 246B. MS (DCI) m/e 266 (M+H)⁺; ¹H NMR (500 MHz, DMSO-d₆) δ 9.76, (s, 1H), 7.46-7.50 (m, 1H), 7.43 (d, J=2.71 Hz, 1H), 6.90 (d, J=8.82 Hz, 1H), 3.67 (s, 3H), 3.52 (s, 3H), 2.00 (s, 3H), 1.39 (s, 6H).

### Example 249C

### methyl 2-[5-(acetylamino)-2-methoxy-4-nitrophenyl]-2-methylropanoate

The title compound was prepared by substituting Example 249B for Example 223A in Example 223B. MS (DCI) m/e 311 (M+H)⁺; ¹H NMR (500 MHz, DMSO-d₆) δ 10.09 (s, 1H), 7.49 (s, 1H), 7.45(s, 1H), 3.80 (s, 3H), 3.55 (s, 3H), 2.03 (s, 3H), 1.43 (s, 6H).

### Example 249D

### methyl 2-(5-amino-2-methoxy-4-nitrophenyl)-2-methylpropanoate

The title compound was prepared by substituting Example 249C for Example 223B in Example 223C. MS (DCI) m/e 269 (M+H)⁺.

### Example 249E

### methyl 4-chloro-2-{[4-methoxy-5-(2-methoxy-1,1-dimethyl-2-oxoethyl)-2-nitrophenyl]amino}benzoate

The title compound was prepared by substituting Example 249D for methyl 3,4-diaminobenzoate in Example 1A. MS (DCI) m/e 437 (M+H)⁺; ¹H NMR (500 MHz, DMSO-d₆) δ 10.65 (s, 1H), 7.95 (d, J=8.82 Hz, 1H), 7.66 (s, 1H), 7.60 (s, 1H), 7.29 (d, J=2.03 Hz, 1H), 7.04 (dd, J=8.48, 2.03 Hz, 1H), 3.89 (s, 3H), 3.83 (s, 3H), 3.58 (s, 3H), 1.41 (s, 6H).

### Example 249F

### methyl 2-{[2-amino-4-methoxy-5-(2-methoxy-1,1-dimethyl-2-oxoethyl)phenyl]amino}-4-chlorobenzoate

The title compound was prepared by substituting Example 249E for Example 6B in Example 6C. MS (DCI) m/e 406 (M+H)⁺; ¹H NMR (500 MHz, DMSO-d₆) δ 8.76 (s, 1H), 7.84 (d, J=8.48 Hz, 1H), 6.84 (s, 1H), 6.67 (dd, J=8.65, 2.20 Hz, 1H), 6.44 (s, 1H), 6.31 (d, J=2.03 Hz, 1H), 4.93 (s, 2H), 3.85 (s, 3H), 3.66 (s, 3H), 3.53 (s, 3H), 1.34 (s, 6H).

### Example 249G

### methyl 2-(3-chloro-8-methoxy-11-oxo-10,11-dihydro-5H-dibenzo[b,e][1,4]diazepin-7-yl)-2-methylpropanoate

The title compound was prepared by substituting Example 249F for Example 6C in Example 6D. MS (DCI) m/e 375 (M+H)⁺; ¹H NMR (500 MHz, DMSO-d₆) δ 9.75 (s, 1H), 7.81 (s, 1H), 7.66 (d, J=8.42 Hz, 1H), 7.03 (d, J=2.18 Hz, 1H), 6.93 (s, 1H), 6.89 (dd, J=8.42, 2.18 Hz, 1H), 6.62 (s, 1H), 3.60 (s, 3H), 3.50 (s, 3H), 1.37 (s, 6H).

### Example 249H

### 3-chloro-7-(2-hydroxy-1,1-dimethylethyl)-8-methoxy-5,10-dihydro-11H-dibenzo[b,e][1,4]diazepin-11-one

The title compound was prepared by substituting Example 249G for Example 6D in Example 204A. MS (DCI) m/e 347 (M+H)⁺; ¹H NMR (500 MHz, DMSO-d₆) δ 9.72 (s, 1H), 7.80 (s, 1H), 7.66 (d, J=8.54 Hz, 1H), 7.04 (d, J=2.14 Hz, 1H), 6.87-6.89 (m, 2H), 6.61 (s, 1H), 4.43 (t, J=5.64 Hz, 1H), 3.68 (s, 3H), 3.54 (d, J=5.80 Hz, 1H), 1.21 (s, 6H).

### Example 249I

### 7-(2-hydroxy-1,1-dimethylethyl)-8-methoxy-3-(3-methoxy-4-nitrophenyl)-5,10-dihydro-11H-dibenzo[b,e][1,4]diazepin-11-one

The title compound was prepared by substituting Example 249H and Example 266G for Example 59B and Example 56A, respectively, in Example 59C. MS (DCI) m/e 464 (M+H)⁺; ¹H NMR (500 MHz, DMSO-d₆) δ 9.96 (s, 1H), 7.99 (d, J=8.59 Hz, 1H), 7.74-7.78 (m, 2H), 7.51 (s, 1H), 7.33-7.35 (m, 2H), 7.24 (d, J=7.98 Hz, 1H), 6.91 (s, 1H), 6.62 (s, 1H), 4.40 (t, J=5.01 Hz, 1H), 4.02 (s, 3H), 3.86 (s, 3H), 3.54 (d, J=4.90 Hz, 1H), 1.21 (s, 6H).

### Example 250

### 7-(3-hydroxypropyl)-3-(3-methoxy-4-nitrophenyl)-8-(trifluoromethoxy)-5,10-dihydro-11H-dibenzo[b,e][1,4]diazepin-11-one

### Example 250A

### N-[5-bromo-2-nitro-4-(trifluoromethoxy)phenyl]acetamide

The title compound was prepared by substituting N-(3-bromo-4-trifluoromethoxyphenyl)acetamide, prepared from acetylation of the corresponding aniline, for Example 223A in Example 223B. MS (DCI) m/e 344 (M+H)⁺; ¹H NMR (500 MHz, DMSO-d₆) δ 10.45 (s, 1H), 8.15 (s, 2H), 2.10 (s, 3H).

### Example 250B

### 5-bromo-2-nitro-4-(trifluoromethoxy)aniline

The title compound was prepared by substituting Example 250A for Example 223B in Example 223C. MS (DCI) m/e 302 (M+H)⁺; ¹H NMR (500 MHz, DMSO-d₆) δ 8.01 (s, 1H), 7.88 (s, 2H), 7.46 (s, 1H).

### Example 250C

### methyl 2-{[5-bromo-2-nitro-4-(trifluoromethoxy)phenyl]amino}-4-chlorobenzoate

The title compound was prepared by substituting Example 250B for methyl 3,4-diaminobenzoate in Example 1A. MS (DCI) m/e 470 (M+H)⁺; ¹H NMR (500 MHz, DMSO-d₆) δ 10.83 (s, 1H), 8.24 (s, 1H), 7.96-7.99 (m, 2H), 7.68 (d, J=2.03 Hz, 1H), 7.25 (dd, J=8.48, 2.03 Hz, 1H), 3.88 (s, 3H).

### Example 250D

### methyl 2-{[5-bromo-2-nitro-4-(trifluoromethoxy)phenyl]amino}-4-chlorobenzoate

The title compound was prepared by substituting Example 250C for Example 6B in Example 6C. MS (DCI) m/e 440 (M+H)⁺; ¹H NMR (500 MHz, DMSO-d₆) δ 8.84 (s, 1H), 7.87 (d, J=8.42 Hz, 1H), 7.41 (s, 1H), 6.95 (s, 1H), 6.78 (dd, J=8.48, 2.18 Hz, 1H), 6.39 (d, J=2.18 Hz, 1H), 3.86 (s, 3H).

### Example 250E

### 2-{[2-amino-5-bromo-4-(trifluoromethoxy)phenyl]amino}-4-chlorobenzoic acid

The title compound was prepared by substituting Example 250D for Example 12 in Example 13. MS (DCI) m/e 426 (M+H)⁺.

### Example 250F

### 7-bromo-3-chloro-8-(trifluoromethoxy)-5,10-dihydro-11H-dibenzo[b,e][1,4]diazepin-11-one

The title compound was prepared by substituting Example 250E for Example 243F in Example 243G. MS (DCI) m/e 426 (M+H)⁺; ¹H NMR (500 MHz, DMSO-d₆) δ 10.12 (s, 1H), 8.31 (s, 1H), 7.71 (d, J=8.48 Hz, 1H), 7.34 (s, 1H), 7.13 (s, 1H), 7.02 (dd, J=7.46, 2.03 Hz, 1H), 6.98 (d, J=2.37 Hz, 1H).

### Example 250G

### methyl 3-[3-chloro-11-oxo-8-(trifluoromethoxy)-10,11-dihydro-5H-dibenzo[b,e][1,4]diazepin-7-yl]acrylate

The title compound was prepared by substituting Example 250F for Example 223G in Example 223H. MS (DCI) m/e 413 (M+H)⁺; ¹H NMR (500 MHz, DMSO-d₆) δ 10.23 (s, 1H), 8.26 (s, 1H), 7.72 (d, J=8.48 Hz, 1H), 7.60 (d, J=15.60 Hz, 1H), 7.45 (s, 1H), 7.08 (s, 1H), 7.06 (d, J=2.18 Hz, 1H), 6.98 (dd, J=8.58, 2.03 Hz, 1H), 6.42 (d, J=16.22 Hz, 1H).

### Example 250H

### methyl 3-[3-chloro-11-oxo-8-(trifluoromethoxy)-10,11-dihydro-5H-dibenzo[b,e][1,4]diazepin-7-yl]propanoate

The title compound was prepared by substituting Example 250G for Example 6B in Example 6C. MS (DCI) m/e 415 (M+H)⁺; ¹H NMR (500 MHz, DMSO-d₆) δ 9.89 (s, 1H), 8.09 (s, 1H), 7.61 (d, J=8.48 Hz, 1H), 6.99 (d, J=1.87 Hz, 1H), 6.86-6.89 (m 3H), 3.52 (s, 3H), 2.70 (t, J=7.49 Hz, 2H), 2.49 (t, J=7.49 Hz, 2H).

### Example 250I

### 3-chloro-7-(3-hydroxypropyl)-8-(trifluoromethoxy)-5,10-dihydro-11H-dibenzo[b,e][1,4]diazepin-11-one

The title compound was prepared by substituting Example 250H for Example 6D in Example 204A. MS (DCI) m/e 387 (M+H)⁺; ¹H NMR (500 MHz, DMSO-d₆) δ 9.98 (s, 1H), 8.17 (s, 1H), 7.59 (d, J=8.64 Hz, 1H), 7.06 (d, J=2.14 Hz, 1H), 6.95-6.97 (m 3H), 4.53 (t, J=5.19 Hz, 1H), 3.40-3.44 (m, 2H), 2.50-2.54 (m, 2H), 1.63-1.66 (m, 2H).

### Example 250

### 7-(3-hydroxypropyl)-3-(3-methoxy-4-nitrophenyl)-8-(trifluoromethoxy)-5,10-dihydro-11H-dibenzo[b,e][1,4]diazepin-11-one

The title compound was prepared by substituting Example 250I and Example 266G for Example 59B and Example 56A, respectively, in Example 59C. MS (DCI) m/e 504 (M+H)⁺; ¹H NMR (500 MHz, DMSO-d₆) δ 9.96 (s, 1H), 8.14 (s, 1H), 8.01 (d, J=8.29 Hz, 1H), 7.82 (d, J=8.29 Hz, 1H), 7.53 (s, 1H), 7.32-7.36 (m, 3H), 7.01 (s, 1H), 6.98 (s, 1H), 4.50 (t, J=5.06 Hz, 1H), 4.04 (s, 3H), 3.41-3.45 (m, 2H), 2.52-2.56 (m, 2H), 1.64-1.68 (m, 2H).

### Example 251

### 7-(3-hydroxy-3-methylbutyl)-3-(3-methoxy-4-nitrophenyl)-8-(trifluoromethoxy)-5,10-dihydro-11H-dibenzo[b,e][1,4]diazepin-11-one

### Example 251A

### 3-chloro-7-(3-hydroxy-3-methylbutyl)-8-(trifluoromethoxy)-5,10-dihydro-11H-dibenzo[b,e][1,4]diazepin-11-one

The title compound was prepared by substituting Example 250H for Example 1B in Example 189A. MS (DCI) m/e 415 (M+H)⁺; ¹H NMR (500 MHz, DMSO-d₆) δ 9.95 (s, 1H), 8.13 (s, 1H), 7.69 (d, J=8.29 Hz, 1H), 7.06 (d, J=2.15 Hz, 1H), 6.94-6.97 (m 3H), 4.29 (s, 1H), 2.50-2.56 (m, 2H), 1.53-1.57 (m, 2H), 1.13 (m, 6H).

### Example 251B

### 7-(3-hydroxy-3-methylbutyl)-3-(3-methoxy-4-nitrophenyl)-8-(trifluoromethoxy)-5,10-dihydro-11H-dibenzo[b,e][1,4]diazepin-11-one

The title compound was prepared by substituting Example 251A and Example 266G for Example 59B and Example 56A, respectively, in Example 59C. MS (DCI) m/e 532 (M+H)⁺; ¹H NMR (500 MHz, DMSO-d₆) δ 9.96 (s, 1H), 8.12 (s, 1H), 8.01 (d, J=8.29 Hz, 1H), 7.81 (d, J=7.98 Hz, 1H), 7.54 (d, J=1.84 Hz, 1H), 7.32-7.37 (m, 3H), 7.01 (s, 1H), 6.97 (d, J=1.23 Hz, 1H), 4.30 (s, 1H), 4.04 (s, 3H), 2.49-2.56 (m, 2H), 1.54-1.58 (m, 2H), 1.14 (m, 6H).

### Example 252

### 7-(3-hydroxy-3-methylbutyl)-3-(3-methoxy-4-nitrophenyl)-8-methyl-5,10-dihydro-11H-dibenzo[b,e][1,4]diazepin-11-one

### Example 252A

### N-(5-bromo-4-methyl-2-nitrophenyl)acetamide

The title compound was prepared by substituting N-(3-bromo-4-methylphenyl)acetamide for Example 223A in Example 223B. MS (DCI) m/e 344 (M+H)⁺; ¹H NMR (500 MHz, DMSO-d₆) δ 10.22 (s, 1H), 7.98 (s, 1H), 7.94 (s, 1H), 2.39 (s, 3H), 2.07 (s, 3H).

### Example 252B

### 5-bromo-4-methyl-2-nitroaniline

The title compound was prepared by substituting Example 252A for Example 223B in Example 223C. MS (DCI) m/e 302 (M+H)⁺; ¹H NMR (500 MHz, DMSO-d₆) δ 7.92 (s, 1H), 7.33 (s, 3H), 2.44 (s, 3H).

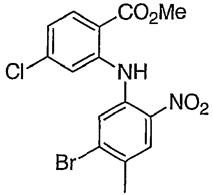

### Example 252C

### methyl 2-[(5-bromo-4-methyl-2-nitrophenyl)amino]-4-chlorobenzoate

The title compound was prepared by substituting Example 252B for methyl 3,4-diaminobenzoate in Example 1A. MS (DCI) m/e 400 (M+H)⁺; ¹H NMR (500 MHz, DMSO-d₆) δ 10.72 (s, 1H), 8.16 (s, 1H), 7.97 (d, J=8.48 Hz, 1H), 7.85 (s, 1H), 7.51 (d, J=2.03 Hz, 1H), 7.14 (dd. J=8.82, 2.03 Hz, 1H), 3.88 (s, 3H), 2.38 (s, 3H).

### Example 252D

### methyl 2-[(2-amino-5-bromo-4-methylphenyl)amino]-4-chlorobenzoate

The title compound was prepared by substituting Example 252C for Example 6B in Example 6C. MS (DCI) m/e 440 (M+H)⁺; ¹H NMR (500 MHz, DMSO-d₆) δ 10.72 (s, 1H), 8.16 (s, 1H), 7.96 (d, J=8.48 Hz, 1H), 7.85 (s, 1H), 7.51 (d, J=2.03 Hz, 1H), 7.14 (dd, J=8.82, 2.03 Hz, 1H), 3.88 (s, 3H), 2.38 (s, 3H).

### Example 252E

### 2-[(2-amino-5-bromo-4-methylphenyl)amino]-4-chlorobenzoic acid

The title compound was prepared by substituting Example 252D for Example 12 in Example 13. MS (DCI) m/e 356 (M+H)⁺.

### Example 252F

### 7-bromo-3-chloro-8-methyl-5,10-dihydro-11H-dibenzo[b,e][1,4]diazepin-11-one

The title compound was prepared by substituting Example 252E for Example 223F in Example 223G. MS (DCI) m/e 338 (M+H)⁺; ¹H NMR (500 MHz, DMSO-d₆) δ 9.94 (s, 1H), 8.06 (s, 1H), 7.69 (d, J=8.73 Hz, 1H), 7.20 (s, 1H), 7.02 (d, J=2.08 Hz, 1H), 6.95 (dd, J=8.42, 1.87 Hz, 1H), 6.91 (s, 1H), 2.20 (s, 3H).

### Example 252G

### methyl (2E)-3-(3-chloro-8-methyl-11-oxo-10,11-dihydro-5H-dibenzo[b,e][1,4]diazepin-7-yl)acrylate

The title compound was prepared by substituting Example 252F for Example 223G in Example 223H. MS (DCI) m/e 343 (M+H)⁺; ¹H NMR (500 MHz, DMSO-d₆) δ 10.02 (s, 1H), 8.00 (s, 1H), 7.73 (d, J=15.59 Hz, 1H), 7.68 (d, J=8.42 Hz, 1H), 7.28 (s, 1H), 7.03 (d, J=1.87 Hz, 1H), 6.92 (dd, J=8.42, 1.87 Hz, 1H), 6.83 (s, 1H), 6.25 (d, J=15.59 Hz, 1H), 3.72 (s, 3H), 2.25 (s, 3H).

### Example 252H

### methyl 3-(3-chloro-8-methyl-11-oxo-10,11-dihydro-5H-dibenzo[b,e][1,4]diazepin-7-yl)propanoate

The title compound was prepared by substituting Example 252G for Example 6B in Example 6C. MS (DCI) m/e 345 (M+H)⁺; ¹H NMR (500 MHz, DMSO-d₆) δ 9.76 (s, 1H), 7.89 (s, 1H), 7.66 (d, J=8.42 Hz, 1H), 7.05 (d, J=2.18 Hz, 1H), 6.89 (dd, J=8.42, 2.18 Hz, 1H), 6.74 (s, 1H), 6.73 (s, 1H), 3.59 (s, 3H), 2.72 (t, J=7.64 Hz, 2H), 2.53 (t, J=7.64 Hz, 2H), 2.13 (s, 3H).

### Example 252I

### 3-chloro-7-(3-hydroxy-3-methylbutyl)-8-methyl-5,10-dihydro-11H-dibenzo[b,e][1,4]diazepin-11-one

The title compound was prepared by substituting Example 252H for Example 1B in Example 189A. MS (DCI) m/e 345 (M+H)⁺; ¹H NMR (500 MHz, DMSO-d₆) δ 9.7 (s, 1H), 7.85 (s, 1H), 7.66 (d, J=8.42 Hz, 1H), 7.04 (d, J=1.87 Hz, 1H), 6.75 (s, 1H), 6.72 (s, 1H), 4.22 (s, 1H), 2.45-2.50 (m, 2H), 2.12 (s, 2H), 1.49-1.53 (m, 2H), 1.15 (s, 6H).

### Example 252J

### 7-(3-hydroxy-3-methylbutyl)-3-(3-methoxy-4-nitrophenyl)-8-methyl-5,10-dihydro-11H-dibenzo[b,e][1,4]diazepin-11-one

The title compound was prepared by substituting Example 252I and Example 266G for Example 59B and Example 56A, respectively, in Example 59C. MS (DCI) m/e 462 (M+H)⁺; ¹H NMR (500 MHz, DMSO-d₆) δ 9.74 (s, 1H), 8.01 (d, J=8.42 Hz, 1H), 7.79-7.82 (m, 2H), 7.53 (d, J=1.84 Hz, 1H), 7.34-7.35 (m, 2H), 7.28 (d, J=7.80 Hz, 1H), 6.81 (s, 1H), 6.84 (s, 1H), 4.24 (s, 1H), 4.04 (s, 3H), 2.47-2.50 (m, 2H), 2.14 (d, 3H), 1.51-1.54 (m, 2H), 1.16 (m, 6H).

### Example 253

### 3-(3-methoxy-4-nitrophenyl)-8-(2-pyridin-4-ylethyl)-5,10-dihydro-11H-dibenzo[b,e][1,4]diazepin-11-one

### Example 253A

### 3-chloro-8-[(E)-2-pyridin-4-ylvinyl]-5,10-dihydro-11H-dibenzo[b,e][1,4]diazepin-11-one

The title compound was prepared by substituting Example 2B and 4-vinylpyridine for Example 223G and methyl acrylate, respectively, in Example 223H. MS (DCI) m/e 348 (M+H)⁺; ¹H NMR (500 MHz, DMSO-d₆) δ 9.94 (s, 1H), 8.52 (d, J=5.52 Hz, 2H), 8.26 (s 1H), 7.72 (d, J=8.59 Hz, 1H), 7.52 (d, J=6.14 Hz, 2H), 7.40 (d, J=16.57 Hz, 1H), 7.31 (dd, J=8.29, 2.15 Hz, 1H), 7.22 (d, J=1.84 Hz, 1H), 7.08 (d, J=2.15 Hz, 1H), 6.99-7.04 (m, 2H), 6.94 (dd, J=8.44, 199, Hz, 1H).

### Example 253B

### 3-chloro-8-(2-pyridin-4-ylethyl)-5,10-dihydro-11H-dibenzo[b,e][1,4]diazepin-11-one

The title compound was prepared by substituting Example 253A for Example 6B in Example 6C. MS (DCI) m/e 351 (M+H)⁺; ¹H NMR (500 MHz, DMSO-d₆) δ 9.61 (s, 1H), 8.67 (d, J=7.93 Hz, 2H), 7.97 (s, 1H), 7.66-7.67 (m, 2H), 7.05 (d, J=2.18 Hz, 1H), 6.91 (dd, J=8.42, 2.18 Hz, 1H), 6.87-6.88 (m, 1H), 6.82-6.84 (m, 1H), 6.80 (d, J=1.87 Hz, 1H), 3.04 (t, J=7.64 Hz, 2H), 2.87 (t, J=7.80 Hz, 2H).

### Example 253C

### 3-(3-methoxy-4-nitrophenyl)-8-(2-pyridin-4-ylethyl)-5,10-dihydro-11H-dibenzo[b,e][1,4]diazepin-11-one

The title compound was prepared by substituting Example 253B and Example 266G for Example 59B and Example 56A, respectively, in Example 59C. MS (DCI) m/e 467 (M+H)⁺; ¹H NMR (500 MHz, DMSO-d₆) δ 9.81 (s, 1H), 8.65 (d, J=5.93 Hz, 2H), 8.01 (d, J=8.42 Hz, 1H), 7.94 (s, 1H), 7.65 (d, J=5.61 Hz, 2H), 7.51 (d, J=1.56 Hz, 1H), 7.32-7.35 (m, 2H), 7.29 (dd, J=8.11, 1.56 Hz, 1H), 6.82 (d, J=7.80 Hz, 1H), 6.82-6.83 (m, 2H), 4.24 (s, 1H), 4.03 (s, 3H), 3.04 (t, J=7.64 Hz, 2H), 2.86 (t, J=7.64 Hz, 2H).

### Example 254

### 3-(3-methoxy-4-nitrophenyl)-8-(2-pyridin-2-ylethyl)-5,10-dihydro-11H-dibenzo[b,e][1,4]diazepin-11-one

### Example 254A

### 3-chloro-8-[(E)-2-pyridin-2-ylvinyl]-5,10-dihydro-11H-dibenzo[b,e][1,41diazepin-11-one

The title compound was prepared by substituting Example 2B and 2-vinylpyridine for Example 223G and methyl acrylate, respectively, in Example 223H. MS (DCI) m/e 348 (M+H)⁺; ¹H NMR (500 MHz, DMSO-d₆) δ 9.91 (s, 1H), 8.55 (d, J=4.06 Hz, 2H), 8.23 (s 1H), 7.72-7.78 (m, 2H), 7.53 (d, J=8.36 Hz, 1H), 7.51 (s, 1H), 7.30 (dd, J=8.27, 1.72 Hz, 1H), 7.22-7.24 (m, 2H), 7.07-7.10 (m, 2H), 6.99 (d, J=8.11 Hz, 1H), 6.94 (dd, J=8.42, 2.18,Hz, 1H).

### Example 254B

### 3-chloro-8-(2-pyridin-2-ylethyl)-5,10-dihydro-11H-dibenzo[b,e][1,4]diazepin-11-one

The title compound was prepared by substituting Example 254A for Example 6B in Example 6C. MS (DCI) m/e 351 (M+H)⁺; ¹H NMR (500 MHz, DMSO-d₆) δ 9.82 (s, 1H), 8.48 (d, J=3099 Hz, 1H), 7.95 (s, 1H), 7.64-7.68 (m, 2H), 7.22 (d, J=7.98 Hz, 1H), 7.17-7.19 (m, 1H), 7.05 (d, J=2.15 Hz, 1H), 6.91 (dd, J=8.59, 2.15 Hz, 1H), 6.80-6.87 (m, 3H), 2.92-2.98 (m, 2H), 2.84-2.89 (m, 2H).

### Example 254C

### 3-(3-methoxy-4-nitrophenyl)-8-(2-pyridin-2-ylethyl)-5,10-dihydro-11H-dibenzo[b,e][1,4]diazepin-11-one

The title compound was prepared by substituting Example 254B and Example 266G for Example 59B and Example 56A, respectively, in Example 59C. MS (DCI) m/e 467 (M+H)⁺; ¹H NMR (500 MHz, DMSO-d₆) δ 9.81 (s, 1H), 8.49 (d, J=4.06 Hz, 1H), 8.01 (d, J=8.42 Hz, 1H), 7.91 (s, 1H), 7.80 (d, J=8.11 Hz, 1H), 7.64-7.68 (m, 1H), 7.52 (d, J=1.56 Hz, 1H), 7.33-7.35 (m, 2H), 7.29 (dd, J=8.11, 1.87 Hz, 1H), 7.22 (d, J=7.80 Hz, 1H), 7.17-7.20 (m, 1H), 6.90 (d, J=8.11 Hz, 1H), 6.86 (d, J=1.86 Hz, 1H), 6.81 (dd, J=7.96, 1.72 Hz, 1H), 4.03 (s, 3H), 2.95-2.98 (m, 2H), 2.86-2.88 (m, 2H).

### Example 255

### 3- 3-methoxy-4-nitrophenyl)-8-[2-(2-oxopyridin-1(2H)-yl)ethyl]-5,10-dihydro-11H-dibenzo[b,e][1,4]diazepin-11-one

The title compound was prepared by substituting Example 239 for Example 204A in Example 221A. MS (DCI) m/e 483 (M+H)⁺; ¹H NMR (500 MHz, DMSO-d₆) δ 9.83 (s, 1H), 8.01 (d, J=8.42 Hz, 1H), 7.96 (s, 1H), 7.80 (d, J=8.42 Hz, 1H), 7.50-7.53 (m, 2H), 7.34-7.39 (m, 3H), 7.29 (d, J=8.11 Hz, 1H), 6.93 (d, J=7.80 Hz, 1H), 6.84 (s, 1H), 6.80 (d, J=8.11 Hz, 1H), 6.37 (d, J=9.04 Hz, 1H), 6.11-6.13 (m, 1H), 4.01-4.03 (m, 5H), 2.81 (t, J=7.49 Hz, 2H).

### Example 256

### 8-[2-(5-fluoro-2-oxopyridin-1(2H)-yl)ethyl]-3-(3-methoxy-4-nitrophenyl)-5,10-dihydro-11H-dibenzo[b,e][1,4]diazepin-11-one

The title compound was prepared by substituting Example 239 and 5-fluoro-pyridin-2-ol for Example 204A and pyridin-2(1H)-one, respectively, in Example 221A. MS (DCI) m/e 501 (M+H)⁺; ¹H NMR (500 MHz, DMSO-d₆) δ 9.84 (s, 1H), 8.01 (d, J=8.42 Hz, 1H), 7.96 (s, 1H), 7.80 (d, J=8.42 Hz, 1H), 7.78-7.81 (m, 2H), 7.50-7.54 (m, 2H), 7.33-7.35 (m, 2H), 7.29 (dd, J=8.11, 1.87 Hz, 1H), 6.94 (d, J=8.11 Hz, 1H), 6.81-6.84 (m, 2H), 6.40 (dd, J=10.14, 5.46 Hz, 1H), 3.97-4.03 (m, 5H), 2.80-2.83 (m, 2H).

### Example 257

### 3-(3-methoxy-4-nitrophenyl)-8-[2-(6-oxopyridazin-1(6H)-yl)ethyl]-5,10-dihydro-11H-dibenzo[b,e][1,4]diazepin-11-one

The title compound was prepared by substituting Example 239 and pyridazin-3-ol for Example 204A and pyridin-2(1H)-one, respectively, in Example 221A. MS (DCI) m/e 484 (M+H)⁺; ¹H NMR (500 MHz, DMSO-d₆) δ 9.83 (s, 1H), 8.01 (d, J=8.42 Hz, 1H), 7.95 (s, 1H), 7.88 (dd, J=3.90, 1.72 Hz, 1H), 7.80 (d, J=8.42 Hz, 1H), 7.52 (d, J=1.56 Hz, 1H), 7.38 (dd, J=9.36, 3.74 Hz, 2H), 7.33-7.35 (m, 2H), 6.92 (m, 1H), 6.82 (s, 1H), 6.80 (dd, J=7.95, 1.72 Hz, 1H), 4.20 (t, J=7.49 Hz, 2H), 4.03 (s, 3H), 2.88-2.83 (t, J=7.49 Hz, 2H).

### Example 258

### 3-(3-methoxy-4-nitrophenyl)-8-[2-(pyridin-2-yloxy)ethyl]-5,10-dihydro-11H-dibenzo[b,e][1,4] diazepin-11-one

The title compound was prepared by substituting Example 239 for Example 204A in Example 221A. MS (DCI) m/e 483 (M+H)⁺; ¹H NMR (500 MHz, DMSO-d₆) δ 9.85 (s, 1H), 8.15 (dd, J=5.06, 1.99 Hz, 1H), 8.01 (d, J=8.29 Hz, 1H), 7.95 (s, 1H), 7.80 (d, J=8.29 Hz, 1H), 7.66-7.70 (m, 1H), 7.52 (d, J=1.53 Hz, 1H), 7.33-7.35 (m, 2H), 7.29 (dd, J=7.98, 1.84 Hz, 1H), 6.89-6.97 (m, 4H), 6.78 (d, J=8.59 Hz, 1H), 4.40 (t, J=6.75 Hz, 2H), 4.03 (s, 3H), 2.90 (t, J=6.75 Hz, 2H).

### Example 259

### 8-{2-[(5-chloropyridin-3-yl)oxy]ethyl}-3-(3-methoxy-4-nitrophenyl)-5,10-dihydro-11H-dibenzo[b,e][1,4]diazepin-11-one

The title compound was prepared by substituting Example 239 and 3-chloro-5-hydroxypyridine for Example 204A and pyridin-2(1H)-one, respectively, in Example 221A. MS (DCI) m/e 517 (M+H)⁺; ¹H NMR (500 MHz, DMSO-d₆) δ 9.89 (s, 1H), 8.26 (d, J=8.44 Hz, 1H), 8.19 (d J=1.82 Hz, 1H), 8.01 (d, J=8.24 Hz, 1H), 7.98 (s, 1H), 7.80 (d, J=8.24 Hz, 1H), 7.59-7.60

(m, 1H), 7.52 (d, J=1.53 Hz, 1H), 7.33-7.35 (m, 2H), 7.30 (dd, J=8.24, 1.53 Hz, 1H), 6.92-6.97 (m, 3H), 4.24 (t, J=6.56 Hz, 2H), 4.03 (s, 3H), 2.92 (t, J=6.56 Hz, 2H).

### Example 260

### 8-methoxy-3-(3-methoxy-4-nitrophenyl)-7-[2-(pyridin-3-yloxy)ethyl]-5,10-dihydro-11H-dibenzo [b,e][1,4]diazepin-11-one

The title compound was prepared by substituting Example 247 and 3-hydroxypyridine for Example 204A and pyridin-2(1H)-one, respectively, in Example 221A. MS (DCI) m/e 513 (M+H)⁺; ¹H NMR (500 MHz, DMSO-d₆) δ 9.72 (s, 1H), 8.33 (d, J=2.76 Hz, 1H), 8.20 (d J=4.91 Hz, 1H), 7.94 (d, J=8.29 Hz, 1H), 7.71-7.73 (m, 2H), 7.57 (m, 1H), 7.43-7.47 (m, 2H), 7.26-7.28 (m, 2H), 7.21 (dd, J=8.29, 1.84 Hz, 1H), 6.85 (s, 1H), 6.61 (s, 1H), 4.15 (t, J=6.90 Hz, 2H), 4.03 (s, 3H), 2.86 (t, J=7.06 Hz, 2H).

### Example 261

### methyl (3-isoquinolin-5-yl-11-oxo-10,11-dihydro-5H-dibenzo[b,e][1,4]diazepin-8-yl)acetate

The title compound was prepared by substituting 5-bromo-isoquinoline and Example 54A for Example 9 and 2-methoxy-4-(4,4,5,5-tetramethyl-1,3,2-dioxaborolan-2-yl)phenol, respectively, in Example 10. MS (DCI) m/e 410 (M+H)⁺; ¹H NMR (500 MHz, DMSO-d₆) δ 9.87 (s, 1H), 9.39 (s, 1H), 8.50 (d, J=6.24 Hz, 1H), 8.19 (d J=7.80 Hz, 1H), 7.97 (s, 1H), 7.84 (d, J=7.80 Hz, 1H), 7.70-7.78 (m, 3H), 7.14 (d, J=1.56 Hz, 1H), 7.02 (dd, J=7.95, 1.72 Hz, 1H), 6.93 (d, J=7.80 Hz, 1H), 6.89 (d, J=1.56 Hz, 1H), 6.86 (dd, J=7.80, 1.87 Hz, 1H), 3.59 (s, 3H), 3.54 (s, 2H).

### Example 262

### methyl [3-(8-nitroisoquinolin-5-yl)-11-oxo-10,11-dihydro-5H-dibenzo[b,e][1,4]diazepin-8-yl]acetate

### Example 262A

### 5-bromo-8-nitroisoquinoline

5-Bromo-isoquinoline (100 mg, 0.48 mmol) was suspended in 0.58 mL of concentrated H₂SO₄. To this solution was added KNO₃ (68 mg, 0.58 mmol) in 0.48 mL of concentrated H₂SO₄. The reaction mixture was stirred at room temperature for 1.5 hours, poured into water/ice, neutralized with 2.0 M Na₂CO₃, and extracted with EtOAc three times The combined organic layers were washed brine, dried (MgSO₄), filtered and concentrated under vacuum to give 121 mg (995) of the title product.. MS (DCI) m/e 255 (M+H)⁺; ¹H NMR (500 MHz, CDCl₃) δ 10.0 (s, 1H), 9.39 (s, 1H), 8.85 (d, J=5.76 Hz, 1H), 8.17-8.22 (m, 2H), 8.12 (d, J=8.14 Hz, 1H).

### Example 262B

### methyl [3-(8-nitroisoquinolin-5-yl)-11-oxo-10,11-dihydro-5H-dibenzo[b,e][1,4]diazepin-8-yl]acetate

The title compound was prepared by substituting Example 262A and Example 54A for Example 9 and 2-methoxy-4-(4,4,5,5-tetramethyl-1,3,2-dioxaborolan-2-yl)phenol, respectively, in Example 10. MS (DCI) m/e 455 (M+H)⁺; ¹H NMR (500 MHz, DMSO-d₆) δ 9.93 (s, 1H), 9.84 (s, 1H), 8.70 (d, J=5.83 Hz, 1H), 8.50 (d J=7.98 Hz, 1H), 8.05 (s, 1H), 7.91 (d, J=7.98 Hz, 1H), 7.87 (d, J=7.98 Hz, 1H), 7.83 (d, J=5.83 Hz, 1H), 7.14 (s, 1H), 7.05 (dd, J=7.98 Hz, 1H), 6.85-6.94 (m, 3H), 3.60 (s, 3H), 3.54 (s, 2H).

### Example 263

### methyl 3-[3-(4-formyl-3-methoxyphenyl)-11-oxo-10,11-dihydro-5H-dibenzo[b,e][1,4]diazepin-8-yl]propanoate

The title compound was prepared by substituting 4-chloro-2-methoxy-benzaldehyde and Example 241A for Example 59B and Example 56A, respectively, in Example 59C. MS (DCI) m/e 431 (M+H)⁺; ¹H NMR (500 MHz, DMSO-d₆) δ 10.38 (s, 1H), 9.81 (s, 1H), 7.92 (s, 1H), 7.78-7.81 (m, 2H), 7.42 (s, 1H), 7.34 (d, J=1.87 Hz, 1H), 7.33 (d, J=8.11 Hz, 1H), 7.29 (dd, J=8.11, 1.87 Hz, 1H), 6.92-6.93 (m, 1H), 6.81-6.82 (m, 2H), 4.03 (s, 3H), 3.58 (s, 3H), 2.75 (t, J=7.49 Hz, 2H), 2.55 (t, J=7.49 Hz, 2H).

### Example 264

### methyl 3-{3-[4-(hydroxymethyl)-3-methoxyphenyl]-11-oxo-10,11-dihydro-5H-dibenzo[b,e][1,4]diazepin-8-yl}propanoate

The title compound was prepared by substituting (4-chloro-2-methoxy-phenyl)-methanol and Example 241A for Example 59B and Example 56A, respectively, in Example 59C. MS (DCI) m/e 433 (M+H)⁺; ¹H NMR (500 MHz, DMSO-d₆) δ 9.74 (s, 1H), 7.85 (s, 1H), 7.75 (d, J=8.11 Hz, 1H), 7.47 (d, J=7.80 Hz, 1H), 7.29 (d, J=1.26 Hz, 1H), 7.20-7.22 (m, 2H), 7.16 (s, 1H), 6.92 (d, J=8.74 Hz, 1H), 6.80-6.82 (m, 2H), 5.03 (t, J=5.03 Hz, 1H), 3.87 (s, 3H), 3.58 (s, 3H), 2.72 (t, J=7.49 Hz, 2H), 2.55 (t, J=7.49 Hz, 2H).

### Example 265

### methyl 3-[3-(3-methoxy-4-propionylphenyl)-11-oxo-10,11-dihydro-5H-dibenzo[b,e][1,4]diazepin-8-yl]propanoate

### Example 265A

### 1-(4-chloro-2-methoxyphenyl)propan-1-one

4-Chloro-2-methoxy-benzaldehyde (0.54 g, 3.2 mmol) in 10 mL of THF was treated with 3.0 M EtMgBr (2 mL, 6.0 mmol) at room temperature. The solution was stirred for 1 hour and quenched with MeOH. The solution was poured into water, treated 10 mL of 10% HCl, extracted with EtOAc several times. The combined organic layers were washed brine, dried (MgSO₄), filtered, and concentrated. The residue was diluted with CH₂Cl₂, treated with PCC (1.33 g, 6.4 mmol) for 2 hours. The reaction mixture was filtered through a pack of silica gel to give 0.42 g of the title compound. MS (DCI) m/e 199 (M+H)⁺.

### Example 265B

### methyl 3-[3-(3-methoxy-4-propionylphenyl)-11-oxo-10,11-dihydro-5H-dibenzo[b,e][1,4]diazepin-8-yl]propanoate

The title compound was prepared by substituting Example 265A and Example 241A for Example 59B and Example 56A, respectively, in Example 59C. MS (DCI) m/e 459 (M+H)⁺; ¹H NMR (500 MHz, DMSO-d₆) δ 9.79 (s, 1H), 7.90 (s, 1H), 7.77 (d, J=8.11 Hz, 1H), 7.66 (d, J=8.11 Hz, 1H), 7.35 (d, J=1.25 Hz, 1H), 7.33 (d, J=1.87 Hz, 1H), 7.25-7.28 (m, 2H), 6.92 (d, J=8.73 Hz, 1H), 6.81-6.82 (m, 2H), 3.98 (s, 3H), 3.58 (s, 3H), 2.96 (q, J=7.18 Hz, 1H), 2.72 (t, J=7.49 Hz, 2H), 2.55 (t, J=7.49 Hz, 2H), 1.07 (t, J=7.18 Hz, 3H).

### Example 266

### 2-[3-(3-methoxy-4-nitrophenyl)-11-oxo-10,11-dihydro-5H-dibenzo[b,e][1,4]diazepin-8-yl]-2-methylN(4-morpholin-4-ylphenyl)propanamide

### Example 266A

### methyl 2-methyl-2-(4-nitrophenyl)propanoate

A solution of 4-nitrophenylacetic acid (60.0g, 0.33mol), iodomethane (130.0 mL, 296.4g, 2.10 mol), and 18-crown-6 (15g, 0.057 mol) in DMF (1.0 L) was cooled to 0° C, then 95 % NaH (30.0g, 1.19 mol) was added in portions, keeping Tᵣₓₙ < 25° C. The reaction was allowed to warm to room temperature overnight, then partitioned between
water (2 L) and Et₂O (400 mL). The aqueous layer was extracted with Et₂O (2 x 300 mL), then the combined organic layers were washed with brine and dried over Na₂SO₄. After filtration and concentration, recovered 74g (99%) product as an orange oil. MS (DCI) m/e 241 (M+NH₄)⁺; ¹H NMR (300 MHz, CDCl₃) δ 8.19 (m, 2H), 7.50 (m, 2H), 3.67 (s, 3H), 1.62 (s, 6H).

### Example 266B

### methyl 2-(4-aminophenyl)-2-methylpropanoate

Dissolved the compound described in Example 266A (59.4g, 0.27 mol) in MeOH (600 mL), then added SnCl₂-2H₂O (120.0 g, 0.53 mol) and concentrated HCl (300 mL). The reaction was allowed to stir at room temperature overnight, then more SnCl₂·2H₂O (49.0 g, 0.22 mol) and concentrated HCl (100 mL) were added, and the reaction heated to 50° C for 4 hours. After cooling the reaction was partitioned between pH =14 water and EtOAc. The aqueous layer was again extracted with EtOAc, then the combined organic layers were dried (Na₂SO₄). The residue was purified by column chromatography using 4/1, then 1/1 hexanes/EtOAc to give 38.0g (74%) product. MS (DCI) m/e 194 (M+H)⁺, 211 (M+NH₄)⁺; ¹H NMR (300 MHz, CDCl₃) δ 7.13 (m, 2H), 6.64 (m, 2H), 3.62 (s, 3H), 1.55 (s, 6H).

### Example 266C

### methyl 2-(4-amino-3-nitrophenyl)-2-methylpropanoate

Example 266B (38.0g, 0.20 mol) was treated with acetic anhydride to give the acetamide, then nitrated, hydrolyzed, and subjected to a Fischer esterification by the method described in Example 6A to give the title compound (43.2g, 0.18 mol, 90 % overall). MS (DCI) m/e 239 (M+H)⁺, 256 (M+NH₄)⁺; ¹H NMR (300 MHz, CDCl₃) δ 8.12 (d, J=2.4 Hz, 1H), 7.37 (dd, J=8.8, 2.4 Hz, 1H), 6.78 (d, J=8.8 Hz, 1H), 6.05 (br s, 2H), 3.66 (s, 3H), 1.57 (s, 6H).

### Example 266D

### methyl 4-chloro-2-{[4-(2-methoxy-1,1-dimethyl-2-oxoethyl)-2-nitrophenyl]amino}benzoate

The title compound was prepared by substituting Example 266C for 4-bromo-2-nitroaniline in Example 2A. MS (DCI) m/e 407 and 409 (M+H)⁺, 424 and 426 (M+NH₄)⁺; ¹H NMR (300 MHz, DMSO-d₆) δ 10.84 (s, 1H), 8.03 (d, J=2.0 Hz, 1H), 7.97 (d, J=8.5 Hz, 1H), 7.66 (m, 2H), 7.50 (d, J=2.0 Hz, 1H), 7.14 (dd, J=8.5, 2.0 Hz, 1H), 3.89 (s, 3H), 3.63 (s, 3H), 1.55 (s, 6H).

### Example 266E

### methyl 2-{[2-amino-4-(2-methoxy-1,1-dimethyl-2-oxoethyl)phenyl]amino}-4-chlorobenzoate

The title compound was prepared by substituting Example 266D for Example 6B in Example 6C. MS (DCI) m/e 377 and 379 (M+H)⁺; ¹H NMR (300 MHz, DMSO-d₆) δ 8.82 (s, 1H), 7.85 (d, J=8.5 Hz, 1H), 6.98 (d, J=8.1 Hz, 1H), 6.78 (d, J=2.0 Hz, 1H), 6.70 (dd, J=8.5, 2.0 Hz, 1H), 6.55 (dd, J=8.1, 2.0 Hz, 1H), 6.42 (d, J=2.0 Hz, 1H),5.01 (br s, 2H), 3.85 (s, 3H), 3.61 (s, 3H), 1.48 (s, 6H).

### Example 266F

### methyl 2-(3-chloro-11-oxo-10,11-dihydro-5H-dibenzo[b,e][1,4]diazepin-8-y)-2-methylpropanoate

The title compound was prepared by substituting Example 266E for Example 5B in Example 5C. MS (DCI) m/e 345 and 347 (M+H)⁺; ¹H NMR (300 MHz, DMSO-d₆) δ 9.84 (s, 1H), 8.06 (s, 1H), 7.68 (d, J=8.5 Hz, 1H), 7.06 (d, J=2.0 Hz, 1H), 6.93 (m, 4H), 3.58 (s, 3H), 1.43 (s, 6H).

### Example 266G

### 2-(3-methoxy-4-nitrophenyl)-4,4,5,5-tetramethyl-1,3,2-dioxaborolane

The title compound was prepared by substituting 4-chloro-2-methoxy-1-nitro-benzene for Example 9 in Example 56A. MS (DCI) m/e 297 (M+NH₄)⁺.

### Example 266H

### methyl 2-[3-(3-methoxy-4-nitrophenyl)-11-oxo-10,11-dihydro-5H-dibenzo[b,e][1,4]diazepin-8-yl]-2-methylpropanoate

Example 266F (7.0g, 20.3 mmol) was slurried in a mixture of DME (130 mL) and MeOH (65 mL). That mixture was subjected to several cycles of vacuum-N₂ refill, then the following compounds were added: Example 266G (8.3g, 29.7 mmol), 2-dicyclohexylphosphino-2'-(N,N-dimethylamino)biphenyl (CyMAP ligand; 0.66g, 1.7 mmol), palladium(II)acetate (0.24g, 1.1 mmol), and cesium fluoride (9.2g, 60.5 mmol). After a few more cycles of vacuum-N₂ refill, the reaction was heated at 70 °C under N₂ overnight. The reaction was then cooled, filtered through Celite^{®}, and the filtrate was slowly diluted with water (1.2 L). The resultant solids were filtered off and dried, giving the crude material (11.6g) as reddish-brown solids. Those solids were slurried in Et₂O (100 mL) overnight, giving 8.4g solids after filtration and drying. Another Et₂O (40 mL) slurry for 2 hours, then filtration and drying gave the product (8.1 g, 86%) as dark red solids. MS (DCI) m/e 462 (M+H)⁺; ¹H NMR (300 MHz, DMSO-d₆) δ 9.84 (s, 1H), 8.03 (s, 1H), 8.01 (d, J=8.5 Hz, 1H), 7.80 (d, J=8.1 Hz, 1H), 7.52 (d, J=1.7Hz, 1H), 7.30-, 7.35 (m, 3H), 6.90-6.98 (m, 3H), 4.03 (s, 3H), 3.58 (s, 3H), 1.44 (s, 6H).

### Example 2661

### 2-[3-(3-methoxy-4-nitrophenyl)-11-oxo-11,11-dihydro-5H-dibenzo[b,e][1,4]diazepin-8-yl]-2-methylpropanoic acid

The title compound was prepared by substituting Example 266H for Example 12 in Example 13. MS (DCI) m/e 448 (M+H)⁺, 465 (M+NH₄)⁺; ¹H NMR (300 MHz, DMSO-d₆) δ 12.25 (v br s, 1H), 9.85 (s, 1H), 8.02 (d, J=8.5 Hz, 1H), 8.01 (s, 1H), 7.80 (d, J=8.1 Hz, 1H), 7.52 (d, J=1.7Hz, 1H), 7.30-7.35 (m, 3H), 6.96-7.02 (m, 3H), 4.03 (s, 3H), 1.41 (s, 6H).

### Example 266J

### 2-[3-(3-methoxy-4-nitrophenyl)-11-oxo-10,11-dihydro-5H-dibenzo[b,e][1,4]diazepin-8-yl]-2-methylN(4-morpholin-4-ylphenyl)propanamide

Example 266I (5.3g, 11.8 mmol), 4-morpholinoaniline (2.7g, 15.3 mmol), triethylamine (2.1 mL, 1.5g, 15.2 mmol), and O-(7-azabenzotriazol-1-yl)-N,N,N',N'-tetramethyluronium hexafluorophosphate (HATU; 5.8g, 15.3 mmol) were dissolved in DMF (70 mL) and stirred at room temperature overnight. The reaction was slowly diluted with water (1 L), and after filtration and drying recovered 7.4g crude material. Those solids were slurried in absolute EtOH (70 mL) for 30 minutes, then that slurry was slowly diluted with water (420 mL), giving 6.5g solids after filtration and drying. Those solids were slurried in CH₃CN/MeOH 1/1 (300 mL), added conc. HCl (1.0 mL), everything dissolved, then slowly added water (1.5 L). After filtration and drying, recovered the product (5.2g, 73%) as brown solids. MS (DCI) m/e 608 (M+H)⁺; ¹H NMR (300 MHz, DMSO-d₆) δ 9.87 (s, 1H), 8.79 (s, 1H), 8.01 (m, 2H), 7.80 (d, J=8.1 Hz, 1H), 7.52 (s, 1H), 7.42 (d, J=8.7 Hz, 2H), 7.30-7.35 (m, 3H), 7.06 (s, 1H), 7.00 (m, 1H), 6.95 (m, 1H), 6.84 (d, J=8.7 Hz, 2H), 4.03 (s, 3H), 3.71 (m, 4H), 3.00 (m, 4H), 1.49 (s, 6H).

### Example 267

### 2-[3-(4-cyano-3-methoxyphenyl)-11-oxo-10,11-dihydro-5H-dibenzo[b,e][1,4]diazepin-8-yl]-2-methylN(4-morpholin-4-ylphenyl)propanamide

### Example 267A

### 4-amino-2-methoxybenzonitrile

The title compound was prepared by substituting 4-nitro-2-methoxy-benzonitrile for Example 6B in Example 6C. MS (DCI) m/e 149 (M+H)⁺, 166 (M+NH₄)⁺; ¹H NMR (300 MHz, CDCl₃) δ 7.29 (d, J=8.1 Hz, 1H), 6.22 (dd, J=8.1, 2.0 Hz, 1H), 6.16 (d, J=2.0 Hz, 1H), 4.16 (br s, 2H), 3.85 (s, 3H).

### Example 267B

### 4-iodo-2-methoxybenzonitrile

The title compound was prepared by substituting Example 267A for Example 57A in Example 57B. MS (DCI) m/e 277 (M+NH₄)⁺; ¹H NMR (300 MHz, CDCl₃) δ 7.40 (m, 1H), 7.33 (m, 1H), 7.25 (d, J=8.1 Hz, 1H), 3.93 (s, 3H).

### Example 267C

### 2-methoxy-4-(4,4,5,5-tetramethyl-1,3,2-dioxaborolan-2-yl)benzonitrile

Example 267B (2.0g, 7.7 mmol), bis(pinacolato)-diboron, (2.2g, 8.7 mmol), KOAc (2.2g, 22.4 mmol), and PdCl₂(dppf)·CH₂Cl₂ (315 mg, 0.39 mmol) in DMSO (40 mL) was heated at 80° C for 2 hours. The reaction was then cooled, filtered through Celite^{®}, then partitioned between toluene and water. The organic layer was washed with brine, dried over Na₂SO₄, filtered and concentrated to give 2.3g crude material. That crude was stirred in 50° C hexane (35 mL) for 1 hour, filtered, and the filtrate was allowed to cool to room temperature, then placed in the refigerator overnight. Recovered 1.3g (65%) grayish-brown crystals. MS (DCI) m/e 277 (M+NH₄)⁺; ¹H NMR (300 MHz, DMSO-d₆) δ 7.73 (d, J=7.8 Hz, 1H), 7.35 (m, 2H), 3.93 (s, 3H), 1.32 (s, 12H).

### Example 267D

### 2-[3-(4-cyano-3-methoxyphenyl)-11-oxo-10,11-dihydro-5H-dibenzo[b,e][1,4]diazepin-8-yl]-2-methylN(4-morpholin-4-ylphenyl)propanamide

The title compound was prepared by substituting Example 267C and Example 266F for Example 56A and Example 59B, respectively, in Example 59C. MS (DCI) m/e 442 (M+H)⁺; ¹H NMR (300 MHz, DMSO-d₆) δ 9.84 (s, 1H), 8.01 (s, 1H), 7.84 (d, J=8.1 Hz, 1H), 7.79 (d, J=8.1 Hz, 1H), 7.42 (d, J=1.4 Hz, 1H), 7.27-7.34 m, 3H), 6.97 (m, 2H), 6.91 (dd, J=8.5 Hz, 2.0 Hz, 1H), 4.03 (s, 3H), 3.58 (s, 3H), 1.44 (s, 6H).

### Example 267E

### 2-[3-(4-cyano-3-methoxyphenyl)-11-oxo-10,11-dihydro-5H-dibenzo[b,e][1,4]diazepin-8-yl]-2-methylN(4-morpholin-4-ylphenyl)propanamide

Example 267D was saponified by the method of Example 13, then that acid was converted to the final compound by the method described in Example 266J, except preparative HPLC was used for the purification. MS (ESI) m/e 588 (M+H)⁺; ¹H NMR (400 MHz, DMSO-d₆) δ 9.85 (s, 1H), 8.81 (s, 1H), 7.96 (s, 1H), 7.82 (d, J=8.0 Hz, 1H), 7.77 (d, J=8.0 Hz, 1H), 7.43 (d, J=8.9 Hz, 2H), 7.40 (s, 1H), 7.26-7.33 (m, 3H), 7.04 (d, J=1.5 Hz, 1H), 6.97 (d, J=6.0 Hz, 1H), 6.93 (dd, J=6.0, 1.5 Hz, 1H), 6.88 (d, J=8.9 Hz, 2H), 4.03 (s, 3H), 3.71 (m, 4H), 3.00 (m, 4H), 1.49 (s, 6H).

### Example 268

### 2-[3-(4-chloro-3-methoxyphenyl)-11-oxo-10,11-dihydro-5H-dibenzo[b,e][1,4]diazepin-8-yl]-2-methylN(4-morpholin-4-ylphenyl)propanamide

### Example 268A

### 2-(4-chloro-3-methoxyphenyl)-4,4,5,5-tetramethyl-1,3,2-dioxaborolane

Starting with 2-methoxy-4-nitro-chlorobenzene, the title compound was prepared by the methods described in Examples 267A, 267B, and 267C. MS (DCI) m/e (M+H)⁺286 and 288 (M+NH₄)⁺.

### Example 268B

### 2-[3-(4-chloro-3-methoxyphenyl)-11-oxo-10,11-dihydro-5H-dibenzo[b,e][1,4]diazepin-8-yl]-2-methylN(4-morpholin-4-ylphenyl)propanamide

Starting with the compounds described in 266F and 268A, the title compound was prepared by the methods of Examples 266H, 266I, and Example 266J, except preparative HPLC was used for the purification. MS (ESI) m/e 595 and 597 (M+H)⁺; ¹H NMR (400 MHz, DMSO-d₆) δ 9.80 (s, 1H), 8.81 (s, 1H), 7.92 (s, 1H), 7.75 (d, J=8.0 Hz, 1H), 7.51 (d, J=8.3 Hz, 1H), 7.43 (d, J=9.2 Hz, 2H), 7.32 (d, J=1.8 Hz, 1H), 7.28 (d, J=1.5 Hz, 1H), 7.03 (d, J=1.5 Hz, 1H), 6.97 (d, J=8.4 Hz, 1H), 6.93 (dd, J=8.4, 1.5 Hz, 1H), 6.88 (d, J=9.2 Hz, 2H), 3.94 (s, 3H), 3.72 (m, 4H), 3.04 (m, 4H), 1.48 (s, 6H).

### Example 269

### 2-[3-(3-methoxy-4-nitrophenyl)-11-oxo-10,11-dihydro-5H-dibenzo[b,e][1,4]diazepin-8-yl]-2-methylNpyridin-2-ylpropanamide

The title compound was prepared by substituting Example 266I and pyridin-2-ylamine for dimethylaminoacetic acid and Example 120, respectively, in Example122. MS (ESI) m/e 524 (M+H)⁺; ¹H NMR (300 MHz, DMSO-d₆) δ 9.86 (s, 1H), 9.19 (s, 1H), 8.23 (m, 1H), 8.04 (s, 1H), 8.00 (d, J=8.5 Hz, 2H), 7.82 (m, 1H), 7.79 (d, J=8.1 Hz, 1H), 7.52 (d, J=1.7 Hz, 1H), 7.28-7.35 (m, 3H), 7.10 (m, 1H), 7.06 (s, 1H), 7.00 (s, 2H), 4.02 (s, 3H), 1.53 (s, 6H).

### Example 270

### 2-[3-(3-methoxy-4-nitrophenyl)-11-oxo-10,11-dihydro-5H-dibenzo[b,e][1,4]diazepin-8-yl]-2-methylNpyridin-3-ylpropanamide

The title compound was prepared by substituting Example 266I and pyridin-3-ylamine for dimethylaminoacetic acid and Example 120, respectively, in Example122. MS (ESI) m/e 524 (M+H)⁺; ¹H NMR (300 MHz, DMSO-d₆) δ 9.89 (s, 1H), 9.50 (s, 1H), 8.92 (d, J=2.4 Hz, 1H), 8.35 (dd, J=4.7, 1.0 Hz, 1H), 8.20 (m, 1H), 8.04 (s, 1H), 8.01 (d, J=8.1 Hz, 1H), 7.79 (d, J=8.1 Hz, 1H), 7.54 (m, 1H), 7.51 (d, J=1.7 Hz, 1H), 7.28- 7.35 (m, 3H), 7.02 (d, J=2.0 Hz, 1H), 7.00 (d, J=8.3 Hz, 1H), 6.96 (dd, J=8.3, 2.0, 1H), 4.03 (s, 3H), 1.52 (s, 6H).

### Example 271

### 2-[3-(3-methoxy-4-nitrophenyl)-11-oxo-10,11-dihydro-5H-dibenzo[b,e][1,4]diazepin-8-yl]-2-methylNpyridin-4-ylpropanamide

The title compound was prepared by substituting Example 266I and pyridin-4-ylamine for dimethylaminoacetic acid and Example 120, respectively, in Example122. MS (ESI) m/e 524 (M+H)⁺; ¹H NMR (300 MHz, DMSO-d₆) δ 10.24 (s, 1H), 9.88 (s, 1H), 8.64 (d, J=7.1 Hz, 2H), 8.11 (d, J=7.1 Hz, 2H), 8.08 (s, 1H), 8.01 (d, J=8.5 Hz, 1H), 7.79 (d, J=8.1 Hz, 1H), 7.51 (d, J=1.7 Hz, 1H), 7.28-7.35 (m, 3H), 7.02 (d, J=8.1 Hz, 1H), 7.00 (d, J=2.0 Hz, 1H), 6.93 (dd, J=8.1, 2.0 Hz, 1H), 4.03 (s, 3H), 1.53 (s, 6H).

### Example 272

### 2-[3-(3-methoxy-4-nitrophenyl)-11-oxo-10,11-dihydro-5H-dibenzo[b,e][1,4]diazepin-8-yl]-2-methylN(pyridin-3-ylmethyl)propanamide

The title compound was prepared by substituting Example 266I and 3-(aminomethyl)pyridine for dimethylaminoacetic acid and Example 120, respectively, in Example122. MS (ESI) m/e 538 (M+H)⁺; ¹H NMR (300 MHz, DMSO-d₆) δ 9.84 (s, 1H), 8.57 (dd, J=5.8, 1.4 Hz, 1H), 8.52 (d, J=1.4 Hz, 1H), 8.02 (s, 1H), 8.01 (d, J=8.5 Hz, 1H), 7.92 (m, 1H), 7.87 (m, 1H), 7.82 (d, J=8.1 Hz, 1H), 7.58 (dd, 7.8, 5.1 Hz, 1H), 7.52 (d, J=1.7 Hz, 1H), 7.29-7.35 (m, 3H), 7.00 (d, J=2.0 Hz, 1H), 6.96 (d, J=8.3 Hz, 1H), 6.89 (dd, J=8.3, 2.0 Hz, 1H), 4.29 (d, J=5.8 Hz, 2H), 4.03 (s, 3H), 1.42 (s, 6H).

### Example 273

### 2-[3-(3-methoxy-4-nitrophenyl)-11-oxo-10,11-dihydro-5H-dibenzo[b,e][1,4]diazepin-8-yl]-2-methylN(pyridin-3-ylmethyl)propanamide

The title compound was prepared by substituting Example 266I and 4-(aminomethyl)pyridine for dimethylaminoacetic acid and Example 120, respectively, in Example122. MS (ESI) m/e 538 (M+H)⁺; ¹H NMR (300 MHz, DMSO-d₆) δ 9.87 (s, 1H), 8.63 (dd, J=6.1 Hz, 2H), 8.03 (s, 1H), 8.01 (d, J=8.5 Hz, 1H), 7.98 (d, J=5.8 Hz, 1H), 7.82 (d, J=8.1 Hz, 1H), 7.51 (m, 3H), 7.29-7.36 (m, 3H), 7.02 (d, J=1.7 Hz, 1H), 7.00 (d, J=8.3 Hz, 1H), 6.93 (dd, J=8.3, 1.7 Hz, 1H), 4.30 (d, J=5.8 Hz, 2H), 4.03 (s, 3H), 1.46 (s, 6H).

### Example 274

### 2-[3-(3-methoxy-4-nitrophenyl)-11-oxo-10,11-dihydro-5H-dibenzo[b,e][1,4]diazepin-8-yl]-2-methylN(tetrahydrofuran-3-ylmethyl)propanamide

The title compound was prepared by substituting Example 266I and (±)-3-aminomethyltetrahydrofuran for dimethylaminoacetic acid and Example 120, respectively, in Example122. MS (DCI) m/e 531 (M+H)⁺; ¹H NMR (500 MHz, DMSO-d₆) δ 9.83 (s, 1H), 8.01 (d, J=8.4 Hz, 1H), 7.97 (s, 1H), 7.80 (d, J=8.1 Hz, 1H), 7.52 (d, J=1.6 Hz, 1H), 7.30-7.35 (m, 4H), 6.98 (d, J=2.0 Hz, 1H), 6.96 (d, J=8.3 Hz, 1H), 6.86 (dd, J=8.3, 2.0 Hz, 1H), 4.03 (s, 3H), 3.63 (m, 1H), 3.54 (m, 2H), 3.29 (m, 1H), 2.99 (m, 2H), 2.34 (m, 1H), 1.79 (m, 1H), 1.44 (m, 1H), 1.38 (s, 6H).

### Example 275

### 2-[3-(3-methoxy-4-nitrophenyl)-11-oxo-10,11-dihydro-5H-dibenzo[b,e][1,4]diazepin-8-yl]-2-methylN1,3-thiazol-2-ylpropanamide

The title compound was prepared by substituting Example 266I and 2-aminothiazole for dimethylaminoacetic acid and Example 120, respectively, in Example122. MS (ESI) m/e 530 (M+H)⁺; ¹H NMR (300 MHz, DMSO-d₆) δ 11.54 (s, 1H), 9.87 (s, 1H), 8.02 (s, 1H), 8.01 (d, J=8.5 Hz, 1H), 7.79 (d, J=8.1 Hz, 1H), 7.52 (d, J=1.7Hz, 1H), 7.42 (d, J=3.7 Hz, 1H), 7.28-7.35 (m, 3H), 7.19 (d, J=3.7 Hz, 1H), 6.99 (m, 2H), 6.90 (dd, J=8.1, 2.0 Hz, 1H), 4.02 (s, 3H), 1.54 (s, 6H).

### Example 276

### 2-[3-(3-methoxy-4-nitrophenyl)-11-oxo-10,11-dihydro-5H-dibenzo[b,e][1,4]diazepin-8-yl]-2-methylN{[6-(trifluoromethyl)pyridin-3-yl]methyl}propanamide

The title compound was prepared by substituting Example 266I and 5-(aminomethyl)-2-(trifluoromethyl)-pyridine for dimethylaminoacetic acid and Example 120, respectively, in Example122. MS (ESI) m/e 606 (M+H)⁺; ¹H NMR (300 MHz, DMSO-d₆) δ 9.87 (s, 1H), 8.55 (s, 1H), 8.02 (d, J=8.5 Hz, 1H), 8.01 (s, 1H), 7.93 (t, J=5.9 Hz, 1H), 7.81 (d, J=8.1 Hz, 1H), 7.76 (d, J=1.4 Hz, 2H), 7.52 (d, J=1.4 Hz, 1H), 7.29-7.35 (m, 3H), 7.01 (d, J=2.0 Hz, 1H), 6.97 (d, J=8.1 Hz, 1H), 6.87 (dd, J=8.1, 2.0 Hz, 1H), 4.31 (d, J=5.9 Hz, 2H), 4.03 (s, 3H), 1.42 (s, 6H).

### Example 277

### N-(4-fluorophenyl)-2-[3-(3-methoxy-4-nitrophenyl)-11-oxo-10,11-dihydro-5H-dibenzo[b,e][1,4]diazepin-8-yl]-2-methylpropanamide

The title compound was prepared by substituting Example 266I and 4-fluoroaniline for dimethylaminoacetic acid and Example 120, respectively, in Example122. MS (ESI) m/e 539 (M-H)⁻; ¹H NMR (300 MHz, DMSO-d₆) δ 9.89 (s, 1H), 9.06 (s, 1H), 8.01 (s, 1H), 8.01 (d, J=8.5 Hz, 1H), 7.79 (d, J=8.1 Hz, 1H), 7.57 (m, 2), 7.51 (d, J=1.4 Hz, 1H), 7.30-7.35 (m, 3H), 7.10 (m, 2H), 7.04 (d, J=2.0 Hz, 1H), 7.00 (d, J=8.1 Hz, 1H), 6.97 (dd, J=8.1, 2.0 Hz, 1H), 4.03 (s, 3H), 1.49 (s, 6H).

### Example 278

### N-(2,5-difluorobenzyl)-2-[3-(3-methoxy-4-nitrophenyl)-11-oxo-10,11-dihydro-5H-dibenzo[b,e][1,4]diazepin-8-yl]-2-methylpropanamide

The title compound was prepared by substituting Example 266I and 2,5-difluoro-benzylamine for dimethylaminoacetic acid and Example 120, respectively, in Example122. MS (ESI) m/e 571 (M-H)⁻; ¹H NMR (300 MHz, DMSO-d₆) δ 9.84 (s, 1H), 8.01 (d, J=8.5 Hz, 1H), 8.01 (s, 1H), 7.83 (t, J=5.8 Hz, 1H), 7.81 (d, J=8.1 Hz, 1H), 7.52 (d, J=1.4 Hz, 1H), 7.35, 7.32, 7.28 (all m, total 3H), 7.17 (m, 1H), 7.07 (m, 1H), 7.02 (d, J=2.0 Hz, 1H), 6.99 (d, J=8.3 Hz, 1H), 6.90 (dd, J=8.3, 2.0 Hz, 1H), 6.77 (m, 1H), 4.22 (d, J=5.8Hz, 2H), 4.03 (s, 3H), 1.43 (s, 6H).

### Example 279

### 2-methyl-2-[11-oxo-3-(pyrimidin-4-ylamino)-10,11-dihydro-5H-dibenzo[b,e][1,4]diazepin-8-yl]N[3-(trifluoromethyl)benzyl]propanamide

### Example 279A

### methyl 2-methyl-2-[11-oxo-3-(pyrimidin-4-ylamino)-10,11-dihydro-5H-dibenzo[b,e][1,4]diazepin-8-yl]propanoate

Example 266F (1.60g, 4.64 mmol), 4-aminopyrimidine (0.76g, 8.00 mmol), CyMAP ligand (0.34g, 0.87 mmol), Pd₂(dba)₃ (0.28g, 0.31 mmol), and CsCO₃ (2.60g, 7.97 mmol) in dioxane (25 mL) were heated at 85° C overnight. The reaction was then cooled, filtered through Celite^{®}, and concentrated. The crude solids were slurried in Et₂O (25 mL) overnight. After filtration and drying, had 1.98g solids that were slurried in Et₂O (20 mL) for 3 hours, then the solids were filtered off. Recovered the product (1.66g, 89%) as pale green solids. MS (DCI) m/e 404 (M+H)⁺; ¹H NMR (300 MHz, DMSO-d₆) δ 9.77 (s, 1H), 9.57 (s, 1H), 8.70 (s, 1H), 8.54 (d, 5.8, J=5.8 Hz, 1H), 7.95 (s, 1H), 7.64 (d, J=8.5 Hz, 1H), 7.51 (d, J=2.0 Hz, 1H), 7.07 (dd, J=8.5, 2.0 Hz, 1H), 6.97 (d, J=8.1 Hz, 1H), 6.91 (m, 2H), 6.88 (d, J=5.8 Hz, 1H), 3.57 (s, 3H), 1.43 (s, 6H).

### Example 279B

### 2-methyl-2-[11-oxo-3-(pyrimidin-4-ylamino)-10,11-dihydro-5H-dibenzo[b,e][1,4]diazepin-8-yl]N[3-(trifluoromethyl)benzyl]propanamide

The title compound was prepared by substituting the acid, from hydrolysis of Example 279A, and 3-(trifluoromethyl)benzylamine for dimethylaminoacetic acid and Example 120, respectively, in Example122. MS (DCI) m/e 547 (M+H)⁺; ¹H NMR (300 MHz, DMSO-d₆) δ 10.50 (s, 1H), 9.66 (s, 1H), 8.85 (s, 1H), 8.39 (d, J=6.4 Hz, 1H), 7.99 (s, 1H), 7.89 (t, J=6.4 Hz, 1H), 7.69 (d, J=8.5 Hz, 1H), 7.55, 7.42, 7.30 (all m, total 5H), 7.14 (dd, J=8.5, 1.9 Hz, 1H), 6.93-7.00 (m, 3H), 6.86 (dd, J=8.1, 2.0 Hz, 1H), 4.28 (d, J=6.4 Hz, 2H), 1.42 (s, 6H).

### Example 280

### 2-methyl-2-[11-oxo-3-(pyrimidin-4-ylamino)-10,11-dihydro-5H-dibenzo[b,e][1,4]diazepin-8-yl]N[3-(trifluoromethoxy)benzyl]propanamide

The title compound was prepared by substituting the acid, from hydrolysis of Example 279A, and 3-(trifluoromethoxy)benzylamine for dimethylaminoacetic acid and Example 120, respectively, in Example 122. MS (DCI) m/e 563 (M+H)⁺; ¹H NMR (300 MHz, DMSO-d₆) δ 10.46 (s, 1H), 9.65 (s, 1H), 8.85 (s, 1H), 8.39 (d, J=6.1 Hz, 1H), 7.98 (s, 1H), 7.86 (t, J=6.4 Hz, 1H), 7.69 (d, J=8.8 Hz, 1H), 7.39 (d, J=2.0 Hz, 1H), 7.34 (d, J=8.1 Hz, 1H), 7.15 (m, 3H), 7.07 (br s, 1H), 6.93-7.00 (m, 3H), 6.86 (dd, J=8.3, 2.2 Hz, 1H), 4.25 (d, J=6.1 Hz, 2H), 1.42 (s, 6H).

### Example 281

### 1-[3-(3-methoxy-4-nitrophenyl)-11-oxo-10,11-dihydro-5H-dibenzo[b,e][1,4]diazepin-8-yl]N(4-morpholin-4-ylphenyl)cyclopropanecarboxamide

### Example 281A

### methyl 1-(4-nitrophenyl)cyclopropanecarboxylate

The title compound was prepared by substituting 1,2-dibromoethane for MeI in Example 266A.. MS (DCI) m/e 239 (M+NH₄)⁺; ¹H NMR (300 MHz, CDCl₃) δ 8.17 (m, 2H), 7.51 (m, 2H), 3.65 (s, 3H), 1.71 (m, 2H), 1.23 (m, 2H).

### Example 281B

### methyl 1-[4-(acetylamino)phenyl]cyclopropanecarboxylate

Example 281A (11.1g, 50.2 mmol) andiron powder (325 mesh, 24.4g, 437 mmol) in CH₃CO₂H (500 mL) were heated under reflux with mechanical stirring overnight. The reaction was cooled and partitioned between water and EtOAc. The organic layer was washed with brine and dried over Na₂SO₄.After filtration and concentration toluene was used to azeotrope off the remaining acetic acid, giving the product (11.8g, 100%) as off-white solids. MS (DCI) m/e 234 (M+H)⁺, 251 (M+NH₄)⁺; ¹H NMR (300 MHz, CDCl₃) δ 7.43 (br d, J=8.5 Hz, 2H), 7.29 (br d, J=8.5 Hz, 2H), 7.13 (br s, 1H), 3.62 (s, 3H), 1.59 (m, 2H), 1.16 (m, 2H).

### Example 281 C

### methyl 1-(4-amino-3-nitrophenyl)cyclopropanecarboxylate

Example 281B was nitrated by the method described in the first paragraph of Example 6A, then the acetamide was hydrolyzed by the following method.

That nitro-acetamide (13.2g, 47.5 mmol) was dissolved in MeOH (650 mL) and conc. H₂SO₄ (65 mL) and heated under reflux for 2 hours. The reaction was cooled, most of the MeOH stripped off, then slowly added to 0.5M Na₂CO₃ (1.2 L). That aqueous layer was extracted with EtOAc (1 L), then the organic layer was washed with brine and dried over Na₂SO₄. After filtration and concentration the product (11.2g, 100%) was obtained as a dark syrup that slowly crystallized upon standing. MS (DCI) m/e 254 (M+NH₄)⁺; ¹H NMR (300 MHz, DMSO-d₆) δ 7.84 (d, J=2.0 Hz, 1H), 7.43 (br s, 2H), 7.39 (dd, J=8.6, 2.0 Hz, 1H), 6.96 (d, J=8.6 Hz, 1H), 3.55 (s, 3H), 1.45 (m, 2H), 1.17 (m, 2H).

### Example 281D

### 1-[3-(3-methoxy-4-nitrophenyl)-11-oxo-10,11-dihydro-5H-dibenzo[b,e][1,4]diazepin-8-yl]cyclopropanecarboxylic acid

The title compound was prepared from the compound described in Example 281C by the methods of Examples 1A, 281B, the second paragraph of 281C, 5C, 266H, and 13. MS (DCI) m/e 446 (M+H)⁺, 463 (M+NH₄)⁺; ¹H NMR (300 MHz, DMSO-d₆) δ 12.26 (v br s, 1H), 9.84 (s, 1H), 8.02 (d, J=8.5 Hz, 1H), 8.01 (s, 1H), 7.80 (d, J=8.1 Hz, 1H), 7.52 (d, J=1.7 Hz, 1H), 7.30-7.35 (m, 3H), 6.93 (m, 3H), 4.03 (s, 3H), 1.40 (m, 2H), 1.05 (m, 2H).

### Example 281E

### 1-[3-(3-methoxy-4-nitrophenyl)-11-oxo-10,11-dihydro-5H-dibenzo[b,e][1,4]diazepin-8-yl]N(4-morpholin-4-ylphenyl)cyclopropanecarboxamide

The title compound was prepared by substituting Example 281I and 4-morpholin-4-yl-phenylamine for dimethylaminoacetic acid and Example 120, respectively, in Example 122. MS (ESI) m/e 606 (M+H)⁺; ¹H NMR (300 MHz, DMSO-d₆) δ 9.86 (s, 1H), 8.55 (s, 1H), 8.06 (s, 1H), 8.01 (d, J=8.1 Hz, 1H), 7.80 (d, J=8.1 Hz, 1H), 7.52 (d, J=1.7 Hz, 1H), 7.29-7.32 (m, 5H), 7.03 (s, 1H), 7.00 (s, 2H), 6.82 (m, 2H), 4.03 (s, 3H), 3.70 (m, 4H), 3.01 (m, 4H), 1.38 (m, 2H), 0.98 (m, 2H).

### Example 282

### 2-[3-(3-methoxy-4-nitrophenyl)-11-oxo-10,11-dihydro-5H-dibenzo[b,e][1,4]diazepin-7-yl]-2-methylN(4-morpholin-4-ylphenyl)propanamide

### Example 282A

### methyl 2-(3-fluoro-4-nitrophenyl)-2-methylpropanoate

2-(3-Fluoro-4-nitro-phenyl)-propionic acid methyl ester was prepared from 2-fluoronitrobenzene by the procedure of T. Lemek, et. al. Tetrahedron, 57, 4753 (2001) and then converted to the title compound using the method of Example 266A. MS (DCI) m/e 259 (M+NH₄)⁺; ¹H NMR (300 MHz, CDCl₃) δ 8.03 (m, 1H), 7.26 (m, 2H), 3.69 (s, 3H), 1.61 (s, 6H).

### Example 282B

### methyl 2-(3-amino-4-nitrophenyl)-2-methylpropanoate

Example 282A (14.5g, 60.2 mmol) in 7.0N NH₃ in MeOH (150 mL) was heated at 70° C in a sealed tube overnight. The reaction was cooled, concentrated, and purified by column chromatography using 85/15 hexane/EtOAc. Recovered the product (8.3g, 58%) as bright yellow solids. MS (DCI) m/e 239 (M+H)⁺, 256 (M+NH₄)⁺; ¹H NMR (300 MHz, DMSO-d₆) δ 7.79 (d, J=8.8 Hz, 1H), 7.41 (br s, 2H), 6.78 (d, J=2.0 Hz, 1H), 6.97 (dd, J=8.8, 2.0 Hz, 1H), 3.61 (s, 3H), 1.46 (s, 6H).

### Example 282C

### methyl 2-(3-chloro-11-oxo-10,11-dihydro-5H-dibenzo[b,e][1,4]diazepin-7-yl)-2-methylpropanoate

Example 282B was converted to the title compound by the methods of Examples 1A, 266B, and 5C. MS (DCI) m/e 345 and 347 (M+H)⁺; ¹H NMR (300 MHz, DMSO-d₆) δ 9.90 (s, 1H), 8.07 (s, 1H), 7.68 (d, J=8.5 Hz, 1H), 7.06 (d, J=2.0 Hz, 1H), 6.95, 6.91 (both m, total 4H), 3.58 (s, 3H), 1.46 (s, 6H).

### Example 282D

### 2-[3-(3-methoxy-4-nitrophenyl)-11-oxo-10,11-dihydro-5H-dibenzo[b,e][l,4]diazepin-7-yl]-2-methylpropanoic acid

The compound described in Example 282C was converted to the title compound by the methods of Examples 266H and 13. MS (DCI) m/e 448 (M+H)⁺, 465 (M+NH₄)⁺; ¹H NMR (300 MHz, DMSO-d₆) δ 12.25 (v br s, 1H), 9.89 (s, 1H), 8.06 (s, 1H), 8.01 (d, J=8.5 Hz, 1H), 7.80 (d, J=8.1 Hz, 1H), 7.53 (d, J=1.7Hz, 1H), 7.29-7.38 m, 3H), 7.06, 6.92 (both m, total 3H), 4.04 (s, 3H), 1.43 (s, 6H).

### Example 282E

### 2-[3-(3-methoxy-4-nitrophenyl)-11-oxo-10,11-dihydro-5H-dibenzo[b,e][1,4]diazepin-7-yl]-2-methylN(4-morpholin-4-ylphenyl)propanamide

The title compound was prepared by substituting Example 282D and 4-morpholin-4-yl-phenylamine for dimethylaminoacetic acid and Example 120, respectively, in Example122. MS (ESI) m/e 608 (M+H)⁺; ¹H NMR (300 MHz, DMSO-d₆) δ 9.89 (s, 1H), 8.85 (s, 1H), 8.06 (s, 1H), 8.01 (d, J=8.5 Hz, 1H), 7.79 (d, J=8.1 Hz, 1H), 7.52 (d, J=1.7 Hz, 1H), 7.42 (d, J=9.2 Hz, 2H), 7.29-7.38 (m, 3H), 7.04 (d, J=1.7 Hz, 1H), 6.91 (m, 2H), 6.83 (d, J=9.2 Hz, 2H), 4.03 (s, 3H), 3.71 (m, 4H), 3.00 (m, 4H), 1.49 (s, 6H).

### Example 283

### 2-[3-(3-methoxy-4-nitrophenyl)-11-oxo-10,11-dihydro-5H-dibenzo[b,e][1,4]diazepin-7-yl]-2-methylN(pyridin-2-ylmethyl)propanamide

The title compound was prepared by substituting Example 282D and 2-(aminomethyl)pyridine for dimethylaminoacetic acid and Example 120, respectively, in Example122. MS (ESI) m/e 538 (M+H)⁺; ¹H NMR (300 MHz, DMSO-d₆) δ 9.91 (s, 1H), 8.52 (m, 1H), 8.05 (s, 1H), 8.03 (d, J=8.5 Hz, 1H), 7.95 (t, J=5.8 Hz, 1H), 7.81 (d, J=8.1 Hz, 1H), 7.78 (m, 1H), 7.52 (d, J=1.7 Hz, 1H), 7.39 (d, J=1.7 Hz, 1H), 7.37-7.30 (m, 3H), 7.16 (br d, J=7.8 Hz, 1H), 7.06 (d, J=2.0 Hz, 1H), 6.95 (d, J=8.1 Hz, 1H), 6.90 (dd, J=8.1, 2.0 Hz, 1H), 4.35 (d, J=5.8 Hz, 1H), 4.04 (s, 3H), 1.46 (s, 6H).

### Example 284

### 2-[3-(3-methoxy-4-nitrophenyl)-11-oxo-10,11-dihydro-5H-dibenzo[b,e][1,4]diazepin-7-yl]-2-methylN(thien-2-ylmethyl)propanamide

The title compound was prepared by substituting Example 282D and 2-(aminomethyl)thiophene for dimethylaminoacetic acid and Example 120, respectively, in Example122. MS (ESI) m/e 543 (M+H)⁺; ¹H NMR (300 MHz, DMSO-d₆) δ 9.88 (s, 1H), 8.03 (s, 1H), 8.02 (d, J=8.5 Hz, 1H), 7.79 (d, J=8.1 Hz, 1H), 7.75 (t, J=5.8 Hz, 1H), 7.52 (d, J=1.7 Hz, 1H), 7.39 (d, J=1.7 Hz, 1H), 7.30-7.37 (m, 3H), 7.04 (m, 2H), 6.88-6.90 (m, 3H), 4.20 (d, J=5.8 Hz, 1H), 4.03 (s, 3H), 1.43 (s, 6H).

### Example 285

### N-(cyclopropylmethyl)-2-[3-(3-methoxy-4-nitrophenyl)-11-oxo-10,11-dihydro-5H-dibenzo[b,e][1,4]diazepin-7-yl]-2-methylpropanamide

The title compound was prepared by substituting Example 282D and (aminomethyl)cyclopropane for dimethylaminoacetic acid and Example 120, respectively, in Example122. MS (ESI) m/e 501 (M+H)⁺; ¹H NMR (300 MHz, DMSO-d₆) δ 9.88 (s, 1H), 8.02 (s, 1H), 8.01 (d, J=8.5 Hz, 1H), 7.79 (d, J=8.1 Hz, 1H), 7.52 (d, J=1.7 Hz, 1H), 7.38 (d, J=1.7 Hz, 1H), 7.36, 7.32, 7.28 (all m, 3H total), 7.00 (d, J=1.7 Hz, 1H), 6.91 (d, J=8.3 Hz, 1H), 6.85 (dd, J=8.3, 1.7 Hz, 1H), 4.03 (s, 3H), 2.91 (m, 2H), 1.39 (s, 6H), 0.87 (m, 1H), 0.29 (m, 2H), 0.08 (m, 2H).

### Example 286

### 7-(3-hydroxypropyl)-3-(3-methoxy-4-nitrophenyl)-5,10-dihydro-11H-dibenzo[b,e][1,4]diazepin-11-one

### Example 286A

### methyl 3-[3-(acetylamino)-4-nitrophenyl]propanoate

Methyl 3-(3'-aminophenyl)propionate was treated with acetic anhydride and nitrated using the method described in the first paragraph of Example 6A. MS (DCI) m/e 267 (M+H)⁺, 284 (M+NH₄)⁺; ¹H NMR (300 MHz, DMSO-d₆) δ 10.22 (s,1H), 7.86 (d, J=8.5 Hz, 1H), 7.49 (d, J=2.0 Hz, 1H), 7.21 (dd, J=8.5, 2.0 Hz, 1H), 3.59 (s, 3H), 2.92 (t, J=7.3 Hz, 2H), 2.68 (t, J=7.3 Hz, 2H), 2.06 (s, 3H).

### Example 286B

### methyl 3-(3-chloro-11-oxo-10,11-dihydro-5H-dibenzo[b,e][1,4]diazepin-7-yl)propanoate

The title compound was prepared from the compound described in Example 286A by the methods described in the second paragraph of Example 281C, Example 1A, Example 6C, and Example 5C. MS (DCI) m/e 331 and 333 (M+H)⁺; ¹H NMR (300 MHz, DMSO-d₆) δ 9.85 (s, 1H), 8.00 (s, 1H), 7.67 (d, J=8.5 Hz, 1H), 7.07 (d, J=2.4 Hz, 1H), 6.92 (dd, J=8.5, 2.4 Hz, 1H), 6.88 (d, J=8.3 Hz, 1H), 6.77-6.80 (m, 2H), 3.58 (s, 3H), 2.73 (t, J=7.3 Hz, 2H), 2.50 (t, J=7.3 Hz, 2H).

### Example 286C

### 3-chloro-7-(3-hydroxypropyl)-5,10-dihydro-11H-dibenzo[b,e][1,4]diazepin-11-one

The title compound was prepared by substituting Example 286B for Example 6D in Example 204A. MS (DCI) m/e 303 and 305 (M+H)⁺; ¹H NMR (300 MHz, DMSO-d₆) δ 9.84 (s, 1H), 7.97 (s, 1H), 7.67 (d, J=8.5 Hz, 1H), 7.06 (d, J=2.0 Hz, 1H), 6.92 (dd, J=8.5, 2.0 Hz, 1H), 6.87 (d, J=8.1 Hz, 1H), 6.80 (d, J=1.7 Hz, 1H), 6.78 (dd, J=8.1, 1.7 Hz, 1H), 4.44 (t, J=5.1 Hz, 1H), 3.38 (m, 2H), 2.50 (m, 2H), 1.66 (m, 2H).

### Example 286D

### 7-(3-hydroxypropyl)-3-(3-methoxy-4-nitrophenyl)-5,10-dihydro-11H-dibenzo[b,e][1,4]diazepin-11-one

The title compound was prepared by substituting Example 286C and Example 266G for Example 59B and Example 56A, respectively, in Example 59C. MS (DCI) m/e 420 (M+H)⁺; ¹H NMR (300 MHz, DMSO-d₆) δ 9.83 (s, 1H), 8.01 (d, J=8.5 Hz, 1H), 7.94 (s, 1H), 7.79 (d, J=8.1 Hz, 1H), 7.52 (d, J=1.7 Hz, 1H), 7.29-7.36 (m, 3H), 6.87 (d, J=8.0 Hz, 1H), 6.85 (d, J=2.0 Hz, 1H), 6.75 (dd, J=8.0, 2.0 Hz, 1H), 4.44 (t, J=5.3 Hz, 1H), 4.03 (s, 3H), 3.39 (m, 2H), 3.30 (m, 2H), 1.66 (m, 2H).

### Example 287

### 7-(3-hydroxy-3-methylbutyl)-3-(3-methoxy-4-nitrophenyl)-5,10-dihydro-11H-dibenzo[b,e][1,4]diazepin-11-one

### Example 287A

### 3-chloro-7-(3-hydroxy-3-methylbutyl)-5,10-dihydro-11H-dibenzo[b,e][1,4]diazepin-11-one

The title compound was prepared by substituting Example 286B for Example 1B in Example 189A. MS (DCI) m/e 331 and 333 (M+H)⁺; ¹H NMR (300 MHz, DMSO-d₆) δ 9.84 (s, 1H), 7.95 (s, 1H), 7.67 (d, J=8.5 Hz, 1H), 7.06 (d, J=2.0 Hz, 1H), 6.92 (dd, J=8.5, 2.0 Hz, 1H), 6.85 (d, J=8.1 Hz, 1H), 6.81 (d, J=2.0 Hz, 1H), 6.75 (dd, J=8.1, 2.0 Hz, 1H), 4.24 (br s, 1H), 2.50 (m, 2H), 1.57 (m, 2H), 1.12 (s, 6H).

### Example 287B

### 7-(3-hydroxy-3-methylbutyl)-3-(3-methoxy-4-nitrophenyl)-5,10-dihydro-11H-dibenzo[b,e][1,4]diazepin-11-one

The title compound was prepared by substituting Example 287A and Example 266G for Example 59B and Example 56A, respectively, in Example 59C. MS (DCI) m/e 448 (M+H)⁺; ¹H NMR (300 MHz, DMSO-d₆) δ 9.84 (s, 1H), 8.01 (d, J=8.5 Hz, 1H), 7.92 (s, 1H), 7.79 (d, J=8.1 Hz, 1H), 7.52 (d, J=1.4 Hz, 1H), 7.29-7.36 (m, 3H), 6.87 (d, J=8.0 Hz, 1H), 6.85 (d, J=2.0 Hz, 1H), 6.75 (dd, J=8.0, 2.0 Hz, 1H), 4.24 (s, 1H), 4.04 (s, 3H), 2.50 (m, 2H), 1.57 (m, 2H), 1.12 (s, 6H).

### Example 288

### 8-(2-hydroxy-1,1-dimethylethyl)-3-(3-methoxy-4-nitrophenyl)-5,10-dihydro-11H-dibenzo[b,e][1,4]diazepin-11-one

### Example 288A

### 3-chloro-8-(2-hydroxy-1,1-dimethylethyl)-5,10-dihydro-11H-dibenzo[b,e][1,4]diazepin-11-one

The title compound was prepared by substituting Example 266F for Example 6D in Example 204A. MS (DCI) m/e 317 and 319 (M+H)⁺; ¹H NMR (300 MHz, DMSO-d₆) δ 9.77 (s, 1H), 7.97 (s, 1H), 7.67 (d, J=8.5 Hz, 1H), 7.05 (d, J=2.0 Hz, 1H), 6.88-6.99 (m, 4H), 4.61 (t, J=5.1 Hz, 1H), 3.32 (m, 2H), 1.15 (s, 6H).

### Example 288B

### 8-(2-hydroxy-1,1-dimethylethyl)-3-(3-methoxy-4-nitrophenyl)-5,10-dihydro-11H-dibenzo[b,e][1,4]diazepin-11-one

The title compound was prepared by substituting Example 288A and Example 266G for Example 59B and Example 56A, respectively, in Example 59C. MS (ESI) m/e 434 (M+H)⁺; ¹H NMR (300 MHz, DMSO-d₆) δ 9.78 (s, 1H), 8.01 (d, J=8.5 Hz, 1H), 7.93 (s, 1H), 7.79 (d, J=8.1 Hz, 1H), 7.52 (d, J=1.7 Hz, 1H), 7.28-7.35 (m, 3H), 7.01 (m, 1H), 6.94 (m, 2H), 4.61 (t, J=5.1 Hz, 1H), 4.03 (s, 3H), 3.33 (m, 2H), 1.16 (s, 6H).

### Example 289

### 8-(2-hydroxy-1,1,2-trimethylpropyl)-3-(3-methoxy-4-nitrophenyl)-5,10-dihydro-11H-dibenzo[b,e][1,4]diazepin-11-one

### Example 289A

### 3-chloro-8-(2-hydroxy-1,1,2-trimethylpropyl)-5,10-dihydro-11H-dibenzo[b,e][1,4]diazepin-11-one

The title compound was prepared by substituting Example 266F for Example 1B in Example 189A.MS (DCI) m/e 345 and 347 (M+H)⁺; ¹H NMR (300 MHz, DMSO-d₆) δ 9.75 (s, 1H), 7.95 (s, 1H), 7.67 (d, J=8.5 Hz, 1H), 7.07 (d, J=2.0 Hz, 1H), 7.05 (d, J=2.0 Hz, 1H), 7.01 (dd, J=8.5, 2.0 Hz, 1H), 6.89 (dd, J=8.5, 2.0 Hz, 1H), 6.83 (d, J=8.5 Hz, 1H), 4.03 (s, 1H), 1.24 (s, 6H), 0.96 (s, 6H).

### Example 289

### 8-(2-hydroxy-1,1,2-trimethylpropyl)-3-(3-methoxy-4-nitrophenyl)-5,10-dihydro-11H-dibenzo[b,e][1,4]diazepin-11-one

The title compound was prepared by substituting Example 289A and Example 266G for Example 59B and Example 56A, respectively, in Example 59C. MS (ESI) m/e 462 (M+H)⁺; ¹H NMR (400 MHz, DMSO-d₆) δ 9.73 (s, 1H), 8.00 (d, J=8.6 Hz, 1H), 7.89 (s, 1H), 7.79 (d, J=8.3 Hz, 1H), 7.51 (d, J=1.5 Hz, 1H), 7.27-7.34 (m, 3H), 7.08 (d, J=1.8 Hz, 1H), 7.00 (dd, J=8.3, 1.8 Hz, 1H), 6.87 (d, J=8.3 Hz, 1H), 4.03, (s, 3H), 4.00 (s, 1H), 1.24 (s, 6H), 0.96 (s, 6H).

### Example 290

### 8-(1,1-dimethyl-2-oxopropyl)-3-(3-methoxy-4-nitrophenyl)-5,10-dihydro-11H-dibenzo[b,e][1,4]diazepin-11-one

### Example 290A

### 3-chloro-8-(1,1-dimethyl-2-oxopropyl)-5,10-dihydro-11H-dibenzo[b,e][1,4]diazepin-11-one

The title compound was isolated as a by-product in Example 289A. MS (ESI) m/e 329 and 331 (M+H)⁺.

### Example 290B

### 8-(1,1-dimethyl-2-oxopropyl)-3-(3-methoxy-4-nitrophenyl)-5,10-dihydro-11H-dibenzo[b,e][1,4]diazepin-11-one

The title compound was prepared by substituting Example 290A and Example 266G for Example 59B and Example 56A, respectively, in Example 59C. MS (ESI) m/e 446 (M+H)⁺; ¹H NMR (500 MHz, DMSO-d₆) δ 9.82 (s, 1H), 8.04 (s, 1H), 8.01 (d, J=8.4 Hz, 1H), 7.80 (d, J=8.1 Hz, 1H), 7.52 (d, J=1.5 Hz, 1H), 7.30-7.35 (m, 3H), 7.01 (d, J=8.4 Hz, 1H), 6.93 (d, J=1.9 Hz, 1H), 6.88 (dd, J=8.4, 1.9 Hz, 1H), 4.03, (s, 3H), 1.89 (s, 3H), 1.35 (s, 6H).

### Example 291

### 7-(2-hydroxy-1,1-dimethylethyl)-3-(3-methoxy-4-nitrophenyl)-5,10-dihydro-11H-dibenzo[b,e][1,4]diazepin-11-one

Example 282D (0.18g, 0.40 mmol) in THF (2 mL) was cooled to 0° C, then 1.0M BH₃ in THF (8 mL) was added dropwise.The reaction was stirred at 0° C for 1 hour, then at room temperature for 1 hour. 1.0M H₃PO₄ was carefully added, then the mixture was extracted with EtOAc. The organic layer was washed with brine and dried over Na₂SO₄. After filtration and concentration the crude material was purified by preperative HPLC to give the product (34 mg, 19%). MS (DCI) m/e 434 (M+H)⁺; ¹H NMR (300 MHz, DMSO-d₆) δ 9.83 (s, 1H), 8.01 (d, J=8.5 Hz, 1H), 7.95 (s, 1H), 7.79 (d, J=8.1 Hz, 1H), 7.53 (d, J=1.4 Hz, 1H), 7.29-7.37 (m, 3H), 7.04 (d, J=1.7 Hz, 1H), 6.90 (m, 2H), 4.64 (t, J=5.4 Hz, 1H), 4.04 (s, 3H), 3.35 (d, J=5.4 Hz, 2H), 1.17 (s, 6H).

### Example 292

### 8-[1-(hydroxymethyl)cyclopropyl]-3-(3-methoxy-4-nitrophenyl)-5,10-dihydro-11H-dibenzo[b,e][1,4]diazepin-11-one

The title compound was prepared by substituting Example 281D for Example 282D in Example 291. MS (DCI) m/e 432 (M+H)⁺; ¹H NMR (300 MHz, DMSO-d₆) δ 9.79 (s, 1H), 8.02 (d, J=8.5 Hz, 1H), 7.93 (s, 1H), 7.79 (d, J=8.1 Hz, 1H), 7.52 (d, J=1.7 Hz, 1H), 7.28-7.34 (m, 3H), 6.92 (m, 3H), 4.60 (t, J=5.8 Hz, 1H), 4.03 (s, 3H), 3.46 (d, J=5.8 Hz, 2H), 0.77 (m, 2H), 0.61 (m, 2H).

### Example 293

### 3-[(2-chloropyridin-4-yl)amino]-8-(2-hydroxy-1,1-dimethylethyl)-5,10-dihydro-11H-dibenzo[b,e][1,4]diazepin-11-one

The title compound was prepared by substituting Example 288A and 4-amino-2-chloropyridine for Example 189A and 4-aminopyridine in Example 191. MS (ESI) m/e 407 (M-H)⁻; ¹H NMR (300 MHz, DMSO-d₆) δ 9.52 (s, 1H), 9.31 (s, 1H), 8.08 (m, 1H), 7.86 (s, 1H), 7.67 (d, J=8.8 Hz, 1H), 6.99 (m, 3H), 6.93 (m, 1H), 6.86 (m, 2H), 6.64 (dd, J=8.5, 2.0, 1H), 3.32 (s, 2H), 1.15 (s, 6H).

### Example 294

### 3-[(2,6-difluoropyridin-4-yl)amino]-8-(2-hydroxy-1,1-dimethylethyl)-5,10-dihydro-11H-dibenzo[b,e][1,4]diazepin-11-one

The title compound was prepared by substituting Example 288A and Example 203A for Example 189A and 4-aminopyridine in Example 191. MS (DCI) m/e 411 (M+H)⁺; ¹H NMR (300 MHz, DMSO-d₆) δ 9.67 (s, 1H), 9.56 (s, 1H), 7.86 (s, 1H), 7.68 (d, J=8.5 Hz, 1H), 6.98 (d, J=1.7 Hz, 1H), 6.94 (m, 1H), 6.89 (m, 2H), 6.67 (dd, J=8.8, 2.0, 1H), 6.56 (s, 2H), 3.37 (s, 1H), 3.32 (s, 2H), 1.15 (s, 6H).

### Example 295

### 3-[(2,6-difluoropyridin-4-yl)amino]-8-(2-hydroxy-1,1,2-trimethylpropyl)-5,10-dihydro-11H-dibenzo[b,e][1,4]diazepin-11-one

The title compound was prepared by substituting Example 289A and Example 203A for Example 189A and 4-aminopyridine in Example 191. MS (DCI) m/e 439 (M+H)⁺; ¹H NMR (300 MHz, DMSO-d₆) δ 9.67 (s, 1H), 9.54 (s, 1H), 7.85 (s, 1H), 7.68 (d, J=8.8 Hz, 1H), 7.06 (d, J=2.0 Hz, 1H), 6.99 (dd, J=8.8, 2.0 Hz, 1H), 6.89 (d, J=2.0 Hz, 1H), 6.83 (d, J=8.5 Hz, 1H), 6.66 (dd, J=8.8, 2.0, 1H), 6.57 (s, 2H), 4.01 (s, 1H), 1.24 (s, 6H), 0.97 (s, 6H).

### Example 296

### 3-(4-chloro-3-methoxyphenyl)-8-(2-hydroxy-1,1-dimethylethyl)-5,10-dihydro-11H-dibenzo[b,e][1,4]diazepin-11-one

The title compound was prepared by substituting Example 288A and Example 268A for Example 59B and Example 56A, respectively, in Example 59C. MS (ESI) m/e 423 and 425 (M+H)⁺; ¹H NMR (300 MHz, DMSO-d₆) δ 9.71 (s, 1H), 7.87 (s, 1H), 7.75 (d, J=8.5 Hz, 1H), 7.53 (d, J=8.1 Hz, 1H), 7.33 (d, J=1.7 Hz, 1H), 7.29 (d, J=1.7 Hz, 1H), 7.20 (m, 2H), 7.00 (d, J=1.4 Hz, 1H), 6.94 (m, 2H), 3.96 (s, 3H), 3.32 (m, 2H), 1.15 (s, 6H).

### Example 297

### 3-(3-methoxy-4-nitrophenyl)-8-[2-(4-morpholin-4-ylphenoxy)ethyl]-5,10-dihydro-11H-dibenzo[b,e][1,4]diazepin-11-one

### Example 297A

### 3-chloro-8-[2-(4-morpholin-4-ylphenoxy)ethyl]-5,10-dihydro-11H-dibenzo[b,e][1,4]diazepin-11-one

Example 204A (0.57g, 2.0 mmol) and 4-morpholinophenol (0.45g, 2.5 mmol; M.C. Harris, et. al., Org. Lett., 4, 2885 (2002)) were dissolved in THF (15 mL), then polymer-supported PPh₃ (2.0g, 6.0 mmol PPh₃; Aldrich, product # 36,645-5) was added, followed by di-tert-butylazodicarboxylate (0.52g, 2.3 mmol), then the reaction was stirred at room temperature overnight. The reaction was then filtered and concentrated to give 1.6g crude material that was then slurried in Et₂O (20 mL) overnight. Filtration gave the product (0.74g, 81%) as off-white solids. MS (DCI) m/e 450 and 452 (M+H)⁺; ¹H NMR (300 MHz, DMSO-d₆) δ 9.8 (s, 1H), 8.00 (s, 1H), 7.67 (d, J=8.5 Hz, 1H), 7.06 (d, J=2.0 Hz, 1H), 6.84-6.91 (m, 8H), 4.03 (t, J=6.6 Hz, 2H), 3.71 (m, 4H), 2.96 (m, 4H), 2.86 (t, J=6.6 Hz, 2H).

### Example 297B

### 3-(3-methoxy-4-nitrophenyl)-8-[2-(4-morpholin-4-ylphenoxy)ethyl]-5,10-dihydro-11H-dibenzo[b,e][1,4]diazepin-11-one

The title compound was prepared by substituting Example 297A and Example 266G for Example 59B and Example 56A, respectively, in Example 59C. MS (ESI) m/e 567 (M+H)⁺; ¹H NMR (300 MHz, DMSO-d₆) δ 9.87 (s, 1H), 8.01 (d, J=8.5 Hz, 1H), 7.96 (s, 1H), 7.79 (d, J=8.1 Hz, 1H), 7.52 (d, J=1.7 Hz, 1H), 7.33-7.35 (m, 2H), 7.28 (dd, J=8.1, 1.7 Hz, 1H), 6.84-6.92, (m, 7H), 4.03 (m, 5H), 3.71 (m, 4H), 2.96 (m, 4H), 2.87 (t, J=6.6 Hz, 2H).

### Example 298

### 3-(4-chloro-3-methoxyphenyl)-8-[2-(4-morpholin-4-ylphenoxy)ethyl]-5,10-dihydro-11H-dibenzo[b,e][1,4]diazepin-11-one

The title compound was prepared by substituting Example 297A and Example 268A for Example 59B and Example 56A, respectively, in Example 59C. MS (ESI) m/e 556 and 558 (M+H)⁺; ¹H NMR (300 MHz, DMSO-d₆) δ 9.81 (s, 1H), 7.96 (s, 1H), 7.75 (d, J=8.1 Hz, 1H), 7.53 (d, J=8.1 Hz, 1H), 7.33 (d, J=2.0 Hz, 1H), 7.29 (d, J=1.7 Hz, 1H), 7.23 (dd, J=8.1, 1.7 Hz, 1H), 7.20 (dd, J=8.1, 2.0 Hz, 1H), 6.92, 6.84 (both m, total 7H), 4.05 (t, J=6.6 Hz, 2H), 3.96 (s, 3H), 3.71 (m, 4H), 3.03 (m, 4H), 2.87 (t, J=6.6 Hz, 2H).

### Example 299

### 2-methoxy-4-{8-[2-(4-morpholin-4-ylphenoxy)ethyl]-11-oxo-10,11-dihydro-5H-dibenzo[b,e] [1,4]diazepin-3-yl }benzonitrile

The title compound was prepared by substituting Example 297A and Example 267C for Example 59B and Example 56A, respectively, in Example 59C. MS (ESI) m/e 547 (M+H)⁺; ¹H NMR (300 MHz, DMSO-d₆) δ 9.87 (s, 1H), 7.95 (s, 1H), 7.84 (d, J=7.8 Hz, 1H), 7.79 (d, J=8.1 Hz, 1H), 7.42 (d, J=1.7 Hz, 1H), 7.32 (m, 2H), 7.28 (dd, J=8.1, 1.7 Hz, 1H), 6.93, 6.84 (both m, total 7H), 4.04 (t, J=6.8 Hz, 2H), 4.02 (s, 3H), 3.71 (m, 4H), 2.96 (m, 4H), 2.86 (t, J=6.8 Hz, 2H).

### Example 300

### 3-(3-methoxy-4-nitrophenyl)-8-{2-[(4-morpholin-4-ylphenyl)amino]ethyl }-5,10-dihydro-11H-dibenzo[b,e][1,4]diazepin-11-one

### Example 300A

### 2-(3-chloro-11-oxo-10,11-dihydro-5H-dibenzo[b,e][1,4]diazepin-8-yl)ethyl 4-methylbenzenesulfonate

Example 204A (2.0g, 6.9 mmol) and p-toluenesulfonyl chloride (1.7g, 8.9 mmol) were dissolved in dioxane (35 mL), then triethylamine (1.3 mL, 0.95g, 9.4 mmol) and 4-(dimethylamino)pyridine (0.09g, 0.7 mmol) were added. The reaction was stirred at room temperature for 3 days, then diluted with acetone and CHCl₃ and washed twice with saturated NaHCO₃. The organic layer was concentrated, then toluene was used to azeotrope off the last of the water. The crude material (2.8g) was slurried in Et₂O (25 mL) overnight, then filtered off to give the product (2.4g, 78%) as pale yellow solids. MS (DCI) m/e 443 and 445 (M+H)⁺; ¹H NMR (300 MHz, DMSO-d₆) δ 9.83 (s, 1H), 8.00 (s, 1H), 7.70 (d, J=8.5 Hz, 1H), 7.70 (d, J=8.1 Hz, 2H), 7.62 (d, J=8.1 Hz, 2H), 7.06 (d, J=2.0 Hz, 1H), 6.94 (dd, J=8.5, 2.0 Hz, 1H), 6.83 (m, 1H), 6.73 (m, 2H), 4.14 (t, J=6.4 Hz, 2H), 2.74 (t, J=6.4 Hz, 2H), 2.36 (s, 3H).

### Example 300B

### 3-chloro-8-{2-[(4-morpholin-4-ylphenyl)amino]ethyl}-5,10-dihydro-11H-dibenzo[b,e][1,4]diazepin-11-one

Example 300A (0.22g, 0.50 mmol) and 4-morpholinoaniline (0.09g, 0.50 mmol) were dissolved in DMF ( 2 mL), then added K₂CO₃ (0.13g, 0.96 mmol) and heated the reaction at 70° C for 24 hours. The reaction was cooled, partitioned between EtOAc and 0.5M NaHCO₃, then the organic layer was washed with brine and dried over Na₂SO₄.After filtration and concentration the crude material was purified by column chromatography using 1/1 then 3/7 hexane/EtOAc. Recovered the product (0.10g, 44%) as yellow solids. MS (DCI) m/e 449 and 451 (M+H)⁺; ¹H NMR (300 MHz, DMSO-d₆) δ 9.8 (s, 1H), 7.98 (s, 1H), 7.67 (d, J=8.5 Hz, 1H), 7.06 (d, J=2.0 Hz, 1H), 6.94 (dd, J=8.5, 2.0 Hz, 1H), 6.86-6.88, (m, 3H), 6.75 (d, J=8.8 Hz, 2H), 6.51 (d, J=8.8 Hz, 2H), 3.71 (m, 4H), 3.13 (m, 2H), 2.89 (m, 4H), 2.67 (m, 2H).

### Example 300C

### 3-(3-methoxy-4-nitrophenyl)-8-{2-[(4-morpholin-4-ylphenyl)amino]ethyl}-5,10-dihydro-11H-dibenzo[b,e][1,4]diazepin-11-one

The title compound was prepared by substituting Example 300B and Example 266G for Example 59B and Example 56A, respectively, in Example 59C. MS (ESI) m/e 566 (M+H)⁺.

### Example 301

### 3-(3-methoxy-4-nitrophenyl)-8-[2-(3-methyl-2-oxopyridin-1(2H)-yl)ethyl] -5,10-dihydro-11H-dibenzo[b,e][1,4]diazepin-11-one

Example 239 (100 mg, 0.25 mmol), PPh₃ (79 mg, 0.30 mmol), and 3-methyl-2-pyridol (33 mg, 0.30 mmole) were dissolved in DMF (1 mL), then di-tert-butylazodicarboxylate (68 mg, 0.30 mmol) was added and the reaction allowed to stir at room temperature overnight. The reaction was then diluted with MeOH (9 mL) and purified using preperative HPLC. The HPLC purification gave not only the title pyridone (27 mg, 22%), but also the pyridyl ether (24 mg, 21 %). MS (ESI) m/e 497 (M+H)⁺; ¹H NMR (300 MHz, DMSO-d₆) δ 9.87 (s, 1H), 8.01 (d, J=8.5 Hz, 1H), 7.97 (s, 1H), 7.80 (d, J=8.1 Hz, 1H), 7.52 (d, J=1.7 Hz, 1H), 7.25-7.39 (m, 5H), 6.93 (d, J=7.8 Hz, 1H), 6.82 (m, 2H), 6.05 (m, 1H), 4.03 (m, 5H), 2.80 (t, J=7.5 Hz, 2H), 2.00 (s, 3H).

### Example 302

### 3-(3-methoxy-4-nitrophenyl)-8-{2-[(5-methylpyridin-2-yl)oxy]ethyl}-5,10-dihydro-11H-dibenzo[b,e][1,4]diazepin-11-one

The title compound was isolated as second product in Example 351. MS (ESI) m/e 497 (M+H)⁺; ¹H NMR (300 MHz, DMSO-d₆) δ 9.86 (s, 1H), 8.01 (d, J=8.5 Hz, 1H), 7.96 (m, 2H), 7.79 (d, J=8.1 Hz, 1H), 7.52 (d, J=1.4 Hz, 1H), 7.50 (dd, J=8.5, 2.0 Hz, 1H), 7.28-7.34 (m, 3H), 6.95 (d, J=7.8 Hz, 1H), 6.90 (m, 2H), 6.69 (d, J=8.5 Hz, 1H), 4.35 (t, J=6.8 Hz, 2H), 4.03 (s, 3H), 2.80 (t, J=6.8 Hz, 2H), 2.19 (s, 3H).

### Example 303

### 3-(3-methoxy-4-nitrophenyl)-8-[2-(4-methyl-2-oxopyridin-1(2H)-yl)ethyl]-5,10-dihydro-11H-dibenzo[b,e][1,4]diazepin-11-one

The title compound was prepared by substituting 4-methyl-2-pyridol for 3-methylpyridin-2(1H)-one in Example 301. MS (ESI) m/e 497 (M+H)⁺; ¹H NMR (300 MHz, DMSO-d₆) δ 9.86 (s, 1H), 8.01 (d, J=8.5 Hz, 1H), 7.97 (s, 1H), 7.80 (d, J=8.1 Hz, 1H), 7.52 (d, J=1.7 Hz, 1H), 7.39 (d, J=7.1 Hz, 1H), 7.28-7.34 (m, 3H), 6.93 (d, J=8.1 Hz, 1H), 6.83 (m, 1H), 6.78 (m, 1H), 6.18 (m, 1H), 5.98 (dd, J=6.9, 1.9 Hz, 1H), 4.03 (s, 3H), 3.97 (t, J=7.5 Hz, 2H), 2.78 (t, J=7.5 Hz, 2H), 2.09 (s, 3H).

### Example 304

### 8-[2-(isoquinolin-3-yloxy)ethyl]-3-(3-methoxy-4-nitrophenyl)-5,10-dihydro-11H-dibenzo[b,e][1,4]diazepin-11-one

The title compound was prepared by substituting 3-hydroxyisoquinoline for 3-methylpyridin-2(1H)-one in Example 301. MS (ESI) m/e 533 (M+H)⁺; ¹H NMR (300 MHz, DMSO-d₆) δ 9.88 (s, 1H), 9.04 (s, 1H), 8.01 (d, J=8.5 Hz, 2H), 7.97 (s, 1H), 7.80 (d, J=8.1 Hz, 2H), 7.65 (m, 1H), 7.52 (d, J=1.7 Hz, 1H), 7.42 (m, 1H), 7.28-7.35 (m, 3H), 7.16 (s, 1H), 6.96 (m, 3H), 4.47 (t, J=6.8 Hz, 2H), 4.03 (s, 3H), 2.96 (t, J=6.8 Hz, 2H).

### Example 305

### 8-[2-(5-chloro-2-oxopyridin-1(2H)-yl)ethyl]-3-(3-methoxy-4-nitrophenyl)-5,10-dihydro-11H-dibenzo[b,e][1,4]diazepin-11-one

The title compound was prepared by substituting 5-chloro-2-pyridol for 3-methylpyridin-2(1H)-one in Example 301. MS (ESI) m/e 517 and 519 (M+H)⁺; ¹H NMR (300 MHz, DMSO-d₆) δ 9.88 (s, 1H), 8.01 (d, J=8.5 Hz, 1H), 7.98 (s, 1H), 7.85 (d, J=2.7 Hz, 1H), 7.80 (d, J=8.1 Hz, 1H), 7.52 (d, J=1.7 Hz, 1H), 7.45 (dd, J=9.8, 2.7 Hz, 1H), 7.28-7.34 (m, 3H), 6.94 (d, J=8.1 Hz, 1H), 6.82 (m, 2H), 6.42 (d, J=9.8 Hz, 1H), 4.03 (s, 3H), 4.00 (t, J=7.5 Hz, 2H), 2.80 (t, J=7.5 Hz, 2H).

### Example 306

### 8-[1,1-dimethyl-2-(pyridin-2-yloxy)ethyl]-3-(3-methoxy-4-nitrophenyl)-5,10-dihydro-11H-dibenzo[b,e][1,4]diazepin-11-one

### Example 306A

### 3-chloro-8-[1,1-dimethyl-2-(pyridin-2-yloxy)ethyl]-5,10-dihydro-11H-dibenzo[b,e][1,4]diazepin-11-one

Example 288A (160 mg, 0.51 mmol) and 2-fluoropyridine (43 µL, 48 mg, 0.50 mmol) were dissolved in DMF (1.2 mL), then 95% NaH (28 mg, 1.11 mmol) was added. After stirring the reaction at room temperature for 4 hours water and EtOAc were added, then the organic layer was washed with brine and dried over Na₂SO₄. After filtration and concentration the crude material was purified by column chromatography using 7/3 hexane/EtOAc, giving the product (47 mg, 24%). MS (DCI) m/e 394 and 396 (M+H)⁺.

### Example 306B

### 8-[1,1-dimethyl-2-(pyridin-2-yloxy)ethyl]-3-(3-methoxy-4-nitrophenyl)-5,10-dihydro-11H-dibenzo[b,e][1,4]diazepin-11-one

The title compound was prepared by substituting Example 306A and Example 266G for Example 59B and Example 56A, respectively, in Example 59C. MS (ESI) m/e 511 (M+H)⁺; ¹H NMR (300 MHz, DMSO-d₆) δ 9.81 (s, 1H), 8.11 (m, 1H), 8.01 (d, J=8.5 Hz, 1H), 7.97 (s, 1H), 7.79 (d, J=8.1 Hz, 1H), 7.65 (m, 1H), 7.52 (d, J=1.7 Hz, 1H), 7.33-7.35 (m, 2H), 7.29 (dd, J=8.3, 1.7 Hz, 1H), 7.10 (d, J=2.0 Hz, 1H), 7.06 (m, 1H), 6.93-6.96 (m, 2H), 6.75 (d, J=8.5 Hz, 1H), 4.22 (s, 2H), 4.03 (s, 3H), 1.32 (s, 6H).

### Example 307

### 8-[1,1-dimethyl-2-(4-morpholin-4-ylphenoxy)ethyl]-3-(3-methoxy-4-nitrophenyl)-5,10-dihydro-11H-dibenzo[b,e][1,4]diazepin-11-one

### Example 307A

### 3-chloro-8-[1,1-dimethyl-2-(4-nitrophenoxy)ethyl]-5,10-dihydro-11H-dibenzo[b,e][1,4]diazepin-11-one

The title compound was prepared by substituting 4-fluoro-nitrobenzene for 2-fluoropyridine in Example 306A. Starting with the compound described in Example 288A and 4-fluoro-nitrobenzene the title compound was prepared using the method of Example 306A. MS (DCI) m/e 438 and 440 (M+H)⁺, 455 and 457 (M+NH₄)⁺; ¹H NMR (300 MHz, DMSO-d₆) δ 9.81 (s, 1H), 8.17 (m, 2H), 8.02 (s, 1H), 7.67 (d, J=8.5 Hz, 1H), 7.13 (m, 2H), 7.05-7.09 (m, 3H), 6.92, 6.90 (m, 2H), 4.08 (s, 2H), 1,34 (s, 6H).

### Example 307B

### 8-[2-(4-aminophenoxy)-1,1-dimethylethyl]-3-chloro-5,10-dihydro-11H-dibenzo[b,e][1,4]diazepin-11-one

The title compound was prepared by substituting Example 307A for Example 6B in Example 6C. MS (DCI) m/e 408 and 410 (M+H)⁺; ¹H NMR (300 MHz, DMSO-d₆) δ 9.79 (s, 1H), 8.00 (s, 1H), 7.67 (d, J=8.5 Hz, 1H), 7.02-7.06 (m, 3H), 6.90 (m, 2H), 6.60 (m, 2H), 6.46 (m, 2H), 4.56 (s, 2H), 3.75 (s, 2H), 1.28 (s, 6H).

### Example 307C

### 3-chloro-8-[1,1-dimethyl-2-(4-morpholin-4-ylphenoxy)ethyl]-5,10-dihydro-11H-dibenzo[b,e][1,4]diazepin-11-one

Example 307B (0.57g, 1.4 mmol) was dissolved in DMA (2.8 mL), then 2-chloroethyl ether (0.40g, 2.8 mmol) and K₂CO₃ (0.39g, 2.8 mmol) were added and the reaction heated at 150° C for 3 hours. Water and EtOAc were added, then the organic layer was washed with brine and dried over Na₂SO₄. After filtration and concentration the crude material was purified by column chromatography using 6/4 hexane/EtOAc, giving the product (0.33g, 49%). MS (ESI) m/e 478 and 480 (M+H)⁺.

### Example 307D

### 8-[1,1-dimethyl-2-(4-morpholin-4-ylphenoxy)ethyl]-3-(3-methoxy-4-nitrophenyl)-5,10-dihydro-11H-dibenzo[b,e][1,4]diazepin-11-one

The title compound was prepared by substituting Example 307C and Example 266G for Example 59B and Example 56A, respectively, in Example 59C.MS (ESI) m/e 595 (M+H)⁺; ¹H NMR (300 MHz, DMSO-d₆) δ 9.87 (s, 1H), 8.00 (d, J=8.4 Hz, 1H), 7.95 (s, 1H), 7.80 (d, J=8.1 Hz, 1H), 7.52 (d, J=1.7 Hz, 1H), 7.34 (m, 2H), 7.29 (dd, J=8.4, 1.7 Hz, 1H), 7.09 (d, J=2.2 Hz, 1H), 7.04 (dd, J=8.4, 2.2 Hz, 1H), 6.96 (d, J=8.4 Hz, 1H), 6.89 (d, J=9.0 Hz, 2H), 6.82 (d, J=9.0 Hz, 2H), 4.03 (s, 3H), 3.85 (s, 2H), 3.72 (m, 4H), 3.00 (m, 4H), 1.31 (s, 6H).

### Example 308

### 2-[3-(3-methoxy-4-nitrophenyl)-11-oxo-10,11-dihydro-5H-dibenzo[b,e][1,4]diazepin-8-yl]-2-methylN(tetrahydrofuran-2-ylmethyl)propanamide

The title compound was prepared by substituting Example 266I and (±)-C-(tetrahydro-furan-2-yl)-methylamine for dimethylaminoacetic acid and Example 120, respectively, in Example122. MS (DCI) m/e 531 (M+H)⁺; ¹H NMR (500 MHz, DMSO-d₆) δ 9.83 (s, 1H), 8.01 (d, J=8.4 Hz, 1H), 7.97 (s, 1H), 7.80 (d, J=8.1 Hz, 1H), 7.52 (d, J=1.6 Hz, 1H), 7.30-7.35 (m, 3H), 7.07 (t, J=5.6 Hz, 1H), 6.95 (m, 2H), 6.90 (dd, J=8.3, 2.0 Hz, 1H), 4.03 (s, 3H), 3.80 (m, 1H), 3.60 (m, 1H), 3.53 (m, 1H), 3.07 (m, 2H), 1.61 (m, 3H), 1.40 (m, 1H), 1.38 (s, 6H).

### Example 309

### 8-(2-hydroxy-1,1-dimethylethyl)-3-(3-methoxy-4-nitrophenyl)-7-methyl-5,10-dihydro-11H-dibenzo[b,e][1,4]diazepin-11-one

The title compound was prepared from 3-methyl-nitrobenzene by the sequential application of the following methods: Examples 282A and 6C, treatment with acetic anhydride, Examples 281C, 1A, 6C, 5C, 286C, and 266H. Instead of the Et₂O slurry described in Example 266H, the title compound was purified by column chromatography using 4/6 hexane/EtOAc, then an EtOAc slurry. MS (DCI) m/e 448 (M+H)⁺; ¹H NMR (300 MHz, DMSO-d₆) δ 9.69 (s, 1H), 8.00 (d, J=8.4 Hz, 1H), 7.83 (s, 1H), 7.79 (d, J=8.1 Hz, 1H), 7.52 (s, 1H), 7.34 (m, 2H), 7.27 (d, J=8.1 Hz, 1H), 6.97 (s, 1H), 6.73 (s, 1H), 4.57 (t, J=5.5 Hz, 1H), 4.03 (s, 3H), 3.50 (d, J=5.5 Hz, 2H), 2.34 (s, 3H), 1.24 (s, 6H).

### Example 310

### 2-[3-(3-methoxy-4-nitrophenyl)-2-methyl-11-oxo-10,11-dihydro-5H-dibenzo[b,e] [1,4]diazepin-8-yl]N(4-morpholin-4-ylphenyl)acetamide

### Example 310A

### 5-chloro-2-iodo-4-methylaniline

3-Chloro-4-methylaniline (1.50g, 10.6 mmol) and benzyltrimethylammonium dichloroiodate (4.60g, 13.2 mmol) were dissolved in CH₂Cl₂ (50 mL) and MeOH (20 mL), then CaCO₃ (3.10g, 31.0 mmol) was added and the reaction stirred at room temperature overnight. The reaction was diluted with Et₂O (200 mL), filtered through Celite^{®}, and concentrated. The crude material was purified by column chromatography using 97.5/2.5 hexane/EtOAc, giving the product (1.80g, 63%) as light tan solids. MS (DCI) m/e 268 and 270 (M+H)⁺; ¹H NMR (300 MHz, CDCl₃) δ 7.4 (s, 1H), 6.76 (s, 1H), 4.00 (v br s, 2H), 2.22 (s, 3H).

### Example 310B

### methyl 2-(acetylamino)-4-chloro-5-methylbenzoate

The compound described in 310A (1.65g, 6.17 mmol) was dissolved in 1,2-dichloroethane (30 mL), then acetyl chloride (0.60 mL, 0.66g, 8.41 mmol) was added. The reaction was stirred at room temperature for 3 hours, then concentrated to give the acetamide (1.91g, 100%), which was carried on with no purification.

That acetamide was dissolved in DMF (12 mL) and MeOH (2.4 mL), then triethylamine (1.80 mL, 1.31g, 1.30 mmol), 1,1'-bis(diphenylphosphino)ferrocene (300 mg, 0.54 mmol), and palladium(II)acetate (60 mg, 0.27 mmol) were added. The reaction was heated at 70° C under a CO balloon overnight. The reaction was then cooled and poured into ice water. The solids wre filtered off, dried, and purified by column chromatogrphy using 9/1, then 4/1 hexane/EtOAc. The product (1.36g, 91%) was recovered as off-white solids. MS (DCI) m/e 242 and 244 (M+H)⁺; ¹H NMR (300 MHz, CDCl₃) δ 8.79 (s, 1H), 7.87 (s, 1H), 3.92 (s, 3H), 2.34 (s, 3H), 2.22 (s, 3H).

### Example 310C

### methyl 4-chloro-2-iodo-5-methylbenzoate

The compound described in Example 310B was converted to the title compound by the methods found in the second paragraph of Example 281C and Example 57B. MS (DCI) m/e 328 and 330 (M+H)⁺.

### Example 310D

### 2-[3-(3-methoxy-4-nitrophenyl)-2-methyl-11-oxo-10,11-dihydro-5H-dibenzo[b,e][1,4]diazepin-8-yl]N(4-morpholin-4-ylphenyl)acetamide

Starting with the compounds described in Examples 6A and 310C, the title compound was prepared by the methods described in Examples 1A, 6C, 5C, 266H, 13, and 266J. The title compound was purified by preparative HPLC. MS (ESI) m/e 594 (M+H)⁺; ¹H NMR (300 MHz, DMSO-d₆) δ 9.88 (s, 1H), 9.85 (s, 1H), 7.95 (d, J=8.1 Hz, 1H), 7.77 (s, 1H), 7.61 (s, 1H), 7.45 (d, J=8.7 Hz, 2H), 7.30 (d, J=1.6 Hz, 1H), 7.06 (dd, J=8.4, 1.6 Hz, 1H), 6.91 (m, 6H), 3.95 (s, 3H), 3.73 (m, 4H), 3.46 (s, 2H), 3.05 (m, 4H), 2.13 (s, 3H).

### Example 311

### methyl {3-[4-(aminomethyl)-3-methoxyphenyl]-11-oxo-10,11-dihydro-5H-dibenzo[b,e][1,4]diazepin-8-yl} acetate

Example 61 (31 mg, 0.075 mmol) was dissolved in MeOH (50 mL) and triethylamine (5 mL), then Raney nickel (100 mg) was added and the reaction stirred under hydrogen at 60 psi overnight. The reaction was filtered, concentrated, and the crude material purified by column chromatography to give 18 mg (45%) of the product. MS (DCI) m/e 418 (M+H)⁺; ¹H NMR (300 MHz, DMSO-d₆) δ 9.85 (s, 1H), 8.04 (v br s, 3H), 7.96 (s, 1H), 7.77 (d, J=8.5 Hz, 1H), 7.46 (d, J=7.8 Hz, 1H), 7.32 (d, J=1.7 Hz, 1H), 7.28, 7.25, 7.23 (all m, total 3H), 6.96 (d, J=8.3 Hz, 1H), 6.87, 6.84 (both m, total 2H), 4.03 (br s, 2H), 3.94 (s, 3H), 3.60 (s, 3H), 3.54 (s, 2H).

### Example 312

### 2-[3-(2-methoxy-5-methylpyridin-4-yl)-11-oxo-10,11-dihydro-5H-dibenzo[b,e][1,4]diazepin-8-yl]-2-methylN(4-morpholin-4-ylphenyl)propanamide

Starting with the compounds described in Examples 71C and 266F the title compound was made using the methods described in Examples 266H, 72 (except no TMSCHN₂ treatment), and 266J. The final compound was purified by column chromatography using EtOAc, then converted to the dihydrochloride salt. MS (ESI) m/e 578 (M+H)⁺; ¹H NMR (300 MHz, DMSO-d₆) δ 9.87 (s, 1H), 8.94 (br s, 1H), 8.09 (m, 1H), 7.97 (s, 1H), 7.74 (d, J=8.1 Hz, 1H), 7.51 (d, J=8.8 Hz, 2H), 6.89-7.08 (m, 7H), 6.66 (s, 1H), 3.85 (s, 3H), 3.91 (m, 4H), 3.16 (br m, 4H), 2.13 (s, 3H), 1.49 (s, 6H).

### Example 313

### 8-(2-hydroxy-1,1-dimethylethyl)-3-[(2-methylpyridin-4-yl)amino]-5,10-dihydro-11H-dibenzo[b,e][1,4]diazepin-11-one

The title compound was prepared by substituting Example 288A and 4-amino-2-picoline for Example 189A and 4-aminopyridine in Example 191. MS (DCI) m/e 389 (M+H)⁺; ¹H NMR (300 MHz, DMSO-d₆) δ 10.4 (s, 1H), 9.69 (s, 1H), 8.25 (d, J=7.5 Hz, 1H), 7.97 (s, 1H), 7.76 (d, J=8.5 Hz, 1H), 7.07 (m, 2H), 6.88 (m, 4H), 6.81 (dd, J=8.5, 2.0, 1H), 4.60 (v br s, 1H), 3.34 (s, 2H), 2.50 (s, 3H), 1.15 (s, 6H).

### Example 314 (A799661.2)

### 8-(2-hydroxy-1,1,2-trimethylpropyl)-3-(pyrimidin-4-ylamino)-5,10-dihydro-11H-dibenzo[b,e][1,4]diazepin-11-one

The title compound was prepared by substituting Example 289A and pyrimidin-4-ylamine for Example 189A and 4-aminopyridine in Example 191. MS (DCI) m/e 404 (M+H)⁺; ¹H NMR (300 MHz, DMSO-d₆) δ 10.48 (s, 1H), 9.58 (s, 1H), 8.85 (s, 1H), 8.38 (d, J=6.4 Hz, 1H), 7.91 (s, 1H), 7.69 (d, J=8.5 Hz, 1H), 7.39 (d, J=2.0 Hz, 1H), 7.11 (dd, J=8.5, 2.0 Hz, 1H), 7.06 (d, J=2.0 Hz, 1H), 6.99 (m, 2H), 6.87 (d, J=8.5 Hz, 1H), 1.24 (s, 6H), 0.96 (s, 6H).

### Example 315

### 2-[3-(3-methoxy-4-nitrophenyl)-11-oxo-10,11-dihydro-5H-dibenzo[b,e][1,4]diazepin-8-yl]NmethylN(3-pyrrolidin-1-ylpropyl)acetamide

The title compound was prepared by substituting Example 73 and methyl(3-pyrrolidin-1-ylpropyl)amine (B.R. de Costa, et. al., J. Med. Chem., 33, 38 (1992)) for dimethylaminoacetic acid and Example 120, respectively, in Example 122. MS (DCI) m/e 544 (M+H)⁺.

### Example 316

### 2-[3-(3-methoxy-4-nitrophenyl)-11-oxo-10,11-dihydro-5H-dibenzo[b,e][1,4]diazepin-8-yl]-2-methylN(3-pyrrolidin-1-ylpropyl)propanamide

The title compound was prepared by substituting Example 266I and (3-pyrrolidin-1-yl-propyl)amine for dimethylaminoacetic acid and Example 120, respectively, in Example 122. MS (DCI) m/e 558 (M+H)⁺.

### Example 317

### 2-[3-(3-methoxy-4-nitrophenyl)-11-oxo-10,11-dihydro-5H-dibenzo[b,e][1,4]diazepin-8-yl]-2-methylNpyrimidin-4-ylpropanamide

The title compound was prepared by substituting Example 266I and 4-aminopyrimidine for dimethylaminoacetic acid and Example 120, respectively, in Example 122. MS (ESI) m/e 525 (M+H)⁺.

### Example 318

### 2-[3-(3-methoxy-4-nitrophenyl)-11-oxo-10,11-dihydro-5H-dibenzo[b,e] [1,4]diazepin-8-yl]-2-methylN(4-methyl-1,3-thiazol-2-yl)propanamide

The title compound was prepared by substituting Example 266I and 2-amino-4-methylthiazole for dimethylaminoacetic acid and Example 120, respectively, in Example 122. MS (ESI) m/e 543 (M+H)⁺.
Examples 319 through 335 were made by the method used for Examples 80-118, substituting the acid described in Example 266I for the acid described in Example 73.

### Example 319

### 2-[3-(3-methoxy-4-nitrophenyl)-11-oxo-10,11-dihydro-5H-dibenzo[b,e][1,4]diazepin-8-yl]-2-methylN(2,2,2-trifluoroethyl)propanamide

The desired product was prepared using 2,2,2-trifluoroethylamine. MS (ESI) m/e 529 (M+H)⁺.

### Example 320

### N-(3-fluorophenyl)-2-[3-(3-methoxy-4-nitrophenyl)-11-oxo-10,11-dihydro-5H-dibenzo[b,e] [1,4]diazepin-8-yl]-2-methylpropanamide

The desired product was prepared using 3-fluoroaniline. MS (ESI) m/e 539 (M-H)⁻.

### Example 321

### 2-[3-(3-methoxy-4-nitrophenyl)-11-oxo-10,11-dihydro-5H-dibenzo[b,e][1,4]diazepin-8-yl]-2-methylN[4-(trifluoromethoxy)phenyl]propanamide

The desired product was prepared using 4-(trifluoromethoxy)aniline. MS (ESI) m/e 605 (M-H)⁻.

### Example 322

### 2-[3-(3-methoxy-4-nitrophenyl)-11-oxo-10,11-dihydro-5H-dibenzo[b,e][1,4]diazepin-8-yl]-2-methylN[3-(trifluoromethyl)phenyl]propanamide

The desired product was prepared using 3-(trifluoromethyl)aniline. MS (ESI) m/e 591 (M+H)⁺.

### Example 323

### 2-[3-(3-methoxy-4-nitrophenyl)-11-oxo-10,11-dihydro-5H-dibenzo[b,e][1,4]diazepin-8-yl]-2-methylN[3-(trifluoromethoxy)phenyl]propanamide

The desired product was prepared using 3-(trifluoromethoxy)aniline. MS (ESI) m/e 607 (M+H)⁺.

### Example 324

### N-[3-fluoro-5-(trifluoromethyl)benzyl]-2-[3-(3-methoxy-4-nitrophenyl)-11-oxo-10,11-dihydro-5H-dibenzo[b,e][1,4]diazepin-8-yl]-2-methylpropanamide

The desired product was prepared using (3-fluoro-5-trifluoromethyl)benzylamine. MS (ESI) m/e 621 (M-H)⁻.

### Example 325

### N-(2-fluorobenzyl)-2-[3-(3-methoxy-4-nitrophenyl)-11-oxo-10,11-dihydro-5H-dibenzo[b,e] [1,4]diazepin-8-yl]-2-methylpropanamide

The desired product was prepared using 2-fluorobenzylamine. MS (ESI) m/e 553 (M-H)⁻.

### Example 326

### N-(3-fluorobenzyl)-2-[3-(3-methoxy-4-nitrophenyl)-11-oxo-10,11-dihydro-5H-dibenzo[b,e][1,4]diazepin-8-yl]-2-methylpropanamide

The desired product was prepared using 3-fluorobenzylamine. MS (ESI) m/e 553 (M-H)⁻.

### Example 327

### N-(4-fluorobenzyl)-2-[3-(3-methoxy-4-nitrophenyl)-11-oxo-10,11-dihydro-5H-dibenzo[b,e] [1,4]diazepin-8-yl]-2-methylpropanamide

The desired product was prepared using 4-fluorobenzylamine. MS (ESI) m/e 553 (M-H)⁻.

### Example 328

### 2-[3-(3-methoxy-4-nitrophenyl)-11-oxo-10,11-dihydro-5H-dibenzo[b,e][1,4]diazepin-8-yl]-2-methylN[4-(trifluoromethoxy)benzyl]propanamide

The desired product was prepared using 4-(trifluoromethoxy)benzylamine. MS (ESI) m/e 621 (M+H)⁺.

### Example 329

### 2-[3-(3-methoxy-4-nitrophenyl)-11-oxo-10,11-dihydro-5H-dibenzo[b,e][1,4]diazepin-8-yl]-2-methylN[3-(trifluoromethyl)benzyl]propanamide

The desired product was prepared using 3-(trifluoromethyl)benzylamine. MS (ESI) m/e 603 (M-H)⁻.

### Example 330

### 2-[3-(3-methoxy-4-nitrophenyl)-11-oxo-10,11-dihydro-5H-dibenzo[b,e][1,4]diazepin-8-yl]-2-methylN[4-(trifluoromethyl)benzyl]propanamide

The desired product was prepared using 4-(trifluoromethyl)benzylamine. MS (ESI) m/e 603 (M-H)⁻.

### Example 331

### 2-[3-(3-methoxy-4-nitrophenyl)-11-oxo-10,11-dihydro-5H-dibenzo[b,e][1,4]diazepin-8-yl]-2-methylN[3-(trifluoromethoxy)benzyl]propanamide

The desired product was prepared using 3-(trifluoromethoxy)benzylamine. MS (ESI) m/e 619 (M-H)⁻.

### Example 332

### N-[2-(2-fluorophenyl)ethyl]-2-[3-(3-methoxy-4-nitrophenyl)-11-oxo-10,11-dihydro-5H-dibenzo[b,e][1,4]diazepin-8-yl]-2-methylpropanamide

The desired product was prepared using 2-(2-fluorophenyl)ethylamine. MS (ESI) m/e 567 (M-H)⁻.

### Example 333

### N-[2-(3-fluorophenyl)ethyl]-2-[3-(3-methoxy-4-nitrophenyl)-11-oxo-10,11-dihydro-5H-dibenzo[b,e] [1,4]diazepin-8-yl]-2-methylpropanamide

The desired product was prepared using 2-(3-fluorophenyl)ethylamine. MS (ESI) m/e 567 (M-H)⁻.

### Example 334

### N-(2,4-difluorobenzyl)-2-[3-(3-methoxy-4-nitrophenyl)-11-oxo-10,11-dihydro-5H-dibenzo[b,e][1,4]diazepin-8-yl]-2-methylpropanamide

The desired product was prepared using 2,4-difluorobenzylamine. MS (ESI) m/e 571 (M-H)⁻.

### Example 335

### N-(2, 6-difluorobenzyl)-2-[3-(3-methoxy-4-nitrophenyl)-11-oxo-10,11-dihydro-5H-dibenzo[b,e] [1,4]diazepin-8-yl]-2-methylpropanamide

The desired product was prepared using 2,6-difluorobenzylamine. MS (ESI) m/e 571 (M-H)⁻.

### Example 336

### N-(cyclopropylmethyl)-2-[3-(3-methoxy-4-nitrophenyl)-11-oxo-10,11-dihydro-5H-dibenzo[b,e][1,4]diazepin-8-yl]-2-methylpropanamide

The title compound was prepared by substituting Example 266I and cyclopropylmethylamine for dimethylaminoacetic acid and Example 120, respectively, in Example 122. MS (DCI) m/e 501 (M+H)⁺.

### Example 337

### N-(3-ethoxypropyl)-2-methyl-2-[11-oxo-3-(pyrimidin-4-ylamino)-10,11-dihydro-5H-dibenzo[b,e] [1,4]diazepin-8-yl]propanamide

### Example 337A

### 2-methyl-2-[11-oxo-3-(pyrimidin-4-ylamino)-10,11-dihydro-5H-dibenzo[b,e][1,4]diazepin-8-yl]propanoic acid

The title compound was prepared by substituting Example 279A for Example 12 in Example 13. MS (DCI) m/e 390 (M+H)⁺.

### Example 337B

### N-(3-ethoxypropyl)-2-methyl-2-[11-oxo-3-(pyrimidin-4-ylamino)-10,11-dihydro-5H-dibenzo[b,e][1,4]diazepin-8-yl]propanamide

The title compound was prepared by substituting Example 337A and 3-ethoxypropylamine for dimethylaminoacetic acid and Example 120, respectively, in Example 122. MS (DCI) m/e 475 (M+H)⁺.

### Example 338

### N-(3,4-difluorobenzyl)-2-methyl-2-[11-oxo-3-(pyrimidin-4-ylamino)-10,11-dihydro-5H-dibenzo[b,e][1,4]diazepin-8-yl]propanamide

The title compound was prepared by substituting Example 337A and (3,4-difluoro)benzylamine for dimethylaminoacetic acid and Example 120, respectively, in Example 122. MS (DCI) m/e 514 (M+H)⁺.

### Example 339

### 8-[1,1-dimethyl-2-oxo-2-(4-phenylpiperazin-1-yl)ethyl]-3-(pyrimidin-4-ylamino)-5,10-dihydro-11H-dibenzo[b,e][1,4]diazepin-11-one

The title compound was prepared by substituting Example 337A and (N-phenyl)piperazine for dimethylaminoacetic acid and Example 120, respectively, in Example 122. MS (DCI) m/e 533 (M+H)⁺.

### Example 340

### N-cyclopentyl-2-methyl-2-[11-oxo-3-(pyrimidin-4-ylamino)-10,11-dihydro-5H-dibenzo[b,e] [1,4]diazepin-8-yl]propanamide

The title compound was prepared by substituting Example 337A and cyclopentylamine for dimethylaminoacetic acid and Example 120, respectively, in Example 122. MS (DCI) m/e 457 (M+H)⁺.

### Example 341

### 8-(1,1-dimethyl-2-morpholin-4-yl-2-oxoethyl)-3-(pyrimidin-4-ylamino)-5,10-dihydro-11H-dibenzo[b,e][1,4]diazepin-11-one

The title compound was prepared by substituting Example 337A and morpholine for dimethylaminoacetic acid and Example 120, respectively, in Example 122. MS (DCI) m/e 459 (M+H)⁺.

### Example 342

### 2-methyl-2-[11-oxo-3-(pyrimidin-4-ylamino)-10,11-dihydro-5H-dibenzo[b,e][1,4]diazepin-8-yl]N(tetrahydrofuran-3-ylmethyl)propanamide

The title compound was prepared by substituting Example 337A and (±)-3-aminomethyltetrahydrofuran for dimethylaminoacetic acid and Example 120, respectively, in Example 122. MS (DCI) m/e 473 (M+H)⁺.

### Example 343

### N-(cyclopentylmethyl)-2-methyl-2-[11-oxo-3-(pyrimidin-4-ylamino)-10,11-dihydro-5H-dibenzo[b,e][1,4]diazepin-8-yl]propanamide

The title compound was prepared by substituting Example 337A and cyclopentylmethylamine for dimethylaminoacetic acid and Example 120, respectively, in Example 122. MS (DCI) m/e 471 (M+H)⁺.

### Example 344

### N-(cyclopropylmethyl)-2-methyl-2-[11-oxo-3-(pyrimidin-4-ylamino)-10,11-dihydro-5H-dibenzo[b,e][1,4]diazepin-8-yl]propanamide

The title compound was prepared by substituting Example 337A and cyclopropylmethylamine for dimethylaminoacetic acid and Example 120, respectively, in Example 122. MS (DCI) m/e 443 (M+H)⁺.

### Example 345

### 2-methyl-2-[11-oxo-3-(pyrimidin-4-ylamino)-10,11-dihydro-5H-dibenzo[b,e][1,4]diazepin-8-yl]N(tetrahydrofuran-2-ylmethyl)propanamide

The title compound was prepared by substituting Example 337A and tetrahydrofuran-2-ylmethylamine for dimethylaminoacetic acid and Example 120, respectively, in Example 122. MS (DCI) m/e 473 (M+H)⁺.

### Example 346

### 2-[3-(3-methoxy-4-nitrophenyl)-11-oxo-10,11-dihydro-5H-dibenzo[b,e][1,4]diazepin-7-yl]-2-methylN1,3-thiazol-2-ylpropanamide

The title compound was prepared by substituting Example 282D and 2-aminothiazole for dimethylaminoacetic acid and Example 120, respectively, in Example 122. MS (ESI) m/e 530 (M+H)⁺.

### Example 347

### N-(2,5-difluorobenzyl)-2-[3-(3-methoxy-4-nitrophenyl)-11-oxo-10,11-dihydro-5H-dibenzo[b,e][1,4]diazepin-7-yl]-2-methylpropanamide

The title compound was prepared by substituting Example 282D and (2,5-difluoro)benzylamine for dimethylaminoacetic acid and Example 120, respectively, in Example 122. MS (ESI) m/e 573 (M+H)⁺.

### Example 348

### N-(2-ethoxyethyl)-2-[3-(3-methoxy-4-nitrophenyl)-11-oxo-10,11-dihydro-5H-dibenzo [b,e][1,4]diazepin-7-yl]-2-methylpropanamide

The title compound was prepared by substituting Example 282D and 2-ethoxyethylamine for dimethylaminoacetic acid and Example 120, respectively, in Example 122. MS (ESI) m/e 533 (M+H)⁺.

### Example 349

### N-(4-fluorophenyl)-2-[3-(3-methoxy-4-nitrophenyl)-11-oxo-10,11-dihydro-5H-dibenzo[b,e][1,4]diazepin-7-yl]-2-methylpropanamide

The title compound was prepared by substituting Example 282D and 4-fluoroaniline for dimethylaminoacetic acid and Example 120, respectively, in Example 122. MS (ESI) m/e 541 (M+H)⁺.

### Example 350

### 3-(3-methoxy-4-nitrophenyl)-8-[2-(quinolin-2-yloxy)ethyl]-5,10-dihydro-11H-dibenzo[b,e][1,4]diazepin-11-one

The title compound was-prepared by substituting quinolin-2(1H)-one for 3-methylpyridin-2(1H)-one in Example 301. MS (ESI) m/e 533 (M+H)⁺.

### Example 351

### 3-(3-methoxy-4-nitrophenyl)-8-[2-(5-methyl-2-oxopyridin-1(2H)-yl)ethyl]-5,10-dihydro-11H-dibenzo[b,e][1,4]diazepin-11-one

The title compound was prepared by substituting 5-methylpyridin-2(1H)-one for 3-methylpyridin-2(1H)-one in Example 301. MS (ESI) m/e 497 (M+H)⁺.

### Example 352

### 2-{2-[3-(3-methoxy-4-nitrophenyl)-11-oxo-10,11-dihydro-5H-dibenzo[b,e][1,4]diazepin-8-yl]ethoxy}-6-methylnicotinonitrile

The title compound was prepared by substituting 6-methyl-2-oxo-1,2-dihydropyridine-3-carbonitrile for 3-methylpyridin-2(1H)-one in Example 301. MS (ESI) m/e 522 (M+H)⁺.

### Example 353

### 3-(3-methoxy-4-nitrophenyl)-8-[2-(1-oxoisoquinolin-2(1H)-yl)ethyl]-5,10-dihydro-11H-dibenzo[b,e][1,4]diazepin-11-one

The title compound was prepared by substituting isoquinolin-1(2H)-one for 3-methylpyridin-2(1H)-one in Example 301. MS (ESI) m/e 533 (M+H)⁺.

### Example 354

### 3-(3-methoxy-4-nitrophenyl)-8-{2-[2-oxo-5-(trifluoromethyl)pyridin-1(2H)-yl]ethyl}-5,10-dihydro-11H-dibenzo[b,e][1,4]diazepin-11-one

The title compound was prepared by substituting 5-(trifluoromethyl)pyridin-2(1H)-one for 3-methylpyridin-2(1H)-one in Example 301. MS (ESI) m/e 551 (M+H)⁺.

### Example 355

### 3-(3-methoxy-4-nitrophenyl)-8-[2-(3-methoxy-2-oxopyridin-1(2H)-yl)ethyl]-5,10-dihydro-11H-dibenzo[b,e][1,4]diazepin-11-one

The title compound was prepared by substituting 3-methoxypyridin-2(1H)-one for 3-methylpyridin-2(1H)-one in Example 301. MS (ESI) m/e 513 (M+H)⁺.

### Example 356

### 3-(3-methoxy-4-nitrophenyl)-8-{2-[(4-methylpyridin-2-yl)oxy]ethyl}-5,10-dihydro-11H-dibenzo[b,e][1,4]diazepin-11-one

The title compound was prepared by substituting 4-methylpyridin-2(1H)-one for 3-methylpyridin-2(1H)-one in Example 301. MS (ESI) m/e 497 (M+H)⁺.

### Example 357

### 3-(3-methoxy-4-nitrophenyl)-8-{2-[(3-methoxypyridin-2-yl)oxy]ethyl}-5,10-dihydro-11H-dibenzo[b,e][1,4]diazepin-11-one

The title compound was prepared by substituting 3-methoxypyridin-2(1H)-one for 3-methylpyridin-2(1H)-one in Example 301. MS (ESI) m/e 513 (M+H)⁺.

### Example 358

### 3-(3-methoxy-4-nitrophenyl)-8-[2-(3-oxoisoquinolin-2(3H)-yl)ethyl]-5,10-dihydro-11H-dibenzo[b,e][1,4]diazepin-11-one

The title compound was prepared by substituting isoquinolin-3(2H)-one for 3-methylpyridin-2(1H)-one in Example 301. MS (ESI) m/e 533 (M+H)⁺.

### Example 359

### 8-{2-[(6-chloropyridin-2-yl)oxy]ethyl}-3-(3-methoxy-4-nitrophenyl)-5,10-dihydro-11H-dibenzo[b,e][1,4]diazepin-11-one

The title compound was prepared by substituting 6-chloropyridin-2(1H)-one for 3-methylpyridin-2(1H)-one in Example 301. MS (ESI) m/e 517 and 519 (M+H)⁺.

### Example 360

### 8-{2-[(5-chloropyridin-2-yl)oxy]ethyl}-3-(3-methoxy-4-nitrophenyl)-5,10-dihydro-11H-dibenzo[b,e][1,4]diazepin-11-one

The title compound was prepared by substituting 5-chloropyridin-2(1H)-one for 3-methylpyridin-2(1H)-one in Example 301. MS (ESI) m/e 515 and 517 (M-H)⁻.

### Example 361

### [[3-(4-chloro-3-methoxyphenyl)-11-oxo-10,11-dihydro-5H-dibenzo[b,e][1,4]diazepin-8-yl]acetic acid

The desired product was prepared by substituting Example 54B for Example 12 in Example 13. MS (DCI) m/e 409 (M+H)⁺.

### Example 362

### methyl [3-(4-acetyl-3-methoxyphenyl)-11-oxo-10,11-dihydro-5H-dibenzo[b,e][1,4]diazepin-8-yl]acetate

The desired product was prepared by substituting Example 54A for Example 56A in Example 59C. MS (DCI) m/e 431 (M+H)⁺, ¹H NMR (300MHz, DMSO-d₆): δ 9.87 (s, 1H), 7.98 (s, 1H), 7.78 (d, J=8.14 Hz, 1H), 7.70 (d, J=8.14 Hz, 1H), 7.35 (m, 2H), 7.28 (d, J=7.80 Hz, 2H), 6.89 (m, 3H), 4.00 (s, 3H), 3.60 (s, 3H), 3.54 (s, 2H), 2.56 (s, 3H).

### Example 363

### [3-(4-acetyl-3-methoxyphenyl)-11-oxo-10,11-dihydro-5H-dibenzo[b,e][1,4]diazepin-8-yl]acetic acid

The desired product was prepared by substituting Example 362 for Example 12 in Example 13. MS (ESI) m/e 417 (M+H)⁺, ¹H NMR (300MHz, DMSO-d₆): δ 12.27 (s, 1H), 9.86 (s, 1H), 7.96 (s, 1H), 7.78 (d, J=8.14 Hz, 1H), 7.70 (d, J=8.14 Hz, 1H), 7.36 (d, J=1.70 Hz, 1H), 7.34 (d, J=1.36 Hz, 1H), 7.29 (d, J=1.70 Hz, 1H), 7.26 (d, J=1.70 Hz, 1H), 6.84-6.97 (m, 3H), 4.00 (s, 3H), 3.42 (s, 2H), 2.56 (s, 3H).

### Example 364

### 2-[3-(4-chloro-3-methoxyphenyl)-11-oxo-10,11-dihydro-5H-dibenzo[b,e][1,4]diazepin-8-yl]-N,N-dimethylacetamide

The desired product was prepared by substituting Example 361 and N,N-dimethylamine hydrochloride for Example 13 and 3-pyrrolidin-1-ylpropylamine, respectively, in Example 14. MS (APCI) m/e 436 (M+H)⁺, ¹H NMR (300MHz, DMSO-d₆): δ 9.83 (s, 1H), 7.91 (s, 1H), 7.77 (s, 1H), 7.63 (s, 1H), 7.34 (d, J=2.03 Hz, 1H), 7.29 (d, J=1.36 Hz, 1H), 7.19-7.24 (m, 2H), 6.80-6.95 (m, 3H), 3.96 (s, 3H), 3.54 (s, 2H), 2.97 (s, 3H), 2.81 (s, 3H).

### Example 365

### tert-butyl 1-{[3-(3-methoxy-4-nitrophenyl)-11-oxo-10,11-dihydro-5H-dibenzo[b,e][1,4]diazepin-8-yl]acetyl}pyrrolidin-3-ylcarbamate

The desired product was prepared by substituting Example 73 and tert-butyl pyrrolidin-3-ylcarbamate for Example 13 and 3-pyrrolidin-1-ylpropylamine respectively, in Example 14. MS (DCI) m/e 588 (M+H)⁺, ¹H NMR (500MHz, DMSO-d₆): δ 9.87 (s, 1H), 8.01 (d, J=8.42 Hz, 1H), 7.95 (s, 1H), 7.79 (d, J=8.11 Hz, 1H), 7.52 (s, 1H), 7.34 (m, 2H), 7.30 (d, J=8.11 Hz, 1H), 7.10 (s, 1H), 6.94 (dd, J=7.96, 2.96 Hz, 1H), 6.80-6.85 (m, 2H), 4.03 (s, 3H), 3.97 (m, 1H), 3.59 (m, 1H), 3.37-3.47 (m, 4H), 3.17 (m, 1H), 1.99 (m, 1H), 1.75 (m, 1H), 1.38 (s, 4H), 1.35 (s, 5H).

### Example 366

### 8-[2-(3-aminopyrrolidin-1-yl)-2-oxoethyl]-3-(3-methoxy-4-nitrophenyl)-5,10-dihydro-11H-dibenzo[b,e][1,4]diazepin-11-one

Trifluoroacetic acid (2 mL) was added to a solution of Example 365 (35 mg, 0.06 mmol) in CH₂Cl₂ (3 mL) and stirred at room temperature for 5 hours. Concentrated under vacuum. Residue was purified by preparative HPLC to provide 25 mg (70%) of the desired product as the trifluoroacetate salt. MS (DCI) m/e 488 (M+H)⁺, ¹H NMR (500MHz, DMSO-d₆): δ 9.87 (s, 1H), 7.99-8.08 (m, 4H), 7.95 (s, 1H), 7.80 (d, J=8.42 Hz, 1H), 7.52 (s, 1H), 7.36 (s, 1H), 7.34 (dd, J=8.42, 1.56 Hz, 1H), 7.30 (dd, J=8.11, 1.25 Hz, 1H), 6.82-6.97 (m, 2H), 4.03 (s, 3H), 3.83 (m, 1H), 3.64-3.74 (m, 2H), 3.38-3.57 (m, 4H), 2.19 (m, 1H), 1.95 (m, 1H).

### Example 367

### [3-(2-methoxy-5-methylpyridin-4-yl)-11-oxo-10,11-dihydro-5H-dibenzo[b,e][1,4]diazepin-8-yl]acetic acid

The desired product was prepared by substituting Example 72 for Example 12 in Example 13. MS (DCI) m/e 390 (M+H)⁺, ¹H NMR (500MHz, DMSO-d₆): δ 9.86 (s, 1H), 8.09(s, 1H), 7.91 (s, 1H), 7.75 (d, J=8.11 Hz, 1H), 7.01 (d, J=1.25 Hz, 1H), 6.84-6.93 (m, 4H), 6.66 (s, 1H), 3.86 (s, 3H), 3.43 (s, 2H), 2.14 (s, 3H).

### Example 368

### 2-[3-(2-methoxy-5-methylpyridin-4-yl)-11-oxo-10,11-dihydro-5H-dibenzo[b,e][1,4]diazepin-8-yl]-N,N-dimethylacetamide

The title compound was prepared by substituting Example 367 and N,N-dimethylamine hydrochloride for dimethylaminoacetic acid and Example 120, respectively, in Example 122. MS (DCI) m/e 417 (M+H)⁺, ¹H NMR (300MHz, DMSO-d₆): δ 9.87 (s, 1H), 8.09 (s, 1H), 7.92 (s, 1H), 7.74 (d, J=8.14 Hz, 1H), 7.00 (d, J=1.70 Hz, 1H), 6.88-6.93 (m, 2H), 6.79-6.83 (m, 2H), 6.65 (s, 1H), 3.85 (s, 3H), 3.55 (s, 2H), 2.97 (s, 3H), 2.81 (s, 3H), 2.14 (s, 3H).

### Example 369

### 8-{2-[(2S)-2-(hydroxymethyl)pyrrolidin-1-yl]-2-oxoethyl}-3-(2-methoxy-5-methylpyridin-4-yl)-5,10-dihydro-11H-dibenzo[b,e][1,4]diazepin-11-one

The desired product was prepared by substituting Example 367 and (2S)-2-pyrrolidinylmethanol for dimethylaminoacetic acid and Example 120, respectively, in Example 122. MS (DCI) m/e 473 (M+H)⁺, ¹H NMR (300MHz, DMSO-d₆): δ 9.85 (s, 1H), 8.09 (s, 1H), 7.88 (d, J=4.99 Hz, 1H), 7.74 (d, J=7.80 Hz, 1H), 6.99 (s, 1H), 6.88-6.91 (m, 2H), 6.80-6.85 (m, 2H), 6.65 (s, 1H), 3.96 (m, 1H), 3.85 (s, 3H), 3.39-3.52 (m, 4H), 3.23-3.26 (m, 2H), 2.14 (s, 3H), 1.76-1.89 (m, 4H).

### Example 370

### tert-butyl 1-{[3-(2-methoxy-5-methylpyridin-4-yl)-11-oxo-10,11-dihydro-5H-dibenzo[b,e][1,4]diazepin-8-yl]acetyl}pyrrolidin-3-ylcarbamate

The desired product was prepared by substituting Example 367 and tert-butyl pyrrolidin-3-ylcarbamate for dimethylaminoacetic acid and Example 120, respectively, in Example 122. MS (DCI) m/e 558 (M+H)⁺, ¹H NMR (500MHz, DMSO-d₆):δ 9.84 (s, 1H), 8.09 (s, 1H), 7.88 (s, 1H), 7.75 (d, J=8.11 Hz, 1H), 7.10 (s, 1H), 6.99 (s, 1H), 6.88-6.91 (m, 2H), 6.80-6.86 (m, 2H), 6.65 (d, J=2.18 Hz, 1H), 3.98 (m, 1H), 3.85 (s, 3H), 3.55-3.65 (m, 2H), 3.39-3.53 (m, 2H), 3.11-3.30 (m, 2H), 2.14 (s, 3H), 1.99 (m, 1H), 1.76 (m 1H), 1.37 (d, J=13.73 Hz, 9H).

### Example 371

### N-{2-[4-(aminosulfonyl)phenyl]ethyl}-2-[3-(4-chloro-3-methoxyphenyl)-11-oxo-10,11-dihydro-5H-dibenzo[b,e][1,4]diazepin-8-yl]acetamide

The desired product was prepared by substituting Example 361 and 4-(2-aminoethyl)benzenesulfonamide for dimethylaminoacetic acid and Example 120, respectively, in Example 122. MS (DCI) m/e 592 (M+H)⁺, ¹H NMR (500MHz, DMSO-d₆): δ 9.83 (s, 1H), 8.05 (t, J=5.15 Hz, 1H), 7.89 (s, 1H), 7.77 (d, J=8.11 Hz, 1H), 7.73 (d, J=8.11 Hz, 1H), 7.53 (d, J=8.11 Hz, 1H), 7.34-7.37 (m, 3H), 7.30 (s, 1H), 7.20-7.26 (m, 4H), 6.93 (d, J=8.11 Hz, 1H), 6.87 (s, 1H), 6.82 (d, J=7.80 Hz, 1H), 3.96 (s, 3H), 3.27 (m, 4H), 2.77 (t, J=7.02 Hz, 1H).

### Example 372

### tert-butyl 1-{[3-(4-chloro-3-methoxyphenyl)-11-oxo-10,11-dihydro-5H-dibenzo[b,e][1,4]diazepin-8-yl]acetyl}pyrrolidin-3-ylcarbamate

The desired product was prepared by substituting Example 361 and tert-butyl pyrrolidin-3-ylcarbamate for dimethylaminoacetic acid and Example 120, respectively, in Example 122. MS (DCI) m/e 578 (M+H)⁺, ¹H NMR (500MHz, DMSO-d₆): δ 9.79 (s, 1H), 7.89 (s, 1H), 7.76 (d, J=8.11 Hz, 1H), 7.53 (d, J=8.11 Hz, 1H), 7.33 (s, 1H), 7.29 (s, 1H), 7.19-7.23 (m, 2H), 7.10 (m, 1H), 6.93 (dd, J=8.11, 3.12 Hz, 1H), 6.79-6.84 (m, 2H), 3.91-4.02 (m, 4H), 3.59 (m, 1H), 3.37-3.49 (m, 4H), 3.18 (m, 1H), 1.96 (m, 1H), 1.75 (m, 1H), 1.37 (d, J=13.10 Hz, 9H).

### Example 373

### 3-(4-chloro-3-methoxyphenyl)-8-[2-(3-hydroxypiperidin-1-yl)-2-oxoethyl]-5,10-dihydro-11H-dibenzo[b,e][1,4]diazepin-11-one

The desired product was prepared by substituting Example 361 and 3-piperidinol for dimethylaminoacetic acid and Example 120, respectively, in Example 122. MS (DCI) m/e 492 (M+H)⁺, ¹H NMR(500MHz, DMSO-d₆): δ 9.81 (d, J=2.81 Hz, 1H), 7.89 (s, 1H), 7.76 (d, J=8.11 Hz, 1H), 7.53 (d, J=8.11 Hz, 1H), 7.34 (d, J=1.87 Hz, 1H), 7.29 (s, 1H), 7.19-7.23 (m, 2H), 6.94 (d, J=7.80 Hz, 1H), 6.79-6.82 (m, 2H), 3.96-4.16 (m, 4H), 3.49-3.65 (m, 4H), 2.93-3.06 (m, 2H), 1.78 (m, 1H), 1.61 (m, 1H), 1.18-1.41 (m, 2H).

### Example 374

### 3-(4-chloro-3-methoxyphenyl)-8-{2-[(2S)-2-(hydroxymethyl)pyrrolidin-1-yl]-2-oxoethyl}-5,10-dihydro-11H-dibenzo[b,e][1,4]diazepin-11-one

The desired product was prepared by substituting Example 361 and (2S)-2-pyrrolidinylmethanol for dimethylaminoacetic acid and Example 120, respectively, in Example 122. MS (DCI) m/e 492 (M+H)⁺, ¹H NMR (500MHz, DMSO-d₆): δ 9.80 (s, 1H), 7.89 (s, 1H), 7.76 (d, J=8.11 Hz, 1H), 7.53 (d, J=8.42 Hz, 1H), 7.34 (d, J=1.87 Hz, 1H), 7.29 (s, 1H), 7.19-7.23 (m, 2H), 6.93 (m, 1H), 6.81-6.84 (m, 2H), 3.89-3.96 (m, 4H), 3.48-3.52 (m, 2H), 3.23-3.27 (m, 4H), 1.77-1.92 (m, 4H).

### Example 375

### 2-[3-(4-chloro-3-methoxyphenyl)-11-oxo-10,11-dihydro-5H-dibenzo[b,e][1,4]diazepin-8-yl]N(4-morpholin-4-ylphenyl)acetamide

The desired product was prepared by substituting Example 361 and 4(4-morpholinyl)aniline for dimethylaminoacetic acid and Example 120, respectively, in Example 122. MS (DCI) m/e 570 (M+H)⁺, ¹H NMR (500MHz, DMSO-d₆): δ 9.86 (s, 1H), 9.84 (s, 1H), 7.90 (s, 1H), 7.76 (d, J=8.11 Hz, 1H), 7.53 (d, J=8.11 Hz, 1H), 7.44 (s, 1H), 7.43 (s, 1H), 7.33 (d, J=1.87 Hz, 1H), 7.29 (d, J=1.56 Hz, 1H), 7.19-7.23 (m, 2H), 6.86-6.97 (m, 5H), 3.95 (s, 3H), 3.71 (m, 4H), 3.46 (s, 2H), 3.02 (m, 4H).

### Example 376

### tert-butyl 4-{[3-(3-methoxy-4-nitrophenyl)-11-oxo-10,11-dihydro-5H-dibenzo[b,e][1,4]diazepin-8-yl]acetyl}piperazine-1-carboxylate

The desired product was prepared by substituting Example 73 and tert-butyl piperazine-1-carboxylate for dimethylaminoacetic acid and Example 120, respectively, in Example 122. MS (DCI) m/e 588 (M+H)⁺, ¹H NMR (400MHz, DMSO-d₆): δ 9.88 (s, 1H), 8.01 (d, J=8.59 Hz, 1H), 7.97 (s, 1H), 7.80 (d, J=8.29 Hz, 1H), 7.52 (d, J=1.84 Hz, 1H), 7.33-7.36 (m, 2H), 7.29-7.31 (dd, J=8.29, 1.84 Hz, 1H), 6.95 (d, J=8.59 Hz, 1H), 6.81-6.85 (m, 2H), 4.03 (s, 3H), 3.60 (s, 2H), 3.43-3.45 (m, 4H), 3.20-3.29 (m, 4H), 1.39 (s, 9H).

### Example 377

### 3-(3-methoxy-4-nitrophenyl)-8-(2-oxo-2-piperazin-1-ylethyl)-5,10-dihydro-11H-dibenzo[b,e][1,4]diazoin-11-one

The desired product was prepared by substituting Example 376 for Example 365 in Example 366. MS (DCI) m/e 488 (M+H)⁺, ¹H NMR (500MHz, DMSO-d₆): δ 9.89 (s, 1H), 8.78 (s, 2H), 7.99-8.02 (m, 2H), 7.80 (d, J=8.42 Hz, 1H), 7.52 (s, 1H), 7.34-7.36 (m, 2H), 7.31 (d, J=8.11 Hz, 1H), 6.97 (d, J=8.42 Hz, 1H), 6.82-6.84 (m, 2H), 4.03 (s, 3H), 3.64 (s, 2H), 3.36-3.51 (m, 2H), 3.03-3.08 (m, 4H).

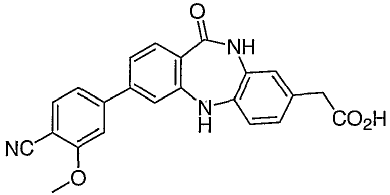

### Example 378 [3-(4-cyano-3-methoxyphenyl)-11-oxo-10,11-dihydro-5H-dibenzo[b,e][1,4]diazepin-8-yl]acetic acid

The desired product was prepared by substituting Example 61 for Example 12 in Example 13. MS (DCI) m/e 400 (M+H)⁺, ¹H NMR (400MHz, DMSO-d₆): δ 12.25 (s, 1H), 9.87 (s, 1H), 7.97 (s, 1H), 7.83 (d, J=7.98 Hz, 1H), 7.79 (d, J=8.29 Hz, 1H), 7.42 (d, J=1.23 Hz, 1H), 7.32-7.35 (m, 2H), 7.28 (dd, J=8.29, 1.53 Hz, 1H), 6.84-6.97 (m, 3H), 4.02 (s, 3H), 3.42 (s, 2H).

### Example 379

### 2-[3-(4-cyano-3-methoxyphenyl)-11-oxo-10,11-dihydro-5H-dibenzo[b,e][1,4]diazepin-8-yl]N(4-morpholin-4-ylphenyl)acetamide

The desired product was prepared by substituting Example 378 and 4-morpholin-4-ylphenylamine for dimethylaminoacetic acid and Example 120, respectively, in Example 122. MS (DCI) m/e 560 (M+H)⁺, ¹H NMR (500MHz, DMSO-d₆): δ 9.89 (s, 1H), 9.86 (s, 1H), 7.94 (s, 1H), 7.83 (d, J=8.11 Hz, 1H), 7.79 (d, J=8.11 Hz, 1H), 7.42-7.44 (m, 3H), 7.32-7.34 (m, 2H), 7.28 (dd, J=8.27, 1.72 Hz, 1H), 6.91-6.97 (m, 3H), 6.88 (s, 1H), 6.86 (s, 1H), 4.02 (s, 3H), 3.70-3.72 (m, 4H), 3.46 (s, 2H), 3.01-3.03 (m, 4H).

### Example 380

### 2-[3-(3-methoxy-4-nitrophenyl)-11-oxo-10,11-dihydro-5H-dibenzo[b,e][1,4]diazepin-8-yl]Nmethylacetamide

The desired product was prepared by substituting Example 73 and methylamine hydrochloride for dimethylaminoacetic acid Example 120, respectively, in Example 122. MS (DCI) m/e 433 (M+H)⁺, ¹H NMR (500MHz, DMSO-d₆): δ 9.86 (s, 1H), 8.01 (m, 1H), 7.95 (s, 1H), 7.82 (d, J=4.37 Hz, 1H), 7.80 (d, J=8.42 Hz, 1H), 7.52 (s, 1H), 7.29-7.35 (m, 3H), 6.84-6.95 (m, 3H), 4.03 (s, 3H), 3.26 (s, 2H), 2.56 (d, J=4.68 Hz, 3H).

### Example 381 3-(3-methoxy-4-nitrophenyl)-8-(2-morpholin-4-yl-2-oxoethyl)-5,10-dihydro-11H-dibenzo[b,e][1,4]diazepin-11-one

The desired product was prepared by substituting Example 73 and morpholine for dimethylaminoacetic acid and Example 120, respectively, in Example 122. MS (DCI) m/e 489 (M+H)⁺, ¹H NMR (500MHz, DMSO-d₆): δ 9.88 (s, 1H), 8.01 (d, J=8.42 Hz, 1H), 7.97 (s, 1H), 7.80 (d, J=8.11 Hz, 1H), 7.52 (d, J=1.25 Hz, 1H), 7.33-7.35 (m, 2H), 7.30 (dd, J=8.27, 1.72 Hz, 1H), 6.95 (d, J=7.80 Hz, 1H), 6.80-6.83 (m, 2H), 4.03 (s, 3H), 3.59 (s, 2H), 3.52 (m, 4H), 3.45 (m, 4H).

### Example 382

### 3-(2-methoxy-5-methylpyridin-4-yl)-8-(2-morpholin-4-yl-2-oxoethyl)-5,10-dihydro-11H-dibenzo[b,e][1,4]diazepin-11-one

The desired product was prepared by substituting Example 367 and morpholine for dimethylaminoacetic acid and Example 120, respectively, in Example 122. MS (DCI) m/e 459 (M+H)⁺, ¹H NMR (400MHz, DMSO-d₆): δ 9.87 (s, 1H), 8.09 (s, 1H), 7.90 (s, 1H), 7.75 (d, J=7.98 Hz, 1H), 6.99 (d, J=1.53 Hz, 1H), 6.88-6.92 (m, 2H), 6.82 (m, 2H), 6.66 (s, 1H), 3.85 (s, 3H), 3.59 (s, 2H), 3.49-3.55 (m, 4H), 3.43-3.46 (m, 4H), 2.14 (s, 3H).

### Example 383

### [3-(2-fluoropyridin-4-yl)-11-oxo-10,11-dihydro-5H-dibenzor[b,e][1,4]diazepin-8-yl]acetic acid

### Example 383A

### methyl [3-(2-fluoropyridin-4-yl)-11-oxo-10,11-dihydro-5H-dibenzo[b,e][1,4]diazepin-8-yl]acetate

The desired product was prepared by substituting Example 69A and Example 6D for Example 56A and Example 59B respectively, in Example 59C. MS (DCI) m/e 378 (M+H)⁺, 396 (M+NH₄)⁺, ¹H NMR (500MHz, DMSO-d₆): δ 9.91 (s, 1H), 8.35 (d, J=5.30 Hz, 1H), 7.99 (s, 1H), 7.81 (d, J=8.11 Hz, 1H), 7.61 (m, 1H), 7.43 (s, 1H), 7.41 (d, J=1.56 Hz, 1H), 7.34 (dd, J=8.27, 1.72 Hz, 1H), 6.96 (d, J=7.80 Hz, 1H), 6.86-6.89 (m, 2H), 3.60 (s, 3H), 3.54 (s, 2H).

### Example 383B

### [3-(2-fluoropyridin-4-yl)-11-oxo-10,11-dihydro-5H-dibenzo[b,e][1,4]diazepin-8-yl]acetic acid

The desired product was prepared by substituting Example 383A for Example 69B in Example 69C. MS (DCI) m/e 364 (M+H)⁺.

### Example 384

### N,N-dimethyl-2-[11-oxo-3-(2-oxo-1,2-dihydropyridin-4-yl)-10,11-dihydro-5H-dibenzo[b,e][1,4]diazepin-8-yl]acetamide

### Example 384A

### 2-[3-(2-fluoropyridin-4-yl)-11-oxo-10,11-dihydro-5H-dibenzo[b,e][1,4]diazepin-8-yl]-N,N-dimethylacetamide

The desired product was prepared by substituting Example 383B and N,N-dimethylamine hydrochloride for dimethylaminoacetic acid,and Example 120, respectively, in Example 122. MS (DCI) m/e 391 (M+H)⁺.

### Example 384B

### N,N-dimethyl-2-[11-oxo-3-(2-oxo-1,2-dihydropyridin-4-yl)-10,11-dihydro-5H-dibenzo[b,e][1,4]diazepin-8-yl]acetamide

The desired product was prepared by substituting Example 384A for Example 69D in Example 70. MS (DCI) m/e 389 (M+H)⁺, ¹H NMR (500MHz, DMSO-d₆): δ 9.84 (s, 1H), 7.88 (s, 1H), 7.74 (d, J=8.42 Hz, 1H), 7.46 (d, J=6.86 Hz, 1H), 7.28 (d, J=1.56 Hz, 1H), 7.16 (dd, J=8.11, 1.56 Hz, 1H), 6.91 (d, J=8.11 Hz, 1H), 6.80-6.83 (m, 2H), 6.51 (s, 1H), 6.40 (m, 1H), 3.53 (s, 2H), 2.96 (s, 3H), 2.80 (s, 3H).

### Example 385

### 8-(2-morpholin-4-yl-2-oxoethyl)-3-(2-oxo-1,2-dihydropyridin-4-yl)-5,10-dihydro-11H-dibenzo[b,e][1,4]diazepin-11-one

### Example 385A

### 3-(2-fluoropyridin-4-yl)-8-(2-morpholin-4-yl-2-oxoethyl)-5,10-dihydro-11H-dibenzo[b,e][1,4]diazepin-11-one

The desired product was prepared by substituting Example 383B and morpholine for dimethylaminoacetic acid and Example 120, respectively, in Example 122. MS (DCI) m/e 433 (M+H)⁺.

### Example 385B

### 8-(2-morpholin-4-yl-2-oxoethyl)-3-(2-oxo-1,2-dihydropyridin-4-yl)-5,10-dihydro-11H-dibenzo[b,e][1,4]diazepin-11-one

The desired product was prepared by substituting Example 385A for Example 69D in Example 70. MS (DCI) m/e 431 (M+H)⁺, ¹H NMR (500MHz, DMSO-d₆): δ 9.86 (s, 1H), 7.89 (s, 1H), 7.74 (d, J=8.11 Hz, 1H), 7.47 (d, J=6.55 Hz, 1H), 7.28 (d, J=1.56 Hz, 1H), 7.16 (dd, J=8.26, 1.72 Hz, 1H), 6.92 (d, J=8.42 Hz, 1H), 6.80-6.82 (m, 2H), 6.52 (d, J=1.25 Hz, 1H), 6.41 (dd, J=6.86, 1.56 Hz, 1H), 3.57 (s, 2H), 3.52-3.53 (m, 2H), 3.48-3.50 (m, 2H), 3.43-3.45 (m, 4H).

### Example 386

### N-(4-morpholin-4-ylphenyl)-2-[11-oxo-3-(2-oxo-1,2-dihydropyridin-4-yl)-10,11-dihydro-5H-dibenzo[b,e][1,4]diazepin-8-yl]acetamide

### Example 386A

### 2-[3-(-fluoropyridin-4-yl)-11-oxo-10,11-dihydro-5H-dibenzo[b,e][1,4]diazepin-8-yl]N(4-morpholin-4-ylphenyl)acetamide

The desired product was prepared by substituting Example 383B and 4(4-morpholinyl)aniline for dimethylaminoacetic acid and Example 120, respectively, in Example 122. MS (DCI) m/e 524 (M+H)⁺.

### Example 386B

### N-(4-morpholin-4-ylphenyl)-2-[11-oxo-3-(2-oxo-1,2-dihydropyridin-4-yl)-10,11-dihydro-5H-dibenzo[b,e][1,4]diazepin-8-yl]acetamide

The desired product was prepared by substituting Example 386A for Example 69D in Example 70. MS (DCI) m/e 522 (M+H)⁺, ¹H NMR (500MHz, DMSO-d₆): δ 9.88 (s, 1H), 9.86 (s, 1H), 7.89 (s, 1H), 7.74 (d, J=8.11 Hz, 1H), 7.46 (d, J=6.86 Hz, 1H), 7.43 (d, J=9.04 Hz, 2H), 7.28 (d, J=1.56 Hz, 1H), 7.16 (dd, J=8.11, 1.56 Hz, 1H), 6.90-6.94 (m, 3H), 6.87 (d, J=9.04 Hz, 2H), 6.51 (d, J=1.56 Hz, 1H), 6.40 (d, J=6.86 Hz, 1H), 3.70-3.72 (m, 4H), 3.45 (s, 2H), 3.01-3.03 (m, 4H).

### Example 387

### methyl [11-oxo-3-(2-oxo-1,2-dihydropyridin-4-yl)-10,11-dihydro-5H-dibenzo[b,e][1,4]diazepin-8-yl]acetate

The desired product was prepared by substituting Example 383A for Example 69D in Example 70. MS (DCI) m/e 376 (M+H)⁺, ¹H NMR (400MHz, DMSO-d₆): δ 11.66 (s, 1H), 9.87 (s, 1H), 7.94 (s, 1H), 7.75 (d, J=8.29 Hz, 1H), 7.48 (d, J=6.75 Hz, 1H), 7.29 (s, 1H), 7.18 (d, J=7.67 Hz, 1H), 6.96 (m, 1H), 6.85-6.87 (m, 2H), 6.53 (s, 1H), 6.41 (d, J=6.75 Hz, 1H), 3.60 (s, 3H), 3.54 (s, 2H).

### Example 388)

### methyl [3-(3-methoxy-4-nitrophenyl)-11-oxo-10,11-dihydro-5H-dibenzo[b,e][1,4]diazepin-7-yl]acetate

### Example 388A

### methyl (3-amino-4-nitrophenyl)acetate

Methyl (3-aminophenyl)acetate was treated with acetic anhydride to provide methyl [3-(acetylamino)phenyl]acetate. The desired product was prepared by substituting methyl [3-(acetylamino)phenyl]acetate for N-[4-(cyanomethyl)phenyl]acetamide in Example 6A. MS (DCI) m/e 211 (M+H)⁺.

### Example 388B

### methyl 4-chloro-2-{[5-(2-methoxy-2-oxoethyl)-2-nitrophenyl]amino}benzoate

The desired product was prepared by substituting Example 388A for methyl 3,4-diaminobenzoate in Example 1A. MS (APCI) m/e 379 (M+H)⁺.

### Example 388C

### methyl 2-{[2-amino-5-(2-methoxy-2-oxoethyl)phenyl]amino}1-4-chlorobenzoate

The desired product was prepared by substituting Example 388B for Example 6B in Example 6C. MS (DCI) m/e 349 (M+H)⁺.

### Example 388D

### methyl (3-chloro-11-oxo-10,11-dihydro-5H-dibenzo[b,e][1,4]diazepin-7-yl)acetate

The desired product was prepared by substituting Example 388C for Example 5B in Example 5C. MS (DCI) m/e 317 (M+H)⁺, 334 (M+NH₄)⁺, ¹H NMR (500MHz, DMSO-d₆): δ 9.88 (s, 1H), 8.05 (s, 1H), 7.68 (d, J=8.42 Hz, 1H), 7.07 (d, J=1.87 Hz, 1H), 6.93 (dd, J=8.58, 2.03 Hz, 1H), 6.88-6.91 (m, 2H), 6.82 (dd, J=8.11, 1.87 Hz, 1H), 3.60 (s, 3H), 3.56 (s, 2H).

### Example 388E

### methyl [3-(3-methoxy-4-nitrophenyl)-11-oxo-10,11-dihydro-5H-dibenzo[b,e][1,4]diazepin-7-yl]acetate

The desired product was prepared by substituting Example 388D and Example 266G for Example 59B and Example 56A, respectively, in Example 59C. MS (DCI) m/e 434 (M+H)⁺, ¹H NMR (500MHz, DMSO-d₆) δ 9.88 (s, 1H), 8.00-8.02 (m, 2H), 7.80 (d, J=8.11Hz, 1H), 7.52 (d, J=1.56 Hz, 1H), 7.37 (d, J=1.56 Hz, 1H), 7.35 (dd, J=8.42, 1.56 Hz, 1H), 7.31 (dd, J=8.11, 1.87 Hz, 1H), 6.91-6.93 (m, 2H), 6.81 (dd, J=8.11, 1.87 Hz, 1H), 4.03 (s, 3H), 3.60 (s, 3H), 3.56 (s, 2H).

### Example 389

### [3-(3-methoxy-4-nitrophenyl)-11-oxo-10,11-dihydro-5H-dibenzo[b,e][1,4]diazepin-7-yl]acetic acid

The desired product was prepared by substituting Example 388E for Example 12 in Example 13. MS (DCI) m/e 420 (M+H)⁺, ¹H NMR (500MHz, DMSO-d₆): δ 12.29 (s, 1H), 9.87 (s, 1H), 8.00-8.02 (m, 2H), 7.80 (d, J=7.80 Hz, 1H), 7.53 (d, J=1.53 Hz, 1H), 7.38 (d, J=1.84 Hz, 1H), 7.34 (dd, J=8.44, 1.69 Hz, 1H), 7.31 (dd, J=8.29, 1.53 Hz, 1H), 6.94 (d, J=1.84 Hz, 1H), 6.91 (d, J=8.29 Hz, 1H), 6.80 (dd, J=8.13, 1.69 Hz, 1H), 4.03 (s, 3H), 3.44 (s, 2H).

### Example 390

### 3-(3-methoxy-4-nitrophenyl)-7-(2-morpholin-4-yl-2-oxoethyl)-5,10-dihydro-11H-dibenzo[b,e][1,4]diazepin-11-one

The desired product was prepared by substituting Example 389 and morpholine for dimethylaminoacetic acid and Example 120, respectively, in Example 122. MS (DCI) m/e 489 (M+H)⁺, 506 (M+NH₄)⁺, ¹H NMR (500MHz, DMSO-d₆): δ 9.86 (s, 1H), 8.00-8.02 (m, 2H), 7.80(d, J=8.42 Hz, 1H), 7.52 (d, J=1.56 Hz, 1H), 7.37 (d, J=1.56 Hz, 1H), 7.34 (dd, J=8.42, 1.56 Hz, 1H), 7.32 (m, 1H), 6.90-6.92 (m, 2H), 6.73 (m, 1H), 4.03 (s, 3H), 3.60 (s, 2H), 3.53 (d, J=4.37 Hz, 2H), 3.49 (d, J=4.37 Hz, 2H), 3.45 (d, J=4.37 Hz, 4H).

### Example 391

### 2-[3-(3-methoxy-4-nitrophenyl)-11-oxo-10,11-dihydro-5H-dibenzo[b,e][1,4]diazepin-7-yl]Npyridin-2-ylacetamide

The desired product was prepared by substituting Example 389 and 2-aminopyridine for dimethylaminoacetic acid and Example 120, respectively, in Example 122. MS (DCI) m/e 496 (M+H)⁺, ¹H NMR (400MHz, DMSO-d₆): δ 10.59 (s, 1H), 9.80 (s, 1H), 8.24 (d, J=4.60 Hz, 1H), 7.92-7.98 (m, 3H), 7.67-7.74 (m, 2H), 7.45 (d, J=1.23 Hz, 1H), 7.31 (d, J=1.53 Hz, 1H), 7.27 (dd, J=8.29, 1.53 Hz, 1H), 7.23 (dd, J=8.29, 1.53 Hz, 1H), 7.03 (dd, J=6.90, 5.37 Hz, 1H), 6.93 (S, 1H), 6.81-6.87 (m, 2H), 3.96 (s, 3H), 3.55 (s, 2H).

### Example 392

### 2-[3-(3-methoxy-4-nitrophenyl)-11-oxo-10,11-dihydro-5H-dibenzo[b,e][1,4]diazepin-7-yl]Npyridin-3-ylacetamide

The desired product was prepared by substituting Example 389 and 3-aminopyridine for dimethylaminoacetic acid and Example 120, respectively, in Example 122. MS (DCI) m/e 496 (M+H)⁺, ¹H NMR (400MHz, DMSO-d₆): δ 10.46 (s, 1H), 9.81 (s, 1H), 8.80 (s, 1H), 8.28 (d, J=4.60 Hz, 1H), 8.07 (d, J=8.29 Hz, 1H), 7.97 (s, 1H), 7.94 (d, J=8.29 Hz, 1H), 7.73 (d, J=8.29 Hz, 1H), 7.42-7.45 (m, 2H), 7.30 (d, J=1.53 Hz, 1H), 7.27 (dd, J=8.29, 1.53 Hz, 1H), 7.24 (dd, J=8.29, 1.53 Hz, 1H), 6.93 (s, 1H), 6.80-6.88 (m, 2H), 3.96 (s, 3H), 3.53 (s, 2H).

### Example 393

### 2-[3-(3-methoxy-4-nitrophenyl)-11-oxo-10,11-dihydro-5H-dibenzo[b,e][1,4]diazepin-7-yl]Npyridin-4-ylacetamide

The desired product was prepared by substituting Example 389 and 4-aminopyridine for dimethylaminoacetic acid and Example 120, respectively, in Example 122. MS (DCI) m/e 496 (M+H)⁺, ¹H NMR (500MHz, DMSO-d₆): δ 11.21 (s, 1H), 9.83 (s, 1H), 8.57 (s, 1H), 8.55 (s, 1H), 7.98 (s, 1H), 7.94 (d, J=8.59 Hz, 1H), 7.88 (s, 1H), 7.86 (s, 1H), 7.74 (d, J=8.29 Hz, 1H), 7.45 (d, J=1.53 Hz, 1H), 7.30 (d, J=1.53 Hz, 1H), 7.23-7.28 (m, 2H), 6.92 (d, J=1.23 Hz, 1H), 6.88 (m, 1H), 6.82 (m, 1H), 3.96 (s, 3H), 3.63 (s, 2H).

### Example 394

### 7-[2-(4-hydroxypiperidin-1-yl)-2-oxoethyl]-3-(3-methoxy-4-nitrophenyl)-5,10-dihydro-11H-dibenzo[b,e][1,4]diazepin-11-one

The desired product was prepared by substituting Example 389 and 4-piperidinol for dimethylaminoacetic acid and Example 120, respectively, in Example 122. MS (DCI) m/e 503 (M+H)⁺, ¹H NMR (400MHz, DMSO-d₆): δ 9.78 (s, 1H), 7.93-7.95 (m, 2H), 7.73 (d, J=8.29 Hz, 1H), 7.45 (d, J=1.84 Hz, 1H), 7.30 (d, J=1.84 Hz, 1H), 7.28 (dd, J=8.59, 1.84 Hz, 1H), 7.23 (dd, J=8.29, 1.84 Hz, 1H), 6.82-6.84 (m, 2H), 6.71 (dd, J=7.98, 1.84 Hz, 1H), 3.97 (s, 3H), 3.85 (m, 1H), 3.51 (s, 2H), 3.03-3.10 (m, 2H), 2.89-2.96 (m, 2H), 1.53-1.62 (m, 2H), 1.08-1.20 (m, 2H).

### Example 395

### 7-[2-(3-hydroxypiperidin-1-yl)-2-oxoethyl]-3-(3-methoxy-4-nitrophenyl)-5,10-dihydro-11H-dibenzo[b,e][1,4]diazepin-11-one

The desired product was prepared by substituting Example 389 and 3-piperidinol for dimethylaminoacetic acid and Example 120, respectively, in Example 120. MS (DCI) m/e 503 (M+H)⁺, ¹H NMR (400MHz, DMSO-d₆): δ 9.85 (s, 1H), 8.00-8.02 (m, 2H), 7.80 (d, J=8.29 Hz, 1H), 7.52 (d, J=1.53 Hz, 1H), 7.37 (d, J=1.53 Hz, 1H), 7.34 (dd, J=8.44, 1.69 Hz, 1H), 7.30 (dd, J=8.29, 1.84 Hz, 1H), 6.84-6.91 (m, 2H), 6.77 (m, 1H), 4.03 (s, 3H), 3.64 (m, 1H), 3.57 (s, 2H), 3.27-3.38 (m, 2H), 2.93-3.10 (m, 2H), 1.58-1.82 (m, 2H), 1.18-1.4 (m, 2H).

### Example 396

### 2-[3-(3-methoxy-4-nitrophenyl)-11-oxo-10,11-dihydro-5H-dibenzo[b,e][1,4]diazepin-7-yl]N(pyridin-3-ylmethyl)acetamide

The desired product was prepared by substituting Example 389 and pyridin-3-ylmethylamine for dimethylaminoacetic acid and Example 120, respectively, in Example 122. MS (DCI) m/e 510 (M+H)⁺, ¹H NMR (500MHz, DMSO-d₆): δ 9.79 (s, 1H), 8.51-8.54 (m, 3H), 7.94-7.95 (m, 2H), 7.88 (d, J=7.80 Hz, 1H), 7.73 (d, J=8.11 Hz, 1H), 7.53 (dd, J=7.80, 5.30 Hz, 1H), 7.46 (d, J=1.56 Hz, 1H), 7.31 (d, J=1.56 Hz, 1H), 7.28 (dd, J=8.42, 1.87 Hz, 1H), 7.24 (dd, J=8.26, 1.72 Hz, 1H), 6.88 (d, J=1.56 Hz, 1H), 6.84 (d, J=8.11 Hz, 1H), 6.75 (dd, J=8.11, 1.82 Hz, 1H), 4.28 (d, J=5.93 Hz, 1H), 3.97 (s, 3H), 3.32 (s, 2H).

### Example 397

### 2-[3-(3-methoxy-4-nitrophenyl)-11-oxo-10,11-dihydro-5H-dibenzo[b,e][1,4]diazepin-7-yl]N(pyridin-4-ylmethyl)acetamide

The desired product was prepared by substituting Example 389 and pyridin-4-ylmethylamine for dimethylaminoacetic acid and Example 120, respectively, in Example 120. MS (DCI) m/e 510 (M+H)⁺, ¹H NMR (500MHz, DMSO-d₆): δ 9.86 (s, 1H), 8.65-8.69 (m, 3H), 7.99-8.01 (m, 2H), 7.79 (d, J=8.11 Hz, 1H), 7.57 (s, 1H), 7.56 (s, 1H), 7.51 (d, J=1.56 Hz, 1H), 7.37 (d, J=1.87 Hz, 1H), 7.33 (dd, J=8.42, 1.56 Hz, 1H), 7.30 (dd, J=8.26, 1.72 Hz, 1H), 6.96 (d, J=1.56 Hz, 1H), 6.91 (d, J=8.11 Hz, 1H), 6.84 (dd, J=8.11, 1.56 Hz, 1H), 4.42 (d, J=5.93 Hz, 1H), 4.02 (s, 3H), 3.43 (s, 2H).

### Example 398

### 2-[3-(3-methoxy-4-nitrophenyl)-11-oxo-10,11-dihydro-5H-dibenzo[b,e][1,4]diazepin-7-yl]N(pyridin-2-ylmethyl)acetamide

The desired product was prepared by substituting Example 389 and pyridin-2-ylmethylamine for dimethylaminoacetic acid and Example 120, respectively, in Example 122. MS (DCI) m/e 510 (M+H)⁺, ¹H NMR (500MHz, DMSO-d₆): δ 9.86 (s, 1H), 8.60 (t, J=5.93 Hz, 1H), 8.54 (d, J=4.68 Hz, 1H), 8.00-8.02 (m, 2H), 7.84 (t, J=7.80 Hz, 1H), 7.80 (d, J=8.42 Hz, 1H), 7.52 (d, J=1.56 Hz, 1H), 7.38 (d, J=1.87 Hz, 1H), 7.30-7.35 (m, 4H), 6.97 (d, J=1.56 Hz, 1H), 6.91 (m, 1H), 6.84 (dd, J=8.11, 1.87 Hz, 1H), 4.39 (d, J=5.93 Hz, 1H), 4.03 (s, 3H), 3.42 (s, 2H).

### Example 399

### 7-(2-azetidin-1-yl-2-oxoethyl)-3-(3-methoxy-4-nitrophenyl)-5,10-dihydro-11H-dibenzo[b,e][1,4]diazepin-11-one

The desired product was prepared by substituting Example 389 and azetidine for dimethylaminoacetic acid and Example 120, respectively, in Example 122. MS (DCI) m/e 459 (M+H)⁺, ¹H NMR (500MHz, DMSO-d₆): δ 9.88 (s, 1H), 8.01-8.03 (m, 2H), 7.80 (d, J=8.24 Hz, 1H), 7.53 (d, J=1.53 Hz, 1H), 7.37 (d, J=1.53 Hz, 1H), 7.35 (dd, J=8.39, 1.68 Hz, 1H), 7.31 (dd, J=8.24, 1.83 Hz, 1H), 6.92 (d, J=1.53 Hz, 1H), 6.89 (d, J=8.24 Hz, 1H), 6.77 (dd, J=8.09, 1.68 Hz, 1H), 4.15 (t, J=7.63 Hz, 2H), 4.04 (s, 3H), 3.82 (t, J=7.63 Hz, 2H), 3.27 (s, 2H), 2.13-2.20 (m, 2H).

### Example 400 1-{[3-(3-methoxy-4-nitrophenyl)-11-oxo-10,11-dihydro-5H-dibenzo[b,e][1,4]diazepin-7-yl]acetyl}piperidine-3-carboxamide

The desired product was prepared by substituting Example 389 and piperidine-3-carboxamide for dimethylaminoacetic acid and Example 120, respectively, in Example 122. MS (DCI) m/e 530 (M+H)⁺, ¹H NMR (500MHz, DMSO-d₆): δ 9.88 (d, J=2.44 Hz, 1H), 8.00-8.02 (m, 2H), 7.80 (d, J=8.24 Hz, 1H), 7.53 (d, J=1.53 Hz, 1H), 7.37 (s, 1H), 7.35 (dd, J=8.54, 1.22 Hz, 1H), 7.30-7.32 (m, 2H), 6.75-6.92 (m, 4H), 4.26 (m, 1H), 4.03 (s, 3H), 3.85 (m, 1H), 3.56-3.68 (m, 2H), 3.01 (m, 1H), 2.60 (m, 1H), 2.15 (m, 1H), 1.84 (m, 1H), 1.47-1.64 (m, 2H), 1.23 (m, 1H).

### Example 401

### 1-{[3-(3-methoxy-4-nitrophenyl)-11-oxo-10,11-dihydro-5H-dibenzo[b,e][1,4]diazepin-7-yl]acetyl}piperidine-4-carboxamide

The desired product was prepared by substituting Example 389 and piperidine-4-carboxamide for dimethylaminoacetic acid and Example 120, respectively, in Example 122. MS (DCI) m/e 528 (M+H)⁺, ¹H NMR (500MHz, DMSO-d₆): δ 9.87 (s, 1H), 8.00-8.02 (m, 2H), 7.80 (d, J=8.24 Hz, 1H), 7.53 (d, J=1.53 Hz, 1H), 7.37 (d, J=1.53 Hz, 1H), 7.35 (dd, J=8.39, 1.68 Hz, 1H), 7.30 (dd, J=8.09, 1.68 Hz, 1H), 7.25 (s, 1H), 6.90 (m, 2H), 6.77-6.79 (m, 2H), 4.33 (d, J=12.82 Hz, 1H), 4.03 (s, 3H), 3.91 (d, J=13.43, 1H), 3.55-3.62 (m, 2H), 2.98 (t, J=11.75 Hz, 1H), 2.59 (m, 1H), 2.29 (m, 1H), 1.64-1.70 (m, 2H), 1.29-1.39 (m, 2H).

### Example 402

### 7-{2-[(2R)-2-(hydroxymethyl)pyrrolidin-1-yl]-2-oxoethyl}-3-(3-methoxy-4-nitrophenyl)-5,10-dihydro-11H-dibenzo[b,e][1,4]diazepin-11-one

The desired product was prepared by substituting Example 389 and (2R)-2-pyrrolidinylmethanol for dimethylaminoacetic acid and Example 120, respectively, in Example 122. MS (ESI) m/e 503 (M+H)⁺, ¹H NMR (500MHz, DMSO-d₆): δ 9.85 (s, 1H), 7.99-8.02 (m, 2H), 7.80 (d, J=8.42 Hz, 1H), 7.52 (d, J=1.56 Hz, 1H), 7.37 (d, J=1.56 Hz, 1H), 7.34 (dd, J=8.42, 1.56 Hz, 1H), 7.30 (dd, J=8.42, 1.56 Hz, 1H), 6.88-6.93 (m, 2H), 6.77 (dd, J=8.11, 1.56 Hz, 1H), 4.03 (s, 3H), 3.93 (m, 1H), 3.40-3.68 (m, 6H), 1.77-1.90 (m, 4H).

### Example 403

### 2-[3-(3-methoxy-4-nitrophenyl)-11-oxo-10,11-dihydro-5H-dibenzo[b,e][1,4]diazepin-7-yl]-N,N-dimethylacetamide

The desired product was prepared by substituting Example 389 and N,N-dimethylamine hydrochloride for dimethylaminoacetic acid and Example 120, respectively, in Example 122. MS (DCI) m/e 447 (M+H)⁺, ¹H NMR (500MHz, DMSO-d₆): δ 9.85 (s, 1H), 8.00 (m, 2H), 7.80 (d, J=8.11 Hz, 1H), 7.52 (d, J=1.56 Hz, 1H), 7.37 (d, J=1.56 Hz, 1H), 7.34 (dd, J=8.42, 1.56 Hz, 1H), 7.30 (dd, J=8.11 Hz, 8.42, 1.56 Hz, 1H), 6.89-6.91 (m, 2H), 6.78 (dd, J=7.95, 1.72 Hz, 1H), 4.03 (s, 3H), 3.56 (s, 2H), 2.98 (s, 3H), 2.82 (s, 3H).

### Example 404

### N,N-bis(2-methoxyethyl)-2-[3-(3-methoxy-4-nitrophenyl)-11-oxo-10,11-dihydro-5H-dibenzo[b,e][1,4]diazepin-7-yl]acetamide

The desired product was prepared by substituting Example 389 and N,N-bis(2-methoxyethyl)amine for dimethylaminoacetic acid and Example 120, respectively, in Example 122. MS (ESI) m/e 535 (M+H)⁺, ¹H NMR (500MHz, DMSO-d₆): δ 9.85 (s, 1H), 7.98-8.01 (m, 2H), 7.80 (d, J=8.11 Hz, 1H), 7.52 (s, 1H), 7.37 (d, J=1.56 Hz, 1H), 7.34 (dd, J=8.42, 1.56 Hz, 1H), 7.30 (dd, J=8.26, 1.72 Hz, 1H), 6.88-6.90 (m, 2H), 6.75 (d, J=8.11 Hz, 1H), 4.03 (s, 3H), 3.60 (s, 2H), 3.40-3.50 (m, 8H), 3.26 (s, 3H), 3.20 (s, 3H).

### Example 405

### 2-[3-(3-methoxy-4-nitrophenyl)-11-oxo-10,11-dihydro-5H-dibenzo[b,e][1,4]-diazepin-7-yl]N[(2S)-tetrahydrofuran-2-ylmethyl]acetamide

The desired product was prepared by substituting Example 389 and (2S)-tetrahydrofuran-2-ylmethylamine for dimethylaminoacetic acid and Example 120, respectively, in Example 122. MS (ESI) m/e 503 (M+H)⁺, ¹H NMR (500MHz, DMSO-d₆): δ 9.84 (s, 1H),

### Example 406

### 2-[3-(3-methoxy-4-nitrophenyl)-11-oxo-10,11-dihydro-5H-dibenzo[b,e][1,4]diazepin-7-yl]N(2-propoxyethyl)acetamide

The desired product was prepared by substituting Example 389 and 2-propoxyethylamine for dimethylaminoacetic acid and Example 120, respectively, in Example 122. MS (DCI) m/e 505 (M+H)⁺, ¹H NMR (500MHz, DMSO-d₆): δ 9.84 (s, 1H), 7.99-8.02 (m, 3H), 7.79 (d, J=8.11 Hz, 1H), 7.52 (d, J=1.25 Hz, 1H), 7.38 (d, J=1.37 Hz, 1H), 7.34 (dd, J=8.42, 1.56 Hz, 1H), 7.30 (dd, J=8.26, 1.72 Hz, 1H), 6.93 (d, J=1.25 Hz, 2H), 6.88 (d, J=8.11 Hz, 1H), 6.80 (dd, J=8.11, 1.56 Hz, 1H), 4.03 (s, 3H), 3.28-3.37 (m, 6H), 3.18 (q, J=5.82 Hz, 2H), 1.43-1.50 (m, 2H), 0.81 (t, J=7.33 Hz, 3H).

### Example 407

### 7-{2-[(2S)-2-(hydroxymethyl)pyrrolidin-1-yl]-2-oxoethyl}-3-(3-methoxy-4-nitrophenyl)-5,10-dihydro-11H-dibenzo[b,e][1,4]diazepin-11-one

The desired product was prepared by substituting Example 389 and (2S)-2-pyrrolidinylmethanol for dimethylaminoacetic acid and Example 120, respectively, in Example 122. MS (DCI) m/e 503 (M+H)⁺, ¹H NMR (500MHz, DMSO-d₆): δ 9.85 (s, 1H), 7.99-8.02 (m, 2H), 7.80 (d, J=8.11 Hz, 1H), 7.52 (s, 1H), 7.33-7.37 (m, 2H), 7.30 (d, J=8.42 Hz, 1H), 6.89-6.92 (m, 2H), 6.77 (d, J=7.80 Hz, 1H), 4.03 (s, 3H), 3.93 (m, 1H), 3.40-3.68 (m, 6H), 1.77-1.87 (m, 4H).

### Example 408

### 7-[2-(3-aminopyrrolidin-1-yl)-2-oxoethyl]-3-(3-methoxy-4-nitrophenyl)-5,10-dihydro-11H-dibenzo[b,e][1,4]diazepin-11-one

The desired product was prepared by substituting Example 389 and tert-butyl pyrrolidin-3-ylcarbamate for dimethylaminoacetic acid and Example 120, respectively, in Example 122. Isolated material was then treated with trifluoracetic acid at room temperature to provide the desired product. MS (DCI) m/e 488 (M+H)⁺, ¹H NMR (500MHz, DMSO-d₆): δ 9.87 (d, J=4.37 Hz, 1H), 8.00-8.02 (m, 4H), 7.81 (d, J=8.11 Hz, 1H), 7.52 (s, 1H), 7.38 (s, 1H), 7.34 (dd, J=8.42, 1.25 Hz, 1H), 7.31 (dd, J=8.11, 1.56 Hz, 1H), 6.90-6.94 (m, 2H), 6.77-6.81 (m, 1H), 4.03 (s, 3H), 3.65-3.85 (m, 2H), 3.47-3.56 (m, 4H), 1.84-2.27 (m, 3H).

### Example 409

### 3-(3-methoxy-4-nitrophenyl)-7-(2-oxo-2-piperazin-1-ylethyl)-5,10-dihydro-11H-dibenzo[b,e][1,4]diazepin-11-one

The desired product was prepared by substituting Example 389 and tert-butyl piperazine-1-carboxylate for dimethylaminoacetic acid and Example 120, respectively, in Example 122. Isolated material was then treated with trifluoracetic acid at room temperature to provide the desired product. MS (DCI) m/e 488 (M+H)⁺, ¹H NMR (500MHz, DMSO-d₆): δ 9.88 (s, 1H), 8.77 (s, 1H), 8.00-8.02 (m, 2H), 7.81 (d, J=8.42 Hz, 1H), 7.52 (d, J=1.56 Hz, 1H), 7.37 (d, J=1.56 Hz, 1H), 7.34 (dd, J=8.42, 1.87 Hz, 1H), 7.31 (dd, J=8.26, 1.72 Hz, 1H), 6.92 (d, J=8.11 Hz, 1H), 6.89 (d, J=1.56 Hz, 1H), 6.79 (dd, J=8.11, 1.56 Hz, 1H), 4.03 (s, 3H), 3.62-3.69 (m, 6H), 3.04-3.09 (m, 4H).

Example 410 to Example 439 were prepared using the same procedure as Example 80 to Example 118 except substituting Example 389 for Example 73.

### Example 410

### 2-[3-(3-methoxy-4-nitrophenyl)-11-oxo-10,11-dihydro-5H-dibenzo[b,e][1,4]diazepin-7-yl]N(3-methoxypropyl)acetamide

The desired product was prepared using 3-methoxypropylamine. MS (ESI) m/e 491 (M+H)⁺, ¹H NMR (500MHz, DMSO-d₆): δ 9.84 (s, 1H), 7.99-8.02 (m, 2H), 7.93 (t, J=5.61 Hz, 1H), 7.80 (d, J=8.11 Hz, 1H), 7.52 (d, J=1.56 Hz, 1H), 7.38 (d, J=1.56 Hz, 1H), 7.34 (dd, J=8.42, 1.87 Hz, 1H), 7.30 (dd, J=8.11, 1.87 Hz, 1H), 6.93 (d, J=1.56 Hz, 1H), 6.89 (d, J=8.11 Hz, 1H), 6.79 (dd, J=7.95, 1.72 Hz, 1H), 4.03 (s, 3H), 3.28 (d, J=6.24 Hz, 2H), 3.18 (s, 3H), 3.05-3.09 (m, 2H), 1.58-1.64 (m, 2H).

### Example 411

### N-(cyanomethyl)-2-[3-(3-methoxy-4-nitrophenyl)-11-oxo-10,11-dihydro-5H-dibenzo[b,e][1,4]diazepin-7-yl]acetamide

The desired product was prepared using aminoacetonitrile. MS (ESI) m/e 458 (M+H)⁺, ¹H NMR (500MHz, DMSO-d₆): δ 9.86 (s, 1H), 8.66 (t, J=5.46 Hz, 1H), 8.00-8.02 (m, 2H), 7.80 (d, J=8.42 Hz, 1H), 7.52 (d, J=1.56 Hz, 1H), 7.38 (d, J=1.56 Hz, 1H), 7.34 (dd, J=8.42, 1.56 Hz, 1H), 7.30 (dd, J=8.11, 1.56 Hz, 1H), 6.93 (d, J=1.56 Hz, 1H), 6.91 (d, J=8.11 Hz, 1H), 6.80 (dd, J=8.11, 1.56 Hz, 1H), 4.11 (d, J=5.61 Hz, 2H), 4.03 (s, 3H), 3.38 (s, 2H).

### Example 412

### N-(cyclopropylmethyl)-2-[3-(3-methoxy-4-nitrophenyl)-11-oxo-10,11-dihydro-5H-dibenzo[b,e][1,4]diazepin-7-yl]acetamide

The desired product was prepared using cyclopropylmethylamine. MS (ESI) m/e 473 (M+H)⁺, ¹H NMR (500MHz, DMSO-d₆): δ 9.84 (s, 1H), 7.99-8.05 (m, 3H), 7.80 (d, J=8.11 Hz, 1H), 7.52 (d, J=1.56 Hz, 1H), 7.38 (d, J=1.56 Hz, 1H), 7.34 (dd, J=8.42, 1.87 Hz, 1H), 7.30 (dd, J=8.11, 1.87 Hz, 1H), 6.95 (d, J=1.56 Hz, 1H), 6.89 (d, J=7.80 Hz, 1H), 6.80 (dd, J=8.11, 1.56 Hz, 1H), 4.04 (s, 3H), 2.92-2.94 (m, 2H), 2.50 (s, 2H), 0.88 (m, 1H), 0.37-0.41 (m, 2H), 0.12-0.15 (m, 2H).

### Example 413

### N-(1,3-dioxolan-2-ylmethyl)-2-[3-(3-methoxy-4-nitrophenyl)-11-oxo-10,11-dihydro-5H-dibenzo[b,e][1,4]diazepin-7-yl]Nmethylacetamide

The desired product was prepared using N-(1,3-dioxolan-2-ylmethyl)Nmethylamine. MS (ESI) m/e 519 (M+H)⁺, ¹H NMR (500MHz, DMSO-d₆): δ 9.85 (s, 1H), 7.99-8.02 (m, 2H), 7.80 (d, J=8.11 Hz, 1H), 7.52 (d, J=1.56 Hz, 1H), 7.37 (s, 1H), 7.34 (d, J=8.42 Hz, 1H), 7.30 (d, J=8.42 Hz, 1H), 6.88-6.91 (m, 2H), 6.76 (m, 1H), 4.92 (m, 1H), 4.03 (s, 3H), 3.86-3.93 (m, 2H), 3.75-3.83 (m, 2H), 3.59-3.62 (m, 2H), 3.52 (d, J=3.74 Hz, 1H), 3.40 (d, J=4.68 Hz, 2H), 3.03 (s, 1H), 2.88 (s, 1H).

### Example 414

### 3-(3-methoxy-4-nitrophenyl)-7-(2-oxo-2-thiomorpholin-4-ylethyl)-5,10-dihydro-11H-dibenzo[b,e][1,4]diazepin-11-one

The desired product was prepared using thiomorpholine. MS (ESI) m/e 505 (M+H)⁺, ¹H NMR (500MHz, DMSO-d₆): δ 9.86 (s, 1H), 8.00-8.02 (m, 2H), 7.80 (d, J=8.11 Hz, 1H), 7.52 (d, J=1.56 Hz, 1H), 7.30-7.37 (m, 3H), 6.90-6.93 (m, 2H), 6.79 (dd, J=8.11, 1.87 Hz, 1H), 4.03 (s, 3H), 3.67-3.76 (m, 4H), 3.61 (d, J=2.18 Hz, 2H), 2.52-2.54 (m, 2H), 2.45-2.47 (m, 2H).

### Example 415

### 2-[3-(3-methoxy-4-nitrophenyl)-11-oxo-10,11-dihydro-5H-dibenzo[b,e][1,41]diazepin-7-yl]N(2-pyridin-3-lethyl)acetamide

The desired product was prepared using 2-pyridin-3-ylethylamine. MS (ESI) m/e 524 (M+H)⁺, ¹H NMR (500MHz, DMSO-d₆): δ 9.85 (s, 1H), 8.64 (s, 1H), 8.05-8.07 (m, 2H), 7.98-8.02 (m, 2H), 7.80 (d, J=8.42 Hz, 1H), 7.67 (m, 1H), 7.52 (d, J=1.56 Hz, 1H), 7.38 (d, J=1.87 Hz, 1H), 7.34 (dd, J=8.42, 1.56 Hz, 1H), 7.31 (dd, J=8.26, 1.72 Hz, 1H), 6.87-6.90 (m, 2H), 6.72 (dd, J=8.11, 1.87 Hz, 1H), 4.03 (s, 3H), 3.33-3.37 (m, 2H), 3.24 (s, 2H), 2.85 (t, J=6.71 Hz, 2H).

### Example 416

### 2-[3-(3-methoxy-4-nitrophenyl)-11-oxo-10,11-dihydro-5H-dibenzo[b,e][1,4]diazepin-7-yl]N(2-pyridin-4-ylethyl)acetamide

The desired product was prepared using 2-pyridin-4-ylethylamine. MS (ESI) m/e 524 (M+H)⁺, ¹H NMR (500MHz, DMSO-d₆): δ 9.85 (s, 1H), 8.68 (d, J=6.24 Hz, 2H), 8.08 (t, J=5.61 Hz, 1H), 7.99-8.02 (m, 2H), 7.80 (d, J=8.11 Hz, 1H), 7.69 (d, J=6.24 Hz, 2H), 7.52 (d, J=1.56 Hz, 1H), 7.38 (d, J=1.56 Hz, 1H), 7.34 (dd, J=8.42, 1.56 Hz, 1H), 7.31 (dd, J=8.26, 1.72 Hz, 1H), 6.88-6.91 (m, 2H), 6.74 (dd, J=7.95, 1.72 Hz, 1H), 4.03 (s, 3H), 3.38-3.41 (m, 2H), 3.25 (s, 2H), 2.93 (t, J=6.86 Hz, 2H).

### Example 417

### N-[2-(2,3-dimethoxyphenyl)ethyl]-2-[3-(3-methoxy-4-nitrophenyl)-11-oxo-10,11-dihydro-5H-dibenzo[b,e][1,4]diazepin-7-yl]acetamide

The desired product was prepared using 2-(2,3-dimethoxyphenyl)ethylamine. MS (ESI) m/e 581 (M+H)⁺, ¹H NMR (500MHz, DMSO-d₆): δ 9.85 (s, 1H), 7.99-8.04 (m, 3H), 7.80 (d, J=8.42 Hz, 1H), 7.52 (d, J=1.56 Hz, 1H), 7.38 (d, J=1.56 Hz, 1H), 7.34 (dd, J=8.42, 1.87 Hz, 1H), 7.31 (dd, J=8.26, 1.72 Hz, 1H), 6.84-6.93 (m, 4H), 6.77 (dd, J=7.95, 1.72 Hz, 1H), 6.68 (dd, J=7.64, 1.40 Hz, 1H), 4.03 (s, 3H), 3.76 (s, 3H), 3.69 (s, 3H), 3.27 (s, 2H), 3.19-3.23 (m, 2H), 2.66-2.69 (m, 2H).

### Example 418

### N-[2-(1,3-benzodioxol-5-yl)ethyl]-2-[3-(3-methoxy-4-nitrophenyl)-11-oxo-10,11-dihydro-5H-dibenzo[b,e][1,4]diazepin-7-yl]acetamide

The desired product was prepared using 2-benzo[1,3]dioxol-5-yl-ethylamine. MS (ESI) m/e 567 (M+H)⁺, ¹H NMR (500MHz, DMSO-d₆): δ 9.84 (s, 1H), 7.96-8.01 (m, 3H), 7.80 (d, J=8.11 Hz, 1H), 7.52 (d, J=1.56 Hz, 1H), 7.38 (d, J=1.56 Hz, 1H), 7.34 (dd, J=8.42, 1.87 Hz, 1H), 7.30 (dd, J=8.26, 1.72 Hz, 1H), 6.92 (d, J=1.87 Hz, 1H), 6.88 (d, J=8.11 Hz, 1H), 6.74-6.76 (m, 3H), 6.59 (dd, J=7.80, 1.56 Hz, 1H), 5.93 (s, 2H), 4.03 (s, 3H), 3.26 (s, 2H), 3.19-3.23 (m, 2H), 2.60 (t, J=7.33 Hz, 2H).

### Example 419

### 2-[3-(3-methoxy-4-nitrophenyl)-11-oxo-10,11-dihydro-5H-dibenzo[b,e][1,4]diazepin-7-yl]N(thien-2-ylmethyl)acetamide

The desired product was prepared using thien-2-ylmethylamine. MS (ESI) m/e 515 (M+H)⁺, ¹H NMR (500Hz, DMSO-d₆): δ 9.85 (s, 1H), 8.56 (t, J=5.77 Hz, 1H), 8.00-8.02 (m, 2H), 7.80 (d, J=8.42 Hz, 1H), 7.52 (d, J=1.56 Hz, 1H), 7.38 (d, J=1.87 Hz, 1H), 7.34-7.36 (m, 2H), 7.30 (dd, J=8.26, 1.72 Hz, 1H), 6.89-6.95 (m, 4H), 6.81 (dd, J=8.11, 1.87 Hz, 1H), 4.41 (d, J=5.93 Hz, 2H), 4.03 (s, 3H), 3.34 (s, 2H).

### Example 420

### 2-[3-(3-methoxy-4-nitrophenyl)-11-oxo-10,11-dihydro-5H-dibenzo[b,e][1,4]diazopin-7-yl]N(1,3-thiazol-5-ylmethyl)acetamide

The desired product was prepared using 1,3-thiazol-5-ylmethylamine. MS (ESI) m/e 516 (M+H)⁺, ¹H NMR (500MHz, DMSO-d₆): δ 9.85 (s, 1H), 9.04 (d, J=1.87 Hz, 1H), 8.51 (t, J=5.46 Hz, 1H), 8.00-8.02 (m, 2H), 7.80 (d, J=8.11 Hz, 1H), 7.52 (d, J=1.56 Hz, 1H), 7.34-7.38 (m, 3H), 7.30 (dd, J=8.26, 1.72 Hz, 1H), 6.79-6.96 (m, 3H), 4.39 (d, J=5.61 Hz, 2H), 4.03 (s, 3H), 3.38 (s, 2H).

### Example 421

### N-[2-(1H-imidazol-4-yl)ethyl]-2-[3-(3-methoxy-4-nitrophenyl)-11-oxo-10,11-dihydro-5H-dibenzo[b,e][1,4]diazepin-7-yl]acetamide

The desired product was prepared using 2-(1H-imidazol-4-yl)ethylamine. MS (ESI) m/e 513 (M+H)⁺, ¹H NMR (500MHz, DMSO-d₆): δ 9.85 (s, 1H), 8.96 (d, J=1.25 Hz, 1H), 8.12 (t, J=5.77 Hz, 1H), 7.99-8.02 (m, 2H), 7.80 (d, J=8.11 Hz, 1H), 7.52 (d, J=1.56 Hz, 1H), 7.38-7.40 (m, 2H), 7.34 (dd, J=8.42, 1.56 Hz, 1H), 7.31 (dd, J=8.27, 1.72 Hz, 1H), 6.91 (d, J=1.56 Hz, 1H), 6.89 (d, J=8.11 Hz, 1H), 6.75 (dd, J=8.11, 1.56 Hz, 1H), 4.03 (s, 3H), 3.31-3.35 (m, 2H), 3.27 (s, 2H), 3.17 (s, 1H), 2.77 (t, J=6.86 Hz, 2H).

### Example 422

### 7-[2-(1,4-dioxa-8-azaspiro[4.5]dec-8-yl)-2-oxoethyl]-3-(3-methoxy-4-nitrophenyl)-5,10-dihydro-11H-dibenzo[b,e][1,4]diazepin-11-one

The desired product was prepared using 1,4-dioxa-8-azaspiro[4.5]decane. MS (ESI) m/e 545 (M+H)⁺, ¹H NMR (500MHz, DMSO-d₆): δ 9.84 (s, 1H), 7.99-8.01 (m, 2H), 7.80 (d, J=8.11 Hz, 1H), 7.53 (d, J=1.25 Hz, 1H), 7.37 (d, J=1.56 Hz, 1H), 7.35 (dd, J=8.42, 1.56 Hz, 1H), 7.30 (dd, J=8.11, 1.56 Hz, 1H), 6.90-6.92 (m, 2H), 6.79 (d, J=8.11 Hz, 1H), 4.06 (s, 3H), 3.89 (s, 4H), 3.62 (s, 2H), 3.53 (d, J=5.61 Hz, 4H), 1.48-1.55 (m, 4H).

### Example 423

### 7-{2-[2,6-dimethylmorpholin-4-yl]-2-oxoethyl}-3-(3-methoxy-4-nitrophenyl)-5,10-dihydro-11H-dibenzo[b,e][1,4]diazepin-11-one

The desired product was prepared using 2,6-dimethylmorpholine. MS (ESI) m/e 545 (M+H)⁺, ¹H NMR (500MHz, DMSO-d₆): δ 9.86 (s, 1H), 7.99-8.02 (m, 2H), 7.80 (d, J=8.11 Hz, 1H), 7.53 (d, J=1.56 Hz, 1H), 7.39 (d, J=1.56 Hz, 1H), 7.34 (dd, J=8.58, 1.72 Hz, 1H), 7.29 (dd, J=8.27, 1.72 Hz, 1H), 6.89-6.93 (m, 2H), 6.80 (m, 1H), 4.26 (d, J=13.10 Hz, 1H), 4.03 (s, 3H), 3.84 (d, J=13.10 Hz, 1H), 3.58 (m, 3H), 3.16 (m, 1H), 2.64 (dd, J=12.94, 10.76 Hz, 1H), 2.25 (dd, J=12.94, 11.07 Hz, 1H), 1.06 (m, 6H).

### Example 424

### 7-[2-(4-acetylpiperazin-1-yl)-2-oxoethyl]-3-(3-methoxy-4-nitxophenyl)-5,10-dihydro-11H-dibenzo[b,e][1,4]diazepin-11-one

The desired product was prepared using 1-acetylpiperazine. MS (ESI) m/e 530 (M+H)⁺, ¹H NMR (500MHz, DMSO-d₆): δ 9.83 (s, 1H), 7.99-8.02 (m, 2H), 7.81 (d, J=8.11 Hz, 1H), 7.51 (d, J=1.25 Hz, 1H), 7.37 (d, J=1.56 Hz, 1H), 7.34 (dd, J=8.42, 1.56 Hz, 1H), 7.29 (dd, J=8.11, 1.87 Hz, 1H), 6.90-6.92 (m, 2H), 6.79 (m, 1H), 4.08 (s, 3H), 3.64 (d, J=7.17 Hz, 2H), 3.35-3.51 (m, 8H), 1.97 (d, J=3.12 Hz, 3H).

### Example 425

### 3-(3-methoxy-4-nitrophenyl)-7-[2-oxo-2-(4-pyridin-2-ylpiperazin-1-yl)ethyl]-5,10-dihydro-11H-dibenzo[b,e][1,4]diazepin-11-one

The desired product was prepared using 1-pyridin-2-yl-piperazine. MS (ESI) m/e 565 (M+H)⁺, ¹H NMR (500MHz, DMSO-d₆): δ 9.87 (s, 1H), 8.06 (dd, J=5.46, 1.40 Hz, 1H), 7.99-8.01 (m, 2H), 7.81 (d, J=8.11 Hz, 1H), 7.75 (t, J=7.33 Hz, 1H), 7.52 (d, J=1.56 Hz, 1H), 7.36 (d, J=1.87 Hz, 1H), 7.33 (dd, J=8.42, 1.56 Hz, 1H), 7.30 (dd, J=8.26, 1.72 Hz, 1H), 7.05 (d, J=8.11 Hz, 1H), 6.91-6.93 (m, 2H), 6.78-6.82 (m, 2H), 4.03 (s, 3H), 3.67 (s, 2H), 3.60-3.65 (m, 4H), 3.54-3.59 (m, 4H).

### Example 426

### 3-({3-(3-methoxy-4-nitrophenyl)-11-oxo-10,11-dihydro-5H-dibenzo[b,e][1,41diazepin-7-yl]acetyl}amino)benzamide

The desired product was prepared using 3-aminobenzamide. MS (ESI) m/e 537 (M+H)⁺, ¹H NMR (500MHz, DMSO-d₆): δ 10.23 (s, 1H), 9.86 (s, 1H), 7.99-8.03 (m, 3H), 7.80 (d, J=8.42 Hz, 1H), 7.76 (dd, J=8.11, 1.25, 1H), 7.51-7.53 (m, 2H), 7.34-7.38 (m, 3H), 7.27-7.31 (m, 2H), 7.00 (d, J=1.25 Hz, 1H), 6.87-6.94 (m, 2H), 4.03 (s, 3H), 3.54 (s, 2H).

### Example 427

### 2-[3-(3-methoxy-4-nitrophenyl)-11-oxo-10,11-dihydro-5H-dibenzo[b,e][1,4]diazepin-7-yl]N(4-morpholin-4-ylphenyl)acetamide

The desired product was prepared using 4-morpholin-4-ylphenylamine. MS (ESI) m/e 580 (M+H)⁺, ¹H NMR (500MHz, DMSO-d₆): δ 9.89 (s, 1H), 9.86 (s, 1H), 7.99-8.02 (m, 2H), 7.80 (d, J=8.11 Hz, 1H), 7.52 (d, J=1.56 Hz, 1H), 7.45 (d, J=9.36 Hz, 2H), 7.37 (d, J=1.56 Hz, 1H), 7.34 (dd, J=8.42, 1.56 Hz, 1H), 7.30 (dd, J=8.27, 1.72 Hz, 1H), 6.99 (d, J=1.56 Hz, 1H), 6.85-6.93 (m, 4H), 4.03 (s, 3H), 3.71-3.73 (m, 4H), 3.48 (s, 2H), 3.01-3.03 (m, 4H).

### Example 428

### 2-[3-(3-methoxy-4-nitrophenyl)-11-oxo-10,11-dihydro-5H-dibenzo[b,e][1,4]diazepin-7-yl]Nquinolin-6-ylacetamide

The desired product was prepared using quinolin-6-ylamine. MS (ESI) m/e 546 (M+H)⁺, ¹H NMR (500MHz, DMSO-d₆): δ 10.52 (s, 1H), 9.88 (s, 1H), 8.85 (m, 1H), 8.39-8.42 (m, 2H), 8.04 (s, 1H), 7.99-8.02 (m, 2H), 7.88 (dd, J=9.20, 2.03 Hz, 1H), 7.80 (d, J=8.11 Hz, 1H), 7.55-7.58 (m, 1H), 7.52 (d, J=1.56 Hz, 1H), 7.37 (d, J=1.87 Hz, 1H), 7.34 (dd, J=8.42, 1.87 Hz, 1H), 7.30 (dd, J=8.11, 1.87 Hz, 1H), 7.04 (m, 1H), 6.90-6.95 (m, 2H), 4.02 (s, 3H), 3.62 (s, 2H).

### Example 429

### N-[(1S)-1-(hydroxymethyl)-2-methylpropyl]-2-[3-(3-methoxy-4-nitrophenyl)-11-oxo-10,11-dihydro-5H-dibenzo[b,e][1,4]diazepin-7-yl]acetamide

The desired product was prepared using (S)-2-amino-3-methyl-1-butanol. MS (ESI) m/e 505 (M+H)⁺, ¹H NMR (500MHz, DMSO-d₆): δ 9.83 (s, 1H), 8.00-8.02 (m, 2H), 7.98 (s, 1H), 7.80 (d, J=8.11 Hz, 1H), 7.62 (d, J=9.04 Hz, 1H), 7.52 (d, J=1.25 Hz, 1H), 7.38 (d, J=1.56 Hz, 1H), 7.34 (dd, J=8.42, 1.87 Hz, 1H), 7.31 (dd, J=8.26, 1.72 Hz, 1H), 6.94 (d, J=1.56 Hz, 1H), 6.81-6.89 (m, 2H), 4.03 (s, 3H), 3.52-3.56 (m, 2H), 3.29-3.37 (m, 3H), 1.81 (m, 1H), 0.81 (dd, J=9.04, 6.86 Hz, 6H).

### Example 430

### (2S)-2-({[3-(3-methoxy-4-nitrophenyl)-11-oxo-10,11-dihydro-5H-dibenzo[b,e][1,4]diazepin-7-yl]acetyl}amino)-4-methylpentanamide

The desired product was prepared using L-leucine amide. MS (ESI) m/e 530 (M+H)⁺, ¹H NMR (500MHz, DMSO-d₆): δ 9.83 (s, 1H), 8.00-8.02 (m, 2H), 7.98 (s, 1H), 7.80 (d, J=8.11 Hz, 1H), 7.52 (d, J=1.56 Hz, 1H), 7.38 (d, J=1.56 Hz, 1H), 7.34 (dd, J=8.42, 1.56 Hz, 1H), 7.29-7.31 (m, 2H), 6.87-6.93 (m, 3H), 6.82 (m, 1H), 4.11 (m, 1H), 4.03 (s, 3H), 3.34-3.39 (m, 4H), 1.55 (m, 1H), 0.81 (d, J=6.85 Hz, 3H), 0.79 (d, J=6.55 Hz, 3H).

### Example 431

### N-[(1S)-2-hydroxy-1-phenylethyl]-2-[3-(3-methoxy-4-nitrophenyl)-11-oxo-10,11-dihydro-5H-dibenzo[b,e][1,4]diazepin-7-yl]acetamide

The desired product was prepared using (S)-phenylglycinol. MS (ESI) m/e 539 (M+H)⁺, ¹H NMR (500MHz, DMSO-d₆): δ 9.83 (s, 1H), 8.37 (d, J=8.11 Hz, 1H), 8.02 (d, J=8.42 Hz, 1H), 7.96 (s, 1H), 7.80 (d, J=8.11 Hz, 1H), 7.52 (d, J=1.56 Hz, 1H), 7.38 (d, J=1.87 Hz, 1H), 7.34 (dd, J=8.58, 1.72 Hz, 1H), 7.31 (dd, J=8.27, 1.72 Hz, 1H), 7.24-7.28 (m, 3H), 7.17 (m, 1H), 6.87-6.93 (m, 2H), 6.82 (m, 1H), 4.81 (m, 1H), 4.03 (s, 3H), 3.54-3.57 (m, 2H), 3.39 (d, J=3.12 Hz, 2H).

### Example 432

### N-(2,3-dihydroxypropyl)-2-[3-(3-methoxy-4-nitrophenyl)-11-oxo-10,11-dihydro-5H-dibenzo[b,e][1,4]diazepin-7-yl]acetamide

The desired product was prepared using 3-amino-propane-1,2-diol. MS (ESI) m/e 491 (M+H)⁺.

### Example 433

### N-[3-(1H-imidazol-1-yl)propyl]-2-[3-(3-methoxy-4-nitrophenyl)-11-oxo-10,11-dihdyro-5H-dibenzo[b,e][1,4]diazepin-7-yl]acetamide

The desired product was prepared using 3-(1H-imidazol-1-yl)propylamine. MS (ESI) m/e 527 (M+H)⁺.

### Example 434

### 2-[3-(3-methoxy-4-nitrophenyl)-11-oxo-10,11-dihydro-5H-dibenzo[b,e][1,4]diazepin-7-yl]N[3-(2-oxopyrrolidin-1-yl)propyl]acetamide

The desired product was prepared using 1-(3-aminopropyl)pyrrolidin-2-one. MS (ESI) m/e 544 (M+H)⁺.

### Example 435

### N-(2,6-difluorobenzyl)-2-[3-(3-methoxy-4-nitrophenyl)-11-oxo-10,11-dihydro-5H-dibenzo[b,e][1,4]diazepin-7-yl]acetamide

The desired product was prepared using 2,6-difluorobenzylamine. MS (ESI) m/e 545 (M+H)⁺.

### Example 436 (A-801182.0)

### N-{2-[4-(aminosulfonyl)phenyl]ethyl}-2-[3-(3-methoxy-4-nitrophenyl)-11-oxo-10,11-dihydro-5H-dibenzo[b,e][1,4]diazepin-7-yl]acetamide

The desired product was prepared using 4-(2-aminoethyl)benzenesulfonamide. MS (ESI) m/e 602 (M+H)⁺.

### Example 437

### N-[(1R)-1-(hydroxymethyl)-2-methylpropyl]-2-[3-(3-methoxy-4-nitrophenyl)-11-oxo-10,11-dihydro-5H-dibenzo[b,e][1,4]diazepin-7-yl]acetamide

The desired product was prepared using (R)-2-amino-3-methyl-1-butanol. MS (ESI) m/e 505 (M+H)⁺.

### Example 438

### N-[(1R)-2-hydroxy-1-phenylethyl]-2-[3-(3-methoxy-4-nitrophenyl)-11-oxo-10,11-dihydro-5H-dibenzo[b,e][1,4]diazepin-7-yl]acetamide

The desired product was prepared using (R)-phenylglycinol. MS (ESI) m/e 539 (M+H)⁺.

### Example 439

### 2-[3-(3-methoxy-4-nitrophenyl)-11-oxo-10,11-dihydro-5H-dibenzo[b,e][1,4]diazepin-7-yl]N(thien-3-ylmethyl)acetamide

The desired product was prepared using thien-3-ylmethylamine. MS (ESI) m/e 515 (M+H)⁺.

### Example 440

### methyl {3-[4-(aminocarbonyl)-3-methoxyphenyl]-11-oxo-10,11-dihydro-5H-dibenzo[b,e][1,4]diazepin-8-yl}acetate

The desired product was prepared by substituting Example 54A and 4-chloro-2-methoxybenzamide for Example 56A and Example 59B, respectively, in Example 59C. MS (DCI) m/e 432 (M+H)⁺, ¹H NMR (500MHz, DMSO-d₆): δ 9.84 (s, 1H), 7.96 (s, 1H), 7.90 (d, J=8.11 Hz, 1H), 7.78 (d, J=8.11 Hz, 1H), 7.66 (s, 1H), 7.54 (s, 1H), 7.34 (d, J=1.56 Hz, 1H), 7.32 (d, J=1.32 Hz, 1H), 7.26-7.29 (m, 2H), 6.96 (d, J=7.80 Hz, 1H), 6.84-6.87 (m, 2H), 3.99 (s, 3H), 3.60 (s, 3H), 3.54 (s, 2H).

### Example 441

### 8-hydroxy-3-(3-methoxy-4-nitrophenyl)-5,10-dihydro-11H-dibenzo[b,e][1,4]diazepin-11-one

The desired product was prepared by substituting Example 8C and Example 266G for Example 59B and Example 56A, respectively, in Example 59C. MS (DCI) m/e 378 (M+H)⁺, ¹H NMR (500MHz, DMSO-d₆): δ 9.80 (s, 1H), 9.12 (s, 1H), 8.00 (d, J=8.42 Hz, 1H), 7.77 (d, J=8.11 Hz, 1H), 7.66 (s, 1H), 7.52 (d, J=1.56 Hz, 1H), 7.34 (dd, J=8.58, 1.72 Hz, 1H), 7.32 (d, J=1.56 Hz, 1H), 7.26 (dd, J=8.27, 1.72 Hz, 1H), 6.82 (d, J=8.73 Hz, 1H), 6.45 (d, J=2.50 Hz, 1H), 6.38 (dd, J=8.58, 2.65 Hz, 1H), 4.03 (s, 3H).

### Example 442

### 8-methoxy-3-(3-methoxy-4-nitrohenyl)-5,10-dihydro-11H-dibenzo[b,e][1,4]diazepin-11-one

### Example 442A

### methyl 4-chloro-2-[(4-methoxy-2-nitrophenyl)amino]benzoate

The desired product was prepared by substituting 4-methoxy-2-nitroaniline for methyl 3,4-diaminobenzoate in Example 1A. MS (DCI) m/e 337 (M+H)⁺, ¹H NMR (500MHz, DMSO-d₆): δ 10.46 (s, 1H), 7.93 (d, J=8.42 Hz, 1H), 7.65 (d, J=9.05 Hz, 1H), 7.62 (d, J=2.81 Hz, 1H), 7.37 (dd, J=9.20, 2.96 Hz, 1H), 7.20 (d, J=1.87 Hz, 1H), 6.99 (dd, J=8.58, 2.03 Hz, 1H), 3.89 (s, 3H), 3.86 (s, 3H).

### Example 442B

### methyl 2-[(2-amino-4-methoxyphenyl)amino]-4-chlorobenzoate

The desired product was prepared by substituting Example 442A for Example 6B in Example 6C. MS (DCI) m/e 307 (M+H)⁺, ¹H NMR (500MHz, DMSO-d₆): δ 8.70 (s, 1H), 7.82 (d, J=8.73 Hz, 1H), 6.90 (d, J=8.42 Hz, 1H), 6.67 (dd, J=8.58, 2.03 Hz, 1H), 6.40 (d, J=2.81 Hz, 1H), 6.32 (d, J=2.18 Hz, 1H), 6.19 (dd, J=8.73, 2.81 Hz, 1H), 5.00 (s, 2H), 3.85 (s, 3H), 3.70 (s, 3H).

### Example 442C

### 3-chloro-8-methoxy-5,10-dihydro-11H-dibenzo[b,e][1,4]diazepin-11-one

Example 442B was substituted for Example 12 in Example 13 to provide 2-(2-amino-4-methoxy-phenylamino)-4-chloro-benzoic acid. This material was then treated with 3 equivalents of HATU in DMF to provide the desired product. MS (DCI) m/e 275 (M+H)⁺, ¹H NMR (300MHz, DMSO-d₆): δ 9.84 (s, 1H), 7.86 (s, 1H), 7.67 (d, J=8.48 Hz, 1H), 7.03 (d, J=2.03 Hz, 1H), 6.87-6.92 (m, 2H), 6.57-6.59 (m, 2H), 3.86 (s, 3H).

### Example 442D

### 8-methoxy-3-(3-methoxy-4-nitrophenyl)-5,10-dihydro-11H-dibenzo[b,e][1,4]diazepin-11-one

The desired product was prepared by substituting Example 442C and Example 266G for Example 59B and Example 56A, respectively, in Example 59C. MS (DCI) m/e 392 (M+H)⁺, ¹H NMR (500MHz, DMSO-d₆): δ 9.83 (s, 1H), 8.01 (d, J=8.29 Hz, 1H), 7.78-7.81 (m, 2H), 7.52 (d, J=1.53 Hz, 1H), 7.32-7.35 (m, 2H), 7.28 (dd, J=8.29, 1.53 Hz, 1H), 6.94 (d, J=8.29 Hz, 1H), 6.57-6.61 (m, 2H), 4.03 (s, 3H), 3.67 (s, 3H).

### Example 443

### 8-ethoxy-3-(3-methoxy-4-nitrophenyl)-5,10-dihydro-11H-dibenzo[b,e][1,4]diazepin-11-one

The desired product was prepared in the same manner as Example 442, beginning with the substitution of 4-ethoxy-2-nitroaniline for 4-methoxy-2-nitroaniline in Example 442A. MS (DCI) m/e 405 (M+H)⁺, ¹H NMR (500MHz, DMSO-d₆): δ 9.82 (s, 1H), 8.01 (d, J=8.42 Hz, 1H), 7.78-7.81 (m, 2H), 7.52 (s, 1H), 7.33-7.35 (m, 2H), 7.28 (d, J=8.11 Hz, 1H), 6.92 (d, J=8.42 Hz, 1H), 6.55-6.59 (m, 2H), 4.03 (s, 3H), 3.92 (d, J=8.42 Hz, 2H), 1.29 (t, J=7.02 Hz, 3H).

### Example 444

### 3-(3-methoxy-4-nitrohenyl)-8-[2-(4-methyl-1,3-thiazol-5-yl)ethoxy]-5,10-dihydro-11H-dibenzo[b,e][1,4]diazepin-11-one

### Example 444A

### 4-[2-(4-methyl-1,3-thiazol-5-yl)ethoxy]-2-nitroaniline

A mixture of 4-amino-3-nitrophenol (386mg, 2.5 mmol), 2-(4-methyl-1,3-thiazol-5-yl)ethanol (0.299 mL, 2.5 mmol), polymer supported triphenylphosphine (1.25g, 3mmol/g, 3.75 mmol), and di-tert-butyl azodicarboxylate (864mg, 3.75mmol) in THF (10 mL) was stirred at room temperature for 16 hours. Filtered through Celite and concentrated under vacuum. The residue was purified by flash column chromatography on silica gel with 1:1 hexanes/ethyl acetate to provide 602mg (86%) of the desired product. MS (DCI) m/e 279 (M+H)⁺, ¹H NMR (300MHz, DMSO-d₆): δ 8.83 (s, 1H), 7.37 (d, J=3.05 Hz, 1H), 7.25 (s, 2H), 7.16 (dd, J=8.99, 2.88 Hz, 1H), 7.01 (s, 1H), 4.10 (t, J=6.27 Hz, 2H), 3.19 (t, J=6.27 Hz, 2H), 2.35 (s, 3H).

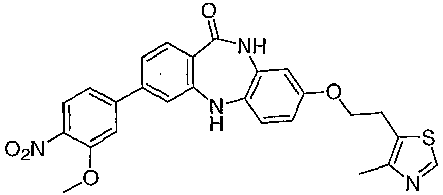

### Example 444B

### 3-(3-methoxy-4-nitrophenyl)-8-[2-(4-methyl-1,3-thiazol-5-yl)ethoxy]-5,10-dihydro-11H-dibenzo[b,e][1,4]diazepin-11-one

The desired product was prepared in the same manner as Example 442, beginning with the substitution of Example 444A for 4-methoxy-2-nitroaniline in Example 442A. MS (ESI) m/e 503 (M+H)⁺, ¹H NMR (400MHz, DMSO-d₆): δ 9.82 (s, 1H), 8.82 (s, 1H), 8.00 (s, J=8.59 Hz, 1H), 7.83 (s, 1H), 7.79 (d, J=7.98 Hz, 1H), 7.52 (d, J=1.23 Hz, 1H), 7.31-7.35 (m, 2H), 7.28 (dd, J=8.13, 1.38 Hz, 1H), 6.93 (d, J=8.59 Hz, 1H), 6.55-6.62 (m, 2H), 4.03-4.07 (m, 5H), 3.18 (t, J=6.14 Hz, 2H), 2.34 (s, 3H).

### Example 445

### 8-[3-(dimethylamino)propoxy]-3-(3-methoxy-4-nitrophenyl)-5,10-dihydro-11H-dibenzo[b,e][1,4]diazoin-11-one

The desired product was prepared in the same manner as Example 444, beginning with the substitution of 3-(dimethylamino)propan-1-ol for 2-(4-methyl-1,3-thiazol-5-yl)ethanol in Example 444A. MS (DCI) m/e 463 (M+H)⁺, ¹H NMR (400MHz, DMSO-d₆): δ 9.86 (s, 1H), 8.00 (d, J=8.59 Hz, 1H), 7.84 (s, 1H), 7.78 (d, J=8.29 Hz, 1H), 7.51 (d, J=1.53 Hz, 1H), 7.33 (m, 2H), 7.28 (dd, J=8.13, 1.69 Hz, 1H), 6.94 (d, J=9.51 Hz, 1H), 6.58-6.61 (m, 2H), 3.92-4.06 (m, 5H), 3.16 (d, J=4.60 Hz, 2H), 2.78 (s, 6H), 2.01-2.04 (m, 2H).

### Example 446

### 3-(3-methoxy-4-nitrophenyl)-8-(2-morpholin-4-ylethoxy)-5,10-dihydro-11H-dibenzo[b,e][1,4]diazepin-11-one

The desired product was prepared in the same manner as Example 444, beginning with the substitution of 2-morpholin-4-ylethanol for 2-(4-methyl-1,3-thiazol-5-yl)ethanol in Example 444A. MS (DCI) m/e 491 (M+H)⁺, ¹H NMR (400MHz, DMSO-d₆): δ 9.89 (s, 1H), 8.01 (d, J=8.42 Hz, 1H), 7.88 (s, 1H), 7.80 (d, J=8.42 Hz, 1H), 7.52 (d, J=1.25 Hz, 1H), 7.33-7.35 (m, 2H), 7.29 (dd, J=8.27, 1.40 Hz, 1H), 6.98 (m, 1H), 6.65-6.68 (m, 2H), 4.21-4.24 (m, 2H), 3.99-4.07 (m, 5H), 3.64-3.76 (m, 2H), 3.47-3.56 (m, 2H), 3.24-3.37 (m, 4H).

### Example 447

### 3-(3-methoxy-4-nitrophenyl)-8-[2-(4-morpholin-4-ylphenyl)ethoxy]-5,10-dihydro-11H-dibenzo[b,e][1,4]diazepin-11-one

### Example 447A

### 2-(4-morpholin-4-ylphenyl)ethanol

A mixture of 2-(4-bromophenyl)ethanol (0.70 mL, 5 mmol), morpholine (0.52 mL, 6 mmol), LHMDS (11 mL, 1M solution in THF), Pd₂(dba)₃ (46mg, 0.05 mmol), and CyMAP (24mg, 0.06 mmol) was heated to reflux for 16 hours. Cooled to room temperature. Acidified with 1M HCl, stirred for 10 minutes, and neutralized with saturated NaHCO₃. Partitioned between ethyl acetate and water. The organic layer was washed with brine, dried (MgSO₄), filtered, and concentrated under vacuum. The residue was purified by flash column chromatography on silica gel with 1:1 hexanes/ethyl acetate to provide 520mg (50 %) of the desired product. MS (ESI) m/e 208 (M+H)⁺, ¹H NMR (300MHz, CDCl₃): δ 7.15 (d, J=8.48 Hz, 2H), 6.88 (d, J=8.48 Hz, 2H), 3.78-3.87 (m, 6H), 3.11-3.14 (m, 4H), 2.79 (t, J=6.61 Hz, 2H), 1.43 (m, 1H).

### Example 447B

### 4-[2-(4-morpholin-4-ylphenyl)ethoxy]-2-nitroaniline

The desired product was prepared by substituting Example 447A for 2-(4-methyl-1,3-thiazol-5-yl)ethanol in Example 444A. MS (ESI) m/e 344 (M+H)⁺.

### Example 447C (A-845393.0)

### 3-(3-methoxy-4-nitrophenyl)-8-[2-(4-morpholin-4-ylphenyl)ethoxy]-5,10-dihydro-11H-dibenzo[b,e][1,4]diazepin-11-one

The desired product was prepared in the same manner as Example 442, beginning with the substitution of Example 447B for 4-methoxy-2-nitroaniline in Example 442A. MS (ESI) m/e 567 (M+H)⁺, ¹H NMR (300MHz, DMSO-d₆): δ 9.82 (s, 1H), 8.00 (m, 1H), 7.77-7.82 (m, 2H), 7.52 (d, J=2.03 Hz, 1H), 7.26-7.35 (m, 4H), 7.14-7.17 (m, 2H), 6.86-6.91 (m, 2H), 6.57-6.61 (m, 2H), 4.02-4.05 (m, 5H), 3.71-3.74 (m, 4H), 3.28-3.30 (m, 2H), 3.04-3.08 (m, 4H).

### Example 448

### 3-(3-methoxy-4-nitrophenyl)-7-piperidin-1-yl-5,10-dihydro-11H-dibenzo[b,e][1,4]diazepin-11-one

### Example 448A

### 2-nitro-5-piperidin-1-ylhenylamine

A mixture of 5-chloro-2-nitroaniline (1.73g, 10 mmol), piperidine (1.09 mL, 11 mmol), and K₂CO₃ (1.52g, 11 mmol) in DMF was heated to 120 °C for 24 hours. Cooled to room temperature. Partitioned between ethyl acetate and water. The organic layer was washed with brine, dried (MgSO₄), filtered, and concentrated under vacuum. The residue was purified by flash column chromatography on silica gel with 4:1 hexanes/ethyl acetate to provide 1.33g (60%) of the desired product. MS (ESI) m/e 222 (M+H)⁺, ¹H NMR (300MHz, DMSO-d₆): δ 7.79 (d, J=9.83 Hz, 1H), 7.22 (s, 2H), 6.37 (dd, J=9.83, 2.71 Hz, 1H), 6.19 (d, J=2.71 Hz, 1H), 3.34-3.38 (m, 4H), 1.53-1.60 (m, 6H).

### Example 448B

### 3-chloro-7-piperidin-1-yl-5,10-dihydro-11H-dibenzo[b,e][1,4]diazepin-11-one

Example 448A was substituted for methyl 3,4-diaminobenzoate in Example 1A to provide 4-chloro-2-(2-nitro-5-piperidin-1-yl-phenylamino)-benzoic acid methyl ester. This material was then substituted for Example 6B in Example 6C to provide 2-(2-amino-5-piperidin-1-yl-phenylamino)-4-chloro-benzoic acid methyl ester. Subsequently, this material was substituted for Example 442B in Example 442C to provide the desired product. MS (ESI) m/e 328 (M+H)⁺.

### Example 448C

### 3-(3-methoxy-4-nitrophenyl)-7-piperidin-1-yl-5,10-dihydro-11H-dibenzo[b,e][1,4]diazepin-11-one

The desired product was prepared by substituting Example 448B and Example 266G for Example 59B and Example 56A, respectively, in Example 59C. MS (ESI) m/e 445 (M+H)⁺, ¹H NMR (400MHz, DMSO-d₆): δ 9.67 (s, 1H), 8.00 (d, J=8.59 Hz, 1H), 7.77-7.80 (m, 2H), 7.52 (d, J=1.84 Hz, 1H), 7.33-7.36 (m, 2H), 7.30 (dd, J=8.13, 1.69 Hz, 1H), 6.81 (d, J=8.59 Hz, 1H), 6.62 (d, J=2.76 Hz, 1H), 6.52 (dd, J=8.59, 2.76 Hz, 1H), 4.03 (s, 3H), 3.03-3.06 (m, 4H), 1.58-1.62 (m, 4H), 1.49-1.54 (m, 2H).

### Example 449

### 7-[(2S)-2-(hydroxymethyl)pyrrolidin-1-yl]-3-(3-methoxy-4-nitrophenyl)-5,10-dihydro-11H-dibenzo[b,e][1,4]diazepin-11-one

### Example 449A

### [(2S)-1-(3-amino-4-nitrophenyl)pyrrolidin-2-yl]methanol

The desired product was prepared by substituting (2S)-2-pyrrolidinylmethanol for piperidine in Example 448A. MS (DCI) m/e 238 (M+H)⁺.

### Example 449B

### 3-chloro-7-[(2S)-2-(hydroxymethyl)pyrrolidin-1-yl]-5,10-dihydro-11H-dibenzo[b,e][1,4]diazepin-11-one

The desired product was prepared by substituting Example 449A for Example 448A in Example 448B. MS (DCI) m/e 344 (M+H)⁺.

### Example 449C

### 7-[(2S)-2-(hydroxymethyl)pyrrolidin-1-yl]-3-(3-methoxy-4-nitrophenyl)-5,10-dihydro-11H-dibenzo[b,e][1,4diazepin-11-one

The desired product was prepared by substituting Example 449B and Example 266G for Example 59B and Example 56A, respectively, in Example 59C. MS (ESI) m/e 461 (M+H)⁺, ¹H NMR (400MHz, DMSO-d₆): δ 9.57 (s, 1H), 8.01 (d, J=8.29 Hz, 1H), 7.84 (s, 1H), 7.78 (d, J=8.29 Hz, 1H), 7.53 (d, J=1.84 Hz, 1H), 7.37 (d, J=1.53 Hz, 1H), 7.34 (dd, J=8.44, 1.69 Hz, 1H), 7.29 (dd, J=8.13, 1.38 Hz, 1H), 6.79 (d, J=8.59 Hz, 1H), 6.33 (d, J=2.15 Hz, 1H), 6.22 (dd, J=8.75, 2.30 Hz, 1H), 4.03 (s, 3H), 3.60 (m, 1H), 3.46 (dd, J=10.43, 3.38 Hz, 1H), 3.31 (m, 1H), 3.19 (dd, J=10.28, 8.44 Hz, 1H), 2.99 (m, 1H), 1.82-2.02 (m, 4H).

### Example 450

### 3-(3-methoxy-4-nitrophenyl)-7-morpholin-4-yl-5,10-dihydro-11H-dibenzo[b,e][1,4]diazepin-11-one

### Example 450A

### 5-morpholin-4-yl-2-nitrophenylamine

The desired product was prepared by substituting morpholine for piperidine in Example 448A: MS (DCI) m/e 224 (M+H)⁺.

### Example 450B

### 3-chloro-7-morpholin-4-yl-5,10-dihydro-11H-dibenzo[b,e][1,4]diazepin-11-one

The desired product was prepared by substituting Example 450A for Example 448A in Example 448B. MS (DCI) m/e 330 (M+H)⁺.

### Example 450C

### 3-(3-methoxy-4-nitrophenyl)-7-morpholin-4-yl-5,10-dihydro-11H-dibenzo[b,e][1,4]diazepin-11-one

The desired product was prepared by substituting Example 450B and Example 266G for Example 59B and Example 56A, respectively, in Example 59C. MS (ESI) m/e 447 (M+H)⁺, ¹H NMR (500MHz, DMSO-d₆): δ 9.70 (s, 1H), 8.01 (d, J=8.42 Hz, 1H), 7.86 (s, 1H), 7.79 (d, J=8.42 Hz, 1H), 7.53 (d, J=1.56 Hz, 1H), 7.33-7.36 (m, 2H), 7.30 (dd, J=8.27, 1.72 Hz, 1H), 6.85 (d, J=8.73 Hz, 1H), 6.62 (d, J=2.49 Hz, 1H), 6.56 (dd, J=8.73, 2.81 Hz, 1H), 4.03 (s, 3H), 3.71-3.73 (m, 4H), 3.00-3.02 (m, 4H).

### Example 451

### 7-(4-hydroxypiperidin-1-yl)-3-(3-methoxy-4-nitrophenyl)-5,10-dihydro-11H-dibenzo[b,e][1,4]diazepin-11-one

### Example 451A

### 1-(3-amino-4-nitrophenyl)piperidin-4-ol

The desired product was prepared by substituting 4-hydroxypiperidine for piperidine in Example 448A. MS (DCI) m/e 238 (M+H)⁺.

### Example 451B

### 3-chloro-7-(4-hydroxypiperidin-1-yl)-5,10-dihydro-11H-dibenzo[b,e][1,4]diazepin-11-one

The desired product was prepared by substituting Example 451A for Example 448A in Example 448B. MS (DCI) m/e 344 (M+H)⁺.

### Example 451C

### 7-(4-hydroxypiperidin-1-yl)-3-(3-methoxy-4-nitrophenyl)-5,10-dihydro-11H-dibenzo[b,e][1,4]diazepin-11-one

The desired product was prepared by substituting Example 451B and Example 266G for Example 59B and Example 56A, respectively, in Example 59C. MS (ESI) m/e 461 (M+H)⁺, ¹H NMR (500MHz, DMSO-d₆): δ 9.80 (s, 1H), 8.01 (d, J=8.42 Hz, 1H), 7.95 (s, 1H), 7.80 (d, J=8.42 Hz, 1H), 7.53 (d, J=1.87 Hz, 1H), 7.34-7.36 (m, 2H), 7.32 (d, J=8.42 Hz, 1H), 6.90 (d, J=8.11 Hz, 1H), 6.81 (m, 1H), 6.73 (m, 1H), 4.03 (s, 3H), 3.69 (m, 1H), 3.45-3.48 (m, 2H), 2.96-3.00 (m, 2H), 1.84-1.87 (m, 2H), 1.55-1.57 (m, 2H),

### Example 452

### methyl {3-[3-methoxy-4-(3-methyl-1,2,4-oxadiazol-5-yl)phenyl]-11-oxo-10,11-dihydro-5H-dibenzo[b,e][1,4]diazepin-8-yl} acetate

### Example 452A

### 5-(4-iodo-2-methoxyphenyl)-3-methyl-1,2,4-oxadiazole

A mixture of 4-iodo-2-methoxy benzoic acid (83mg, 0.3 mmol), HATU (114mg, 0.3 mmol), DIEA (26µL, 0.15 mmol), and HOBt (8mg, 0.06 mmol) in DMF was stirred at room temperature for 5 minutes. Acetamide oxime (22mg, 0.3 mmol) was added, and reaction was stirred for 30 minutes at room temperature. The reaction was then heated to 110°C for 16 hours. Cooled to room temperature. Partitioned between ethyl acetate and water. The organic layer was washed with brine, dried (MgSO₄), filtered, and concentrated under vacuum. The residue was purified by HPLC to provide 37mg (39%) of desired product. MS (ESI) m/e 317 (M+H)⁺, ¹H NMR (300MHz, DMSO-d₆): δ 7.70 (d, J=8.14 Hz, 1H), 7.64 (d, J=1.36 Hz, 1H), 7.53 (dd, J=8.31-1.53 Hz, 1H), 3.94 (s, 3H), 2.40 (s, 3H).

### Example 452B

### methyl {3-[3-methoxy-4-(3-methyl-1,2,4-oxadiazol-5-yl)phenyl]-11-oxo-10,11-dihydro-5H-dibenzo[b,e][1,4]diazepin-8-yl} acetate

The desired product was prepared by substituting Example 452A and Example 54A for Example 9 and 2-methoxy-4-(4,4,5,5-tetramethyl-1,3,2-diozborolan-2-yl)phenol, respectively, in Example 10. MS (ESI) m/e 471 (M+H)⁺, ¹H NMR (500MHz, DMSO-d₆): δ 9.89 (s, 1H), 8.09 (d, J=8.24 Hz, 1H), 8.00 (s, 1H), 7.80 (d, J=7.93 Hz, 1H), 7.47 (s, 1H), 7.39-7.41 (m, 2H), 7.33 (dd, J=8.24, 1.53 Hz, 1H), 6.97 (d, J=7.93 Hz, 1H), 6.85-6.88 (m, 2H), 4.04 (s, 3H), 3.60 (s, 3H), 3.54 (s, 2H), 2.42 (s, 3H).

### Example 453

### 8-(2-ethyl-2-hydroxybutyl)-3-(3-methoxy-4-nitrophenyl)-5,10-dihydro-11H-dibenzo[b,e][1,4]diazepin-11-one

### Example 453A

### 3-chloro-8-(2-ethyl-2-hydroxybutyl)-5,10-dihydro-11H-dibenzo[b,e][1,4]diazepin-11-one

The desired product was prepared by substituting Example 6D and ethylmagnesium bromide for Example 1B and methylmagnesium bromide, respectively, in Example 189A. MS (DCI) m/e 345 (M+H)⁺.

### Example 453B

### 8-(2-ethyl-2-hydroxybutyl)-3-(3-methoxy-4-nitrophenyl)5,10-dihydro-11H-dibenzo[b,e][1,4]diazepin-11-one

The desired product was prepared by substituting Example 453A and Example 266G for Example 59B and Example 56A, respectively, in Example 59B. MS (DCI) m/e 461 (M+H)⁺,¹H NMR (400MHz, DMSO-d₆): δ 9.8 (s, 1H), 8.01 (d, J=8.29 Hz, 1H), 7.89 (s, 1H), 7.79 (d, J=8.29 Hz, 1H), 7.52 (d, J=1.84 Hz, 1H), 7.33-7.35 (m, 2H), 7.29 (dd, J=8.29, 1.84 Hz, 1H), 6.81-6.91 (m, 3H), 4.03 (s, 3H), 2.49-2.51 (m, 2H), 1.27 (q, J=7.36 Hz, 4H), 0.81 (t, J=7.36 Hz, 6H).

### Example 454

### 3-[(2-chloropyridin-4-yl)amino]-8-(2-ethyl-2-hydroxybutyl)-5,10-dihydro-11H-dibenzo[b,e][1,4]diazepin-11-one

The title compound was prepared by substituting Example 453A and 2-chloro-4-aminopyridine for Example 189A and 4-aminopyridine, respectively, in Example 191. MS (DCI) m/e 462 (M+H)⁺, ¹H NMR (500MHz, DMSO-d₆): δ 9.56 (s, 1H), 9.29 (s, 1H), 8.08 (d, J=6.14 Hz, 1H), 7.82 (s, 1H), 7.66 (d, J=8.59 Hz, 1H), 6.99-7.01 (m, 2H), 6.78-6.87 (m, 4.H), 6.65 (dd, J=8.59, 2.15 Hz, 1H), 2.47 (s, 1H), .1.27 (q, J=7.36 Hz, 4H), 0.81 (t, J=7.36 Hz, 6H).

### Example 455

### N,N-dimethyl-2-[11-oxo-3-(pyridin-4-ylamino)-10,11-dihydro-5H-dibenzo[b,e][1,4]diazepin-8-yl]acetamide

### Example 455A

### [11-oxo-3-(pyridin-4-ylamino)-10,11-dihydro-5H-dibenzo[b,e][1,4]diazepin-8-yl]acetic acid

The desired product was prepared by substituting Example 209 for Example 12 in Example 13. MS (DCI) m/e 361 (M+H)⁺.

### Example 455B

### N,N-dimethyl-2-[11-oxo-3-(pyridin-4-ylamino)-10,11-dihydro-5H-dibenzo[b,e][1,4]diazepin-8-yl]acetamide

The desired product was prepared by substituting Example 455A and N,N-dimethylamine hydrochloride for dimethylaminoacetic acid and Example 120, respectively, in Example 122. MS (DCI) m/e 388 (M+H)⁺, ¹H NMR (500MHz, DMSO-d₆): δ 10.49 (s, 1H), 9.78 (s, 1H), 8.36 (d, J=7.48 Hz, 2H), 7.99 (s, 1H), 7.77 (d, J=8.42 Hz, 1H), 7.22 (d, J=7.49 Hz, 2H), 6.94 (d, J=1.87 Hz, 1H), 6.91 (d, J=8.11 Hz, 1H), 6.80-6.84 (m, 3H), 3.54 (s, 2H), 2.98 (s, 3H), 2.82 (s, 3H).

### Example 456

### N-(4-morpholin-4-ylphenyl)-2-[11-oxo-3-(pyridin-4-ylamino)-10,11-dihydro-5H-dibenzo[b,e][1,4]diazepin-8-yl]acetamide

The desired product was prepared by substituting Example 455A and 4-morpholin-4-ylphenylamine for dimethylaminoacetic acid and Example 120, respectively, in Example 122. MS (DCI) m/e 520 (M+H)⁺, ¹H NMR (500MHz, DMSO-d₆): δ 10.55 (s, 1H), 9.90 (s, 1H), 9.82 (s, 1H), 8.35 (d, J=7.48 Hz, 2H), 8.01 (s, 1H), 7.76 (d, J=8.42 Hz, 1H), 7.44 (d, J=9.04 Hz, 2H), 7.22 (d, J=7.48 Hz, 2H), 6.87- 6.94 (m, 5H), 6.83 (dd, J=8.58, 2.03 Hz, 2H), 3.70-3.72 (m, 4H), 3.46 (s, 2H), 3.02-3.04 (m, 4H).

### Example 457

### 8-(2-hydroxy-2-methylpropyl)-3-(pyrimidin-4-ylamino)-5,10-dihydro-11H-dibenzo[b,e][1,4]diazepin-11-one

The desired product was prepared by substituting Example 208A and 4-aminopyrimidine for Example 189A and 4-aminopyridine, respectively, in Example 191. MS (DCI) m/e 376 (M+H)⁺, ¹H NMR (500MHz, DMSO-d₆): δ 10.49 (s, 1H), 9.63(s, 1H), 8.85 (s, 1H), 8.39 (d, J=6.24 Hz, 1H), 7.89 (s, 1H), 7.69 (d, J=8.42 Hz, 1H), 7.39 (s, 1H), 7.12 (d, J=8.42 Hz, 1H), 6.98 (d, J=6.24 Hz, 1H), 6.90 (d, J=7.80 Hz, 1H), 6.77-6.81 (m, 2H), 2.50 (s, 2H), 1.03 (s, 6H).

### Example 458

### 8-(2-hydroxy-2-methylpropyl)-3-[(2-methylpyridin-4-yl)amino]-5,10-dihydro-11H-dibenzo[b,e][1,4]diazepin-11-one

The desired product was prepared by substituting Example 208A and 4-amino-2-picoline for Example 189A and 4-aminopyridine, respectively, in Example 191. MS (DCI) m/e 389 (M+H)⁺, ¹H NMR (500MHz, DMSO-d₆): δ 10.41 (s, 1H), 9.73 (s, 1H), 8.26 (d, J=7.48 Hz, 1H), 7.95 (s, 1H), 7.76 (d, J=8.73 Hz, 1H), 7.07-7.10 (m, 2H), 6.93 (d, J=1.87 Hz, 1H), 6.87 (d, J=7.80 Hz, 1H), 6.79-6.84 (m, 3H), 2.50-2.52 (m, 5H), 1.04 (s, 6H).

### Example 459

### 3-(3-methoxy-4-nitrophenyl)-8-(2-oxopropyl)-5,10-dihydro-11H-dibenzo[b,e][1,4]diazepin-11-one

### Example 459A

### 3-chloro-8-(2-oxopropyl)-5,10-dihydro-11H-dibenzo[b,e][1,4]diazepin-11-one

The desired product was obtained as a side product from Example 208A. MS (DCI) m/e 301 (M+H)⁺.

### Example 459B

### 3-(3-methoxy-4-nitrophenyl)-8-(2-oxopropyl)-5,10-dihydro-11H-dibenzo[b,e][1,4]diazepin-11-one

The desired product was prepared by substituting Example 459A and Example 266G for Example 59B and Example 56A, respectively, in Example 59C. MS (DCI) m/e 418 (M+H)⁺,¹H NMR (500MHz, DMSO-d₆): δ 9.89 (s, 1H), 8.01 (m, 2H), 7.80 (d, J=7.93 Hz, 1H), 7.52 (s, 1H), 7.33-7.35 (m, 2H), 7.30 (d, J=8.24 Hz, 1H), 6.96 (d, J=8.54 Hz, 1H), 6.79-6.81 (m, 2H), 4.03 (s, 3H), 3.62 (s, 2H), 2.10 (s, 3H).

### Example 460

### 3-[(2-chloropyridin-4-yl)amino]-8-(2-oxopropyl)-5,10-dihydro-11H-dibenzo[b,e][1,4]diazepin-11-one

The desired product was prepared by substituting Example 459A and 2-chloro-4-aminopyridine for Example 189A and 4-aminopyridine, respectively, in Example 191. MS (DCI) m/e 393 (M+H)⁺, ¹H NMR (500MHz, DMSO-d₆): δ 9.64 (s, 1H), 9.31 (s, 1H), 8.09 (d, J=6.10 Hz, 1H), 7.92 (s, 1H), 7.67 (d, J=8.54 Hz, 1H), 7.00-7.02 (m, 2H), 6.91 (d, J=8.54 Hz, 1H), 6.86 (d, J=2.14 Hz, 1H), 6.76-6.78 (m, 2H), 6.66 (dd, J=8.70, 1.98 Hz, 1H), 3.61 (s, 2H), 2.09 (s, 3H).

### Example 461

### 3-({2-[(2-chloropyridin-4-yl)amino]pyridin-4-yl}amino)-8-(2-oxopropyl)-5,10-dihydro-11H-dibenzo[b,e][1,4]diazepin-11-one

The desired product was obtained as a side product from Example 460. MS (DCI) m/e 485 (M+H)⁺,1H NMR (500MHz, DMSO-d₆): δ 9.63 (s, 1H), 9.59 (s, 1H), 9.02 (s, 1H), 8.05-8.08 (m, 2H), 7.99 (d, J=1.83 Hz, 1H), 7.87 (s, 1H), 7.66 (d, J=8.24 Hz, 1H), 7.41 (dd, J=5.80, 1.83 Hz, 1H), 6.92 (d, J=8.54 Hz, 1H), 6.83 (d, J=2.14 Hz, 1H), 6.75-6.78 (m, 2H), 6.62-6.68 (m, 3H), 3.61 (s, 2H), 2.09 (s, 3H).

### Example 462

### methyl 2-methyl-2-[11-oxo-3-(pyrimidin-4-ylamino)-10,11-dihydro-5H-dibenzo[b,e][1,4]ldiazepin-8-yl]propanoate

The desired product was prepared by substituting Example 266F and 4-aminopyrimidine for Example 189A and 4-aminopyridine, respectively, in Example 191. MS (DCI) m/e 404 (M+H)⁺,¹H NMR (500MHz, DMSO-d₆): δ 10.60 (s, 1H), 9.66 (s, 1H), 8.87 (s, 1H), 8.39 (d, J=6.55 Hz, 1H), 8.02 (s, 1H), 7.70 (d, J=8.73 Hz, 1H), 7.39 (d, J=1.56 Hz, 1H), 7.14 (dd, J=8.58, 1.72 Hz, 1H), 7.00 (d, J=6.55 Hz, 1H), 6.97 (m, 1H), 6.95 (d, J=1.87 Hz, 1H), 6.89 (dd, J=8.26, 2.03 Hz, 1H), 3.58 (s, 3H), 1.44 (s, 6H).

### Example 463

### methyl 2-methyl-2-{3-[(2-methylpyridin-4-yl)amino]-11-oxo-10,11-dihydro-5H-dibenzo[b,e][1,4]diazepin-8-yl}propanoate

The desired product was prepared by substituting Example 266F and 4-amino-2-picoline for Example 189A and 4-aminopyridine, respectively, in Example 191. MS (DCI) m/e 417 (M+H)⁺,¹H NMR (500MHz, DMSO-d₆): δ 10.43 (s, 1H), 9.74 (s, 1H), 8.26 (d, J=7.49 Hz, 1H), 8.06 (s, 1H), 7.77 (d, J=8.42 Hz, 1H), 7.07-7.09 (m, 2H), 6.90-6.97 (m, 4H), 6.83 (dd, J=8.58, 2.03 Hz, 1H), 3.58 (s, 3H), 3.54 (s, 3H), 1.44 (s, 6H).

### Example 464

### 2-methylN(4-morpholin-4-ylphenyl)-2-[11-oxo-3-(pyrimidin-4-ylamino)-10,11-dihydro-5H-dibenzo[b,e][1,4]diazepin-8-yl]propanamide

### Example 464A

### 2-methyl-2-[11-oxo-3-(pyrimidin-4-ylamino)-10,11-dihydro-5H-dibenzo[b,e][1,4]diazepin-8-yl]propanoic acid

The desired product was prepared by substituting Example 462 for Example 12 in Example 13. MS (DCI) m/e 390 (M+H)⁺.

### Example 464B

### 2-methylN(4-morpholin-4-ylphenyl)-2-[11-oxo-3-(pyrimidin-4-ylamino)-10,11-dihydro-5H-dibenzo[b,e][1,4]diazepin-8-yl]propanamide

The desired product was prepared by substituting Example 464A and 4-(4-morpholino)aniline for dimethylaminoacetic acid and Example 120, respectively, in Example 208. MS (DCI) m/e 510 (M+H)⁺, ¹H NMR (500MHz, DMSO-d₆): δ 10.67 (s, 1H), 9.70 (s. 1H), 8.88 (s, 1H), 8.79 (s, 1H), 8.39 (d, J=6.55 Hz, 1H), 7.99 (s, 1H), 7.70 (d, J=8.42 Hz, 1H), 7.43 (s, 1H), 7.41 (s, 1H), 7.37 (s, 1H), 7.13 (dd, J=8.58,1.72 Hz, 1H), 6.91-7.02 (m, 4H), 6.86 (s, 1H), 6.84 (s, 1H), 3.71-3.73 (m, 4H), 3.01-3.03 (m, 4H), 1.48 (s, 6H).

### Example 465

### 2-methyl-2-{3-[(2-methylpyridin-4-yl)amino]-11-oxo-10,11-dihydro-5H-dibenzo[b,e][1,4]diazapin-8-yl}N(4-morpholin-4-ylphenyl)propanamide

### Example 465A

### 2-methyl-2-{3-[(2-methylpyridin-4-yl)amino]-11-oxo-10,11-dihydro-5H-dibenzo[b,e][1,4]diazepin-8-yl}propanoic acid

The desired product was prepared by substituting Example 463 for Example 12 in Example 13. MS (DCI) m/e 403 (M+H)⁺.

### Example 465B

### 2-methyl-2-{3-[(2-methylpyridin-4-yl)amino]-11-oxo-10,11-dihydro-5H-dibenzo[b,e][1,4]diazepin-8-yl}N(4-morpholin-4-ylphenyl)propanamide

The desired product was prepared by substituting Example 465A and 4-(4-morpholino)aniline for dimethylaminoacetic acid and Example 120, respectively, in Example 122. MS (ESI) m/e 563 (M+H)⁺. ¹H NMR (500MHz, DMSO-d₆): δ 9.53 (s, 1H), 8.91 (s, 1H), 8.77 (s, 1H), 8.16 (d, J=4.99 Hz, 1H), 7.84 (s, 1H), 7.62 (d, J=8.73 Hz, 1H), 7.41 (d, J=9.04 Hz, 2H), 7.01 (d, J=1.56 Hz, 1H), 6.82-6.92 (m, 7H), 6.60 (dd, J=8.58, 2.03 Hz, 1H), 3.71 (m, 4H), 3.01 (m, 4H), 2.37 (s, 3H), 1.47 (s, 6H).

### Example 466

### 3-{[3-(2-hydroxyethyl)pyridin-4-yl]amino}-8-(1-hydroxy-1-methylethyl)-5,10-dihydro-11H-dibenzo[b,e][1,4]diazepin-11-one

### Example 466A

### 2-(4-aminopyridin-3-yl)ethanol

A solution of di-tert-butyl dicarbonate (3.274g, 15 mmol) in CH₂Cl₂ (8 mL) was added to a solution of 4-aminopyridine (1.412g, 15 mmol) in CH₂Cl₂ (15 mL), and was stirred at room temperature for 30 minutes. Acidified with 1M HCl. Washed with CH₂Cl₂. Neutralized aqueous layer with K₂CO₃. Partitioned between CH₂Cl₂ and water. The organic layer was washed with brine, dried (MgSO₄), filtered, and concentrated under vacuum to provide 2.41g (83%) of pyridin-4-yl-carbamic acid tert-butyl ester.

Solution A was prepared by combining pyridin-4-yl-carbamic acid tert-butyl ester (388mg, 2mmol) in THF (5 mL) and cooling to -78°C. t-BuLi (2.8 mL, 1.7M solution in pentane) was added dropwise. Once the addition was complete, the solution was stirred at -78°C for 15 minutes, then warmed to -15°C and stirred for 90 minutes. In a separate flask, solution B was prepared by combining bromoethanol (0.21mL, 3 mmol) in THF (5 mL) and cooling to - 78°C. n-BuLi (1.44 mL, 2.5M solution in hexanes) was added dropwise. Once the addition was complete, the solution was stirred at -78°C for 10 minutes. Solution A was recooled to -78°C, and solution B was added to solution A via cannula. Stirred with warming to room temperature for 2 hours. The reaction was recooled, and quenched with water. Partitioned between CH₂Cl₂ and water. The organic layer was washed with brine, dried (MgSO₄), filtered, and concentrated under vacuum. The residue was purified by flash column chromatography on silica gel with 100% ethyl acetate to provide 91mg (19%) of [3-(2-hydroxy-ethyl)-pyridin-4-yl]-carbamic acid tert-butyl ester.

A solution of [3-(2-hydroxy-ethyl)-pyridin-4-yl]-carbamic acid tert-butyl ester (91mg, 0.38 mmol) in CH₂Cl₂ (3 mL) was treated with TFA (2mL) and stirred at room temperature for 3 hours. Concentrated under vacuum to provide 21mg (23%) of desired product. MS (DCI) m/e 139 (M+H)⁺.

### Example 466B

### 3-{[3-(2-hydroxyethyl)pyridin-4-yl]amino}-8-(1-hydroxy-1-methylethyl)-5,10-dihydro-11H-dibenzo[b,e][1,4]diazepin-11-one

The desired product was prepared by substituting Example 466A for 4-aminopyridine in Example 191. MS (DCI) m/e 387 (M+H)⁺,¹H NMR (300MHz, DMSO-d₆): δ 9.77 (s, 1H), 9.60 (s, 1H), 8.23-8.27 (m, 2H), 7.96 (s, 1H), 7.78 (d, J=8.48 Hz, 1H), 7.17 (m, 1H), 7.12 (d, J=2.03 Hz, 1H), 7.03 (m, 1H), 6.88-6.93 (m, 2H), 6.82 (dd, J=8.48,2.03 Hz, 1H), 3.73 (t, J=5.93 Hz, 2H), 2.90 (t, J=5.76 Hz, 2H), 1.36 (s, 6H).

### Example 467

### 8-(2-hydroxy-2-methylpropyl)-3-[(2-methoxypyridin-4-yl)amino]-5,10-dihydro-11H-dibenzo[b,e][1,4]diazepin-11-one

### Example 467A

### 2-methoxypyridin-4-amine

Na (0.7g, 30 mmol), was added to methanol (10 mL) at room temperature. Once all Na had dissolved, 2-chloro-4-aminopyridine (0.5g, 3.9 mmol) was added. The solution was heated to reflux for 16 hours. After the reaction solution cooled to room, it was partitioned between ethyl acetate and water. The aqueous layer was extracted twice with additional ethyl acetate. The combined organic layers were washed with brine, dried (MgSO₄), filtered, and concentrated under vacuum to give the title product. MS (DCI) m/e 125 (M+H)⁺.

### Example 467B

### 8-(2-hydroxy-2-methylpropyl)-3-[(2-methoxypyridin-4-yl)amino]-5,10-dihydro-11H-dibenzo[b,e][1,4]diazepin-11-one

The desired product was prepared by substituting Example 208A and Example 467A for Example 189A and 4-aminopyridine, respectively, in Example 191. MS (DCI) m/e 404 (M+H)⁺, ¹H NMR (500MHz, DMSO-d₆): δ 9.48 (s, 1H), 8.97 (s, 1H), 7.91 (d, J=5.83 Hz, 1H), 7.82 (s, 1H), 7.62 (d, J=8.59 Hz, 1H), 7.08 (d, J=2.15 Hz, 1H), 7.00 (m, 1H), 6.85-6.89 (m, 2H), 6.66 (dd, J=5.83, 1.84 Hz, 1H), 6.59 (dd, J=8.59, 2.15 Hz, 1H), 6.42 (d, J=1.84 Hz, 1H), 4.86 (s, 1H), 3.81 (s, 3H), 1.36 (s, 3H).

### Example 468

### 8-(2-hydroxy-2-methylpropyl)-3-[(2-methylpyridin-4-yl)amino]-5,10-dihydro-11H-dibenzo[b,e][1,4]diazepin-11-one

The desired product was prepared by substituting Example 208A and 4-amino-2-picoline for Example 189A and 4-aminopyridine, respectively, in Example 191. MS (DCI) m/e 388 (M+H)⁺,¹H NMR (300MHz, DMSO-d₆): δ 9.48 (s, 1H), 8.91 (s, 1H), 8.16 (d, J=5.52 Hz, 1H), 7.78-7.80 (m, 2H), 7.63 (d, J=8.59 Hz, 1H), 7.08 (d, J=1.84 Hz, 1H), 7.00 (m, 1H), 6.83-6.89 (m, 3H), 6.61 (dd, J=8.90, 2.15 Hz, 1H), 2.37 (s, 3H), 1.36 (s, 6H).

### Example 469

### methyl 11-oxo-3-(pyrimidin-4-ylamino)-10,11-dihydro-5H-dibenzo[b,e][1,4]diazepine-7-carboxylate

### Example 469A

### methyl 3-amino-4-nitrobenzoate

A solution of 5-chloro-2-nitro-aniline (6.902g, 40 mmol), Zn(CN)₂ (2.818g, 24 mmol), and Pd(PPh₃)₄ (2.311g, 2 mmol) in DMF (40 mL) was heated to 120°C for 4 days. Cooled to room temperature. Partitioned between ethyl acetate and water. The organic layer was washed with brine, dried (MgSO₄), filtered, and concentrated under vacuum. The residue was purified by flash column chromatography on silica gel with 4:1 hexanes/ethyl acetate to provide 1.49g (23%) of 3-amino-4-nitro-benzonitrile.

A mixture of 3-amino-4-nitro-benzonitrile (1.2g, 7.36 mmol), concentrated HCl (50 mL), and water (100 mL) was heated to reflux for 2 days. Cooled to room temperature and collected orange solid by filtration. Washed solid with water until wash was neutral, to provide 1.11g (83%) of 3-amino-4-nitro-benzoic acid.

(Trimethylsilyl)diazomethane (6mL, 2M solution in hexanes) was added to a solution of 3-amino-4-nitro-benzoic acid in CH₂Cl₂ (25 mL) and methanol (25 mL). Stirred at room temperature until bubbling ceased. Concentrated under vacuum to provide 1.2g of desired product. MS (DCI) m/e 197 (M+H)⁺.

### Example 469B

### methyl 4-chloro-2-{[5-(methoxycarbonyl)-2-nitrophenyl]amino}benzoate

The desired product was prepared by substituting Example 469A for methyl 3,4-diaminobenzoate in Example 1A. MS (DCI) m/e 365 (M+H)⁺.

### Example 469C

### methyl 3-chloro-11-oxo-10,11-dihydro-5H-dibenzo[b,e][1,4]diazepine-7-carboxylate

The desired product was prepared by substituting Example 469B for Example 2A in Example 2B. MS (DCI) m/e 303 (M+H)⁺.

### Example 469D

### methyl 11-oxo-3-(pyrimidin-4-ylamino)-10,11-dihydro-5H-dibenzo[b,e][1,4]diazepine-7-carboxylate

The desired product was prepared by substituting Example 469C and 4-aminopyrimidine for Example 189A and 4-aminopyridine, respectively, in Example 191. MS (DCI) m/e 360 (M+H)⁺, ¹H NMR (400MHz, DMSO-d₆): δ 10.43 (s, 1H), 9.94 (s, 1H), 8.80 (s, 1H), 8.34 (d, J=6.44 Hz, 1H), 8.17 (s, 1H), 7.65 (m, 2H), 7.42 (m, 2H), 7.07 (dd, J=8.75, 1.99 Hz, 1H), 6.98 (d, J=8.29 Hz, 1H), 6.93 (dd, J=6.44, 0.92 Hz, 1H), 3.75 (s, 3H).

### Example 470

### 7-(1-hydroxy-1-methylethyl)-3-(3-methoxy-4-nitrophenyl)-5,10-dihydro-11H-dibenzo[b,e][1,4]diazepin-11-one

### Example 470A

### 3-chloro-7-(1-hydroxy-1-methylethyl)-5,10-dihydro-11H-dibenzo[b,e][1,4]diazepin-11-one

The desired product was prepared by substituting Example 469C for Example 1B in Example 189A. MS (DCI) m/e 303 (M+H)⁺.

### Example 470B

### 7-(1-hydroxy-1-methylethyl)-3-(3-methoxy-4-nitrophenyl)-5,10-dihydro-11H-dibenzo[b,e][1,4]diazepin-11-one

The desired product was prepared by substituting Example 470A and Example 266G for Example 59B and Example 56A, respectively, in Example 59C. MS (DCI) m/e 420 (M+H)⁺,¹H NMR (500MHz, DMSO-d₆): δ 9.82 (s, 1H), 8.01 (d, J=8.42 Hz, 1H), 7.96 (s, 1H), 7.80 (d, J=8.11 Hz, 1H), 7.52 (d, J=1.56 Hz, 1H), 7.40 (d, J=1.56 Hz, 1H), 7.35 (dd, J=8.58, 1.72 Hz, 1H), 7.30 (dd, J=8.26, 1.72 Hz, 1H), 7.19 (d, J=1.87 Hz, 1H), 6.96 (m, 1H), 6.89 (d, J=8.42 Hz, 1H), 4.04 (s, 3H), 1.38 (s, 6H).

### Example 471

### 7-(1-hydroxy-1-methylethyl)-3-(pyrimidin-4-ylamino)-5,10-dihydro-11H-dibenzo[b,e][1,4]diazepin-11-one

The desired product was prepared by substituting Example 470A and 4-aminopyrimidine for Example 189A and 4-aminopyridine, respectively, in Example 191. MS (DCI) m/e 362 (M+H)⁺, ¹H NMR (500MHz, DMSO-d₆): δ 10.47 (s, 1H), 9.63 (s, 1H), 8.85 (s, 1H), 8.39 (d, J=6.55 Hz, 1H), 7.95 (s, 1H), 7.69 (s, 1H), 7.43 (d, J=2.18 Hz, 1H), 7.20 (d, J=1.87 Hz, 1H), 7.12 (dd, J=8.73, 1.87 Hz, 1H), 6.98 (d, J=6.55 Hz, 1H), 6.94 (m, 1H), 6.86 (d, J=8.11 Hz, 1H), 1.38 (s, 6H).

### Example 472

### 2-{3-[(6-methoxypyrimidin-4-yl)amino]-11-oxo-10,11-dihydro-5H-dibenzo[b,e][1,4]diazepin-8-yl}-2-methylN(4-morpholin-4-ylphenyl)propanamide

The desired product was prepared by substituting Example 202B and 6-methoxy-4-aminopyrimidine for Example 189A and 4-aminopyridine, respectively, in Example 191. MS (DCI) m/e 580 (M+H)⁺,¹H NMR (500MHz, DMSO-d₆): δ 9.56 (d, J=9.67 Hz, 2H), 8.79 (s, 1H), 8.44 (s, 1H), 7.88 (s, 1H), 7.61 (d, J=8.73 Hz, 1H), 7.42 (d, J=9.04 Hz, 2H), 7.37 (d, J=1.87 Hz, 1H), 6.95-7.00 (m, 3H), 6.89 (dd, J=8.26, 2.03 Hz, 1H), 6.86 (d, J=9.04 Hz, 2H), 6.20 (s, 1H), 3.87 (s, 3H), 3.72 (m, 4H), 3.03 (m, 4H), 1.47 (s, 6H).

### Example 473

### 3-(3-methoxy-4-nitrophenyl)-8-{2-[(6-morpholin-4-ylpyridin-3-yl)oxy]ethyl}-5,10-dihydro-11H-dibenzo[b,e][1,4]diazepin-11-one

### Example 473A

### 6-morpholin-4-ylpyridin-3-ol

The desired product was prepared by substituting 6-chloro-3-hydroxypyridine for 2-(4-bromo-phenyl)-ethanol in Example 447A. MS (DCI) m/e 181 (M+H)⁺.

### Example 473B

### 3-(3-methoxy-4-nitrophenyl)-8-{2-[(6-morpholin-4-ylpyridin-3-yl)oxy]ethyl}-5,10-dihydro-11H-dibenzo[b,e][1,4]diazepin-11-one

The desired product was prepared by substituting Example 239 and Example 473A for Example 204A and 2-hydropyridine, respectively, in Example 221A. MS (DCI) m/e 568 (M+H)⁺,¹H NMR (500MHz, DMSO-d₆): δ 9.85 (s, 1H), 8.01 (d, J=8.11 Hz, 1H), 7.95 (s, 1H), 7.87 (d, J=2.81 Hz, 1H), 7.79 (d, J=8.11 Hz, 1H), 7.52 (d, J=1.56 Hz, 1H), 7.28-7.35 (m, 4H), 6.92-6.96 (m, 3H), 6.81 (d, J=9.05 Hz, 1H), 4.10 (t, J=6.71 Hz, 2H), 4.03 (s, 3H), 3.68-3.70 (m, 4H), 3.29-3.31 (m, 4H), 2.87 (t, J=6.55 Hz, 2H).

### Example 474

### 3-(4-hydroxy-3-methoxyphenyl)-8-[2-(4-morpholin-4-ylphenoxy)ethyl]-5,10-dihydro-11H-dibenzo[b,e][1,4]diazepin-11-one

The desired product was prepared by substituting Example 297A and 2-methoxy-4-(4,4,5,5-tetramethyl-1,3,2-dioxaborolan-2-yl)phenol for Example 59B and Example 56A, respectively, in Example 59C. MS (DCI) m/e 538 (M+H)⁺,¹H NMR (300MHz, DMSO-d₆): δ 9.70 (s, 1H), 9.22 (s, 1H), 7.80 (s, 1H), 7.70 (d, J=8.11 Hz, 1H), 7.22 (d, J=0.94 Hz, 1H), 7.17 (d, J=1.87 Hz, 1H), 7.14 (d, J=8.11 Hz, 1H), 7.07 (dd, J=8.27, 2.03 Hz, 1H), 6.81-6.94 (m, 8H), 4.04 (t, J=6.71 Hz, 2H), 3.85 (s, 3H), 3.70-3.72 (m, 4H), 2.96-2.98 (m, 4H), 2.86 (t, J=6.71 Hz, 2H).

### Example 475

### 3-[(2,6-difluoropyridin-4-yl)amino]-8-[2-(4-morpholin-4-ylphenoxy)ethyl]-5,10-dihydro-11H-dibenzo[b,e][1,4]diazepin-11-one

The desired product was prepared by substituting Example 297A and Example 203A for Example 189A and 4-aminopyridine, respectively, in Example 191. MS (DCI) m/e 544 (M+H)⁺, ¹H NMR (300MHz, DMSO-d₆): δ 9.65 (s, 1H), 9.62 (s, 1H), 7.87 (s, 1H), 7.67 (d, J=8.73 Hz, 1H), 6.80-6.88 (m, 8H), 6.66 (dd, J=8.58, 2.03 Hz, 1H), 6.55 (s, 2H), 4.02 (t, J=6.08 Hz, 2H), 3.69-3.71 (m, 4H), 2.94-2.96 (m, 4H), 2.84 (t, J=6.55 Hz, 2H).

### Example 476

### 7-hydroxy-8-methoxy-3-(3-methoxy-4-nitrophenyl)-5,10-dihydro-11H-dibenzo[b,e][1,4]diazepin-11-one

### Example 476A

### {2-[(2-methoxy-5-nitrophenoxy)methoxy]ethyl}(trimethyl)silane

A mixture of 2-methoxy-5-nitrophenol (10g, 59.1 mmol) in dichloromethane (150 mL) was treated with 2-(trimethylsilyl)ethoxymethyl chloride (10.5 mL, 59.3 mmol) and N,N-diisopropylethylamine (11.3 mL, 64.9 mmol) at room temperature and stirred for 1.5 hours. The reaction mixture was then concentrated under vacuum, diluted with ethyl acetate, washed with water and brine, dried (MgSO₄), filtered, and concentrated under vacuum to provide the desired product.

### Example 476B

### 4-methoxy-3-{[2-(trimethylsilyl)ethoxy]methoxy}phenylamine

A mixture Example 476A, 5% Pt/C (1g) and ethanol (500 mL) was equipped with a balloon of hydrogen gas and stirred at room temperature. After uptake of the hydrogen was complete, the solution was filtered through diatomaceous earth (Celite^{®}). The filtrate was concentrated under vacuum to provide the desired product.

### Example 476C

### N-(4-methoxy-3-{[2-(trimethylsilyl)ethoxy]methoxy}phenyl)acetamide

A mixture of Example 476B, acetic anhydride (20 mL), and 4-(dimethylamino)pyridine (722 mg, 5.9 mmol) in dichloroethane (150 mL) was stirred at room temperature for 2 hours. The reaction mixture was then concentrated under vacuum, diluted with ethyl acetate, washed with water and brine, dried (MgSO₄), filtered, and concentrated under vacuum. The residue was diluted with hexanes and stirred vigorously until a solid formed. The solid was filtered, rinsed with hexane, and dried under vacuum to produce 15g (81% over 3 steps from Examples 476A-C) of the desired product. MS (ESI) m/e 334 (M+Na)⁺; ¹H NMR (300 MHz, DMSO-d₆) δ 9.74 (s, 1H), 7.33 (d, J=2.7 Hz, 1H), 7.18 (dd, J=2.5, 8.7 Hz, 1H), 6.89 (d, J=8.8 Hz, 1H), 5.13 (s, 2H), 3.67-3.73 (m, 2H), 3.71 (s, 3H), 1.98 (s, 3H), 0.86-0.92 (m, 2H), -0.02 (s, 9H).

### Example 476D

### N-(4-methoxy-2-nitro-5-{[2-(trimethylsilyl)ethoxy]methoxy}phenyl)acetamide

The desired product was prepared by substituting Example 476C for Example 223A in Example 223B. MS (DCI) m/e 374 (M+NH₄)⁺; ¹H NMR (300 MHz, DMSO-d₆) δ 10.14 (s, 1H), 7.56 (s, 1H), 7.51 (s, 1H), 5.32 (s, 2H), 3.85 (s, 3H), 3.69-3.75 (m, 2H), 2.06 (s, 3H), 0.87-0.93 (m, 2H), -0.02 (s, 9H).

### Example 476E

### 4-methoxy-2-nitro-5-{[2-(trimethylsilyl)ethoxy]methoxy}aniline

A mixture of Example 476D and K₂CO₃ (7.0g, 50.6 mmol) in methanol (220 mL) was stirred at room temperature overnight, concentrated under vacuum, diluted with ethyl acetate, washed with water and brine, dried (MgSO₄), filtered and concentrated under vacuum to produce 9.7g (91%) of the desired product. MS (ESI) m/e 315 (M+H)⁺; ¹H NMR (300 MHz, DMSO-d₆) δ 7.43 (br s, 2H), 7.37 (s, 1H), 6.66 (s, 1H), 5.28 (s, 2H), 3.69-3.75 (m, 2H), 3.73 (s, 3H), 0.89-0.94 (m, 2H), -0.01 (s, 9H).

### Example 476F

### methyl 4-chloro-2-[(4-methoxy-2-nitro-5-{[2-(trimethylsilyl)ethoxy]methoxy}phenyl)amino]benzoate

The desired product was prepared by substituting Example 476E for methyl 3,4-diaminobenzoate in Example 1A. MS (ESI) m/e 483 (M+H)⁺; ¹H NMR (300 MHz, DMSO-d₆) δ 10.9 (s, 1H), 7.96 (d, J=8.5 Hz, 1H), 7.67 (s, 1H), 7.50 (d, J=1.7 Hz, 1H), 7.34 (s, 1H), 7.09 (dd, J=1.9, 8.7 Hz, 1H), 5.32 (s, 2H), 3.89 (s, 3H), 3.86 (s, 3H), 3.69-3.74 (m, 2H), 0.89-0.95 (m, 2H), -0.06 (s, 9H).

### Example 476G

### 3-chloro-8-methoxy-7-{[2-(trimethylsilyl)ethoxy]methoxy}-5,10-dihydro-11H-dibenzo[b,e][1,4]diazepin-11-one

A mixture of Example 476F (11.4g, 23.6 mmol), LiOH·H₂O (2.97g, 70.8 mmol), methanol (200 mL), THF (100 mL) and water (10 mL) was heated at 65 °C for 4.25 hours. When hydrolysis was complete, triethylamine (65.8 mL, 472 mmol) was added, followed by SnCl₂·2H₂O (26.6g, 118 mmol). The mixture was heated at 65 °C overnight, then cooled to room temperature when hydrogenation was complete. DMF (200 mL), triethylamine (16.5 mL, 118 mmol) and HATU (17.95g, 47.2 mmol) were added and the mixture was stirred overnight, filtered through diatomaceous earth (Celite^{®}), concentrated under vacuum, diluted with ethyl acetate, washed with water and brine, dried (MgSO₄), filtered, and concentrated under vacuum. Hexane was added to a solution of the concentrate in diethyl ether to invoke formation of a solid which was filtered, rinsed with hexane, and dried under vacuum to give 8.8g (89%) of the desired product. MS (ESI) m/e 421 (M+H)⁺; ¹H NMR (300 MHz, DMSO-d₆) δ 9.69 (s, 1H), 7.81 (s, 1H), 7.65 (d, J=8.5 Hz, 1H), 7.04 (d, J=2.0 Hz, 1H), 6.91 (dd, J=2.0, 8.5 Hz, 1H), 6.76 (s, 1H), 6.65 (s, 1H), 5.11 (s, 2H), 3.67 (s, 3H), 3.65-3.72 (m, 2H), 0.86-0.94 (m, 2H), -0.03 (s, 9H).

### Example 476H

### 8-methoxy-3-(3-methoxy-4-nitrophenyl)-7-{2-(trimethylsilyl)ethoxylmethoxy}-5,10-dihydro-11H-dibenzo[b,e][1,4]diazepin-11-one

A mixture of Example 476G (60mg, 0.14 mmol), Example 266G (60mg, 0.22 mmol), Pd(PPh₃)₄ (66 mg, 0.057 mmol), 1M Na₂CO₃ solution (0.2 mL, 0.2 mmol), and a 7:2:3 mixture of ethylene glycol dimethyl ether/ethanol/water (4 mL) was placed in a microwave process vial , crimped and heated at 160 °C for 30 minutes in an Emrys Synthesizer set at 300W. It was then removed from the instrument, cooled to room temperature, filtered through diatomaceous earth (Celite^{®}), concentrated under vacuum, diluted with ethyl acetate, washed with water and brine, dried (MgSO₄), filtered, and concentrated under vacuum. The residue was purified by column chromatography to give 47mg (62%) of the desired product. MS (ESI) m/e 538 (M+H)⁺; ¹H NMR (300 MHz, DMSO-d₆) δ 9.68 (s, 1H), 8.01 (d, J=8.5 Hz, 1H), 7.76-7.79 (m, 2H), 7.51 (d, J=1.7 Hz, 1H), 7.27-7.35 (m, 3H), 6.82 (s, 1H), 6.67 (s, 1H), 5.11 (s, 2H), 4.03 (s, 3H), 3.68 (s, 3H), 3.67-3.72 (m, 2H), 0.86-0.92 (m, 2H), -0.04 (s, 9H).

### Example 476I

### 7-hydroxy-8-methoxy-3-(3-methoxy-4-nitrophenyl)-5,10-dihydro-11H-dibenzo[b,e][1,4]diazepin-11-one

A mixture of Example 476H (429mg, 0.8 mmol) in methylene chloride (20 mL) and methanol (10 mL) was treated with 4N HCl/dioxane (1 mL, 4.0 mmol) and stirred one hour at room temperature. The mixture was concentrated under vacuum to provide 325mg (quantitative) of desired product. MS (ESI) m/e 408 (M+H)⁺; ¹H NMR (300 MHz, DMSO-d₆) δ 9.57 (s, 1H), 8.89 (s, 1H), 8.01 (d, J =8.1 Hz, 1H), 7.76 (d, J =8.1 Hz, 1H), 7.65 (s, 1H), 7.51 (d, J =1.7 Hz, 1H), 7.26-7.35 (m, 3H), 6.59 (s, 1H), 6.52 (s, 1H), 4.03 (s, 3H), 3.67 (s, 3H).

### Example 477

### 8-hydroxy-7-methoxy-3-(3-methoxy-4-nitrophenyl)-5,10-dihydro-11H-dibenzo[b,e][1,4]diazepin-11-one

### Example 477A

### {2-[(2-methoxy-4-nitrophenoxy)methoxy]ethyl}(trimethyl)silane

The desired product was prepared by substituting 2-methoxy-4-nitrophenol for 2-methoxy-5-nitrophenol in Example 476A. MS (ESI) m/e 317 (M+NH₄)⁺; ¹H NMR (300 MHz, DMSO-d₆) δ 7.89 (dd, J=2.7, 8.8 Hz, 1H), 7.70 (d, J=2.7 Hz, 1H), 7.27 (d, J=8.8 Hz, 1H), 5.38 (s, 2H), 3.90 (s, 3H), 3.70-3.75 (m, 2H), 0.86-0.92 (m, 2H), -0.03 (s, 9H).

### Example 477B

### 3-methoxy-4-{[2-(trimethylsilyl)ethoxy]methoxy}aniline

The desired product was prepared by substituting Example 477A for Example 476A in Example 476B.

### Example 477C

### N-(3-methoxy-4-{[2-(trimethylsilyl)ethoxy]methoxy}phenyl)acetamide

The desired product was prepared by substituting Example 477B for Example 476B in Example 476C. ¹H NMR (300 MHz, DMSO-d₆) δ 9.80 (s, 1H), 7.31 (d, J=2.0 Hz, 1H), 7.02 (dd, J=2.4, 8.8 Hz, 1H), 6.95 (d, J=8.8 Hz, 1H), 5.09 (s, 2H), 3.72 (s, 3H), 3.67-3.74 (m, 2H), 2.00 (s, 3H), 0.84-0.90 (m, 2H), -0.02 (s, 9H).

### Example 477D

### N-(5-methoxy-2-nitro-4-{[2-(trimethylsilyl)ethoxy]methoxy}phenyl)acetamide

The desired product was prepared by substituting Example 477C for Example 223A in Example 223B. MS (ESI) m/e 357 (M+H)⁺; ¹H NMR (300 MHz, DMSO-d₆) δ 10.20 (s, 1H), 7.74 (s, 1H), 7.51 (s, 1H), 5.27 (s, 2H), 3.86 (s, 3H), 3.70-3.75 (m, 2H), 2.10 (s, 3H), 0.86-0.92 (m, 2H), -0.03 (s, 9H).

### Example 477E

### 5-methoxy-2-nitro-4-{[2-(trimethylsilyl)ethoxy]methoxy} aniline

The desired product was prepared by substituting Example 477D for Example 476D in Example 476E. MS (ESI) m/e 313 (M-H)⁻; ¹H NMR (300 MHz, DMSO-d₆) δ 7.59 (s, 1H), 7.43 (br s, 2H), 6.52 (s, 1H), 5.11 (s, 2H), 3.80 (s, 3H), 3.68-3.73 (m, 2H), 0.86-0.91 (m, 2H), -0.02 (s, 9H).

### Example 477F

### methyl 4-chloro-2-[(5-methoxy-2-nitro-4-{[2-(trimethylsilyl)ethoxy]methoxy}phenyl)amino]benzoate

The desired product was prepared by substituting Example 477E for methyl 3,4-diaminobenzoate in Example 1A. MS (ESI) m/e 483 (M+H)⁺; ¹H NMR (300 MHz, DMSO-d₆) δ 10.96 (s, 1H), 7.96 (d, J=8.8 Hz, 1H), 7.85 (s, 1H), 7.62 (d, J=2.0 Hz, 1H), 7.19 (s, 1H), 7.11 (dd, J=2.0, 8.5 Hz, 1H), 5.27 (s, 2H), 3.89 (s, 3H), 3.83 (s, 3H), 3.72-3.78 (m, 2H), 0.89-0.94 (m, 2H), -0.01 (s, 9H).

### Example 477G

### 3-chloro-7-methoxy-8-{[2-(trimethylsilyl)ethoxy]methoxy}-5,10-dihydro-11H-dibenzo[b,e][1,4]diazepin-11-one

The desired product was prepared by substituting Example 477F for Example 476F in Example 476G. MS (ESI) m/e 421 (M+H)⁺; ¹H NMR (300 MHz, DMSO-d₆) δ 9.70 (s, 1H), 7.84 (s, 1H), 7.65 (d, J=8.5 Hz, 1H), 7.03 (d, J=2.0 Hz, 1H), 6.92 (dd, J=2.0, 8.5 Hz, 1H), 6.75 (s, 1H), 6.65 (s, 1H), 5.03 (s, 2H), 3.71 (s, 3H), 3.65-3.71 (m, 2H), 0.86-0.91 (m, 2H), -0.02 (s, 9H).

### Example 477H

### 7-methoxy-3-(3-methoxy-4-nitrophenyl)-8-{[2-(trimethylsilyl)ethoxy]methoxy}-5,10-dihydro-11H-dibenzo[b,e][1,4]diazepin-11-one

The desired product was prepared by substituting Example 477G for Example 476G in Example 476H. MS (ESI) m/e 538 (M+H)⁺.

### Example 477I

### 8-hydroxy-7-methoxy-3-(3-methoxy-4-nitrophenyl)-5,10-dihydro-11H-dibenzo[b,e][1,4]diazepin-11-one

The desired product was prepared by substituting Example 477H for Example 476H in Example 476I. MS (ESI) m/e 408 (M+H)⁺; ¹H NMR (400 MHz, DMSO-d₆) δ 9.64 (s, 1H), 8.62 (s, 1H), 8.01 (d, J =8.6 Hz, 1H), 7.76 (d, J =8.3 Hz, 1H), 7.62 (s, 1H), 7.52 (d, J =1.2 Hz, 1H), 7.26-7.35 (m, 3H), 6.64 (s, 1H), 6.48 (s, 1H), 4.03 (s, 3H), 3.70 (s, 3H).

### Example 478

### 8-methoxy-3-(3-methoxy-4-nitrophenyl)-7-(tetrahydro-2H-pyran-2-ylmethoxy)-5,10-dihydro-11H-dibenzo[b,e][1,4]diazepin-11-one

A mixture of Example 476I (40mg, 0.098 mmol), 2-bromomethyl-tetrahydro-2H-pyran (0.13 mL, 0.98 mmol), K₂CO₃ (136mg, 0.98 mmol) and DMF (2 mL) was heated at 100 °C overnight, cooled to room temperature, diluted with ethyl acetate, washed with water and brine, dried (MgSO₄), filtered and concentrated under vacuum. The residue was purified by column chromatography on silica gel with 49:1 dichloromethane/methanol to provide 23mg (47%) of the desired product. MS (ESI) m/e 506 (M+H)⁺; ¹H NMR (300 MHz, DMSO-d₆) δ 9.66 (s, 1H), 8.01 (d, J =8.5 Hz, 1H), 7.77 (d, J =8.8 Hz, 1H), 7.71 (s, 1H), 7.53 (d, J =1.7 Hz, 1H), 7.35 (dd, J =1.7, 8.5 Hz, 1H), 7.26-7.31 (m, 2H), 6.69 (s, 1H), 6.64 (s, 1H), 4.03 (s, 3H), 3.72-3.94 (m, 3H), 3.67 (s, 3H), 3.60 (m, 1H), 3.39 (m, 1H), 1.82 (m, 1H), 1.64 (m, 1H), 1.41-1.55 (m, 3H), 1.27 (m, 1H).

### Example 479

### 8-methoxy-3-(3-methoxy-4-nitrophenyl)-7-[(1-methylpiperidin-3-yl)methoxy]-5,10-dihydro-11H-dibenzo[b,e][1,4]diazepin-11-one

The desired product was prepared by substituting 3-chloromethyl-1-methyl-piperidine for 2-bromomethyl-tetrahydro-2H-pyran in Example 478. MS (ESI) m/e 519 (M+H)⁺; ¹H NMR (300 MHz, DMSO-d₆) δ 9.66 (s, 1H), 8.02 (d, J =8.5 Hz, 1H), 7.78 (d, J =8.8 Hz, 1H), 7.72 (s, 1H), 7.53 (d, J =1.7 Hz, 1H), 7.36 (dd, J =1.7, 8.5 Hz, 1H), 7.25-7.30 (m, 2H), 6.71 (s, 1H), 6.65 (s, 1H), 4.03 (s, 3H), 3.69-3.87 (m, 2H), 3.68 (s, 3H), 3.29 (s, 3H), 2.85 (m, 1H), 2.12-2.29 (m, 2H), 2.00 (m, 1H), 1.58-1.75 (m, 2H), 1.49 (m, 1H), 1.10 (m, 1H), 0.88 (m, 1H).

### Example 480

### 8-methoxy-3-(3-methoxy-4-nitrophenyl)-7-(pyridin-2-ylmethoxy)-5,10-dihydro-11H-dibenzo[b,e][1,4]diazepin-11-one

The desired product was prepared by substituting 2-(chloromethyl)pyridine hydrochloride for 2-bromomethyl-tetrahydro-2H-pyran in Example 478. MS (ESI) m/e 499 (M+H)⁺; ¹H NMR (300 MHz, DMSO-d₆) δ 9.65 (s, 1H), 8.58 (m, 1H), 8.00 (d, J =8.4 Hz, 1H), 7.85 (m, 1H), 7.78 (d, J =8.1 Hz, 1H), 7.73 (s, 1H), 7.51 (d, J=1.6 Hz, 1H), 7.49 (d, J=7.8 Hz, 1H), 7.32-7.35 (m, 2H), 7.27-7.30 (m, 2H), 6.77 (s, 1H), 6.70 (s, 1H), 5.09 (s, 2H), 4.03 (s, 3H), 3.71 (s, 3H).

### Example 481

### 8-methoxy-3-(3-methoxy-4-nitrophenyl)-7-(pyridin-3-ylmethoxy)-5,10-dihydro-11H-dibenzo[b,e][1,4]diazepin-11-one

The desired product was prepared by substituting 3-(chloromethyl)pyridine hydrochloride for 2-bromomethyl-tetrahydro-2H-pyran in Example 478. MS (ESI) m/e 499 (M+H)⁺; ¹H NMR (500 MHz, DMSO-d₆) δ 9.66 (s, 1H), 8.65 (d, J=1.9 Hz, 1H), 8.54 (dd, J=1.6, 4.7 Hz, 1H), 8.01 (d, J =8.4 Hz, 1H), 7.83 (m, 1H), 7.77-7.79 (m, 2H), 7.52 (d, J =1.6 Hz, 1H), 7.43 (dd, J=5.0, 7.8 Hz, 1H), 7.35 (dd, J=1.6, 8.4 Hz, 1H), 7.32 (d, J=1.6 Hz, 1H), 7.29 (dd, J=1.6, 8.1 Hz, 1H), 6.81 (s, 1H), 6.69 (s, 1H), 5.05 (s, 2H), 4.03 (s, 3H), 3.69 (s, 3H).

### Example 482

### 8-methoxy-3-(3-methoxy-4-nitrophenyl)-7-(-pyridin-4-ylmethoxy)-5,10-dihydro-11H-dibenzo[b,e][1,4]diazepin-11-one

The desired product was prepared by substituting 4-(chloromethyl)pyridine hydrochloride for 2-bromomethyl-tetrahydro-2H-pyran in Example 478. MS (ESI) m/e 499 (M+H)⁺; ¹H NMR (500 MHz, DMSO-d₆) δ 9.69 (s, 1H), 8.72 (d, J =5.3 Hz, 2H), 8.01 (d, J =8.4 Hz, 1H), 7.78 (d, J =8.7 Hz, 1H), 7.75 (s, 1H), 7.66 (d, J =5.6 Hz, 2H), 7.52 (d, J =1.6 Hz, 1H), 7.33 (dd, J=1.6, 8.4 Hz, 1H), 7.28-7.30 (m, 2H), 6.75 (s, 1H), 6.72 (s, 1H), 5.19 (s, 2H), 4.03 (s, 3H), 3.73 (s, 3H).

### Example 483

### 8-methoxy-3-(3-methoxy-4-nitrophenyl)-7-[(5-methylisoxazol-3-yl)methoxy]-5,10-dihydro-11H-dibenzo[b,e][1,4]diazepin-11-one

The desired product was prepared by substituting 3-bromomethyl-5-methyl-isoxazole for 2-bromomethyl-tetrahydro-2H-pyran in Example 478. MS (ESI) m/e 503 (M+H)⁺; ¹H NMR (500 MHz, DMSO-d₆) δ 9.69 (s, 1H), 8.01 (d, J =8.5 Hz, 1H), 7.76-7.79 (m, 2H), 7.52 (d, J =1.7 Hz, 1H), 7.27-7.36 (m, 3H), 6.77 (s, 1H), 6.68 (s, 1H), 6.30 (d, J =0.7 Hz, 1H), 5.03 (s, 2H), 4.03 (s, 3H), 3.69 (s, 3H), 2.41 (d, J =0.7 Hz, 3H).

### Example 484

### 8-methoxy-3-(3-methoxy-4-nitrophenyl)-7-[(1-methyl-1H-imidazol-5-yl)methoxy]-5,10-dihydro-11H-dibenzo[b,e][1,4]diazepin-11-one

### Example 484A

### 5-(chloromethyl)-1-methyl-1H-imidazole hydrochloride

A mixture of (1-methyl-1H-imidazol-5-yl)methanol (500mg, 4.5 mmol) in DMF (5 mL) at -5 °C was slowly treated with thionyl chloride (0.49 mL, 6.7 mmol), stirred an additional 10 minutes at -5 °C, warmed to room temperature, stirred 1.5 hours, cooled to -5 °C, quenched with isopropyl alcohol and ethyl acetate, stirred 30 minutes at -5 °C, concentrated under vacuum, and filtered to collect the solid. The solid was rinsed with ethyl acetate and dried under vacuum to give (212mg, 28%) of the desired product as the hydrochloride salt. MS (DCI) m/e 131 (M+H)⁺; ¹H NMR (300 MHz, DMSO-d₆) δ 9.10 (s, 1H), 7.76 (s, 1H), 5.02 (s, 2H), 3.88 (s, 3H).

### Example 484B

### 8-methoxy-3-(3-methoxy-4-nitrophenyl)-7-[(1-methyl-1H-imidazol-5-yl)methoxy]-5,10-dihydro-11H-dibenzo[b,e][1,4]diazepin-11-one

The desired product was prepared by substituting Example 484A for 2-bromomethyl-tetrahydro-2H-pyran in Example 478. MS (ESI) m/e 502 (M+H)⁺; ¹H NMR (300 MHz, DMSO-d₆) δ 10.08 (s, 1H), 8.94 (s, 1H), 9.04 (m, 1H), 8.02 (d, J=8.5 Hz, 1H), 7.71 (d, J=8.1 Hz, 1H), 7.65 (s, 2H), 7.49-7.56 (m, 2H), 7.46 (dd, J=1.7, 8.5 Hz, 1H), 6.81 (s, 1H), 6.68 (s, 1H), 5.05-5.30 (m, 2H), 4.06 (s, 3H), 3.87 (s, 3H), 3.69 (s, 3H).

### Example 485

### 8-methoxy-3-(3-methoxy-4-nitrophenyl)-7-[(2-methyl-1,3-thiazol-4-yl)methoxy]-5,10-dihydro-11H-dibenzo[b,e][1,4]diazepin-11-one

The desired product was prepared by substituting 4-chloromethyl-2-methyl-thiazole hydrochloride for 2-bromomethyl-tetrahydro-2H-pyran in Example 478. MS (ESI) m/e 519 (M+H)⁺; ¹H NMR (500 MHz, DMSO-d₆) δ 9.65 (s, 1H), 8.00 (d, J =8.4 Hz, 1H), 7.78 (d, J =8.1 Hz, 1H), 7.72 (s, 1H), 7.52 (d, J =1.6 Hz, 1H), 7.46 (s, 1H), 7.27-7.35 (m, 3H), 6.79 (s, 1H), 6.67 (s, 1H), 5.00 (s, 2H), 4.03 (s, 3H), 3.68 (s, 3H), 2.65 (s, 3H)

### Example 486

### 8-methoxy-3-(3-methoxy-4-nitrophenyl)-7-[(2-oxo-1,3-oxazolidin-5-yl)methoxy]-5,10-dihydro-11H-dibenzo[b,e][1,4]diazepin-11-one

The desired product was prepared by substituting 5-chloromethyl-2-oxazolidinone for 2-bromomethyl-tetrahydro-2H-pyran in Example 478. MS (ESI) m/e 507 (M+H)⁺; ¹H NMR (500 MHz, DMSO-d₆) δ 9.66 (s, 1H), 8.00 (d, J =8.4.0 Hz, 1H), 7.77 (d, J =7.8 Hz, 1H), 7.72 (s, 1H), 7.54 (s, 1H), 7.52 (d, J =1.3 Hz, 1H), 7.27-7.34 (m, 3H), 6.73 (s, 1H), 6.67 (s, 1H), 4.86 (m, 1H), 4.02 (s, 3H), 3.97-4.09 (m, 3H), 3.68 (s, 3H), 3.58 (t, J =8.9 Hz, 1H).

### Example 487

### 8-methoxy-3-(3-methoxy-4-nitrophenyl)-7-(tetrahydrofuran-2-ylmethoxy)-5,10-dihydro-11H-dibenzo[b,e][1,4]diazepin-11-one

The desired product was prepared by substituting 2-bromomethyl-tetrahydro-furan for 2-bromomethyl-tetrahydro-2H-pyran in Example 478. MS (ESI) m/e 492 (M+H)⁺; ¹H NMR (400 MHz, DMSO-d₆) δ 9.63 (s, 1H), 8.00 (d, J =8.3 Hz, 1H), 7.76 (d, J =8.0 Hz, 1H), 7.69 (s, 1H), 7.51, (d, J =1.2 Hz, 1H), 7.26-7.35 (m, 3H), 6.70 (s, 1H), 6.64 (s, 1H), 4.10 (m, 1H), 3.81-3.84 (m, 2H), 3.77 (m, 1H), 4.02 (s, 3H), 3.67 (s, 3H), 3.67 (m, 1H), 1.97 (m, 1H), 1.77-1.90 (m, 2H), 1.63 (m, 1H).

### Example 488 (A-836095.0)

### 7-[(2,2-dimethyl-1,3-dioxolan-4-yl)methoxy]-8-methoxy-3-(3-methoxy-4-nitrophenyl)-5,10-dihydro-11H-dibenzo[b,e][1,4]diazepin-11-one

The desired product was prepared by substituting 4-chloromethyl-2,2-dimethyl-[1,3]dioxolane for 2-bromomethyl-tetrahydro-2H-pyran in Example 478. MS (ESI) m/e 522 (M+H)⁺; ¹H NMR (300 MHz, DMSO-d₆) δ 9.68 (s, 1H), 8.01 (d, J =8.5 Hz, 1H), 7.77 (d, J =8.5 Hz, 1H), 7.72 (s, 1H), 7.53 (d, J =1.7 Hz, 1H), 7.35 (dd, J =1.7, 8.5 Hz, 1H), 7.27-7.32 (m, 2H), 6.73 (s, 1H), 6.66 (s, 1H), 4.38 (m, 1H), 4.08 (dd, J =6.6, 8.3 Hz, 1H), 4.03 (s, 3H), 3.89 (d, J =5.4 Hz, 2H), 3.74 (dd, J =5.9, 8.3 Hz, 1H), 3.68 (s, 3H), 1.35 (s, 3H), 1.30 (s, 3H).

### Example 489

### 8-methoxy-3-(3-methoxy-4-nitrophenyl)-7-[(2R)-pyrrolidin-2-ylmethoxy]-5,10-dihydro-11H-dibenzo[b,e][1,4]diazepin-11-one

### Example 489A

### tert-butyl (2R)-2-({[(4-methylphenyl)sulfonyl]oxy}methyl)pyrrolidine-1-carboxylate

A mixture of Boc-D-prolinol (201mg, 1.0 mmol), p-toluenesulfonyl chloride (400mg, 2.1 mmol), triethylamine (420 µL, 3.0 mmol), and 4-(dimethylamino)pyridine (12mg, 0.1 mmol) in dichloromethane (10 mL) was heated at 40 °C for 48 hours, cooled to room temperature, concentrated under vacuum, diluted with ethyl acetate, washed with water and brine, dried (MgSO₄), filtered, and concentrated. The residue was purified by column chromatography on silica gel to provide 160mg (45%) of the desired product.

### Example 489B

### tert-butyl (2R)-2-({[8-methoxy-3-(3-methoxy-4-nitrophenyl)-11-oxo-10,11-dihydro-5H-dibenzo[b,e][1,4]diazepin-7-yl]oxy}methyl)pyrrolidine-1-carboxylate

The desired product was prepared by substituting Example 489A for 2-bromomethyl-tetrahydro-2H-pyran in Example 478.

### Example 489C

### 8-methoxy-3-(3-methoxy-4-nitrophenyl)-7-[(2R)-pyrrolidin-2-ylmethoxy]-5,10-dihydro-11H-dibenzo[b,e][1,4]diazepin-11-one

A mixture of Example 489B in methanol (2 mL) was treated with 4.0N HCl/dioxane (0.15 mL, 0.6 mmol), stirred overnight at room temperature, concentrated under vacuum, and purified by preparative HPLC to give 3.9mg (9%) of the desired product as the TFA salt. MS (ESI) m/e 491 (M+H)⁺; ¹H NMR (300 MHz, DMSO-d₆) δ 9.72 (s, 1H), 8.70 (s, 1H), 8.02 (d, J =8.1 Hz, 1H), 7.77-7.80 (m, 2H), 7.53 (d, J =1.4 Hz, 1H), 7.29-7.36 (m, 3H), 6.75 (s, 1H), 6.71 (s, 1H), 4.15 (m, 1H), 4.03 (s, 3H), 3.83-3.96 (m, 1H), 3.71 (s, 3H), 3.17-3.27 (m, 3H), 2.13 (m, 1H), 1.84-2.01 (m, 2H), 1.72 (m, 1H).

### Example 490

### 7,8-dimethoxy-3-(3-methoxy-4-nitrophenyl)-5,10-dihydro-11H-dibenzo[b,e][1,4]diazepin-11-one

The desired product was obtained by substituting iodomethane for 2-bromomethyl-tetrahydro-2H-pyran in Example 478. MS (ESI) m/e 422 (M+H)⁺; ¹H NMR (400 MHz, DMSO-d₆) δ 9.61 (s, 1H), 8.00 (d, J =8.3 Hz, 1H), 7.77 (d, J =8.3 Hz, 1H), 7.73 (s, 1H), 7.52 (d, J =1.5 Hz, 1H), 7.34 (dd, J=1.8, 8.6 Hz, 1H), 7.31 (d, J=1.8 Hz, 1H), 7.28 (dd, J=1.7, 8.1 Hz, 1H), 6.69 (s, 1H), 6.64 (s, 1H), 4.02 (s, 3H), 3.69 (s, 3H), 3.66 (s, 3H).

### Example 491

### 8-methoxy-7-[2-(2-methoxyethoxy)ethoxy]-3-(3-methoxy-4-nitrophenyl)-5,10-dihydro-11H-dibenzo[b,e][1,4]diazepin-11-one

The desired product was prepared by substituting 1-bromo-2-(2-methoxyethoxy)ethane for 2-bromomethyl-tetrahydro-2H-pyran in Example 478. MS (ESI) m/e 510 (M+H)⁺; ¹H NMR (500 MHz, DMSO-d₆) δ 9.67 (s, 1H), 8.01 (d, J =8.2 Hz, 1H), 7.77 (d, J =7.9 Hz, 1H), 7.73 (s, 1H), 7.53 (d, J =1.5 Hz, 1H), 7.35 (dd, J =1.5, 8.5 Hz, 1H), 7.28-7.30 (m, 2H), 6.70 (s, 1H), 6.65 (s, 1H), 4.03 (s, 3H), 3.98-4.00 (m, 2H), 3.70-3.72 (m, 2H), 3.68 (s, 3H) 3.57-3.59 (m, 2H), 3.44-3.46 (m, 2H), 3.23 (s, 3H).

### Example 492

### 7-(2,3-dihydroxypropoxy)-8-methoxy-3-(3-methoxy-4-nitrophenyl)-5,10-dihydro-11H-dibenzo[b,e][1,4]diazepin-11-one

The desired product was prepared by substituting 3-chloro-1,2-propanediol for 2-bromomethyl-tetrahydro-2H-pyran in Example 478. MS (ESI) m/e 482 (M+H)⁺; ¹H NMR (300 MHz, DMSO-d₆) δ 9.65 (s, 1H), 8.01 (d, J =8.5 Hz, 1H), 7.78 (d, J =8.5 Hz, 1H), 7.72 (s, 1H), 7.53 (d, J =1.7 Hz, 1H), 7.35 (dd, J=1.5, 8.3 Hz, 1H), 7.27-7.30 (m, 2H), 6.72 (s, 1H), 6.65 (s, 1H), 4.91 (d, J =4.8 Hz, 1H), 4.63 (t, J =5.6 Hz, 1H), 4.03 (s, 3H), 3.90 (m, 1H), 3.73-3.80 (m, 2H), 3.68 (s, 3H), z,-3.45 (m, 2H).

### Example 493

### 7-[3-hydroxy-2,2-bis(hydroxymethyl)propoxy]-8-methoxy-3-(3-methoxy-4-nitrophenyl)-5,10-dihydro-11H-dibenzo[b,e][1,4]diazepin-11-one

The desired product was prepared by substituting 2-(bromomethyl)-2-(hydroxymethyl)-1,3-propanediol for 2-bromomethyl-tetrahydro-2H-pyran in Example 478. MS (ESI) m/e 526 (M+H)⁺; ¹H NMR (300 MHz, DMSO-d₆) δ 9.64 (s, 1H), 8.00 (d, J =8.5 Hz, 1H), 7.77 (d, J =7.8 Hz, 1H), 7.72 (s, 1H), 7.54 (d, J =1.7 Hz, 1H), 7.36 (dd, J =1.7, 8.5 Hz, 1H), 7.27-7.30 (m, 2H), 6.74 (s, 1H), 6.63 (s, 1H), 4.34 (t, J =5.1 Hz, 3H), 4.04 (s, 3H), 3.80 (s, 2H), 3.68 (s, 3H), 3.48 (d, J =5.4 Hz, 6H).

### Example 494

### 2-hydroxy-3-{[8-methoxy-3-(3-methoxy-4-nitrophenyl)-11-oxo-10,11-dihydro-5H-dibenzo[b,e][1,4]diazepin-7-yl]oxy}propane-1-sulfonic acid

The desired product was prepared by substituting 3-chloro-2-hydroxy-1-propanesulfonic acid, sodium salt hydrate for 2-bromomethyl-tetrahydro-2H-pyran in Example 478. MS (ESI) m/e 546 (M+H)⁺; ¹H NMR (400 MHz, DMSO-d₆) δ 9.62 (s, 1H), 8.00 (d, J =8.6 Hz, 1H), 7.77 (d, J =8.0 Hz, 1H), 7.73 (s, 1H), 7.53 (d, J =1.5 Hz, 1H), 7.26-7.37 (m, 3H), 6.71 (s, 1H), 6.65 (s, 1H), 4.15 (m, 1H), 4.07 (m, 1H), 4.03 (s, 3H), 3.96 (m, 1H), 3.83 (m, 1H), 3.69 (s, 3H), 2.76 (m, 1H), 2.60 (m, 1H).

### Example 495

### 7-(3-aminopropoxy)-8-methoxy-3-(3-methoxy-4-nitrophenyl)-5,10-dihydro-11H-dibenzo[b,e][1,4]diazepin-11-one

The desired product was prepared by substituting N-(3-bromo-propyl)phthalimide for 2-bromomethyl-tetrahydro-2H-pyran in Example 478, followed by the addition of hydrazine hydrate and THF. The mixture was heated at 50 °C for 2 hours, cooled to room temperature, and concentrated under vacuum. The residue was purified by preparative HPLC to provide 6mg (8%) of the desired product as the TFA salt. MS (ESI) m/e 465 (M+H)⁺; ¹H NMR (300 MHz, DMSO-d₆) δ 9.67 (s, 1H), 8.02 (d, J =8.1 Hz, 1H), 7.76-7.79 (m, 2H), 7.52 (d, J =1.7 Hz, 1H), 7.28-7.35 (m, 3H), 6.73 (s, 1H), 6.67 (s, 1H), 4.03 (s, 3H), 3.95-3.99 (m, 2H), 3.68 (s, 3H), 2.91-2.95 (m, 2H), 1.93-2.04 (m, 2H).

### Example 496

### 7-[2-(dimethylamino)ethoxy]-8-methoxy-3-(3-methoxy-4-nitrophenyl)-5,10-dihydro-11H-dibenzo[b,e][1,4]diazepin-11-one

The desired product was prepared by substituting (2-chloroethyl)dimethylamine for 2-bromomethyl-tetrahydro-2H-pyran in Example 478. MS (ESI) m/e 479 (M+H)⁺; ¹H NMR (400 MHz, DMSO-d₆) δ 9.63 (s, 1H), 8.01 (d, J =8.6 Hz, 1H), 7.78 (d, J=8.0 Hz, 1H), 7.71 (s, 1H), 7.53 (d, J =1.5 Hz, 1H), 7.35 (dd, J =1.5, 8.3 Hz, 1H), 7.27-7.31 (m, 2H), 6.72 (s, 1H), 6.65 (s, 1H), 4.03 (s, 3H), 3.95 (t, J =6.1 Hz, 2H), 3.67 (s, 3H), 2.60 (t, J=6.1 Hz, 2H), 2.21 (s, 6H).

### Example 497

### 7-(2-chloroethoxy)-8-methoxy-3-(3-methoxy-4-nitrophenyl)-5,10-dihydro-11H-dibenzo[b,e][1,4]diazepin-11-one

The desired product was prepared by substituting 2-chloroethyl methanesulfonate for 2-bromomethyl-tetrahydro-2H-pyran in Example 478. MS (ESI) m/e 470 (M+H)⁺; ¹H NMR (300 MHz, DMSO-d₆) δ 9.68 (s, 1H), 8.01 (d, J =8.1 Hz, 1H), 7.78 (d, J =8.8 Hz, 1H), 7.74 (s, 1H), 7.53 (d, J =1.7 Hz, 1H), 7.34 (dd, J=1.7, 8.5 Hz, 1H), 7.27-7.30 (m, 2H), 6.73 (s, 1H), 6.68 (s, 1H), 4.13-4.16 (m, 2H), 4.03 (s, 3H), 3.89-3.93 (m, 2H), 3.69 (s, 3H).

### Example 498

### 8-methoxy-3-(3-methoxy-4-nitrophenyl)-7-(2-pyrrolidin-1-ylethoxy)-5,10-dihydro-11H-dibenzo[b,e][1,4]diazepin-11-one

A mixture of Example 497 (11mg, 0.023 mmol), pyrrolidine (3.9 µL, 0.046 mmol), K₂CO₃ (6.5mg, 0.046 mmol) and DMF (0.5 mL) was heated overnight at 50 °C, cooled to room temperature, and filtered. The filtrate was purified by preparative HPLC to provide 6mg (51%) of the desired product. MS (ESI) m/e 505 (M+H)⁺; ¹H NMR (500 MHz, DMSO-d₆) δ 9.72 (s, 1H), 8.02 (d, J =8.4 Hz, 1H), 7.78-7.80 (m, 2H), 7.53 (d, J =1.6 Hz, 1H), 7.29-7.35 (m, 3H), 6.78 (s, 1H), 6.73 (s, 1H), 4.18 (t, J =5.0 Hz, 2H), 4.03 (s, 3H), 3.71 (s, 3H), 3.44-3.68 (m, 4H), 3.11-3.17 (m, 2H), 2.00-2.07 (m, 2H), 1.95-1.93 (m, 2H).

### Example 499

### 8-methoxy-3-(3-methoxy-4-nitrophenyl)-7-(2-morpholin-4-ylethoxy)-5,10-dihydro-11H-dibenzo[b,e][1,41 diazepin-11-one

The desired product was prepared by substituting 4-(2-chloroethyl)morpholine hydrochloride for 2-bromomethyl-tetrahydro-2H-pyran in Example 478. MS (ESI) m/e 521 (M+H)⁺; ¹H NMR (500 MHz, DMSO-d₆) δ 9.71 (s, 1H), 8.00 (d, J =8.4 Hz, 1H), 7.77-7.78 (m, 2H), 7.51 (s, 1H), 7.29-7.33 (m, 3H), 6.76 (s, 1H), 6.71 (s, 1H), 4.20-4.22 (m, 2H), 4.02 (s, 3H), 3.87-3.97 (m, 2H), 3.60-3.89 (m, 2H), 3.69 (s, 3H), 3.48-3.55 (m, 2H), 3.10-3.40 (m, 4H).

### Example 500

### 7-(4-hydroxybutoxy)-8-methoxy-3-(3-methoxy-4-nitrophenyl)-5,10-dihydro-11H-dibenzo[b,e][1,4]diazepin-11-one

### Example 500A

### 7-[4-(tert-butyl-dimethyl-silanyloxy)-butoxyl-8-methoxy-3-(3-methoxy-4-nitro-phenyl)-5,10-dihydro-dibenzo[b,e][1,4]diazepin-11-one

The desired product was prepared by substituting tert-butyl(4-iodobutoxy)dimethylsilane for 2-bromomethyl-tetrahydro-2H-pyran in Example 478.

### Example 500B

### 7-(4-hydroxybutoxy)-8-methoxy-3-(3-methoxy-4-nitrophenyl)-5,10-dihydro-11H-dibenzo[b,e][1,4]diazepin-11-one

A mixture of Example 500A, Et₄NF·H₂O (10 equiv.) and THF was heated overnight at 65 °C, concentrated under vacuum, diluted with ethyl acetate, washed with water and brine, dried (MgSO₄), filtered and concentrated under vacuum. The residue was purified by preparative HPLC to provide 50mg (28%) of the desired product. MS (ESI) m/e 480 (M+H)⁺; ¹H NMR (400 MHz, DMSO-d₆) δ 9.63 (s, 1H), 8.01 (d, J =8.6 Hz, 1H), 7.78 (d, J =8.3 Hz, 1H), 7.71 (s, 1H), 7.53 (d, J =1.2 Hz, 1H), 7.35 (dd, J=1.5, 8.6 Hz, 1H), 7.28-7.30 (m, 2H), 6.70 (s, 1H), 6.65 (s, 1H), 4.03 (s, 3H), 3.89 (t, J=6.6 Hz, 2H), 3.67 (s, 3H), 3.44 (t, J=6.4 Hz, 2H), 1.69-1.76 (m, 2H), 1.51-1.58 (m, 2H).

### Example 501

### 7-(4-hydroxybutoxy)-3-(4-hydroxy-3-methoxyphenyl)-8-methoxy-5,10-dihydro-11H-dibenzo[b,e][1,4]diazepin-11-one

### Example 501A

### 3-chloro-7-hydroxy-8-methoxy-5,10-dihydro-11H-dibenzo[b,e][1,4]diazepin-11-one

The desired product was prepared by substituting Example 476G for Example 476H in Example 4761. MS (ESI) m/e 291 (M+H)⁺; ¹H NMR (300 MHz, DMSO-d₆) δ 9.57 (s, 1H), 8.94 (s, 1H), 7.69 (s, 1H), 7.64 (d, J=8.5 Hz, 1H), 7.02 (d, J=2.4 Hz, 1H), 6.89 (dd, J=2.0, 8.5 Hz, 1H), 6.57 (s, 1H), 6.47 (s, 1H), 3.66 (s, 3H).

### Example 501B

### 7-[4-(tert-butyl-dimethyl-silanyloxy)-butoxyl-3-chloro-8-methoxy-5,10-dihydro-dibenzo[b,e][1,4]diazepin-11-one

The desired product was prepared by substituting Example 501A and tert-butyl(4-iodobutoxy)dimethylsilane for Example 476 and 2-bromomethyl-tetrahydro-2H-pyran, respectively, in Example 478. MS (ESI) m/e 477 (M+H)⁺; ¹H NMR (300 MHz, DMSO-d₆) δ 9.64 (s, 1H), 7.73 (s, 1H), 7.64 (d, J=8.5 Hz, 1H), 7.00 (d, J=2.4 Hz, 1H), 6.90 (dd, J=2.0, 8.5 Hz, 1H), 6.62 (s, 2H), 3.89 (t, J=6.4 Hz, 2H), 3.65 (s, 3H), 3.63 (t, J=6.4 Hz, 2H), 1.68-1.77 (m, 2H), 1.53-1.62 (m. 2H), 0.84 (s, 9H), 0.02 (s, 6H).

### Example 501C

### 7-[4-(tert-butyl-dimethyl-silanyloxy)-butoxy]-3-(4-hydroxy-3-methoxy-phenyl)-8-methoxy-5,10-dihydro-dibenzo[b,e][1,4]diazepin-11-one

The desired product was prepared by substituting Example 501B and 2-methoxy-4-(4,4,5,5-tetramethyl-[1,3,2]dioxaborolan-2-yl)-phenol for Example 476G and Example 266G respectively, in Example 476H. MS (ESI) m/e 565 (M+H)⁺.

### Example 501D

### 7-(4-hydroxybutoxy)-3-(4-hydroxy-3-methoxyphenyl)-8-methoxy-5,10-dihydro-11H-dibenzo[b,e][1,4]diazepin-11-one

The desired product was prepared by substituting Example 501C for Example A-500A in Example 500B. MS (ESI) m/e 451 (M+H)⁺; ¹H NMR (300 MHz, DMSO-d₆) δ 9.51 (s, 1H), 9.25 (s, 1H), 7.68 (d, J =8.1 Hz, 1H), 7.58 (s, 1H), 7.04-7.20 (m, 4H), 6.86 (d, J =8.1 Hz, 1H), 6.69 (s, 1H) 6.53 (s, 1H), 4.43 (t, J =5.3 Hz, 1H), 3.88 (t, J =6.4 Hz, 2H), 3.86 (s, 3H), 3.66 (s, 3H), 3.39-3.50 (m, 2H), 1.68-1.77 (m, 2H), 1.49-1.59 (m, 2H).

### Example 502

### 7-(4-hydroxybutoxy)-8-methoxy-3-(pyrimidin-4-ylamino)-5,10-dihydro-11H-dibenzo[b,e][1,4]diazepin-11-one

### Example 502A

### 7-[4-(tert-butyl-dimethyl-silanyloxy)-butoxy]-8-methoxy-3-(pyrimidin-4-ylamino)-5,10-dihydro-dibenzo[b,e][1,4]diazepin-11-one

The desired product was prepared by substituting Example 501B and 4-aminopyrimidine for Example 189A and 4-aminopyridine, respectively, in Example 191.

### Example 502B

### 7-(4-hydroxybutoxy)-8-methoxy-3-(pyrimidin-4-ylamino)-5,10-dihydro-11H-dibenzo[b,e][1,4]diazepin-11-one

The desired product was prepared by substituting Example 502A for Example 500A in Example 500B. MS (ESI) m/e 422 (M+H)⁺; ¹H NMR (400 MHz, DMSO-d₆) δ 9.73 (s, 1H), 9.34 (s, 1H), 8.70 (s, 1H), 8.33 (d, J =5.5 Hz, 1H), 7.61-7.65 (m, 2H), 7.49 (s, 1H), 7.03 (dd, J =1.5, 8.6 Hz, 1H), 6.87 (d, J =5.8 Hz, 1H), 6.72 (s, 1H), 6.61 (s, 1H), 4.42 (t, J =5.1 Hz, 1H), 3.88 (t, J =6.6 Hz, 1H), 3.66 (s, 3H), 3.45 (q, J =6.1 Hz, 2H), 1.69-1.76 (m, 2H), 1.51-1.58 (m, 2H).

### Example 503

### 4-{[7-(4-hydroxybutoxy)-8-methoxy-11-oxo-10,11-dihydro-5H-dibenzo[b,e][1,4]diazepin-3-yl]amino}pyridine-2-carbonitrile

### Example 503A

### 4-{7-[4-(tert-butyl-dimethyl-silanyloxy)-butoxy]-8-methoxy-11-oxo-10,11-dihydro-5H-dibenzo[b,e][1,4]diazepin-3-ylamino}-pyridine-2-carbonitrile

The desired product was prepared by substituting Example 501B and Example 193A for Example 189A and 4-aminopyridine, respectively, in Example 191.

### Example 503B

### 4-{[7-(4-hydroxybutoxy)-8-methoxy-11-oxo-10,11-dihydro-5H-dibenzo[b,e][1,4]diazepin-3-yl]amino}pyridine-2-carbonitrile

The desired product was prepared by substituting Example 503A for Example 500A in Example 500B. MS (ESI) m/e 446 (M+H)⁺; ¹H NMR (500 MHz, DMSO-d₆) δ 9.44 (s, 1H), 9.39, (s, 1H), 8.38 (d, J =5.6 Hz, 1H), 7.66 (d, J =8.4 Hz, 1H), 7.62, (s, 1H), 7.48 (d, J =2.5 Hz, 1H), 7.24 (dd, J =2.5, 5.9 Hz, 1H), 6.84 (d, J =1.9 Hz, 1H), 6.68 (dd, J =2.0, 8.6 Hz, 1H), 6.625 (s, 1H), 6.63 (s, 1H), 4.45 (t, J =6.4 Hz, 1H), 3.87 (t, J =6.7 Hz, 2H), 3.66 (s, 3H), 3.44 (t, J =6.4 Hz, 2H), 1.69-1.75 (m, 2H), 1.52-1.55 (m, 2H).

### Example 504

### 3-[(2,6-difluoropyridin-4-yl)amino]-7-(4-hydroxybutoxy)-8-methoxy-5,10-dihydro-11H-dibenzo[b,e][1,4]diazepin-11-one

### Example 504A

### 7-[4-(tert-butyl-dimethyl-silanyloxy)-butoxy]-3-(2,6-difluoro-pyridin-4-ylamino)-8-methoxy-5,10-dihydro-dibenzo[b,e][1,4]diazepin-11-one

The desired product was prepared by substituting Example 501B and Example 203A for Example 189A and 4-aminopyridine, respectively, in Example 191.

### Example 504B

### 3-[(2,6-difluoropyridin-4-yl)amino]-7-(4-hydroxybutoxy)-8-methoxy-5,10-dihydro-11H-dibenzo[b,e][1,4]diazepin-11-one

The desired product was prepared by substituting Example 504A for Example 500A in Example 500B. MS (ESI) m/e 457 (M+H)⁺; ¹H NMR (300 MHz, DMSO-d₆) δ 9.66 (s, 1H), 9.42 (s, 1H), 7.65-7.68 (m, 2H), 6.86 (d, J =3.0 Hz, 1H), 6.67 (dd, J =3.0, 9.0 Hz, 1H), 6.62 (s, 2H), 6.57 (s, 2H), 4.43 (t, J =6.0 Hz, 1H), 3.87 (t, J =6.0 Hz, 2H), 3.66 (s, 3H), 3.44 (q, J =6.0 Hz, 2H), 1.67-1.75 (m, 2H), 1.50-1.58 (m, 2H).

### Example 505 ,

### 7-(4-hydroxybutoxy)-8-methoxy-3-[(2,3,6-trifluoropyridin-4-yl)amino]-5,10-dihydro-11H-dibenzo[b,e][1,4]diazepin-11-one

### Example 505A

### 7-[4-(tert-butyl-dimethyl-silanyloxy)-butoxy]-8-methoxy-3-(2,3,6-trifluoro-pyridin-4-ylamino)-5,10-dihydro-dibenzo[b,e][1,4]diazepin-11-one

The desired product was prepared by substituting Example 501B and Example 199A for Example 189A and 4-aminopyridine, respectively, in Example 191.

### Example 505B

### 7-(4-hydroxybutoxy)-8-methoxy-3-[(2,3,6-trifluoropyridin-4-yl)amino]-5,10-dihydro-11H-dibenzo[b,e][1,4]diazepin-11-one

The desired product was prepared by substituting Example 505A for Example 500A in Example 500B. MS (ESI) m/e 475 (M+H)⁺; ¹H NMR (500 MHz, DMSO-d₆) δ 9.52 (s, 1H), 9.44 (s, 1H), 7.55-7.68 (m, 2H), 6.87 (d, J =1.5 Hz, 1H), 6.76-6.81 (m, 2H), 6.62-6.64 (m, 2H), 4.42 (t, J =6.5 Hz, 1H), 3.77-3.97 (m, 2H), 3.66 (s, 3H), 3.44 (t, J =6.5 Hz, 2H), 1.69-1.75 (m, 2H), 1.51-1.57 (m, 2H).

### Example 506

### 7-ethoxy-3-(3-methoxy-4-nitrophenyl)-5,10-dihydro-11H-dibenzo[b,e][1,4]diazepin-11-one

### Example 506A

### 5-ethoxy-2-nitroaniline

A mixture of sodium metal (1.86g, 80.9 mmol) and ethanol (65 mL) was stirred at ambient temperature until all of the sodium metal had been consumed. 5-Chloro-2-nitroaniline (4.64g, 26.9 mmol) was added and the mixture was heated at 80 °C for 48 hours, cooled to room temperature, quenched with water, concentrated under vacuum, diluted with ethyl acetate, washed with water and brine, dried (MgSO₄), filtered, and concentrated under vacuum. The residue was purified by column chromatography on silica gel with 7:3 hexane/ethyl acetate to provide 4.6g (94%) of the desired product. MS (ESI) m/e 183 (M+H)⁺; ¹H NMR (300 MHz, DMSO-d₆) δ 7.90 (d, J=9.5 Hz, 1H), 7.46 (br s, 2H), 6.43 (d, J=2.7 Hz, 1H), 6.23 (dd, J=2.7, 9.5 Hz, 1H), 4.04 (q, J=7.1 Hz, 2H), 1.33 (t, J=7.0 Hz, 3H).

### Example 506B

### methyl 4-chloro-2-[(5-ethoxy-2-nitrophenyl)amino]benzoate

The desired product was prepared by substituting Example 506A for methyl 3,4-diaminobenzoate in Example 1A. MS (ESI) m/e 351 (M+H)⁺; ¹H NMR (300 MHz, DMSO-d₆) δ 11.03 (s, 1H), 8.16 (d, J=9.2 Hz, 1H), 7.97 (d, J=8.8 Hz, 1H), 7.66 (d, J=2.0 Hz, 1H), 7.18 (dd, J=2.0, 8.8 Hz, 1H), 7.00 (d, J=2.4 Hz, 1H), 6.71 (dd, J=2.5, 9.3 Hz, 1H), 4.11 (q, J=6.8 Hz, 2H), 3.88 (s, 3H), 1.33 (t, J=6.9 Hz, 3H).

### Example 506C

### 3-chloro-7-ethoxy-5,10-dihydro-11H-dibenzo[b,e][1,4]diazepin-11-one

The desired product was prepared by substituting Example 506B for Example 476F in Example 476G. MS (ESI) m/e 289 (M+H)⁺; ¹H NMR (300 MHz, DMSO-d₆) δ 9.76 (s, 1H), 7.98 (s, 1H), 7.67 (d, J=8.5 Hz, 1H), 7.05 (d, J=2.0 Hz, 1H), 6.91 (dd, J=2.0, 8.5 Hz, 1H), 6.86 (d, J=8.5 Hz, 1H), 6.50-6.56 (m, 2H), 3.94 (q, J=7.1 Hz, 2H), 1.29 (t, J=7.0 Hz, 3H).

### Example 506D

### 7-ethoxy-3-(3-methoxy-4-nitrophenyl)-5,10-dihydro-11H-dibenzo[b,e][1,4]diazepin-11-one

The desired product was prepared by substituting Example 506C for Example 476G in Example 476H. MS (ESI) m/e 406 (M+H)⁺; ¹H NMR (300 MHz, DMSO-d₆) δ 9.76 (s, 1H), 8.02 (d, J =8.5 Hz, 1H), 7.96 (s, 1H), 7.80, (d, J =8.1 Hz, 1H), 7.53 (d, J =1.4 Hz, 1H), 7.30-7.36 (m, 3H), 6.88 (d, J =8.8 Hz, 1H), 6.62 (d, J =2.7 Hz, 1H), 6.51 (dd, J =2.7, 8.5 Hz, 1H), 4.03 (s, 3H), 3.95 (q, J =7.1 Hz, 2H), 1.29 (t, J =7.1 Hz, 3H).

### Example 507

### 7-(4-hydroxybutoxy)-3-(3-methoxy-4-nitrophenyl)-5,10-dihydro-11H-dibenzo[b,e][1,4]diazepin-11-one

### Example 507A

### 3-chloro-7-hydroxy-5,10-dihydro-11H-dibenzo[b,e][1,4]diazepin-11-one

A mixture of Example 506C (1.87g, 6.5 mmol) in dichloromethane (50 mL) at -78 °C was treated dropwise with a 1.0M solution of BBr₃ in dichloromethane, stirred overnight and allowed to reach room temperature, quenched with a saturated solution of ammonium chloride, concentrated under vacuum, and extracted with ethyl acetate. The extracts were combined, washed with brine, dried (MgSO₄), filtered, and concentrated under vacuum to provide the desired product.

### Example 507B

### 3-chloro-7-hydroxy-5-{[2-(trimethylsilyl)ethoxy]methyl}-5,10-dihydro-11H-dibenzo[b,e][1,4]diazepin-11-one

The desired product was prepared by substituting Example 507A for 2-methoxy-5-nitrophenol in Example 476A. MS (ESI) m/e 391 (M+H)⁺; ¹H NMR (300 MHz, DMSO-d₆) δ 10.04 (s, 1H), 9.37 (br s, 1H), 7.63 (d, J=8.1 Hz, 1H), 7.54 (d, J=2.0 Hz, 1H), 7.19 (dd, J=2.0, 8.5 Hz, 1H), 6.91 (d, J=2.7 Hz, 1H), 6.84 (d, J=8.8 Hz, 1H), 6.51 (dd, J=2.5, 8.7 Hz, 1H), 4.90-5.03 (m, 2H), 3.62 (t, J=8.0 Hz, 2H), 0.93-0.98 (m, 2H), 0.01 (s, 9H).

### Example 507C

### 7-[4-(tert-butyl-dimethyl-silanyloxy)-butoxy]-3-chloro-5-(2-trimethylsyilanyl-ethoxymethyl)-5,10-dihydro-dibenzo[b,e][1,4]diazepin-11-one

The desired product was prepared by substituting Example 507B and tert-butyl(4-iodobutoxy)dimethylsilane for Example 476 and 2-bromomethyl-tetrahydro-2H-pyran, respectively, in Example 478. MS (ESI) m/e 577 (M+H)⁺.

### Example 507D

### 7-[4-(tert-butyl-dimethyl-silanyloxy)-butoxy]-8-methoxy-3-(3-methoxy-4-nitro-phenyl)-5,10-dihydro-dibenzo[b,e][1,4]diazepin-11-one

The desired product was prepared by substituting Example 507C for Example 476G in Example 476H.

### Example 507E

### 7-(4-hydroxbutoxy)-3-(3-methoxy-4-nitrophenyl)-5,10-dihydro-11H-dibenzo[b,e][1,4]diazepin-11-one

The desired product was prepared by substituting Example 507D for Example 500A in Example 500B. MS (ESI) m/e 450 (M+H)⁺; ¹H NMR (300 MHz, DMSO-d₆) δ 9.76 (s, 1H), 8.02 (d, J =8.5 Hz, 1H), 7.95 (s, 1H), 7.79 (d, J =8.1 Hz, 1H), 7.53 (d, J =1.7 Hz, 1H), 7.28-7.38 (m, 3H), 6.87 (d, J =8.8 Hz, 1H), 6.63 (d, J =2.7 Hz, 1H), 6.51 (dd, J =2.9, 8.7 Hz, 1H), 4.03 (s, 3H), 3.89 (t, J =6.4 Hz, 2H), 3.43 (t, J =6.4 Hz, 2H), 1.65-1.78 (m 2H), 1.46-1.59 (m, 2H).

### Example 508

### 7-(2-hydroxyethoxy)-3-(3-methoxy-4-nitrophenyl)-5,10-dihydro-11H-dibenzo[b,e][1,4]diazepin-11-one

### Example 508A

### 7-[2-(tert-butyl-dimethyl-silanyloxy)-ethoxy]-3-chloro-5-(2-trimethylsilanyl-ethoxymethyl)-5,10-dihydro-dibenzo[b,e][1,4]diazepin-11-one

The desired product was prepared by substituting Example 507B and (2-bromoethoxy)-tert-butyldimethylsilane for Example 476 and 2-bromomethyl-tetrahydro-2H-pyran, respectively, in Example 478.

### Example 508B

### 7-[2-(tert-butyl-dimethyl-silanyloxy)-ethoxy]-3-(3-methoxy-4-nitro-phenyl)-5,10-dihydro-dibenzo[b,e][1,4]diazepin-11-one

The desired product was prepared by substituting Example 508A for Example 476G in Example 476H.

### Example 508C

### 7-(2-hydroxyethoxy)-3-(3-methoxy-4-nitrophenyl)-5,10-dihydro-11H-dibenzo[b,e][1,4]diazepin-11-one

The desired product was prepared by substituting Example 508B for Example 500A in Example 500B. MS (ESI) m/e 422 (M+H)⁺; ¹H NMR (300 MHz, DMSO-d₆) δ 9.77 (s, 1H), 8.02 (d, J =8.5 Hz, 1H), 7.97 (s, 1H), 7.79 (d, J =7.8 Hz, 1H), 7.53 (d, J =1.4 Hz, 1H), 7.30-7.37 (m, 3H), 6.88 (d, J =8.8 Hz, 1H), 6.64 (d, J =2.7 Hz, 1H), 6.53 (dd, J =2.7, 8.8 Hz, 1H), 4.83 (t, J =5.6 Hz, 1H), 4.03 (s, 3H), 3.90 (t, J =4.9 Hz, 2H), 3.68 (q, J =5.2 Hz, 2H).

### Example 509

### 7-(2,3-dihydroxypropoxy)-3-(3-methoxy-4-nitrophenyl)-5,10-dihydro-11H-dibenzo[b,e][1,4]diazepin-11-one

### Example 509A

### 3-chloro-7-(2,3-dihydroxypropoxy)-5,10-dihydro-11H-dibenzo[b,e][1,4]diazepin-11-one

The desired product was prepared by substituting Example 507B and 3-chloro-1,2-propanediol for Example 476 and 2-bromomethyl-tetrahydro-2H-pyran, respectively, in Example 478.

### Example 509B

### 7-(2,3-dihydroxypropoxy)-3-(3-methoxy-4-nitrophenyl)-5,10-dihydro-11H-dibenzo[b,e][1,4]diazepin-11-one

The desired product was prepared by substituting Example 509A for Example 476G in Example 476H. MS (ESI) m/e 452 (M+H)⁺; ¹H NMR (300 MHz, DMSO-d₆) δ 9.77 (s, 1H), 8.01 (d, J =8.1 Hz, 1H), 7.96 (s, 1H), 7.79 (d, J =8.1 Hz, 1H), 7.53 (d, J =1.7 Hz, 1H), 7.30-7.37 (m, 3H), 6.87 (d, J =8.5 Hz, 1H), 6.65 (d, J =2.4 Hz, 1H), 6.52 (dd, J =2.7, 8.8 Hz, 1H), 4.03 (s, 3H), 3.86 (m, 1H), 3.69-3.82 (m, 2H), 3.40-3.60 (m, 4H).

### Example 510

### 7-[2-(2-methoxyethoxy)ethoxy]-3-(3-methoxy-4-nitrophenyl)-5,10-dihydro-11H-dibenzo[b,e][1,4]diazepin-11-one

### Example 510A

### 3-chloro-7-[2-(2-methoxyethoxy)ethoxy]-5,10-dihydro-11H-dibenzo[b,e][1,4]diazepin-11-one

The desired product was prepared by substituting Example 507B and 1-bromo-2-(2-methoxyethoxy)ethane for Example 476 and 2-bromomethyl-tetrahydro-2H-pyran, respectively, in Example 478.

### Example 510B

### 7-[2-(2-methoxyethoxy)ethoxy]-3-(3-methoxy-4-nitrophenyl)-5,10-dihydro-11H-dibenzo[b,e][1,4]diazepin-11-one

The desired product was prepared by substituting Example 510A for Example 476G in Example 476H. MS (ESI) m/e 480 (M+H)⁺; ¹H NMR (300 MHz, DMSO-d₆) δ 9.78 (s, 1H), 8.02 (d, J =8.1 Hz, 1H), 7.97 (s, 1H), 7.79 (d, J =8.5 Hz, 1H), 7.53 (d, J =1.7 Hz, 1H), 7.30-7.37 (m, 3H), 6.89 (d, J =8.8 Hz, 1H), 6.64 (d, J =2.7 Hz, 1H), 6.54 (dd, J =2.7, 8.5 Hz, 1H), 4.03 (s, 3H), 4.01 (dd, J =3.7, 5.4 Hz, 2H), 3.70 (dd, J =3.9, 5.6 Hz, 2H), 3.54-3.60 (m, 2H), 3.42-3.48 (m, 2H), 3.24 (s, 3H).

### Example 511

### 7-(methoxymethyl)-3-(3-methoxy-4-nitrophenyl)-5,10-dihydro-11H-dibenzo[b,e][|1,4]diazepin-11-one

### Example 511A

### methyl 4-[(tert-butoxycarbonyl)amino]-3-iodobenzoate

A mixture of methyl 4-amino-3-iodobenzoate (5.0g, 18.0 mmol), di-tert-butyl dicarbonate (4.7g, 21.7 mmol), triethylamine (7.6 mL, 54.2 mmol), 4-(dimethylamino)pyridine (22mg, 0.18 mmol), and dichloromethane (150 mL) was heated overnight at 40 °C, cooled to room temperature, concentrated under vacuum, diluted with ethyl acetate, washed with water and brine, dried (MgSO₄), filtered and concentrated under vacuum. The residue was purified by column chromatography on silica gel to give the 3.4g (50%) of the desired product. MS (ESI) m/e 395 (M+NH₄)⁺; ¹H NMR (300 MHz, DMSO-d₆) δ 8.51 (s, 1H), 8.33 (d, J=2.0 Hz, 1H), 7.92 (dd, J=2.0, 8.5 Hz, 1H), 7.67 (d, J=8.5 Hz, 1H), 3.84 (s, 3H), 1.48 (s, 9H).

### Example 511B

### 4-[(tert-butoxycarbonyl)amino]-3-iodobenzoic acid

The desired product was prepared by substituting Example 511A for Example 476F in the first step of Example 476G. The mixture was then cooled to room temperature, acidified to pH 6-7 with a saturated solution of citric acid, concentrated under vacuum, diluted with ethyl acetate, washed with water and brine, dried (MgSO₄), filtered, and concentrated under vacuum to provide 3.27g (quantitative) of the desired product. MS (ESI) m/e 362 (M-H)⁻; ¹H NMR (300 MHz, DMSO-d₆) δ 8.49 (s, 1H), 8.31 (d, J=1.7 Hz, 1H), 7.90 (d, J=1.9, 8.3 Hz, 1H), 7.61 (d, J=8.5 Hz, 1H), 1.48 (s, 9H).

### Example 511C

### tert-butyl 4-(hydroxymethyl)-2-iodophenylcarbamate

A mixture of Example 511B (363mg, 1.0 mmol) in THF (2 mL) at 0 °C was slowly treated with a 1.0M solution of borane in THF (2.0 mL, 2.0 mmol), allowed to reach room temperature, slowly quenched with a saturated solution of ammonium chloride and extracted with ethyl acetate. The combined organic extracts were dried (MgSO₄), filtered, and concentrated under vacuum. The residue was purified by column chromatography on silica gel to provide 275mg (79%) of the desired product. MS (ESI) m/e 350 (M+H)⁺; ¹H NMR (300 MHz, DMSO-d₆) δ 8.44 (s, 1H), 7.77 (d, J=0.7 Hz, 1H), 7.25-7.32 (m, 2H), 5.25 (t, J=5.8 Hz, 1H), 4.44 (d, J=5.4 Hz, 2H), 1.45 (s, 9H).

### Example 511D

### methyl 2-{[2-[(tert-butoxycarbonyl)amino]-5-(hydroxymethyl)phenyl]amino}-4-chlorobenzoate

The desired product was prepared by substituting Example 511C and methyl 2-amino-4-chlorobenzoate for methyl 4-chloro-2-iodobenzoate and methyl 3,4-diaminobenzoate, respectively, in Example 1A. MS (ESI) m/e 407 (M+H)⁺; ¹H NMR (300 MHz, DMSO-d₆) δ 9.18 (s, 1H), 8.73 (s, 1H), 7.86 (d, J=8.5 Hz, 1H), 7.32 (d, J=8.1 Hz, 1H), 7.28 (d, J=1.7 Hz, 1H), 7.15 (dd, J=1.7, 8.1 Hz, 1H), 6.75 (dd, J=2.0, 8.5 Hz, 1H), 6.00 (d, J=2.0 Hz, 1H), 5.22 (t, J=5.8 Hz, 1H), 4.48 (d, J=5.4 Hz, 2H), 3.84 (s, 3H), 1.38 (s, 9H).

### Example 511E

### 2-{[2-[(tert-butoxycarbonyl)amino]-5-(hydroxymethyl)phenyl]amino}-4-chlorobenzoic acid

The desired product was prepared by substituting Example 511D for Example 511A in Example 511B. ¹H NMR (300 MHz, DMSO-d₆) δ 13.08 (br s, 1H), 9.44 (s, 1H), 8.66 (s, 1H), 7.85 (d, J=8.8 Hz, 1H), 7.32 (d, J=8.5 Hz, 1H), 7.27 (d, J=1.7 Hz, 1H), 7.13 (dd, J=2.0, 8.1 Hz, 1H), 6.72 (dd, J=2.0, 8.5 Hz, 1H), 6.61 (d, J=2.0 Hz, 1H), 5.21 (t, J=5.8 Hz, 1H), 4.47 (d, J=5.4 Hz, 2H), 1.38 (s, 9H).

### Example 511F

### 2-{[2-amino-5-(hydroxymethyl)phenyl]amino}-4-chlorobenzoic acid

The desired product was prepared by substituting Example 511E for Example 489B in Example 489C.

### Example 511 G

### 3-chloro-7-(methoxymethyl)-5,10-dihydro-11H-dibenzo[b,e][1,4]diazepin-11-one

The desired product was prepared by substituting Example 511F for Example 224F in Example 224G. MS (ESI) m/e 289 (M+H)⁺; ¹H NMR (300 MHz, DMSO-d₆) δ 9.92 (s, 1H), 8.05 (s, 1H), 7.68 (d, J=8.5 Hz, 1H), 7.07 (d, J=2.0 Hz, 1H), 6.94 (m, 3H), 6.85 (dd, J=1.7, 8.1 Hz, 1H), 4.29 (s, 2H), 3.26 (s, 3H).

### Example 511H

### 7-(methoxymethyl)-3-(3-methoxy-4-nitrophenyl)-5,10-dihydro-11H-dibenzo[b,e][1,4]diazepin-11-one

The desired product was prepared by substituting Example 511G for Example 476G in Example 476H. MS (ESI) m/e 406 (M+H)⁺; ¹H NMR (300 MHz, DMSO-d₆) δ 9.92 (s, 1H), 8.02 (s, 1H), 8.01 (d, J =8.5 Hz, 1H), 7.80 (d, J =8.1 Hz, 1H), 7.53 (d, J =1.7 Hz, 1H), 7.29-7.36 (m, 3H), 7.01 (d, J =1.4 Hz, 1H), 6.95 (d, J =8.1 Hz, 1H), 6.85 (dd, J =1.7, 8.1 Hz, 1H), 4.30 (s, 2H), 4.03 (s, 3H), 3.27 (s, 3H).

### Example 512 (A-838047.0)

### 7-(3-methoxy-4-nitrobenzyl)-3-(3-methoxy-4-nitrophenyl)-5,10-dihydro-11H-dibenzo[b,e][1,4]diazepin-11-one

### Example 512A

### 7-(bromomethyl)-3-chloro-5,10-dihydro-11H-dibenzo[b,e][1,4]diazepin-11-one

The desired product was prepared by substituting Example 511G for Example 506C in Example 507A. MS (ESI) m/e 337 (M+H)⁺.

### Example 512B

### 7-(3-methoxy-4-nitrobenzyl)-3-(3-methoxy-4-nitrophenyl)-5,10-dihydro-11H-dibenzo[b,e][1,4]diazepin-11-one

The desired product was prepared by substituting Example 512A for Example 476G in Example 476H. MS (ESI) m/e 527 (M+H)⁺; ¹H NMR (300 MHz, DMSO-d₆) δ 9.87 (s, 1H), 8.01 (d, J =8.5 Hz, 1H), 7.98 (s, 1H), 7.81 (t, J =8.5 Hz, 2H), 7.51 (d, J =1.7 Hz, 1H), 7.26-7.34 (m, 4H), 6.81-6.94 (m, 4H), 4.02 (s, 3H), 3.94 (s, 2H), 3.91 (s, 3H).

### Example 513

### 7-{[[2-(dimethylamino)ethyl](methyl)amino]methyl}-3-(3-methoxy-4-nitrophenyl)-5,10-dihydro-11H-dibenzo[b,e][1,4]diazepin-11-one

### Example 513A

### 3-chloro-7-{[[2-(dimethylamino)ethyl](methyl)amino]methyl}-5,10-dihydro-11H-dibenzo[b,e][1,4]diazepin-11-one

A mixture of Example 512A (50mg, 0.15 mmol) and N,N,N'-trimethylethylenediamine (300 µL, 2.3 mmol) was heated at 50 °C overnight, cooled to room temperature, diluted with ethyl acetate, washed with water and brine, dried (MgSO₄), filtered, and concentrated under vacuum to provide 31.5mg (59%) of the desired product.

### Example 513B

### 7-{{[[2-(dimethylamino)ethyl](methyl)amino]methyl}-3-(3-methoxy-4-nitrophenyl)-5,10-dihydro-11H-dibenzo[b,e][1,4]diazepin-11-one

The desired product was prepared by substituting Example 513A for Example 476G in Example 476H. MS (ESI) m/e 476 (M+H)⁺; ¹H NMR (300 MHz, DMSO-d₆) δ 10.02 (s, 1H), 8.11 (s, 1H), 8.03 (d, J =8.5 Hz, 1H), 7.82 (d, J =8.1 Hz, 1H), 7.52 (d, J =1.7 Hz, 1H), 7.30-7.40 (m, 3H), 6.99-7.05 (m, 3H), 4.04 (s, 3H), 3.80-4.20 (m, 6H), 3.33 (br s, 3H), 2.79 (s, 6H).

### Example 514

### 3-(3-methoxy-4-nitrophenyl)-7-{[(2-tetrahydro-2H-pyran-4-ylethyl)amino]methyl}-5,10-dihydro-11H-dibenzo[b,e][1,4]diazepin-11-one

### Example 514A

### 3-chloro-7-{[(2-tetrahydro-2H-pyran-4-ylethyl)amino]methyl}-5,10-dihydro-11H-dibenzo [b,e][1,4]diazepin-11-one

A mixture of Example 512A (50mg, 0.15 mmol) and 2-(tetrahydro-pyran-4-yl)-ethylamine (38.3 mg, 0.30 mmol) in dioxane (2.0 mL) was heated 11 hours at 50 °C, cooled to room temperature, and concentrated under vacuum. The residue was purified by preparative HPLC to give 30.3 mg (53%) of the desired product. ¹H NMR (300 MHz, DMSO-d₆) δ 10.05 (s, 1H), 8.56 (br s, 1H), 8.16 (s, 1H), 7.70 (d, J=8.5 Hz, 1H), 7.11 (d, J=2.0 Hz, 1H), 7.00-7.08 (m, 3H), 6.97 (dd, J=2.0, 8.5 Hz, 1H), 4.00-4.04 (m, 2H), 3.78-3.86 (m, 2H), 3.20-3.30 (m, 3H), 2.88-3.00 (m, 2H), 1.49-1.58 (m, 4H), 1.11-1.19 (m, 2H).

### Example 514B

### 3-(3-methoxy-4-nitrophenyl)-7-{[(2-tetrahydro-2H-pyran-4-ylethyl)amino]methyl}-5,10-dihydro-11H-dibenzo[b,e][1,4]diazepin-11-one

The desired product was prepared by substituting Example 514A for Example 476G in Example 476H. MS (ESI) m/e 503 (M+H)⁺; ¹H NMR (300 MHz, DMSO-d₆) δ 10.05 (s, 1H), 8.59 (m, 1H), 8.15 (s, 1H), 8.03 (d, J =8.5 Hz, 1H), 7.82 (d, J =8.1 Hz, 1H), 7.51 (d, J =1.7 Hz, 1H), 7.40 (d, J =1.7 Hz, 1H), 7.30-7.38 (m, 2H), 7.00-7.08 (m, 3H), 4.03 (s, 3H), 3.98-4.07 (m, 2H), 3.77-3.85 (m, 2H), 3.18-3.31 (m, 3H), 2.87-3.01 (m, 2H), 1.46-1.59 (m, 4H), 1.06-1.26 (m, 2H).

### Example 515

### 8-ethyl-7-methoxy-3-(3-methoxy-4-nitrophenyl)-5,10-dihydro-11H-dibenzo[b,e][1,4]diazepin-11-one

### Example 515A

### 3-chloro-8-hydroxy-7-methoxy-5,10-dihydro-11H-dibenzo[b,e][1,4]diazepin-11-one

The desired product was prepared by substituting Example 477G for Example 476H in Example 476I. ¹H NMR (300 MHz, DMSO-d₆) δ 9.65 (s, 1H), 7.69 (s, 1H), 7.63 (d, J=8.5 Hz, 1H), 7.02 (d, J=2.0 Hz, 1H), 6.89 (dd, J=2.0, 8.5 Hz, 1H), 6.58 (s, 1H), 6.46 (s, 1H), 3.70 (s, 3H).

### Example 515B

### 3-chloro-7-methoxy-11-oxo-10,11-dihydro-5H-dibenzo[b,e][1,4]diazepin-8-yl trifluoromethanesulfonate

A mixture of Example 515A (290.3mg, 1.0 mmol) in THF (10 mL) was treated with NaH (60% oil dispersion, 44mg, 1.1 mmol) and stirred 10 minutes at room temperature. N-phenyltrifluoromethanesulfonimide (429mg, 1.2 mmol) was added and the mixture was stirred overnight, quenched with water, concentrated under vacuum, diluted with ethyl acetate, washed with water and brine, dried (MgSO₄), filtered, and concentrated under vacuum. The residue was triturated with ethyl acetate and hexane, filtered, and dried under vacuum to provide 211mg (50%) of the desired product. MS (ESI) m/e 423 (M+H)⁺; ¹H NMR (300 MHz, DMSO-d₆) δ 9.67 (s, 1H), 7.70 (s, 1H), 7.65 (d, J=8.5 Hz, 1H), 7.03 (d, J=2.0 Hz, 1H), 6.91 (dd, J=2.0, 8.5 Hz, 1H), 6.59 (s, 1H), 6.48 (s, 1H), 3.71 (s, 3H).

### Example 515C

### 3-chloro-7-methoxy-8-vinyl-5,10-dihydro-11H-dibenzo[b,e][1,4]diazepin-11-one

A mixture of Example 515B (210mg, 0.5 mmol), tributyl(vinyl)tin (175 µL, 0.6 mmol), Pd(PPh₃)₂Cl₂ (35mg, 0.05 mmol), LiCl (169mg, 4.0 mmol) and DMF was flushed with nitrogen, heated at 50 °C overnight, cooled to room temperature, diluted with a saturated potassium fluoride solution, stirred 15 minutes, diluted with ethyl acetate, washed with water and brine, dried (MgSO₄), filtered, and concentrated under vacuum. The residue was purified by column chromatography on silica gel with a solvent gradient of 4:1 to 1:1 hexane/ethyl acetate to give 77mg (52%) of the desired product. MS (ESI) m/e 301 (M+H)⁺; ¹H NMR (300 MHz, DMSO-d₆) δ 9.72 (s, 1H), 8.14 (s, 1H), 7.68 (d, J=8.5 Hz, 1H), 7.11 (s, 1H), 7.05 (d, J=2.0 Hz, 1H), 6.94 (dd, J=2.0, 8.5 Hz, 1H), 6.80 (dd, J=11.2, 18.0 Hz, 1H), 6.63 (s, 1H), 5.56 (dd, J=1.5, 17.8 Hz, 1H), 5.14 (dd, J=1.7, 11.2 Hz, 1H), 3.75 (s, 3H).

### Example 515D

### 3-chloro-8-ethyl-7-methoxy-5,10-dihydro-11H-dibenzo[b,e][1,4]diazepin-11-one

The desired product was prepared by substituting Example 515C for Example 476A in Example 476B. MS (ESI) m/e 303 (M+H)⁺; ¹H NMR (300 MHz, DMSO-d₆) δ 9.68 (s, 1H), 7.93 (s, 1H), 7.66 (d, J=8.5 Hz, 1H), 7.05 (d, J=2.0 Hz, 1H), 6.92 (dd, J=2.0, 8.5 Hz, 1H), 6.75 (s, 1H), 6.59 (s, 1H), 3.73 (s, 3H), 2.43 (q, J=7.6 Hz, 2H), 1.06 (t, J=7.5 Hz, 3H).

### Example 515E

### 8-ethyl-7-methoxy-3-(3-methoxy-4-nitrophenyl)-5,10-dihydro-11H-dibenzo[b,e][1,4]diazepin-11-one

The desired product was prepared by substituting Example 515D for Example 476G in Example 476H. MS (ESI) m/e 420 (M+H)⁺; ¹H NMR (300 MHz, DMSO-d₆) δ 9.67 (s, 1H), 8.01 (d, J =8.5 Hz, 1H), 7.90 (s, 1H), 7.78 (d, J =8.1 Hz, 1H), 7.53 (d, J =1.7 Hz, 1H), 7.27-7.38 (m, 3H), 6.76 (s, 1H), 6.66 (s, 1H), 4.03 (s, 3H), 3.73 (s, 3H), 2.43 (q, J =7.5 Hz, 2H), 1.06 (t, J =7.6 Hz, 3H).

### Example 516

### 8-methoxy-3-(3-methoxy-4-nitrophenyl)-7-vinyl-5,10-dihydro-11H-dibenzo[b,e][1,4]diazepin-11-one

### Example 516A

### 3-chloro-8-methoxy-11-oxo-10,11-dihydro-5H-dibenzo[b,e][1,4]diazepin-7-yl trifluoromethanesulfonate

The desired product was prepared by substituting Example 501A for Example 515A in Example 515B. MS (ESI) m/e 423 (M+H)⁺; ¹H NMR (300 MHz, DMSO-d₆) δ 10.03 (s, 1H), 8.05 (s, 1H), 7.69 (d, J=8.5 Hz, 1H), 7.07 (s, 1H), 7.01 (d, J=2.0 Hz, 1H), 6.98 (dd, J=2.0, 8.5 Hz, 1H), 6.92 (s, 1H), 3.79 (s, 3H).

### Example 516B

### 3-chloro-8-methoxy-7-vinyl-5,10-dihydro-11H-dibenzo[b,e][1,4]diazepin-11-one

The desired product was prepared by substituting Example 516A for Example 515B in Example 515C. MS (ESI) m/e 301 (M+H)⁺; ¹H NMR (300 MHz, DMSO-d₆) δ 9.89 (s, 1H), 7.88 (s, 1H), 7.67 (d, J=8.5 Hz, 1H), 7.12 (s, 1H), 7.04 (d, J=2.0 Hz, 1H), 6.91 (dd, J=2.2, 8.6 Hz, 1H), 6.82 (dd, J=11.2, 18.0 Hz, 1H), 6.67 (s, 1H), 5.60 (dd, J=1.7,17.6 Hz, 1H), 5.19 (dd, J=1.4, 11.2 Hz, 1H), 3.71 (s, 3H).

### Example 516C

### 8-methoxy-3-(3-methoxy-4-nitrophenyl)-7-vinyl-5,10-dihydro-11H-dibenzo[b,e][1,4]diazepin-11-one

The desired product was prepared by substituting Example 516B for Example 476G in Example 476H. MS (ESI) m/e 418 (M+H)⁺; ¹H NMR (400 MHz, DMSO-d₆) δ 9.88 (s, 1H), 8.01 (d, J =8.3 Hz, 1H), 7.83 (s, 1H), 7.80 (d, J =8.3 Hz, 1H), 7.54 (s, 1H), 7.26-7.40 (m, 3H), 7.18 (s, 1H), 6.83 (dd, J =11.2, 17.6 Hz, 1H), 6.69 (s, 1H), 5.61 (d, J =17.8 Hz, 1H), 5.19 (d, J =11.4 Hz, 1H), 4.04 (s, 3H), 3.72 (s, 3H).

### Example 517

### 8-(3-hydroxypropyl)-7-methoxy-3-(3-methoxy-4-nitrophenyl)-5,10-dihydro-11H-dibenzo[b,e][1,4]diazepin-11-one

### Example 517A

### N-(4-bromo-3-methoxyphenyl)acetamide

The desired product was prepared by substituting 1-bromo-2-methoxy-4-nitro-benzene for 2-bromo-1-methoxy-4-nitro-benzene in Example 223A. MS (DCI) m/e 244 (M+H)⁺; ¹H NMR (300 MHz, DMSO-d₆) δ 10.05 (s, 1H), 7.448 (d, J=2.4 Hz, 1H), 7.445 (d, J=8.5 Hz, 1H), 7.10 (dd, J=2.2, 8.6 Hz, 1H), 3.79 (s, 3H), 2.04 (s, 3H).

### Example 517B

### N-(4-bromo-5-methoxy-2-nitrophenyl)acetamide

The desired product was prepared by substituting Example 517A for Example 223A in Example 223B. MS (DCI) m/e 306 (M+NH₄)⁺; ¹H NMR (300 MHz, DMSO-d₆) δ 10.37 (s, 1H), 8.25 (s, 1H), 7.63 (s, 1H), 3.94 (s, 3H), 2.13 (s, 3H).

### Example 517C

### 4-bromo-5-methoxy-2-nitroaniline

The desired product was prepared by substituting Example 517B for Example 223B in Example 223C. MS (DCI) m/e 247 (M+H)⁺; ¹H NMR (300 MHz, DMSO-d₆) δ 8.12 (s, 1H), 7.62 (s, 2H), 6.60 (s, 1H), 3.86 (s, 3H).

### Example 517D

### methyl 2-[(4-bromo-5-methoxy-2-nitrophenyl)amino]-4-chlorobenzoate

The desired product was prepared by substituting Example 517C for methyl 3,4-diaminobenzoate in Example 1A. MS (ESI) m/e 415 (M+H)⁺.

### Example 517E

### 8-bromo-3-chloro-7-methoxy-5,10-dihydro-11H-dibenzo[b,e][1,4]diazepin-11-one

The desired product was prepared by substituting Example 517D for Example 476F in Example 476G. MS (ESI) m/e 353 (M+H)⁺; ¹H NMR (300 MHz, DMSO-d₆) δ 9.81 (s, 1H), 8.16 (s, 1H), 7.68 (d, J=8.5 Hz, 1H), 7.15 (s, 1H), 7.05 (d, J=2.0 Hz, 1H), 6.96 (dd, J=2.0, 8.5 Hz, 1H), 6.73 (s, 1H), 3.78 (s, 3H).

### Example 517F

### ethyl 3-(3-chloro-7-methoxy-11-oxo-10,11-dihydro-5H-dibenzo[b,e][1,4]diazepin-8-yl)acrylate

The desired product was prepared by substituting Example 517E and ethyl acrylate for Example 223G and methyl acrylate, respectively, in Example 223H. MS (ESI) m/e 373 (M+H)⁺; ¹H NMR (300 MHz, DMSO-d₆) δ 9.75 (s, 1H), 8.41 (s, 1H), 7.72 (d, J=8.5 Hz, 1H), 7.68 (d, J=15.9 Hz, 1H), 7.23 (s, 1H), 7.06 (d, J=2.0 Hz, 1H), 6.97 (dd, J=2.0, 8.5 Hz, 1H), 6.69 (s, 1H), 6.31 (d, J=16.3 Hz, 1H), 4.16 (q, J=7.1 Hz, 2H), 3.83 (s, 3H), 1.24 (t, J=7.0 Hz, 3H).

### Example 517G

### ethyl 3-(3-chloro-7-methoxy-11-oxo-10,11-dihydro-5H-dibenzo[b,e][1,4]diazepin-8-yl)propanoate

The desired product was prepared by substituting Example 517F and methanol for Example 476A and ethanol, respectively, in Example 476B. MS (ESI) m/e 375 (M+H)⁺; ¹H NMR (300 MHz, DMSO-d₆) δ 9.70 (s, 1H), 7.95 (s, 1H), 7.66 (d, J=8.5 Hz, 1H), 7.04 (d, J=2.0 Hz, 1H), 6.92 (dd, J=2.0, 8.5 Hz, 1H), 6.74 (s, 1H), 6.60 (s, 1H), 4.03 (q, J=7.1 Hz, 2H), 3.73 (s, 3H), 2.67 (t, J=7.5 Hz, 2H), 2.45 (t, J=7.3 Hz, 2H), 1.15 (t, J=7.1 Hz, 3H).

### Example 517H

### 3-chloro-8-(3-hydroxypropyl)-7-methoxy-5,10-dihydro-11H-dibenzo[b,e][1,4]diazepin-11-one

The desired product was prepared by substituting 517G for Example 6D in Example 204A. MS (ESI) m/e 333 (M+H)⁺; ¹H NMR (300 MHz, DMSO-d₆) δ 9.67 (s, 1H), 7.92 (s, 1H), 7.66 (d, J=8.5 Hz, 1H), 7.04 (d, J=2.0 Hz, 1H), 6.92 (dd, J=2.0, 8.5 Hz, 1H), 6.73 (s, 1H), 6.59 (s, 1H), 4.39 (t, J=5.1 Hz, 1H), 3.72 (s, 3H), 3.35-3.41 (m, 2H), 2.40-2.45 (m, 2H), 1.54-1.64 (m, 2H).

### Example 517I

### 8-(3-hydroxypropyl)-7-methoxy-3-(3-methoxy-4-nitrophenyl)-5,10-dihydro-11H-dibenzo[b,e][1,4]diazepin-11-one

The desired product was prepared by substituting Example 517H for Example 476G in Example 476H. MS (ESI) m/e 450 (M+H)⁺; ¹H NMR (300 MHz, DMSO-d₆) δ 9.68 (s, 1H), 8.02 (d, J =8.5 Hz, 1H), 7.90 (s, 1H), 7.78 (d, J =8.1 Hz, 1H), 7.53 (d, J =1.7 Hz, 1H), 7.27-7.38 (m, 3H), 6.75 (s, 1H), 6.66 (s, 1H), 4.40 (t, J =5.1 Hz, 1H), 4.04 (s, 3H), 3.72 (s, 3H), 3.34-3.43 (m, 2H), 2.39-2.47 (m, 2H), 1.57-1.64 (m, 2H).

### Example 518

### 7-methoxy-3-(3-methoxy-4-nitrophenyl)-8-{3-[(2-methylpyridin-3-yl)oxy]propyl}-5,10-dihydro-11H-dibenzo[b,e][1,4]diazepin-11-one

### Example 518A

### 3-chloro-7-methoxy-8-{3-[(2-methylpyridin-3-yl)oxy]propyl}-5,10-dihydro-11H-dibenzo[b,e][1,4]diazepin-11-one

The desired product was prepared by substituting Example 517H and 2-methyl-3-hydroxypyridine for Example 204A and 4-morpholinophenol, respectively, in Example 297A. MS (ESI) m/e 424 (M+H)⁺; ¹H NMR (300 MHz, DMSO-d₆) δ 9.67 (s, 1H), 7.97 (dd, J=1.4, 4.8 Hz, 1H), 7.94 (s, 1H), 7.65 (d, J=8.5 Hz, 1H), 7.26 (m, 1H), 7.14 (m, 1H), 7.04 (d, J=2.0 Hz, 1H), 6.92 (dd, J=2.0, 8.5 Hz, 1H), 6.77 (s, 1H), 6.60 (s, 1H), 3.97 (t, J=6.3 Hz, 2H), 3.69 (s, 3H), 2.58-2.63 (m, 2H), 2.36 (s, 3H), 1.88-1.97 (m, 2H).

### Example 518B

### 7-methoxy-3-(3-methoxy-4-nitrophenyl)-8-{3-[(2-methylpyridin-3-yl)oxy]propyl}-5,10-dihydro-11H-dibenzo[b,e][1,4]diazepin-11-one

The desired product was prepared by substituting Example 518A for Example 476G in Example 476H. MS (ESI) m/e 541 (M+H)⁺; ¹H NMR (300 MHz, DMSO-d₆) δ 9.67 (s, 1H), 8.17 (d, J =4.8 Hz, 1H), 8.02 (d, J =8.5 Hz, 1H), 7.93 (s, 1H), 7.78 (d, J =8.5 Hz, 1H), 7.69 (in, 1H), 7.51 (m, 1H), 7.53 (d, J =1.4 Hz, 1H), 7.29-7.36 (m, 3H), 6.78 (s, 1H), 6.67 (s, 1H), 4.08 (t, J =6.1 Hz, 2H), 4.03 (s, 3H), 3.69 (s, 3H), 2.62 (t, J =7.5 Hz, 2H), 2.47 (s, 3H), 1.90-2.02 (m, 2H).

### Example 519

### 8-{3-[(2-chloropyridin-3-yl)oxy]propyl}-7-methoxy-3-(3-methoxy-4-nitrophenyl)-5,10-dihydro-11H-dibenzo[b,e][1,4]diazepin-11-one

The desired product was prepared by substituting Example 517I and 2-chloro-3-hydroxypyridine for Example 204A and 4-morpholinophenol, respectively, in Example 297A. MS (ESI) m/e 561 (M+H)⁺; ¹H NMR (300 MHz, DMSO-d₆) δ 9.68 (s, 1H), 8.01 (d, J =8.5 Hz, 1H), 7.96 (dd, J =1.4,4.8 Hz, 1H), 7.92 (s, 1H), 7.78 (d, J =7.8 Hz, 1H), 7.52-7.56 (m, 2H), 7.29-7.39 (m, 4H), 6.78 (s, 1H), 6.67 (s, 1H), 4.08 (t, J =6.3 Hz, 2H), 4.03 (s, 3H), 3.69 (s, 3H), 2.58-2.65 (m, 2H), 1.90-1.98 (m, 2H).

### Example 520

### 7-methoxy-3-(3-methoxy-4-nitrophenyl)-8-[3-(4-morpholin-4-ylphenoxy)propyl]-5,10-dihydro-11H-dibenzo[b,e][1,4]diazepin-11-one

### Example 520A

### 3-chloro-7-methoxy-8-[3-(4-morpholin-4-ylphenoxy)propyl]-5,10-dihydro-11H-dibenzo[b,e][1,4]diazepin-11-one

The desired product was prepared by substituting Example 517H for Example 204A in Example 297A. MS (ESI) m/e 494 (M+H)⁺; ¹H NMR (300 MHz, DMSO-d₆) δ 9.68 (s, 1H), 7.95 (s, 1H), 7.60 (d, J=8.5 Hz, 1H), 7.05 (d, J=2.0 Hz, 1H), 6.92 (dd, J=2.0, 8.5 Hz, 1H), 6.79-6.86 (m, 4H), 6.76 (s, 1H), 6.60 (s, 1H), 3.87 (t, J=6.3 Hz, 2H), 3.72 (s, 3H), 3.70-3.73 (m, 4H), 2.95-2.98 (m, 4H), 2.53-2.58 (m, 2H), 1.81-1.90 (m, 2H).

### Example 520B

### 7-methoxy-3-(3-methoxy-4-nitrophenyl)-8-[3-(4-morpholin-4-ylphenoxy)propyl]-5,10-dihydro-11H-dibenzo[b,e][1,4]diazepin-11-one

The desired product was prepared by substituting Example 520A for Example 476G in Example 476H. MS (ESI) m/e 611 (M+H)⁺; ¹H NMR (300 MHz, DMSO-d₆) δ 9.68 (s, 1H), 8.01 (d, J =8.1 Hz, 1H), 7.92 (s, 1H), 7.78 (d, J =8.1 Hz, 1H), 7.53 (d, J =1.7 Hz, 1H), 7.29-7.37 (m, 3H), 6.87-6.95 (m, 2H), 6.80-6.86 (m, 2H), 6.77 (s, 1H), 6.67 (s, 1H), 4.03 (s, 3H), 3.88 (t, J =6.4 Hz, 2H) 3.70-3.75 (m, 4H), 3.72 (s, 3H), 2.98-3.04 (m, 4H), 2.50-2.60 (m, 2H), 1.84-1.91 (m, 2H).

### Example 521

### 8-[3-(isoquinolin-3-yloxy)propyl]-7-methoxy-3-(3-methoxy-4-nitrophenyl)-5,10-dihydro-11H-dibenzo[b,e][1,4]diazepin-11-one

The desired product was prepared by substituting Example 517I and 2-hydroxyisoquinoline for Example 204A and 3-methyl-2-pyridol , respectively, in Example 301. MS (ESI) m/e 577 (M+H)⁺; ¹H NMR (500 MHz, DMSO-d₆) δ 9.66 (s, 1H), 9.03 (s, 1H), 8.01 (d, J =8.4 Hz, 2H), 7.91 (s, 1H), 7.76-7.81 (m, 2H), 7.64 (m, 1H), 7.53 (d, J =1.3 Hz, 1H), 7.42 (t, J =7.5 Hz, 1H), 7.33-7.37 (m, 2H), 7.30 (m, 1H), 7.15 (s, 1H), 6.81 (s, 1H), 6.68 (s, 1H), 4.30 (t, J =6.4 Hz, 2H), 4.04 (s, 3H), 3.71 (s, 3H), 2.62 (t, J =7.6 Hz, 2H), 1.93-2.00 (m, 2H).

### Example 522

### 7-methoxy-3-(3-methoxy-4-nitrophenyl)-8-[3-(3-oxoisoquinolin-2(3H)-yl)propyl]-5,10-dihydro-11H-dibenzo[b,e][1,4]diazepin-11-one

The title compound was isolated as a second product in Example 521. MS (ESI) m/e 577 (M+H)⁺; ¹H NMR (500 MHz, DMSO-d₆) δ 9.63 (s, 1H), 8.74 (s, 1H), 8.01 (d, J =8.1 Hz, 1H), 7.91 (s, 1H), 7.78 (d, J =8.1 Hz, 1H), 7.51-7.54 (m, 2H), 7.23-7.36 (m, 5H), 6.89 (m, 1H), 6.78 (s, 1H), 6.66 (s, 1H), 6.50 (s, 1H), 4.16 (t, J =7.2 Hz, 2H), 4.03 (s, 3H), 3.70 (s, 3H), 2.46-2.54 (m, 2H), 1.91-1.98 (m, 2H).

### Example 523

### methyl 7-methoxy-3-(3-methoxy-4-nitrophenyl)-11-oxo-10,11-dihydro-5H-dibenzo[b,e][1,4]diazepine-8-carboxylate

### Example 523A

### methyl 4-amino-2-methoxy-5-nitrobenzoate

The desired product was prepared by substituting methyl 4-acetylamino-2-methoxy-5-nitrobenzoate for Example 476D in Example 476E. MS (ESI) m/e 225 (M-H)⁻; ¹H NMR (300 MHz, DMSO-d₆) δ 8.50 (s, 1H), 7.85 (s, 2H), 6.55 (s, 1H), 3.82 (s, 3H), 3.74 (s, 3H).

### Example 523B

### methyl 4-{[5-chloro-2-(methoxycarbonyl)phenyl]amino}-2-methoxy-5-nitrobenzoate

The desired product was prepared by substituting Example 523A for methyl 3,4-diaminobenzoate in Example 1A. MS (ESI) m/e 395 (M+H)⁺; ¹H NMR (300 MHz, DMSO-d₆) δ 11.24 (s, 1H), 8.61 (s, 1H), 8.00 (d, J=8.5 Hz, 1H), 7.91 (d, J=2.0 Hz, 1H), 7.31 (dd, J=2.0, 8.5 Hz, 1H), 7.09 (s, 1H), 3.88 (s, 3H), 3.84 (s, 3H), 3.81 (s, 3H).

### Example 523C

### methyl 3-chloro-7-methoxy-11-oxo-10,11-dihydro-5H-dibenzo[b,e][1,4]diazepine-8-carboxylate

The desired product was prepared by substituting Example 523B for Example 476F in Example 476G. MS (ESI) m/e 333 (M+H)⁺; ¹H NMR (300 MHz, DMSO-d₆) δ 9.86 (s, 1H), 8.44 (s, 1H), 7.71 (d, J=8.5 Hz, 1H), 7.41 (s, 1H), 7.06 (d, J=1.7 Hz, 1H), 6.98 (dd, J=2.0, 8.5 Hz, 1H), 6.72 (s, 1H), 3.77 (s, 3H), 3.73 (s, 3H).

### Example 523D

### methyl 7-methoxy-3-(3-methoxy-4-nitrophenyl)-11-oxo-10,11-dihydro-5H-dibenzo[b,e][1,4]diazepine-8-carboxylate

The desired product was prepared by substituting Example 523C for Example 476G in Example 476H. MS (ESI) m/e 450 (M+H)⁺; ¹H NMR (500 MHz, DMSO-d₆) δ 9.84 (s, 1H), 8.44 (s, 1H), 8.01 (d, J =8.4 Hz, 1H), 7.84 (d, J =8.7 Hz, 1H), 7.55 (d, J =1.3 Hz, 1H), 7.43 (s, 1H), 7.34-7.37 (m, 3H), 6.79 (s, 1H), 4.04 (s, 3H), 3.77 (s, 3H), 3.74 (s, 3H).

### Example 524

### methyl 7-methoxy-11-oxo-3-(pyrimidin-4-ylamino)-10,11-dihydro-5H-dibenzo[b,e][1,4]diazepine-8-carboxylate

The desired product was prepared by substituting Example 523C and 4-aminopyrimidine for Example 189A and 4-aminopyridine, respectively, in Example 191. MS (ESI) m/e 392 (M+H)⁺; ¹H NMR (500 MHz, DMSO-d₆) δ 10.49 (s, 1H), 9.65 (s, 1H), 8.86 (s, 1H), 8.39-8.40 (m, 2H), 7.73 (d, J =8.5 Hz, 1H), 7.50 (d, J =2.0 Hz, 1H), 7.40 (s, 1H), 7.12 (dd, J =2.0, 8.5 Hz, 1H), 7.00 (d, J =5.0 Hz, 1H), 6.80 (s, 1H), 3.76 (s, 3H), 3.73 (s, 3H).

### Example 525

### 3-[(2,6-difluoropyridin-4-yl)amino]-8-(1,1-dimethyl-2-morpholin-4-yl-2-oxoethyl)-5,10-dihydro-11H-dibenzo[b,e][1,4]diazepin-11-one

The desired product was prepared by substituting Example 205B and morpholine for dimethylaminoacetic acid and Example 120, respectively, in Example 122. MS (ESI) m/e 494 (M+H)⁺; ¹H NMR (300 MHz, DMSO-d₆) δ 9.69 (s, 2H), 7.96 (s, 1H), 7.69 (d, J= 8.48 Hz, 1H), 6.69-6.96 (m, 2H), 6.79-6.82 (m, 2H), 6.68 (dd, J= 8.82, 2.03 Hz, 1H), 6.58 (s, 2H), 2.8-3.5 (br, 8H), 1.36 (s, 6H).

### Example 526

### 2-{3-[(2,6-difluoropyridin-4-yl)amino]-11-oxo-10,11-dihydro-5H-dibenzo[b,c][1,4]diazepin-8-yl}-N,N,2-trimethylpropanamide

The desired product was prepared by substituting Example 205B and dimethylamine hydrochloride for dimethylaminoacetic acid and Example 120, respectively, in Example 122. MS (ESI) m/e 452 (M+H)⁺; ¹H NMR (300 MHz, DMSO-d₆) δ 9.68 (s, 1H), 9.62 (s, 1H), 7.95 (s, 1H), 7.69 (d, J= 8.48 Hz, 1H), 6.9 (m, 2H), 6.82 (d, J= 2.03 Hz, 1H), 6.77 (dd, J= 8.14, 2.03 Hz, 1H), 6.68 (dd, J= 8.65, 2.2 Hz, 1H), 6.58 (s, 2H), 2.51 (br, 6H), 1.36 (m, 6H).

### Example 527

### 2-{3-[(2,6-difluoropyridin-4-yl)amino]-11-oxo-10,11-dihydro-5H-dibenzo[b,e][1,4]diazepin-8-yl }-2-methylNpyridin-4-ylpropanamide

The desired product was prepared by substituting Example 205B and 4-aminopyridine for dimethylaminoacetic acid and Example 120, respectively, in Example 122. MS (ESI) m/e 501 (M+H)⁺; ¹H NMR (300 MHz, DMSO-d₆) δ 10.3 (s, 1H), 9.67 (d, J= 9.83 Hz, 2H), 8.65 (d, J= 7.12 Hz, 2H), 8.14 (d, J= 7.46 Hz, 2H), 7.99 (s, 1H), 7.69 (d, J= 8.48 Hz, 1H), 6.93 (m, 4H), 6.67 (dd, J= 8.65, 2.20 Hz, 1H), 6.57 (s, 2H), 1.53 (s, 6H).

### Example 528

### 2-{3-[(2,6-difluoropyridin-4-yl)amino]-11-oxo-10,11-dihydro-5H-dibenzo[b,e][1,4]diazepin-8-yl }-2-methylN1,3-thiazol-2-ylpropanamide

The desired product was prepared by substituting Example 205B and 2-aminothiazole for dimethylaminoacetic acid and Example 120, respectively, in Example 122. MS (ESI) m/e 507 (M+H)⁺; ¹H NMR (300 MHz, DMSO-d₆) δ 9.67 (d, J= 15.93 Hz, 2H), 7.95 (s, 1H), 7.69 (d, J= 8.48 Hz, 1H), 7.50 (d, J= 4.75 Hz, 1H), 7.23 (d, J= 5.09 Hz, 1H), 6.89 (m, 5H), 6.68 (dd, J= 8.65, 2.2 Hz, 1H), 6.57 (s, 2H), 1.51 (m, 6H).

### Example 529

### 3-[(2,6-difluoropyridin-4-yl)amino]-8-{2-[(3R)-3-hydroxypiperidin-1-yl]-1,1-dimethyl-2-oxoethyl}-5,10-dihydro-11H-dibenzo[b,e][1,4]diazepin-11-one

The desired product was prepared by substituting Example 205B and (3R)-piperidin-3-ol hydrochloride for dimethylaminoacetic acid and Example 120, respectively, in Example 122. MS (ESI) m/e 508 (M+H)⁺; ¹H NMR (300 MHz, DMSO-d₆) & 9.67 (d, J= 8.81 Hz, 2H), 7.94 (s, 1H), 7.69 (d, J= 8.81 Hz, 2H), 6.91 (m, 2H), 6.78 (d, J= 8.14 Hz, 2H), 6.67 (dd, J= 8.65, 2.20 Hz, 1H), 6.58 (s, 2H), 2.73 (m, 2H), 2.27 (s, 2H), 1.75 (s, 2H), 1.35 (s, 6H), 1.13 (m, 2H).

### Example 530

### 3-[(2,6-difluoropyridin-4-yl)amino]-8-[2-(4-hydroxypiperidin-1-yl)-1,1-dimethyl-2-oxoethyl]-5,10-dihydro-11H-dibenzo[b,e][1,4]diazepin-11-one

The desired product was prepared by substituting Example 205B and 4-hydroxy-piperidine for dimethylaminoacetic acid and Example 120, respectively, in Example 122. MS (ESI) m/e 508 (M+H)⁺; ¹H NMR (300 MHz, DMSO-d₆) δ 9.66 (d, J= 6.44 Hz, 2H), 7.94 (s, 1H), 7.7 (d, J= 8.81 Hz, 2H), 6.9 (m, 2H), 6.79 (m, 2H), 6.67 (dd, J= 8.48, 2.03 Hz, 1H), 6.58 (s, 2H), 3.16 (s, 4H), 3.0-2.0 (m, 4H), 1.35 (s, 6H).

### Example 531

### 3-[(2,6-difluoropyridin-4-yl)amino]-8-{2-[(2S)-2-(hydroxymethyl)pyrrolidin-1-yl]-1,1-dimethyl-2-oxoethyl}-5,10-dihydro-11H-dibenzo[b,e][1,4]diazepin-11-one

The desired product was prepared by substituting Example 205B and (2S)-pyrrolidin-2-ylmethanol for dimethylaminoacetic acid and Example 120, respectively, in Example 122. MS (ESI) m/e 508 (M+H)⁺; ¹H NMR (300 MHz, DMSO-d₆) δ 9.64 (m, 2H), 7.96 (m, 1H), 7.69 (m, 1H), 6.93 (m, 3H), 6.79 (m, 2H), 6.67 (m, 1H), 6.57 (d, J= 2.37 Hz, 2H), 3.96 (s, 2H), 3.55 (s, 2H), 2.89 (s, 2H), 2.71 (d, J= 9.83 Hz, 2H), 1.35 (s, 6H).

### Example 532

### 3-[(2,6-difluoropyridin-4-yl)amino]-8-(1,1-dimethyl-2-oxo-2-pyrrolidin-1-ylethyl)-5,10-dihydro-11H-dibenzo[b,e][1,4]diazepin-11-one

The desired product was prepared by substituting Example 205B and pyrrolidine for dimethylaminoacetic acid and Example 120, respectively, in Example 122. MS (ESI) m/e 478 (M+H)⁺; ¹H NMR (300 MHz, DMSO-d₆) δ 9.65 (d, J= 15.26 Hz, 2H), 7.95 (s, 1H), 7.69 (d, J= 8.48 Hz, 1H), 6.91 (m, 2H), 6.83 (d, J= 2.03 Hz, 1H), 6.78 (dd, J= 8.14, 2.37 Hz, 1H), 6.67 (dd, J= 8.81, 2.03 Hz, 1H), 6.58 (s, 2H), 2.75 (d, J= 11.19 Hz, 2H), 1.56 (s, 6H).

### Example 533

### 2-{3-[(2,6-difluoropyridin-4-yl)amino]-11-oxo-10,11-dihydro-5H-dibenzo[b,e][1,4]diazepin-8-yl}-2-methylNpyridin-2-ylpropanamide

The desired product was prepared by substituting Example 205B and 2-aminopyridine for dimethylaminoacetic acid and Example 120, respectively, in Example 122. MS (ESI) m/e 501 (M+H)⁺.

### Example 534

### 2-{3-[(2,6-difluoropyridin-4-yl)amino]-11-oxo-10,11-dihydro-5H-dibenzo[b,e][1,4]diazepin-8-yl}-2-methylNpyridin-3-ylpropanamide

The desired product was prepared by substituting Example 205B and 3-aminopyridine for dimethylaminoacetic acid and Example 120, respectively, in Example 122. MS (ESI) m/e 501 (M+H)⁺; ¹H NMR (499 MHz, DMSO-d₆) δ 9.66 (d, J= 15.28 Hz, 2H), 9.55 (s, 1H), 8.97 (s, 1H), 8.39 (d, J= 3.12 Hz, 1H), 8.25 (d, J= 8.73 Hz, 1H), 7.96 (s, 1H), 7.69 (d, J= 8.42 Hz, 1H), 7.59 (dd, J= 8.42, 4.99 Hz, 1H), 7.02 (s, 1H), 6.95 (s, 2H), 6.9 (d, J= 2.18 Hz, 1H), 6.68 (dd, J= 8.58, 2.03, 1H), 6.57 (s, 2H), 1.52 (s, 6H).

### Example 535

### 2-{3-[(2,6-difluoropyridin-4-yl)amino]-11-oxo-10,11-dihydro-5H-dibenzo[b,e][1,4]diazoin-8-yl}N(4-fluorophenyl)-2-methylpropanamide

The desired product was prepared by substituting Example 205B and 4-fluoroaniline for dimethylaminoacetic acid and Example 120, respectively, in Example 122. MS (ESI) m/e 518 (M+H)⁺; ¹H NMR (300 MHz, DMSO-d₆) δ 9.67 (s, 2H), 9.06 (s, 1H), 7.94 (s, 1H), 7.68 (d, J= 8.48 Hz, 1H), 7.59 (m, 2H), 7.08 (m, 3H), 6.91 (m, 3H), 6.67 (dd, J= 8.83, 2.03 Hz, 1H), 6.57 (s, 2H), 1.49 (s, 6H).

### Example 536

### 3-[(2,6-difluoropyridin-4-yl)amino]-8-{2-[(2R)-2-(hydroxymethyl)pyrrolidin-1-yl]-1,1-dimethyl-2-oxoethyl}-5,10-dihydro-11H-dibenzo[b,e][1,4]diazepin-11-one

The desired product was prepared by substituting Example 205B and (2R)-pyrrolidin-2-ylmethanol for dimethylaminoacetic acid and Example 120, respectively, in Example 122. MS (ESI) m/e 508 (M+H)⁺; ¹H NMR (300 MHz, DMSO-d₆) & 9.66 (d, J= 14.58 Hz, 2H), 7.95 (s, 1H), 7.7 (d, J= 8.48 Hz, 1H), 6.92 (m, 2H), 6.8 (m. 2H), 6.68 (d, J= 8.82 Hz, 1H), 6.58 (s, 2H), 4.03 (s, 1H), 3.56 (d, J= 6.78 Hz, 1H), 3.45-3.0 (m, 2H), 2.73 (s, 2H), 1.68 (d, J= 6.44 Hz, 3H), 1.35 (s, 6H).

### Example 537

### 8-methoxy-3-(3-methoxy-4-nitrophenyl)-7-(3-morpholin-4-yl-3-oxopropyl)-5,10-dihydro-11H-dibenzo[b,e][1,4]diazepin-11-one

### Example 537A

### 3-[8-methoxy-3-(3-methoxy-4-nitrophenyl)-11-oxo-10,11-dihydro-5H-dibenzo[b,e][1,4]diazepin-7-yl]propanoic acid

The desired product was prepared by substituting Example 245 for Example 12 in Example 13. MS (ESI) m/e 464 (M+H)⁺.

### Example 537B

### 8-methoxy-3-(3-methoxy-4-nitrophenyl)-7-(3-morpholin-4-yl-3-oxopropyl)-5,10-dihydro-11H-dibenzo[b,e][1,4]diazepin-11-one

The desired product was prepared by substituting Example 537A and morpholine for dimethylaminoacetic acid and Example 120, respectively, in Example 122. MS (ESI) m/e 533 (M+H)⁺; ¹H NMR (300 MHz, DMSO-d₆) δ 9.76 (s, 1H), 8.01 (d, J= 8.48 Hz, 1H), 7.76 (m, 2H), 7.51 (d, J= 1.7 Hz, 1H), 7.32 (m, 2H), 7.27 (dd, J= 8.48, 1.7 Hz, 1H), 6.83 (s, 1H), 6.63 (s, 1H), 4.03 (s, 3H), 3.7 (s, 3H), 3.4-3.2 (m, 10H), 2.65 (d, J= 7.8 Hz, 2H).

### Example 538

### 3-[3-(3-methoxy-4-nitrophenyl)-11-oxo-10,11-dihydro-5H-dibenzo[b,e][1,4]diazepin-8-yl]Npyridin-3-ylpropanamide

The desired product was prepared by substituting Example 230 and pyridin-3-ylamine dimethylaminoacetic acid and Example 120, respectively, in Example 122. MS (ESI) m/e 510 (M+H)⁺; ¹H NMR (300 MHz, DMSO-d₆) δ 10.33 (s, 1H), 9.87 (s, 1H), 8.86 (d, J= 2.03 Hz, 1H), 8.34 (m, 1H), 8.13 (m, 1H), 8.01 (d, J= 8.48, 1H), 7.95 (s, 1H), 7.79 (d, J= 8.14 Hz, 1H), 7.52 (m, 2H), 7.33 (m, 2H), 7.28 (d, J= 1.7 Hz, 1H), 6.93 (m, 1H), 6.85 (d, J= 5.76 Hz, 2H), 4.03 (s, 3H), 3.8-3.4 (br, 1H), 2.81 (t, J= 7.46 Hz, 2H), 2.62 (t, J= 7.46 Hz, 2H).

### Example 539

### 3-[3-(3-methoxy-4-nitrophenyl)-11-oxo-10,11-dihydro-5H-dibenzo[b,e][1,4]diazepin-8-yl]Npyridin-4-ylpropanamide

The desired product was prepared by substituting Example 230 and pyridin-4-ylamine for dimethylaminoacetic acid and Example 120, respectively, in Example 122. MS (ESI) m/e 510 (M+H)⁺; ¹H NMR (300 MHz, DMSO-d₆) δ 11.06 (s, 1H), 9.89 (s, 1H), 8.61 (d, J= 6.78 Hz, 1H), 8.03-7.9 (m, 5H), 7.79 (d, J= 8.14 Hz, 1H), 7.52 (d, J= 1.36 Hz, 1H), 7.33 (m, 2H), 7.29 (dd, J= 8.14, 1.7 Hz, 1H), 6.94 (m, 1H), 6.85 (dd, J= 4.07, 2.37 Hz, 2H), 3.8-3.2 (m, 1H), 2.82 (t, J= 6.95 Hz, 2H), 2.71 (m, 2H).

### Example 540

### 8-[3-(3-hydroxypiperidin-1-yl)-3-oxopropyl]-3-(3-methoxy-4-nitrophenyl)-5,10-dihydro-11H-dibenzo[b,e][1,4]diazepin-11-one

The desired product was prepared by substituting Example 230 and 3-hydroxypiperidine for dimethylaminoacetic acid and Example 120, respectively, in Example 122. MS (ESI) m/e 517 (M+H)⁺; ¹H NMR (300 MHz, DMSO-d₆) δ 9.82 (s, 1H), 8.01 (d, J= 8.48 Hz, 1H), 7.93 (s, 1H), 7.79 (d, J= 8.14 Hz, 1H), 7.52 (d, J= 1.7 Hz, 1H), 7.33(m, 2H), 7.29 (dd, J= 8.14, 1.7 Hz, 1H), 6.92 (m, 1H), 6.85 (d, J= 2.37 Hz, 2H), 4.82 (m, 1H), 3.55 (m, 2H), 2.67 (t, J= 7.12 Hz, 2H), 1.63 (m, 2H), 1.24 (m, 2H).

### Example 541

### N-(4-fluorophenyl)-3-[3-(3-methoxy-4-nitrophenyl)-11-oxo-10,11-dihydro-5H-dibenzo[b,e][1,4]diazepin-8-yl]propanamide

The desired product was prepared by substituting Example 230 and 4-fluoroaniline dimethylaminoacetic acid and Example 120, respectively, in Example 122. MS (ESI) m/e 527 (M+H)⁺; ¹H NMR (400 MHz, DMSO-d₆) δ 9.84 (d, J= 21.79 Hz, 2H), 7.96 (d, J= 8.29 Hz, 1H), 7.88 (s, 1H), 7.74 (d, J= 7.98 Hz, 1H), 7.52 (dd, J= 8.9, 4.91 Hz, 2H), 7.47 (d, J= 1.23 Hz, 1H), 7.28 (m, 2H), 7.23 (m, 1H), 7.06 (t, J= 8.9 Hz, 2H), 6.88 (m, 1H), 6.80 (d, J= 8.29 Hz, 2H), 3.98 (s, 3H), 2.73 (t, J= 7.67 Hz, 2H), 2.48 (m, 2H).

### Example 542

### 3-[3-(3-methoxy-4-nitrophenyl)-11-oxo-10,11-dihydro-5H-dibenzo[b,e][1,4]diazepin-8-yl]N[4-(trifluoromethyl)phenyl]propanamide

The desired product was prepared by substituting Example 230 and 4-(trifluoromethyl) aniline for dimethylaminoacetic acid and Example 120, respectively, in Example 122. MS (ESI) m/e 577 (M+H)⁺; ¹H NMR (400 MHz, DMSO-d₆) δ 10.23 (s, 1H), 9.85 (s, 1H), 7.99 (d, J= 8.59 Hz, 1H), 7.92 (s, 1H), 7.77 (t, J= 7.67 Hz, 3H), 7.64 (s, 2H), 7.5 (s, 1H), 7.29 (m, 3H), 6.92 (d, J= 7.36 Hz, 1H), 6.85 (s, 1H), 4.02 (s, 3H), 2.79 (s, 2H), 2.59 (s, 2H).

### Example 543

### 3-[3-(3-methoxy-4-nitrophenyl)-11-oxo-10,11-dihydro-5H-dibenzo[b,e][1,4]diazepin-8-yl]Nmeth ylpropanamide

The desired product was prepared by substituting Example 230 and methylamine hydrochloride for dimethylaminoacetic acid and Example 120, respectively, in Example 122. MS (ESI) m/e 447 (M+H)⁺; ¹H NMR (300 MHz, DMSO-d₆) δ 9.86 (s, 1H), 8.01 (d, J= 8.48 Hz, 1H), 7.93 (s, 1H), 7.79 (d, J= 8.14 Hz, 1H), 7.72 (d, J= 4.41 Hz, 1H), 7.52 (d, J= 1.70 Hz, 1H), 7.34 (m, 2H), 7.29 (dd, J= 8.14, 1.70 Hz, 1H), 6.92 (m, 1H), 6.79 (d, J= 7.12 Hz, 2H), 4.03 (s, 3H), 2.67 (t, J= 7.8 Hz, 2H), 2.54 (d, J= 4.41 Hz, 3H), 2.28 (t, J= 7.8 Hz, 2H).

### Example 544

### 3-[8-methoxy-3-(3-methoxy-4-nitrophenyl)-11-oxo-10,11-dihydro-5H-dibenzo[b,e][1,4]diazepin-7-yl]-N,N-dimethylpropanamide

The desired product was prepared by substituting Example 537A and dimethylamine hydrochloride for dimethylaminoacetic acid and Example 120, respectively, in Example 122. MS (ESI) m/e 491 (M+H)⁺; ¹H NMR (300 MHz, DMSO-d₆) δ 9.76 (s, 1H), 8.01 (d, J= 8.48 Hz, 1H), 7.79 (s, 1H), 7.76 (d, J= 4.07 Hz, 1H), 7.52 (d, J= 1.36 Hz, 1H), 7.34 (m, 2H), 7.27 (dd, J= 8.14, 1.7 Hz, 1H), 6.83 (s, 1H), 6.63 (s, 1H), 4.03 (s, 3H), 3.7 (s, 3), 2.91 (s, 3H), 2.8 (s, 3H), 2.64 (m, 2H), 2.44 (d, J= 6.78 Hz, 2H).

### Example 545

### 8-{2-[(6-chloropyridin-3-yl)oxy]ethyl}-3-(3-methoxy-4-nitrophenyl)-5,10-dihydro-11H-dibenzo[b,e][1,4]diazepin-11-one

A mixture of Example 239 (41 mg, 0.1 mmol), 6-chloro-pyridin-3-ol (19 mg, 0.15 mmol), PPh₃ (39 mg, 0.15 mmol), and di-tert-butyl azodicarboxylate (35 mg, 0.15 mmol) in THF (2 ml) was stirred at room temperature for 16 hours. The reaction mixture was diluted with MeOH/DMSO (1:1), and purified by preparative HPLC to provide the desired product. MS (ESI) m/e 517 (M+H)⁺; ¹H NMR (499 MHz, DMSO-d₆) δ 9.86 (s, 1H), 8.11 (d, J= 3.12 Hz, 1H), 8.01 (d, J= 8.42 Hz, 1H), 7.96 (s, 1H), 7.80 (d, J= 8.11 Hz, 1H), 7.52 (s, 1H), 7.47 (m, 1H), 7.40 (d, J= 8.73 Hz, 1H), 7.34 (m, 2H), 7.30 (dd, J= 8.11, 1.56 Hz, 2H), 6.95 (m, 2H), 4.21 (t, J= 6.55 Hz, 2H), 4.03 (s, 3H), 2.92 (t, J= 6.55 Hz, 2H).

### Example 546

### 8-{2-[(2-chloropyridin-3-yl)oxy]ethyl}-3-(3-methoxy-4-nitrophenyl)-5,10-dihydro-11H-dibenzo[b,e][1,4]diazepin-11-one

The desired product was prepared by substituting 2-chloro-3-hydroxypyridine for 6-chloro-pyridin-3-ol in Example 545. MS (ESI) m/e 517 (M+H)⁺; ¹H NMR (300 MHz, DMSO-d₆) δ 9.88(s, 1H), 8.01 (d, J= 8.48 Hz, 1H), 7.95 (m, 2H), 7.79 (d, J= 8.14 Hz, 1H), 7.57 (dd, J= 8.31, 1.53 Hz, 1H), 7.52 (d, J= 1.70 Hz, 1H), 7.33 (m, 4H), 6.96 (s, 3H), 4.23 (t, J= 6.61 Hz, 2H), 4.03 (s, 3H), 2.96 (t, J= 6.61 Hz, 2H).

### Example 547

### 3-(3-methoxy-4-nitrophenyl)-8-{2-[(6-methylpyridin-3-yl)oxy]ethyl}-5,10-dihydro-11H-dibenzo[b,e][1,4]diazepin-11-one

The desired product was prepared by substituting 6-methyl-pyridin-3-ol for 6-chloro-pyridin-3-ol in Example 545. MS (ESI) m/e 497 (M+H)⁺; ¹H NMR (300 MHz, DMSO-d₆) δ 9.89 (s, 1H), 8.36 (d, J= 3.05 Hz, 1H), 8.03 (s, 1H), 7.99 (d, J= 2.71 Hz, 1H), 7.78 (m, 2H), 7.53 (m, 2H), 7.32 (m, 3H), 6.95 (m, 3H), 4.25 (t, J= 6.61 Hz, 2H), 3.9-3.25 (m, 3H), 2.94 (t, J= 6.61 Hz, 2H).

### Example 548

### 3-(3-methoxy-4-nitrophenyl)-8-{2-[(2-methylpyridin-3-yl)oxy]ethyl}-5,10-dihydro-11H-dibenzo[b,e][1,4]diazepin-11-one

The desired product was prepared by substituting 2-methyl-pyridin-3-ol for 6-chloro-pyridin-3-ol in Example 545. MS (ESI) m/e 497 (M+H)⁺; ¹H NMR (300 MHz, DMSO-d₆) & 9.91 (s, 1H), 8.23 (d, J= 5.09, 1H), 8.01 (m, 2H), 7.89 (d, J= 9.16 Hz, 1H), 7.79 (d, J= 8.14 Hz, 1H), 7.63 (dd, J= 7.97, 5.26 Hz, 1H), 7.52 (d, J= 1.36 Hz, 1H), 7.32 (m, 3H), 6.96 (m, 3H), 4.30 (t, J= 6.27 Hz, 2H), 4.03 (s, 3H), 2.97 (t, J= 6.27 Hz, 2H), 2.47 (s, 3H).

### Example 549

### 3-(3-methoxy-4-nitrophenyl)-8-[2-(pyridin-3-yloxy)ethyl]-5,10-dihydro-11H-dibenzo[b,e][1,4]diazepin-11-one

The desired product was prepared by substituting 3-hydroxy-pyridine for 6-chloro-pyridin-3-ol in Example 545. MS (ESI) m/e 483 (M+H)⁺; ¹H NMR (300 MHz, DMSO-d₆) δ 9.89 (s, 1H), 8.35 (d, J= 2.71 Hz, 1H), 8.22 (d, J= 4.75 Hz, 1H), 8.03 (s, 1H), 7.99 (d, J= 6.10 Hz, 1H), 7.80 (d, J= 8.14 Hz, 1H), 7.52 (m, 2H), 7.42 (dd, J= 8.48, 4.75 Hz, 1H), 7.33 (m, 2H), 7.28 (d, J= 1.7 Hz, 1H), 6.95 (m, 3H), 4.23 (t, J= 6.61 Hz, 2H), 4.03 (s, 3H), 2.94 (t, J= 6.61 Hz, 2H).

### Example 550

### 8-{2-[(2,6-dimethylpyridin-3-yl)oxy]ethyl}-3-(3-methoxy-4-nitrophenyl)-5,10-dihydro-11H-dibenzo[b,e][1,4]diazepin-11-one

The desired product was prepared by substituting 2,6-dimethyl-pyridin-3-ol for 6-chloro-pyridin-3-ol in Example 545. MS (ESI) m/e 511 (M+H)⁺; ¹H NMR (300 MHz, DMSO-d₆) δ 9.89 (s, 1H), 8.03 (s, 1H), 7.99 (d, J= 5.42 Hz, 1H), 7.80 (d, J= 8.14 Hz, 2H), 7.52 (d, J= 1.7 Hz, 2H), 7.33 (m, 3H), 7.28 (d, J= 2.03 Hz, 1H), 6.95 (m, 2H), 6.91 (s, 1H), 4.26 (m, 2H), 4.03 (s, 3H), 2.41 (s, 3H), 1.39 (s, 3H).

### Example 551

### 8-[2-({2-[(dimethylamino)methyl]pyridin-3-yl}oxy)ethyl]-3-(3 -methoxy-4-nitrophenyl)-5,10-dihydro-11H-dibenzo[b,e][1,4]diazepin-11-one

The desired product was prepared by substituting 2-[(dimethylamino)methyl]pyridin-3-ol for 6-chloro-pyridin-3-ol in Example 545. MS (ESI) m/e 540 (M+H)⁺; ¹H NMR (300 MHz, DMSO-d₆) δ 9.89 (s, 1H), 8.23 (d, J= 3.73 Hz., 1H), 8.02 (m, 2H), 7.80 (d, J= 8.14 Hz, 1H), 7.61 (d, J= 7.46 Hz, 1H), 7.52 (d, J= 1.70 Hz, 1H), 7.47 (dd, J= 8.31, 4.58 Hz, 1H), 7.32 (m, 3H), 6.96 (m, 3H), 4.35 (d, J= 3.73 Hz, 2H), 4.25 (t, J= 6.44 Hz, 2H), 4.03 (s, 3H), 2.97 (t, J= 6.44 Hz, 2H), 2.5 (m, 6H).

### Example 552

### 8-[2-(isoquinolin-7-yloxy)ethyl]-3-(3-methoxy-4-nitrophenyl)-5,10-dihydro-11H-dibenzo[b,e][1,4]diazepin-11-one

The desired product was prepared by substituting isoquinolin-7-ol for 6-chloro-pyridin-3-ol in Example 545. MS (ESI) m/e 533 (M+H)⁺; ¹H NMR (500 MHz, DMSO-d₆) δ 9.87 (s, 1H), 9.17 (s, 1H), 8.35 (d, J= 5.6 Hz, 1H), 8.01 (d, J= 8.42 Hz, 1H), 7.97 (s, 1H), 7.87 (d, J= 8.73 Hz, 1H), 7.80 (d, J= 8.42 Hz, 1H), 7.73 (d, J= 5.62 Hz, 1H), 7.52 (s, 2H), 7.42 (dd, J= 8.74, 2.5 Hz, 1H), 7.34 (m, 2H), 7.3 (dd, J= 8.42, 1.56 Hz, 1H), 6.99 (m, 3H), 4.29 (t, J= 6.71 Hz, 2H), 4.03 (s, 3H), 3.0 (t, J= 6.71 Hz, 2H).

### Example 553

### 7-methoxy-3-(3-methoxy-4-nitrophenyl)-N,N-dimethyl-11-oxo-10,11-dihydro-5H-dibenzo[b,e][1,4]diazepine-8-carboxamide

### Example 553A

### 7-methoxy-3-(3-methoxy-4-nitrophenyl)-11-oxo-10,11-dihydro-5H-dibenzo[b,e][1,4]diazepine-8-carboxylic acid

The desired product was prepared by substituting Example 523D for Example 12 in Example 13. MS (ESI) m/e 436 (M+H)⁺.

### Example 553B

### 7-methoxy-3-(3-methoxy-4-nitrophenyl)-N,N-dimethyl-11-oxo-10,11-dihydro-5H-dibenzo[b,e] [1,4]diazepine-8-carboxamide

The desired product was prepared by substituting Example 553A and N, N-dimethylamine hydrochloride dimethylaminoacetic acid and Example 120, respectively, in Example 122. MS (ESI) m/e 463 (M+H)⁺; ¹H NMR (300 MHz, DMSO-d₆) δ 9.82 (s, 1H), 8.16 (s, 1H), 8.02 (d, J= 8.14 Hz, 1H), 7.8 (d, J= 8.14 Hz, 1H), 7.55 (d, J= 1.70 Hz, 1H), 7.35 (m, 3H), 6.77 (d, J= 7.12 Hz, 2H), 4.04 (s, 3H), 3.74 (s, 3H), 2.93 (s, 3H), 2.76 (s, 3H).

### Example 554

### 7-{2-[(2-chloropyridin-3-yl)oxy]ethyl}-8-methoxy-3-(3-methoxy-4-nitrophenyl)-5,10-dihydro-11H-dibenzo[b,e][1,4]diazepin-11-one

The desired product was prepared by substituting Example 247 and 2-chloro-3-hydroxypyridine for Example 239 and 6-chloro-pyridin-3-ol respectively, in Example 545. MS (ESI) m/e 547 (M+H)⁺; ¹H NMR (400 MHz, DMSO-d₆) δ 9.79 (s, 1H), 8.02 (d, J= 8.59 Hz, 1H), 7.93 (dd, J= 4.76, 1.38 Hz, 1H), 7.77 (m, 2H), 7.57 (dd, J= 8.13, 1.38 Hz, 1H), 7.52 (d, J= 1.53 Hz, 1H), 7.35 (m, 3H), 7.29 (dd, J= 8.29, 1.84 Hz, 1H), 6.95 (s, 1H), 6.67 (s, 1H), 4.2 (t, J= 6.75, 2H), 4.04 (s, 3H), 3.72 (s, 3H), 2.94 (t, J= 6.6 Hz, 2H).

### Example 555

### 8-[2-(isoquinolin-5-yloxy)ethyl]-3-(3-methoxy-4-nitrophenyl)-5,10-dihydro-11H-dibenzo[b,e][1,4]diazepin-11-one

The desired product was prepared by substituting isoquinolin-5-ol for 6-chloro-pyridin-3-ol in Example 545. MS (ESI) m/e 533 (M+H)⁺; ¹H NMR (500 MHz, DMSO-d₆) δ 9.82 (s, 1H), 9.39 (s, 1H), 8.46 (d, J= 6.24 Hz, 1H), 8.06 (d, J= 5.93 Hz, 1H), 7.93 (d, J= 8.42 Hz, 1H), 7.89 (s, 1H), 7.72 (m, 2H), 7.63 (t, J= 7.96 Hz, 1H), 7.44 (d, J= 1.56 Hz, 1H), 7.32 (d, J= 7.8 Hz, 1H), 7.26 (m, 2H), 7.22 (dd, J= 8.11, 1.56 Hz, 1H), 6.95 (d, J= 8.11 Hz, 1H), 6.91 (d, J= 7.8 Hz, 2H), 4.3 (t, J= 6.4 Hz, 2H), 3.95 (s, 3H), 3.01 (t, J= 6.24 Hz, 2H).

### Example 556

### 3-(3-methoxy-4-nitrophenyl)-8-[2-(quinolin-5-yloxy)ethyl]-5,10-dihydro-11H-dibenzo[b,e][1,4]diazepin-11-one

The desired product was prepared by substituting quinolin-5-ol for 6-chloro-pyridin-3-ol in Example 545. MS (ESI) m/e 533 (M+H)⁺; ¹H NMR (300 MHz, DMSO-d₆) δ 9.86 (s, 1H), 8.01 (d, J= 8.42 Hz, 1H), 7.96 (s, 1H), 7.86 (d, J= 8.73 Hz, 1H), 7.8 (d, J= 8.11 Hz, 1H), 7.62 (m, 2H), 7.56 (m, 1H), 7.52 (d, J= 1.25 Hz, 1H), 7.45 (d, J= 2.5 Hz, 1H), 7.34 (dd, J= 8.74, 1.56 Hz, 2H), 7.29 (dd, J= 8.11, 1.56 Hz, 1H), 7.02 (dd, J= 8.89, 2.34 Hz, 1H), 6.96 (m, 3H), 4.2 (t, J= 6.71 Hz, 2H), 4.03 (s, 3H), 2.94 (t, J= 6.71 Hz, 2H).

### Example 557

### 3-(3-methoxy-4-nitrophenyl)-8-[2-(4-methoxyphenoxy)ethyl]-5,10-dihydro-11H-dibenzo[b,e][1,4]diazepin-11-one

The desired product was prepared by substituting 4-methoxyphenol for 6-chloro-pyridin-3-ol in Example 545. MS (ESI) m/e 512 (M+H)⁺; ¹H NMR (400 MHz, DMSO-d₆) δ 9.85 (s, 1H), 8.01 (d, J= 8.29 Hz, 1H), 7.95 (s,1H), 7.80 (d, J= 8.29 Hz, 1H), 7.52 (d, J= 1.23 Hz, 1H), 7.34 (m, 2H), 7.29 (m, 1H), 6.93 (m, 4H), 6.84 (d, J= 3.38 Hz, 3H), 4.05 (m, 2H), 4.03 (s, 3H), 3.68 (s, 3H), 2.87 (t, J= 6.60, 2H).

### Example 558

### 3-(3-methoxy-4-nitrophenyl)-8-[2-(3-methoxyphenoxy)ethyl]-5,10-dihydro-11H-dibenzo[b,e][1,4]diazepin-11-one

The desired product was prepared by substituting 3-methoxyphenol for 6-chloro-pyridin-3-ol in Example 545. MS (ESI) m/e 512 (M+H)⁺; ¹H NMR (400 MHz, DMSO-d₆) δ 9.86 (s, 1H), 8.01 (d, J= 8.29 Hz, 1H), 7.96 (s, 1H), 7.8 (d, J= 8.29 Hz, 1H), 7.52 (d, J= 1.23 Hz, 1H), 7.36 (m, 2H), 7.29 (m, 1H), 7.15 (t, J= 8.13 Hz, 1H), 6.94 (m, 3H), 6.49 (m, 3H), 4.10 (t, J= 6.75 Hz, 2H), 4.03 (s, 3H), 2.89 (t, J= 6.60 Hz, 2H).

### Example 559

### 3-{2-[3-(3-methoxy-4-nitrophenyl)-11-oxo-10,11-dihydro-5H-dibenzo[b,e][1,4]diazepin-8-yl]ethoxy}pyridine-2-carboxamide

The desired product was prepared by substituting 3-hydroxypyridine-2-carboxamide for 6-chloro-pyridin-3-ol in Example 545. MS (ESI) m/e 526 (M+H)⁺; ¹H NMR (500 MHz, DMSO-d₆) δ 9.85 (s, 1H), 9.25 (s, 1H), 8.34 (s, 1H), 8.09 (s, 1H), 8.01 (d, J= 8.42 Hz, 2H), 7.81 (d, J= 8.11 Hz, 2H), 7.71 (s, 1H), 7.52 (s, 1H), 7.32 (dd, J= 19.03, 7.49 Hz, 3H), 6.96 (d, J= 7.8 Hz, 1H), 6.82 (m, 2H), 7.75 (t, J= 7.49 Hz, 2H), 4.03 (s, 3H), 3.09 (t, J= 7.33 Hz, 2H).

### Example 560

### 3-(3-methoxy-4-nitrophenyl)-8-[3-(4-morpholin-4-ylphenoxy)propyl]-5,10-dihydro-11H-dibenzo[b,e][1,4]diazepin-11-one

The desired product was prepared by substituting Example 240 and 4-morpholin-4-ylphenol (M.C. Harris, et.al., Org. Lett., 2002, 4, 2885) for Example 239 and 6-chloro-pyridin-3-ol respectively, in Example 545. MS (ESI) m/e 581 (M+H)⁺; ¹H NMR (400 MHz, DMSO-d₆) δ 9.82 (s, 1H), 8.01 (d, J= 8.29 Hz, 1H), 7.93 (s, 1H), 7.80 (d, J= 8.28 Hz, 1H), 7.34 (m, 2H), 7.29 (d, J= 8.29, 1H), 6.93 (d, J= 7.67 Hz, 3H), 6.83 (dd, J= 8.13, 6.60 Hz, 4H), 4.0 (s, 3H), 3.88 (t, J= 6.29 Hz, 2H), 3.74 (m, 4H), 3.02 (s, 4H), 2.60 (t, J= 7.67 Hz, 2H), 1.92 (m, 2H).

### Example 561

### 3-(3-methoxy-4-nitrophenyl)-8-[3-(pyridin-3-yloxy)propyl]-5,10-dihydro-11H-dibenzo[b,e][1,4]diazepin-11-one

The desired product was prepared by substituting Example 240 and pyridin-3-ol for Example 239 and 6-chloro-pyridin-3-ol, respectively, in Example 545. MS (ESI) m/e 497 (M+H)⁺; ¹H NMR (400 MHz, DMSO-d₆) δ 9.83 (s, 1H), 8.35 (s, 1H), 8.20 (s, 1H), 8.01 (d, J= 8.29 Hz, 1H), 7.94 (s, 1H), 7.79 (d, J= 8.29 Hz, 1H), 7.52 (d, J= 1.23 HZ, 1H), 7.48 (m, 1H), 7.40 (dd, J= 8.29, 4.60 Hz, 1H), 7.34 (m, 2H), 7.29 (dd, J= 8.13, 1.69 Hz, 1H), 6.94 (d, J= 7.98 Hz, 1H), 6.84 (d, J= 8.59 Hz, 2H), 4.05 (s, 3H), 2.63 (t, J= 7.52, 2H), 1.98 (m, 2H), 1.39 (s, 2H).

### Example 562

### 8-[2-(3-aminophenoxy)ethyl]-3-(3-methoxy-4-nitrophenyl)-5,10-dihydro-11H-dibenzo[b,e][1,4]diazepin-11-one

The desired product was prepared by substituting 3-aminophenol for 6-chloro-pyridin-3-ol in Example 545. MS (ESI) m/e 497 (M+H)⁺; ¹H NMR (400 MHz, DMSO-d₆) δ 9.85 (s, 1H), 8.01 (d, J= 8.59 Hz, 1H), 7.95 (s, 1H), 7.8 (d, J= 8.29 Hz, 1H), 7.52 (d, J= 1.23 Hz, 1H), 7.34 (m, 2H), 7.29 (dd, J= 8.13, 1.69 Hz, 1H), 6.97-6.85 (m, 4H), 6.13 (m, 1H), 6.07 (dd, J= 7.52, 1.69 Hz, 1H), 6.97-6.85 (m, 4H), 4.97 (s, 2H), 4.03 (s, 3H), 4.0 (t, J= 6.75 Hz, 2H), 2.86 (t, J= 6.60 Hz, 2H).

### Example 563

### 3-(3-methoxy-4-nitrophenyl)-8-2-[(2-methyl-1,3-benzothiazol-7-yl)oxy]ethyl-5,10-dihydro-11H-dibenzo[b,e][1,4]diazepin-11-one

The desired product was prepared by substituting 2-methyl-1,3-benzothiazol-7-ol for 6-chloro-pyridin-3-ol in Example 545. MS (ESI) m/e 553 (M+H)⁺; ¹H NMR (499 MHz, DMSO-d₆) δ 9.86 (s, 1H), 8.01 (d, J= 8.42 Hz, 1H), 7.96 (s, 1H), 7.86 (d, J= 8.74 Hz, 1H), 7.8 (d, J= 8.11 Hz, 1H), 7.52 (d, J= 1.56 HZ, 1H), 7.45 (d, J= 2.50 Hz, 1H), 7.34 (m, 2H), 7.29 (dd, J= 8.11, 1.87 Hz, 1H), 7.02 (dd, J= 8.74, 2.5 Hz, 1H), 6.98 (m, 3H), 4.20 (t, J= 6.71 Hz, 2H), 4.03 (s, 3H), 2.94 (t, J= 6.71 Hz, 2H), 2.76 (s, 3H).

### Example 564

### 2-[3-(3-methoxy-4-nitrophenyl)-11-oxo-10,11-dihydro-5H-dibenzo[b,e][1,4]diazepin-8-yl]-2-methylN(pyridin-2-lymethyl)propanamide

The title compound was prepared by substituting Example 266I and pyridin-2-ylmethylamine for dimethylaminoacetic acid and Example 120, respectively, in Example122. MS (ESI) m/e 538 (M+H)⁺; ¹H NMR (300 MHz, DMSO-d₆) δ 9.86 (s, 1H), 8.54 (m, 1H), 8.02 (s, 1H), 8.01 (d, J= 8.5 Hz, 1H), 7.92 (m, 2H), 7.81 (d, J= 8.5 Hz, 1H), 7.52 (d, J= 1.7 Hz, 1H), 7.40 (m, 1H), 7.36, 7.32, 7.30 (all m, total 3H), 7.22 (d, J= 7.8 Hz, 1H), 7.03 (d, J= 1.7 Hz, 1H), 6.97 (m, 2H), 4.36 (d, J= 5.8 Hz, 2H), 4.03 (s, 3H), 1.45 (s, 6H).

### Example 565

### 8-(2-hydroxy-2-methylpropyl)-3-(3-methoxy-4-nitrophenyl)-5,10-dihydro-11H-dibenzo[b,e][1,4]diazepin-11-one

The title compound was prepared by substituting Example 208A and Example 266G for Example 59B and Example 56A, respectively, in Example 59C. MS (DCI) m/e 434 (M+H)⁺; ¹H NMR (500 MHz, DMSO-d₆) δ 9.79 (s, 1H), 7.98 (d, J= 8.42 Hz, 1H), 7.87 (s, 1H), 7.76 (d, J= 8.11 Hz, 1H), 7.49 (s, 1H), 7.31-7.32 (m, 2H), 7.26 (dd, J= 8.26, 1.40 Hz, 1H), 6.88 (d, J= 8.11 Hz, 1H), 6.77-6.81 (m, 2H), 4.21 (s, 1H), 4.00 (s, 3H), 2.49 (s, 2H), 1.02 (s, 6H).

### Example 566

### 3-(3-methoxy-4-nitrophenyl)-8-[(4-methylpiperazin-1-yl)methyl]-5,10-dihydro-11H-dibenzo[b,e][1,4]diazepin-11-one

### Example 566A

### N-(4-formyl-2-nitrophenyl)acetamide

To fuming HNO₃ (30mL) at 0°C was added N-(4-formylphenyl)acetamide (10.3g, 63.1mmoles) portionwise. After two hours, the reaction mixture was poured into 4°C water (1L). The resulting solid was filtered, washed with water (0.5Lx3), and dried in vacuo to give the desired product (10.57g, 80%). MS (APCI)⁺ m/e 209 (M+H)⁺; ¹H NMR (300 MHz, DMSO-d₆) δ 10.61 (s, 1H), 10.00 (s, 1H), 8.46 (d, J= 1.70 Hz, 1H), 8.17 (dd, J= 8.31, 1.86 Hz, 1H), 7.91 (d, J= 8.48 Hz, 1H), 2.13 (s, 3H).

### Example 566B

### N-{4-[(4-methylpiperazin-1-yl)methyl]-2-nitrophenyl acetamide

Example 566A (2.02g, 9.71mmoles), 1-methyl-piperazine (1.1mL, 9.71mmoles), NaBH(OAc)₃ (3.09g, 14.57mmoles) and 1,2-dichloroethane were mixed, and stirred under N₂ at room temperature for 3 hours. After the reaction, the solvent was removed. Silica gel column was used to purify and give the desired product (2.74g, 96.5%). MS (APCI) m/e 293 (M+H)⁺; 1H NMR (300 MHz, DMSO-d₆) δ 10.22 (s, 1H), 7.81 (d, J= 1.70 Hz, 1H), 7.60 (m, 1H), 7.55 (d, J= 8.14 Hz, 1H), 3.50 (s, 2H), 2.32 (m, 8H), 2.14 (s, 3H), 2.05 (s, 3H).

### Example 566C

### 4-[(4-methylpiperazin-1-yl)methyl]-2-nitrophenylamine

Example 566B (2.74g, 9.37mmoles) was added to concentrated hydrochloric acid (40mL). The reaction was heated at 65°C for 15 minutes. The solvents were removed to give a yellow solid. 2N NaOH solution was added until the solution became basic. The water was removed. Silica gel column was used to purify and give the desired product (1.88g, 77%). MS (APCI) m/e 251 (M+H)⁺; ¹H NMR (300 MHz, CD₃OD) δ 7.98 (d, J= 2.03 Hz, 1H), 7.35 (dd, J= 8.82, 2.03 Hz, 1H), 6.93 (d, J= 8.48 Hz, 1H), 3.43 (s, 2H) 2.49 (br. s, 8H), 2.27 (s, 3H).

### Example 566D

### methyl 4-chloro-2-({4-[(4-methylpiperazin-1-yl)methyl]-2-nitrophenyl }amino)benzoate

The title compound was prepared by substituting Example 566C for methyl 3,4-diaminobenzoate in Example 1A. MS (APCI) m/e 419 (M+H)⁺; 1H NMR (300 MHz, DMSO-d₆) δ 10.83 (s, 1H), 8.03 (d, J= 2.03 Hz, 1H), 7.96 (d, J= 8.48 Hz, 1H), 7.67 (d, J= 8.50, 1H), 7.60 (dd, J= 8.65, 1.87 Hz, 1H), 7.48 (d, J= 2.03 Hz, 1H), 7.11 (dd, J= 8.48, 2.03 Hz, 1H), 3.49 (s, 2H), 3.87 (s, 3H), 2.39 (m, 8H), 2.16 (m, 3H).

### Example 566E

### methyl 2-({2-amino-4-[(4-methylpiperazin-1-yl)methyl]phenyl}amino)-4-chlorobenzoate

Example 566D (2.45g, 5.85mmoles), Fe (2.34g, 42mmoles), NH₄Cl (0.22g, 4.2mmoles), ethanol (30mL) and water (10.5mL) were mixed, stirred, and heated to reflux overnight. The reaction mixture was purified by silica gel column to give 1.11 g of the desired product (49%). MS (APCI) m/e 389 (M+H)⁺; 1H NMR (300 MHz, CD₃OD) δ 9.00 (s, 1H), 7.89 (d, J= 8.48 Hz, 1H), 7.01 (d, J= 7.80 Hz, 1H), 6.88 (d, J= 1.70 Hz, 1H), 6.71 (dd, J= 7.80, 2.03 Hz, 1H), 6.64 (dd, J= 8.48, 2.03 Hz, 1H), 6.50 (d, J= 2.03 Hz, 1H), 3.90 (s, 3H), 3.48 (s, 2H), 2.53 (m, 8H), 2.28 (s, 3H).

### Example 566F

### 3-chloro-8-[(4-methylpiperazin-1-yl)methyl]-5,10-dihydro-11H-dibenzo[b,e][1,4]diazepin-11-one

The title compound was prepared by substituting Example 566E for Example 6C in Example 6D. MS (APCI) m/e 357 (M+H)⁺; 1H NMR (300 MHz, DMSO-d₆) δ 9.86 (s, 1H), 8.02 (s, 1H), 7.67 (d, J= 8.48 Hz, 1H), 7.06 (d, J= 2.03 Hz, 1H), 6.90 (m, 4H), 2.30 (m, 8H), 3.29 (s, 2H), 2.13 (s, 3H).

### Example 566G

### 3-(3-methoxy-4-nitrophenyl)-8-[(4-methylpiperazin-1-yl)methyl]-5,10-dihydro-11H-dibenzo[b,e][1,4]diazepin-11-one

The title compound was prepared by substituting Example 566F and Example 266G for Example 59B and Example 56A, respectively, in Example 59C. MS (APCI) m/e 474 (M+H)⁺; 1H NMR (500 MHz, DMSO-d₆) δ 9.94 (s, 1H), 8.10 (s, 1H), 8.02 (d, J= 8.54 Hz, 1H), 7.80 (d, J= 8.24 Hz, 1H), 7.52 (d, J= 1.53 Hz, 1H), 7.37 (d, J= 1.53 Hz, 1H), 7.34 (dd, J= 8.54, 1.53 Hz, 1H), 7.32 (dd, J= 8.09, 1.68 Hz, 1H), 7.01 (s, 1H), 6.95 (s, 2H), 4.04 (s, 3H), 3.78 (m, 4 H,) 3.37 (m, 2H), 2.99 (m, 4H), 2.76 (s, 3H).

### Example 567

### 3-(4-chloro-3-methoxyphenyl)-8-[(4-methylpiperazin-1-yl)methyl]-5,10-dihydro-11H-dibenzo[b,e][1,4]diazepin-11-one

### Example 567A

### 8-[(4-methylpiperazin-1-yl)methyl]-3-(4,4,5,5-tetramethyl-1,3,2-dioxaborolan-2-yl)-5,10-dihydro-11H-dibenzo[b,e][1,4]diazepin-11-one

The title compound was prepared by substituting Example 566F for Example 6D in Example 54A. MS (APCI) m/e 449 (M+H)⁺.

### Example 567B

### 3-(4-chloro-3-methoxyphenyl)-8-[(4-methylpiperazin-1-yl)methyl]-5,10-dihydro-11H-dibenzo[b,e][1,4]diazepin-11-one

The desired product was prepared by substituting Example 567A and 2-chloro-5-iodoanisole for 2-methoxy-4-(4,4,5,5-tetramethyl-1,3,2-dioxaborolan-2-yl)phenol and Example 1B, respectively, in Example 10. MS (APCI) m/e 464 (M+H)⁺; 1H NMR (400 MHz, DMSO-d₆) δ 9.88 (s, 1H), 8.04 (s, 1H), 7.77 (d, J= 8.30 Hz, 1H), 7.54 (d, J= 8.29 Hz, 1H), 7.33 (d, J= 2.15 Hz, 1H), 7.32 (d, J= 1.84 Hz, 1H), 7.25 (dd, J= 8.13, 1.69 Hz, 1H), 7.21 (dd, J= 8.29, 1.84 Hz, 1H), 7.02 (d, J= 8.60Hz, 1H), 6.95 (m, 2H), 3.96 (s, 3H) 3.68 (m, 4H), 3.41 (m, 2H), 3.11 (m, 4H), 2.77 (s, 3H).

### Example 568

### 2-methoxy-4-{8-[(4-methylpiperazin-1-yl)methyl]-11-oxo-10,11-dihydro-5H-dibenzo[b,e][1,4]diazepin-3-yl}benzonitrile

The desired product was prepared by substituting Example 567A and 4-iodo-2-methoxy-benzonitrile for 2-methoxy-4-(4,4,5,5-tetramethyl-1,3,2-dioxaborolan-2-yl)phenol and Example 1B, respectively, in Example 10. MS (APCI) m/e 454 (M+H)⁺; 1H NMR (400 MHz, DMSO-d₆) δ 9.92 (s, 1H), 8.07 (s, 1H), 7.84 (d, J= 7.90 Hz 1H), 7.80 (d, J= 8.29 Hz, 1H), 7.42 (s, 1H), 7.36 (d, J= 1.23 Hz, 1H), 7.33 (dd, J= 7.98, 1.23 Hz, 1H), 7.30 (dd, J= 8.29, 1.53 Hz, 1H), 7.02 (d, J= 8.30 Hz 1H), 6.95 (m, 2H), 4.02 (s, 3H), 3.62 (m, 4H), 3.41 (m, 2H), 3.10 (m, 4H), 2.78 (s, 3H).

### Example 569

### 3-(4-hydroxy-3-methoxyphenyl)-8-(hydroxymethyl)-5,10-dihydro-11H-dibenzo[b,e][1,4]diazepin-11-one

### Example 569A

### (4-amino-3-nitrophenyl)methanol

(4-chloro-3-nitrophenyl)methanol (7.0g, 37.32 mmoles), liquid NH₃ (100 mL) and methanol (125 mL) were mixed in a sealed reactor. The temperature was set to 160°C, and the pressure became about 600psi. After 16 hours, the reaction was cooled down. The high pressure was released. All the solvents were removed. The solid residual was purified by silica gel column to give 4.51 g of the above intermediate (72%). MS (DCI) m/e 169 (M+H)⁺; 1H NMR (300 MHz, CD₃OD) δ 8.02 (d, J= 1.70 Hz, 1H), 7.36 (dd, J= 8.48, 2.03 Hz, 1H), 6.95 (d, J= 8.48 Hz, 1H), 4.48 (s, 2H).

### Example 569B

### methyl 4-chloro-2-{[4-(hydroxymethyl)-2-nitrophenyl]amino}benzoate

The title compound was prepared by substituting Example 569A for methyl 3,4-diaminobenzoate in Example 1A. MS (APCI) m/e 337 (M+H)⁺; ¹H NMR (300 MHz, DMSO-d₆) δ 10.82 (s, 1H), 8.09 (s, 1H), 7.97 (d, J= 8.48 Hz, 1H), 7.69 (m, 1H), 7.64 (d, J= 1.70 Hz, 1H), 7.43 (d, J= 2.03 Hz, 1H), 7.10 (dd, J= 8.65, 1.87 Hz, 1H), 5.42 (t, J= 5.76 Hz, 1H), 4.54 (d, J= 5.76 Hz, 1H), 3.89 (s, 3H).

### Example 569C

### methyl 2-{[2-amino-4-(hydroxymethyl)phenyl]amino}-4-chlorobenzoate

The title compound was prepared by substituting Example 569B for Example 566D in Example 566E. MS (APCI) m/e 389 (M+H)⁺; 1H NMR (300 MHz, CD₃OD) δ 9.00 (s, 1H), 7.89 (d, J= 8.48 Hz, 1H), 7.01 (d, J= 7.80 Hz, 1H), 6.88 (d, J=1.70 Hz, 1H), 6.71 (dd, J= 7.80, 2.03 Hz, 1H), 6.64 (dd, J= 8.48, 2.03 Hz, 1H), 6.50 (d, J= 2.03 Hz, 1H), 3.90 (s, 3H), 3.48 (s, 2H), 2.53 (m, 8H), 2.28 (s, 3H).

### Example 569D

### 2-{[2-amino-4-(hydroxymethyl)phenyl]amino}-4-chlorobenzoic acid

Example 569C (1.44 g, 4.69 mmole) was dissolved in DMF (45 mL). To this solution was added LiOH (1.12 g, 46.95 mmole) in water (5 mL). The mixture was stirred for 3 hours. Then the reaction mixture was poured into water (100 mL). Adjust the pH to 5 with 2N hydrochloric solution. Extract the solution with ethyl acetate (50 mL×4). The organic phases were dried (MgSO₄) and concentrated to give 1.35 g of the desired product (98%). MS (APCI) m/e 293 (M+H)⁺; 1H NMR (300 MHz, CD₃OD) δ 7.90 (d, J= 8.48 Hz, 1H), 7.03 (d, J= 7.80 Hz, 1H), 6.91 (d, J= 1.70 Hz, 1H), 6.74 (dd, J= 7.97, 1.86 Hz, 1 H, 6.62 (dd, J= 8.65, 2.20 Hz, 1H), 6.49 (d, J= 2.03 Hz, 1H), 4.54 (s, 2H).

### Example 569E

### 3-chloro-8-(hydroxymethyl)-5,10-dihydro-11H-dibenzo[b,e][1,4]diazepin-11-one

The title compound was prepared by substituting Example 569D for Example 233F in Example 233G. MS (APCI)⁺ m/e 275 (M+H)⁺; 1H NMR (300 MHz, DMSO-d₆) δ 9.90 (s, 1H), 8.00 (s, 1H), 7.68 (d, J= 8.48 Hz, 1H), 7.06 (d, J= 2.03 Hz, 1 H,) 6.92 (m, 4H), 5.09 (t, J= 5.76 Hz, 1H), 4.35 (d, J= 5.09 Hz, 2H).

### Example 569F

### 3-(4-hydroxy-3-methoxyphenyl)-8-(hydroxymethyl)-5,10-dihydro-11H-dibenzo[b,e][1,4]diazepin-11-one

The title compound was prepared by substituting Example 569E and 2-methoxy-4-(4,4,5,5-tetramethyl-[1,3,2]dioxaborolan-2-yl)-phenol for Example 59B and Example 56A, respectively, in Example 59C. MS (APCI) m/e 363 (M+H)⁺; ¹H NMR (300 MHz, DMSO-d₆) δ 9.76 (s, 1H), 9.25 (s, 1H), 7.83 (s, 1H), 7.71 (d, J= 8.14 Hz, 1H), 7.22 (d, J= 1.70 Hz, 1H), 7.17 (d, J= 2.03 Hz, 1H), 7.14 (dd, J= 8.14, 1.70 Hz, 1H), 7.07 (dd, J= 8.14, 2.03 Hz, 1H), 6.88 (m, 2H), 6.94 (m, 2H), 5.07 (t, J= 5.59 Hz, 1H), 4.35 (d, J= 5.76 Hz, 2H), 3.86 (s, 3H).

### Example 570

### 3-(3-methoxy-4-nitrophenyl)-8-(morpholin-4-ylmethyl)-5,10-dihydro-11H-dibenzo[b,e][1,4]diazepin-11-one

### Example 570A

### methyl 2-{[4-(bromomethyl)-2-nitrophenyl]amino}-4-chlorobenzoate

A mixture of Example 569B (0.336 g, 1.0 mmol) and LiBr (0.1 g) in 3 mL of DMF was treated with PBr₃ (0.1 mL) at 0 °C. The solution was stirred for additional two hours, and partitioned between water and ethyl acetate. The organic layer was washed with brine, dried (MgSO₄), filtered, and concentrated under vacuum to give 0.38 g of the title compound (97%). MS (DCI) m/e 401 (M+H)⁺; ¹H NMR (300 MHz, DMSO-d₆) δ 10.94 (s, 1H), 8.29 (d, J= 2.03 Hz, 1H), 7.98 (d, J= 8.82 Hz, 1 H,) 7.70-7.75 (m, 2H), 7.54 (d, J= 2.03 Hz, 1H), 7.18 (dd, J= 8.48, 2.03 Hz, 1H), 4.80 (s, 2H), 3.89 (s, 3H).

### Example 570B

### methyl 4-chloro-2-{[4-(morpholin-4-ylmethyl)-2-nitrophenyl]amino}benzoate

A mixture of Example 570A (80 mg, 0.2 mmol) and morpholine (35 mg, 0.4 mmol) in 4 mL of toluene was heated under reflux for two hours. After the reaction mixture was cooled to room temperature, it was loaded onto silica gel for purification that yielded 76 mg of the desired product (94%). MS (DCI) m/e 406 (M+H)⁺; ¹H NMR (300 MHz, DMSO-d₆) δ 10.61 (s, 1H), 8.04 (d, J= 1.87 Hz, 1H), 7.95 (d, J= 8.73 Hz, 1 H,) 7.65-7.66 (m, 1H), 7.61 (dd, J= 8.73, 1.87 Hz, 1H), 7.46 (d, J= 1.87 Hz, 1H), 7.10 (dd, J= 8.58, 2.03 Hz, 1H), 3.88 (s, 3H), 3.57-3.59 (m, 4H), 3.39 (s, 2H), 2.28-2.90 (m, 4H).

### Example 570C

### methyl 2-{[2-amino-4-(morpholin-4-ylmethyl)phenyl]amino}-4-chlorobenzoate

The title compound was prepared by substituting Example 570B for Example 6B in Example 6C. MS (DCI) m/e 376 (M+H)⁺; ¹H NMR (300 MHz, DMSO-d₆) δ 8.81 (s, 1H), 7.85 (d, J= 8.73 Hz, 1H), 6.96 (d, J= 7.80 Hz, 1 H,) 6.80 (d, J= 1.56 Hz, 1H), 6.69 (dd, J= 8.73, 2.18 Hz, 1H), 6.55 (dd, J= 7.95, 1.72 Hz, 1H), 6.41 (d, J= 2.08 Hz, 1H), 4.95 (s, 2H), 3.66 (s, 3H), 3.58-3.60 (m, 4H), 3.35 (s, 2H), 2.35-2.37 (m, 4H).

### Example 570D

### 2-{[2-amino-4-(morpholin-4-ylmethyl)phenyl]amino}-4-chlorobenzoic acid

The title compound was prepared by substituting Example 570C for Example 12 in Example 13. MS (DCI) m/e 362 (M+H)⁺.

### Example 570E

### 3-chloro-8-(morpholin-4-ylmethyl)-5,10-dihydro-11H-dibenzo[b,e][1,4]diazepin-11-one

The title compound was prepared by substituting Example 570D for Example 233F in Example 233G. MS (APCI) m/e 344 (M+H)⁺; 1H NMR (300 MHz, DMSO-d₆) δ 10.02 (s, 1H), 8.29 (s, 1H), 7.86 (d, J= 8.73 Hz, 1H), 7.24 (d, J= 1.87 Hz, 1 H,) 7.04-7.12 (m, 4H), 3.72-3.74 (m, 4H), 3.37 (s, 2H), 2.47-2.49 (m, 2H).

### Example 570F

### 3-(3-methoxy-4-nitrophenyl)-8-(morpholin-4-ylmethyl)-5,10-dihydro-11H-dibenzo[b,e][1,4]diazepin-11-one

The title compound was prepared by substituting Example 570E and Example 266G for Example 59B and Example 56A, respectively, in Example 59C. MS (APCI) m/e 474 (M+H)⁺; 1H NMR (500 MHz, DMSO-d₆) δ 9.84 (s, 1H), 8.00 (d, J= 8.54 Hz, 1H), 7.97 (s, 1H), 7.89 (d, J= 8.24 Hz, 1H), 7.51 (d, J= 1.56 Hz, 1H), 7.32-7.34 (m, 2H), 7.29 (dd, J= 8.11, 1.56 Hz, 1H), 6.87-6.97 (m 4H), 4.02 (s, 3H), 3.55 (br, s, 4H,) 3.40-3.44 (m, 2H), 2.31 (br, s, 4H).

### Example 571

### 8-{[(2R)-2-(hydroxymethyl)pyrrolidin-1-yl]methyl}-3-(3-methoxy-4-nitrophenyl)-5,10-dihydro-11H-dibenzo[b,e][1,4]diazepin-11-one

The title compound was prepared by substituting (2R)-pyrrolidin-2-ylmethanol for morpholine in Example 570B, 570C, 570D, 570E, and 570F. MS (APCI) m/e 478 (M+H)⁺.

### Example 572

### 7-(2-hydroxyethoxy)-8-methoxy-3-(3-methoxy-4-nitrophenyl)-5,10-dihydro-11H-dibenzo[b,e][1,4]diazepin-11-one

### Example 572A

### 7-[2-(tert-butyl-dimethyl-silanyloxy)-ethoxy]-8-methoxy-3-(3-methoxy-4-nitro-phenyl)-5,10-dihydro-dibenzo[b,e][1,4]diazepin-11-one

The desired product was prepared by substituting (2-bromoethoxy)-tert-butyldimethylsilane for 2-bromomethyl-tetrahydro-2H-pyran in Example 478.

### Example 572B

### 7-(2-hydroxyethoxy)-8-methoxy-3-(3-methoxy-4-nitrophenyl)-5,10-dihydro-11H-dibenzo[b,e][1,4]diazepin-11-one

The desired product was prepared by substituting Example 572A for Example 500A in Example 500B. MS (ESI) m/e 450 (M-H)⁻; ¹H NMR (500 MHz, DMSO-d₆) δ 9.63 (s, 1H), 8.01 (d, J = 8.0 Hz, 1H), 7.78 (d, J = 8.0 Hz, 1H), 7.72 (s, 1H), 7.53 (s, 1H), 7.28-7.36 (m, 3H), 6.71 (s, 1H), 6.65 (s, 1H), 4.81 (t, J=5.3 Hz, 1H), 4.03 (s, 3H), 3.90 (t, J=4.7 Hz, 2H), 3.68-3.70 (m, 2H), 3.68 (s, 3H).

### Example 573

### 8-{3-[2-(hydroxymethyl)pyrrolidin-1-yl]-3-oxopropyl}-3-(3-methoxy-4-nitrophenyl)-5,10-dihydro-11H-dibenzo[b,e][1,4]diazepin-11-one

The desired product was prepared by substituting Example 230 and pyrrolidin-2-ylmethanol for dimethylaminoacetic acid and Example 120, respectively, in Example 122. MS (ESI) m/e517 (M+H)⁺; ¹H NMR (300 MHz, DMSO-d₆) δ 9.83 (s, 1H), 8.01 (d, J=8.48 Hz, 1H), 7.93 (s, 1H), 7.79 (d, J=8.14 Hz, 1H), 7.52 (d, J=1.36 Hz, 1H), 7.34 (m, 2H), 7.29 (dd, J=8.14, 1.7 Hz, 1H), 6.92 (m, 1H), 6.84 (dd, J=4.07, 2.37 Hz, 2H), 4.03 (s, 3H), 3.78 (s, 1H), 3.48 (dd, J=10.51, 4.07 Hz, 1H), 3.21 (m, 2H), 2.86 (d, J=35.6 Hz, 2H), 2.7 (m, 2H), 1.79 (m, 3H).

### Example 574

### (cis) 4-hydroxyN[3-(3-methoxy-4-nitrophenyl)-11-oxo-10,11-dihydro-5H-dibenzo[b,e][1,4]diazepin-7-yl]cyclohexanecarboxamide

The title compound was prepared by substituting Example 179 and (cis) 4-hydroxycyclohexanecarboxylic acid for Example 120 and dimethylaminoacetic acid, respectively, in Example 122. MS (DCI) m/e 503 (M+H)⁺; ¹H NMR (500 MHz, DMSO-d₆) δ 9.78 (s, 1H), 9.66 (s, 1H), 8.00-8.02 (m, 2H), 7.79 (d, J=8.11 Hz, 1H), 7.62 (d, J=1.87 Hz, 1H), 7.52 (d, J=1.56 Hz, 1H), 7.42 (d, J=1.56 Hz, 1H), 7.35 (dd, J=8.42, 1.56 Hz, 1H), 7.31 (dd, J=8.26, 1.72 Hz, 1H), 6.91-6.93 (m, 1H), 6.87 (d, J=8.42 Hz, 1H), 4.04 (s, 3H), 3.79 (s, 1H), 2.29 (m, 1H), 1.80-1.86 (m 2H), 1.67-1.70 (m, 2H), 1.42-1.49 (m, 4H).

It will be evident to one skilled in the art that the present invention is not limited to the foregoing illustrative examples, and that it can be embodied in other specific forms without departing from the essential attributes thereof. It is therefore desired that the examples be considered in all respects as illustrative and not restrictive, reference being made to the appended claims, rather than to the foregoing examples, and all changes which come within the meaning and range of equivalency of the claims are therefore intended to be embraced therein.

## Claims

1. A compound of formula (I) 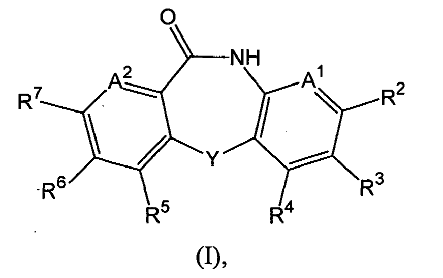 or a therapeutically acceptable salt thereof, wherein
(a)
A¹ is CH;
A² is CH;
R² is selected from the group consisting of hydrogen, alkenyl, alkoxy, alkoxyalkoxy, alkoxyalkoxyalkoxy, alkoxyalkyl, alkoxycarbonyl, alkoxycarbonylalkyl, alkyl, alkylcarbonyl, alkylcarbonylalkyl, amino, aminoalkoxy, aminoalkyl, aminocarbonyl, aminocarbonylalkyl, aminosulfonyl, aryl, arylalkoxy, arylalkyl, aryloxyalkyl, carboxy, carboxyalkyl, cyano, cyanoalkyl, cycloalkyl, cycloalkylalkyl, halo, haloalkoxy, haloalkyl, heterocyclyl, heterocyclylalkoxy, heterocyclylalkyl, heterocyclylcarbonylalkyl, heterocyclyloxyalkyl, hydroxy, hydroxyalkoxy, hydroxyalkyl, nitro, nitroalkyl, and -(CR⁹R¹⁰)ₘC(O)NR¹¹R¹²;
R³, R⁴ and R⁵ are each hydrogen;
R⁶ is selected from the group consisting of aryl, cycloalkyl, halo, heterocyclyl, and -XR¹³;
R⁷ is hydrogen;
R⁹ and R¹⁰ are independently selected from the group consisting of hydrogen, alkenyl, alkyl, aminoalkyl, and hydroxyalkyl; or
R⁹ and R¹⁰, together with the carbon atom to which they are attached, form a cycloalkyl group;
R¹¹ and R¹² are each independently selected from the group consisting of hydrogen, alkenyl, alkoxyalkyl, alkyl, amino, aminoalkyl, aryl, arylalkyl, cyanoalkyl, cycloalkyl, cycloalkylalkyl, haloalkyl, heterocyclyl, heterocyclylalkyl, hydroxyalkyl, -NR^{c}R^{d}, (NR^{c}R^{d})alkyl, and (NR^{c}R^{d})carbonylalkyl; or
R¹¹ and R¹², together with the nitrogen atom to which they are attached, form a heterocyclyl ring selected from the group consisting of azetidinyl, diazepanyl, imidazolidinyl, morpholinyl, piperazinyl, piperidinyl, pyrrolidinyl and thiomorpholinyl, which can each be optionally substituted with one, two, or three substituents independently selected from the group consisting of alkoxy, alkoxycarbonyl, alkenyl, alkyl, alkylcarbonyl, aryl, carboxy, carboxyalkyl, heterocyclyl, heterocyclylalkyl, hydroxy, hydroxyalkyl, -NR^{c}R^{d}, (NR^{c}R^{d})alkyl, and (NR^{c}R^{d})carbonyl;
R¹³ is selected from the group consisting of aryl, cycloalkyl, and heterocyclyl;
X is selected from the group consisting of -O-, -NR¹⁴-, -C(O)-, -S-, -SO₂- , -(CH₂)ₙ-, -C(O)NR¹⁴-, -NR¹⁴C(O)-, -SO₂NR¹⁴-, -NR¹⁴SO₂, -O(CH₂)ₘ-, -(CH₂)ₘO-, -CH=CH-, and -C≡C-; wherein R¹⁴ is selected from the group consisting of hydrogen, alkenyl, alkyl, aminoalkyl, and hydroxyalkyl;
Y is NH;
m is 0-3; and
n is 1-3;
or wherein
(b)
A¹ is CH;
A² is CH;
Y is NH;
R⁴, R⁵, and R⁷ are each hydrogen;
R⁶ is phenyl substituted in the three position with methoxy and substituted in the four position with alkoxycarbonyl, alkoxycarbonylalkenyl, alkylcarbonyl, alkylsulfonyl, cyano, halo, haloalkenyl, hydroxy, nitro, NH₂SO₂-, or NH₂CO-; and either
(i) R² is hydrogen and R³ is -(CR⁹R¹⁰)C(O)NHR¹² where R⁹ and R¹⁰ are independently selected from the group consisting of hydrogen, alkenyl, alkyl, aminoalkyl, and hydroxyalkyl, or R⁹ and R¹⁰, together with the carbon atom to which they are attached, form a cycloalkyl group, and R¹² is phenyl optionally substituted with 1 morpholinyl group;
(ii) R² is hydrogen and R³ is hydroxyalkyl;
(iii) R² is hydrogen and R³ is alkoxy;
(iv) R² and R³ are independently -(CR⁹R¹⁰)C(O)NHR¹² where R⁹ and R¹⁰ are independently selected from the group consisting of hydrogen, alkenyl, alkyl, aminoalkyl, and hydroxyalkyl, or R⁹ and R¹⁰, together with the carbon atom to which they are attached, form a cycloalkyl group, and R¹² is phenyl optionally substituted with 1 morpholinyl group;
(v) R² and R³ are independently hydroxalkyl; or
(vi) R² and R³ are independently alkoxy;
wherein
alkyl is a C₁-C₁₀ alkyl;
alkenyl is a C₂-C₆ alkenyl;
alkoxy is a C₁-C₁₀ alkoxy;
aryl is phenyl group, or a bicyclic or tricyclic fused ring system wherein one or more of the fused rings is a phenyl group, wherein aryl can be optionally substituted with one, two, three, four, or five substituents independently selected from the group consisting of alkoxy, alkoxyalkyl, alkoxycarbonyl, alkoxycarbonylalkenyl, alkoxycarbonylalkyl, alkoxysulfonyl, alkyl, alkylcarbonyl, alkylsulfonyl, amino, aminocarbonyl, aminosulfonyl, a second aryl group, arylalkyl, arylsulfonyl, carboxy, cyano, formyl, halo, haloalkoxy, haloalkenyl, haloalkyl, heterocyclyl, heterocyclylalkyl, hydroxy, hydroxyalkyl, nitro, (NR^{c}R^{d})alkyl, and oxo, wherein the second aryl group, the aryl part of the arylalkyl and the arylsulfonyl, the heterocyclyl, and the heterocyclyl part of the heterocyclylalkyl can be optionally substituted with one, two, three, four, or five substituents independently selected from the group consisting of alkoxy, alkoxyalkyl, alkoxycarbonyl, alkyl, alkylcarbonyl, carboxy, cyano, halo, haloalkoxy, haloalkyl, hydroxy, nitro, and oxo;
heterocyclyl is selected from a five-, six-, or seven-membered ring containing one, two, or three heteroatoms independently selected from the group consisting of nitrogen, oxygen, and sulfur, wherein the five-membered ring has zero to two double bonds and the six- and seven-membered rings have zero to three double bonds; bicyclic groups in which the heterocyclyl ring is fused to a phenyl group, a monocyclic cycloalkenyl group, a monocyclic cycloalkyl group, or another monocyclic heterocyclyl group; and tricyclic groups in which a bicyclic system is fused to a phenyl group, a monocyclic cycloalkenyl group, a monocyclic cycloalkyl group, or another monocyclic heterocyclyl group; wherein heterocyclyl can be optionally substituted with one, two, three, four, or five substituents independently selected from the group consisting of alkoxy, alkoxyalkyl, alkoxycarbonyl, alkyl, alkylcarbonyl, alkylsulfonyl, amino, aminocarbonyl, aminosulfonyl, aryl, arylalkyl, arylsulfonyl, carboxy, cyano, halo, a second heterocyclyl group, heterocyclylalkyl, hydroxy, hydroxyalkyl, nitro, -NR^{c}R^{d}, (NR^{c}R^{d})alkyl, and oxo, wherein the aryl, the aryl part of the arylalkyl and the arylsulfonyl, the second heterocyclyl group, and the heterocyclyl part of the heterocyclylalkyl can be optionally substituted with one, two, three, four, or five substituents independently selected from the group consisting of alkoxy, alkoxyalkyl, alkoxycarbonyl, alkyl, alkylcarbonyl, carboxy, cyano, halo, haloalkoxy, haloalkyl, hydroxy, methylenedioxy, nitro, and oxo;
cycloalkyl is a saturated monocyclic, bicyclic, or tricyclic hydrocarbon ring system having three to twelve carbon atoms, wherein cycloalkyl can be optionally substituted with one, two, three, or four substituents independently selected from the group consisting of alkoxy, alkoxyalkyl, alkoxycarbonyl, alkyl, alkylcarbonyl, alkylsulfonyl, -NR^{c}R^{d}, (NR^{c}R^{d})alkyl, (NR^{c}R^{d})carbonyl, a second aryl group, arylalkyl, arylsulfonyl, carboxy, cyano, halo, heterocyclyl, heterocyclylalkyl, hydroxy, hydroxyalkyl, nitro, and oxo, wherein the second aryl group, the aryl part of the arylalkyl and the arylsulfonyl, the heterocyclyl, and the heterocyclyl part of the heterocyclylalkyl can be optionally substituted with one, two, three, four, or five substituents independently selected from the group consisting of alkoxy, alkoxyalkyl, alkoxycarbonyl, alkyl, alkylcarbonyl, carboxy, cyano, halo, haloalkoxy, haloalkyl, hydroxy, nitro, and oxo;
cycloalkenyl is a non-aromatic cyclic or bicyclic ring system having three to ten carbon atoms and one to three rings, wherein each five-membered ring has one double bond, each six-membered ring has one or two double bonds, each seven-and eight-membered ring has one to three double bonds, and each nine-to ten-membered ring has one to four double bonds;
amino refers to -NR^{a}R^{b}, wherein R^{a} and R^{b} are independently selected from the group consisting of hydrogen, alkoxyalkylcarbonyl, alkoxycarbonyl, alkyl, alkylcarbonyl, alkylsulfonyl, aryl, arylalkyl, arylalkylcarbonyl, arylalkylsulfonyl, arylsulfonyl, arylsulfonylalkyl, arylsulfonylalkylcarbonyl, cycloalkyl, cycloalkylalkyl, cycloalkylcarbonyl, haloalkylsulfonyl, heterocyclyl, heterocyclylalkylcarbonyl, heterocyclylalkylsulfonyl, heterocyclylcarbonyl, heterocyclylsulfonyl, -NR^{c}R^{d}, (NR^{c}R^{d})alkyl, (NR^{c}R^{d})alkylcarbonyl, (NR^{c}R^{d})alkylsulfonyl, (NR^{c}R^{d})carbonyl, (NR^{c}R^{d})carbonylalkyl, and (NR^{c}R^{d})carbonylalkylcarbonyl, wherein aryl can be optionally substituted with one, two, three, four, or five substituents independently selected from the group consisting of alkoxy, alkoxyalkyl, alkoxycarbonyl, alkyl, alkylcarbonyl, alkylsulfonyl, a second aryl group, arylalkyl, arylsulfonyl, carboxy, cyano, halo, heterocyclyl, heterocyclylalkyl, hydroxy, nitro, and oxo, wherein the second aryl group, the aryl part of the arylalkyl and the arylsulfonyl, the heterocyclyl, and the heterocyclyl part of the heterocyclylalkyl can be optionally substituted with one, two, three, four, or five substituents independently selected from the group consisting of alkoxy, alkoxyalkyl, alkoxycarbonyl, alkyl, alkylcarbonyl, carboxy, cyano, halo, haloalkoxy, haloalkyl, hydroxy, nitro, and oxo; and
R^{c} and R^{d} are each independently hydrogen, alkoxycarbonyl, alkyl, alkylcarbonyl, aryl, arylsulfonyl, heterocyclcarbonyl, or formyl;
provided that 3-chloro-11-oxo-10,11-dihydro-SH-dibenzo[b,e][1,4]diazepine and 3-fluoro-11-oxo-10,11-dihydro-5H-dibenzo[b,e][1,4]diazepine are excluded.

2. The compound of claim 1, part (a) wherein R² is hydrogen.

3. The compound of claim 1, part (a) wherein R² is selected from the group consisting of alkoxycarbonyl and alkoxycarbonylalkyl.

4. The compound of claim 1, part (a) wherein R² is selected from the group consisting of amino and aryl.

5. The compound of claim 1, part (a) wherein R² is selected from the group consisting of carboxy, carboxyalkyl, cyano, nitro, and heterocyclyl.

6. The compound of claim 1, part (a) wherein R² is -(CR⁹R¹⁰)ₘC(O)NR¹¹R¹².

7. The compound of claim 6 wherein m is 0.

8. The compound of claim 7 wherein one of R¹¹ and R¹² is hydrogen and the other is heterocyclylalkyl.

9. The compound of claim 7 wherein one of R¹¹ and R¹² is hydrogen and the other is selected from the group consisting of amino, aminoalkyl, arylalkyl, and hydroxyalkyl.

10. The compound of claim 6 wherein R¹¹ and R¹², together with the nitrogen atom to which they are attached, form a heterocyclyl ring selected from the group consisting of diazepanyl, imidazolidinyl, morpholinyl, piperazinyl, piperidinyl, and pyrrolidinyl, which can each be optionally substituted with one, two, or three substituents independently selected from the group consisting of alkoxy, alkoxycarbonyl, alkenyl, alkyl, alkylcarbonyl, aryl, carboxy, carboxy, heterocyclyl, heterocyclylalkyl, hydroxy, and hydroxyalkyl.

11. The compound of claim 6 wherein m is 1.

12. The compound of claim 11 wherein one of R¹¹ and R¹² is selected from the group consisting of hydrogen and alkyl and the other is heterocyclylalkyl.

13. The compound of claim 11 wherein one of R¹¹ and R¹² is selected from the group consisting of hydrogen and alkyl and the other is selected from the group consisting of alkoxyalkyl and hydroxyalkyl.

14. The compound of claim 11 wherein one of R¹¹ and R¹² is selected from the group consisting of hydrogen and alkyl and the other is selected from the group consisting of alkyl, aminoalkyl, aryl, aryalkyl, and heterocyclyl.

15. The compound of claim 11 wherein R¹¹ and R¹², together with the nitrogen atom to which they are attached, form a heterocyclyl ring selected from the group consisting of diazepanyl, imidazolidinyl, morpholinyl, piperazinyl, piperidinyl, and pyrrolidinyl, which can each be optionally substituted with one, two, or three substituents independently selected from the group consisting of alkoxy, alkoxycarbonyl, alkenyl, alkyl, alkylcarbonyl, aryl, carboxy, carboxy, heterocyclyl, heterocyclylalkyl, hydroxy, and hydroxyalkyl.

16. The compound of claim 1 selected from the group consisting of
3-(4-hydroxy-3-methoxyphenyl)-5,10-dihydro-11H-dibenzo[b,e][1,4]diazepin-11-one;
3-(11-oxo-10,11-dihydro-5H-dibenzo[b, e][1,4] diazepin-3-yl)benzonitrile;
3-(3-methoxy-4-nitrophenyl)-5,10-dihydro-1H-dibenzo[b,e][1,4] diazepin-11-one;
3-(4-chloro-3-methoxyphenyl)-5,10-dihydro-11H-dibenzo[b,e][1,4]diazepin-11-one;
3-(4-bromo-3-methoxyphenyl)-5,10-dihydro-11H-dibenzo[b,e][1,4]diazepin-11-one;
3-(4-acetyl-3-methoxyphenyl)-5,10-dihydro-11H-dibenzo[b,e][1,4]diazepin-11-one;
2-methoxy-4-(11-oxo-10,11-dihydro-5H-dibenzo[b,e][1,4]diazepin-3-yl)benzonitrile;
methyl 2-methoxy-4-(l-oxo-10,1l-dihydro-5H-dibenzo[b,e][l,4]diazepin-3-yl)benzoate;
2-methoxy-4-(11-oxo-10,11-dihydro-5H-dibenzo[b,e][1,4]diazepin-3-yl)benzoic acid;
N-(3,4-dihydroxybenzyl)-2-methoxy-4-(11-oxo-10,11-dihydro-5H-dibenzo[b,e][1,4]diazepin-3-yl)benzamide;
2-methoxy-4-(11-oxo-10,11-dihydro-5H-dibenzo[b,e][1,4]diazepin-3-yl)benzamide;
3-(2-methoxy-4-pyridinyl)-5,10-dihydro-11H-dibenzo[b,e][1,4]diazepin-11-one;
methyl 3-(4-hydroxy-3-methoxyphenyl)-11-oxo-10,11-dihydro-5H-dibenzo[b,e][1,4]diazepine-8-carboxylate;
methyl [3-(3-methoxy-4-nitrophenyl)-11-oxo-10,11-dihydro-5H-dibenzo[b,e][1,4]diazepm-8-yl]acetate;
ethyl [3-(4-hydroxy-3-methoxyphenyl)-11-oxo-10,11-dihydro-5H-dibenzo[b,e][1,4]diazepni-4-yl]acetate:
methyl [3-(3-methoxyphenyl)-11-oxo-10,11-dihydro-5H-dibenzo[b,e][1,4]diazepin-8-yl]acetate;
methyl [3-(4-chloro-3-methoxyphenyl)-11-oxo-10,11-dihydro-5H-dibenzo[b,e][1,4]diazepin-8-yl]acetafe;
methyl [3-(4-bromo-3-methoxyphenyl)-11-oxo-10,11-dihydro-5H-dibenzo[b,e][1,4]diazepin-8-yl]acetate;
methyl [3-(4-cyano-3-methoxyphenyl)-11-oxo-10,11-dihydro-5H-dibenzo[b,e][1,4]diazepin-8-yl]acetate;
methyl [3-(5-methoxy-2-methyl-4-nitrophenyl)-11-oxo-10,11.-dihydro-5H-dibenzo[b,e][1,4]diazepin-8-yl]acetate;
methyl [3-(4-cyano-5-methoxy-2-methylphenyl)-11-oxo-10,11-dihydro-5H-dibenzo[b,e][1,4]diazepin-8-yl]acetate,;
methyl [3-(2-fluoro-5-methyl-4-pyridinyl)-11-oxo-10,11-dihydro-5H-dibenzo[b,e][1,4]diazepin-8-yl]acetate;
methyl [3-(2-methoxy-5-methyl-4-pyridinyl)-11-oxo-10,11-dihydro-5H-dibenzo[b,e][1,4]diazepin-8-yl]acetate;
8-amino-3-(4-hydroxy-3-methoxyphenyl)-5,14-dihydro-11H-dibenzo[b,e][1,4]diazepin-11-one;
N-[3-(4-hydroxy-3-methoxyphenyl)-11-oxo-10,11-dihydro-5H-dibenzo[b,e][1,4]diazepin-8-yl]methanesulfonamide;
N-[3-(3-methoxy-4-nitrophenyl)-11-oxo-10,11-dihydro-5H-dibenzo[b,e][1,4]diazepin-8-yl]methanesulfonamide;
N-[3-(4-hydroxy-3-methoxyphenyl)-11-oxo-10,11-dihydro-5H-dibenzo[b,e][1,4]diazepin-8-yl]acetamide;
N-[3-(3-methoxy-4-nitrophenyl)-11-oxo-10,11-dihydro-5H-dibenzo[b,e][1,4]diazepin-8-yl]acetamide;
8-(3-aminophenyl)-3-(4-hydroxy-3-methoxyphenyl)-5,10-dihydro-11H-dibenzo[b,e][1,4]diazepin-11-one;
3-(4-hydroxy-3-methoxyphenyl)-8-(3-hydroxyphenyl)-5,10-dihydro-11H-dibenzo[b,e][1,4]diazepin-11-one; and
8-amino-3-(3-methoxy-4-nitrophenyl)-5,10-dihydro-11H-dibenzo[b,e][1,4]diazepin-11-one,
3-(4-hydroxy-3-methoxyphenyl)-11-oxo-10,11-dihydro-5H-dibenzo[b,e][1,4]diazepine-8-carboxylic acid;
3-(4-hydroxy-3-methoxyphenyl)-11-oxo-10,11-dihydro-5H-dibenzo[b,e][1,4]diazepine-8-carbonitrile;
3-(4-hydroxy-3-methoxyphenyl)-8-nitro-5,10-dihydro-11H-dibenzo[b,e][1,4]diazepin-11-one;
3-(4-hydroxy-3-methoxyphenyl)-8-(3-pyridinyl)-5,10-dihydro-11H-dihenzo[b,e][1,4]diazepin-11-one;
3-(4-hydroxy-3-methoxyphenyl)-8-(1H-pyrol-2-yl)-5,10-dihydro-11H-dibenzo[b,e][1,4]diazepin-11-one;
3-(3-methoxy-4-nitrophenyl)-8-(1H-pyrrol-2-yl)-5,10-dihydro-11H-dibenzo[b,e][1,4]diazepin-11-ante;
[3-(3-methoxy-4-nitrophenyl)-11-oxo-10,11-dihydro-5H-dibenzo[b,e][1,4]diazepin-8-yl]acetic acid;
3-(4-hydroxy-3-methoxyphenyl)-11-oxoN[3-(1-pyrrolidinyl)propyl]-10,11-dihydro-5H-dibenzo[b,e][1,4]diazepine-8-carboxamide;
3-(4-hydroxy-3-methoxyphenyl)N[3-(4-morpholinyl)propyl]-11-oxo-10,11-dihydro-5H-dibenzo[b,e][1,4]diazepine-8-carboxamide;
3-(4-hydroxy-3-methoxyphenyl)-11-oxoN[3-(2-oxo-1-pyrrolidinyl)propyl]-10,11-dihydro-5H-dibenzo[b,e][1,4]diazepine-8-carboxamide;
3-(4-hydroxy-3-methoxyphenyl)-11-oxoN(3-pyridinylmethyl)-10,11-dihydro-5H-dibenzo[b,e][1,4]diazepine-8-carboxamide;
3-(4-hydroxy-3-methoxyphenyl)-11-oxoN(2-pyridinylmethyl)-10,11-dihydro-5H-dibenzo[b,e][1,4]diazepine-8-carboxamide;
3-(4-hydroxy-3-methoxyphenyl)-11-oxoN(4-pyridinylniethyl)-10,11-dihydro-5H-dibenzo[b,e][1,4]diazepine-8-carboxamide;
3-(2-methoxy-4-pyridinyl)-11-oxoN[3-(1-pyrrolidinyl)propyl]-10,11-dihydro-5H-dibenzo[b,e][1,4]diazepine-8-carboxamide;
11-oxo-3-(2-oxo-1,2-dihydro-4-pyridinyl)N[3-(1-pyrrolidinyl)propyl]-10,11-dihydro-5H-dibenzo[b,e][1,4]diazepine-8-carboxamid;
N-[3-(dimethylamino)propyl]-3-(4-hydroxy-3-methoxyphenyl)-11-oxo-10,11-dihydro-5H-dibenzo[b,e][1,4]diazepine-8-carboxamide;
N-(2-hydroxyethyl)-3-(4-hydroxy-3-methoxyphenyl)-11-oxo-10,11-dihydro-5H-dibenzo[b,e][1,4]diazepine-8-carboxamide;
N-(2,3-dihydroxypropyl)-3-(4-hydroxy-3-methoxyphenyl)-11-oxo-10, 11-dihydro-5H-dibenzo [b,e][1,4] diazepine-8-carboxamide;
N-[2-(acetylamino)ethyl]-3-(4-hydroxy-3-methoxyphenyl)-11-oxo-10,11-dihydro-5H-dibenzo[b,e] [1,4]diazepine-8-carboxamide;
3-(4-hydroxy-3-methoxyphenyl)N[4-(methylsulfonyl)benzyl]-11-oxo-10,11-dihydro-5H-dibenzo[b,e][1,4]diazepine-8-carboxamide;
N-(2-fluorobenzyl)-3-(4-hydroxy-3-methoxyphenyl)-11-oxo-10,11-dihydro-5H-dibenzo[b,e][1,4]diazepine-8-carboxamide;
3-(4-hydroxy-3-methoxyphenyl)N(2-methoxybenzyl)-11-oxo-10,11-dihydro-5H-dibenzo[b,e][1,4]diazepine-8-carboxamide;
3-(4-hydroxy-3-methoxyphenyl)N[2-(4-methoxyphenyl)ethyl]-11-oxo-10,11-dihydro-5H-dibenzo[b,e][1,4]diazepine-8-carboxamide;
tert-butyl 2-{[3-(4-hydroxy-3-methoxyphenyl)-11-oxo-10,11-dihydro-5H-dibenzo[b,e][1,4]diazepin-8-yl]carbonyl}hydrazinecarboxylate;
3-(4-hydroxy-3-methoxyphenyl)-8-[(3-hydroxy-1-pyrrolidinyl)carbonyl]-5,10-dihydro-11H-dibenzo[b,e][1,4]diazepin-11-one;
3-(4-hydroxy-3-methoxyphenyl)-8-{[(2S)-2-(hydroxymethyl)-1-pyrrolidinyl]carbonyl}-5,10-dihydro-11H-dibenzo[b,e][1,4]diazepin-11-one;
3-(4-hydroxy-3-methoxyphenyl)-8-{[2-(hydroxymethyl)-1-piperidinyl]carbonyl}-5,10-dihydro-11H-dibenzo[b,e][1,4]diazepin-11-one;
ethyl 1-{[3-(4-hydroxy-3-methoxyphenyl)-11-oxo-10,11-dihydro-5H-dibenzo[b,e][1,4]diazepin-8-yl]carbonyl}-2-piperidinecarboxylate;
ethyl 1-{[3-(4-hydroxy-3-methoxyphenyl)-11-oxo-10,11-dihydro-5H-dibenzo[b,e][1,4]diazepin-8-yl]carbonyl}-3-piperidinecarboxylate;
ethyl 1-{[3-(4-hydroxy-3-methoxyphenyl)-11-oxo-10,11-dihydro-5H-dibenzo[b,e][1,4]diazepin-8-yl]carbonyl}-4-piperidinecarboxylate;
3-(4-hydroxy-3-methoxyphenyl)-8-[(3-hydroxy-1-piperidinyl)carbonyl]-5,10-dihydro-11H-dibenzo[b,e][1,4]diazepin-11-one;
1-{[3-(4-hydroxy-3-methoxyphenyl)-11-oxo-10,11-dihydro-5H-dibenzo[b,e][1,4]diazepin-8-yl]carbonyl}-2-pipendinecarboxylic acid;
1-{[3-(4-hydroxy-3-methoxyphenyl)-11-oxo-10,11-dihydro-5H-dibenzo[b,e][1,4]diazepin-8-yl]carbonyl}-3-piperidinecarboxylic acid;
1-{[3-(4-hydroxy-3-methoxyphenyl)-11-oxo-10,11-dihydro-5H-dibenzo[b,e][1,4]diazepin-8-yl]carbonyl}-4-piperidinecarboxylic acid;
2-[3-(3-methoxy-4-nitrophenyl)-11-oxo-10,11-dihydro-5H-dibenzo[b,e][1,4]diazepin-8-yl]N[3-(1-pyrrolidinyl)propyl]acetamide;
2-[3-(3-methoxy-4-nitrophenyl)-11-oxo-10,11-dihydro-5H-dibenzo[b,e][1,4]diazepin-8~yl]N[2-(2-pyridinyl)ethyl]acetamide;
2-[3-(3-methoxy-4-nitrophenyl)-11-oxo-10,11-dihydro-5H-dibenzo[b,e][1,4]diazepin-8-yl]N[2-(3-pyridinyl)ethyl]acetamide;
2-[3-(3-Methoxy-4-nitro-phenyl)-11-oxo-10,11-dihydro-5H-dibenzo[b,e][1,4]diazepin-8-yl]N(4-pyridin-2-yl-ethyl)-acetamide;
N-[3-(1H-imidazol-1-yl)propyl]-2-[3-(3-methoxy-4-nitrophenyl)-11-oxo-10,11-dihydro-5H-dibenzo[b,e][1,4]diazepin-8-yl]acetamide;
2-[3-(3-methoxy-4-nitrophenyl)-11-oxo-10,11-dihydro-5H-dibenzo[b,e][1,4]diazepin-8-yl]NmethylN[2-(2-pyridinyl)ethyl]acetamide;
2-[3-(3-methoxy-4-nitrophenyl)-11-oxo-10,11-dihydro-5H-dibenzo[b,e][1,4]diazepin-8-yl]N(3-pyiidinylmethyl)acetaniide;
2-[3-(3-methoxy-4-nitrophenyl)-11-oxo-10,11-dihydro-5H-dibenzo[b,e][1,4]diazepin-8-yl]N(4-pyridmy]methyl)acetamide;
2-[3-(3-methoxy-4-nitrophenyl)-11-oxo-10,11-dihydro-5H-dibenezo[b,e][1,4]diazepin-8-yl]N(2-pyridinylmethyl)acetamide;
2-[3-(3-methoxy-4-nitrophenyl)-11-oxo-10,11-dihydro-5H-dibenzo[b,e][1,4]diazepin-8-yl]N[2-(1-pyrrolidinyl)ethyl]acetamide;
2-[3-(3-methoxy-4-nitrophenyl)-11-oxo-10,11-dihydro-5H-dibenzo[b,e][1,4]diazepin-8-yl]N[2-(1-piperidinyl)ethyl]acetamide;
2-[3-(3-methoxy-4-nitrophenyl)-11-oxo-10,11-dihydro-5H-dibenzo[b,e][1,4]diazepin-8-yl]N[3-(1-piperidinyl)propyl]acetamide;
2-[3-(3-methoxy-4-nitrophenyl)-11-oxo-10,11-dihydro-5H-dibenzo[b,e][1,4]diazepin-8-yl]N[2-(4-morpholinyl)ethyl]acetamide;
2-[3-(3-methoxy-4-nitrophenyl)-11-oxo-10,11-dihydro-5H-dibenzo[b,e][1,4]diazepin-8-yl]N[3-(4-morpholinyl)propyl]acetamide;
2-[3-(3-methoxy-4-nitrophenyl)-11-oxo-10,11-dihydro-5H-dibenzo[b,e][1,4]diazepin-8-yl]N[2-(4-methyl-1-piperazinyl)ethyl] acetamide;
N-[(1S)-1-(hydroxymethyl)-2-methylpropyl]-2-[3-(3-methoxy-4-nitrophenyl)-11-oxo-10,11-dihydro-5H-dibenzo[b,e][1,4]diazepin-8-yl]acetamide;
N-[(1R)-1-(hydroxymethyl)-2-methylpropyl]-2-[3-(3-methoxy-4-nitrophenyl)-11-oxo-10,11-dihydro-5H-dibenzo[b,e][1,4]diazepin-8-yl]acetamide;
N-(3-ethoxypropyl)-2-[3-(3-methoxy-4-nitrophenyl)-11-oxo-10,11-diliydro-5H-dibenzo[b,e][1,4]diazepin-8-yl]acetamide;
N-[(1R)-1-(hydroxymethyl)-3-methylbutyl]-2-[3-(3-methoxy-4-nitrophenyl)-11-oxo-10,11-dihydro-5H-dibenzo[b,e][1,4]diazepin-8-yl]acetamide;
N-[(1S)-1-(hydroxymethyl)-3-methylbutyl]-2-[3-(3-methoxy-4-nitrophenyl)-11-oxo-10,11-dihydro-5H-dibenzo[b,e][1,4]diazepin-8-yl]acetamide;
N-(3-hydroxypropyl)-2-[3-(3-methoxy-4-nitrophenyl)-11-oxo-10,11-dihydro-5H-dibenzo[b,e][1,4]diazepin-8-yl]acetamide;
N-[(1S)-2-hydroxy-1-phenylethyl]-2-[3-(3-methoxy-4-nitrophenyl)-11-oxo-10,11-dihydro-5H-dibenzo[b,e][1,4]diazepin-8-yl]acetamide;
N-[(1R)-2-hydroxy-1-phenylethyl]-2-[3-(3-methoxy-4-nitrophenyl)-11-oxo-10,11-dihydro-5H-dibenzo[b,e][1,4]diazepin-8-yl]acetamide;
N-(4-hydroxybutyl)-2-[3-(3-methoxy-4-nitrophenyl)-11-oxo-10,11-dihydro-5H-dibenzo[b,e][1,4]diazepin-8-y]acetamide;
N-(2-hydroxyethyl)-2-[3-(3-methoxy-4-nitrophenyl)-11-oxo-10,11-dihydro-5H-dibenzo[b,e][1,4]diazepin-8-yl]Npropylacetamide;
N-[2-(dimethylamino)ethyl]-2-[3-(3-methoxy-4-nitrophenyl)-11-oxo-10,11-dihydro-5H-dibenzo[b,e][1,4]diazepin-8-yl]Nmethylacetamide;
2-[3-(3-methoxy-4-nitrophenyl)-11-oxo-10,11-dihydro-5H-dibenzo[b,e][1,4]diazepin-8-yl]-N,N-dimethylacetamide;
2-[3-(3-methoxy-4-nitrophenyl)-11-oxo-10,11-dihydro-5H-dibenzo[b,e][1,4]diazepin-8-yl]N3-quinolinylacetamide;
2-[3-(3-methoxy-4-nitrophenyl)-11-oxo-10,11-dihydro-5H-dibenzo[b,e][1,4]diazepin-8-yl]N6-quinolinylacetamide;
2-[3-(3-methoxy-4-nitrophenyl)-11-oxo-10,11-dihydro-5H-dibenzo[b,e][1,4]diazepin-8-yl]N[4-(4-morpholinyl)phenyl]acetamide;
N-[2-(diethylamino)ethyl]-2-[3-(3-methoxy-4-nitrophenyl)-11-oxo-10,11-dihydro-5H-dibenzo[b,e][1,4]diazepin-8-yl]acetamide;
N-[3-(diethylamino)propyl]-2-[3-(3-methoxy-4-nitrophenyl)-11-oxo-10,11-dihydro-5H-dibenzo[b,e][1,4]diazepin-8-yl]acetamide;
N-(3,4-difluorobenzyl)-2-[3-(3-methoxy-4-nitrophenyl)-11-oxo-10,11-dihydro-5H-dibenzo[b,e][1,4]diazepin-8-yl]acetamide;
N-(1-benzyl-4-piperidinyl)-2-[3-(3-methoxy-4-nitrophenyl)-11-oxo-10,11-dihydro-5H-dibenzo[b,e][1,4]diazepin-8-yl]acetamide;
N-[4-(dimethylamino)butyl]-2-[3-(3-methoxy-4-nitrophenyl)-11-oxo-10,11-dihydro-5H-dibenzo[b,e][1,4]diazepin-8-yl]acetamide;
N-{2-[4-(aminosulfonyl)phenyl]ethyl}-2-[3-(3-methoxy-4-nitrophenyl)-11-oxo-10,11-dihydro-5H-dibenzo[b,e][1,4]diazepin-8-yl]acetamide;
N-[2-(dimethylamino)ethyl]-2-[3-(3-methoxy-4-nitrophenyl)-11-oxo-10,11-dihydro-5H-dibenzo[b,e][1,4]diazepin-8-yl]acetamide;
N-[3-(dimethylamino)propyl]-2-[3-(3-methoxy-4-nitrophenyl)-11-oxo-10,11-dihydro-5H-dibenzo[b,e][1,4]diazepin-8-yl]acetamide;
8-[2-(3-hydroxy-1-piperidinyl)-2-oxoethyl]-3-(3-methoxy-4-nitrophehyl)-5,10-dihydro-11H-dibenzo[b,e][1,4]diazepin-11-one;
3-(3-methoxy-4-nitrophenyl)-8-[2-(4-methyl-1,4-diazepan-1-yl)-2-oxoethyl]-5,10-dihydro-11H-dibenzo[b,e][1,4]diazepin-11-one;
8-[2-(4-hydroxy-1-piperidinyl)-2-oxoethyl]-3-(3-methoxy-4-nitrophenyl)-5,10-dihydro-11H-dibenzo[b,e][1,4]diazepin-11-one;
8-[2-(4-ethyl-1-piperazinyl)-2-oxoethyl]-3-(3-methoxy-4-nitrophenyl)-5,10-dihydro-11H-dibenzo[b,e][1,4]diazepin-11-one;
8-{2-[4-(2-hydroxyethyl)-1-piperazinyl]-2-oxoethyl}-3-(3-methoxy-4-nitrophenyl)-5,10-dihydro-11H-dibenzo[b,e][1,4]diazepin-11-one;
3-(3-methoxy-4-nitrophenyl)-8-[2-oxo-2-(4-phenyl-1-piperazinyl)ethyl]-5,10-dihydro-11H-dibenzo[b,e][1,4]diazepin-11-one;
3-(3-methoxy-4-nitrophenyl)-8-{2-oxo-2-[4-(2-pyridinyl)-1-piperazinyl]ethyl}-5,10-dihydro-11H-dibenzo[b,e][1,4]diazepin-11-one;
8-{2-[(2S)-2-(hydroxymethyl)-1-pyrrolidinyl]-2-oxoethyl}-3-(3-methoxy-4-nitrophenyl)-5,10-dihydro-1H-dibenzo[b,e][1,4]diazepin-11-one;
N-[3-(3-methoxy-4-nitrophenyl)-11-oxo-10,11-dihydro-5H-dibenzo[b,e][1,4]diazepin-8-yl]urea;
2-(dimethylamino)N[3-(3-methoxy-4-nitrophenyl)-11-oxo-10,11-dihydro-5H-dibenzo[b,e][1,4]diazepin-8-yl]acetamide;
(2S)-2-aminoN[3-(3-methoxy-4-nitrophenyl)-11-oxo-10,11-dihydro-5H-dibenzo[b,e][1,4]diazepin-8-yl]-4-methylpentanamide;
4-AminoN[3-(3-methoxy-4-nitrophenyl)-11-oxo-10,11-dihydro-5H-dibenzo[b,e][1,4]diazepin-8-yl]butyramide;
3-AminoN[3-(3-methoxy-4-nitropbenyl)-11-oxo-10,11-dihydro-5H-dibenzo[b,e][1,4]diazepin-8-yl]propionamide;
N-[3-(3-Methoxy-4-nitrophenyl)-11-oxo-10,11-dihydro-5H-dibenzo[b,e][1,4]diazepin-8-yl]-2-(3-methyl-3H-imidazol-4-yl)acetamide;
2-(3H-Imidazol-4-yl)N[3-(3-methoxy-4-nitrophenyl)-11-oxo-10,11-dihydro-5H-dibenzo[b,e][1,4]diazepin-8-yl]acetamide;
N-[3-(3-methoxy-4-nitrophenyl)-11-oxo-10,11-dihydro-5H-dibenzo[b,e][1,4]diazepin-8-yl]thiophene-3-carboxamide;
N-[3-(3-methoxy-4-nitrophenyl)-11-oxo-10,11-dihydro-5H-dibenzo[b,e][1,4]diazepin-8-yl]-1H-pyrroie-2-carboxamide;
N-[3-(3-methoxy-4-nitrophenyl)-11-oxo-10,11-dihydro-5H-dibenzo[b,e][1,4]diazepin-8-yl]-2,5-dimethyl-1H-pyrrole-3-carboxamide;
N-[3-(3-methoxy-4-nitrophenyl)-11-oxo-10,11-dihydro-5H-dibenzo[b,e][1,4]diazepin-8-yl]-1,3-thiazole-4-carboxamide;
N-[3-(3-methoxy-4-nitrophenyl)-11-oxo-10,11-dihydro-5H-dibenzo[b,e][1,4]diazepin-8-yl]-1H-pyrazole-5-carboxamide;
N-[3-(3-methoxy-4-nitrophenyl)-11-oxo-10,11-dihydro-5H-dibenzo[b,e][1,4]diazepin-8-yl]-1H-pyrazole-4-carboxamide;
N-[3-(3-methoxy-4-nitrophenyl)-11-oxo-10,11-dihydro-5H-dibenzo[b,e][1,4]diazepin-8-yl]isonicotinamide;
N-[3-(3-methoxy-4-nitrophenyl)-11-oxo-10,11-dihydro-5H-dibenzo[b,e][1,4]diazepin-8-yl]-3-pyrrolidin-1-ylpropanamide;
N-[3-(3-methoxy-4-nitrophenyl)-11-oxo-10,11-dihydro-5H-dibenzo[b,e][1,4]diazepin-8-yl]-3-piperidin-1-ylpropanamide;
N-[3-(3-methoxy-4-nitrophenyl)-11-oxo-10,11-dihydro-5H-dibenzo[b,e][1,4]diazepin-8-yl]-3-morpholin-4-ylpropanamide;
(2R)-2-hydroxyN[3-(3-methoxy-4-nitrophenyl)-11-oxo-10,11-dihydro-5H-dibenzo[b,e][1,4]diazepin-8-yl]-4-phenylbutanamide;
N-[3-(3-methoxy-4-nitrophenyl)-11-oxo-10,11-dihydro-5H-dibenzo[b,e][1,4]diazepin-8-yl]-3-(phenylsulfonyl)propanamide;
N-[3-(3-methoxy-4-nitrophenyl)-11-oxo-10,11-dihydro-5H-dibenzo[b,e][1,4]diazepin-8-yl]-2-{[(4-methylphenyl)sulfonyl]amino}acetamide;
N-[3-(3-methoxy-4-nitrophenyl)-11-oxo-10,11-dihydro-5H-dibenzo[b,e][1,4]diazepin-8-yl]pyridine-2-carboxamide;
N-[3-(3-methoxy-4-mirophenyl)-11-oxo-10,11-dihydro-5H-dibenzo[b,e][1,4]diazepin-8-yl]nicatinamide;
N-[3-(3-methoxy-4-nitrophenyl)-11-oxo-10,11-dihydro-5H-dibenzo[b,e][1,4]diazepin-8-yl]-2-pyridin-3-ylacetamide;
N-[3-(3-methoxy-4-nitrophenyl)-11-oxo-10,11-dihydro-5H-dibenzo[b,e][1,4]diazepin-8-yl]-2-(4-methylpiperazin-1-yl)acetamide;
3-ethoxyN[3-(3-methoxy-4-nitrophenyl)-11-oxo-10,11-dihydro-5H-dibenzo[b,e][1,4]diazepin-8-yl]propanamide;
(2R)N[3-(3-methoxy-4-nitrophenyl)-11-oxo-10,11-dihydro-5H-dibenzo[b,e][1,4]diazepin-8-yl]-5-oxopyrrolidine-2-carboxamide;
4-methoxyN[3-(3-methoxy-4-nitrophenyl)-11-oxo-10,11-dihydro-5H-dibenzo[b,e][1,4]diazepin-8-yl]cyclohexanecarboxamide;
(2R)-2-methoxyN[3-(3-methoxy-4-nitrophenyl)-11-oxo-10,11-dihydro-5H-dibenzo[b,e][1,4]diazepin-8-yl]-2-phenylacetamide;
(2S)-2-methoxyN[3-(3-methoxy-4-nitrophenyl)-11-oxo-10,11-dihydro-5H-dibenzo[b,e][1,4]diazepin-8-yl]-2-phenylacetamide;
N-(2-{[3-(3-methoxy-4-nitrophenyl)-11-oxo-10,11-dihydro-5H-dibenzo[b,e][1,4]diazepin-8-yl]amino)-2-oxoethyl)-2-furamide;
1-acetylN[3-(3-methoxy-4-nitrophenyl)-11-oxo-10,11-dihydro-5H-dibenzo[b,e][1,4]diazepin-8-yl]piperidine-4-carboxamide;
N-[3-(3-methoxy-4-nitrophenyl)-11-oxo-10,11-dihydro-5H-dibenzo[b,e][1,4]di azepin-8-yl]-N'-phenylpentanediamide;
N-[3-(3-methoxy-4-nitrophenyl)-11-oxo-10,11-dihydro-5H-dibenzo[b,e][1,4]diazepin-8-yl]-2-[4-(mnethylsulfonyl)phenyl]acetamide;
(2S)N[3-(3-methoxy-4-nitrophenyl)-11-oxo-10,11-dihydro-5H-dibenzo[b,e][1,4]diazepin-8-yl]-5-oxopyrrolidine-2-carboxamide;
4-(8-amino-11-oxo-10,11-dihydro-5H-dibenzo[b,e][1,4]diazepin-3-yl)-2-methoxybenzonitrile;
(2S)N[3-(4-cyano-3-methoxyphenyl)-11-oxo-10,11-dihydro-5H-dibenzo[b,e][1,4]diazepin-8-yl]-2-methoxy-2-phenylacetamide;
N-[3-(4-cyano-3-methoxyphenyl)-11-oxo-10,11-dihydro-5H-dibenzo[b,e][1,4]diazepin-8-yl]-3-piperidin-1-ylpropanamide;
N-[3-(4-cyano-3-methoxyphenyl)-11-oxo-10,11-diliydro-5H-dibenzo[b,e][1,4]diazepin-8-yl]methanesulfonamide;
5-amino-3-(4-chloro-3-methoxyphenyl)-5,10-dihydro-11H-dibenzo[b,e][1,4]diazepin-11-one;
N-[3-(4-chloro-3-methoxyphenyl)-11-oxo-10,11-dihydro-5H-dibenzo[b,e][1,4]diazepin-8-yl]-3-piperidin-1-ylpropanamide;
3-(3-methoxy-4-nitrophenyl)-8-(2-oxopyrrolidin-1-yl)-5,10-dihydro-11H-dibenzo[b,e][1,4]diazepin-11-one;
3-(3-methoxy-4-nitrophenyl)-8-(2-oxopiperidin-1-yl)-5,10-dihydro-11H-dibenzo[b,e][1,4]diazepin-11-one;
3-(4-chloro-3-methoxyphenyl)-8-(2-oxopyrrolidin-1-yl)-5,10-dihydro-11H-dibenzo[b,e][1,4]diazepin-11-one;
3-chloroN[3-(4-chloro-3-methoxyphenyl)-11-oxo-10,11-dihydro-5H-dibenzo[b,e][1,4]diazepin-8-yl]propane-1-sulfonamide;
N-[3-(4-chloro-3-methoxyphenyl)-11-oxo-10,11-dihydro-5H-dibenzo[b,e][1,4]diazepin-8-yl]-1-phenylmethanesulfonamide;
3-(4-chloro-3-methoxyphenyl)-8-(1,1-dioxidoisothiazolidin-2-yl)-5,10-dihydro-11H-dibenzo[b,e][1,4]diazepin-11-one;
2,2,2-trifluoroN[3-(3-methoxy-4-nitrophenyl)-11-oxo-10,11-dihydro-5H-dibenzo[b,e][1,4]diazepin-8-yl]ethanesulfonamide;
N-[3-(3-methoxy-4-nitrophenyl)-11-oxo-10,11-dihydro-5H-dibenzo[b,e][1,4]diazepin-8-yl]-1-methyl-1H-imidazole-4-sulfonamide;
N-[3-(3-methoxy-4-nitrophenyl)-11-oxo-10,11-dihydro-5H-dibenzo[b,e][1,4]diaxepin-8-yl]-1-phenylmethanesulfonamide;
3-chloroN[3-(3-methoxy-4-nitrophenyl)-11-oxo-10,11-dihydro-5H-dibenzo[b,e][1,4-]diazepin-8-yl]propane-1-sulfonamide;
8-(1,1-dioxidoisothiazolidin-2-yl)-3-(3-methoxy-4-nitrophenyl)-5,10-dihydro-11H-dibenzo[b,e][1,4]diazepin-11-one;
N-[3-(3-methoxy-4-nitrophenyl)-11-oxo-10,11-dihydro-5H-dibenzo[b,e][1,4]diazepin-8-yl]-3-morpholin-4-ylpropane-1-sulfonamide;
N-[3-(3-methoxy-4-nitrophenyl)-11-oxo-10,11-dihydro-5H-dibenzo[b,e][1,4]diazepin-8-yl]-3-piperidin-1-ylpropane-1-sulfonamide;
3-(diethylamino)N[3-(3-methoxy-4-nitrophenyl)-11-oxo-10,11-dihydro-5H-dibenzo[b,e][1,4]diazepin-8-yl]propane-1-sulfonamide;
3-(dimethylamino)N[3-(3-methoxy-4-nitrophenyl)-11-oxo-10,11-dihydro-5H-dibenzo[b,e][1,4]diazepin-8-yl]propane-1-sulfonamide;
1-chloroN[3-(3-methoxy-4-nitrophenyl)-11-oxo-10,11-dihydro-5H-dibenzo[b,e][1,4]diazepin-8-yl]methanesulfonamide;
N-[3-(3-methoxy-4-nitrophenyl)-11-oxa-10,11-dihydro-5H-dibenzo[b,e][1,4]diazepin-8-yl]-4-morpholin-4-ylbenzamide;
7-amino-3-(3-methoxy-4-nitrophenyl)-5,10-dihydro-11H-dibenzo[b,e][1,4]diazepin-11-one;
3-chloroN[3-(3-methoxy-4-nitrophenyl)-11-oxo-10,11-dihydro-5H-dibenzo[b,e][1,4]diazepin-7-yl]propane-1-sulfonamide;
N-[3-(3-methoxy-4-nitrophenyl)-11-oxo-10,11-dihydro-5H-dibenzo[b,e][1,4]diazepin-7-yl]-1-phenylmethanesulfonamide;
1-(4-chlorophenyl)N[3-(3-methoxy-4-nitrophenyl)-11-oxo-10,11-dihydro-5H-dibenzo[b,e][1,b]diazepin-7-yl]methanesulfonamide;
N-[3-(3-methoxy-4-nitrophenyl)-11-oxo-10,11-dihydro-5H-dibenzo[b,e][1,4]diazepin-7-yl]-1-methyl-1H-imidazole-4-sulfonamide;
N-[3-(3-methoxy-4-nitrophenyl)-11-oxo-10,11-dihydro-5H-dibenzo[b,e][1,4]diazepin-7-yl]methanesulfonaimde;
N-[3-(3-methoxy-4-nitrophenyl)-11-oxo-10,11-dihydro-5H-dibenzo[b,e][1,4]diazepin-7-yl]-3-morpholin-4-ylpropane-1-sulfonamide;
N-[3-(3-methoxy-4-nitrophenyl)-11-oxo-10,11-dihydro-5H-dibenzo[b,e][1,4]diazepin-7-yl]-3-piperidin-1-ylpropane-1-sulfonamide;
3-(diethylamino)N[3-(3-methoxy-4-nitrophenyl)-11-oxo-10,11-dihydro-5H-dibenzo[b,e][1,4]diazepin-7-yl]propane-1-sulfonamide;
3-(dimethylamino)N[3-(3-methoxy-4-nitrophenyl)-11-oxo-10,11-dihydro-5H-dibenzo[b,e][1,4]diazepin-7-yl]propane-1-sulfonamide;
N-[3-(3-methoxy-4-nitrophenyl)-11-oxo-10,11-dihydro-5H-dibenzo[b,e][1,4]diazepin-7-yl]tetrahydro-2H-pyran-4-carboxamide;
8-(1-hydroxy-1-methylethyl)-3-(3-methoxy-4-nitrophenyl)-5,10-dihydro-11H-dibenzo[b,e][1,4]diazepin-11-one;
8-(1-ethyl-3-hydroxypropyl)-3-(3-methoxy-4-nitrophenyl)-5,14-dihydro-11H-dibenzo[b,e][1,4]diazepin-11-one;
8-(1-hydroxy-1-methylethyl)-3-(pyridin-4-ylamino)-5,10-dihydro-11H-dibenzo[b,e][1,4]diazepin-11-one;
3-[(2-chloropyridin-4-yl)amino]-8-(1-hydroxy-1-methylethyl)-5,10-dihydro-11H-dibenzo[b,e][1,4]diazepin-11-one;
4-{[8-(1-hydroxy-1-methylethyl)-11-oxo-10,11-dihydro-5H-dibenzo[b,e][1,4]diazepin-3-yl]amino}pyridine-2-carbonitrile;
8-(1-hydroxy-1-methylethyl)-3-(pyrimidin-4-ylamino)-5,10-dihydro-11H-dibenzo[b,e][1,4]diazepin-11-one;
5-(1-hydroxy-1-methylethyl)-3-[(2,3,5,6-tetrafluoropyridin-4-yl)amino]-5,10-dihydro-11H-dibenzo[b,e][1,4]diazepin-11-one;
8-(1-ethyl-1-hydroxypropyl)-3-(pyridin-4-ylamino)-5,10-dihydro-11H-dibenzo[b,e][1,4]diazepin-11-one;
3-[(2-aminopyrimidin-4-yl)amino]-8-(1-hydroxy-1-methylethyl)-5,10-dihydro-11H-dibenzo[b,e][1,4]diazepin-11-one;
3-[(2-chloropyridin-4-yl)amino]-8-(1-ethyl-1-hydroxypropyl)-5,10-dihydro-11H-dibenzo[b,e][1,4]diazepin-11-one;
8-(1-hydroxy-1-methylethyl)-3-[(2,3,6-trifluoropyridin-4-yl)amino]-5,10-dihydro-11H-dibenzo[b,e][1,4]diazepin-11-one;
3-({2-[(2-chloropyridin-4-yl)amino]pyridin-4-yl}amino)-8-(1-hydroxy-1-methylethyl)-5,10-dihydro-11H-dibenzo[b,e][1,4]diazepin-11-one;
methyl 2-methyl-2-{11-oxo-3-[(2,3,6-trifluoropyridin-4-yl)amino]-10,11-dihydro-5H-dibenzo[b,e][1,4]diazepin-8-yl}propanoate;
2-methylN(4-morpholin-4-ylphenyl)-2-{11-oxo-3-[(2,3,6-trifluoropyridin-4-yl)amino]-10,11-dihydro-5H-dibenzo[b,e][1,4-]diazepin-8-yl}propanamide;
2-{3-[(2,6-difluoropyridin-4-yl)amino]-11-oxo-10,11-dihydro-5H-dibenzo[b,e][1,4]diazepin-8-yl}-2-methylN(4-morpholin-4-ylphenyl)propanamide;
3-[(2,6-difluoropyridin-4-yl)amino]-8-(2-hydroxyethyl)-5,10-dihydro-11H-dibenzo[b,e][1,4]diazepin-11-one;
2-{3-[(2,6-difluoropyridin-4-yl)amino]-11-oxo-10,11-dihydro-5H-dibenzo[b,e][1,4]diazepin-8-yl}-2-methylpropanoic acid;
3-[(2,6-difluoropyridin-4-yl)amino]-7-morpholin-4-yl-5,10-dihydro-11H-dibenzo[b,e][1,4]diazepin-11-one;
7-morpholin-4-yl-3-[(2,3,6-trifluoropyridin-4-yl)amino]-5,10-dihydro-11H-dibenzo[b,e][1,4]diazepin-11-one;
3-[(2,6-difluoropyridin-4-yl)amino]-8-(2-hydroxy-2-methylpropyl)-5,10-dihydro-11H-dibenzo[b,e][1,4]diazepin-11-one;
methyl [11-oxo-3-(pyridin,-4-ylamino)-10,11-dihydro-5H-dibenzo[b,e][1,4]diazepin-8-yl]acetate;
methyl {3-[(2-chloropyridin-4-yl)amino]-11-oxo-10,11-dihydro-5H-dibenzo[b,e][1,4]diazepin-8-yl}acetate;
methyl {3-[(2-methylpyridin-4-yl)amino]-11-oxo-10,11-dihydro-5H-dibenzo[b,e][1,4]diazepin-8-yl}acetate;
3-[(2-chloropyridin-4-yl)amino]-5,10-dihydro-11H-dibenzo[b,e][1,4]diazepin-11-one;
8-acetyl-3-[(2-chloropyridin-4-yl)amino]-5,10-dihydro-11H-dibenzo[b,e][1,4]diazepin-11-one;
{3-[(2-chloropyridin-4-yl)amino]-11-oxo-10,11-dihydro-5H-dibenzo[b,e][1,4]diazepin-8-yl}acetic acid;
3-[(2-chloropyridin-4-yl)amino]-8-isopropenyl-5,10-dihydro-11H-dibenzo[b,e][1,4]diazepin-11-one;
2-{3-[(2-chloropyridin-4-yl)amino]-11-oxo-10,11-dihydro-5H-dibenzo[b,e][1,4]diazepin-8-yl}N(4-morpholin-4-ylphenyl)acetamide;
3-[(2-chloropyridin-4-yl)amino]-8-(2-hydroxy-2-methylpropyl)-5,10-dihydro-11H-dibenzo[b,e][1,4]diazepin-11-one;
2-{3-[(2-chloropyridin-4-yl)amino]-11-oxo-10,11-dihydro-5H-dibenzo[b,e][1,4]diazepin-8-yl}-2-methylN(4-morpholin-4-ylphenyl)propanamide;
3-[(2-chloropyridin-4-yl)amino]-8-(2-oxopyrrolidin-1-yl)-5,10-dihydro-11H-dibenzo[b,e][1,4]diazepin-11-one;
methyl {3-[(2-chloropyridin-4-yl)amino]-11-oxo-10,11-dihydro-5H-dibenzo[b,e][1,4]diazepin-7-yl}acetate;
8-[2-(pyridin-2-yloxy)ethyl]-3-[(2,3,6-trifluoropyridin-4-yl)amino]-5,10-dihydro-11H-dibenzo[b,e][1,4]diazepin-11-one;
8-(2-hydroxy-2-methylpropyl)-3-[(2,3,5-trifluorophenyl)amino]-5,10-dihydro-11H-dibenzo[b,e][1,4]diazepin-11-one;
3-[(3,5-difluorophenyl)amino]-7-(3-hydroxy-3-methylbutyl)-8-methoxy-5,10-dihydro-11H-dibenzo[b,e][1,4]diazepin-11-one;
7-(3-hydroxy-3-methylbutyl)-8-methoxy-3-(3-methoxy-4-nitrophenyl)-5,10-dihydro-11H-dibenzo[b,e][1,4]diazepin-11-one;
3-[(2,6-difluoropyridin-4-yl)amino]-7-(3-hydroxypropyl)-8-methoxy-5,10-dihydro-11H-dibenzo[b,e][1,4]diazepin-11-one;
7-(3-hydroxypropyl)-8-methoxy-3-(3-methoxy-4-nitrophenyl)-5,10-dihydro-11H-dibenzo[b,e][1,4]diazepin-11-one;
3-[(2,6-difluoropyridin-4-yl)amino]-8-(3-hydroxy-3-methylbutyl)-5,10-dihydro-11H-dibenzo[b,e][1,4]diazepin-11-one;
8-(3-hydroxy-3-methylbutyl)-3-(3-methoxy-4-nitrophenyl)-5,10-dihydro-11H-dibenzo[b,e][1,4]diazepin-11-one;
methyl 3-[3-(3-methoxy-4-nitrophenyl)-11-oxo-10,11-dihydro-5H-dibenzo[b,e] [1,4]diazepin-8-yl]propanoate;
3-[3-(3-methoxy4-nitrophenyl)-11-oxo-10,11-dihydro-5H-dibenzo[b,e][1,4]diazepin-8-yl]propanoic acid;
3-[3-(3-methoxy-4-nitrophenyl)-11-oxo-10,11-dihydro-5H-dibenzo[b,e][1,4]diazepin-8-yl]-N,N-dimethylpropanamide;
3-[3-(3-methoxy-4-nitrophenyl)-11-oxo-10,11-dihydro-5H-dibenzo[b,e][1,4]diazepin-8-yl]N(4-morpholin-4-ylphenyl)propanamide;
8-(3-azetidin-1-yl-3-oxopropyl)-3-(3-methoxy-4-nitrophenyl)-5,10-dihydro-11H-dibenzo[b,e][1,4]diazepin-11-one;
3-(3-methoxy-4-nitrophenyl)-8-(3-oxo-3-pyrrolidin-1-ylpropyl)-5,10-dihydro-11H-dibenzo[b,e][1,4]diazepin-11-one;
3-(3-methoxy-4-nitrophenyl)-8-(3-morpholin-4-yl-3-oxopropyl)-5,10-dihydro-11H-dibenzo[b,e][1,4]diazepin-11-one;
N,N-diethyl-3-[3-(3-methoxy-4-nitrophenyl)-11-oxo-10,11-dihydro-5H-dibenzo[b,e][1,4]diazepin-8-yl]propanamide;
8-[3-(4-hydroxypiperidin-1-yl)-3-oxopropyl]-3-(3-methoxy-4-nitrophenyl)-5,10-dihydro-1 1H-dibenzo[b,e][1,4]diazepin-11-one;
3-[3-(3-methoxy-4-nitrophenyl)-11-oxo-10,11-dihydro-5H-dibenzo[b,e][1,4]diazepin-8-yl]N1,3-thiazol-2-ylpropanamide;
8-(2-hydroxyethyl)-3-(3-methoxy-4-nitrophenyl)-5,10-dihydro-11H-dibenzo[b,e][1,4]diazepin-11-one;
8-(3-hydroxypropyl)-3-(3-methoxy-4-nitrophenyl)-5,10-dihydro-11H-dibenzo[b,e][1,4]diazepan-11-one;
methyl 3-[3-(4-chloro-3-methoxyphenyl)-11-oxo-10,11-dihydro-5H-dibenzo[b,e][1,4]diazepin-8-yl]propanoate;
3-[3-(4-chloro-3-methoxyphenyl)-11-oxo-10,11-dihydro-5H-dibenzo[b,e][1,4]diazepin-8-yl]-N,N-dimethylpropanamide;
3-(4-chloro-3-methoxyphenyl)-8-(3-hydroxy-3-methylbutyl)-5,10-dihydro-11H-dibenzo[b,e][1,4]diazepin-11-one;
3-(3-methoxy-4-nitrophenyl)-8-[2-(3-methyl-1,2,4-oxadiazol-5-yl)ethyl]-5,10-dihydro-11H-dibenzo[b,e][1,4]diazepin-11-one;
methyl 3-[8-methoxy-3-(3-methoxy-4-nitrophenyl)-11-oxo-10,11-dihydro-5H-dibenzo[b,e][1,4]diazepin-7-yl]propanoate;
7-(2-hydroxy-2-methylpropyl)-8-methoxy-3-(3-methoxy-4-nitrophenyl)-5,10-dihydro-11H-dibenzo[b,e][1,4]diazepin-11-one;
7-(2-hydroxyethyl)-8-methoxy-3-(3-methoxy-4-nitrophenyl)-5,10-dihydro-11H-dibenzo[b,e][1,4]diazepin-11-one;
8-methoxy-3-(3-methoxy-4-nitrophenyl)-7-(2-oxopropyl)-5,10-dihydro-11H-dibenzo[b,e][1,4]diazepin-11-one;
7-(2-hydroxy-11-dimethylethyl)-8-methoxy-3-(3-methoxy-4-nitrophenyl)-5,10-dihydro-1 1H-dibenzo[b,e][1,4]diazepin-11-one;
7-(3-hydroxypropyl)-3-(3-methoxy-4-nitrophenyl)-8-(trifluoromethoxy)-5,10-dihydro-11H-dibenzo[b,e][1,4]diazepin-11-one;
7-(3-hydroxy-3-methylbutyl)-3-(3-methoxy-4-nitrophenyl)-8-(trifluoromethoxy)-5,10-dihydro-11H-dibenzo[b,e][1,4]diazepin-11-one;
7-(3-hydroxy-3-methylbutyl)-3-(3-methoxy-4-nitrophenyl)-8-methyl-5,10-dihydro-11H-dibenzo[b,e][1,4]diazepin-11-one;
3-(3-methoxy-4-nitrophenyl)-8-(2-pyridin-4-ylethyl)-5,10-dihydro-11H-dibenzo[b,e][1,4]diazepin-11-one;
3-(3-methoxy-4-nitrophenyl)-8-(2-pyridin-2-ylethyl)-5,10-dihydro-11H-dibenzo[b,e][1,4]diazepin-11-one;
3-(3-methoxy-4-nitrophenyl)-8-[2-(2-oxopyridin-1(2H)-yl)methyl]-5,10-dihydro-11H-dibenzo[b,e][1,4]diazepin-11-one;
8-[2-(5-fluoro-2-oxopyridin-1(2H)-yl)ethyl]-3-(3-methoxy-4-nitrophenyl)-5,10-dihydro-1 1H-dibenzo[b,e][1,4]diazepin-11-one;
3-(3-methoxy-4-nitrophenyl)-8-[2-(6-oxopyridazin-1(6H)-yl)ethyl]-5,10-dihydro-11H-dibenzo[b,e][1,4]diazepin-11-one;
3-(3-methoxy-4-nitrophenyl)-8-[2-(pyridin-2-yloxy)ethyl]-5,10-dihydro-11H-dibenzo[b,e][1,4]diazepin-11-one;
8-{2-[(5-chloropyridin-3-yl)oxy]ethyl}-3-(3-methoxy-4-nitrophenyl)-5,10-dihydro-11H-dibenzo[b,e][1,4]diazepin-11-one;
8-methoxy-3-(3-methoxy-4-nitrophenyl)-7-[2-(pyridin-3-yloxy)ethyl]-5,10-dihydro-11H-dibenzo[b,e][1,4]diazepin-11-one;
methyl (3-isoquinolin-5-yl-11-oxo-10,11-dihydro-5H-dibenzo[b,e][1,4]diazepin-8-yl)acetate;
methyl [3-(8-nitroisoquinolin-5-yl)-11-oxo-10,11-dihydro-5H-dibenzo[b,e][1,4]diazepin-8-yl]acetate;
methyl 3-[3-(4-formyl-3-methoxyphenyl)-11-oxo-10,11-dihydro-5H-dibenzo[b,e][1,4]diazepin-8-yl]propanoate;
methyl 3-{3-[4-(hydroxymethyl)-3-methoxyphenyl]-11-oxo-10,11-dihydro-5H-dibenzo[b,e][1,4]diazepin-8-yl}propanoate;
methyl 3-[3-(3-methoxy-4-propionylphenyl)-11-oxo-10,11-dihydro-5H-dibenzo[b,e][1,4]diazepin-8-yl]propanoate;
2-[3-(3-methoxy-4-nitrophenyl)-11-oxo-10,11-dihydro-5H-dibenzo[b,e][1,4]diazepin-8-yl]-2-methylN(4-morpholin-4-ylphenyl)propanamide;
2-[3-(4-cyano-3-methoxyphenyl)-11-oxo-10,11-dihydro-5H-dibenzo[b,e][1,4]diazepin-8-yl]-2-methylN(4-morpholin-4-ylphenyl)propanamide;
2-[3-(4-chloro-3-methoxyphenyl)-11-oxo-10,11-dihydro-5H-dibenzo[b,e][1,4]diazepin-8-yl]-2-methylN(4-morpholin-4-ylphenyl)propanamide;
2-[3-(3-methoxy-4-nitrophenyl)-11-oxo-10,11-dihydro-5H-dibenzo[b,e][1,4]diazepin-8-yl]-2-methylNpyridin-2-ylpropanamide;
2-[3-(3-methoxy-4-nitrophenyl)-11-oxo-10,11-dihydro-5H-dibenzo[b,e][1,4]diazepin-8-yl]-2-methylNpyridin-3-ylpropanamide;
2-[3-(3-methoxy-4-nitrophenyl)-11-oxo-10,11-dihydro-5H-dibenzo[b,e][1,4]diazepin-8-yl]-2-methylNpyridin-4-ylpropanamide;
2-[3-(3-methoxy-4-nitrophenyl)-11-oxo-10,11-dihydro-5H-dibenzo[b,e][1,4]diazepin-8-yl]-2-methylN(pyridin-3-ylmethyl)propanamide;
2-[3-(3-methoxy-4-nitrophenyl)-11-oxo-10,11-dihydro-5H-dibenzo[b,e][1,4]diazepin-8-yl]-2-methylN(pyridin-3-ylmethyl)propanamide;
2-[3-(3-methoxy-4-nitrophenyl)-11-oxo-10,11-dihydro-5H-dibenzo[b,e][1,4]diazepin-8-yl]-2-methylN(tetrahydrofuran-3-ylmethyl)propanamide;
2-[3-(3-methoxy-4-nitrophenyl)-11-oxo-10,11-dihydro-5H-dibenzo[b,e][1,4]diazepin-8-yl]-2-methylN1,3-thiazol-2-ylpropanamide;
2-[3-(3-methoxy-4-nitrophenyl)-11-oxo-10,11-dihydro-5H-dibenzo[b,e][1,4]diazepin-8-yl]-2-methylN{[-(trifluoromethyl)pyridin-3-yl]methyl}propanamide;
N-(4-fluorophenyl)-2-[3-(3-methoxy-4-nitrophenyl)-11-oxo-10,11-dihydro-5H-dibenzo[b,e][1,4]diazepin-8-yl]-8-methylpropanamide;
N-(2,5-difluorobenzyl)-2-[3-(3-methoxy-4-nitrophenyl)-11-oxo-10,11-dihydro-5H-dibenzo[b,e][1,4]diazepin-8-yl]-2-methylpropanamide;
2-methyl-2-[11-oxo-3-(pyrimidin-4-ylamino)-10,11-dihydro-5H-dibenzo[b,e][1,4]diazepin-8-yl]N[3-(trifluororomethyl)benzyl]propanamide;
2-methyl-2-[11-oxo-3-(pyrimidin-4-ylamino)-10,11-dihydro-5H-dibenzo[b,e][1,4]diazepin-8-yl]N[3-(trifluoromethoxy)benzyl]propanamide;
1-[3-(3-methoxy-4-nitrophenyl)-11-oxo-10,11-dihydro-5H-dibenzo[b,e][1,4]diazepin-8-yl]N(4-morpholin-4-ylphenyl)cyclopropanecarboxamide;
2-[3-(3-methoxy-4-nitrophenyl)-11-oxo-10,11-dihydro-5H-dibenzo[b,e][1,4]diazepin-7-yl]-2-methylN(4-morpholin-4-ylphenyl)propanamide;
2-[3-(3-methoxy-4-nitrophenyl)-11-oxo-10,11-diliydro-5H-dibenzo[b,e][1,4]diazepin-7-yl]-2-methylN(pyridin-2-ylmethyl)propanamide;
2-[3-(3-methoxy-4-nitrophenyl)-11-oxo-10,11-dihydro-5H-dibenzo[b,e][1,4]diazepin-7-yl]-2-methylN(thien-2-ylmethyl)propanamide;
N-(cyclopropylmethyl)-2-[3-(3-methoxy-4-nitrophenyl)-11-oxo-10,11-dihydro-5H-dibenzo[b,e][1,4]diazepin-7-yl]-2-methylpropanamide;
7-(3-hydroxypropyl)-3-(3-methoxy-4-nitrophenyl)-5,10-dihydro-11H-dibenzo[b,e][1,4]diazepin-11-one;
7-(3-hydroxy-3-methylbutyl)-3-(3-methoxy-4-nitrophenyl)-5,10-dihydro-11H-dibenzo[b,e][1,4]diazepin-11-one;
8-(2-hydroxy-1,1-dimethylethyl)-3-(3-methoxy-4-nitrophenyl)-5,10-dihydro-11H-dibenzo[b,e][1,4]diazepin-11-one;
8-(2-hydroxy-1,1,2-trimethylpropyl)-3-(3-methoxy-4-nitrophenyl)-5,10-dihydro-11H-dibenzo[b,e][1,4]diazepin-11-one;
8-(1,1-dimethyl-2-oxopropyl)-3-(3-methoxy-4-nitrophenyl)-5,10-dihydro-11H-dibenzo[b,e][1,4]diazepin-11-one;
7-(2-hydroxy-1,1-dimethylethyl)-3-(3-methoxy-4-nitrophenyl)-5,10-dihydro-11H-dibenzo[b,e][1,4]diazepin-11-one;
8-[1-(hydroxymethyl)cyclopropyl]-3-(3-methoxy-4-nitrophenyl)-5,10-dihydro-11H-dibenzo[b,e][1,4]diazepin-11-one;
3-[(2-chloropyridin-4-yl)amino]-8-(2-hydroxy-1,1-dimethylethyl)-5,10-dihydro-11H-dibenzo[b,e][1,4]diazepin-11-one;
3-[(2,6-difluoropyridin-4-yl)amino]-8-(2-hydroxy-1,1-dimethylethyl)-5,10-dihydro-11H-dibenzo[b,e][1,4]diazepin-11-one;
3-[(2,6-difluoropyridin-4-yl)amino]-8-(2-hydroxy-1,1,2-trimethylpropyl)-5,10-dihydro-11H-dibenzo[b,e][1,4]diazepin-11-one;
3-(4-chloro-3-methoxyphenyl)-8-(2-hydroxy-1,1-dimethylethyl)-5,10-dihydro-11H-dibenzo[b,e][1,4]diazepin-11-one;
3-(3-methoxy-4-nitrophenyl)-8-[2-(4-morpholin-4-ylphenoxy)ethyl]-5,10-dihydro-11H-dibenzo[b,e][1,4]diazepin-11-one;
3-(4-chloro-3-methoxyphenyl)-8-[2-(4-morpholin-4-ylphenoxy)ethyl]-5,10-dihydro-11H-dibenzo[b,e][1,4]diazepin-11-one;
2-methoxy-4-{8-[2-(4-morpholin-4-ylphenoxy)ethyl]-11-oxo-10,11-dihydro-5H-dibenzo[b,e][1,4]diazepin-3-yl}benzonitrile;
3-(3-methoxy-4-nitrophenyl)-8-{2-[(4-morpholin-4-ylphenyl)amino]ethyl}-5,10-dihydro-11H-dibenzo[b,e][1,4]diazepin-11-one;
3-(3-methoxy-4-nitrophenyl)-8-[2-(3-methyl-2-oxopyridin-1(2H)-yl)ethyl]-5,10-dihydro-11H-dibenzo[b,e][1,4]diazepin-11-one;
3-(3-methoxy-4-nitrophenyl)-8-{2-[(5-methylpyridin-2-yl)oxy]ethyl}-5,10-dihydro-11H-dibenzo[b,e][1,4]diazepin-11-one;
3-(3-methoxy-4-nitrophenyl)-8-[2-(4-methyl-2-oxopyridin-1(2H)-yl)ethyl]-5,10-dihydro-11H-dibenzo[b,e][1,4]diazepin-11-one;
8-[2-(isoquinolin-3-yloxy)ethyl]-3-(3-methoxy-4-nitrophenyl)-5,10-dihydro-11H-dibenzo[b,e][1,4]diazepin-11-one;
8-[2-(5-chloro-2-oxopyridin-1(2)-yl)ethyl]-3-(3-methoxy-4-nitrophenyl)-5,10-dihydro-11H-dibenzo[b,e][1,4]diazepin-11-one;
8-[1,1-dimethyl-2-(pyridin-2-yloxy)ethyl]-3-(3-methoxy-4-nitrophenyl)-5,10-dihydro-11H-dibenzo[b,e][1,4]diazepin-11-one;
8-[1,1-dimethyl-2-(4-morpholin-4-ylphenoxy)ethyl]-3-(3-methoxy-4-nitrophenyl)-5,10-dihydro-11H-dibenzo[b,e][1,4]diazepin-11-one;
2-[3-(3-methoxy-4-nitrophenyl)-11l-oxo-10,11-dihydro-5H-dibenzo[b,e][1,4]diazepin-8-yl]-2-methylN(tetrahydrofuran-2-ylmethyl)propanamide;
8-(2-hydroxy-11-dimethylethyl)-3-(3-methoxy-4-nitrophenyl)-7-methyl-5,10-dihydro-11H-dibenzo[b,e][1,4]diazepin-11-one;
2-[3-(3-methoxy-4-nitrophenyl)-2-methyl-11-oxo-10,11-dihydro-5H-dibenzo[b,e][1,4]diazepin-8-yl]N(4-morpholin-4-ylphenyl)acetamide;
methyl {3-[4-(aminomethyl)-3-methoxyphenyl]-11-oxo-10,11-dihydro-5H-dibenzo[b,e][1,4]diazepin-8-yl}acetate;
2-[3-(2-methoxy-5-methylpyridin-4-yl)-11-oxo-10,11-dihydro-5H-dibenzo[b,e][1,4]diazepin-8-yl]-2-methylN(4-morpholin-4-ylphenyl)propanamide;
8-(2-hydroxy-1,1-dimethylethyl)-3-[(2-methylpyridin-4-yl)amino]-5,10-dihydro-11H-dibenzo[b,e][1,4]diazepin-11-one;
8-(2-hydroxy-1,1,2-trimethylpropyl)-3-(pyrimidin-4-ylamino)-5,10-dihydro-11H-dibenzo[b,e] [1,4]diazepin-11-one;
2-[3-(3-methoxy-4-nitrophenyl)-11-oxo-10,11-dihydro-5H-dibenzo[b,e][1,4]diazepin-8-yl]NmethylN(3-pyrrolidin-1-ylpropyl)acetamide;
2-[3-(3-methoxy-4-nitrophenyl)-11-oxo-10,11-dihydro-5H-dibenzo[b,e][1,4]diazepin-8-yl]-2-methylN(3-pyrrolidin-1-ylpropyl)propanamide;
2-[3-(3-methoxy-4-nitrophenyl)-11-oxo-10,11-dihydro-5H-dibenzo[b,e][1,4]diazepin-8-yl]-2-methylNpyrimidin-4-ylpropanamide;
2-[3-(3-methoxy-4-nitrophenyl)-11-oxo-10,11-dihydro-5H-dibenzo[b,e][1,4]diazepin-8-yl]-2-methylN(4-methyl-1,3-thiazol-2-yl)propanamide;
2-[3-(3-methoxy-4-nitrophenyl)-11-oxo-10,11-dihydro-5H-dibenzo[b,e][1,4]diazepin-8-yl]-2-methylN(2,2,2-trifluoroethyl)propanamide;
N-(3-fluorophenyl)-2-[3-(3-methoxy-4-nitrophenyl)-11-oxo-10,11-dihydro-5H-dibenzo[b,e][1,4]diazepin-8-yl]-2-methylpropanamide;
2-[3-(3-methoxy-4-nitrophenyl)-11-oxo-10,11-dihydro-5H-dibenzo[b,e][1,4]diazepin-8-yl]-2-methylN[4-(trifluoromethoxy)phenyl]propanamide;
2-[3-(3-methoxy-4-nitrophenyl)-11-oxo-10,11-dihydro-5H-dibenzo[b,e][1,4]diazepin-8-yl]-2-methylN[3-(trifluoromethyl)phenyl]propanamide;
2-[3-(3-methoxy-4-nitrophenyl)-11-oxo-10,11-dihydro-5H-dibenzo[b,e][1,4]diazepin-8-yl]-2-methylN[3-(trifluoromethoxy)phenyl]propanamide;
N-[3-fluoro-5-(trifluoromethyl)benzyl]-2-[3-(3-methoxy-4-nitrophenyl)-11-oxo-10,11-dihydro-5H-dibenzo[b,e][1,4]diazepin-8-yl]-2-methylpropanamide;
N-(2-fluorobenzyl)-2-[3-(3-methoxy-4-nitrophenyl)-11-oxo-10,11-dihydro-5H-dibenzo[b,e][1,4]diazepin-8-yl]-2-methylpropanamide;
N-(3-fluorobenzyl)-2-[3-(3-methoxy-4-nitrophenyl)-11-oxo-10,11-dihydro-5H-dibenzo[b,e][1,4]diazepin-8-yl]-2-methylpropanamide;
N-(4-fluorobenzyl)-2-[3-(3-methoxy-4-nitrophenyl)-11-oxo-10,11-dihydro-5H-dibenzo[b,e][1,4]diazepin-8-yl]-2-methylpropanamide;
2-[3-(3-methoxy-4-nitrophenyl)-11-oxo-10,11-dihydro-5H-dibenzo[b,e][1,4]diazepin-8-yl]-2-methylN[4-(trifluoromethoxy)benzyl]propanamide;
2-[3-(3-methoxy-4-ntrophenyl)-11-oxo-10,11-dihydro-5H-dibenzo[b,e][1,4]diazepin-8-yl]-2-methylN[3-(trifluoromethyl)benzyl]propanamide;
2-[3-(3-methoxy-4-nitrophenyl)-11-oxo-10,11-dihydro-5H-dibenzo[b,e][1,4]diazepin-8-yl]-2-methylN[4-(trifluoromethyl)benzyl]propanamide;
2-[3-(3-methoxy-4-nitrophenyl)-11-oxo-10,11-dihydro-5H-dibenzo[b,e][1,4]diazepin-8-yl]-2-methylN[3-(trifluoromethoxy)benzyl]propanamide;
N-[2-(2-fluorophenyl)ethyl]-2-[3-(3-methoxy-4-nitrophenyl)-11-oxo-10,11-dihydro-5H-dibenzo[b,e][1,4]diazepin-8-yl]-2-methylpropanamide;
N-[2-(3-fluorophenyl)ethyl]-2-[3-(3-methoxy-4-nitrophenyl)-11-oxo-10,11-dihydro-5H-dibenzo[b,e][1,4]diazepin-8-yl]-2-methylpropanamide;
N-(2,4-difluorobenzyl)-2-[3-(3-methoxy-4-nitrophenyl)-11-oxo-10,11-dihydro-5H-dibenzo[b,e][1,4]diazepin-8-yl]-2-methylpropanamide;
N-(2,6-difluorobenzyl)-2-[3-(3-methoxy-4-nitrophenyl)-11-oxo-10,11-dihydro-5H-dibenzo[b,e][1,4]diazepin-8-yl]-2-methylpropanamide;
N-(cyclopropylmethyl)-2-[3-(3-methoxy-4-nitrophenyl)-11-oxo-10,11-dihydro-5H-dibenzo[b,e][1,4]diazepin-8-yl]-2-methylpropanamide;
N-(3-ethoxypropyl)-2-methyl-2-[11-oxo-3-(pyrimidin-4-ylamino)-10,11-dihydro-5H-dibenzo[b,e][1,4]diazepin-8-yl]propanamide;
N-(3,4-difluorobenzyl)-2-methyl-2-[11-oxo-3-(pyrimidin-4-ylamino)-10,11-dihydro-5H-dibenzo[b,e][1,4]diazepin-8-yl]propanamide;
8-[1,1-dimethyl-2-oxo-2-(4-phenylpiperazin-1-yl)ethyl]-3-(pyrimidin-4-ylamino)-5,10-dihydro-11H-dibenzo[b,e][1,4]diazepin-11-one;
N-cyclopentyl-2-methyl-2-[11-oxo-3-(pyrimidin-4-ylamino)-10,11-dihydro-5H-dibenzo[b,e][1,4]diazepin-8-yl]propanamide;
8-(1,1-dimethyl-2-morpholin-4-yl-2-oxoethyl)-3-(pyrimidin-4-ylamino)-5,10-dihydro-11H-dibenzo[b,e][1,4]diazepin-11-one;
2-methyl-2-[11-oxo-3-(pyrimidin-4-ylamino)-10,11-dihydro-5H-dibenzo[b,e][1,4]diazepin-8-yl]N(tetrahydrofuran-3-ylmethyl)propanamide;
N-(cyclopentylmethyl)-2-methyl-2-[11-oxo-3-(pyrimidin-4-ylamino)-10,11-dihydro-5H-dibenzo[b,e][1,4]diazepin-8-yl]propanamide;
N-(cyclopropylmethyl)-2-methyl-2-[11-oxo-3-(pyrimidin-4-ylamino)-10,11-dihydro-5H-dibenzo[b,e][1,4]diazepin-8-yl]propanamide;
2-methyl-2-[11-oxo-3-(pyrimidin-4-ylamino)-10,11-dihydro-5H-dibenzo[b,e][1,4]diazepin-8-yl]N(tetrahydrofuran-2-ylmethyl)propanamide;
2-[3-(3-methoxy-4-nitrophenyl)-11-oxo-10,11-dihydro-5H-dibenzo[b,e][1,4]diazepin-7-yl]-2-methyl1N1,3-thiazol-2-ylpropanamide;
N-(2,5-difluorobenzyl)-2-[3-(3-methoxy-4-nitrophenyl)-1-oxo-10,11-dihydro-5H-dibenzo[b,e][1,4]diazepin-7-yl]-2-methylpropanamide;
N-(2-ethoxyethyl)-2-[3-(3-methoxy-4-nitrophenyl)-11-oxo-10,11-dihydro-5H-dibenzo[b,e][1,4]diazepin-7-yl]-2-methylpropanamide;
N-(4-fluorophenyl)-2-[3-(3-methoxy-4-nitrophenyl)-11-oxo-10,11-dihydro-5H-dibenzo[b,e][1,4]diazepin-7-yl]-2-methylpropanamide;
3-(3-methoxy-4-nitrophenyl)-8-[2-(quinolin-2-yloxy)ethyl]-5,10-dihydro-11H-dibenzo[b,e][1,4]diazepin-11-one;
3-(3-methoxy-4-nitrophenyl)-8-[2-(5-methyl-2-oxopyridin-1(2H)-yl)ethyl]-5,10-dihydro-11H-dibenzo[b,e][1,4]diazepin-11-one;
2-{2-[3-(3-methoxy-4-nitrophenyl)-11-oxo-10,11-dihydro-5H-dibenzo[b,e][1,4]diazepin-8-yl]ethoxy}-6-methylnicotinonitrile;
3-(3-methoxy-4-nitrophenyl)-8-[2-(1-oxoisoquinolin-2(1H)-yl)ethyl]-5,10-dihydro-11H-dibenzo[b,e][1,4]diazepin-1,1-one;
3-(3-methoxy-4-nitrophenyl)-8-{2-[2-oxo-5-(trifluoromethyl)pyridin-1(2H)-yl]ethyl}-5,10-dihydro-11H-dibenzo[b,e][1,4]diazepin-11-one;
3-(3-methoxy-4--nitrophenyl)-8-[2-(3-methoxy-2-oxopyridin-1(2H)-yl)ethyl]-5,10-dihydro-11H-dibenzo[b,e][1,4]diazepin-11-one;
3-(3-methoxy-4-nitrophenyl)-8-{2-[(4-methylpyridin-2-yl)oxy]ethyl}-5,10-dihydro-11H-dibenzo[b,e][1,4]diazepin-11-one;
3-(3-methoxy-4-nitrophenyl)-8-{2-[(3-methoxypyridin-2-yl)oxy]ethyl}-5,10-dihydro-11H-dibenzo[b,e][1,4]diazepin-11-one;
3-(3-methoxy-4-nitrophenyl)-8-[2-(3-oxoisoquinolin-2(3H)-yl)ethyl]-5,10-dihydro-11H-dibenzo[b,e][1,4]diazepin-11-one;
8-{2-[(6-chloropyridin-2-yl)oxy]ethyl}-3-(3-methoxy-4-nitrophenyl)-5,10-dihydro-11H-dibenzo[b,e][1,4]diazepin-11-one;
8-{2-[(5-chloropyridin-2-yl)oxy]ethyl}-3-(3-methoxy-4-nitrophenyl)-5,10-dihydro-11H-dibenzo[b,e][1,4]diazepin-11-one;
[[3-(4-chloro-3-methoxyphenyl)-11-oxo-10,11-dihydro-5H-dibenzo[b,e][1,4]diazepin-8-yl]acetic acid;
methyl [3-(4-acetyl-3-methoxyphenyl)-11-oxo-10,11-dihydro-5H-dibenzo[b,e][1,4]diazepin-8-yl]acetate;
[3-(4-acetyl-3-methoxyphenyl)-11-oxo-10,11-dihydro-5H-dibenzo[b,e][1,4]diazepin-8-yl]acetic acid;
2-[3-(4-chloro-3-methoxyphenyl)-11-oxo-10,11-dihydro-5H-dibenzo[b,e][1,4]diazepin-8-yl]-N,N-dimethylacetamide;
tert-butyl 1-{[3-(3-methoxy-4-nitrophenyl)-11-oxo-10,11-dihydro-5H-dibenzo[b,e][1,4]diazepin-8-yl]acetyl}pyrrolidin-3-ylcarbamate;
8-[2-(3-aminopyrrolidin-1-yl)-2-oxoethyl]-3-(3-methoxy-4-nitrophenyl)-5,10-dihydro-11H-dibenzo[b,e][1,4]diazepin-11-one;
[3-(2-methoxy-5-methylpyridin-4-yl)-11-oxo-10,11-dihydro-5H-dibenxo[b,e][1,4]diazepin-8-yl]acetic acid;
2-[3-(2-methoxy-5-methylpyridin-4-yl)-11-oxo-10,11-dihydro-5H-dibenzo[b,e][1,4]diazepin-8-yl]-N,N-dimethylacetamide;
8-{2-[(2S)-2-(hydroxymethyl)pyrrolidin-1-yl]-2-oxoethyl}-3-(2-methoxy-5-methylpyridin-4-yl)-5,10-dihydro-11H-dibenzo[b,e][1,4]diazepin-11-one;
tert-butyl 1-{[3-(2-methoxy-5-methylpyridin-4-yl)-11-oxo-10,11-dihydro-5H-dibenzo[b,e][1,4]diazepin-8-yl]acetyl}pyrrolidin-3-ylcarbamate;
N-{2-[4-(aminosulfonyl)phenyl]ethyl}-2-[3-(4-chloro-3-methoxyphenyl)-11-oxo-10,11-dihydro-5H-dibenzo[b,e][1,4]diazepin-8-yl]acetamide;
tert-butyl 1-{[3-(4-chloro-3-methoxyphenyl)-11-oxo-10,11-dihydro-5H-dibenzo[b,e][1,4]diazepin-8-yl]acetyl}pyrrolidin-3-ylcarbamate;
3-(4-chloro-3-methoxyphenyl)-8-[2-(3-hydroxypiperidin-1-yl)-2-oxoethyl]-5,10-dihydro-11H-dibenzo[b,e][1,4]diazepin-11-one;
3-(4-chloro-3-methoxyphenyl)-8-{2-[(2S)-2-(hydroxymethyl)pyrrolidin-1-yl]-2-oxoethyl}-5,10-dihydro-11H-dibenzo[b,e][1,4]diazepin-11-one;
2-[3-(4-chloro-3-methoxyphenyl)-11-oxo-10,11-dihydro-5H-dibenzo[b,e][1,4]diazepin-8-yl]N(4-morpholin-4-ylphenyl)acetamide;
tert-butyl 4-{[3-(3-methoxy-4-nitrophenyl)-11-oxo-10,11-dihydro-5H-dibenzo[b,e][1,4]diazepin-8-yl]acetyl}piperazine-1-carboxylate;
3-(3-methoxy-4-nitrophenyl)-8-(2-oxo-2-piperazin-1-ylethyl)-5,10-dihydro-11H-dibenzo[b,e][1,4]diazepin-11-one;
[3-(4-cyano-3-methoxyphenyl)-11-oxo-10,11-dihydro-5H-dibenzo[b,e][1,4]diazepin-8-yl]acetic acid;
2-[3-(4-cyano-3-methoxyphenyl)-11-oxo-10,11-dihydro-5H-dibenzo[b,e][1,4]diazepin-8-yl]N(4-morpholin-4-ylphenyl)acetamide;
2-[3-(3-methoxy-4-nitrophenyl)-11-oxo-10,11-dihydro-5H-dibeno[b,e][1,4]diazepin-8-yl]Nmethylacetamide;
3-(3-methoxy-4-nitrophenyl)-8-(2-morpholin-4-yl-2-oxoethyl)-5,10-dihydro-11H-dibenzo[b,e][1,4]diazepin-11-one;
3-(2-methoxy-5-methylpyridin-4-yl)-8-(2-morpholin-4-yl-2-oxoethyl)-5,10-dihydro-11H-dibenzo[b,e][1,4]diazepin-11-one;
[3-(2-fluoropyridin-4-yl)-11-oxo-10,11-dihydro-5H-dibenzo[b,e][1,4]diazepin-8-yl]acetic acid;
N,N-dimethyl-2-[11-oxo-3-(2-oxo-1,2-dihydropyridin-4-yl)-10,11-dihydro-5H-dibenzo[b,e][1,4]diazepin-8-yl]acetamide;
8-(2-morpholin-4-yl-2-oxoethyl)-3-(2-oxo-1,2-dihydropyridin-4-yl)-5,10-dihydro-11H-dibenzo[b,e][1,4]diazepin-11-one;
N-(4-morpholin-4-ylphenyl)-2-[11-oxo-3-(2-oxo-1,2-dihydropyridin-4-yl)-10,11-dihydro-5H-dibenzo[b,e][1,4]diazepin-8-yl]acetamide;
methyl [11-oxo-3-(2-oxo-1,2-dihydropyridin-4-yl)-10,11-dihydro-5H-dibenzo[b,e][1,4]diazepin-8-yl]acetate;
methyl [3-(3-methoxy-4-nitrophenyl)-11-oxo-10,11-dihydro-5H-dibenzo[b,e][1,4]diazepin-7-yl]acetate;
[3-(3-methoxy-4-nitrophenyl)-11-oxo-10,11-dihydro-5H-dibenzo[b,e][1,4]diazepin-7-yl]acetic acid;
3-(3-methoxy-4-nitrophenyl)-7-(2-morpholin-4-yl-2-oxoethyl)-5,10-dihydro-11H-dibenzo[b,e][1,4]diazepin-11-one;
2-[3-(3-methoxy-4-nitrophenyl)-11-oxo-10,11-dihydro-5H-dibenzo[b,e][1,4]diazepin-7-yl]Npyridin-2-ylacetamide;
2-[3-(3-methoxy-4-nitrophenyl)-11-oxo-10,11-dihydro-5H-dibenzo[b,e][1,4]diazepin-7-yl]Npyridin-3-ylacetamide;
2-[3-(3-methoxy-4-nitrophenyl)-11-oxo-10,11-dihydro-5H-dibenzo[b,e][1,4]diazepin-7-yl]Npyridin-4-ylacetamide;
7-[2-(4-hydroxypiperidin-1-yl)-2-oxoethyl]-3-(3-methoxy-4-nitrophenyl)-5,10-dihydro-11H-dibenzo[b,e][1,4]diazepin-11-one;
7-[2-(3-hydroxypiperidin-1-yl)-2-oxoethyl]-3-(3-methoxy-4-nitrophenyl)-5,10-dihydro-11H-dibenzo[b,e][1,4]diazepin-11-one;
2-[3-(3-methoxy-4-nitrophenyl)-11-oxo-10,11-dihydro-5H-dibenzo[b,e][1,4]diazepin-7-yl]N(pyridin-3-ylmethyl)acetamide;
2-[3-(3-methoxy-4-nitrophenyl)-11-oxo-10,11-dihydro-5H-dibenzo[b,e][1,4]diazepin-7-yl]N(pyridin-4-ylmethyl)acetamide;
2-[3-(3-methoxy-4-nitrophenyl)-11-oxo-10,11-dihydro-5H-dibenzo[b,e][1,4]diazepin-7-yl]N(pyridin-2-ylmethyl)acetamide;
7-(2-azetidin-1-yl-2-oxoethyl)-3-(3-methoxy-4-nitrophenyl)-5,10-dihydro-11H-dibenzo[b,e][1,4]diazepin-11-one;
1-{[3-(3-methoxy-4-nitrophenyl)-11-oxo-10,11-dihydro-5H-dibenzo[b,e][1,4]diazepin-7-yl]acetyl}piperidine-3-carboxamide;
1-{[3-(3-methoxy-4-nitrophenyl)-11-oxo-10,11-dihydro-5H-dibenzo[b,e][1,4]diazepin-7-yl] acetyl}piperidine4-carboxamide;
7-{2-[(2R)-2-(hydroxymethyl)pyrrolidin-1-yl]-2-oxoethyl)-3-(3-methoxy-4-nitrophenyl)-5,10-dihydro-11H-dibenzo[b,e][1,4]diazepin-11-one;
2-[3-(3-methoxy-4-nitrophenyl)-11-oxo-10,11-dihydro-5H-dibenzo[b,e][1,4]diazepin-7-yl]-N,N-dimethylacetamide;
N,N-'bis(2-methoxyethyl)-2-[3-(3-methoxy-4-nitrophenyl)-11-oxo-10,11-dihydro-5H-dibenzo[b,e][1,4]diazepin-7-yl]acetamide;
2-[3-(3-methoxy-4-nitrophenyl)-11-oxo-10,11-dihydro-5H-dibenzo[b,e][1,4]diazepin-7-yl]N[(2S)-tetrahydrofuran-2-ylmethyl]acetamide;
2-[3-(3-methoxy-4-nitrophenyl)-11-oxo-10,11-dihydro-5H-dibenzo[b,e][1,4]diazepin-7-yl]N(2-propoxyethyl)acetamide;
7-{2-[(2S)-2-(hydroxymethyl)pyrrolidin-1-yl]-2-oxoethyl}-3-(3-methoxy-4-nitrophenyl)-5,10-dihydro-11H-dibenzo[b,e][1,4]diazepin-11-one;
7-[2-(3-aminopyrrolidin-1-yl)-2-oxoethyl]-3-(3-methoxy-4-nitrophenyl)-5,10-dihydro-11H-dibenzo[b,e][1,4]diazepin-11-one;
3-(3-methoxy-4-nitrophenyl)-7-(2-oxo-2-piperazin-1-ylethyl)-5,10-dihydro-11H-dibenzo[b,e][1,4]diazepin-11-one;
2-[3-(3-methoxy-4-nitrophenyl)-11-oxo-10,11-dihydro-5H-dibenzo[b,e][1,4]diazepin-7-yl]N(3-methoxypropyl)acetamide;
N-(cyanomethyl)-2-[3-(3-methoxy-4-nitrophenyl)-11-oxo-10,11-dihydro-5H-dibenzo[b,e][1,4]diazepin-7-yl]acetamide;
N-(cyclopropylmethyl)-2-[3-(3-methoxy-4-nitrophenyl)-11-oxo-10,11-dihydro-5H-dibenzo[b,e][1,4]diazepin-7-yl]acetamide;
N-(1,3-dioxolan-2-ylmethyl)-2-[3-(3-methoxy-4-nitrophenyl)-11-oxo-10,11-dihydro-5H-dibenzo[b,e][1,4]diazepin-7-yl]Nmethylacetamide;
3-(3-methoxy-4-nitrophenyl)-7-(2-oxo-2-thiomorpholin-4-ylethyl)-5,10-dihydro-11H-dibenzo[b,e][1,4]diazepin-11-one;
2-[3-(3-methoxy-4-nitrophenyl)-11-oxo-10,11-dihydro-5H-dibenzo[b,e][1,4]diazepin-7-yl]N(2-pyridin-3-ylethyl)acetamide;
2-[3-(3-methoxy-4-nitrophenyl)-11-oxo-10,11-dihydro-5H-dibenzo[b,e][1,4]diazepin-7-yl]N(2-pyridin-4-ylethyl)acetamide;
N-[2-(2,3-dimethoxyphenyl)ethyl]-2-[3-(3-methoxy-4-nitrophenyl)-11-oxo-10,11-dihydro-5H-dibenzo[b,e][1,4]diazepin-7-yl]acetamide;
N-[2-(1,3-benzodioxol-5-yl)ethyl]-2-[3-(3-methoxy-4-nitrophenyl)-11-oxo-10,11-dihydro-5H-dibenzo[b,e][1,4]diazepin-7-yl]acetamide;
2-[3-(3-methoxy-4-nitrophenyl)-11-oxo-10,11-dihydro-5H-dibenzo[b,e][1,4]diazepin-7-yl]N(thien-2-ylmethyl)acetamide;
2-[3-(3-methoxy-4-nitrophenyl)-11-oxo-10,11-dihydro-5H-dibenzo[b,e][1,4]diazepin-7-yl]N(1,3-thiazol-5-ylmethyl)acetamide;
N-[2-(1H-imidazol-4-yl)ethyl]-2-[3-(3-methoxy-4-nitrophenyl)-11-oxo-10,11-dihydro-5H-dibenzo[b,e][1,4]diazepin-7-yl]acetamide;
7-[2-(1,4-dioxa-8-azaspiro[4.5]dec-8-yl)-2-oxoethyl]-3-(3-methoxy-4-nitrophenyl)-5,10-dihydro-11H-dibenzo[b,e][1,4]diazepin-11-one;
7-{2-[2,6-dimethylmorpholin-4-yl]-2-oxoethyl}-3-(3-methoxy-4-nitrophenyl)-5,10-dihydro-11H-dibenzo[b,e][1,4]diazepin-11-one;
7-[2-(4-acetylpiperazin-1-yl)-2-oxoethyl]-3-(3-methoxy-4-nitrophenyl)-5,10-dihydro-11H-dibenzo[b,e][1,4]diazepin-11-one;
3-(3-methoxy-4-nitrophenyl)-7-[2-oxo-2-(4-pyridin-2-ylpiperazin-1-yl)ethyl]-5,10-dihydro-11H-dibenzo[b,e][1,4]diazepin-11-one;
3-({[3-(3-methoxy-4-nitrophenyl)-11-oxo-10,11-dihydro-5H-dibenzo[b,e][1,4]diazepin-7-yl]acetyl}amino)benzamide;
2-[3-(3-methoxy-4-nitrophenyl)-11-oxo-10,11-dihydro-5H-dibenzo[b,e][1,4]diazepin-7-yl]N(4-morpholin-4-ylphenyl)acetamide;
2-[3-(3-methoxy-4-nitrophenyl)-11-oxo-10,11-dihydro-5H-dibenzo[b,e][1,4]diazepin-7-yl]Nquinolin-6-ylacetamide;
N-[(1S)-1-(hydroxymethyl)-2-methylpropyl]-2-[3-(3-methoxy-4-nitrophenyl)-11-oxo-10,11-dihydro-5H-dibenzo[b,e][1,4]diazepin-7-yl]acetamide;
(2S)-2-({[3-(3-methoxy-4-nitrophenyl)-11-oxo-10,11-dihydro-5H-dibenzo[b,e][1,4]diazepin-7-yl]acetyl}amino)-4-methylpentanamide;
N-[(1S)-2-hydroxy-1-phenylethyl]-2-[3-(3-methoxy-4-nitrophenyl)-11-oxo-10,11-dihydro-5H-dibenzo[b,e][1,4]diazepin-7-yl]acetamide;
N-(2,3-dihydroxypropyl)-2-[3-(3-methoxy-4-nitrophenyl)-11-oxo-10,11-dihydro-5H-dibenzo[b,e][1,4]diazepin-7-yl]acetamide;
N-[3-(1H-imidazol-1-yl)propyl]-2-[3-(3-methoxy-4-nitrophenyl)-11-oxo-10,11-dihydro-5H-dibenzo[b,e][1,4]diazepin-7-yl]acetamide;
2-[3-(3-methoxy-4-nitrophenyl)-11-oxo-10,11-dihydro-5H-dibenzo[b,e][1,4]diazepin-7-yl]N[3-(2-oxopyrrolidin-1-yl)propyl]acetamide;
N-(2,6-difluorobenzyl)-2-[3-(3-methoxy-4-nitrophenyl)-11-oxo-10,11-dihydro-5H-dibenzo[b,e][1,4]diazepin-7-yl]acetamide;
N-{2-[4-(aminosulfonyl)phenyl]ethyl}-2-[3-(3-methoxy-4-nitrophenyl)-11-oxo-10,11-dihydro-5H-dibenzo[b,e][1,4]diazepin-7-yl]acetamide;
N-[(1R)-1-(hydroxymethyl)-2-methylpropyl]-2-[3-(3-methoxy-4-nitrophenyl)-11-oxo-10,11-dihydro-5H-dibenzo[b,e][1,4]diazepin-7-yl]acetamide;
N-[(1R)-2-hydroxy-1-phenylethyl]-2-[3-(3-methoxy-4-nitrophenyl)-11-oxo-10,11-dihydro-5H-dibenzo[b,e][1,4]diazepin-7-yl]acetamide;
2-[3-(3-methoxy-4-nitrophenyl)-11-oxo-10,11-dihydro-5H-dibenzo[b,e][1,4]diazepin-7-yl]N(thien-3-ylmethyl)acetamide;
methyl{3-[4-(aminocarbonyl)-3-methoxyphenyl]-11-oxo-10,11-dihydro-5H-dibenzo[b,e][1,4]diazepin-8-yl}acetate;
8-hydroxy-3-(3-methoxy-4-nitrophenyl)-5,10-dihydro-11H-dibenzo[b,e][1,4]diazepin-11-one;
8-methoxy-3-(3-methoxy-4-nitrophenyl)-5,10-dihydro-11H-dibenzo[b,e][1,4]diazepin-11-one;
8-ethoxy-3-(3-methoxy-4-nitrophenyl)-5,10-dihydro-11H-dibenzo[b,e][1,4]diazepin-11-one;
3-(3-methoxy-4-nitrophenyl)-8-[2-(4-methyl-1,3-thiazol-5-yl)ethoxy]-5,10-dihydro-11H-dibenzo[b,e][1,4]diazepin-11-one;
8-[3-(dimethylamino)propoxy]-3-(3-methoxy-4-nitrophenyl)-5,10-dihydro-11H-dibenzo[b,e][1,4-]diazepin-11-one;
3-(3-methoxy-4-nitrophenyl)-8-(2-morpholin-4-ylethoxy)-5,10-dihydro-11H-dibenzo[b,e][1,4]diazepin-11-one;
3-(3-methoxy-4-nitrophenyl)-8-[2-(4-morpholin-4-ylphenyl)ethoxy] -5,10-dihydro-11H-dibenzo[b,e][1,4]diazepin-11-one;
3-(3-methoxy-4-nitrophenyl)-7-piperidin-1-yl-5,10-dihydro-11H-dibenzo[b,e][1,4]diazepin-11-one;
7-[(2S)-2-(hydroxymethyl)pyrrolidin-1-yl]-3-(3-methoxy-4-nitrophenyl)-5,10-dihydro-11H-dibenzo[b,e][1,4]diazepin-11-one;
3-(3-methoxy-4-nitrophenyl)-7-morpholin-4-yl-5,10-dihydro-11H-dibenzo[b,e][1,4]diazepin-11-one;
7-(4-hydroxypiperidin-1-yl)-3-(3-methoxy-4-nitrophenyl)-5,10-dihydro-11H-dibenzo[b,e][1,4]diazepin-11-one;
methyl{3-[3-methoxy-4-(3-methyl-1,2,4-oxadiazol-5-yl)phenyl]-11-oxo-10,11-dihydro-5H-dibenzo[b,e][1,4]diazepin-8-yl}acetate;
8-(2-ethyl-2-hydroxybutyl)-3-(3-methoxy-4-nitrophenyl)-5,10-dihydro-11H-dibenzo[b,e][1,4]diazepin-11-one;
3-[(2-chloropyridin-4-yl)amino]-8-(2-ethyl-2-hydroxybutyl)-5,10-dihydro-11H-dibenzo[b,e][1,4]diazepin-11-one;
N,N-dimethyl-2-[11-oxo-3-(pyridin-4-ylamino)-10,11-dihydro-5H-dibenzo[b,e][1,4]diazepin-8-yl]acetamide;
N-(4-morpholin-4-ylphenyl)-2-[11-oxo-3-(pyridin-4-ylamino)-10,11-dihydro-5H-dibenzo[b,e][1,4]diazepin-8-yl]acetamide;
8-(2-hydroxy-2-methylpropyl)-3-(pyrimidin-4-ylamino)-5,10-dihydro-11H-dibenzo[b,e][1,4]diazepin-11-one;
8-(2-hydroxy-2-methylpropyl)-3-[(2-methylpyridin-4-yl)amino]-5,10-dihydro-11H-dibenzo[b,e][1,4]diazepin-11-one;
3-(3-methoxy-4-nitrophenyl)-8-(2-oxopropyl)-5,10-dihydro-11H-dibenzo[b,e][1,4]diazepin-11-one;
3-[(2-chloropyridin-4-yl)amino]-8-(2-oxopropyl)-5,10-dihydro-11H-dibenzo[b,e][1,4]diazepin-11-one;
3-({2-[(2-chhloropyridin-4-yl)amino]pyridin-4-yl}amino)-8-(2-oxopropyl)-5,10-dihydro-11H-dibenzo[b,e][1,4]diazepin-11-one;
methyl 2-methyl-2-[11-oxo-3-(pyrimidin-4-ylamino)-10,11-dihydro-5H-dibenzo[b,e][1,4]diazepin-8-yl]propanoate;
methyl 2-methyl-2-{3-[(2-methylpyridin-4-yl)amino]-11-oxo-10,11-dihydro-5H-dibenzo[b,e][1,4]diazepin-8-yl}propanoate;
2-methylN(4-morpholin-4-ylphenyl)-2-[11-oxo-3-(pyrimidin-4-ylamino)-10,11-dihydro-5H-dibenzo[b,e][1,4]diazepin-8-yl]propanamide;
2-methyl-2-{3-[(2-methylpyridin-4-yl)amino]-11-oxo-10,11-dihydro-5H-dibenzo[b,e][1,4]diazepin-8-yl}N(4-morpholin-4-ylphenyl)propanamide;
3-{[3-(2-hydroxyethyl)pyridin-4-yl]amino}-8-(1-hydroxy-1-methylethyl)-5,10-dihydro-11H-dibenzo[b,e][1,4]diazepin-11-one;
8-(2-hydroxy-2-methylpropyl)-3-[(2-methoxypyridin-4-yl)amino]-5,10-dihydro-11H-dibenzo[b,e][1,4]diazepin-11-one;
8-(2-hydroxy-2-methylpropyl)-3-[(2-methylpyridin-4-yl)amino]-5,10-dihydro-11H-dibenzo[b,e] [1,4]diazepin-11-one;
methyl 11-oxo-3-(pyrimidin-4-ylamino)-10,11-dihydro-5H-dibenzo[b,e][1,4]diazepine-7-carboxylate;
7-(1-hydroxy-1-methylethyl)-3-(3-methoxy-4-nitrophenyl)-5,10-dihydro-11H-dibenzn[b,e][1,4]diazepin-11-one;
7-(1-hydroxy-1-methylethyl)-3-(pyrimidin-4-ylamino)-5,10-dihydro-11H-dibenzo[b,e][1,4]diazepin-11-one;
2-{3-[(6-methoxypyrimidin-4-yl)amino]-11-oxo-10,11-dihydro-5H-dibenzo[b,e][1,4]diazepin-8-yl}-2-methylN(4-morpholin-4-ylphenyl)propanamide;
3-(3-methoxy-4-nitrophenyl)-8-{2-[(6-morpholin-4-ylpyridin-3-yl)oxy]ethyl}-5,10-dihydro-11H-dibenzo[b,e][1,4]diazepin-11-one;
3-(4-hydroxy-3-methoxyphenyl)-8-[2-(4-morpholin-4-ylphenoxy)ethyl]-5,10-dihydro-11H-dibenzo[b,e][1,4-]diazepin-11-one;
3-[(2,6-difluoropyridin-4-yl)amino]-8-[2-(4-morpholin-4-ylphenoxy)ethyl]-5,10-dihydro-11H-dibenzo[b,e][1,4]diazepin-11-one;
7-hydroxy-8-methoxy-3-(3-methoxy-4-nitrophenyl)-5,10-dihydro-11H-dibenzo[b,e][1,4]diazepin-11-one;
8-hydroxy-7-methoxy-3-(3-methoxy-4-nitrophenyl)-5,10-dihydro-11H-dibenzo[b,e][1,4]diazepin-11-one;
8-methoxy-3-(3-methoxy-4-nitrophenyl)-7-(tetrahydro-2H-pyran-2-ylmethoxy)-5,10-dihydro-11H-dibenzo[b,e][1,4]diazepin-11-one;
8-methoxy-3-(3-methoxy-4-nitrophenyl)-7-[(1-methylpiperidin-3-yl)methoxy]-5,10-dihydro-11H-dibenzo[b,e][1,4]diazepin-11-one;
8-methoxy-3-(3-methoxy-4-nitrophenyl)-7-(pyridin-2-ylmethoxy)-5,10-dihydro-11H-dibenzo[b,e][1,4]diazepin-11-one;
8-methoxy-3-(3-methoxy-4-nitrophenyl)-7-(pyridin-3-ylmethoxy)-5,10-dihydro-11H-dibenzo[b,e][1,4]diazepin-11-one;
8-methoxy-3-(3-methoxy-4-nitrophenyl)-7-(pyridin-4-ylmethoxy)-5,10-dihydro-11H-dibenzo[b,e][1,4]diazepin-11-one;
8-methoxy-3-(3-methoxy-4-nitrophenyl)-7-[(5-methylisoxazol-3-yl)metboxy]-5,10-dihydro-11H-dibenzo[b,e][1,4]diazepin-11-one;
8-methoxy-3-(3-methoxy-4-nitrophenyl)-7-[(1-methyl-1H-imidazol-5-yl)methoxy]-5,10-dihydro-11H-dibenzo[b,e][1,4]diazepin-11-one;
8-methoxy-3-(3-methoxy-4-nitrophenyl)-7-[(2-methyl-l,3-thiazol-4-yl)methoxy]-5,10-dihydro-11H-dibenzo[b,e][1,4]diazepin-11-one;
8-methoxy-3-(3-methoxy-4-nitrophenyl)-7-[(2-oxo-1,3-oxazolidin-5-yl)methoxy]-5,10-dihydro-11H-dibenzo[b,e][1,4]diazepin-11-one;
8-methoxy-3-(3-methoxy-4-nitrophenyl)-7-(tetrahydrofuran-2-ylmethoxy)-5,10-dihydro-11H-dibenzo[b,e][1,4]diazepin-11-one;
7-[(2,2-dimethyl-1,3-dioxolan-4-yl)methoxy]-8-methoxy-3-(3-methoxy-4-nitrophenyl)-5,10-dihydro-11H-dibenzo[b,e] [1,4]diazepin-11-one;
8-methoxy-3-(3-methoxy-4-nitrophenyl)-7-[(2R)-pyrrolidin-2-ylmethoxy]-5,10-dihydro-11H-dibenzo[b,e][1,4]diazepin-11-one;
7,8-dimethoxy-3-(3-methoxy-4-nitrophenyl)-5,10-dihydro-11H-dibenzo[b,e][1,4]diazepin-11-one;
8-methoxy-7-[2-(2-methoxyethoxy)ethoxy]-3-(3-methoxy-4-nitrophenyl)-5,10-dihydro-11H-dibenzo[b,e][1,4]diazepin-11-one;
7-(2,3-dihydroxypropoxy)-8-methoxy-3-(3-methoxy-4-nitrophenyl)-5,10-dihydro-11H-dibenzo[b,e,][1,4]diazepin-11-one;
7-[3-hydroxy-2,2-bis(hydroxymethyl)propoxy]-8-methoxy-3-(3-methoxy-4-nitrophenyl)-5,10-dihydro-11H-dibenzo[b,e][1,4]diazepin-11-one;
2-hydroxy-3-{[8-methoxy-3-(3-methoxy-4-nitrophenyl)-11-oxo-10,11-dihydro-5H-dibenzo[b,e][1,4]diazepin-7-yl]oxy}propane-1-sulfonic acid;
7-(3-aminopropoxy)-8-methoxy-3-(3-methoxy-4-nitrophenyl)-5,10-dihydro-11H-dibenzo[b,e][1,4]diazepin-11-one;
7-[2-(dimethylamino)ethoxy]-8-methoxy-3-(3-methoxy-4-nitrophenyl)-5,10-dihydro-11H-dibenzo[b,e][1,4]diazepin-11-one;
7-(2-chloroethoxy)-8-methoxy-3-(3-methoxy-4-nitrophenyl)-5,10-dihydro-11H-dibenzo[b,e][1,4]diazepin-11-one;
8-methoxy-3-(3-methoxy-4-nitrophenyl)-7-(2-pyrrolidin-1-ylethoxy)-5,10-dihydro-11H-dibenzo[b,e][1,4]diazepin-11-one;
8-methoxy-3-(3-methoxy-4-nitrophenyl)-7-(2-morpholin-4-ylethoxy)-5,10-dihydro-11H-dibenzo[b,e][1,4]diazepin-11-one;
7-(4-hydroxybutoxy)-8-methoxy-3-(3-methoxy-4-nitrophenyl)-5,10-dihydro-11H-dibenzo[b,e][1,4]diazepin-11-one;
7-(4-hydroxybutoxy)-3-(4-hydroxy-3-methoxyphenyl)-8-methoxy-5,10-dihydro-11H-dibenzo[b,e][1,4]diazepin-11-one;
7-(4-hydroxybutoxy)-8-methoxy-3-(pyrimidin-4-ylamino)-5,10-dihydro-11H-dibenzo[b,e][1,4]diazepin-11-one;
4-{[7-(4-hydroxybutoxy)-8-methoxy-11-oxo-10,11-dilydro-5H-dibenzo[b,e][1,4]diazepin-3-yl]amino}pyridine-2-carbonitrile;
3-[(2,6-difluoropyridin-4-yl)amino]-7-(4-hydroxybutoxy)-8-methoxy-5,10-dihydro-11H-dibenzo[b,e][1,4]diazepin-11-one;
7-(4-hydroxybutoxy)-8-methoxy-3-[(2,3,6-trifluoropyridin-4-yl)amino]-5,10-dihydro-11H-dibenzo[b,e][1,4]diazepin-11-one;
7-ethoxy-3-(3-methoxy-4-nitrophenyl)-5,10-dihydro-11H-dibenzo[b,e][1,4]diazepin-11-one;
7-(4-hydroxybutoxy)-3-(3-methoxy-4-nitrophenyl)-5,10-dihydro-11H-dibenzo[b,e][1,4]diazepin-11-one;
7-(2-hydroxyethoxy)-3-(3-methoxy-4-nitrophenyl)-5,10-dihydro-11H-dibenzo[b,e][1,4]diazepin-11-one;
7-(2,3-dihydroxypropoxy)-3-(3-methoxy-4-nitrophenyl)-5,10-dihydro-11H-dibenzo[b,e][1,4]diazepin-11-one;
7-[2-(2-methoxyethoxy)ethoxy]-3-(3-methoxy-4-nitrophenyl)-5,10-dihydro-11H-dibenzo[b,e][1,4]diazepin-11-one;
7-(methoxymethyl)-3-(3-methoxy-4-nitrophenyl)-5,10-dihydro-11H-dibenzo[b,e][1,4]diazepin-11-one;
7-(3-methoxy-4-nitrobenzyl)-3-(3-methoxy-4-nitrophenyl)-5,10-dihydro-11H-dibenzo[b,e][1,4]diazepin-11-one;
7-{[[2-(dimethylamino)ethyl](methyl)amino]methyl}-3-(3-methoxy-4-nitrophenyl)-5,10-dihydro-11H-dibenzo[b,e][1,4]diazepin-11-one;
3-(3-methoxy-4-nitrophenyl)-7-{[(2-tetrahydro-2H-pyran-4-ylethyl)amino]methy}-5,10-dihydro-11H-dibenzo[b,e][1,4]diazepin-11-one;
8-ethyl-7-methoxy-3-(3-methoxy-4-nitrophenyl)-5,10-dihydro-11H-dibenzo[b,e][1,4]diazepin-11-one;
8-methoxy-3-(3-methoxy-4-nitrophenyl)-7-vinyl-5,10-dihydro-11H-dibenzo[b,e][1,4]diazepin-11-one;
8-(3-hydroxypropyl)-7-methoxy-3-(3-methoxy-4-nitrophenyl)-5,10-dihydro-11H-dibenzo[b,e][1,4]diazepin-11-one;
7-methoxy-3-(3-methoxy-4-nitrophenyl)-8-{3-[(2-methylpyridin-3-yl)oxy]propy}-5,10-dihydro-11H-dibenzo[b,e][1,4]diazepin-11-one;
8-{3-[(2-chloropyridin-3-yl)oxy]propyl}-7-methoxy-3-(3-methoxy-4-nitropheny])-5,10-dihydro-11H-dibenzo[b,e][1,4]diazepin-11-one;
7-methoxy-3-(3-methoxy-4-nitrophenyl)-8-[3-(4-morpholin-4-ylphenoxy)propyl]-5,10-dihydro-11H-dibenzo[b,e][1,4]diazepin-11-one;
8-[3-(isoquinolin-3-yloxy)propy]]-7-methoxy-3-(3-methoxy-4-nitrophenyl)-5,10-dihydro-11H-dibenzo[b,e][1,4]diazepin-11-one;
7-methoxy-3-(3-methoxy-4-nitrophenyl)-8-[3-(3-oxoisoquinolin-2(3H)-yl)propyl]-5,10-dihydro-11H-dibenzo[b,e][1,4]diazepin-11-one;
methyl 7-methoxy-3-(3-methoxy-4-nitrophenyl)-11-oxo-10,11-dihydro-5H-dibenzo[b,e][1,4]diazepine-8-carboxylate;
methyl 7-methoxy-11-oxo-3-(pyrimidin-4-ylamino)-10,11-dihydro-5H-dibenzo[b,e][1,4]diazepine-8-carboxylate;
3-[(2,6-difluoropyridin-4-yl)amino]-8-(1,1-dimethyl-2-morpholin-4-yl-2-oxoethyl)-5,10-dihydro-11H-dibenzo[b,e][1,4]diazepin-11-one;
2-{3-[(2,6-difluoropyridin-4-yl)amino]-11-oxo-10,11-dihydro-5H-dibenzo[b,e][1,4]diazepin-8-yl}-N,N,2-trimethylpropanamide;
2-{3-[(2,6-difluoropyridin-4-yl)amino]-11-oxo-10,11-dihydro-5H-dibenzo[b,e][1,4]diazepin-8-yl}-2-methylNpyridin-4-ylpropanamide;
2-{3-[(2,6-difluoropyridin-4-yl)amino]-11-oxo-10,11-dihydro-5H-dibenzo[b,e][1,4]diazepin-8-yl}-2-methylN1,3-thiazol-2-ylpropanamide;
3-[(2,6-difluoropyridin-4-yl)amino]-8-{2-[(3R)-3-hydroxypiperidin-1-yl]-1,1-dimethyl-2-oxoethyl}-5,10-dihydro-11H-dibenzo[b,e][1,4]diazepin-11-one;
3-[(2,6-difluoropyridin-4-yl)amino]-8-[2-(4-hydroxypiperidin-1-yl)-1,1-dimethyl-2-oxoethyl]-5,10-dihydro-11H-dibenzo[b,e][1,4]diazepin-11-one;
3-[(2,6-difluoropyridin-4-yl)amino]-8-{2-[(2S)-2-(hydroxymethyl)pyrrolidin-1-yl]-1,1-dimethyl-2-oxoethyl}-5,10-dihydro-11H-dibenzo[b,e][1,4]diazepin-11-one;
3-[(2,6-difluoropyridin-4-yl)amino]-8-(1,1-dimethyl-2-oxo-2-pyrrolidin-1-ylethyl)-5,10-dihydro-11H-dibenzo[b,e][1,4]diazepin-11-one;
2-{3-[(2,6-difluoropyridin-4-yl)amino]-11-oxo-10,11-dihydro-5H-dibenzo[b,e][1,4]diazepin-8-yl}-2-methylNpyridin-2-ylpropanamide;
2-{3-[(2,6-difluoropyridin-4-yl)amino]-11-oxo-10,11-dihydro-5H-dibenzo[b,e][1,4]diazepin-8-yl}-2-methylNpyridin-3-ylpropanamide;
2-{3-[(2,6-difluoropyridin-4-yl)amino]-11-oxo-10,11-dihydro-5H-dibenzo[b,e][1,4]diazepin-8-yl}N(4-fluorophenyl)-2-methylpropanamide;
3-[(2,6-difluoropyridin-4-yl)amino]-8-{2-[(2R)-2-(hydroxymethyl)pyrrolidin-1-yl]-1,1-dimethyl-2-oxoethyl}-5,10-dihydro-11H-dibenzo[b,e][1,4]diazepin-11-one;
8-methoxy-3-(3-methoxy-4-nitrophenyl)-7-(3-morpholin-4-yl-3-oxopropyl)-5,10-dihydro-11H-dibenzo[b,e][1,4]diazepin-11-one;
3-[3-(3-methoxy-4-nitrophenyl)-11**-**oxo**-**10,11-dihydro-5H-dibenzo[b,e][1,4]diazepin-8-yl]Npyridin-3-ylpropanamide;
3-[3-(3-methoxy-4-nitrophenyl)-11-oxo-10,11-dihydro-5H-dibenzo[b,e][1,4]diazepin-8-yl]Npyridin-4-ylpropanamide;
8-[3-(3-hydroxypiperidin-1-yl)-3-oxopropyl]-3-(3-methoxy-4-nitrophenyl)-5,10-dihydro-11H-dibenzo[b,e][1,4]diazepin-11-one;
N-(4-fluorophenyl)-3-[3-(3-methoxy-4-nitrophenyl)-11-oxo-10,11-dihydro-5H-dibenzo[b,e][1,4]diazepin-8-yl]propanamide;
3-[3-(3-methoxy-4-nitrophenyl)-11-oxo-10,11-dihydro-5H-dibenzo[b,e][1,4]diazepin-8-yl]N[4-(trifluoromethyl)phenyl]propanamide;
3-[3-(3-methoxy-4-nitrophenyl)-11-oxo-10,11-dihydro-5H-dibenzo[b,e][1,4]diazepin-8-yl]Nmethylpropanamide;
3-[8-methoxy-3-(3-methoxy-4-nitrophenyl)-11-oxo-10,11-dihydro-5H-dibenzo[b,e][1,4]diazepin-7-yl]-N,N-dimethylpropanamide;
8-{2-[(6-chloropyridin-3-yl)oxy]ethyl}-3-(3-methoxy-4-nitrophenyl)-5,10-dihydro-11H-dibenzo[b,e][1,4]diazepin-11-one;
8-{2-[(2-chloropyridin-3-yl)oxy]ethyl}-3-(3-methoxy-4-nitrophenyl)-5,10-dihydro-11H-dibenzo[b,e][1,4]diazepin-11-one;
3-(3-methoxy-4-nitrophenyl)-8-{2-[(6-methylpyridin-3-yl)oxy]ethyl}-5,10-dihydro-11H-dibenzo[b,e][1,4]diazepin-11-one;
3-(3-methoxy-4-nitrophenyl)-8-{2-[(2-methylpyridin-3-yl)oxy]ethyl}-5,10-dihydro-11H-dibenzo[b,e][1,4]diazepin-11-one;
3-(3-methoxy-4-nitrophenyl)-8-[2-(pyridin-3-yloxy)ethyl]-5,10-dihydro-11H-dibenzo[b,e][1,4]diazepin-11-one;
8-{2-[(2,6-dimethylpyridin-3-yl)oxy]ethyl}-3-(3-methoxy-4-nitrophenyl)-5,10-dihydro-11H-dibenzo[b,e][1,4]diazepin-11-one;
8-[2-({2-[(dimethylamino)methyl]pyridin-3-yl}oxy)ethyl3-3-(3-methoxy-4-nitrophenyl)-5,10-dihydro-11H-dibenzo[b,e][1,4]diazepin-11-one;
8-[2-(isoquinolin-7-yloxy)ethyl]-3-(3-methoxy-4-nitrophenyl)-5,10-dihydro-11H-dibenzo[b,e][1,4]diazepin-11-one;
7-methoxy-3-(3-methoxy-4-nitrophenyl)-N,N-dimethyl-11-oxo-10,11-dihydro-5H-dibenzo[b,e][1,4]diazepine-8-carboxamide;
7-methoxy-3-(3-methoxy-4-nitrophenyl)-11-oxo-10,11-dihydro-5H-dibenzo[b,e][1,4]diazepine-8-carboxylic acid;
7-{2-[(2-chloropyridin-3-yl)oxy]ethyl}-8-methoxy-3-(3-methoxy-4-nitrophenyl)-5,10-dihydro-11H-dibenzo[b,e][1,4]diazepin-11-one;
8-[2-(isoquinolin-5-yloxy)ethyl]-3-(3-methoxy-4-nitrophenyl)-5,10-dihydro-11H-dibenzo[b,e][1,4]diazepin-11-one;
3-(3-methoxy-4-nitrophenyl)-8-[2-(quinolin-5-yloxy)ethyl]-5,10-dihydro-11H-dibenzo[b,e][1,4]diazepin-11-one;
3-(3-methoxy-4-nitrophenyl)-8-[2-(4-methoxyphenoxy)ethyl]-5,10-dihydro-11H-dibenzo[b,e][1,4]diazepin-11-one;
3-(3-methoxy-4-nitrophenyl)-8-[2-(3-methoxyphenoxy)ethyl]-5,10-dihydro-11H-dibenzo[b,e][1,4]diazepin-11-one;
3-{2-[3-(3-methoxy-4-nitrophenyl)-11-oxo-10,11-dihydro-5H-dibenzo[b,e][1,4]diazepin-8-yl]ethoxy}pyridine-2-carboxamide;
3-(3-methoxy-4-nitrophenyl)-8-[3-(4-morpholin-4-ylphenoxy)propyl]-5,10-dihydro-11H-dibenzo[b,e][1,4]diazepin-11-one;
3-(3-methoxy-4-nitrophenyl)-8-[3-(pyridin-3-yloxy)propyl]-5,10-dihydro-11H-dibenzo[b,e][1,4]diazepin-11-one;
8-[2-(3-aminophenoxy)ethyl]-3-(3-methoxy-4-nitrophenyl)-5,10-dihydro-11H-dibenzo[b,e][1,4]diazepin-11-one;
3-(3-methoxy-4-nitrophenyl)-8-{2-[(2-methyl-1,3-benzothiazol-7-yl)oxy]ethyl}-5,10-dihydro-11H-dibenzo[b,e][1,4]diazepin-11-one;
2-[3-(3-methoxy-4-nitrophenyl)-11-oxo-10,11-dihydro-5H-dibenzo[b,e][1,4]diazepin-8-yl]-2-methylN(pyridin-2-ylmethyl)propanamide;
8-(2-hydroxy-2-methylpropyl)-3-(3-methoxy-4-nitrophenyl)-5,10-dihydro-11H-dibenzo[b,e][1,4]diazepin-11-one;
3-(3-methoxy-4-nitrophenyl)-8-[(4-methylpiperazin-1-yl)methyl]-5,10-dihydro-11H-dibenzo[b,e][1,4]diazepin-11-one;
3-(4-chloro-3-methoxyphenyl)-8-[(4-methylpiperazin-1-yl)methyl]-5,10-dihydro-11H-dibenzo[b,e][1,4]diazepin-11-one;
2-methoxy-4-{8-[(4-methylpiperazin-1-yl)methyl]-11-oxo-10,11-dihydro-5H-dibenzo[b,e][1,4]diazepin-3-yl}benzonitrile;
3-(4-hydroxy-3-methoxyphenyl)-8-(hydroxymethyl)-5,10-dihydro-11H-dibenzo[b,e][1,4]diazepin-11-one;
3-(3-methoxy-4-nitrophenyl)-8-(morpholin-4-ylmethyl)-5,10-dihydro-11H-dibenzo[b,e][1,4]diazepin-11-one;
8-{[(2R)-2-(hydroxymethyl)pyrrolidin-1-yl]methyl}-3-(3-methoxy-4-nitrophenyl)-5,10-dihydro-11H-dibenzo[b,e][1,4]diazepin-11-one;
7-(2-hydroxyethoxy)-8-methoxy-3-(3-methoxy-4-nitrophenyl)-5,10-dihydro-11H-dibenzo[b,e][1,4]diazepin-11-one;
8-{3-[2-(hydroxymethyl)pyrrolidin-1-yl]-3-oxopropyl}-3 -(3 -methoxy-4-nitrophenyl)-5,10-dihydro-11H-dibenzo[b,e][1,4]diazepin-11-one;
(cis) 4-hydroxyN[3-(3-methoxy-4-nitrophenyl)-11-oxo-10,11-dihydro-5H-dibenzo[b,e][1,4]diazepin-7-yl]cyclohexanecarboxamide.

17. A pharmaceutical composition comprising a compound of formula (I) 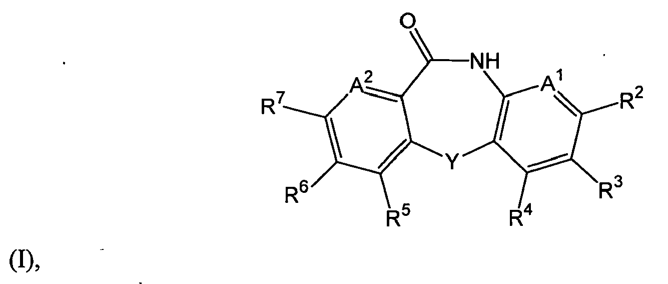 or a therapeutically acceptable salt thereof, in combination with a therapeutically acceptable carrier,wherein
(a)
A¹ is CH;
A² is CH;
R² is selected from the group consisting of hydrogen, alkenyl, alkoxy, alkoxyalkoxy, alkoxyalkoxyalkoxy, alkoxyalkyl, alkoxycarbonyl, alkoxycarbonylalkyl, alkyl, alkylcarbonyl, alkylcarbonylalkyl, amino, aminoalkoxy, aminoalkyl, aminocarbonyl, aminocarbonylalkyl, aminosulfonyl, aryl, arylalkoxy, arylalkyl, aryloxyalkyl, carboxy, carboxyalkyl, cyano, cyanoalkyl, cycloalkyl, cycloalkylalkyl, halo, haloalkoxy, haloalkyl, heterocyclyl, heterocyclylalkoxy, heterocyclylalkyl, heterocyclylcarbonylalkyl, heterocyclyloxyalkyl, hydroxy, hydroxyalkoxy, hydroxyalkyl, nitro, nitroalkyl, and -(CR⁹R¹⁰)ₘC(O)NR¹¹R¹²;
R³, R⁴ and R⁵ are each hydrogen;
R⁶ is selected from the group consisting of aryl, cycloalkyl, halo, heterocyclyl, and -XR¹³;
R⁷ is hydrogen;
R⁹ and R¹⁰ are independently selected from the group consisting of hydrogen, alkenyl, alkyl, aminoalkyl, and hydroxyalkyl; or
R⁹ and R¹⁰, together with the carbon atom to which they are attached, form a cycloalkyl group;
R¹¹ and R¹² are each independently selected from the group consisting of hydrogen, alkenyl, alkoxyalkyl, alkyl, amino, aminoalkyl, aryl, arylalkyl, cyanoalkyl, cycloalkyl, cycloalkylalkyl, haloalkyl, heterocyclyl, heterocyclylalkyl, hydroxyalkyl, -NR^{c}R^{d}, (NR^{c}R^{d})alkyl, and (NR^{c}R^{d})carbonylalkyl; or
R¹¹ and R¹², together with the nitrogen atom to which they are attached, form a heterocyclyl ring selected from the group consisting of azetidinyl, diazepanyl, imidazolidinyl, morpholinyl, piperazinyl, piperidinyl, pyrrolidinyl and thiomorpholinyl, which can each be optionally substituted with one, two, or three substituents independently selected from the group consisting of alkoxy, alkoxycarbonyl, alkenyl, alkyl, alkylcarbonyl, aryl, carboxy, carboxyalkyl, heterocyclyl, heterocyclylalkyl, hydroxy, hydroxyalkyl, -NR^{c}R^{d}, (NR^{c}R^{d})alkyl, and (NR^{c}R^{d})carbonyl;
R¹³ is selected from the group consisting of aryl, cycloalkyl, and heterocyclyl;
X is selected from the group consisting of -O-, -NR¹⁴-, -C(O)-, -S-, -SO₂- , -(CH₂)ₙ-, -C(O)NR¹⁴-, -NR¹⁴C(O)-, -SO₂NR¹⁴-, -NR¹⁴SO₂, -O(CH₂)ₘ-, -(CH₂)ₘO-, -CH=CH-, and -C≡C-; wherein R¹⁴ is selected from the group consisting of hydrogen, alkenyl, alkyl, aminoalkyl, and hydroxyalkyl;
Y is NH;
m is 0-3; and
n is 1-3;
or wherein
(b)
A¹ is CH;
A² is CH;
Y is NH;
R⁴, R⁵, and R⁷ are each hydrogen;
R⁶ is phenyl substituted in the three position with methoxy and substituted in the four position with alkoxycarbonyl, alkoxycarbonylalkenyl, alkylcarbonyl, alkylsulfonyl, cyano, halo, haloalkenyl, hydroxy, nitro, NH₂SO₂-, or NH₂CO-; and either
(i) R² is hydrogen and R³ is -(CR⁹R¹⁰)C(O)NHR¹² where R⁹ and R¹⁰ are independently selected from the group consisting of hydrogen, alkenyl, alkyl, aminoalkyl, and hydroxyalkyl, or R⁹ and R¹⁰, together with the carbon atom to which they are attached, form a cycloalkyl group, and R¹² is phenyl optionally substituted with 1 morpholinyl group;
(ii) R² is hydrogen and R³ is hydroxyalkyl;
(iii) R² is hydrogen and R³ is alkoxy;
(iv) R² and R³ are independently -(CR⁹R¹⁰)C(O)NHR¹² where R⁹ and R¹⁰ are independently selected from the group consisting of hydrogen, alkenyl, alkyl, aminoalkyl, and hydroxyalkyl, or R⁹ and R¹⁰, together with the carbon atom to which they are attached, form a cycloalkyl group, and R¹² is phenyl optionally substituted with 1 morpholinyl group;
(v) R² and R³ are independently hydroxalkyl; or
(vi) R² and R³ are independently alkoxy;
wherein
alkyl is a C₁-C₁₀ alkyl;
alkenyl is a C₂-C₆ alkenyl;
alkoxy is a C₁-C₁₀ alkoxy;
aryl is phenyl group, or a bicyclic or tricyclic fused ring system wherein one or more of the fused rings is a phenyl group, wherein aryl can be optionally substituted with one, two, three, four, or five substituents independently selected from the group consisting of alkoxy, alkoxyalkyl, alkoxycarbonyl, alkoxycarbonylalkenyl, alkoxycarbonylalkyl, alkoxysulfonyl, alkyl, alkylcarbonyl, alkylsulfonyl, amino, aminocarbonyl, aminosulfonyl, a second aryl group, arylalkyl, arylsulfonyl, carboxy, cyano, formyl, halo, haloalkoxy, haloalkenyl, haloalkyl, heterocyclyl, heterocyclylalkyl, hydroxy, hydroxyalkyl, nitro, (NR^{c}R^{d})alkyl, and oxo, wherein the second aryl group, the aryl part of the arylalkyl and the arylsulfonyl, the heterocyclyl, and the heterocyclyl part of the heterocyclylalkyl can be optionally substituted with one, two, three, four, or five substituents independently selected from the group consisting of alkoxy, alkoxyalkyl, alkoxycarbonyl, alkyl, alkylcarbonyl, carboxy, cyano, halo, haloalkoxy, haloalkyl, hydroxy, nitro, and oxo;
heterocyclyl is selected from a five-, six-, or seven-membered ring containing one, two, or three heteroatoms independently selected from the group consisting of nitrogen, oxygen, and sulfur, wherein the five-membered ring has zero to two double bonds and the six- and seven-membered rings have zero to three double bonds; bicyclic groups in which the heterocyclyl ring is fused to a phenyl group, a monocyclic cycloalkenyl group, a monocyclic cycloalkyl group, or another monocyclic heterocyclyl group; and tricyclic groups in which a bicyclic system is fused to a phenyl group, a monocyclic cycloalkenyl group, a monocyclic cycloalkyl group, or another monocyclic heterocyclyl group; wherein heterocyclyl can be optionally substituted with one, two, three, four, or five substituents independently selected from the group consisting of alkoxy, alkoxyalkyl, alkoxycarbonyl, alkyl, alkylcarbonyl, alkylsulfonyl, amino, aminocarbonyl, aminosulfonyl, aryl, arylalkyl, arylsulfonyl, carboxy, cyano, halo, a second heterocyclyl group, heterocyclylalkyl, hydroxy, hydroxyalkyl, nitro, -NR^{c}R^{d}, (NR^{c}R^{d})alkyl, and oxo, wherein the aryl, the aryl part of the arylalkyl and the arylsulfonyl, the second heterocyclyl group, and the heterocyclyl part of the heterocyclylalkyl can be optionally substituted with one, two, three, four, or five substituents independently selected from the group consisting of alkoxy, alkoxyalkyl, alkoxycarbonyl, alkyl, alkylcarbonyl, carboxy, cyano, halo, haloalkoxy, haloalkyl, hydroxy, methylenedioxy, nitro, and oxo;
cycloalkyl is a saturated monocyclic, bicyclic, or tricyclic hydrocarbon ring system having three to twelve carbon atoms, wherein cycloalkyl can be optionally substituted with one, two, three, or four substituents independently selected from the group consisting of alkoxy, alkoxyalkyl, alkoxycarbonyl, alkyl, alkylcarbonyl, alkylsulfonyl, -NR^{c}R^{d}, (NR^{c}R^{d})alkyl, (NR^{c}R^{d})carbonyl, a second aryl group, arylalkyl, arylsulfonyl, carboxy, cyano, halo, heterocyclyl, heterocyclylalkyl, hydroxy, hydroxyalkyl, nitro, and oxo, wherein the second aryl group, the aryl part of the arylalkyl and the arylsulfonyl, the heterocyclyl, and the heterocyclyl part of the heterocyclylalkyl can be optionally substituted with one, two, three, four, or five substituents independently selected from the group consisting of alkoxy, alkoxyalkyl, alkoxycarbonyl, alkyl, alkylcarbonyl, carboxy, cyano, halo, haloalkoxy, haloalkyl, hydroxy, nitro, and oxo;
cycloalkenyl is a non-aromatic cyclic or bicyclic ring system having three to ten carbon atoms and one to three rings, wherein each five-membered ring has one double bond, each six-membered ring has one or two double bonds, each seven-and eight-membered ring has one to three double bonds, and each nine-to ten-membered ring has one to four double bonds;
amino refers to -NR^{a}R^{b}, wherein R^{a} and R^{b} are independently selected from the group consisting of hydrogen, alkoxyalkylcarbonyl, alkoxycarbonyl, alkyl, alkylcarbonyl, alkylsulfonyl, aryl, arylalkyl, arylalkylcarbonyl, arylalkylsulfonyl, arylsulfonyl, arylsulfonylalkyl, arylsulfonylalkylcarbonyl, cycloalkyl, cycloalkylalkyl, cycloalkylcarbonyl, haloalkylsulfonyl, heterocyclyl, heterocyclylalkylcarbonyl, heterocyclylalkylsulfonyl, heterocyclylcarbonyl, heterocyclylsulfonyl, -NR^{c}R^{d}, (NR^{c}R^{d})alkyl, (NR^{c}R^{d})alkylcarbonyl, (NR^{c}R^{d})alkylsulfonyl, (NR^{c}R^{d})carbonyl, (NR^{c}R^{d})carbonylalkyl, and (NR^{c}R^{d})carbonylalkylcarbonyl, wherein aryl can be optionally substituted with one, two, three, four, or five substituents independently selected from the group consisting of alkoxy, alkoxyalkyl, alkoxycarbonyl, alkyl, alkylcarbonyl, alkylsulfonyl, a second aryl group, arylalkyl, arylsulfonyl, carboxy, cyano, halo, heterocyclyl, heterocyclylalkyl, hydroxy, nitro, and oxo, wherein the second aryl group, the aryl part of the arylalkyl and the arylsulfonyl, the heterocyclyl, and the heterocyclyl part of the heterocyclylalkyl can be optionally substituted with one, two, three, four, or five substituents independently selected from the group consisting of alkoxy, alkoxyalkyl, alkoxycarbonyl, alkyl, alkylcarbonyl, carboxy, cyano, halo, haloalkoxy, haloalkyl, hydroxy, nitro, and oxo; and
R^{c} and R^{d} are each independently hydrogen, alkoxycarbonyl, alkyl, alkylcarbonyl, aryl, arylsulfonyl, heterocyclcarbonyl, or formyl.

18. A pharmaceutical composition as defined in claim 17, or a therapeutically acceptable salt thereof, for use in the treatment of primary and metastatic solid tumors.

19. A pharmaceutical composition of claim 18 for use in the treatment of carcinomas of breast, colon, rectum, lung, oropharynx, hypopharynx, esophagus, stomach, pancreas, liver, gallbladder and bile ducts, small intestine, urinary tract, female genital tract, male genital tract, endocrine glands and skin; hemangiomas, melanomas, sarcomas including those arising from bone and soft tissues as well as Kaposi's sarcoma; tumors of the brain, nerves, eyes, and meninges including astrocytomas, gliomas, glioblastomas, retinoblastomas, neuromas, neuroblastomas, Schwannomas, and meningiomas; solid tumors arising from hematopoietic malignancies such as leukemias; or lymphomas; or for preventing metastases from the above tumors when used alone or in combination with radiotherapy and/or other chemotherapeutic agents.

20. A pharmaceutical composition as defined in claim 17, or a therapeutically acceptable salt thereof, for use in the treatment of cancer in a patient in recognized need of such treatment.

21. A pharmaceutical composition as defined in claim 17 for use as a pharmaceutical.

## Patentansprüche

1. Eine Verbindung mit der Formel (I) 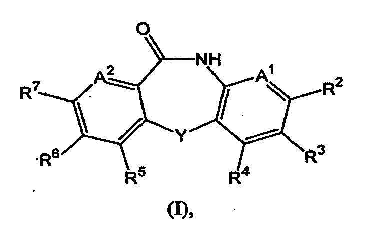 oder ein therapeutisch verträgliches Salz davon, worin
(a)
A¹ CH ist;
A² CH ist;
R² gewählt ist aus der Gruppe bestehend aus Wasserstoff, Alkenyl, Alkoxy, Alkoxyalkoxy, Alkoxyalkoxyalkoxy, Alkoxyalkyl, Alkoxycarbonyl, Alkoxycarbonylalkyl, Alkyl, Alkylcarbonyl, Alkylcarbonylalkyl, Amino, Aminoalkoxy, Aminoalkyl, Aminocarbonyl, Aminocarbonylalkyl, Aminosulfonyl, Aryl, Arylalkoxy, Arylalkyl, Aryloxyalkyl, Carboxy, Carboxyalkyl, Cyano, Cyanoalkyl, Cycloalkyl, Cycloalkylalkyl, Halo, Haloalkoxy, Haloalkyl, Heterocyclyl, Heterocyclylalkoxy, Heterocyclylalkyl, Heterocyclylcarbonylalkyl, Heterocyclyloxyalkyl, Hydroxy, Hydroxyalkoxy, Hydroxyalkyl, Nitro, Nitroalkyl und - (CR⁹R¹⁰)ₘC(O)NR¹¹R¹²;
R³, R⁴ und R⁵ jeweils Wasserstoff sind;
R⁶ gewählt ist aus der Gruppe bestehend aus Aryl, Cycloalkyl, Halo, Heterocyclyl und -XR¹³;
R⁷ Wasserstoff ist;
R⁹ und R¹⁰ sind unabhängig gewählt aus der Gruppe bestehend aus Wasserstoff, Alkenyl, Alkyl, Aminoalkyl und Hydroxyalkyl; oder
R⁹ und R¹⁰ bilden zusammen mit dem Kohlenstoffatom, an welches sie gebunden sind, eine Cycloalkylgruppe;
R¹¹ und R¹² sind jeweils unabhängig gewählt aus der Gruppe bestehend aus Wasserstoff, Alkenyl, Alkoxyalkyl, Alkyl, Amino, Aminoalkyl, Aryl, Arylalkyl, Cyanoalkyl, Cycloalkyl, Cycloalkylalkyl, Haloalkyl, Heterocyclyl, Heterocyclylalkyl, Hydroxyalkyl, -NR^{c}R^{d}, (NR^{c}R^{d}) Alkyl und (NR^{c}R^{d}) Carbonylalkyl; oder
R¹¹ und R¹², zusammen mit dem Stickstoffatom, an welches sie gebunden sind, bilden einen Heterocyclylring, gewählt aus der Gruppe bestehend aus Azetidinyl, Diazepanyl, Imidazolidinyl, Morpholinyl, Piperazinyl, Piperidinyl, Pyrrolidinyl und Thiomorpholinyl, die jeweils wahlweise substituiert sein können mit einem, zwei oder drei Substituenten, unabhängig gewählt aus der Gruppe bestehend aus Alkoxy, Alkoxycarbonyl, Alkenyl, Alkyl, Alkylcarbonyl, Aryl, Carboxy, Carboxyalkyl, Heterocyclyl, Heterocyclylalkyl, Hydroxy, Hydroxyalkyl, -NR^{c}R^{d}, (NR^{c}R^{d}) Alkyl und (NR^{c}R^{d}) Carbonyl;
R¹³ ist gewählt aus der Gruppe bestehend aus Aryl, Cycloalkyl und Heterocyclyl;
X ist gewählt aus der Gruppe bestehend aus -O-, -NR¹⁴-, -C(O)-, -S-, -SO₂-, -(CH₂)ₙ-, -C(O)NR¹⁴-, -NR¹⁴C(O), -SO₂NR¹⁴-, -NR¹⁴SO₂, -O(CH₂)ₘ-, -(CH₂)ₘO-, -CH=CH- und -C=C-; worin R¹⁴ gewählt ist aus der Gruppe bestehend aus Wasserstoff, Alkenyl, Alkyl, Aminoalkyl und Hydroxyalkyl;
Y ist NH;
m ist 0-3 und
n ist 1-3;
oder worin
(b)
A¹ CH ist;
A² CH ist;
Y NH ist;
R⁴, R⁵ und R⁷ jeweils Wasserstoff sind;
R⁶ Phenyl ist, substituiert in der dritten Position mit Methoxy und substituiert in der vierten Position mit Alkoxycarbonyl, Alkoxycarbonylalkenyl, Alkylcarbonyl, Alkylsulfonyl, Cyano, Halo, Haloalkenyl, Hydroxy, Nitro, NH₂SO₂- oder NH₂CO- und entweder
(i) ist R² Wasserstoff und R³ ist - (CR⁹R¹⁰)C(O)NHR¹², worin R⁹ und R¹⁰ unabhängig gewählt sind aus der Gruppe bestehend aus Wasserstoff, Alkenyl, Alkyl, Aminoalkyl und Hydroxyalkyl, oder R⁹ und R¹⁰, zusammen mit dem Kohlenstoffatom, an welches sie gebunden sind, bilden eine Cycloalkylgruppe und R¹² ist Phenyl wahlweise substituiert mit einer Morpholinylgruppe;
(ii) R² ist Wasserstoff und R³ ist Hydroxyalkyl;
(iii) R² ist Wasserstoff und R³ ist Alkoxy;
(iv) R² und R³ sind unabhängig - (CR⁹R¹⁰)C(O)NHR¹², worin R⁹ und R¹⁰ unabhängig gewählt sind aus der Gruppe bestehend aus Wasserstoff, Alkenyl, Alkyl, Aminoalkyl und Hydroxyalkyl, oder R⁹ und R¹⁰, zusammen mit dem Kohlenstoffatom, an welches sie gebunden sind, bilden eine Cycloalkylgruppe und R¹² ist Phenyl, wahlweise substituiert mit einer Morpholinylgruppe;
(v) R² und R³ sind unabhängig Hydroxyalkyl; oder
(vi) R² und R³ sind unabhängig Alkoxy;
worin
Alkyl ein C₁-C₁₀-Alkyl ist;
Alkenyl ein C₂-C₆-Alkenyl ist;
Alkoxy ein C₁-C₁₀-Alkoxy ist;
Aryl ist eine Phenylgruppe oder ein bizyklisches oder trizyklisches ankondensiertes Ringsystem, worin ein oder mehrere der ankondensierten Ringe eine Phenylgruppe ist, worin Aryl wahlweise substituiert sein kann mit ein, zwei, drei, vier oder fünf Substituenten, unabhängig gewählt aus der Gruppe bestehend aus Alkoxy, Alkoxyalkyl, Alkoxycarbonyl, Alkoxycarbonylalkenyl, Alkoxycarbonylalkyl, Alkoxysulfonyl, Alkyl, Alkylcarbonyl, Alkylsulfonyl, Amino, Aminocarbonyl, Aminosulfonyl, einer zweiten Arylgruppe, Arylalkyl, Arylsulfonyl, Carboxy, Cyano, Formyl, Halo, Haloalkoxy, Haloalkenyl, Haloalkyl, Heterocyclyl, Heterocyclylalkyl, Hydroxy, Hydroxyalkyl, Nitro, (NR^{c}R^{d})Alkyl und Oxo, worin die zweite Arylgruppe, der Arylteil von Arylalkyl und dem Arylsulfonyl, das Heterocyclyl und der Heterocyclylteil von Heterocyclylalkyl wahlweise substituiert sein können mit einem, zwei, drei, vier oder fünf Substituenten, unabhängig gewählt aus der Gruppe bestehend aus Alkoxy, Alkoxyalkyl, Alkoxycarbonyl, Alkyl, Alkylcarbonyl, Carboxy, Cyano, Halo, Haloalkoxy, Haloalkyl, Hydroxy, Nitro und Oxo;
Heterocyclyl ist gewählt aus einem fünf-, sechs- oder siebengliedrigen Ring, der ein, zwei oder drei Heteroatome enthält, unabhängig gewählt aus der Gruppe bestehend aus Stickstoff, Sauerstoff und Schwefel, worin der fünfgliedrige Ring null bis zwei Doppelbindungen hat und der sechs- und siebengliedrige Ring null bis drei Doppelbindungen hat; bizyklischen Gruppen, in welchen der Heterocyclylring ankondensiert ist an eine Phenylgruppe, eine monozyklische Cycloalkenylgruppe, eine monozyklische Cycloalkylgruppe oder eine andere monozyklische Heterocyclylgruppe; und trizyklischen Gruppen, in welchen ein bizyklisches System ankondensiert ist an eine Phenylgruppe, eine monozyklische Cycloalkenylgruppe, eine monozyklische Cycloalkylgruppe oder eine andere monozyklische Heterocyclylgruppe; worin das Heterocyclyl wahlweise substituiert sein kann mit einem, zwei, drei, vier oder fünf Substituenten, unabhängig gewählt aus der Gruppe bestehend aus Alkoxy, Alkoxyalkyl, Alkoxycarbonyl, Alkyl, Alkylcarbonyl, Alkylsulfonyl, Amino, Aminocarbonyl, Aminosulfonyl, Aryl, Arylalkyl, Arylsulfonyl, Carboxy, Cyano, Halo, einer zweiten Heterocyclylgruppe, Heterocyclylalkyl, Hydroxy, Hydroxyalkyl, Nitro, -NR^{c}R^{d}, (NR^{c}R^{d}) Alkyl und Oxo, worin das Aryl, der Arylteil von Arylalkyl und dem Arylsulfonyl, die zweite Heterocyclylgruppe und der Heterocyclylteil von Heterocyclylalkyl wahlweise substituiert sein können mit einem, zwei, drei, vier oder fünf Substituenten, unabhängig gewählt aus der Gruppe bestehend aus Alkoxy, Alkoxyalkyl, Alkoxycarbonyl, Alkyl, Alkylcarbonyl, Carboxy, Cyano, Halo, Haloalkoxy, Haloalkyl, Hydroxy, Methylendioxy, Nitro und Oxo;
Cycloalkyl ist ein gesättigtes monozyklisches, bizyklisches oder trizyklisches Kohlenwasserstoffringsystem, das drei bis zwölf Kohlenstoffatome hat, worin Cycloalkyl wahlweise substituiert sein kann mit einem, zwei, drei oder vier Substituenten, unabhängig gewählt aus der Gruppe bestehend aus Alkoxy, Alkoxyalkyl, Alkoxycarbonyl, Alkyl, Alkylcarbonyl, Alkylsulfonyl, -NR^{c}R^{d}, (NR^{c}R^{d}) Alkyl, (NR^{c}R^{d}) Carbonyl, einer zweiten Arylgruppe, Arylalkyl, Arylsulfonyl, Carboxy, Cyano, Halo, Heterocyclyl, Heterocyclylalkyl, Hydroxy, Hydroxyalkyl, Nitro und Oxo, worin die zweite Arylgruppe, der Arylteil von Arylalkyl und dem Arylsulfonyl, das Heterocyclyl und der Heterocyclylteil von Heterocyclylalkyl wahlweise substituiert sein können mit einem, zwei, drei, vier oder fünf Substituenten, unabhängig gewählt aus der Gruppe bestehend aus Alkoxy, Alkoxyalkyl, Alkoxycarbonyl, Alkyl, Alkylcarbonyl, Carboxy, Cyano, Halo, Haloalkoxy, Haloalkyl, Hydroxy, Nitro und Oxo;
Cycloalkenyl ist ein nicht aromatisches zyklisches oder bizyklisches Ringsystem, das drei bis zehn Kohlenstoffatome und und ein bis drei Ringe hat, worin jeder fünfgliedrige Ring eine Doppelbindung hat, jeder sechsgliedrige Ring eine oder zwei Doppelbindungen hat, jeder sieben- und achtgliedrige Ring eine bis drei Doppelbindungen hat und jeder neun- bis zehngliedrige Ring eine bis vier Doppelbindungen hat;
Amino bezeichnet -NR^{a}R^{b}, worin R^{a} und R^{b} unabhängig gewählt sind aus der Gruppe bestehend aus Wasserstoff, Alkoxyalkylcarbonyl, Alkoxycarbonyl, Alkyl, Alkylcarbonyl, Alkylsulfonyl, Aryl, Arylalkyl, Arylalkylcarbonyl, Arylalkylsulfonyl, Arylsulfonyl, Arylsulfonylalkyl, Arylsulfonylalkylcarbonyl, Cycloalkyl, Cycloalkylalkyl, Cycloalkylcarbonyl, Haloalkylsulfonyl, Heterocyclyl, Heterocyclylalkylcarbonyl, Heterocyclylalkylsulfonyl, Heterocyclylcarbonyl, Heterocyclylsulfonyl, -NR^{c}R^{d}, (NR^{c}R^{d}) Alkyl, (NR^{c}R^{d}) Alkylcarbonyl, (NR^{c}R^{d}) Alkylsulfonyl, (NR^{c}R^{d}) Carbonyl, (NR^{c}R^{d}) Carbonylalkyl und (NR^{c}R^{d}) Cabonylalkylcarbonyl, worin Aryl wahlweise substituiert sein kann mit einem, zwei, drei, vier oder fünf Substituenten, unabhängig gewählt aus der Gruppe bestehend aus Alkoxy, Alkoxyalkyl, Alkoxycarbonyl, Alkyl, Alkylcarbonyl, Alkylsulfonyl, einer zweiten Arylgruppe, Arylalkyl, Arylsulfonyl, Carboxy, Cyano, Halo, Heterocyclyl, Heterocyclylalkyl, Hydroxy, Nitro und Oxo, worin die zweite Arylgruppe, der Arylteil von Arylalkyl und dem Arylsulfonyl, das Heterocyclyl und der Heterocyclylteil von Heterocyclylalkyl wahlweise substituiert sein können mit einem, zwei, drei, vier oder fünf Substituenten, unabhängig gewählt aus der Gruppe bestehend aus Alkoxy, Alkoxyalkyl, Alkoxycarbonyl, Alkyl, Alkylcarbonyl, Carboxy, Cyano, Halo, Haloalkoxy, Haloalkyl, Hydroxy, Nitro und Oxo; und
R^{c} und R^{d} sind jeweils unabhängig Wasserstoff, Alkoxycarbonyl, Alkyl, Alkylcarbonyl, Aryl, Arylsulfonyl, Heterocyclcarbonyl oder Formyl;
vorausgesetzt, dass 3-Chlor-11-oxo-10,11-dihydro-5H-dibenzo[b,e][1,4]diazepin und 3-Fluor-11-oxo-10,11-dihydro-5H-dibenzo[b,e][1,4]diazepin ausgeschlossen sind.

2. Die Verbindung gemäß Anspruch 1, Teil (a), worin R² Wasserstoff ist.

3. Die Verbindung gemäß Anspruch 1, Teil (a), worin R² gewählt ist aus der Gruppe bestehend aus Alkoxycarbonyl und Alkoxycarbonylalkyl.

4. Die Verbindung gemäß Anspruch 1, Teil (a), worin R² gewählt ist aus der Gruppe bestehend aus Amino und Aryl.

5. Die Verbindung gemäß Anspruch 1, Teil (a), worin R² gewählt ist aus der Gruppe bestehend aus Carboxy, Carboxyalkyl, Cyano, Nitro und Heterocyclyl.

6. Die Verbindung gemäß Anspruch 1, Teil (a), worin R²-(CR⁹R¹⁰)ₘC(O)NR¹¹R¹² ist.

7. Die Verbindung gemäß Anspruch 6, worin m 0 ist.

8. Die Verbindung gemäß Anspruch 7, worin eins von R¹¹ und R¹² Wassserstoff ist und das andere Heterocyclylalkyl ist.

9. Die Verbindung gemäß Anspruch 7, worin eins von R¹¹ und R¹² Wasserstoff ist und das andere gewählt ist aus der Gruppe bestehend aus Amino, Aminoalkyl, Arylalkyl und Hydroxyalkyl.

10. Die Verbindung gemäß Anspruch 6, worin R¹¹ und R¹² zusammen mit dem Stickstoffatom, an welches sie gebunden sind, einen Heterocyclylring bilden, gewählt aus der Gruppe bestehend aus Diazepanyl, Imidazolidinyl, Morpholinyl, Piperazinyl, Piperidinyl und Pyrrolidinyl, welche jeweils wahlweise substituiert sein können mit einem, zwei oder drei Substituenten, unabhängig gewählt aus der Gruppe bestehend aus Alkoxy, Alkoxycarbonyl, Alkenyl, Alkyl, Alkylcarbonyl, Aryl, Carboxy, Carboxy, Heterocyclyl, Heterocyclylalkyl, Hydroxy und Hydroxyalkyl.

11. Die Verbindung gemäß Anspruch 6, worin m 1 ist.

12. Die Verbindung gemäß Anspruch 11, worin eins von R¹¹ und R¹² gewählt ist aus der Gruppe bestehend aus Wasserstoff und Alkyl und das andere Heterocyclylalkyl ist.

13. Die Verbindung gemäß Anspruch 11, worin eins von R¹¹ und R¹² gewählt ist aus der Gruppe bestehend aus Wasserstoff und Alkyl und das andere gewählt ist aus der Gruppe bestehend aus Alkoxyalkyl und Hydroxyalkyl.

14. Die Verbindung gemäß Anspruch 11, worin eins von R¹¹ und R¹² gewählt ist aus der Gruppe bestehend aus Wasserstoff und Alkyl und das andere gewählt ist aus der Gruppe bestehend aus Alkyl, Aminoalkyl, Aryl, Arylalkyl und Heterocyclyl.

15. Die Verbindung gemäß Anspruch 11, worin R¹¹ und R¹², zusammen mit dem Stickstoffatom, an welches sie gebunden sind, einen Heterocyclylring bilden, gewählt aus der Gruppe bestehend aus Diazepanyl, Imidazolidinyl, Morpholinyl, Piperazinyl, Piperidinyl und Pyrrolidinyl, welche jeweils wahlweise substituiert sein können mit einem, zwei oder drei Substituenten, unabhängig gewählt aus der Gruppe bestehend aus Alkoxy, Alkoxycarbonyl, Alkenyl, Alkyl, Alkylcarbonyl, Aryl, Carboxy, Carboxy, Heterocyclyl, Heterocyclylalkyl, Hydroxy und Hydroxyalkyl.

16. Die Verbindung gemäß Anspruch 1, gewählt aus der Gruppe bestehend aus
3-(4-Hydroxy-3-methoxyphenyl)-5,10-dihydro-11H-dibenzo[b,e] [1,4]diazepin-11-on;
3-(11-Oxo-10,11-dihydro-5H-dibenzo[b,e][1,4]diazepin-3-yl)benzonitril;
3-(3-Methoxy-4-nitrophenyl)-5,10-dihydro-11H-dibenzo[b,e] [1,4]diazepin-11-on;
3-(4-Chlor-3-methoxyphenyl)-5,10-dihydro-11H-dibenzo[b,e] [1,4]diazepin-11-on;
3-(4-Brom-3-methoxyphenyl)-5,10-dihydro-11H-dibenzo[b,e][1,4] diazepin-11-on;
3-(4-Acetyl-3-methoxyphenyl)-5,10-dihydro-11H-dibenzo[b,e][1,4] diazepin-11-on;
2-Methoxy-4-(11-oxo-10,11-dihydro-5H-dibenzo[b,e][1,4]diazepin-3-yl)benzonitril;
Methyl 2-methoxy-4-(11-oxo-10,11-dihydro-5H-dibenzo[b,e] [1,4]diazepin-3-yl)benzoat;
2-Methoxy-4-(11-oxo-10,11-dihydro-5H-dibenzo[b,e][1,4]diazepin-3-yl)benzoesäure;
N-(3,4-Dihydroxybenzyl)-2-methoxy-4-(11-oxo-10,11-dihydro-5H-dibenzo[b,e][1,4]diazepin-3-yl)benzamid;
2-Methoxy-4-(11-oxo-10,11-dihydro-5H-dibenzo[b,e][1,4]diazepin-3-yl)benzamid;
3-(2-Methoxy-4-pyridinyl)-5,10-dihydro-11H-dibenzo[b,e] [1,4]diazepin-11-on;
Methyl 3-(4-hydroxy-3-methoxyphenyl)-11-oxo-10,11-dihydro-5H-dibenzo[b,e][1,4]diazepin-8-carboxylat;
Methyl [3-(3-methoxy-4-nitrophenyl)-11-oxo-10,11-dihydro-5H-dibenzo[b,e][1,4]diazepin-8-yl]acetat;
Methyl [3-(4-hydroxy-3-methoxyphenyl)-11-oxo-10,11-dihydro-5H-dibenzo[b,e][1,4]diazepin-8-yl]acetat;
Methyl [3-(3-methoxyphenyl)-11-oxo-10,11-dihydro-5H-dibenzo[b,e] [1,4]diazepin-8-yl]acetat;
Methyl [3-(4-chlor-3-methoxyphenyl)-11-oxo-10,11-dihydro-5H-dibenzo[b,e][1,4]diazepin-8-yl]acetat;
Methyl [3-(4-brom-3-methoxyphenyl)-11-oxo-10,11-dihydro-5H-dibenzo[b,e][1,4]diazepin-8-yl]acetat;
Methyl [3-(4-cyano-3-methoxyphenyl)-11-oxo-10,11-dihydro-5H-dibenzo[b,e][1,4]diazepin-8-yl]acetat;
Methyl [3-(5-methoxy-2-methyl-4-nitrophenyl)-11-oxo-10,11-dihydro-5H-dibenzo[b,e][1,4]diazepin-8-yl]acetat;
Methyl [3-(4-cyano-5-methoxy-2-methylphenyl)-11-oxo-10,11-dihydro-5H-dibenzo[b,e][1,4]diazepin-8-yl]acetat;
Methyl [3-(2-fluor-5-methyl-4-pyridinyl)-11-oxo-10,11-dihydro-5H-dibenzo[b,e][1,4]diazepin-8-yl]acetat;
Methyl [3-(2-methoxy-5-methyl-4-pyridinyl)-11-oxo-10,11-dihydro-5H-dibenzo[b,e][1,4]diazepin-8-yl]acetat;
8-Amino-3-(4-hydroxy-3-methoxyphenyl)-5,10-dihydro-11H-dibenzo[b,e][1,4]diazepin-11-on;
N-[3-(4-Hydroxy-3-methoxyphenyl)-11-oxo-10,11-dihydro-5H-dibenzo[b,e][1,4]diazepin-8-yl]methansulfonamid;
N-[3-(3-Methoxy-4-nitrophenyl)-11-oxo-10,11-dihydro-5H-dibenzo[b,e][1,4]diazepin-8-yl]methansulfonamid;
N-[3-(4-Hydroxy-3-methoxyphenyl)-11-oxo-10,11-dihydro-5H-dibenzo[b,e][1,4]diazepin-8-yl]acetamid;
N-[3-(3-Methoxy-4-nitrophenyl)-11-oxo-10,11-dihydro-5H-dibenzo[b,e][1,4]diazepin-8-yl]acetamid;
8-(3-Aminophenyl)-3-(4-hydroxy-3-methoxyphenyl)-5,10-dihydro-11H-dibenzo[b,e][1,4]diazepin-11-on;
3-(4-Hydroxy-3-methoxyphenyl)-8-(3-hydroxyphenyl)-5,10-dihydro-11H-dibenzo[b,e][1,4]diazepin-11-on und
8-Amino-3-(3-methoxy-4-nitrophenyl)-5,10-dihydro-11H-dibenzo[b,e][1,4]diazepin-11-on;
3-(4-Hydroxy-3-methoxyphenyl)-11-oxo-10,11-dihydro-5H-dibenzo[b,e][1,4]diazepin-8-carbonsäure;
3-(4-Hydroxy-3-methoxyphenyl)-11-oxo-10,11-dihydro-5H-dibenzo[b,e][1,4]diazepin-8-carbonitril;
3-(4-Hydroxy-3-methoxyphenyl)-8-nitro-5,10-dihydro-11H-dibenzo[b,e][1,4]diazepin-11-on;
3-(4-Hydroxy-3-methoxyphenyl)-8-(3-pyridinyl)-5,10-dihydro-11H-dibenzo[b,e][1,4]diazepin-11-on;
3-(4-Hydroxy-3-methoxyphenyl)-8-(1H-pyrrol-2-yl)-5,10-dihydro-11H-dibenzo[b,e][1,4]diazepin-11-on;
3-(3-Methoxy-4-nitrophenyl)-8-(1H-pyrrol-2-yl)-5,10-dihydro-11H-dibenzo[b,e][1,4]diazepin-11-on;
[3-(3-Methoxy-4-nitrophenyl)-11-oxo-10,11-dihydro-5H-dibenzo[b,e][1,4]diazepin-8-yl]essigsäure;
3-(4-Hydroxy-3-methoxyphenyl)-11-oxoN[3-(1-pyrrolidinyl)propyl]-10,11-dihydro-5H-dibenzo[b,e][1,4]diazepin-8-carboxamid;
3-(4-Hydroxy-3-methoxyphenyl)N[3-(4-morpholinyl)propyl]-11-oxo-10,11-dihydro-5H-dibenzo[b,e][1,4]diazepin-8-carboxamid;
3-(4-Hydroxy-3-methoxyphenyl)-11-oxoN[3-(2-oxo-1-pyrrolidinyl)propyl]-10,11-dihydro-5H-dibenzo[b,e][1,4]diazepin-8-carboxamid;
3-(4-Hydroxy-3-methoxyphenyl)-11-oxoN(3-pyridinylmethyl)-10,11-dihydro-5H-dibenzo[b,e][1,4]diazepin-8-carboxamid;
3-(4-Hydroxy-3-methoxyphenyl)-11-oxoN(2-pyridinylmethyl)-10,11-dihydro-5H-dibenzo[b,e][1,4]diazepin-8-carboxamid;
3-(4-Hydroxy-3-methoxyphenyl)-11-oxoN(4-pyridinylmethyl)-10,11-dihydro-5H-dibenzo[b,e][1,4]diazepin-8-carboxamid;
3-(2-Methoxy-4-pyridinyl)-11-oxoN[3-(1-pyrrolidinyl)propyl]-10,11-dihydro-5H-dibenzo[b,e][1,4]diazepin-8-carboxamid;
11-Oxo-3-(2-oxo-1,2-dihydro-4-pyridinyl)N[3-(1-pyrrolidinyl)propyl]-10,11-dihydro-5H-dibenzo[b,e][1,4]diazepin-8-carboxamid;
N-[3-(Dimethylamino)propyl]-3-(4-hydroxy-3-methoxyphenyl)-11-oxo-10,11-dihydro-5H-dibenzo[b,e][1,4]diazepin-8-carboxamid;
N-(2-Hydroxyethyl)-3-(4-hydroxy-3-methoxyphenyl)-11-oxo-10,11-dihydro-5H-dibenzo[b,e][1,4]diazepin-8-carboxamid;
N-(2,3-Dihydroxypropyl)-3-(4-hydroxy-3-methoxyphenyl)-11-oxo-10,11-dihydro-5H-dibenzo[b,e][1,4]diazepin-8-carboxamid;
N-[2-(Acetylamino)ethyl]-3-(4-hydroxy-3-methoxyphenyl)-11-oxo-10,11-dihydro-5H-dibenzo[b,e][1,4]diazepin-8-carboxamid;
3-(4-Hydroxy-3-methoxyphenyl)N[4-(methylsulfonyl)benzyl]-11-oxo-10,11-dihydro-5H-dibenzo[b,e][1,4]diazepin-8-carboxamid;
N-(2-Fluorbenzyl)-3-(4-hydroxy-3-methoxyphenyl)-11-oxo-10,11-dihydro-5H-dibenzo[b,e][1,4]diazepin-8-carboxamid;
3-(4-Hydroxy-3-methoxyphenyl)N(2-methoxybenzyl)-11-oxo-10,11-dihydro-5H-dibenzo[b,e][1,4]diazepin-8-carboxamid;
3-(4-Hydroxy-3-methoxyphenyl)N[2-(4-methoxyphenyl)ethyl]-11-oxo-10,11-dihydro-5H-dibenzo[b,e][1,4]diazepin-8-carboxamid;
tert-Butyl 2-{[3-(4-hydroxy-3-methoxyphenyl)-11-oxo-10,11-dihydro-5H-dibenzo[b,e][1,4]diazepin-8-yl]carbonyl)hydrazincarboxylat;
3-(4-Hydroxy-3-methoxyphenyl)-8-[(3-hydroxy-1-pyrrolidinyl)carbonyl]-5,10-dihydro-11H-dibenzo[b,e][1,4] diazepin-11-on;
3-(4-Hydroxy-3-methoxyphenyl)-8-{[(2S)-2-(hydroxymethyl)-1-pyrrolidinyl]carbonyl}-5,10-dihydro-11H-dibenzo[b,e] [1,4]diazepin-11-on;
3-(4-Hydroxy-3-methoxyphenyl)-8-{[2-(hydroxymethyl)-1-piperidinyl]carbonyl}-5,10-dihydro-11H-dibenzo[b,e] [1,4]diazepin-11-on;
Ethyl 1-{[3-(4-hydroxy-3-methoxyphenyl)-11-oxo-10,11-dihydro-5H-dibenzo[b,e][1,4]diazepin-8-yl]carbonyl}-2-piperidincarboxylat;
Ethyl 1-{[3-(4-hydroxy-3-methoxyphenyl)-11-oxo-10,11-dihydro-5H-dibenzo[b,e][1,4]diazepin-8-yl]carbonyl}-3-piperidincarboxylat;
Ethyl 1-{[3-(4-hydroxy-3-methoxyphenyl)-11-oxo-10,11-dihydro-5H-dibenzo[b,e][1,4]diazepin-8-yl]carbonyl}-4-piperidincarboxylat;
3-(4-Hydroxy-3-methoxyphenyl)-8-[(3-hydroxy-1-piperidinyl)carbonyl]-5,10-dihydro-11H-dibenzo[b,e] [1,4]diazepin-11-on;
1-{[3-(4-Hydroxy-3-methoxyphenyl)-11-oxo-10,11-dihydro-5H-dibenzo[b,e][1,4]diazepin-8-yl]carbonyl}-2-piperidincarbonsäure;
1-{[3-(4-Hydroxy-3-methoxyphenyl)-11-oxo-10,11-dihydro-5H-dibenzo[b,e][1,4]diazepin-8-yl]carbonyl}-3-piperidincarbonsäure;
1-{[3-(4-Hydroxy-3-methoxyphenyl)-11-oxo-10,11-dihydro-5H-dibenzo[b,e][1,4]diazepin-8-yl]carbonyl}-4-piperidincarbonsäure;
2-[3-(3-Methoxy-4-nitrophenyl)-11-oxo-10,11-dihydro-5H-dibenzo[b,e][1,4]diazepin-8-yl]N[3-(1-pyrrolidinyl)propyl]acetamid;
2-[3-(3-Methoxy-4-nitrophenyl)-11-oxo-10,11-dihydro-5H-dibenzo[b,e][1,4]diazepin-8-yl]N[2-(2-pyridinyl)ethyl]acetamid;
2-[3-(3-Methoxy-4-nitrophenyl)-11-oxo-10,11-dihydro-5H-dibenzo[b,e][1,4]diazepin-8-yl]N[2-(3-pyridinyl)ethyl]acetamid;
2-[3-(3-Methoxy-4-nitro-phenyl)-11-oxo-10,11-dihydro-5H-dibenzo[b,e][1,4]diazepin-8-yl]N(4-pyridin-2-yl-ethyl)-acetamid;
N-[3-(1H-Imidazol-1-yl)propyl]-2[3-(3-methoxy-4-nitrophenyl)-11-oxo-10,11-dihydro-5H-dibenzo[b,e][1,4]diazepin-8-yl]acetamid;
2-[3-(3-Methoxy-4-nitrophenyl)-11-oxo-10,11-dihydro-5H-dibenzo[b,e][1,4]diazepin-8-yl]NmethylN[2-(2-pyridinyl)ethyl]acetamid;
2-[3-(3-Methoxy-4-nitrophenyl)-11-oxo-10,11-dihydro-5H-dibenzo[b,e][1,4]diazepin-8-yl]N(3-pyridinylmethyl)acetamid;
2-[3-(3-Methoxy-4-nitrophenyl)-11-oxo-10,11-dihydro-5H-dibenzo[b,e][1,4]diazepin-8-yl]N(4-pyridinylmethyl)acetamid;
2-[3-(3-Methoxy-4-nitrophenyl)-11-oxo-10,11-dihydro-5H-dibenzo[b,e][1,4]diazepin-8-yl]N(2-pyridinylmethyl)acetamid;
2-[3-(3-Methoxy-4-nitrophenyl)-11-oxo-10,11-dihydro-5H-dibenzo[b,e][1,4]diazepin-8-yl]N[2-(1-pyrrolidinyl)ethyl]acetamid;
2-[3-(3-Methoxy-4-nitrophenyl)-11-oxo-10,11-dihydro-5H-dibenzo[b,e][1,4]diazepin-8-yl]N[2-(1-piperidinyl)ethyl]acetamid;
2-[3-(3-Methoxy-4-nitrophenyl)-11-oxo-10,11-dihydro-5H-dibenzo[b,e][1,4]diazepin-8-yl]N[3-(1-piperidinyl)propyl]acetamid;
2-[3-(3-Methoxy-4-nitrophenyl)-11-oxo-10,11-dihydro-5H-dibenzo[b,e][1,4]diazepin-8-yl]N[2-(4-morpholinyl)ethyl]acetamid;
2-[3-(3-Methoxy-4-nitrophenyl)-11-oxo-10,11-dihydro-5H-dibenzo[b,e][1,4]diazepin-8-yl]N[3-(4-morpholinyl)propyl]acetamid;
2-[3-(3-Methoxy-4-nitrophenyl)-11-oxo-10,11-dihydro-5H-dibenzo[b,e][1,4]diazepin-8-yl]N[2-(4-methyl-1-piperazinyl)ethyl]acetamid;
N-[(1S)-1-(Hydroxymethyl)-2-methylpropyl]-2-[3-(3-methoxy-4-nitrophenyl)-11-oxo-10,11-dihydro-5H-dibenzo[b,e][1,4]diazepin-8-yl]acetamid;
N-[(1R)-1-(Hydroxymethyl)-2-methylpropyl]-2-[3-(3-methoxy-4-nitrophenyl)-11-oxo-10,11-dihydro-5H-dibenzo[b,e][1,4]diazepin-8-yl]acetamid;
N-(3-Ethoxypropyl)-2-[3-(3-methoxy-4-nitrophenyl)-11-oxo-10,11-dihydro-5H-dibenzo[b,e][1,4]diazepin-8-yl]acetamid;
N-[(1R)-1-(Hydroxymethyl)-3-methylbutyl]-2-[3-(3-methoxy-4-nitrophenyl)-11-oxo-10,11-dihydro-5H-dibenzo[b,e][1,4]diazepin-8-yl]acetamid;
N-[(1S)-1-(Hydroxymethyl)-3-methylbutyl]-2-[3-(3-methoxy-4-nitrophenyl)-11-oxo-10,11-dihydro-5H-dibenzo[b,e][1,4]diazepin-8-yl]acetamid;
N-(3-Hydroxypropyl)-2-[3-(3-methoxy-4-nitrophenyl)-11-oxo-10,11-dihydro-5H-dibenzo[b,e][1,4]diazepin-8-yl]acetamid;
N-[(1S)-2-Hydroxy-1-phenylethyl]-2-[3-(3-methoxy-4-nitrophenyl)-11-oxo-10,11-dihydro-5H-dibenzo[b,e][1,4]diazepin-8-yl]acetamid;
N-[(1R)-2-Hydroxy-1-phenylethyl]-2-[3-(3-methoxy-4-nitrophenyl)-11-oxo-10,11-dihydro-5H-dibenzo[b,e][1,4]diazepin-8-yl]acetamid;
N-(4-Hydroxybutyl)-2-[3-(3-methoxy-4-nitrophenyl)-11-oxo-10,11-dihydro-5H-dibenzo[b,e][1,4]diazepin-8-yl]acetamid;
N-(2-Hydroxyethyl)-2-[3-(3-methoxy-4-nitrophenyl)-11-oxo-10,11-dihydro-5H-dibenzo[b,e][1,4]diazepin-8-yl]Npropylacetamid;
N-[2-(Dimethylamino)ethy]]-2-[3-(3-methoxy-4-nitrophenyl)-11-oxo-10,11-dihydro-5H-dibenzo[b,e][1,4]diazepin-8-yl]Nmethylacetamid;
2-[3-(3-Methoxy-4-nitrophenyl)-11-oxo-10,11-dihydro-5H-dibenzo[b,e][1,4]diazepin-8-yl]-N,N-dimethylacetamid;
2-[3-(3-Methoxy-4-nitrophenyl)-11-oxo-10,11-dihydro-5H-dibenzo[b,e][1,4]diazepin-8-yl]N3-chinolinylacetamid;
2-[3-(3-Methoxy-4-nitrophenyl)-11-oxo-10,11-dihydro-5H-dibenzo[b,e][1,4]diazepin-8-yl]N6-chinolinylacetamid;
2-[3-(3-Methoxy-4-nitrophenyl)-11-oxo-10,11-dihydro-5H-dibenzo [b,e][1,4]diazepin-8-yl]N[4-(4-morpholinyl)phenyl]acetamid;
N-[2-(Diethylamino)ethyl]-2-[3-(3-methoxy-4-nitrophenyl)-11-oxo-10,11-dihydro-5H-dibenzo[b,e][1,4]diazepin-8-yl]acetamid;
N-[3-(Diethylamino)propyl]-2-[3-(3-methoxy-4-nitrophenyl)-11-oxo-10,11-dihydro-5H-dibenzo[b,e][1,4]diazepin-8-yl]acetamid;
N-(3,4-Difluorbenzyl)-2-[3-(3-methoxy-4-nitrophenyl)-11-oxo-10,11-dihydro-5H-dibenzo[b,e][1,4]diazepin-8-yl]acetamid;
N-(1-Benzyl-4-piperidinyl)-2-[3-(3-methoxy-4-nitrophenyl)-11-oxo-10,11-dihydro-5H-dibenzo[b,e][1,4]diazepin-8-yl]acetamid;
N-[4-(Dimethylamino)butyl]-2-[3-(3-methoxy-4-nitrophenyl)-11-oxo-10,11-dihydro-5H-dibenzo[b,e][1,4]diazepin-8-yl]acetamid;
N-{2-[4-(Aminosulfonyl)phenyl]ethyl}-2-[3-(3-methoxy-4-nitrophenyl)-11-oxo-10,11-dihydro-5H-dibenzo[b,e][1,4]diazepin-8-yl]acetamid;
N-[2-(Dimethylamino)ethyl]-2-[3-(3-methoxy-4-nitrophenyl)-11-oxo-10,11-dihydro-5H-dibenzo[b,e][1,4]diazepin-8-yl]acetamid;
N-[3-(Dimethylamino)propyl]-2-[3-(3-methoxy-4-nitrophenyl)-11-oxo-10,11-dihydro-5H-dibenzo[b,e][1,4]diazepin-8-yl]acetamid;
8-[2-(3-Hydroxy-1-piperidinyl)-2-oxoethyl]-3-(3-methoxy-4-nitrophenyl)-5,10-dihydro-11H-dibenzo[b,e][1,4]diazepin-11-on;
3-(3-Methoxy-4-nitrophenyl)-8-[2-(4-methyl-1,4-diazepan-1-yl)-2-oxoethyl]-5,10-dihydro-11H-dibenzo[b,e][1,4]diazepin-11-on;
8-[2-(4-Hydroxy-1-piperidinyl)-2-oxoethyl]-3-(3-methoxy-4-nitrophenyl)-5,10-dihydro-11H-dibenzo[b,e][1,4-]diazepin-11-on;
8-[2-(4-Ethyl-1-piperazinyl)-2-oxoethyl]-3-(3-methoxy-4-nitrophenyl)-5,10-dihydro-11H-dibenzo[b,e][1,4]diazepin-11-on;
8-{2-[4-(2-Hydroxyethyl)-1-piperazinyl]-2-oxoethyl}-3-(3-methoxy-4-nitrophenyl)-5,10-dihydro-11H-dibenzo[b,e] [1,4]diazepin-11-on;
3-(3-Methoxy-4-nitrophenyl)-8-[2-oxo-2-(4-phenyl-1-piperazinyl)ethyl]-5,10-dihydro-11H-dibenzo[b,e][1,4]diazepin-11-on;
3-(3-Methoxy-4-nitrophenyl)-8-{2-oxo-2-[4-(2-pyridinyl)-1-piperazinyl]ethyl}-5,10-dihydro-11H-dibenzo[b,e][1,4]diazepin-11-on;
8-{2-[(2S)-2-(Hydroxymethyl)-1-pyrrolidinyl]-2-oxoethyl}-3-(3-methoxy-4-nitrophenyl)-5,10-dihydro-11H-dibenzo[b,e] [1,4]diazepin-11-on;
N-[3-(3-Methoxy-4-nitrophenyl)-11-oxo-10,11-dihydro-5H-dibenzo[b,e][1,4]diazepin-8-yl]harnstoff;
2-(Dimethylamino)N[3-(3-methoxy-4-nitrophenyl)-11-oxo-10,11-dihydro-5H-dibenzo[b,e][1,4]diazepin-8-yl]acetamid;
(2S)-2-AminoN[3-(3-methoxy-4-nitrophenyl)-11-oxo-10,11-dihydro-5H-dibenzo[b,e][1,4]diazepin-8-yl]-4-methylpentanamid;
4-AminoN[3-(3-methoxy-4-nitrophenyl)-11-oxo-10,11-dihydro-5H-dibenzo[b,e][1,4]diazepin-8-yl]butyramid;
3-AminoN[3-(3-methoxy-4-nitrophenyl)-11-oxo-10,11-dihydro-5H-dibenzo[b,e][1,4]diazepin-8-yl]butyramid;
N-[3-(3-Methoxy-4-nitrophenyl)-11-oxo-10,11-dihydro-5H-dibenzo[b,e][1,4]diazepin-8-yl]-2-(3-methyl-3H-imidazol-4-yl)acetamid;
2-(3H-Imidazol-4-yl)N[3-(3-methoxy-4-nitrophenyl)-11-oxo-10,11-dihydro-5H-dibenzo[b,e][1,4]diazepin-8-yl]acetamid;
N-[3-(3-Methoxy-4-nitrophenyl)-11-oxo-10,11-dihydro-5H-dibenzo[b,e][1,4]diazepin-8-yl]thiophen-3-carboxamid;
N-[3-(3-Methoxy-4-nitrophenyl)-11-oxo-10,11-dihydro-5H-dibenzo[b,e][1,4]diazepin-8-yl]-1H-pyrrol-2-carboxamid;
N-[3-(3-Methoxy-4-nitrophenyl)-11-oxo-10,11-dihydro-5H-dibenzo[b,e][1,4]diazepin-8-yl]-2,5-dimethyl-1H-pyrrol-3-carboxamid;
N-[3-(3-Methoxy-4-nitrophenyl)-11-oxo-10,11-dihydro-5H-dibenzo[b,e][1,4]diazepin-8-yl]-1,3-thiazol-4-carboxamid;
N-[3-(3-Methoxy-4-nitrophenyl)-11-oxo-10,11-dihydro-5H-dibenzo[b,e][1,4]diazepin-8-yl]-1H-pyrazol-5-carboxamid;
N-[3-(3-Methoxy-4-nitrophenyl)-11-oxo-10,11-dihydro-5H-dibenzo[b,e][1,4]diazepin-8-yl]-1H-pyrazol-4-carboxamid;
N-[3-(3-Methoxy-4-nitrophenyl)-11-oxo-10,11-dihydro-5H-dibenzo[b,e][1,4]diazepin-8-yl]isonicotinamid;
N-[3-(3-Methoxy-4-nitrophenyl)-11-oxo-10,11-dihydro-5H-dibenzo[b,e][1,4]diazepin-8-yl]-3-pyrrolidin-1-ylpropanamid;
N-[3-(3-Methoxy-4-nitrophenyl)-11-oxo-10,11-dihydro-5H-dibenzo[b,e][1,4]diazepin-8-yl]-3-piperidin-1-ylpropanamid;
N-[3-(3-Methoxy-4-nitrophenyl)-11-oxo-10,11-dihydro-5H-dibenzo[b,e][1,4]diazepin-8-yl]-3-morpholin-4-ylpropanamid;
(2R)-2-HydroxyN[3-(3-methoxy-4-nitrophenyl)-11-oxo-10,11-dihydro-5H-dibenzo[b,e][1,4]diazepin-8-yl]-4-phenylbutanamid;
N-[3-(3-Methoxy-4-nitrophenyl)-11-oxo-10,11-dihydro-5H-dibenzo[b,e][1,4]diazepin-8-yl]-3-(phenylsulfonyl)propanamid;
N-[3-(3-Methoxy-4-nitrophenyl)-11-oxo-10,11-dihydro-5H-dibenzo[b,e][1,4]diazepin-8-yl]-2-{[(4-methylphenyl)sulfonyl]amino}acetamid;
N-[3-(3-Methoxy-4-nitrophenyl)-11-oxo-10,11-dihydro-5H-dibenzo[b,e][1,4]diazepin-8-yl]pyridin-2-carboxamid;
N-[3-(3-Methoxy-4-nitrophenyl)-11-oxo-10,11-dihydro-5H-dibenzo[b,e][1,4]diazepin-8-yl]nicotinamid;
N-[3-(3-Methoxy-4-nitrophenyl)-11-oxo-10,11-dihydro-5H-dibenzo[b,e][1,4]diazepin-8-yl]-2-pyridin-3-ylacetamid;
N-[3-(3-Methoxy-4-nitrophenyl)-11-oxo-10,11-dihydro-5H-dibenzo[b,e][1,4]diazepin-8-yl]-2-(4-methylpiperazin-1-yl)acetamid;
3-EthoxyN[3-(3-methoxy-4-nitrophenyl)-11-oxo-10,11-dihydro-5H-dibenzo[b,e][1,4]diazepin-8-yl]propanamid;
(2R)N[3-(3-Methoxy-4-nitrophenyl)-11-oxo-10,11-dihydro-5H-dibenzo[b,e][1,4]diazepin-8-yl]-5-oxopyrrolidin-2-carboxamid;
4-MethoxyN[3-(3-methoxy-4-nitrophenyl)-11-oxo-10,11-dihydro-5H-dibenzo[b,e][1,4]diazepin-8-yl]cyclohexancarboxamid;
(2R)-2-MethoxyN[3-(3-methoxy-4-nitrophenyl)-11-oxo-10,11-dihydro-5H-dibenzo[b,e][1,4]diazepin-8-yl]-2-phenylacetamid;
(2S)-2-MethoxyN[3-(3-methoxy-4-nitrophenyl)-11-oxo-10,11-dihydro-5H-dibenzo[b,e][1,4]diazepin-8-yl]-2-phenylacetamid;
N-(2-{[3-(3-Methoxy-4-nitrophenyl)-11-oxo-10,11-dihydro-5H-dibenzo[b,e][1,4]diazepin-8-yl]amino}-2-oxoethyl)-2-furamid;
1-AcetylN[3-(3-methoxy-4-nitrophenyl)-11-oxo-10,11-dihydro-5H-dibenzo[b,e][1,4]diazepin-8-yl]piperidin-4-carboxamid;
N-[3-(3-Methoxy-4-nitrophenyl)-11-oxo-10,11-dihydro-5H-dibenzo[b,e][1,4]diazepin-8-yl]-N'-phenylpentandiamid;
N-[3-(3-Methoxy-4-nitrophenyl)-11-oxo-10,11-dihydro-5H-dibenzo[b,e][1,4]diazepin-8-yl]-2-[4-(methylsulfonyl)phenyl]acetamid;
(2S)N[3-(3-Methoxy-4-nitrophenyl)-11-oxo-10,11-dihydro-5H-dibenzo[b,e][1,4]diazepin-8-yl]-5-oxopyrrolidin-2-carboxamid;
4-(8-Amino-11-oxo-10,11-dihydro-5H-dibenzo[b,e] [1,4]diazepin-3-yl)-2-methoxybenzonitril;
(2S)N[3-(4-Cyano-3-methoxyphenyl)-11-oxo-10,11-dihydro-5H-dibenzo[b,e][1,4]diazepin-8-yl]-2-methoxy-2-phenylacetamid;
N-[3-(4-Cyano-3-methoxyphenyl)-11-oxo-10,11-dihydro-5H-dibenzo[b,e][1,4]diazepin-8-yl]-3-piperidin-1-ylpropanamid;
N-[3-(4-Cyano-3-methoxyphenyl)-11-oxo-10,11-dihydro-5H-dibenzo[b,e][1,4]diazepin-8-yl]methansulfonamid;
8-Amino-3-(4-chlor-3-methoxyphenyl)-5,10-dihydro-11H-dibenzo[b,e][1,4]diazepin-11-on;
N-[3-(4-Chlor-3-methoxyphenyl)-11-oxo-10,11-dihydro-5H-dibenzo[b,e][1,4]diazepin-8-yl]-3-piperidin-1-ylpropanamid;
3-(3-Methoxy-4-nitrophenyl)-8-(2-oxopyrrolidin-1-yl)-5,10-dihydro-11H-dibenzo[b,e][1,4]diazepin-11-on;
3-(3-Methoxy-4-nitrophenyl)-8-(2-oxopiperidin-1-yl)-5,10-dihydro-11H-dibenzo[b,e][1,4]diazepin-11-on;
3-(4-Chlor-3-methoxyphenyl)-8-(2-oxopyrrolidin-1-yl)-5,10-dihydro-11H-dibenzo[b,e][1,4]diazepin-11-on;
3-ChlorN[3-(4-chlor-3-methoxyphenyl)-11-oxo-10,11-dihydro-5H-dibenzo[b,e][1,4]diazepin-8-yl]propan-1-sulfonamid;
N-[3-(4-Chlor-3-methoxyphenyl)-11-oxo-10,11-dihydro-5H-dibenzo[b,e][1,4]diazepin-8-yl]-1-phenylmethanesulfonamid;
3-(4-Chlor-3-methoxyphenyl)-8-(1,1-dioxidoisothiazolidin-2-yl)-5,10-dihydro-11H-dibenzo[b,e][1,4]diazepin-11-on;
2,2,2-TrifluorN[3-(3-methoxy-4-nitrophenyl)-11-oxo-10,11-dihydro-5H-dibenzo[b,e][1,4]diazepin-8-yl]ethansulfonamid;
N-[3-(3-Methoxy-4-nitrophenyl)-11-oxo-10,11-dihydro-5H-dibenzo[b,e][1,4]diazepin-8-yl]-1-methyl-1H-imidazol-4-sulfonamid;
N-[3-(3-Methoxy-4-nitrophenyl)-11-oxo-10,11-dihydro-5H-dibenzo[b,e][1,4]diazepin-8-yl]-1-phenylmethansulfonamid;
3-ChlorN[3-(3-methoxy-4-nitrophenyl)-11-oxo-10,11-dihydro-5H-dibenzo[b,e][1,4]diazepin-8-yl]propan-1-sulfonamid;
8-(1,1-Dioxidoisothiazolidin-2-yl)-3-(3-methoxy-4-nitrophenyl)-5,10-dihydro-11H-dibenzo[b,e][1,4]diazepin-11-on;
N-[3-(3-Methoxy-4-nitrophenyl)-11-oxo-10,11-dihydro-5H-dibenzo[b,e][1,4]diazepin-8-yl]-3-morpholin-4-ylpropan-1-sulfonamid;
N-[3-(3-Methoxy-4-nitrophenyl)-11-oxo-10,11-dihydro-5H-dibenzo[b,e][1,4]diazepin-8-yl]-3-piperidin-1-ylpropan-1-sulfonamid;
3-(Diethylamino)N[3-(3-methoxy-4-nitrophenyl)-11-oxo-10,11-dihydro-5H-dibenzo[b,e][1,4]diazepin-8-yl]propan-1-sulfonamid;
3-(Dimethylamino)N[3-(3-methoxy-4-nitrophenyl)-11-oxo-10,11-dihydro-5H-dibenzo[b,e][1,4]diazepin-8-yl]propan-1-sulfonamid;
1-ChlorN[3-(3-methoxy-4-nitrophenyl)-11-oxo-10,11-dihydro -5H-dibenzo[b,e][1,4]diazepin-8-yl]methansulfonamid;
N-[3-(3-Methoxy-4-nitrophenyl)-11-oxo-10,11-dihydro-5H-dibenzo[b,e][1,4]diazepin-8-yl]-4-morpholin-4-ylbenzamid;
7-Amino-3-(3-methoxy-4-nitrophenyl)-5,10-dihydro-11H-dibenzo[b,e][1,4]diazepin-11-on;
3-ChlorN[3-(3-methoxy-4-nitrophenyl)-11-oxo-10,11-dihydro-5H-dibenzo[b,e][1,4]diazepin-7-yl]propan-1-sulfonamid;
N-[3-(3-Methoxy-4-nitrophenyl)-11-oxo-10,11-dihydro-5H-dibenzo[b,e][1,4]diazepin-7-yl]-1-phenylmethansulfonamid;
1-(4-Chlorphenyl)N[3-(3-methoxy-4-nitrophenyl)-11-oxo-10,11-dihydro-5H-dibenzo[b,e][1,4]diazepin-7-yl]methansulfonamid;
N-[3-(3-Methoxy-4-nitrophenyl)-11-oxo-10,11-dihydro-5H-dibenzo[b,e][1,4]diazepin-7-yl]-1-methyl-1H-imidazol-4-sulfonamid;
N-[3-(3-Methoxy-4-nitrophenyl)-11-oxo-10,11-dihydro-5H-dibenzo[b,e][1,4]diazepin-7-yl]methansulfonamid;
N-[3-(3-Methoxy-4-nitrophenyl)-11-oxo-10,11-dihydro-5H-dibenzo[b,e][1,4]diazepin-7-yl]-3-morpholin-4-ylpropan-1-sulfonamid;
N-[3-(3-Methoxy-4-nitrophenyl)-11-oxo-10,11-dihydro-5H-dibenzo[b,e][1,4]diazepin-7-yl]-3-piperidin-1-ylpropan-1-sulfonamid;
3-(Diethylamino)N[3-(3-methoxy-4-nitrophenyl)-11-oxo-10,11-dihydro-5H-dibenzo[b,e][1,4]diazepin-7-yl]propan-1-sulfonamid;
3-(Dimethylamino)N[3-(3-methoxy-4-nitrophenyl)-11-oxo-10,11-dihydro-5H-dibenzo[b,e][1,4]diazepin-7-yl]propan-1-sulfonamid;
N-[3-(3-Methoxy-4-nitrophenyl)-11-oxo-10,11-dihydro-5H-dibenzo[b,e][1,4]diazepin-7-yl]tetrahydro-2H-pyran-4-carboxamid;
8-(1-Hydroxy-1-methylethyl)-3-(3-methoxy-4-nitrophenyl)-5,10-dihydro-11H-dibenzo[b,e][1,4]diazepin-11-on;
8-(1-Ethyl-1-hydroxypropyl)-3-(3-methoxy-4-nitrophenyl)-5,10-dihydro-11H-dibenzo[b,e][1,4]diazepin-11-on;
8-(1-Hydroxy-1-methylethyl)-3-(pyridin-4-ylamino)-5,10-dihydro-11H-dibenzo[b,e][1,4]diazepin-11-on;
3-[(2-Chlorpyridin-4-yl)amino]-8-(1-hydroxy-1-methylethyl)-5,10-dihydro-11H-dibenzo[b,e][1,4]diazepin-11-on;
4-{[8-(1-Hydroxy-1-methylethyl)-11-oxo-10,11-dihydro-5H-dibenzo[b,e][1,4]diazepin-3-yl]amino}pyridin-2-carbonitril;
8-(1-Hydroxy-1-methylethyl)-3-(pyrimidin-4-ylamino)-5,10-dihydro-11H-dibenzo[b,e][1,4]diazepin-11-on;
8-(1-Hydroxy-1-methylethyl)-3-[(2,3,5,6-tetrafluorpyridin-4-yl)amino]-5,10-dihydro-11H-dibenzo[b,e][1,4]diazepin-11-on;
8-(1-Ethyl-1-hydroxypropyl)-3-(pyridin-4-ylamino)-5,10-dihydro-11H-dibenzo[b,e][1,4]diazepin-11-on;
3-[(2-Aminopyrimidin-4-yl)amino]-8-(1-hydroxy-1-methylethyl)-5,10-dihydro-11H-dibenzo[b,e][1,4]diazepin-11-on;
3-[(2-Chlorpyridin-4-yl)amino]-8-(1-ethyl-1-hydroxypropyl)-5,10-dihydro-11H-dibenzo[b,e][1,4]diazepin-11-on;
8-(1-Hydroxy-1-methylethyl)-3-[(2,3,6-trifluorpyridin-4-yl)amino]-5,10-dihydro-11H-dibenzo[b,e][1,4]diazepin-11-on;
3-({2-[(2-Chlorpyridin-4-yl)amino]pyridin-4-yl}amino)-8-(1-hydroxy-1-methylethyl)-5,10-dihydro-11H-dibenzo[b,e] [1,4]diazepin-11-on;
Methyl 2-methyl-2-{11-oxo-3-[(2,3,6-trifluorpyridin-4-yl)amino]-10,11-dihydro-5H-dibenzo[b,e][1,4]diazepin-8-yl}propanoat;
2-MethylN(4-morpholin-4-ylphenyl)-2-{11-oxo-3-[(2,3,6-trifluorpyridin-4-yl)amino]-10,11-dihydro-5H-dibenzo[b,e] [1,4]diazepin-8-yllpropanamid;
2-{3-[(2,6-Difluorpyridin-4-yl)amino]-11-oxo-10,11-dihydro-5H-dibenzo[b,e][1,4]diazepin-8-yl}-2-methylN(4-morpholin-4-ylphenyl)propanamid;
3-[(2,6-Difluorpyridin-4-yl)amino]-8-(2-hydroxyethyl)-5,10-dihydro-11H-dibenzo[b,e][1,4]diazepin-11-on;
2-{3-[(2,6-Difluorpyridin-4-yl)amino]-11-oxo-10,11-dihydro-5H-dibenzo[b,e][1,4]diazepin-8-yl}-2-methylpropansäure;
3-[(2,6-Difluorpyridin-4-yl)amino]-7-morpholin-4-yl-5,10-dihydro-11H-dibenzo[b,e][1,4]diazepin-11-on;
7-Morpholin-4-yl-3-[(2,3,6-trifluorpyridin-4-yl)amino]-5,10-dihydro-11H-dibenzo[b,e][1,4]diazepin-11-on;
3-[(2,6-Difluorpyridin-4-yl)amino]-8-(2-hydroxy-2-methylpropyl)-5,10-dihydro-11H-dibenzo[b,e][1,4]diazepin-11-on;
Methyl [11-oxo-3-(pyridin-4-ylamino)-10,11-dihydro-5H-dibenzo[b,e][1,4]diazepin-8-yl]acetat;
Methyl {3-[(2-chlorpyridin-4-yl)amino]-11-oxo-10,11-dihydro-5H-dibenzo[b,e][1,4]diazepin-8-yl}acetat;
Methyl {3-[(2-methylpyridin-4-yl)amino]-11-oxo-10,11-dihydro-5H-dibenzo[b,e][1,4]diazepin-8-yl}acetat;
3-[(2-Chlorpyridin-4-yl)amino]-5,10-dihydro-11H-dibenzo[b,e][1,4]diazepin-11-on;
8-Acetyl-3-[(2-chlorpyridin-4-yl)amino]-5,10-dihydro-11H-dibenzo[b,e][1,4]diazepin-11-on;
{3-[(2-Chlorpyridin-4-yl)amino]-11-oxo-10,11-dihydro-5H-dibenzo[b,e][1,4]diazepin-8-yl}essigsäure;
3-[(2-Chlorpyridin-4-yl)amino]-8-isopropenyl-5,10-dihydro-11H-dibenzo[b,e][1,4]diazepin-11-on;
2-{3-[(2-Chlorpyridin-4-yl)amino]-11-oxo-10,11-dihydro-5H-dibenzo[b,e][1,4]diazepin-8-yl}N(4-morpholin-4-ylphenyl)acetamid;
3-[(2-Chlorpyridin-4-yl)amino]-8-(2-hydroxy-2-methylpropyl)-5,10-dihydro-11H-dibenzo[b,e][1,4]diazepin-11-on;
2-{3-[(2-Chlorpyridin-4-yl)amino]-11-oxo-10,11-dihydro-5H-dibenzo[b,e][1,4]diazepin-8-yl}-2-methylN(4-morpholin-4-ylphenyl)propanamid;
3-[(2-Chlorpyridin-4-yl)amino]-8-(2-oxopyrrolidin-1-yl)-5,10-dihydro-11H-dibenzo[b,e][1,4]diazepin-11-on;
Methyl {3-[(2-chlorpyridin-4-yl)amino]-11-oxo-10,11-dihydro-5H-dibenzo[b,e][1,4]diazepin-7-yl}acetat;
8-[2-(Pyridin-2-yloxy)ethyl]-3-[(2,3,6-trifluorpyridin-4-yl)amino]-5,10-dihydro-11H-dibenzo[b,e][1,4]diazepin-11-on;
8-(2-Hydroxy-2-methylpropyl)-3-[(2,3,5-trifluorphenyl)amino]-5,10-dihydro-11H-dibenzo[b,e] [1,4]diazepin-11-on;
3-[(3,5-Difluorphenyl)amino]-7-(3-hydroxy-3-methylbutyl)-8-methoxy-5,10-dihydro-11H-dibenzo[b,e][1,4]diazepin-11-on;
7-(3-Hydroxy-3-methylbutyl)-8-methoxy-3-(3-methoxy-4-nitrophenyl)-5,10-dihydro-11H-dibenzo[b,e][1,4]diazepin-11-on;
3-[(2,6-Difluorpyridin-4-yl)amino]-7-(3-hydroxypropyl)-8-methoxy-5,10-dihydro-11H-dibenzo[b,e][1,4]diazepin-11-on;
7-(3-Hydroxypropyl)-8-methoxy-3-(3-methoxy-4-nitrophenyl)-5,10-dihydro-11H-dibenzo[b,e][1,4]diazepin-11-on;
3-[(2,6-Difluorpyridin-4-yl)amino]-8-(3-hydroxy-3-methylbutyl)-5,10-dihydro-11H-dibenzo[b,e][1,4]diazepin-11-on;
8-(3-Hydroxy-3-methylbutyl)-3-(3-methoxy-4-nitrophenyl)-5,10-dihydro-1H-dibenzo[b,e][1,4]diazepin-11-on;
Methyl 3-[3-(3-methoxy-4-nitrophenyl)-11-oxo-10,11-dihydro-5H-dibenzo[b,e][1,4]diazepin-8-yl]propanoat;
3-[3-(3-Methoxy-4-nitrophenyl)-11-oxo-10,11-dihydro-5H-dibenzo[b,e][1,4]diazepin-8-yl]propansäure;
3-[3-(3-Methoxy-4-nitrophenyl)-11-oxo-10,11-dihydro-5H-dibenzo[b,e][1,4]diazepin-8-yl]-N,N-dimethylpropanamid;
3-[3-(3-Methoxy-4-nitrophenyl)-11-oxo-10,11-dihydro-5H-dibenzo[b,e][1,4]diazepin-8-yl]N(4-morpholin-4-ylphenyl)propanamid;
8-(3-Azetidin-1-yl-3-oxopropyl)-3-(3-methoxy-4-nitrophenyl)-5,10-dihydro-11H-dibenzo[b,e][1,4]diazepin-11-on;
3-(3-Methoxy-4-nitrophenyl)-8-(3-oxo-3-pyrrolidin-1-ylpropyl)-5,10-dihydro-11H-dibenzo[b,e][1,4]diazepin-11-on;
3-(3-Methoxy-4-nitrophenyl)-8-(3-morpholin-4-yl-3-oxopropyl)-5,10-dihydro-11H-dibenzo[b,e][1,4]diazepin-11-on;
N,N-Diethyl-3-[3-(3-methoxy-4-nitrophenyl)-11-oxo-10,11-dihydro-5H-dibenzo[b,e][1,4]diazepin-8-yl]propanamid;
8-[3-(4-Hydroxypiperidin-1-yl)-3-oxopropyl]-3-(3-methoxy-4-nitrophenyl)-5,10-dihydro-11H-dibenzo[b,e][1,4]diazepin-11-on;
3-[3-(3-Methoxy-4-nitrophenyl)-11-oxo-10,11-dihydro-5H-dibenzo[b,e][1,4]diazepin-8-yl]N1,3-thiazol-2-ylpropanamid;
8-(2-Hydroxyethyl)-3-(3-methoxy-4-nitrophenyl)-5,10-dihydro-11H-dibenzo[b,e][1,4]diazepin-11-on;
8-(3-Hydroxypropyl)-3-(3-methoxy-4-nitrophenyl)-5,10-dihydro-11H-dibenzo[b,e][1,4]diazepin-11-on;
Methyl 3-[3-(4-Chlor-3-methoxyphenyl)-11-oxo-10,11-dihydro-5H-dibenzo[b,e][1,4]diazepin-8-yl]propanoat;
3-[3-(4-Chlor-3-methoxyphenyl)-11-oxo-10,11-dihydro-5H-dibenzo[b,e][1,4]diazepin-8-yl]-N,N-dimethylpropanamid;
3-(4-Chlor-3-methoxyphenyl)-8-(3-hydroxy-3-methylbutyl)-5,10-dihydro-11H-dibenzo[b,e][1,4]diazepin-11-on;
3-(3-Methoxy-4-nitrophenyl)-8-[2-(3-methyl-1,2,4-oxadiazol-5-yl)ethyl]-5,10-dihydro-11H-dibenzo[b,e][1,4]diazepin-11-on;
Methyl 3-[8-methoxy-3-(3-methoxy-4-nitrophenyl)-11-oxo-10,11-dihydro-5H-dibenzo[b,e][1,4]diazepin-7-yl]propanoat;
7-(2-Hydroxy-2-methylpropyl)-8-methoxy-3-(3-methoxy-4-nitrophenyl)-5,10-dihydro-11H-dibenzo[b,e][1,4]diazepin-11-on;
7-(2-Hydroxyethyl)-8-methoxy-3-(3-methoxy-4-nitrophenyl)-5,10-dihydro-11H-dibenzo[b,e][1,4]diazepin-11-on;
8-Methoxy-3-(3-methoxy-4-nitrophenyl)-7-(2-oxopropyl)-5,10-dihydro-11H-dibenzo[b,e][1,4]diazepin-11-on;
7-(2-Hydroxy-1,1-dimethylethyl)-8-methoxy-3-(3-methoxy-4-nitrophenyl)-5,10-dihydro-11H-dibenzo[b,e][1,4]diazepin-11-on;
7-(3-Hydroxypropyl)-3-(3-methoxy-4-nitrophenyl)-8-(trifluormethoxy)-5,10-dihydro-11H-dibenzo[b,e][1,4]diazepin-11-on;
7-(3-Hydroxy-3-methylbutyl)-3-(3-methoxy-4-nitrophenyl)-8-(trifluormethoxy)-5,10-dihydro-11H-dibenzo[b,e][1,4]diazepin-11-on;
7-(3-Hydroxy-methylbutyl)-3-(3-methoxy-4-nitrophenyl)-8-methyl-5,10-dihydro-11H-dibenzo[b,e][1,4]diazepin-11-on;
3-(3-Methoxy-4-nitrophenyl)-8-(2-pyridin-4-ylethyl)-5,10-dihydro-11H-dibenzo[b,e][1,4]diazepin-11-on;
3-(3-Methoxy-4-nitrophenyl)-8-(2-pyridin-4-ylethyl)-5,10-dihydro-11H-dibenzo[b,e][1,4]diazepin-11-on;
3-(3-Methoxy-4-nitrophenyl)-8-[2-(2-oxopyridin-1(2H)-yl)ethyl]-5,10-dihydro-11H-dibenzo[b,e][1,4]diazepin-11-on;
8-[2-(5-Fluor-2-oxopyridin-1(2H)-yl)ethyl]-3-(3-methoxy-4-nitrophenyl)-5,10-dihydro-11H-dibenzo[b,e][1,4]diazepin-11-on;
3-(3-Methoxy-4-nitrophenyl)-8-[2-(6-oxopyridazin-1(6H)-yl)ethyl]-5,10-dihydro-11H-dibenzo[b,e][1,4]diazepin-11-on;
3-(3-Methoxy-4-nitrophenyl)-8-[2-(pyridin-2-yloxy)ethyl]-5,10-dihydro-11H-dibenzo[b,e][1,4]diazepin-11-on;
8-{2-[(5-Chlorpyridin-3-yl)oxy]ethyl}-3-(3-methoxy-4-nitrophenyl)-5,10-dihydro-11H-dibenzo[b,e][1,4]diazepin-11-on;
8-Methoxy-3-(3-methoxy-4-nitrophenyl)-7-[2-(pyridin-3-yloxy)ethyl]-5,10-dihydro-11H-dibenzo[b,e][1,4]diazepin-11-on;
Methyl (3-isochinolin-5-yl-1-oxo-10,11-dihydro-5H-dibenzo[b,e][1,4]diazepin-8-yl)acetat;
Methyl [3-(8-nitroisochinolin-5-yl)-11-oxo-10,11-dihydro-5H-dibenzo[b,e][1,4]diazepin-8-yl]acetat;
Methyl 3-[3-(4-formyl-3-methoxyphenyl)-11-oxo-10,11-dihydro-5H-dibenzo[b,e][1,4]diazepin-8-yl]propanoat;
Methyl 3-{3-[4-(hydroxymethyl)-3-methoxyphenyl]-11-oxo-10,11-dihydro-5H-dibenzo[b,e][1,4]diazepin-8-yl}propanoat;
Methyl 3-[3-(3-methoxy-4-propionylphenyl)-11-oxo-10,11-dihydro-5H-dibenzo[b,e][1,4]diazepin-8-yl]propanoat;
2-[3-(3-Methoxy-4-nitrophenyl)-11-oxo-10,11-dihydro-5H-dibenzo[b,e][1,4]diazepin-8-yl]-2-methylN(4-morpholin-4-ylphenyl)propanamid;
2-[3-(4-Cyano-3-methoxyphenyl)-11-oxo-10,11-dihydro-5H-dibenzo[b,e][1,4]diazepin-8-yl]-2-methylN(4-morpholin-4-ylphenyl)propanamid;
2-[3-(4-Chlor-3-methoxyphenyl)-11-oxo-10,11-dihydro-5H-dibenzo[b,e][1,4]diazepin-8-yl]-2-methylN(4-morpholin-4-ylphenyl)propanamid;
2-[3-(3-Methoxy-4-nitrophenyl)-11-oxo-10,11-dihydro-5H-dibenzo[b,e][1,4]diazepin-8-yl]-2-methylNpyridin-2-ylpropanamid;
2-[3-(3-Methoxy-4-nitrophenyl)-11-oxo-10,11-dihydro-5H-dibenzo[b,e][1,4]diazepin-8-yl]-2-methylNpyridin-3-ylpropanamid;
2-[3-(3-Methoxy-4-nitrophenyl)-11-oxo-10,11-dihydro-5H-dibenzo[b,e][1,4]diazepin-8-yl]-2-methylNpyridin-3-ylpropanamid;
2-[3-(3-Methoxy-4-nitrophenyl)-11-oxo-10,11-dihydro-5H-dibenzo[b,e][1,4]diazepin-8-yl]-2-methylN(pyridin-3-ylmethyl)propanamid;
2-[3-(3-Methoxy-4-nitrophenyl)-11-oxo-10,11-dihydro-5H-dibenzo[b,e][1,4]diazepin-8-yl]-2-methylN(pyridin-3-ylmethyl)propanamid;
2-[3-(3-Methoxy-4-nitrophenyl)-11-oxo-10,11-dihydro-5H-dibenzo[b,e][1,4]diazepin-8-yl]-2-methylN(tetrahydrofuran-3-ylmethyl)propanamid;
2-[3-(3-Methoxy-4-nitrophenyl)-11-oxo-10,11-dihydro-5H-dibenzo[b,e][1,4]diazepin-8-yl]-2-methylNl,3-thiazol-2-ylpropanamid;
2-[3-(3-Methoxy-4-nitrophenyl)-11-oxo-10,11-dihydro-5H-dibenzo[b,e][1,4]diazepin-8-yl]-2-methylN{[6-(trifluormethyl)pyridin-3-yl]methyl}propanamid;
N-(4-Fluorphenyl)-2-[3-(3-methoxy-4-nitrophenyl)-11-oxo-10,11-dihydro-5H-dibenzo[b,e][1,4]diazepin-8-yl]-2-methylpropanamid;
N-(2,5-Difluorbenzyl)-2-[3-(3-methoxy-4-nitrophenyl)-11-oxo-10,11-dihydro-5H-dibenzo[b,e][1,4]diazepin-8-yl]-2-methylpropanamid;
2-Methyl-2-[11-oxo-3-(pyrimidin-4-ylamino)-10,11-dihydro-5H-dibenzo[b,e][1,4]diazepin-8-yl]N[3-(trifluormethyl)benzyl]propanamid;
2-Methyl-2-[11-oxo-3-(pyrimidin-4-ylamino)-10,11-dihydro-5H-dibenzo[b,e][1,4]diazepin-8-yl]N[3-(trifluormethoxy)benzyl]propanamid;
1-[3-(3-Methoxy-4-nitrophenyl)-11-oxo-10,11-dihydro-5H-dibenzo[b,e][1,4]diazepin-8-yl]N(4-morpholin-4-ylphenyl)cyclopropancarboxamid;
2-[3-(3-Methoxy-4-nitrophenyl)-11-oxo-10,11-dihydro-5H-dibenzo[b,e][1,4]diazepin-7-yl]-2-methylN(4-morpholin-4-ylphenyl)propanamid;
2-[3-(3-Methoxy-4-nitrophenyl)-11-oxo-10,11-dihydro-5H-dibenzo[b,e][1,4]diazepin-7-yl]-2-methylN(pyridin-2-ylmethyl)propanamid;
2-[3-(3-Methoxy-4-nitrophenyl)-11-oxo-10,11-dihydro-5H-dibenzo[b,e][1,4]diazepin-7-yl]-2-methylN(thien-2-ylmethyl)propanamid;
N-(Cyclopropylmethyl)-2-[3-(3-methoxy-4-nitrophenyl)-11-oxo-10,11-dihydro-5H-dibenzo[b,e][1,4]diazepin-7-yl]-2-methylpropanamid;
7-(3-Hydroxypropyl)-3-(3-methoxy-4-nitrophenyl)-5,10-dihydro-11H-dibenzo[b,e][1,4]diazepin-11-on;
7-(3-Hydroxy-3-methylbutyl)-3-(3-methoxy-4-nitrophenyl)-5,10-dihydro-11H-dibenzo[b,e][1,4]diazepin-11-on;
8-(2-Hydroxy-1,1-dimethylethyl)-3-(3-methoxy-4-nitrophenyl)-5,10-dihydro-11H-dibenzo[b,e][1,4]diazepin-11-on;
8-(2-Hydroxy-1,1,2-trimethylpropyl)-3-(3-methoxy-4-nitrophenyl)-5,10-dihydro-11H-dibenzo[b,e][1,4]diazepin-11-on;
8-(1,1-Dimethyl-2-oxopropyl)-3-(3-methoxy-4-nitrophenyl)-5,10-dihydro-11H-dibenzo[b,e][1,4]diazepin-11-on;
7-(2-Hydroxy-1,1-dimethylethyl)-3-(3-methoxy-4-nitrophenyl)-5,10-dihydro-11H-dibenzo[b,e][1,4]diazepin-11-on;
8-[1-(Hydroxymethyl)cyclopropyl]-3-(3-methoxy-4-nitrophenyl)-5,10-dihydro-11H-dibenzo[b,e][1,4]diazepin-11-on;
3-[(2-Chlorpyridin-4-yl)amino]-8-(2-hydroxy-1,1-dimethylethyl)-5,10-dihydro-11H-dibenzo[b,e][1,4]diazepin-11-on;
3-[(2,6-Difluorpyridin-4-yl)amino]-8-(2-hydroxy-1,1-dimethylethyl)-5,10-dihydro-11H-dibenzo[b,e][1,4]diazepin-11-on;
3-[(2,6-Difluorpyridin-4-yl)amino]-8-(2-hydroxy-1,1,2-trimethylpropyl)-5,10-dihydro-11H-dibenzo[b,e][1,4]diazepin-11-on;
3-(4-Chlor-3-methoxyphenyl)-8-(2-hydroxy-1,1-dimethylethyl)-5,10-dihydro-11H-dibenzo[b,e][1,4]diazepin-11-on;
3-(3-Methoxy-4-nitrophenyl)-8-[2-(4-morpholin-4-ylphenoxy)ethyl]-5,10-dihydro-11H-dibenzo[b,e][1,4]diazepin-11-on;
3-(4-Chlor-3-methoxyphenyl)-8-[2-(4-morpholin-4-ylphenoxy)ethyl]-5,10-dihydro-11H-dibenzo[b,e][1,4]diazepin-11-on;
2-Methoxy-4-{8-[2-(4-morpholin-4-ylphenoxy)ethyl]-11-oxo-10,11-dihydro-5H-dibenzo[b,e][1,4]diazepin-3-yl}benzonitril;
3-(3-Methoxy-4-nitrophenyl)-8-{2-[(4-morpholin-4-ylphenyl)amino]ethyl}-5,10-dihydro-11H-dibenzo[b,e] [1,4]diazepin-11-on;
3-(3-Methoxy-4-nitrophenyl)-8-[2-(3-methyl-2-oxopyridin-1(2H)-yl)ethyl]-5,10-dihydro-11H-dibenzo[b,e][1,4]diazepin-11-on;
3-(3-Methoxy-4-nitrophenyl)-8-{2-[(5-methylpyridin-2-yl)oxy]ethyl}-5,10-dihydro-11H-dibenzo[b,e][1,4]diazepin-11-on;
3-(3-Methoxy-4-nitrophenyl)-8-[2-(4-methyl-2-oxopryridin-1(2H)-yl)ethyl]-5,10-dihydro-11H-dibenzo[b,e][1,4]diazepin-11-on;
8-[2-(Isochinolin-3-yloxy)ethyl]-3-(3-methoxy-4-nitrophenyl)-5,10-dihydro-11H-dibenzo[b,e][1,4]diazepin-11-on;
8-[2-(5-Chlor-2-oxopyridin-1(2H)-yl)ethyl]-3-(3-methoxy-4-nitrophenyl)-5,10-dihydro-11H-dibenzo[b,e][1,4]diazepin-11-on;
8-[1,1-Dimethyl-2-(pyridin-2-yloxy)ethyl]-3-(3-methoxy-4-nitrophenyl)-5,10-dihydro-11H-dibenzo[b,e][1,4]diazepin-11-on;
8-[1,1-Dimethyl-2-(4-morpholin-4-ylphenoxy)ethyl]-3-(3-methoxy-4-nitrophenyl)-5,10-dihydro-11H-dibenzo[b,e] [1,4]diazepin-11-on;
2-[3-(3-Methoxy-4-nitrophenyl)-11-oxo-10,11-dihydro-5H-dibenzo[b,e][1,4]diazepin-8-yl]-2-methylN(tetrahydrofuran-2-ylmethyl)propanamid;
8-(2-Hydroxy-1,1-dimethylethyl)-3-(3-methoxy-4-nitrophenyl)-7-methyl-5,10-dihydro-11H-dibenzo[b,e] [1,4]diazepin-11-on;
2-[3-(3-Methoxy-4-nitrophenyl)-2-methyl-11-oxo-10,11-dihydro-5H-dibenzo[b,e][1,4]diazepin-8-yl]N(4-morpholin-4-ylphenyl)acetamid;
Methyl {3-[4-(aminomethyl)-3-methoxyphenyl]-11-oxo-10,11-dihydro-5H-dibenzo[b,e][1,4]diazepin-8-yl}acetat;
2-[3-(2-Methoxy-5-methylpyridin-4-yl)-11-oxo-10,11-dihydro-5H-dibenzo[b,e][1,4]diazepin-8-yl]-2-methylN(4-morpholin-4-ylphenyl)propanamid;
8-(2-Hydroxy-1,1-dimethylethyl)-3-[(2-methylpyridin-4-yl)amino]-5,10-dihydro-11H-dibenzo[b,e][1,4]diazepin-11-on;
8-(2-Hydroxy-1,1,2-trimethylpropyl)-3-(pyrimidin-4-ylamino)-5,10-dihydro-11H-dibenzo[b,e][1,4]diazepin-11-on;
2-[3-(3-Methoxy-4-nitrophenyl)-11-oxo-10,11-dihydro-5H-dibenzo[b,e][1,4]diazepin-8-yl]NmethylN(3-pyrrolidin-1-ylpropyl)acetamid;
2-[3-(3-Methoxy-4-nitrophenyl)-11-oxo-10,11-dihydro-5H-dibenzo[b,e][1,4]diazepin-8-yl]-2-methylN(3-pyrrolidin-1-ylpropyl)propanamid;
2-[3-(3-Methoxy-4-nitrophenyl)-11-oxo-10,11-dihydro-5H-dibenzo[b,e][1,4]diazepin-8-yl]-2-methylNpyrimidin-4-ylpropanamid;
2-[3-(3-Methoxy-4-nitrophenyl)-11-oxo-10,11-dihydro-5H-dibenzo[b,e][1,4]diazepin-8-yl]-2-methylN(4-methyl-1,3-thiazol-2-yl)propanamid;
2-[3-(3-Methoxy-4-nitrophenyl)-11-oxo-10,11-dihydro-5H-dibenzo[b,e][1,4]diazepin-8-yl]-2-methylN(2,2,2-trifluorethyl)propanamid;
N-(3-Fluorphenyl)-2-[3-(3-methoxy-4-nitrophenyl)-11-oxo-10,11-dihydro-5H-dibenzo[b,e][1,4]diazepin-8-yl]-2-methylpropanamid;
2-[3-(3-Methoxy-4-nitrophenyl)-11-oxo-10,11-dihydro-5H-dibenzo[b,e][1,4]diazepin-8-yl]-2-methylN[4-(trifluormethoxy)phenyl]propanamid;
2-[3-(3-Methoxy-4-nitrophenyl)-11-oxo-10,11-dihydro-5H-dibenzo[b,e][1,4]diazepin-8-yl]-2-methylN[3-(trifluormethyl)phenyl]propanamid;
2-[3-(3-Methoxy-4-nitrophenyl)-11-oxo-10,11-dihydro-5H-dibenzo[b,e][1,4]diazepin-8-yl]-2-methylN[3-(trifluormethoxy)phenyl]propanamid;
N-[3-Fluor-5-(trifluormethyl)benzyl]-2-[3-(3-methoxy-4-nitrophenyl)-11-oxo-10,11-dihydro-5H-dibenzo[b,e][1,4]diazepin-8-yl]-2-methylpropanamid;
N-(2-Fluorbenzyl)-2-[3-(3-methoxy-4-nitrophenyl)-11-oxo-10,11-dihydro-5H-dibenzo[b,e][1,4]diazepin-8-yl]-2-methylpropanamid;
N-(3-Fluorbenzyl)-2-[3-(3-methoxy-4-nitrophenyl)-11-oxo-10,11-dihydro-5H-dibenzo[b,e][1,4]diazepin-8-yl]-2-methylpropanamid;
N-(4-Fluorbenzyl)-2-[3-(3-methoxy-4-nitrophenyl)-11-oxo-10,11-dihydro-5H-dibenzo[b,e][1,4]diazepin-8-yl]-2-methylpropanamid;
2-[3-(3-Methoxy-4-nitrophenyl)-11-oxo-10,11-dihydro-5H-dibenzo[b,e][1,4]diazepin-8-yl]-2-methylN[4-(trifluormethoxy)benzyl]propanamid;
2-[3-(3-Methoxy-4-nitrophenyl)-11-oxo-10,11-dihydro-5H-dibenzo[b,e][1,4]diazepin-8-yl]-2-methylN[3-(trifluormethyl)benzyl]propanamid;
2-[3-(3-Methoxy-4-nitrophenyl)-11-oxo-10,11-dihydro-5H-dibenzo[b,e][1,4]diazepin-8-yl]-2-methylN[4-(trifluormethyl)benzyl]propanamid;
2-[3-(3-Methoxy-4-nitrophenyl)-11-oxo-10,11-dihydro-5H-dibenzo[b,e][1,4]diazepin-8-yl]-2-methylN[3-(trifluormethoxy)benzyl]propanamid;
N-[2-(2-Fluorphenyl)ethyl]-2-[3-(3-methoxy-4-nitrophenyl)-11-oxo-10,11-dihydro-5H-dibenzo[b,e][1,4]diazepin-8-yl]-2-methylpropanamid;
N-[2-(3-Fluorphenyl)ethyl]-2-[3-(3-methoxy-4-nitrophenyl)-11-oxo-10,11-dihydro-5H-dibenzo[b,e][1,4]diazepin-8-yl]-2-methylpropanamid;
N-(2,4-Difluorbenzyl)-2-[3-(3-methoxy-4-nitrophenyl)-11-oxo-10,11-dihydro-5H-dibenzo[b,e][1,4]diazepin-8-yl]-2-methylpropanamid;
N-(2,6-Difluorbenzyl)-2-[3-(3-methoxy-4-nitrophenyl)-11-oxo-10,11-dihydro-5H-dibenzo[b,e][1,4]diazepin-8-yl]-2-methylpropanamid;
N-(Cyclopropylmethyl)-2-[3-(3-methoxy-4-nitrophenyl)-11-oxo-10,11-dihydro-5H-dibenzo[b,e][1,4]diazepin-8-yl]-2-methylpropanamid;
N-(3-Ethoxypropyl)-2-methyl-2-[11-oxo-3-(pyrimidin-4-ylamino)-10,11-dihydro-5H-dibenzo[b,e][1,4]diazepin-8-yl]propanamid;
N-(3,4-Difluorbenzyl)-2-methyl-2-[11-oxo-3-(pyrimidin-4-ylamino)-10,11-dihydro-5H-dibenzo[b,e][1,4]diazepin-8-yl]propanamid;
8-[1,1-Dimethyl-2-oxo-2-(4-phenylpiperazin-1-yl)ethyl]-3-(pyrimidin-4-ylamino)-5,10-dihydro-11H-dibenzo[b,e] [1,4]diazepin-11-on;
N-Cyclopentyl-2-methyl-2-[11-oxo-3-(pyrimidin-4-ylamino)-10,11-dihydro-5H-dibenzo[b,e][1,4]diazepin-8-yl]propanamid;
8-(1,1-Dimethyl-2-morpholin-4-yl-2-oxoethyl)-3-(pyrimidin-4-ylamino)-5,10-dihydro-11H-dibenzo[b,e][1,4]diazepin-11-on;
2-Methyl-2-[11-oxo-3-(pyrimidin-4-ylamino)-10,11-dihydro-5H-dibenzo[b,e][1,4]diazepin-8-yl]N(tetrahydrofuran-3-ylmethyl)propanamid;
N-(Cyclopentylmethyl)-2-methyl-2-[11-oxo-3-(pyrimidin-4-ylamino)-10,11-dihydro-5H-dibenzo[b,e][1,4]diazepin-8-yl]propanamid;
N-(Cyclopropylmethyl)-2-methyl-2-[11-oxo-3-(pyrimidin-4-ylamino)-10,11-dihydro-5H-dibenzo[b,e][1,4]diazepin-8-yl]propanamid;
2-Methyl-2-[11-oxo-3-(pyrimidin-4-ylamino)-10,11-dihydro-5H-dibenzo[b,e][1,4]diazepin-8-yl]N(tetrahydrofuran-2-ylmethyl)propanamid;
2-[3-(3-Methoxy-4-nitrophenyl)-11-oxo-10,11-dihydro-5H-dibenzo[b,e][1,4]diazepin-7-yl]-2-methylN1,3-thiazol-2-ylpropanamid;
N-(2,5-Difluorbenzyl)-2-[3-(3-methoxy-4-nitrophenyl)-11-oxo-10,11-dihydro-5H-dibenzo[b,e][1,4]diazepin-7-yl]-2-methylpropanamid;
N-(2-Ethoxyethyl)-2-[3-(3-methoxy-4-nitrophenyl)-11-oxo-10,11-dihydro-5H-dibenzo[b,e][1,4]diazepin-7-yl]-2-methylpropanamid;
N-(4-Fluorphenyl)-2-[3-(3-methoxy-4-nitrophenyl)-11-oxo-10,11-dihydro-5H-dibenzo[b,e][1,4]diazepin-7-yl]-2-methylpropanamid;
3-(3-Methoxy-4-nitrophenyl)-8-[2-(chinolin-2-yloxy)ethyl]-5,10-dihydro-11H-dibenzo[b,e][1,4]diazepin-11-on;
3-(3-Methoxy-4-nitrophenyl)-8-[2-(5-methyl-2-oxopyridin-1(2H)-yl)ethyl]-5,10-dihydro-11H-dibenzo[b,e][1,4]diazepin-11-on;
2-{2-[3-(3-Methoxy-4-nitrophenyl)-11-oxo-10,11-dihydro-5H-dibenzo[b,e][1,4]diazepin-8-yl]ethoxy}-6-methylnicotinonitril;
3-(3-Methoxy-4-nitrophenyl)-8-[2-(1-oxoisochinolin-2(1H)-yl)ethyl]-5,10-dihydro-11H-dibenzo[b,e][1,4]diazepin-11-on;
3-(3-Methoxy-4-nitrophenyl)-8-{2-[2-oxo-5-(trifluormethyl)pyridin-1(2H)-yl]ethyl}-5,10-dihydro-11H-dibenzo[b,e][1,4]diazepin-11-on;
3-(3-Methoxy-4-nitrophenyl)-8-[2-(3-methoxy-2-oxopyridin-1(2H)-yl)ethyl]-5,10-dihydro-11H-dibenzo[b,e][1,4]diazepin-11-on;
3-(3-Methoxy-4-nitrophenyl)-8-{2-[(4-methylpyridin-2-yl)oxy]ethyl}-5,10-dihydro-11H-dibenzo[b,e][1,4]diazepin-11-on;
3-(3-Methoxy-4-nitrophenyl)-8-{2-[(3-methoxypyridin-2-yl)oxy]ethyl}-5,10-dihydro-11H-dibenzo[b,e][1,4]diazepin-11-on;
3-(3-Methoxy-4-nitrophenyl)-8-[2-(3-oxoisochinolin-2(3H)-yl)ethyl]-5,10-dihydro-11H-dibenzo[b,e][1,4]diazepin-11-on;
8-{2-[(6-Chlorpyridin-2-yl)oxy]ethyl}-3-(3-methoxy-4-nitrophenyl)-5,10-dihydro-11H-dibenzo[b,e][1,4]diazepin-11-on;
8-{2-[(5-Chlorpyridin-2-yl)oxy]ethyl}-3-(3-methoxy-4-nitrophenyl)-5,10-dihydro-11H-dibenzo[b,e][1,4]diazepin-11-on;
[[3-(4-Chlor-3-methoxyphenyl)-11-oxo-10,11-dihydro-5H-dibenzo[b,e][1,4]diazepin-8-yl]essigsäure;
Methyl [3-(4-acetyl-3-methoxyphenyl)-11-oxo-10,11-dihydro-5H-dibenzo[b,e][1,4]diazepin-8-yl]acetat;
[3-(4-Acetyl-3-methoxyphenyl)-11-oxo-10,11-dihydro-5H-dibenzo[b,e][1,4]diazepin-8-yl]essigsäure;
2-[3-(4-Chlor-3-methoxyphenyl)-11-oxo-10,11-dihydro-5H-dibenzo[b,e][1,4]diazepin-8-yl]-N,N-dimethylacetamid;
tert-Butyl 1-{[3-(3-methoxy-4-nitrophenyl)-11-oxo-10,11-dihydro-5H-dibenzo[b,e][1,4]diazepin-8-yl]acetyl}pyrrolidin-3-ylcarbamat;
8-[2-(3-Aminopyrrolidin-1-yl)-2-oxoethyl]-3-(3-methoxy-4-nitrophenyl)-5,10-dihydro-11H-dibenzo[b,e][1,4]diazepin-11-on;
[3-(2-Methoxy-5-methylpyridin-4-yl)-11-oxo-10,11-dihydro-5H-dibenzo[b,e][1,4]diazepin-8-yl]essigsäure;
2-[3-(2-Methoxy-5-methylpyridin-4-yl)-11-oxo-10,11-dihydro-5H-dibenzo[b,e][1,4]diazepin-8-yl]-N,N-dimethylacetamid;
8-{2-[(2S)-2-(Hydroxymethyl)pyrrolidin-1-yl]-2-oxoethyl}-3-(2-methoxy-5-methylpyridin-4-yl)-5,10-dihydro-11H-dibenzo[b,e] [1,4]diazepin-11-on;
tert-Butyl 1-{[3-(2-methoxy-5-methylpyridin-4-yl)-11-oxo-10,11-dihydro-5H-dibenzo[b,e][1,4]diazepin-8-yl]acetyl}pyrrolidin-3-ylcarbamat;
N-{2-[4-(Aminosulfonyl)phenyl]ethyl}-2-[3-(4-chlor-3-methoxyphenyl)-11-oxo-10,11-dihydro-5H-dibenzo[b,e] [1,4]diazepin-8-yl]acetamid;
tert-Butyl 1-{[3-(4-chlor-3-methoxyphenyl)-11-oxo-10,11-dihydro-5H-dibenzo[b,e][1,4]diazepin-8-yl]acetyl}pyrrolidin-3-ylcarbamat;
3-(4-Chlor-3-methoxyphenyl)-8-[2-(3-hydroxypiperidin-1-yl)-2-oxoethyl]-5,10-dihydro-11H-dibenzo[b,e][1,4]diazepin-11-on;
3-(4-Chlor-3-methoxyphenyl)-8-{2-[(2S)-2-(hydroxymethyl)pyrrolidin-1-yl]-2-oxoethyl}-5,10-dihydro-11H-dibenzo[b,e][1,4]diazepin-11-on;
2-[3-(4-Chlor-3-methoxyphenyl)-11-oxo-10,11-dihydro-5H-dibenzo[b,e][1,4]diazepin-8-yl]N(4-morpholin-4-ylphenyl)acetamid;
tert-Butyl 4-{[3-(3-methoxy-4-nitrophenyl)-11-oxo-10,11-dihydro-5H-dibenzo[b,e][1,4]diazepin-8-yl]acetyl}piperazin-1-carboxylat;
3-(3-Methoxy-4-nitrophenyl)-8-(2-oxo-2-piperazin-1-ylethyl)-5,10-dihydro-11H-dibenzo[b,e][1,4]diazepin-11-on;
[3-(4-Cyano-3-methoxyphenyl)-11-oxo-10,11-dihydro-5H-dibenzo[b,e][1,4]diazepin-8-yl]essigsäure;
2-[3-(4-Cyano-3-methoxyphenyl)-11-oxo-10,11-dihydro-5H-dibenzo[b,e][1,4]diazepin-8-yl]N(4-morpholin-4-ylphenyl)acetamid;
2-[3-(3-Methoxy-4-nitrophenyl)-11-oxo-10,11-dihydro-5H-dibenzo[b,e][1,4]diazepin-8-yl]Nmethylacetamid;
3-(3-Methoxy-4-nitrophenyl)-8-(2-morpholin-4-yl-2-oxoethyl)-5,10-dihydro-11H-dibenzo[b,e][1,4]diazepin-11-on;
3-(2-Methoxy-5-methylpyridin-4-yl)-8-(2-morpholin-4-yl-2-oxoethyl)-5,10-dihydro-11H-dibenzo[b,e][1,4]diazepin-11-on;
[3-(2-Fluorpyridin-4-yl)-11-oxo-10,11-dihydro-5H-dibenzo[b,e][1,4]diazepin-8-yl]essigsäure;
N,N-Dimethyl-2-[11-oxo-3-(2-oxo-1,2-dihydropyridin-4-yl)-10,11-dihydro-5H-dibenzo[b,e][1,4]diazepin-8-yl]acetamid;
8-(2-Morpholin-4-yl-2-oxoethyl)-3-(2-oxo-1,2-dihyropyridin-4-yl)-5,10-dihydro-11H-dibenzo[b,e][1,4]diazepin-11-on;
N-(4-Morpholin-4-ylphenyl)-2-[11-oxo-3-(2-oxo-1,2-dihydropyridin-4-yl)-10,11-dihydro-5H-dibenzo[b,e][1,4]diazepin-8-yl]acetamid;
Methyl [11-oxo-3-(2-oxo-1,2-dihydropyridin-4-yl)-10,11-dihydro-5H-dibenzo[b,e][1,4]diazepin-8-yl]acetat;
Methyl [3-(3-methoxy-4-nitrophenyl)-11-oxo-10,11-dihydro-5H-dibenzo[b,e][1,4]diazepin-7-yl]acetat;
[3-(3-Methoxy-4-nitrophenyl)-11-oxo-10,11-dihydro-5H-dibenzo[b,e][1,4]diazepin-7-yl]essigsäure;
3-(3-Methoxy-4-nitrophenyl)-7-(2-morpholin-4-yl-2-oxoethyl)-5,10-dihydro-11H-dibenzo[b,e][1,4]diazepin-11-on;
2-[3-(3-Methoxy-4-nitrophenyl)-11-oxo-10,11-dihydro-5H-dibenzo[b,e][1,4]diazepin-7-yl]Npyridin-2-ylacetamid;
2-[3-(3-Methoxy-4-nitrophenyl)-11-oxo-10,11-dihydro-5H-dibenzo[b,e][1,4]diazepin-7-yl]Npyridin-3-ylacetamid;
2-[3-(3-Methoxy-4-nitrophenyl)-11-oxo-10,11-dihydro-5H-dibenzo[b,e][1,4]diazepin-7-yl]Npyridin-4-ylacetamid;
7-[2-(4-Hydroxypiperidin-1-yl)-2-oxoethyl]-3-(3-methoxy-4-nitrophenyl)-5,10-dihydro-11H-dibenzo[b,e][1,4]diazepin-11-on;
7-[2-(3-Hydroxypiperidin-1-yl)-2-oxoethyl]-3-(3-methoxy-4-nitrophenyl)-5,10-dihydro-11H-dibenzo[b,e][1,4]diazepin-11-on;
2-[3-(3-Methoxy-4-nitrophenyl)-11-oxo-10,11-dihydro-5H-dibenzo[b,e][1,4]diazepin-7-yl]N(pyridin-3-ylmethyl)acetamid;
2-[3-(3-Methoxy-4-nitrophenyl)-11-oxo-10,11-dihydro-5H-dibenzo[b,e][1,4]diazepin-7-yl]N(pyridin-4-ylmethyl)acetamid;
2-[3-(3-Methoxy-4-nitrophenyl)-11-oxo-10,11-dihydro-5H-dibenzo[b,e][1,4]diazepin-7-yl]N(pyridin-2-ylmethyl)acetamid;
7-(2-Azetidin-1-yl-2-oxoethyl)-3-(3-methoxy-4-nitrophenyl)-5,10-dihydro-11H-dibenzo[b,e][1,4]diazepin-11-on;
1-{[3-(3-Methoxy-4-nitrophenyl)-11-oxo-10,11-dihydro-5H-dibenzo[b,e][1,4]diazepin-7-yl]acetyl}piperidin-3-carboxamid;
1-{[3-(3-Methoxy-4-nitrophenyl)-11-oxo-10,11-dihydro-5H-dibenzo[b,e][1,4]diazepin-7-yl]acetyl}piperidin-4-carboxamid;
7-{2-[(2R)-2-(Hydroxymethyl)pyrrolidin-1-yl]-2-oxoethyl}-3-(3-methoxy-4-nitrophenyl)-5,10-dihydro-11H-dibenzo[b,e] [1,4]diazepin-11-on;
2-[3-(3-Methoxy-4-nitrophenyl)-11-oxo-10,11-dihydro-5H-dibenzo[b,e][1,4]diazepin-7-yl]-N,N-dimethylacetamid;
N,N-bis(2-Methoxyethyl)-2-[3-(3-methoxy-4-nitrophenyl)-11-oxo-10,11-dihydro-5H-dibenzo[b,e][1,4]diazepin-7-yl]acetamid;
2-[3-(3-Methoxy-4-nitrophenyl)-11-oxo-10,11-dihydro-5H-dibenzo[b,e][1,4]diazepin-7-yl]N[(2S)-tetrahydrofuran-2-ylmethyl]acetamid;
2-[3-(3-Methoxy-4-nitrophenyl)-11-oxo-10,11-dihydro-5H-dibenzo[b,e][1,4]diazepin-7-yl]N(2-propoxyethyl)acetamid;
7-{2-[(2S)-2-(Hydroxymethyl)pyrrolidin-1-yl]-2-oxoethyl}-3-(3-methoxy-4-nitrophenyl)-5,10-dihydro-11H-dibenzo[b,e] [1,4]diazepin-11-on;
7-[2-(3-Aminopyrrolidin-1-yl)-2-oxoethyl]-3-(3-methoxy-4-nitrophenyl)-5,10-dihydro-11H-dibenzo[b,e][1,4]diazepin-11-on;
3-(3-Methoxy-4-nitrophenyl)-7-(2-oxo-2-piperazin-1-ylethyl)-5,10-dihydro-11H-dibenzo[b,e][1,4]diazepin-11-on;
2-[3-(3-Methoxy-4-nitrophenyl)-11-oxo-10,11-dihydro-5H-dibenzo[b,e][1,4]diazepin-7-yl]N(3-methoxypropyl)acetamid;
N-(Cyanomethyl)-2-[3-(3-methoxy-4-nitrophenyl)-11-oxo-10,11-dihydro-5H-dibenzo[b,e][1,4]diazepin-7-yl]acetamid;
N-(Cyclopropylmethyl)-2-[3-(3-methoxy-4-nitrophenyl)-11-oxo-10,11-dihydro-5H-dibenzo[b,e][1,4]diazepin-7-yl]acetamid;
N-(1,3-Dioxolan-2-ylmethyl)-2-[3-(3-methoxy-4-nitrophenyl)-11-oxo-10,11-dihydro-5H-dibenzo[b,e][1,4]diazepin-7-yl]Nmethylacetamid;
3-(3-Methoxy-4-nitrophenyl)-7-(2-oxo-2-thiomorpholin-4-ylethyl)-5,10-dihydro-1H-dibenzo[b,e][1,4]diazepin-11-on;
2-[3-(3-Methoxy-4-nitrophenyl)-11-oxo-10,11-dihydro-5H-dibenzo[b,e][1,4]diazepin-7-yl]N(2-pyridin-3-ylethyl)acetamid;
2-[3-(3-Methoxy-4-nitrophenyl)-11-oxo.-10,11-dihydro-5H-dibenzo[b,e][1,4]diazepin-7-yl]N(2-pyridin-4-ylethyl)acetamid;
N-[2-(2,3-Dimethoxyphenyl)ethyl]-2-[3-(3-methoxy-4-nitrophenyl)-11-oxo-10,11-dihydro-5H-dibenzo[b,e][1,4]diazepin-7-yl]acetamid;
N-[2-(1,3-Benzodioxol-5-yl)ethyl]-2-[3-(3-methoxy-4-nitrophenyl)-11-oxo-10,11-dihydro-5H-dibenzo[b,e][1,4]diazepin-7-yl]acetamid;
2-[3-(3-Methoxy-4-nitrophenyl)-11-oxo-10,11-dihydro-5H-dibenzo[b,e][1,4]diazepin-7-yl]N(thien-2-ylmethyl)acetamid;
2-[3-(3-Methoxy-4-nitrophenyl)-11-oxo-10,11-dihydro-5H-dibenzo[b,e][1,4]diazepin-7-yl]N(1,3-thiazol-5-ylmethyl)acetamid;
N-[2-(1H-Imidazol-4-yl)ethyl]-2-[3-(3-methoxy-4-nitrophenyl)-11-oxo-10,11-dihydro-5H-dibenzo[b,e][1,4]diazepin-7-yl]acetamid;
7-[2-(1,4-Dioxa-8-azaspiro[4.5]dec-8-yl)-2-oxoethyl]-3-(3-methoxy-4-nitrophenyl)-5,10-dihydro-11H-dibenzo[b,e] [1,4]diazepin-11-on;
7-{2-[2,6-Dimethylmorpholin-4-yl]-2-oxoethyl}-3-(3-methoxy-4-nitrophenyl)-5,10-dihydro-11H-dibenzo[b,e][1,4]diazepin-11-on;
7-[2-(4-Acetylpiperazin-1-yl)-2-oxoethyl]-3-(3-methoxy-4-nitrophenyl)-5,10-dihydro-11H-dibenzo[b,e][1,4]diazepin-11-on;
3-(3-Methoxy-4-nitrophenyl)-7-[2-oxo-2-(4-pyridin-2-ylpiperazin-1-yl)ethyl]-5,10-dihydro-11H-dibenzo[b,e] [1,4]diazepin-11-on;
3-({[3-(3-Methoxy-4-nitrophenyl)-11-oxo-10,11-dihydro-5H-dibenzo[b,e][1,4]diazepin-7-yl]acetyl}amino)benzamid;
2-[3-(3-Methoxy-4-nitrophenyl)-11-oxo-10,11-dihydro-5H-dibenzo[b,e][1,4]diazepin-7-yl]N(4-morpholin-4-ylphenyl)acetamid;
2-[3-(3-Methoxy-4-nitrophenyl)-11-oxo-10,11-dihydro-5H-dibenzo[b,e][1,4]diazepin-7-yl]Nchinolin-6-ylacetamid;
N-[(1S)-1-(Hydroxymethyl)-2-methylpropyl]-2-[3-(3-methoxy-4-nitrophenyl)-11-oxo-10,11-dihydro-5H-dibenzo[b,e] [1,4]diazepin-7-yl]acetamid;
(2S)-2-({[3-(3-Methoxy-4-nitrophenyl)-11-oxo-10,11-dihydro-5H-dibenzo[b,e][1,4]diazepin-7-yl]acetyl}amino)-4-methylpentanamid;
N-[(1S)-2-Hydroxy-1-phenylethyl]-2-[3-(3-methoxy-4-nitrophenyl)-11-oxo-10,11-dihydro-5H-dibenzo[b,e][1,4]diazepin-7-yl]acetamid;
N-(2,3-Dihydroxypropyl)-2-[3-(3-methoxy-4-nitrophenyl)-11-oxo-10,11-dihydro-5H-dibenzo[b,e][1,4]diazepin-7-yl]acetamid;
N-[3-(1H-Imidazol-1-yl)propyl]-2-[3-(3-methoxy-4-nitrophenyl)-11-oxo-10,11-dihydro-5H-dibenzo[b,e][1,4]diazepin-7-yl]acetamid;
2-[3-(3-Methoxy-4-nitrophenyl)-11-oxo-10,11-dihydro-5H-dibenzo[b,e][1,4]diazepin-7-yl]N[3-(2-oxopyrrolidin-1-yl)propyl]acetamid;
N-(2,6-Difluorbenzyl)-2-[3-(3-methoxy-4-nitrophenyl)-11-oxo-10,11-dihydro-5H-dibenzo[b,e][1,4]diazepin-7-yl]acetamid;
N-{2-[4-(Aminosulfonyl)phenyl]ethyl}-2-[3-(3-methoxy-4-nitrophenyl)-11-oxo-10,11-dihydro-5H-dibenzo[b,e][1,4]diazepin-7-yl]acetamid;
N-[(1R)-1-(Hydroxymethyl)-2-methylpropyl]-2-[3-(3-methoxy-4-nitrophenyl)-11-oxo-10,11-dihydro-5H-dibenzo[b,e] [1,4]diazepin-7-yl]acetamid;
N-[(1R)-2-Hydroxy-1-phenylethyl]-2-[3-(3-methoxy-4-nitrophenyl)-11-oxo-10,11-dihydro-5H-dibenzo[b,e][1,4]diazepin-7-yl]acetamid;
2-[3-(3-Methoxy-4-nitrophenyl)-11-oxo-10,11-dihydro-5H-dibenzo[b,e][1,4]diazepin-7-yl]N(thien-3-ylmethyl)acetamid;
Methyl {3-[4-(aminocarbonyl)-3-methoxyphenyl]-11-oxo-10,11-dihydro-5H-dibenzo[b,e][1,4]diazepin-8-yl}acetat;
8-Hydroxy-3-(3-methoxy-4-nitrophenyl)-5,10-dihydro-11H-dibenzo[b,e][1,4]diazepin-11-on;
8-Methoxy-3-(3-methoxy-4-nitrophenyl)-5,10-dihydro-1H-dibenzo[b,e][1,4]diazepin-11-on;
8-Ethoxy-3-(3-methoxy-4-nitrophenyl)-5,10-dihydro-1H-dibenzo[b,e][1,4]diazepin-11-on;
3-(3-Methoxy-4-nitrophenyl)-8-[2-(4-methyl-1,3-thiazol-5-yl)ethoxy]-5,10-dihydro-11H-dibenzo[b,e][1,4]diazepin-11-on;
8-[3-(Dimethylamino)propoxy]-3-(3-methoxy-4-nitrophenyl)-5,10-dihydro-11H-dibenzo[b,e][1,4]diazepin-11-on;
3-(3-Methoxy-4-nitrophenyl)-8-(2-morpholin-4-ylethoxy)-5,10-dihydro-11H-dibenzo[b,e][1,4]diazepin-11-on;
3-(3-Methoxy-4-nitrophenyl)-8-[2-(4-morpholin-4-ylphenyl)ethoxy]-5,10-dihydro-11H-dibenzo[b,e][1,4]diazepin-11-on;
3-(3-Methoxy-4-nitrophenyl)-7-piperidin-1-yl-5,10-dihydro-11H-dibenzo[b,e][1,4]diazepin-11-on;
7-[(2S)-2-(Hydroxymethyl)pyrrolidin-1-yl]-3-(3-methoxy-4-nitrophenyl)-5,10-dihydro-11H-dibenzo[b,e][1,4]diazepin-11-on;
3-(3-Methoxy-4-nitrophenyl)-7-morpholin-4-yl-5,10-dihydro-11H-dibenzo[b,e][1,4]diazepin-11-on;
7-(4-Hydroxypiperidin-1-yl)-3-(3-methoxy-4-nitrophenyl)-5,10-dihydro-11H-dibenzo[b,e][1,4]diazepin-11-on;
Methyl {3-[3-methoxy-4-(3-methyl-1,2,4-oxadiazol-5-yl)phenyl]-11-oxo-10,11-dihydro-5H-dibenzo[b,e][1,4]diazepin-8-yl}acetat;
8-(2-Ethyl-2-hydroxybutyl)-3-(3-methoxy-4-nitrophenyl)-5,10-dihydro-11H-dibenzo[b,e][1,4]diazepin-11-on;
3-[(2-Chlorpyridin-4-yl)amino]-8-(2-ethyl-2-hydroxybutyl)-5,10-diyhdro-11H-dibenzo[b,e][1,4]diazepin-11-on;
N,N-Dimethyl-2-[11-oxo-3-(pyridin-4-ylamino)-10,11-dihydro-5H-dibenzo[b,e][1,4]diazepin-8-yl]acetamid;
N-(4-Morpholin-4-ylphenyl)-2-[11-oxo-3-(pyridin-4-ylamino)-10,11-dihydro-5H-dibenzo[b,e][1,4]diazepin-8-yl]acetamid;
8-(2-Hydroxy-2-methylpropyl)-3-(pyrimidin-4-ylamino)-5,10-dihydro-11H-dibenzo[b,e][1,4]diazepin-11-on;
8-(2-Hydroxy-2-methylpropyl)-3-[(2-methylpyridin-4-yl)amino]-5,10-dihydro-11H-dibenzo[b,e][1,4]diazepin-11-on;
3-(3-Methoxy-4-nitrophenyl)-8-(2-oxopropyl)-5,10-dihydro-11H-dibenzo[b,e][1,4]diazepin-11-on;
3-[(2-Chlorpyridin-4-yl)amino]-8-(2-oxopropyl)-5,10-dihydro-11H-dibenzo[b,e][1,4]diazepin-11-on;
3-({2-[(2-Chlorpyridin-4-yl)-amino]pyridin-4-yl}amino)-8-(2-oxopropyl)-5,10-dihydro-11H-dibenzo[b,e][1,4]diazepin-11-on;
Methyl 2-methyl-2-[11-oxo-3-(pyrimidin-4-ylamino)-10,11-dihydro-5H-dibenzo[b,e][1,4]diazepin-8-yl]propanoat;
Methyl 2-methyl-2-13-[(2-methylpyridin-4-yl)amino]-11-oxo-10,11-dihydro-5H-dibenzo[b,e] [1,4]diazepin-8-yllpropanoat;
2-MethylN(4-morpholin-4-ylphenyl)-2-[11-oxo-3-(pyrimidin-4-ylamino)-10,11-dihydro-5H-dibenzo[b,e][1,4]diazepin-8-yl]propanamid;
2-Methyl-2-{3-[(2-methylpyridin-4-yl)amino]-11-oxo-10,11-dihydro-5H-dibenzo[b,e][1,4]diazepin-8-yl}N(4-morpholin-4-ylphenyl)propanamid;
3-{[3-(2-Hydroxyethyl)pyridin-4-yl]amino}-8-(1-hydroxy-1-methylethyl)-5,10-dihydro-11H-dibenzo[b,e][1,4]diazepin-11-on;
8-(2-Hydroxy-2-methylpropyl)-3-[(2-methoxypyridin-4-yl)amino]-5,10-dihydro-11H-dibenzo[b,e][1,4]diazepin-11-on;
8-(2-Hydroxy-2-methylpropyl)-3-[(2-methylpyridin-4-yl)amino]-5,10-dihydro-11H-dibenzo[b,e][1,4]diazepin-11-on;
Methyl 11-oxo-3-(pyrimidin-4-ylamino)-10,11-dihydro-5H-dibenzo[b,e][1,4]diazepin-7-carboxylat;
7-(1-Hydroxy-1-methylethyl)-3-(3-methoxy-4-nitrophenyl)-5,10-dihydro-11H-dibenzo[b,e][1,4]diazepin-11-on;
7-(1-Hydroxy-1-methylethyl)-3-(pyrimidin-4-ylamino)-5,10-dihydro-11H-dibenzo[b,e][1,4]diazepin-11-on;
2-{3-[(6-Methoxypyrimidin-4-yl)amino]-11-oxo-10,11-dihydro-5H-dibenzo[b,e][1,4]diazepin-8-yl)-2-methylN(4-morpholin-4-ylphenyl)propanamid;
3-(3-Methoxy-4-nitrophenyl)-8-{2-[(6-morpholin-4-ylpyridin-3-yl)oxy]ethyl}-5,10-dihydro-11H-dibenzo[b,e][1,4]diazepin-11-on;
3-(4-Hydroxy-3-methoxyphenyl)-8-[2-(4-morpholin-4-ylphenoxy)ethyl]-5,10-dihydro-11H-dibenzo[b,e][1,4]diazepin-11-on;
3-[(2,6-Difluorpyridin-4-yl)amino]-8-[2-(4-morpholin-4-ylphenoxy)ethyl]-5,10-dihydro-11H-dibenzo[b,e][1,4]diazepin-11-on;
7-Hydroxy-8-methoxy-3-(3-methoxy-4-nitrophenyl)-5,10-dihydro-11H-dibenzo[b,e][1,4]diazepin-11-on;
8-Hydroxy-7-methoxy-3-(3-methoxy-4-nitrophenyl)-5,10-dihydro-11H-dibenzo[b,e][1,4]diazepin-11-on;
8-Methoxy-3-(3-methoxy-4-nitrophenyl)-7-(tetrahydro-2H-pyran-2-ylmethoxy)-5,10-dihydro-11H-dibenzo[b,e][1,4]diazepin-11-on;
8-Methoxy-3-(3-methoxy-4-nitrophenyl)-7-[(1-methylpiperidin-3-yl)methoxy]-5,10-dihydro-11H-dibenzo[b,e] [1,4]diazepin-11-on;
8-Methoxy-3-(3-methoxy-4-nitrophenyl)-7-(pyridin-2-ylmethoxy)-5,10-dihydro-11H-dibenzo[b,e][1,4]diazepin-11-on;
8-Methoxy-3-(3-methoxy-4-nitrophenyl)-7-(pyridin-3-ylmethoxy)-5,10-dihydro-11H-dibenzo[b,e][1,4]diazepin-11-on;
8-Methoxy-3-(3-methoxy-4-nitrophenyl)-7-(pyridin-4-ylmethoxy)-5,10-dihydro-11H-dibenzo[b,e][1,4]diazepin-11-on;
8-Methoxy-3-(3-methoxy-4-nitrophenyl)-7-[(5-methylisoxazol-3-yl)methoxy]-5,10-dihydro-11H-dibenzo[b,e][1,4]diazepin-11-on;
8-Methoxy-3-(3-methoxy-4-nitrophenyl)-7-[(1-methyl-1H-imidazol-5-yl)methoxy]-5,10-dihydro-1H-dibenzo[b,e] [1,4]diazepin-11-on;
8-Methoxy-3-(3-methoxy-4-nitrophenyl)-7-[(2-methyl-1,3-thiazol-4-yl)methoxy]-5,10-dihydro-11H-dibenzo[b,e] [1,4]diazepin-11-on;
8-Methoxy-3-(3-methoxy-4-nitrophenyl)-7-[(2-oxo-1,3-oxazolidin-5-yl)methoxy]-5,10-dihydro-11H-dibenzo[b,e] [1,4]diazepin-11-on;
8-Methoxy-3-(3-methoxy-4-nitrophenyl)-7-(tetrahydrofuran-2-ylmethoxy)-5,10-dihydro-11H-dibenzo[b,e][1,4]diazepin-11-on;
7-[(2,2-Dimethyl-1,3-dioxolan-4-yl)methoxy]-8-methoxy-3-(3-methoxy-4-nitrophenyl)-5,10-dihydro-11H-dibenzo[b,e] [1,4]diazepin-11-on;
8-Methoxy-3-(3-methoxy-4-nitrophenyl)-7-[(2R)-pyrrolidin-2-ylmethoxy]-5,10-dihydro-11H-dibenzo[b,e][1,4]diazepin-11-on;
7,8-Dimethoxy-3-(3-methoxy-4-nitrophenyl)-5,10-dihydro-11H-dibenzo[b,e][1,4]diazepin-11-on;
8-Methoxy-7-[2-(2-methoxyethoxy)ethoxy]-3-(3-methoxy-4-nitrophenyl)-5,10-dihydro-11H-dibenzo[b,e][1,4]diazepin-11-on;
7-(2,3-Dihydroxypropoxy)-8-methoxy-3-(3-methoxy-4-nitrophenyl)-5,10-dihydro-11H-dibenzo[b,e][1,4]diazepin-11-on;
7-[3-Hydroxy-2,2-bis(hydroxymethyl)propoxy]-8-methoxy-3-(3-methoxy-4-nitrophenyl)-5,10-dihydro-11H-dibenzo[b,e] [1,4]diazepin-11-on;
2-Hydroxy-3-{[8-methoxy-3-(3-methoxy-4-nitrophenyl)-11-oxo-10,11-dihydro-5H-dibenzo[b,e][1,4]diazepin-7-yl]oxy}propan-1-sulfonsäure;
7-(3-Aminopropoxy)-8-methoxy-3-(3-methoxy-4-nitrophenyl)-5,10-dihydro-11H-dibenzo[b,e][1,4]diazepin-11-on;
7-[2-(Dimethylamino)ethoxy]-8-methoxy-3-(3-methoxy-4-nitrophenyl)-5,10-dihydro-11H-dibenzo[b,e][1,4]diazepin-11-on;
7-(2-Chlorethoxy)-8-methoxy-3-(3-methoxy-4-nitrophenyl)-5,10-dihydro-11H-dibenzo[b,e][1,4]diazepin-11-on;
8-Methoxy-3-(3-methoxy-4-nitrophenyl)-7-(2-pyrrolidin-1-ylethoxy)-5,10-dihydro-11H-dibenzo[b,e][1,4]diazepin-11-on;
8-Methoxy-3-(3-methoxy-4-nitrophenyl)-7-(2-morpholin-4-ylethoxy)-5,10-dihydro-11H-dibenzo[b,e][1,4]diazepin-11-on;
7-(4-Hydroxybutoxy)-8-methoxy-3-(3-methoxy-4-nitrophenyl)-5,10-dihydro-11H-dibenzo[b,e][1,4]diazepin-11-on;
7-(4-Hydroxybutoxy)-3-(4-hydroxy-3-methoxyphenyl)-8-methoxy-5,10-dihydro-11H-dibenzo[b,e][1,4]diazepin-11-on;
7-(4-Hydroxybutoxy)-8-methoxy-3-(pyrimidin-4-ylamino)-5,10-dihydro-11H-dibenzo[b,e][1,4]diazepin-11-on;
4-{[7-(4-Hydroxybutoxy)-8-methoxy-11-oxo-10,11-dihydro-5H-dibenzo[b,e][1,4]diazepin-3-yl]amino}pyridin-2-carbonitril;
3-[(2,6-Difluorpyridin-4-yl)amino]-7-(4-hydroxybutoxy)-8-methoxy-5,10-dihydro-11H-dibenzo[b,e][1,4]diazepin-11-on;
7-(4-Hydroxybutoxy)-8-methoxy-3-[(2,3,6-trifluorpyridin-4-yl)amino]-5,10-dihydro-11H-dibenzo[b,e][1,4]diazepin-11-on;
7-Ethoxy-3-(3-methoxy-4-nitrophenyl)-5,10-dihydro-11H-dibenzo[b,e][1,4]diazepin-11-on;
7-(4-Hydroxybutoxy)-3-(3-methoxy-4-nitrophenyl)-5,10-dihydro-11H-dibenzo[b,e][1,4]diazepin-11-on;
7-(2-Hydroxyethoxy)-3-(3-methoxy-4-nitrophenyl)-5,10-dihydro-11H-dibenzo[b,e][1,4]diazepin-11-on;
7-(2,3-Dihydroxypropoxy)-3-(3-methoxy-4-nitrophenyl)-5,10-dihydro-11H-dibenzo[b,e][1,4]diazepin-11-on;
7-[2-(2-Methoxyethoxy)ethoxy]-3-(3-methoxy-4-nitrophenyl)-5,10-dihydro-11H-dibenzo[b,e][1,4]diazepin-11-on;
7-(Methoxymethyl)-3-(3-methoxy-4-nitrophenyl)-5,10-dihydro-11H-dibenzo[b,e][1,4]diazepin-11-on;
7-(3-Methoxy-4-nitrobenzyl)-3-(3-methoxy-4-nitrophenyl)-5,10-dihydro-11H-dibenzo[b,e][1,4]diazepin-11-on;
7-{[[2-(Dimethylamino)ethyl](methyl)amino]methyl}-3-(3-methoxy-4-nitrophenyl)-5,10-dihydro-11H-dibenzo[b,e] [1,4]diazepin-11-on;
3-(3-Methoxy-4-nitrophenyl)-7-{[(2-tetrahydro-2H-pyran-4-ylethyl)amino]methyl}-5,10-dihydro-11H-dibenzo[b,e] [1,4]diazepin-11-on;
8-Ethyl-7-methoxy-3-(3-methoxy-4-nitrophenyl)-5,10-dihydro-11H-dibenzo[b,e][1,4]diazepin-11-on;
8-Methoxy-3-(3-methoxy-4-nitrophenyl)-7-vinyl-5,10-dihydro-11H-dibenzo[b,e][1,4]diazepin-11-on;
8-(3-Hydroxypropyl)-7-methoxy-3-(3-methoxy-4-nitrophenyl)-5,10-dihydro-11H-dibenzo[b,e][1,4]diazepin-11-on;
7-Methoxy-3-(3-methoxy-4-nitrophenyl)-8-{3-[(2-methylpyridin-3-yl)oxy]propyl}-5,10-dihydro-11H-dibenzo[b,e] [1,4]diazepin-11-on;
8-{3-[(2-Chlorpyridin-3-yl)oxy]propyl}-7-methoxy-3-(3-methoxy-4-nitrophenyl)-5,10-dihydro-11H-dibenzo[b,e] [1,4]diazepin-11-on;
7-Methoxy-3-(3-methoxy-4-nitrophenyl)-8-[3-(4-morpholin-4-ylphenoxy)propyl]-5,10-dihydro-11H-dibenzo[b,e][1,4]diazepin-11-on;
8-[3-(Isochinolin-3-yloxy)propyl]-7-methoxy-3-(3-methoxy-4-nitrophenyl)-5,10-dihydro-11H-dibenzo[b,e][1,4]diazepin-11-on;
7-Methoxy-3-(3-methoxy-4-nitrophenyl)-8-[3-(3-oxoisochinolin-2(3H)-yl)propyl]-5,10-dihydro-11H-dibenzo[b,e] [1,4]diazepin-11-on;
Methyl 7-methoxy-3-(3-methoxy-4-nitrophenyl)-11-oxo-10,11-dihydro-5H-dibenzo[b,e][1,4]diazepin-8-carboxylat;
Methyl 7-methoxy-11-oxo-3-(pyrimidin-4-ylamino)-10,11-dihydro-5H-dibenzo[b,e][1,4]diazepin-8-carboxylat;
3-[(2,6-Difluorpyridin-4-yl)amino]-8-(1,1-dimethyl-2-morpholin-4-yl-2-oxoethyl)-5,10-dihydro-11H-dibenzo[b,e] [1,4]diazepin-11-on;
2-{3-[(2,6-Difluorpyridin-4-yl)amino]-11-oxo-10,11-dihydro-5H-dibenzo[b,e][1,4]diazepin-8-yl}-N,N,2-trimethylpropanamid;
2-{3-[(2,6-Difluorpyridin-4-yl)amino]-11-oxo-10,11-dihydro-5H-dibenzo[b,e][1,4]diazepin-8-yl}-2-methylNpyridin-4-ylpropanamid;
2-{3-[(2,6-Difluorpyridin-4-yl)amino]-11-oxo-10,11-dihydro-5H-dibenzo[b,e][1,4]diazepin-8-yl}-2-methylN1,3-thiazol-2-ylpropanamid;
3-[(2,6-Difluorpyridin-4-yl)amino]-8-{2-[(3R)-3-hydroxypiperidin-1-yl]-1,1-dimethyl-2-oxoethyl}-5,10-dihydro-11H-dibenzo[b,e][1,4]diazepin-11-on;
3-[(2,6-Difluorpyridin-4-yl)amino]-8-[2-(4-hydroxypiperidin-1-yl)-1,1-dimethyl-2-oxoethyl]-5,10-dihydro-11H-dibenzo[b,e][1,4]diazepin-11-on;
3-[(2,6-Difluorpyridin-4-yl)amino]-8-{2-[(2S)-2-(hydroxymethyl)pyrrolidin-1-yl]-1,1-dimethyl-2-oxoethyl}-5,10-dihydro-11H-dibenzo[b,e][1,4]diazepin-11-on;
3-[(2,6-Difluorpyridin-4-yl)amino]-8-(1,1-dimethyl-2-oxo-2-pyrrolidin-1-ylethyl)-5,10-dihydro-11H-dibenzo[b,e] [1,4]diazepin-11-on;
2-{3-[(2,6-Difluorpyridin-4-yl)amino]-11-oxo-10,11-dihydro-5H-dibenzo[b,e][1,4]diazepin-8-yl}-2-methylNpyridin-2-ylpropanamid;
2-{3-[(2,6-Difluorpyridin-4-yl)amino]-11-oxo-10,11-dihydro-5H-dibenzo[b,e][1,4]diazepin-8-yl}-2-methylNpyridin-3-ylpropanamid;
2-{3-[(2,6-Difluorpyridin-4-yl)amino]-11-oxo-10,11-dihydro-5H-dibenzo[b,e][1,4]diazepin-8-yl}N(4-fluorphenyl)-2-methylpropanamid;
3-[(2,6-Difluorpyridin-4-yl)amino]-8-{2-[(2R)-2-(hydroxymethyl)pyrrolidin-1-yl]-1,1-dimethyl-2-oxoethyl}-5,10-dihydro-11H-dibenzo[b,e][1,4]diazepin-11-on;
8-Methoxy-3-(3-methoxy-4-nitrophenyl)-7-(3-morpholin-4-yl-3-oxopropyl)-5,10-dihydro-11H-dibenzo[b,e][1,4]diazepin-11-on;
3-[3-(3-Methoxy-4-nitrophenyl)-11-oxo-10,11-dihydro-5H-dibenzo[b,e][1,4]diazepin-8-yl]Npyridin-3-ylpropanamid;
3-[3-(3-Methoxy-4-nitrophenyl)-11-oxo-10,11-dihydro-5H-dibenzo[b,e][1,4]diazepin-8-yl]Npyridin-4-ylpropanamid;
8-[3-(3-Hydroxypiperidin-1-yl)-3-oxopropyl]-3-(3-methoxy-4-nitrophenyl)-5,10-dihydro-11H-dibenzo[b,e][1,4]diazepin-11-on;
N-(4-Fluorphenyl)-3-[3-(3-methoxy-4-nitrophenyl)-11-oxo-10,11-dihydro-5H-dibenzo[b,e][1,4]diazepin-8-yl]propanamid;
3-[3-(3-Methoxy-4-nitrophenyl)-11-oxo-10,11-dihydro-5H-dibenzo[b,e][1,4]diazepin-8-yl]N[4-(trifluormethyl)phenyl]propanamid;
3-[3-(3-Methoxy-4-nitrophenyl)-11-oxo-10,11-dihydro-5H-dibenzo[b,e][1,4]diazepin-8-yl]Nmethylpropanamid;
3-[8-Methoxy-3-(3-methoxy-4-nitrophenyl)-11-oxo-10,11-dihydro-5H-dibenzo[b,e][1,4]diazepin-7-yl]-N,N-dimethylpropanamid;
8-{2-[(6-Chlorpyridin-3-yl)oxy]ethyl}-3-(3-methoxy-4-nitrophenyl)-5,10-dihydro-11H-dibenzo[b,e][1,4]diazepin-11-on;
8-{2-[(2-Chlorpyridin-3-yl)oxy]ethyl}-3-(3-methoxy-4-nitrophenyl)-5,10-dihydro-11H-dibenzo[b,e][1,4]diazepin-11-on;
3-(3-Methoxy-4-nitrophenyl)-8-{2-[(6-methylpyridin-3-yl)oxy]ethyl}-5,10-dihydro-11H-dibenzo[b,e][1,4]diazepin-11-on;
3-(3-Methoxy-4-nitrophenyl)-8-{2-[(2-methylpyridin-3-yl)oxy]ethyl}-5,10-dihydro-11H-dibenzo[b,e][1,4]diazepin-11-on;
3-(3-Methoxy-4-nitrophenyl)-8-[2-(pyridin-3-yloxy)ethyl]-5,10-dihydro-11H-dibenzo[b,e][1,4]diazepin-11-on;
8-{2-[(2,6-Dimethylpyridin-3-yl)oxy]ethyl}-3-(3-methoxy-4-nitrophenyl)-5,10-dihydro-11H-dibenzo[b,e][1,4]diazepin-11-on;
8-[2-({2-[(Dimethylamino)methyl]pyridin-3-yl}oxy)ethyl]-3-(3-methoxy-4-nitrophenyl)-5,10-dihydro-11H-dibenzo[b,e] [1,4]diazepin-11-on;
8-[2-(Isochinolin-7-yloxy)ethyl]-3-(3-methoxy-4-nitrophenyl)-5,10-dihydro-11H-dibenzo[b,e][1,4]diazepin-11-on;
7-Methoxy-3-(3-methoxy-4-nitrophenyl)-N,N-dimethyl-11-oxo-10,11-dihydro-5H-dibenzo[b,e][1,4]diazepin-8-carboxamid;
7-Methoxy-3-(3-methoxy-4-nitrophenyl)-11-oxo-10,11-dihydro-5H-dibenzo[b,e][1,4]diazepin-8-carbonsäure;
7-{2-[(2-Chlorpyridin-3-yl)oxy]ethyl}-8-methoxy-3-(3-methoxy-4-nitrophenyl)-5,10-dihydro-11H-dibenzo[b,e] [1,4]diazepin-11-on;
8-[2-(Isochinolin-5-yloxy)ethyl]-3-(3-methoxy-4-nitrophenyl)-5,10-dihydro-11H-dibenzo[b,e][1,4]diazepin-11-on;
3-(3-Methoxy-4-nitrophenyl)-8-[2-(chinolin-5-yloxy)ethyl]-5,10-dihydro-11H-dibenzo[b,e][1,4]diazepin-11-on;
3-(3-Methoxy-4-nitrophenyl)-8-[2-(4-methoxyphenoxy)ethyl]-5,10-dihydro-11H-dibenzo[b,e][1,4]diazepin-11-on;
3-(3-Methoxy-4-nitrophenyl)-8-[2-(3-methoxyphenoxy)ethyl]-5,10-dihydro-11H-dibenzo[b,e][1,4]diazepin-11-on;
3-{2-[3-(3-Methoxy-4-nitrophenyl)-11-oxo-10,11-dihydro-5H-dibenzo[b,e][1,4]diazepin-8-yl]ethoxy}pyridin-2-carboxamid;
3-(3-Methoxy-4-nitrophenyl)-8-[3-(4-morpholin-4-ylphenoxy)propyl]-5,10-dihydro-11H-dibenzo[b,e][1,4]diazepin-11-on;
3-(3-Methoxy-4-nitrophenyl)-8-[3-(pyridin-3-yloxy)propyl]-5,10-dihydro-11H-dibenzo[b,e][1,4]diazepin-11-on;
8-[2-(3-Aminophenoxy)ethyl]-3-(3-methoxy-4-nitrophenyl)-5,10-dihydro-11H-dibenzo[b,e][1,4]diazepin-11-on;
3-(3-Methoxy-4-nitrophenyl)-8-{2-[(2-methyl-1,3-benzothiazol-7-yl)oxy]ethyl}-5,10-dihydro-11H-dibenzo[b,e] [1,4]diazepin-11-on;
2-[3-(3-Methoxy-4-nitrophenyl)-11-oxo-10,11-dihydro-5H-dibenzo[b,e][1,4]diazepin-8-yl]-2-methylN(pyridin-2-ylmethyl)propanamid;
8-(2-Hydroxy-2-methylpropyl)-3-(3-methoxy-4-nitrophenyl)-5,10-dihydro-11H-dibenzo[b,e][1,4]diazepin-11-on;
3-(3-Methoxy-4-nitrophenyl)-8-[(4-methylpiperazin-1-yl)methyl]-5,10-dihydro-11H-dibenzo[b,e][1,4]diazepin-11-on;
3-(4-Chlor-3-methoxyphenyl)-8-[(4-methylpiperazin-1-yl)methyl]-5,10-dihydro-11H-dibenzo[b,e][1,4]diazepin-11-on;
2-Methoxy-4-{8-[(4-methylpiperazin-l-yl)methyl]-ll-oxo-10,11-dihydro-5H-dibenzo[b,e][1,4]diazepin-3-yl}benzonitril;
3-(4-Hydroxy-3-methoxyphenyl)-8-(hydroxymethyl)-5,10-dihydro-11H-dibenzo[b,e][1,4]diazepin-11-on;
3-(3-Methoxy-4-nitrophenyl)-8-(morpholin-4-ylmethyl)-5,10-dihydro-11H-dibenzo[b,e][1,4]diazepin-11-on;
8-{[(2R)-2-(Hydroxymethyl)pyrrolidin-1-yl]methyl}-3-(3-methoxy-4-nitrophenyl)-5,10-dihydro-11H-dibenzo[b,e] [1,4]diazepin-11-on;
7-(2-Hydroxyethoxy)-8-methoxy-3-(3-methoxy-4-nitrophenyl)-5,10-dihydro-11H-dibenzo[b,e][1,4]diazepin-11-on;
8-{3-[2-(Hydroxymethyl)pyrrolidin-l-yl]-3-oxopropyl}-3-(3-methoxy-4-nitrophenyl)-5,10-dihydro-11H-dibenzo[b,e] [1,4]diazepin-11-on;
(cis)4-HydroxyN[3-(3-methoxy-4-nitrophenyl)-11-oxo-10,11-dihydro-5H-dibenzo[b,e][1,4]diazepin-7-yl]cyclohexancarboxamid.

17. Eine pharmazeutische Zusammensetzung, die eine Verbindung mit der Formel (I) 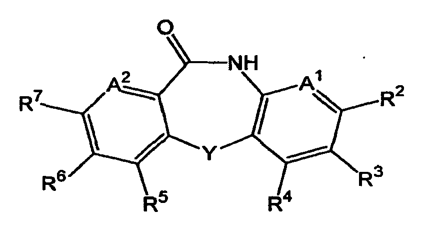 umfasst, oder ein therapeutisch verträgliches Salz davon, in Kombination mit einem therapeutisch verträglichen Träger, worin
(a)
A¹ CH ist;
A² CH ist;
R² gewählt ist aus der Gruppe bestehend aus Wasserstoff, Alkenyl, Alkoxy, Alkoxyalkoxy, Alkoxyalkoxyalkoxy, Alkoxyalkyl, Alkoxycarbonyl, Alkoxycarbonylalkyl, Alkyl, Alkylcarbonyl, Alkylcarbonylalkyl, Amino, Aminoalkoxy, Aminoalkyl, Aminocarbonyl, Aminocarbonylalkyl, Aminosulfonyl, Aryl, Arylalkoxy, Arylalkyl, Aryloxyalkyl, Carboxy, Carboxyalkyl, Cyano, Cyanoalkyl, Cycloalkyl, Cycloalkylalkyl, Halo, Haloalkoxy, Haloalkyl, Heterocyclyl, Heterocyclylalkoxy, Heterocyclylalkyl, Heterocyclylcarbonylalkyl, Heterocyclyloxyalkyl, Hydroxy, Hydroxyalkoxy, Hydroxyalkyl, Nitro, Nitroalkyl und - (CR⁹R¹⁰)ₘC (O) NR¹¹R¹²;
R³, R⁴ und R⁵ jeweils Wasserstoff sind;
R⁶ gewählt ist aus der Gruppe bestehend aus Aryl, Cycloalkyl, Halo, Heterocyclyl und -XR¹³;
R⁷ Wasserstoff ist;
R⁹ und R¹⁰ sind unabhängig gewählt aus der Gruppe bestehend aus Wasserstoff, Alkenyl, Alkyl, Aminoalkyl und Hydroxyalkyl; oder
R⁹ und R¹⁰ bilden, zusammen mit dem Kohlenstoffatom, an welches sie gebunden sind, eine Cycloalkylgruppe;
R¹¹ und R¹² sind jeweils unabhängig gewählt aus der Gruppe bestehend aus Wasserstoff, Alkenyl, Alkoxyalkyl, Alkyl, Amino, Aminoalkyl, Aryl, Arylalkyl, Cyanoalkyl, Cycloalkyl, Cycloalkylalkyl, Haloalkyl, Heterocyclyl, Heterocyclylalkyl, Hydroxyalkyl, -NR^{c}R^{d}, (NR^{c}R^{d})Alkyl und (NR^{c}R^{d}) Carbonylalkyl; oder
R¹¹ und R¹² bilden, zusammen mit dem Stickstoffatom, an welches sie gebunden sind, einen Heterocyclylring, gewählt aus der Gruppe bestehend aus Azetidinyl, Diazepanyl, Imidazolidinyl, Morpholinyl, Piperazinyl, Piperidinyl, Pyrrolidinyl und Thiomorpholinyl, welche jeweils wahlweise substituiert sein können mit einem, zwei oder drei Substituenten, unabhängig gewählt aus der Gruppe bestehend aus Alkoxy, Alkoxycarbonyl, Alkenyl, Alkyl, Alkylcarbonyl, Aryl, Carboxy, Carboxyalkyl, Heterocyclyl, Heterocyclylalkyl, Hydroxy, Hydroxyalkyl, -NR^{c}R^{d}, (NR^{c}R^{d}) Alkyl und (NR^{c}R^{d}) Carbonyl;
R¹³ ist gewählt aus der Gruppe bestehend aus Aryl, Cycloalkyl und Heterocyclyl;
X ist gewählt aus der Gruppe bestehend aus -O-, -NR¹⁴-, -C(O)-, -S-, -SO₂-, -(CH₂)ₙ-, -C(O)NR¹⁴-, -NR¹⁴C(O)-, -SO₂NR¹⁴-, -NR¹⁴SO₂, -O(CH₂)ₘ-, -(CH₂)ₘO-, -CH=CH- und -C≡C-; worin R¹⁴ gewählt ist aus der Gruppe bestehend aus Wasserstoff, Alkenyl, Alkyl, Aminoalkyl und Hydroxyalkyl;
Y ist NH;
m ist 0-3 und
n ist 1-3;
oder worin
(b)
A¹ CH ist;
A² CH ist;
Y NH ist;
R⁴, R⁵ und R⁷ jeweils Wasserstoff sind;
R⁶ Phenyl ist, substituiert in der dritten Position mit Methoxy und substituiert in der vierten Position mit Alkoxycarbonyl, Alkoxycarbonylalkenyl, Alkylcarbonyl, Alkylsulfonyl, Cyano, Halo, Haloalkenyl, Hydroxy, Nitro, NH₂SO₂- oder NH₂CO-; und
entweder
(i) ist R² Wasserstoff und R³ ist -(CR⁹R¹⁰)C(O)NHR¹², worin R⁹ und R¹⁰ unabhängig gewählt sind aus der Gruppe bestehend aus Wasserstoff, Alkenyl, Alkyl, Aminoalkyl und Hydroxyalkyl, oder R⁹ und R¹⁰, zusammen mit dem Kohlenstoffatom, an welches sie gebunden sind, bilden eine Cycloalkylgruppe und R¹² ist Phenyl, wahlweise substituiert mit einer Morpholinylgruppe;
(ii) ist R² Wasserstoff und R³ ist Hydroxyalkyl;
(iii) ist R² Wasserstoff und R³ ist Alkoxy;
(iv) sind R² und R³ unabhängig -(CR⁹R¹⁰)C(O)NHR¹², worin R⁹ und R¹⁰ unabhängig gewählt sind aus der Gruppe bestehend aus Wasserstoff, Alkenyl, Alkyl, Aminoalkyl und Hydroxyalkyl, oder R⁹ und R¹⁰, zusammen mit dem Kohlenstoffatom, an welches sie gebunden sind, bilden eine Cycloalkylgruppe und R¹² ist Phenyl,
wahlweise substituiert mit einer Morpholinylgruppe;
(v) sind R² und R³ unabhängig Hydroxyalkyl; oder
(vi) R² und R³ sind unabhängig Alkoxy;
worin
Alkyl ein C₁-C₁₀-Alkyl ist;
Alkenyl ein C₂-C₆-Alkenyl ist;
Alkoxy ein C₁-C₁₀-Alkoxy ist;
Aryl eine Phenylgruppe oder ein bizyklisches oder trizyklisches ankondensiertes Ringsystem ist, worin einer oder mehrere der ankondensierten Ringe eine Phenylgruppe ist, worin Aryl wahlweise substituiert sein kann mit ein, zwei, drei, vier oder fünf Substituenten, unabhängig gewählt aus der Gruppe bestehend aus Alkoxy, Alkoxyalkyl, Alkoxycarbonyl, Alkoxycarbonylalkenyl, Alkoxycarbonylalkyl, Alkoxysulfonyl, Alkyl, Alkylcarbonyl, Alkylsulfonyl, Amino, Aminocarbonyl, Aminosulfonyl, einer zweiten Arylgruppe, Arylalkyl, Arylsulfonyl, Carboxy, Cyano, Formyl, Halo, Haloalkoxy, Haloalkenyl, Haloalkyl, Heterocyclyl, Heterocyclylalkyl, Hydroxy, Hydroxyalkyl, Nitro, (NR^{c}R^{d}) Alkyl und Oxo, worin die zweite Arylgruppe, der Arylteil von Arylalkyl und das Arylsulfonyl, das Heterocyclyl und der Heterocyclylteil von Heterocyclylalkyl wahlweise substituiert sein können mit einem, zwei, drei, vier oder fünf Substituenten, unabhängig gewählt aus der Gruppe bestehend aus Alkoxy, Alkoxyalkyl, Alkoxycarbonyl, Alkyl, Alkylcarbonyl, Carboxy, Cyano, Halo, Haloalkoxy, Haloalkyl, Hydroxy, Nitro und Oxo;
Heterocyclyl gewählt ist aus einem fünf-, sechs- oder siebengliedrigen Ring, der ein, zwei oder drei Heteroatome enthält, unabhängig gewählt aus der Gruppe bestehend aus Stickstoff, Sauerstoff und Schwefel, worin der fünfgliedrige Ring null bis zwei Doppelbindungen hat und die sechs- und siebengliedrigen Ringe null bis drei Doppelbindungen haben; bizyklischen Gruppen, in welchen der Heterocyclylring ankondensiert ist an eine Phenylgruppe, eine monozyklische Cycloalkenylgruppe, eine monozyklische Cycloalkylgruppe oder eine andere monozyklische Heterocyclylgruppe; und trizyklischen Gruppen, in welchen ein bizyklisches System ankondensiert ist an eine Phenylgruppe, eine monozyklische Cycloalkenylgruppe, eine monozyklische Cycloalkylgruppe oder eine andere monozyklische Heterocyclylgruppe; worin Heterocyclyl wahlweise substituiert sein kann mit einem, zwei, drei, vier oder fünf Substituenten, unabhängig gewählt aus der Gruppe bestehend aus Alkoxy, Alkoxyalkyl, Alkoxycarbonyl, Alkyl, Alkylcarbonyl, Alkylsulfonyl, Amino, Aminocarbonyl, Aminosulfonyl, Aryl, Arylalkyl, Arylsulfonyl, Carboxy, Cyano, Halo, einer zweiten Heterocyclylgruppe, Heterocyclylalkyl, Hydroxy, Hydroxyalkyl, Nitro, -NR^{c}R^{d}, (NR^{c}R^{d})Alkyl und Oxo, worin das Aryl, der Arylteil des Arylalkyls und des Arylsulfonyls, die zweite Heterocyclylgruppe und der Heterocyclylteil von Heterocyclylalkyl wahlweise substituiert sein können mit einem, zwei, drei, vier oder fünf Substituenten, unabhängig gewählt aus der Gruppe bestehend aus Alkoxy, Alkoxyalkyl, Alkoxycarbonyl, Alkyl, Alkylcarbonyl, Carboxy, Cyano, Halo, Haloalkoxy, Haloalkyl, Hydroxy, Methylendioxy, Nitro und Oxo;
Cycloalkyl ein gesättigtes monozyklisches, bizyklisches oder trizyklisches Kohlenwasserstoffringsystem ist, das drei bis zwölf Kohlenstoffatome hat, worin Cycloalkyl wahlweise substituiert sein kann mit einem, zwei, drei oder vier Substituenten, unabhängig gewählt aus der Gruppe bestehend aus Alkoxy, Alkoxyalkyl, Alkoxycarbonyl, Alkyl, Alkylcarbonyl, Alkylsulfonyl, -NR^{c}R^{d}, (NR^{c}R^{d})Alkyl, (NR^{c}R^{d})Carbonyl, einer zweiten Arylgruppe, Arylalkyl, Arylsulfonyl, Carboxy, Cyano, Halo, Heterocyclyl, Heterocyclylalkyl, Hydroxy, Hydroxyalkyl, Nitro und Oxo, worin die zweite Arylgruppe, der Arylteil des Arylalkyls und des Arylsulfonyls, das Heterocyclyl und der Heterocyclylteil von Heterocyclylalkyl wahlweise substituiert sein können mit einem, zwei, drei, vier oder fünf Substituenten, unabhängig gewählt aus der Gruppe bestehend aus Alkoxy, Alkoxyalkyl, Alkoxycarbonyl, Alkyl, Alkylcarbonyl, Carboxy, Cyano, Halo, Haloalkoxy, Haloalkyl, Hydroxy, Nitro und Oxo;
Cycloalkenyl ein nicht aromatisches zyklisches oder bizyklisches Ringsystem ist, das drei bis zehn Kohlenstoffatome und und einen bis drei Ringe hat, worin jeder fünfgliedrige Ring eine Doppelbindung hat, jeder sechsgliedrige Ring eine oder zwei Doppelbindungen hat, jeder sieben- und achtgliedrige Ring eine bis drei Doppelbindungen hat und jeder neun- bis zehngliedrige Ring eine bis vier Doppelbindungen hat;
Amino bezeichnet -NR^{a}R^{b}, worin R^{a} und R^{b} unabhängig gewählt sind aus der Gruppe bestehend aus Wasserstoff, Alkoxyalkylcarbonyl, Alkoxycarbonyl, Alkyl, Alkylcarbonyl, Alkylsulfonyl, Aryl, Arylalkyl, Arylalkylcarbonyl, Arylalkylsulfonyl, Arylsulfonyl, Arylsulfonylalkyl, Arylsulfonylalkylcarbonyl, Cycloalkyl, Cycloalkylalkyl, Cycloalkylcarbonyl, Haloalkylsulfonyl, Heterocyclyl, Heterocyclylalkylcarbonyl, Heterocyclylalkylsulfonyl, Heterocyclylcarbonyl, Heterocyclylsulfonyl, -NR^{c}R^{d}, (NR^{c}R^{d})Alkyl, (NR^{c}R^{d}) Alkylcarbonyl, (NR^{c}R^{d}) Alkylsulfonyl, (NR^{c}R^{d}) Carbonyl, (NR^{c}R^{d})Carbonylalkyl und (NR^{c}R^{d}) Cabonylalkylcarbonyl, worin Aryl wahlweise substituiert sein kann mit einem, zwei, drei, vier oder fünf Substituenten, unabhängig gewählt aus der Gruppe bestehend aus Alkoxy, Alkoxyalkyl, Alkoxycarbonyl, Alkyl, Alkylcarbonyl, Alkylsulfonyl, einer zweiten Arylgruppe, Arylalkyl, Arylsulfonyl, Carboxy, Cyano, Halo, Heterocyclyl, Heterocyclylalkyl, Hydroxy, Nitro und Oxo, worin die zweite Arylgruppe, der Arylteil des Arylalkyls und des Arylsulfonyls, das Heterocyclyl und der Heterocyclylteil von Heterocyclylalkyl wahlweise substituiert sein können mit einem, zwei, drei, vier oder fünf Substituenten, unabhängig gewählt aus der Gruppe bestehend aus Alkoxy, Alkoxyalkyl, Alkoxycarbonyl, Alkyl, Alkylcarbonyl, Carboxy, Cyano, Halo, Haloalkoxy, Haloalkyl, Hydroxy, Nitro und Oxo; und
R^{c} und R^{d} jeweils unabhängig Wasserstoff, Alkoxycarbonyl, Alkyl, Alkylcarbonyl, Aryl, Arylsulfonyl, Heterocyclcarbonyl oder Formyl sind.

18. Eine pharmazeutische Zusammensetzung, wie definiert in Anspruch 17, oder ein therapeutisch verträgliches Salz davon, für die Verwendung in der Behandlung primärer und metastatischer fester Tumore.

19. Eine pharmazeutische Zusammensetzung gemäß Anspruch 18 für die Verwendung in der Behandlung von Karzinomen der Brust, des Kolons, des Rektums, der Lunge, des Oropharynx, des Hypopharynx, der Speiseröhre, des Magens, der Bauchspeicheldrüse, der Leber, der Gallenblase und der Gallengänge, des Dünndarms, des Harntrakts, des weiblichen Genitaltrakts, des männlichen Genitaltrakts, der endokrinen Drüsen und der Haut; Hämangiomen, Melanomen, Sarkomen, einschließlich solcher, die aus Knochen und Bindegewebe entstehen, sowie Kaposi-Sarkom, Tumoren des Gehirns, der Nerven, Augen und Hirnhäute, einschließlich Astrozytomen, Gliomen, Glioblastomen, Retinoblastomen, Neuromen, Neuroblastomen, Schwannomen und Meningiomen; festen Tumoren, die aus hämatopoietischen Malignitäten, wie zum Beispiel Leukämien oder Lymphomen, entstehen; oder zur Vorbeugung von Metastasen aus den oben genannten Tumoren, wenn sie alleine oder in Kombination mit Strahlentherapie und/oder anderen Chemotherapeutika verwendet wird.

20. Eine pharmazeutische Zusammensetzung wie in Anspruch 17 definiert oder ein therapeutisch verträgliches Salz davon für die Verwendung in der Behandlung von Krebs bei einem Patienten, bei dem die Notwendigkeit einer solchen Behandlung erkannt wurde.

21. Eine pharmazeutische Zusammensetzung wie in Anspruch 17 definiert, für die Verwendung als ein pharmazeutisches Mittel.

## Revendications

1. Composé de formule (I) 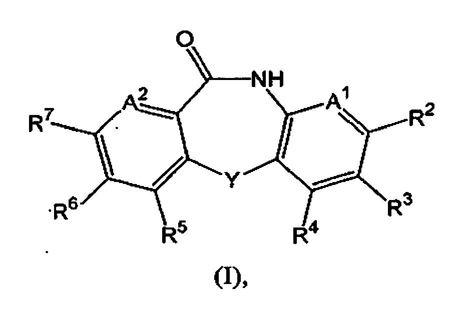 ou un sel thérapeutiquement acceptable de celui-ci, dans lequel
(a)
A¹ est CH ;
A² est CH ;
R² est choisi dans le groupe constitué d'hydrogène, alcényle, alcoxy, alcoxyalcoxy, alcoxyalcoxyalcoxy, alcoxyalkyle, alcoxycarbonyle, alcoxycarbonylalkyle, alkyle, alkylcarbonyle, alkylcarbonylalkyle, amino, aminoalcoxy, aminoalkyle, aminocarbonyle, aminocarbonylalkyle, aminosulfonyle, aryle, arylalcoxy, arylalkyle, aryloxyalkyle, carboxy, carboxyalkyle, cyano, cyanoalkyle, cycloalkyle, cycloalkylalkyle, halogéno, halogénoalcoxy, halogénoalkyle, hétérocyclyle, hétérocyclylalcoxy, hétérocyclylalkyle, hétérocyclylcarbonylalkyle, hétérocyclyloxyalkyle, hydroxy, hydroxyalcoxy, hydroxyalkyle, nitro, nitroalkyle, et - (CR⁹R¹⁰)ₘC(O)NR¹¹R¹² ;
R³, R⁴ et R⁵ sont chacun hydrogène ;
R⁶ est choisi dans le groupe constitué d'aryle, cycloalkyle, halogéno, hétérocyclyle, et -XR¹³ ;
R⁷ est hydrogène ;
R⁹ et R¹⁰ sont indépendamment choisis dans le groupe constitué d'hydrogène, alcényle, alkyle, aminoalkyle, et hydroxyalkyle ; ou
R⁹ et R¹⁰, conjointement avec l'atome de carbone auquel ils sont liés, forment un groupe cycloalkyle ;
R¹¹ et R¹² sont chacun indépendamment choisis dans le groupe constitué d'hydrogène, alcényle, alcoxyalkyle, alkyle, amino, aminoalkyle, aryle, arylalkyle, cyanoalkyle, cycloalkyle, cycloalkylalkyle, halogénoalkyle, hétérocyclyle, hétérocyclylalkyle, hydroxyalkyle, -NR^{c}R^{d}, (NR^{c}R^{d})alkyle, et (NR^{c}R^{d})carbonylalkyle ; ou
R¹¹ et R¹², conjointement avec l'atome d'azote auquel ils sont liés, forment un cycle hétérocyclyle choisi dans le groupe constitué d'azétidinyle, diazépanyle, imidazolidinyle, morpholinyle, pipérazinyle, pipéridinyle, pyrrolidinyle et thiomorpholinyle, qui peuvent chacun être facultativement substitués par un, deux ou trois substituants indépendamment choisis dans le groupe constitué d'alcoxy, alcoxycarbonyle, alcényle, alkyle, alkylcarbonyle, aryle, carboxy, carboxyalkyle, hétérocyclyle, hétérocyclylalkyle, hydroxy, hydroxyalkyle, -NR^{c}R^{d}, (NR^{c}R^{d})alkyle, et (NR^{c}R^{d})carbonyle ;
R¹³ est choisi dans le groupe constitué d'aryle, cycloalkyle, et hétérocyclyle ;
X est choisi dans le groupe constitué de -O-, -NR¹⁴-, -C(O)-, -S-, -SO₂-, -(CH₂)ₙ-, -C(O)NR¹⁴-, -NR¹⁴C(O)-, -SO₂NR¹⁴-, -NR¹⁴SO₂, -O(CH₂)ₘ-, - (CH₂)ₘO-, -CH=CH-, et -C≡C- ; où R¹⁴ est choisi dans le groupe constitué d'hydrogène, alcényle, alkyle, aminoalkyle, et hydroxyalkyle ;
Y est NH ;
m est 0 à 3 ; et
n est 1 à 3 ;
ou dans lequel
(b)
A¹ est CH ;
A² est CH ;
Y est NH;
R⁴, R⁵, et R⁷ sont chacun hydrogène ;
R⁶ est phényle substitué à la position trois par méthoxy et substitué à la position quatre par alcoxycarbonyle, alcoxycarbonylalcényle, alkylcarbonyle, alkylsulfonyle, cyano, halogéno, halogénoalcényle, hydroxy, nitro, NH₂SO₂-, ou NH₂CO- ; et soit
(i) R² est hydrogène et R³ est -(CR⁹R¹⁰)C(O)NHR¹² où R⁹ et R¹⁰ sont indépendamment choisis dans le groupe constitué d'hydrogène, alcényle, alkyle, aminoalkyle, et hydroxyalkyle, ou R⁹ et R¹⁰, conjointement avec l'atome de carbone auquel ils sont liés, forment un groupe cycloalkyle, et R¹² est phényle facultativement substitué par 1 groupe morpholinyle ;
(ii) R² est hydrogène et R³ est hydroxyalkyle ;
(iii) R² est hydrogène et R³ est alcoxy ;
(iv) R² et R³ sont indépendamment -(CR⁹R¹⁰)C(O)NHR¹² où R⁹ et R¹⁰ sont indépendamment choisis dans le groupe constitué d'hydrogène, alcényle, alkyle, aminoalkyle, et hydroxyalkyle, ou R⁹ et R¹⁰, conjointement avec l'atome de carbone auquel ils sont liés, forment un groupe cycloalkyle, et R¹² est phényle facultativement substitué par 1 groupe morpholinyle ;
(v) R² et R³ sont indépendamment hydroxalkyle ; ou
(vi) R² et R³ sont indépendamment alcoxy ;
où
alkyle est un alkyle en C₁C₁₀ ;
alcényle est un alcényle en C₂C₆ ;
alcoxy est un alcoxy en C₁C₁₀ ;
aryle est un groupe phényle, ou un système cyclique condensé bicyclique ou tricyclique dans lequel un ou plusieurs des cycles condensés est un groupe phényle, où l'aryle peut être facultativement substitué par un, deux, trois, quatre, ou cinq substituants indépendamment choisis dans le groupe constitué d'alcoxy, alcoxyalkyle, alcoxycarbonyle, alcoxycarbonylalcényle, alcoxycarbonylalkyle, alcoxysulfonyle, alkyle, alkylcarbonyle, alkylsulfonyle, amino, aminocarbonyle, aminosulfonyle, un deuxième groupe aryle, arylalkyle, arylsulfonyle, carboxy, cyano, formyle, halogéno, halogénoalcoxy, halogénoalcényle, halogénoalkyle, hétérocyclyle, hétérocyclylalkyle, hydroxy, hydroxyalkyle, nitro, (NR^{c}R^{d})alkyle, et oxo, où le deuxième groupe aryle, la partie aryle de l'arylalkyle et de l'arylsulfonyle, l'hétérocyclyle, et la partie hétérocyclyle de l'hétérocyclylalkyle peuvent être facultativement substitués par un, deux, trois, quatre, ou cinq substituants indépendamment choisis dans le groupe constitué d'alcoxy, alcoxyalkyle, alcoxycarbonyle, alkyle, alkylcarbonyle, carboxy, cyano, halogéno, halogénoalcoxy, halogénoalkyle, hydroxy, nitro, et oxo ;
hétérocyclyle est choisi parmi un cycle de cinq, six, ou sept chaînons contenant un, deux, ou trois hétéroatomes indépendamment choisis dans le groupe constitué de l'azote, l'oxygène et le soufre, le cycle de cinq chaînons ayant zéro à deux doubles liaisons et les cycles de six et sept chaînons ayant zéro à trois doubles liaisons ; des groupes bicycliques dans lesquels le cycle hétérocyclyle est condensé avec un groupe phényle, un groupe cycloalcényle monocyclique, un groupe cycloalkyle monocyclique, ou un autre groupe hétérocyclyle monocyclique ; et des groupes tricycliques dans lesquels un système bicyclique est condensé avec un groupe phényle, un groupe cycloalcényle monocyclique, un groupe cycloalkyle monocyclique, ou un autre groupe hétérocyclyle monocyclique ; où un hétérocyclyle peut être facultativement substitué par un, deux, trois, quatre, ou cinq substituants indépendamment choisis dans le groupe constitué d'alcoxy, alcoxyalkyle, alcoxycarbonyle, alkyle, alkylcarbonyle, alkylsulfonyle, amino, aminocarbonyl, aminosulfonyle, aryle, arylalkyle, arylsulfonyle, carboxy, cyano, halogéno, un deuxième groupe hétérocyclyle, hétérocyclylalkyle, hydroxy, hydroxyalkyle, nitro, -NR^{c}R^{d}, (NR^{c}R^{d})alkyle, et oxo, où l'aryle, la partie aryle de l'arylalkyle et l'arylsulfonyle, le deuxième groupe hétérocyclyle, et la partie hétérocyclyle de l'hétérocyclylalkyle peuvent être facultativement substitués par un, deux, trois, quatre, ou cinq substituants indépendamment choisis dans le groupe constitué d'alcoxy, alcoxyalkyle, alcoxycarbonyle, alkyle, alkylcarbonyle, carboxy, cyano, halogéno, halogénoalcoxy, halogénoalkyle, hydroxy, méthylènedioxy, nitro, et oxo ;
cycloalkyle est un système cyclique hydrocarboné monocyclique, bicyclique, ou tricyclique saturé ayant trois à douze atomes de carbone, un cycloalkyle pouvant être facultativement substitué par un, deux, trois, ou quatre substituants indépendamment choisis dans le groupe constitué d'alcoxy, alcoxyalkyle, alcoxycarbonyle, alkyle, alkylcarbonyle, alkylsulfonyle, -NR^{c}R^{d}, (NR^{c}R^{d})alkyle, (NR^{c}R^{d})carbonyle, un deuxième groupe aryle, arylalkyle, arylsulfonyle, carboxy, cyano, halogéno, hétérocyclyle, hétérocyclylalkyle, hydroxy, hydroxyalkyle, nitro, et oxo, où le deuxième groupe aryle, la partie aryle de l'arylalkyle et de l'arylsulfonyle, l'hétérocyclyle, et la partie hétérocyclyle de l'hétérocyclylalkyle peuvent être facultativement substitués par un, deux, trois, quatre, ou cinq substituants indépendamment choisis dans le groupe constitué d'alcoxy, alcoxyalkyle, alcoxycarbonyle, alkyle, alkylcarbonyle, carboxy, cyano, halogéno, halogénoalcoxy, halogénoalkyle, hydroxy, nitro, et oxo ;
cycloalcényle est un système cyclique ou bicyclique non aromatique ayant trois à dix atomes de carbone et un à trois cycles, dans lequel chaque cycle de cinq chaînons a une double liaison, chaque cycle de six chaînons a une ou deux doubles liaisons, chaque cycle de sept et huit chaînons a une à trois doubles liaisons, et chaque cycle de neuf à dix chaînons a une à quatre doubles liaisons ;
amino désigne -NR^{a}R^{b}, où R^{a} et R^{b} sont indépendamment choisis dans le groupe constitué d'hydrogène, alcoxyalkylcarbonyle, alcoxycarbonyle, alkyle, alkylcarbonyle, alkylsulfonyle, aryle, arylalkyle, arylalkylcarbonyle, arylalkylsulfonyle, arylsulfonyle, arylsulfonylalkyle, arylsulfonylalkylcarbonyle, cycloalkyle, cycloalkylalkyle, cycloalkylcarbonyle, halogénoalkylsulfonyle, hétérocyclyle, hétérocyclylalkylcarbonyle, hétérocyclylalkylsulfonyle, hétérocyclylcarbonyle, hétérocyclylsulfonyle, -NR^{c}R^{d}, (NR^{c}R^{d})alkyle, (NR^{c}R^{d})alkylcarbonyle, (NR^{c}R^{d})alkylsulfonyle, (NR^{c}R^{d})carbonyle, (NR^{c}R^{d})carbonylalkyle, et (NF^{c}R^{d})carbonylalkylcarbonyle, où aryle peut être facultativement substitué par un, deux, trois, quatre, ou cinq substituants indépendamment choisis dans le groupe constitué d'alcoxy, alcoxyalkyle, alcoxycarbonyle, alkyle, alkylcarbonyle, alkylsulfonyle, un deuxième groupe aryle, arylalkyle, arylsulfonyle, carboxy, cyano, halogéno, hétérocyclyle, hétérocyclylalkyle, hydroxy, nitro, et oxo, où le deuxième groupe aryle, la partie aryle de l'arylalkyle et de l'arylsulfonyle, l'hétérocyclyle, et la partie hétérocyclyle de l'hétérocyclylalkyle peuvent être facultativement substitués par un, deux, trois, quatre, ou cinq substituants indépendamment choisis dans le groupe constitué d'alcoxy, alcoxyalkyle, alcoxycarbonyle, alkyle, alkylcarbonyle, carboxy, cyano, halogéno, halogénoalcoxy, halogénoalkyle, hydroxy, nitro, et oxo ; et
R^{c} et R^{d} sont chacun indépendamment hydrogène, alcoxycarbonyle, alkyle, alkylcarbonyle, aryle, arylsulfonyle, hétérocyclcarbonyle, ou formyle ;
à condition que la 3-chloro-11-oxo-10,11-dihydro-5H-dibenzo[b,e][1,4]diazépine et la 3-fluoro-11-oxo-10,11-dihydro-5H-dibenzo[b,e][1,4]diazépine soient exclues.

2. Composé de la revendication 1, partie (a) dans lequel R² est hydrogène.

3. Composé de la revendication 1, partie (a) dans lequel R² est choisi dans le groupe constitué d'alcoxycarbonyle et alcoxycarbonylalkyle.

4. Composé de la revendication 1, partie (a) dans lequel R² est choisi dans le groupe constitué d'amino et aryle.

5. Composé de la revendication 1, partie (a) dans lequel R² est choisi dans le groupe constitué de carboxy, carboxyalkyle, cyano, nitro, et hétérocyclyle.

6. Composé de la revendication 1, partie (a) dans lequel R² est (CR⁹R¹⁰)ₘC(O)NR¹¹R¹².

7. Composé de la revendication 6 dans lequel m est 0.

8. Composé de la revendication 7 dans lequel l'un de R¹¹ et R¹² est hydrogène et l'autre est hétérocyclylalkyle.

9. Composé de la revendication 7 dans lequel l'un de R¹¹ et R¹² est hydrogène et l'autre est choisi dans le groupe constitué d'amino, aminoalkyle, arylalkyle, et hydroxyalkyle.

10. Composé de la revendication 6 dans lequel R¹¹ et R¹², conjointement avec l'atome d'azote auquel ils sont liés, forment un cycle hétérocyclyle choisi dans le groupe constitué de diazépanyle, imidazolidinyle, morpholinyle, pipérazinyle, pipéridinyle, et pyrrolidinyle, qui peuvent chacun être facultativement substitués par un, deux ou trois substituants indépendamment choisis dans le groupe constitué d'alcoxy, alcoxycarbonyle, alcényle, alkyle, alkylcarbonyle, aryle, carboxy, carboxy, hétérocyclyle, hétérocyclylalkyle, hydroxy, et hydroxyalkyle.

11. Composé de la revendication 6 dans lequel m est 1.

12. Composé de la revendication 11 dans lequel l'un de R¹¹ et R¹² est choisi dans le groupe constitué d'hydrogène et alkyle et l'autre est hétérocyclylalkyle.

13. Composé de la revendication 11 dans lequel l'un de R¹¹ et R¹² est choisi dans le groupe constitué d'hydrogène et alkyle et l'autre est choisi dans le groupe constitué d'alcoxyalkyle et hydroxyalkyle.

14. Composé de la revendication 11 dans lequel l'un de R¹¹ et R¹² est choisi dans le groupe constitué d'hydrogène et alkyle et l'autre est choisi dans le groupe constitué d'alkyle, aminoalkyle, aryle, aryalkyle, et hétérocyclyle.

15. Composé de la revendication 11 dans lequel R¹¹ et R¹², conjointement avec l'atome d'azote auquel ils sont liés, forment un cycle hétérocyclyle choisi, dans le groupe constitué de diazépanyle, imidazolidinyle, morpholinyle, pipérazinyte, pipéridinyle, et pyrrolidinyle, qui peuvent chacun être facultativement substitués par un, deux ou trois substituants indépendamment choisis dans le groupe constitué d'alcoxy, alcoxycarbonyle, alcényle, alkyle, alkylcarbonyle, aryle, carboxy, carboxy, hétérocyclyle, hétérocyclylalkyle, hydroxy, et hydroxyalkyle.

16. Composé de la revendication 1 choisi dans le groupe constitué de
3-(4-hydroxy-3-méthoxyphényl)-5,10-dihydro-11H-dibenzo[b,e] [1,4]diazépin-11-one ;
3-(11-oxo-10,11-dihydro-5H-dibenzo[b,e][1,4]diazépin-3-yl)benzonitrile ;
3-(3-méthoxy-4-nitrophényl)-5,10-dihydro-11H-dibenzo[b,e][1,4]diazépin-11-one ;
3-(4-chloro-3-méthoxyphényl)-5,10-dihydro-11H-dibenzo[b,e] [1,4]diazépin-11-one ;
3-(4-bromo-3-méthoxyphényl)-5,10-dihydro-11H-dibenzo[b,e] [1,4]diazépin-11-one ;
3-(4-acétyl-3-méthoxyphényl)-5,10-dihydro-11H-dibenzo[b,e] [1,4]diazépin-11-one ;
2-méthoxy-4-(11-oxo-10,11-dihydro-5H-dibenzo[b,e][1,4]diazépin-3-yl)benzonitrile ;
2-méthoxy-4-(11-oxo-10,11-dihydro-5H-dibenzo[b,e][1,4]diazépin-3-yl)benzoate de méthyle ;
acide 2-méthoxy-4-(11-oxo-10,11-dihydro-5H-dibenzo[b,e][1,4]diazépin-3-yl)benzoïque ;
N-(3,4-dihydroxybenzyl)-2-méthoxy-4-(11-oxo-10,11-dihydro-5H-dibenzo[b,e][1,4]diazépin-3-yl)benzamide ;
2-méthoxy-4-(11-oxo-10,11-dihydro-5H-dibenzo[b,e][1,4]diazépin-3-yl)benzamide ;
3-(2-méthoxy-4-pyridinyl)-5,10-dihydro-11H-dibenzo[b,e][1,4]diazépin-11-one ;
3-(4-hydroxy-3-méthoxyphényl)-11-oxo-10,11-dihydro-5H-dibenzo[b,e] [1,4]diazépine-8-carboxylate de méthyle ;
[3-(3-méthoxy-4-nitrophényl)-11-oxo-10,11-dihydro-5H-dibenzo[b,e] [1,4]diazépin-8-yl]acétate de méthyle ;
[3-(4-hydroxy-3-méthoxyphényl)-11-oxo-10,11-dihydro-5H-dibenzo[b,e] [1,4]diazépin-8-yl]acétate de méthyle ;
[3-(3-méthoxyphényl)-11-oxo-10,11-dihydro-5H-dibenzo[b,e] [1,4]diazépin-8-yl]acétate de méthyle ;
[3-(4-chloro-3-méthoxyphényl)-11-oxo-10,11-dihydro-5H-dibenzo[b,e] [1,4]diazépin-8-yl]acétate de méthyle ;
[3-(4-bromo-3-méthoxyphényl)-11-oxo-10,11-dihydro-5H-dibenzo[b,e] [1,4]diazépin-8-yl]acétate de méthyle ;
[3-(4-cyano-3-méthoxyphényl)-11-oxo-10,11-dihydro-5H-dibenzo[b,e] [1,4]diazépin-8-yl]acétate de méthyle ;
[3-(5-méthoxy-2-méthyl-4-nitrophényl)-11-oxo-10,11-dihydro-5H-dibenzo[b,e][1,4]diazépin-8-yl]acétate de méthyle ;
[3-(4-cyano-5-méthoxy-2-méthylphényl)-11-oxo-10,11-dihydro-5H-dibenzo[b,e][1,4]diazépin-8-yl]acétate de méthyle ;
[3-(2-fluoro-5-méthyl-4-pyridinyl)-11-oxo-10,11-dibydro-5H-dibenzo[b,e] [1,4]diazépin-8-yl]acétate de méthyle ;
(3-(2-méthoxy-5-méthyl-4-pyridinyl)-11-oxo-10,11-dihydro-5H-dibenzo[b,e][1,4]diazépin-8-yl]acétate de méthyle ;
8-amino-3-(4-hydroxy-3-méthoxyphényl)-5,10-dihydro-11H-dibenzo[b,e] [1,4]diazépin-11-one ;
N-[3-(4-hydroxy-3-méthoxyphényl)-11-oxo-10,11-dihydro-5H-dibenzo[b,e] [1,4]diazépin-8-yl]méthanesulfonamide ;
N-[3-(3-méthoxy-4-nitrophényl)-11-oxo-10,11-dihydro-5H-dibenzo[b,e] [1,4]diazépin-8-yl]méthanesulfonamide ;
N-[3-(4-hydroxy-3-méthoxyphényl)-11-oxo-10,11-dihydro-5H-dibenzo[b,e] [1,4]diazépin-8-yl]acétamide ;
N-[3-(3-méthoxy-4-nitxophényl)-11-oxo-10,11-dihydro-5H-dibenzo[b,e] [1,4]diazépin-8-yl]acétamide ;
8-(3-aminophényl)-3-(4-hydroxy-3-méthoxyphényl)-5,10-dihydro-11H-dibenzo[b,e][1,4]diazépin-11-one ;
3-(4-hydroxy-3-méthoxyphényl)-8-(3-hydroxyphényl)-5,10-dihydro-11H-dibenzo[b,e][1,4]diazépin-11-one ; et
8-amino-3-(3-méthoxy-4-nitrophényl)-5,10-dihydro-11H-dibenzo[b,e] [1,4]diazépin-11-one ;
acide 3-(4-hydroxy-3-méthoxyphényl)-11-oxo-10,11-dihydro-5H-dibenzo[b,e][1,4]diazépine-8-carboxylique ;
3-(4-hydroxy-3-méthoxyphényl)-11-oxo-10,11-dihydro-5H-dibenzo[b,e] [1,4]diazépine-8-carbonitrile ;
3-(4-hydroxy-3-méthoxyphényl)-8-nitro-5,10-dihydro-11H-dibenzo[b,e] [1,4]diazépin-11-one ;
3-(4-hydroxy-3-méthoxyphényl)-8-(3-pyridinyl)-5,10-dihydro-11H-dibenzo[b,e][1,4]diazépin-11-one ;
3-(4-hydroxy-3-méthoxyphényl)-8-(1H-pyrrol-2-yl)-5,10-dihydro-11H-dibenzo[b,e][1,4]diazépin-11-one ;
3-(3-méthoxy-4-nitrophényl)-8-(1H-pyrrol-2-yl)-5,10-dihydro-11H-dibenzo[b,e][1,4]diazépin-11-one ;
acide [3-(3-méthoxy-4-nitrophényl)-11-oxo-10,11-dihydro-5H-dibenzo[b,e] [1,4]diazépin-8-yl]acétique ;
3-(4-hydroxy-3-méthoxyphényl)-11-oxo-N[3-(1-pyrrolidinyl)propyl]-10,11-dihydro-5H-dibenzo[b,e][1,4]diazépine-8-carboxamide ;
3-(4-hydroxy-3-méthoxyphényl)N[3-(4-morpholinyl)propyl]-11-oxo-10,11-dihydro-5H-dibenzo[b,e][1,4]diazépine-8-carboxamide ;
3-(4-hydroxy-3-méthoxyphényl)-11-oxoN[3-(2-oxo-1-pyirolidinyl)propyl]-10,11-dihydro-5H-dibenzo[b,e][1,4]diazépine-8-carboxamide ;
3-(4-hydroxy-3-méthoxyphényl)-11-oxoN(3-pyridinylméthyl)-10,11-dihydro-5H-dibenzo[b,e][1,4]diazépine-8-carboxamide ;
3-(4-hydroxy-3-méthoxyphényl)-11-oxoN(2-pyridinylméthyl)-10,11-dihydro-5H-dibenzo[b,e][1,4]diazépine-8-carboxamide ;
3-(4-hydroxy-3-méthoxyphényl)-11-oxoN(4-pyridinylméthyl)-10,11-dihydro-5H-dibenzo[b,e][1,4]diazépine-8-carboxamide ;
3-(2-méthoxy-4-pyridinyl)-11-oxoN[3-(1-pyrrolidinyl)propyl]-10,11-dihydro-5H-dibenzo[b,e][1,4]diazépine-8-carboxamide ;
11-oxo-3-(2-oxo-1,2-dihydro-4-pyridinyl)N[3-(1-pyrrolidinyl)propyl]-10,11-dihydro-5H-dibenzo[b,e][1,4]diazépine-8-carboxamide ;
N-[3-(diméthylamino)propyl]-3-(4-hydroxy-3-méthoxyphényl)-11-oxo-10,11-dihydro-5H-dibenzo[b,e][1,4]diazépine-8-carboxamide ;
N-(2-hydroxyéthyl)-3-(4-hydroxy-3-méthoxyphényl)-11-oxo-10,11-dihydro-5H-dibenzo[b,e][1,4]diazépine-8-carboxamide ;
N-(2,3-dihydroxypropyl)-3-(4-hydroxy-3-méthoxyphényl)-11-oxo-10,11-dihydro-5H-dibenzo[b,e][1,4]diazépine-8-carboxamide ;
N-[2-(acétylamino)éthyl]-3-(4-hydroxy-3-méthoxyphényl)-11-oxo-10,11-dihydro-5H-dibenzo[b,e][1,4]diazépine-8-carboxamide ;
3-(4-hydroxy-3-méthoxyphényl)N[4-(méthylsulfonyl)benzyl]-11-oxo-10,11-dihydro-5H-dibenzo[b,e][1,4]diazépine-8-carboxamide ;
N-(2-fluorobenzyl)-3-(4-hydroxy-3-méthoxyphényl)-11-oxo-10,11-dihydro-5H-dibenzo[b,e][1,4]diazépine-8-carboxamide ;
3-(4-hydroxy-3-méthoxyphényl)N(2-méthoxybenzyl)-11-oxo-10,11-dihydro-5H-dibenzo[b,e][1,4]diazépine-8-carboxamide ;
3-(4-hydroxy-3-méthoxyphényl)N[2-(4-méthoxyphényl)éthyl]-11-oxo-10,11-dihydro-5H-dibenzo[b,e][1,4]diazépine-8-carboxamide ;
2-{[3-(4-hydroxy-3-méthoxyphényl)-11-oxo-10,11-dihydro-5H-dibenzo[b,e][1,4]diazépin-8-yl]carbonyl}hydrazinecarboxylate de tert-butyle ;
3-(4-hydroxy-3-méthoxyphényl)-8-[(3-hydroxy-1-pyrrolidinyl)carbonyl]-5,10-dihydro-11H-dibenzo[b,e][1,4]diazépin-11-one ;
3-(4-hydroxy-3-méthoxyphényl)-8-([(2S)-2-(hydroxyméthyl)-1-pyrrolidinyl)carbonyle]-5,10-dihydro-11H-dibenzo[b,e][1,4]diazépin-11-one
3-(4-hydroxy-3-méthoxyphényl)-8-{[2-(hydroxyméthyl)-1-pipéridinyl]carbonyl}-5,10-dihydro-11H-dibenzo[b,e][1,4]diazépin-11-one ;
1-{[3-(4-hydroxy-3-méthoxyphényl)-11-oxo-10,11-dihydro-5H-dibenzo[b,e][1,4]diazépin-8-yl]carbonyl}-2-pipéridinecarboxylate d'éthyle ;
1-{[3-(4-hydroxy-3-méthoxyphényl)-11-oxo-10,11-dihydro-5H-dibenzo[b,e][1,4]diazépin-8-yl]carbonyl}-3-pipéridinecarboxylate d'éthyle ;
1-{[3-(4-hydroxy-3-méthoxyphényl)-11-oxo-10,11-dihydro-5H-dibenzo[b,e][1,4]diazépin-8-yl]carbonyl}-4-pipéridinecarboxylate d'éthyle ;
3-(4-hydroxy-3-méthoxyphényl)-8-[(3-hydroxy-1-pipéridinyl)carbonyl]-5,10-dihydro-11H-dibenzo[b,e][1,4]diazépin-11-one ;
acide 1-{[3-(4-hydroxy-3-méthoxyphényl)-11-oxo-10,11-dihydro-5H-dibenzo[b,e][1,4]diazépin-8-yl]carbonyl}-2-pipéridinecarboxylique ;
acide 1-{[3-(4-hydroxy-3-méthoxyphényl)-11-oxo-10,11-dihydro-5H-dibenzo[b,e][1,4]diazépin-8-yl]carbonyl}-3-pipéridinecarboxylique ;
acide 1-{[3-(4-hydroxy-3-méthoxyphényl)-11-oxo-10,11-dihydro-5H-dibenzo[b,e][1,4]diazépin-8-yl]carbonyle}-4-pipéridinecarboxylique ;
2-[3-(3-méthoxy-4-nitrophényl)-11-oxo-10,11-dihydro-5H-dibenzo[b,e] [1,4]diazépin-8-yl]N[3-(1-pyrrolidinyl)propyl]acétamide ;
2-[3-(3-méthoxy-4-nitrophényl)-11-oxo-10,11-dihydro-5H-dibenzo[b,e] [1,4]diazépin-8-yl]N[2-(2-pyridinyl)éthyl]acétamide ;
2-[3-(3-méthoxy-4-nitrophényl)-11-oxo-10,11-dihydro-5H-dibenzo[b,e] [1,4]diazépin-8-yl]N[2-(3-pyridinyl)éthyl)acétamide ;
2-[3-(3-méthoxy-4-nitro-phényl)-11-oxo-10,11-dihydro-5H-dibenzo[b,e] [1,4]diazépin-8-yl]N(4-pyridin-2-yl-éthyl)-acétamide ;
N-[3-(1H-imidazol-1-yl)propyl-2-[3-(3-méthoxy-4-nitrophényl)-11-oxo-10,11-dihydxo-5H-dibenzo[b,e][1,4]diazépin-8-yl]acétamide ;
2-[3-(3-méthoxy-4-nitrophényl)-11-oxo-10,11-dihydro-5H-dibenzo[b,e] [1,4]diazépin-8-yl]NméthylN[2-(2-pyridinyl)éthyl]acétamide ;
2-[3-(3-méthoxy-4-nitrophényl)-11-oxo-10,11-dihydro-5H-dibenzo[b,e] [1,4]diazépin-8-yl]N(3-pyridinylméthyl)acétamide ;
2-[3-(3-méthoxy-4-nitrophényl)-11-oxo-10,11-dihydro-5H-dibenzo[b,e] [1,4]diazépin-8-yl]N(4-pyridinylméthyl)acétamide ;
2-[3-(3-méthoxy-4-nitrophényl)-11-oxo-10,11-dihydro-5H-dibenzo[b,e] [1,4]diazépin-8-yl]N(2-pyridinylméthyl)acétamide ;
2-[3-(3-méthoxy-4-nitrophényl)-11-oxo-10,11-dihydro-5H-dibenzo[b,e] [1,4]diazépin-8-yl]N[2-(1-pyrrolidinyl)éthyl]acétamide ;
2-[3-(3-méthoxy-4-nitrophényl)-11-oxo-10,11-dihydro-5H-dibenzo[b,e] [1,4]diazépin-8-yl]N[2-(1-pipéridinyl)éthyl]acétamide ;
2-[3-(3-méthoxy-4-nitrophényl)-11-oxo-10,11-dihydro-5H-dibenzo[b,e] [1,4]diazépin-8-yl]N[3-1-pipéridinyl)propyl] acétamide ;
2-[3-(3-méthoxy-4-nitrophényl)-11-oxo-10,11-dihydro-5H-dibenzo[b,e] [1,4]diazépin-8-yl]N[2-(4-morpholinyl)éthyl]acétamide ;
2-[3-(3-méthoxy-4-nitrophényl)-11-oxo-10,11-dihydro-5H-dibenzo[b,e] [1,4]diazépin-8-yl]N[3-(4-morpholinyl)propyl]acétamide ;
2-[3-(3-méthoxy-4-nitrophényl)-11-oxo-10,11-dihydro-5H-dibenzo[b,e] [1,4]diazépin-8-yl]N[2-(4-méthyl-1-pipérazinyl)éthyl]acétamide ;
N-[(1S)-1-(hydroxyméthyl)-2-méthylpropyl]-2-[3-(3-méthoxy-4-nitrophényi)-11-oxo-10,11-dihydro-5H-dibenzo[b,e][1,4]diazépin-8-yl]acétamide ;
N-[(1R)-1-(hydroxyméthyl)-2-méthylpropyl]-2-[3-(3-méthoxy-4-nitrophényl)-11-oxo-10,11-dihydro-5H-dibenzo[b,e][1,4]diazépin-8-yl]acétamide ;
N-(3-éthoxypropyl)-2-[3-(3-méthoxy-4-nitrophényl)-11-oxo-10,11-dihydro-5H-dibenzo[b,e][1,4]diazépin-8-yl]acétamide ;
N-[(1R)-1-(hydroxyméthyl)-3-méthylbutyl]-2-[3-(3-méthoxy-4-nitrophényl)-11-oxo-10,11-dihydro-5H-dibenzo[b,e][1,4]diazépin-8-yl]acétamide ;
N-[(1S)-1-(hydroxyméthyl)-3-méthylbutyl]-2-[3-(3-méthoxy-4-nitrophényl)-11-oxo-10,11-dihydro-5H-dibenzo[b,e][1,4]diazépin-8-yl]acétamide ;
N-(3-hydroxypropyl)-2-[3-(3-méthoxy-4-nitrophényl)-11-oxo-10,11-dihydro-5H-dibenzo[b,e][1,4]diazépin-8-yl]acétamide ;
N-[(1S)-2-hydroxy-1-phényléthyl]-2-[3-(3-méthoxy-4-nitrophényl)-11-oxo-10,11-dihydro-5H-dibenzo[b,e][1,4]diazépin-8-yl]acétamide ;
N-[(1R)-2-hydroxy-1-phényléthyl]-2-[3-(3-méthoxy-4-nitrophényl)-11-oxo-10,11-dihydro-5H-dibenzo[b,e][1,4]diazépin-8-yl]acétamide ;
N-(4-hydroxybutyl)-2-[3-(3-méthoxy-4-nitrophényl)-11-oxo-10,11-dihydro-5H-dibenzo[b,e][1,4]diazépin-8-yl]acétamide ;
N-(2-hydroxyéthyl)-2-[3-(3-méthoxy-4-nitrophényl)-11-oxo-10,11-dihydro-5H-dibenzo[b,e][1,4]diazépin-8-yl]Npropylacétamide ;
N-[2-(diméthylamino)éthyl]-2-[3-(3-méthoxy-4-nitrophényl)-11-oxo-10,11-dihydro-5H-dibenzo[b,e][1,4]diazépin-8-yl]Nméthylacétamide ;
2-[3-(3-méthoxy-4-nitrophényl)-11-oxo-10,11-dihydro-5H-dibenzo[b,e] [1,4]diazépin-8-yl]-N,N-diméthylacétamide ;
2-[3-(3-méthoxy-4-nitrophényl)-11-oxo-10,11-dihydro-5H-dibenzo[b,e] [1,4]diazépin-8-yl]N3-quinoléinylacétamide ;
2-[3-(3-méthoxy-4-nitrophényl)-11-oxo-10,11-dihydro-5H-dibenzo[b,e] [1,4]diazépin-8-yl]N6-quinoléinylacétamide ;
2-[3-(3-méthoxy-4-nitrophényl)-11-oxo-10,11-dihydro-5H-dibenzo[b,e] [1,4]diazépin-8-yl]N[4-morpholinyl)phényl]acétamide ;
N-[2-(diéthylamino)éthyl]-2-[3-(3-méthoxy-4-nitrophényl)-11-oxo-10,11-dihydro-5H-dibenzo[b,e][1,4]diazépin-8-yl]acétamide ;
N-[3-(diéthylamino)propyl]-2-[3-(3-méthoxy-4-nitrophényl)-11-oxo-10,11-dihydro-5H-dibenzo[b,e][1,4]diazépin-8-yl] acétamide ;
N-(3,4-difluorobenzyl)-2-[3-(3-méthoxy-4-nitrophényl)-11-oxo-10,11-dihydro-5H-dibenzo[b,e][1,4]diazépin-8-yl]acétamide ;
N-(1-benzyl-4-pipéridinyl)-2-[3-(3-méthoxy-4-nitrophényl)-11-oxo-10,11-dihydro-5H-dibenzo[b,e][1,4]diazépin-8-yl]acétamide ;
N-[4-(diméthylamino)butyl]-2-[3-(3-méthoxy-4-nitrophényl)-11-oxo-10,11-dihydro-5H-dibenzo[b,e][1,4]diazépin-8-yl]acétamide ;
N-{2-[4-(aminosulfonyl)phényl]éthyl}-2-[3-(3-méthoxy-4-nitrophényl)-11-oxo-10,11-dihydro-5H-dibenzo[b,e][1,4]diazépin-8-yl]acétamide ;
N-[2-(diméthylamino)éthyl]-2-[3-(3-méthoxy-4-nitrophényl)-11-oxo-10,11-dihydro-5H-dibenzo[b,e][1,4]diazépin-8-yl]acétamide ;
N-[3-(diméthylamino)propyl]-2-[3-(3-méthoxy-4-nitrophényl)-11-oxo-10,11-dihydro-5H-dibenzo[b,e][1,4]diazépin-8-yl]acétamide ;
8-[2-(3-hydroxy-1-pipéridinyl)-2-oxoéthyl]-3-(3-méthoxy-4-nitrophényl)-5,10-dihydro-11H-dibenzo[b,e][1,4]diazépin-11-one ;
3-(3-méthoxy-4-nitrophényl)-8-[2-(4-méthyl-1,4-diazépan-1-yl)-2-oxoéthyl]-5,10-dihydro-11H-dibenzo[b,e][1,4]diazépin-11-one ;
8-[2-(4-hydroxy-1-pipéridinyl)-2-oxoéthyl]-3-(3-méthoxy-4-nitrophényl)-5,10-dihydro-11H-dibenzo[b,e][1,4]diazépin-11-one ;
8-[2-(4-éthyl-1-pipérazinyl)-2-oxoéthyl]-3-(3-méthoxy-4-nitrophényl)-5,10-dihydro-11H-dibenzo[b,e][1,4]diazépin-11-one ;
8-{2-[4-(2-hydroxyéthyl)-1-pipérazinyl]-2-oxoéthyl}-3-(3-méthoxy-4-nitrophényl)-5,10-dihydro-11H-dibenzo[b,e][1,4]diazépin-11-one ;
3-(3-méthoxy-4-nitrophényl)-8-[2-oxo-2-(4-phényl-1-pipérazinyl)éthyl]-5,10-dihydro-11H-dibenzo[b,e][1,4]diazépin-11-one ;
3-(3-méthoxy-4-nitrophényl)-8-{2-oxo-2-[4-(2-pyridinyl)-1-pipérazinyl]éthyl)-5,10-dihydro-1H-dibenzo[b,e][1,4]diazépin-11-one ;
8-{2-[(2S)-2-(hydroxyméthyl)-1-pyrrolidinyl]-2-oxoéthyl]-3-(3-méthoxy-4-nitrophényl)-5,10-dihydro-11H-dibenzo[b,e][1,4]diazépin-11-one ;
N-[3-(3-méthoxy-1-nitrophényl)-11-oxo-10,11-dihydro-5H-dibenzo[b,e] [1,4]diazépin-8-yl]urée ;
2-(diméthylamino)N[3-(3-méthoxy-4-nitrophényl)-11-oxo-10,11-dihydro-5H-dibenzo[b,e][1,4]diazépin-8-yl]acétamide ;
(2S)-2-aminoN[3-(3-méthoxy-4-nitrophényl)-11-oxo-10,11-dihydro-5H-dibenzo[b,e][1,4]diazépin-8-yl]-4-méthylpentanamide ;
4-aminoN[3-(3-méthoxy-4-nitrophényl)-11-oxo-10,11-dihydro-5H-dibenzo[b,e][1,4]diazépin-8-yl]butyramide ;
3-AminoN[3-(3-méthoxy-4-nitrophényl)-11-oxo-10,11-dihydro-5H-dibenzo[b,e][1,4]diazépin-8-yl]propionamide ;
N-[3-(3-méthoxy-4-nitrophényl)-11-oxo-10,11-dihydro-5H-dibenzo[b,e] [1,4]diazépin-8-yl]-2-(3-méthyl-3H-imidazol-4-yl)acétamide ;
2-(3H-imidazol-4-yl)N[3-(3-méthoxy-4-nitrophényl)-11-oxo-10,11-dihydro-5H-dibenzo[b,e][1,4]diazépin-8-yl]acétamide ;
N-[3-(3-méthoxy-4-nitrophényl)-11-oxo-10,11-dihydro-5H-dibenzo[b,e] [1,4]diazépin-8-yl]thiophène-3-carboxamide ;
N-[3-(3-méthoxy-4-nitrophényl)-11-oxo-10,11-dihydro-5H-dibenzo[b,e] [1,4]diazépin-8-yl]-1H-pyrrole-2-carboxamide ;
N-[3-(3-méthoxy-4-nitrophényl)-11-oxo-10,11-dihydro-5H-dibenzo[b,e] [1,4]diazépin-8-yl]-2,5-diméthyl-1H-pyrrole-3-carboxamide ;
N-[3-(3-méthoxy-4-nitrophényl)-11-oxo-10,11-dihydro-5H-dibenzo[b,e] [1,4]diazépin-8-yl]-1,3-thiazole-4-carboxamide ;
N-[3-(3-méthoxy-4-nitrophényl)-11-oxo-10,11-dihydro-5H-dibenzo[b,e] [1,4]diazépin-8-yl]-1H-pyrazole-5-carboxamide ;
N-[3-(3-méthoxy-4-nitrophényl)-11-oxo-10,11-dihydro-5H-dibenzo[b,e] [1,4]diazépin-8-yl]-1H-pyrazole-4-carboxamide ;
N-[3-(3-méthoxy-4-nitrophényl)-11-oxo-10,11-dihydro-5H-dibenzo[b,e] [1,4]diazépin-8-yl]isonicotinamide ;
N-[3-(3-méthoxy-4-nitrophényl)-11-oxo-10,11-dihydro-5H-dibenzo[b,e] [1,4]diazépin-8-yl]-3-pyrrolidin-1-ylpropanamide ;
N-[3-(3-méthoxy-4-nitrophényl)-11-oxo-10,11-dihydro-5H-dibenzo[b,e] [1,4]diazépin-8-yl]-3-pipéridin-1-ylpropanamide ;
N-[3-(3-méthoxy-1-nitrophényl)-11-oxo-10,11-dihydro-5H-dibenzo[b,e] [1,4]diazépin-8-yl]-3-morpholin-4-ylpropanamide ;
(2R)-2-hydroxyN[3-(3-méthoxy-4-nitrophényl)-11-oxo-10,11-dihydro-5H-dibenzo[b,e][1,4]diazépin-8-yl]-4-phénylbutanamide ;
N-[3-(3-méthoxy-4-nitrophényl)-11-oxo-10,11-dihydro-5H-dibenzo[b,e] [1,4]diazépin-8-yl]-3-(phénylsulfonyl)propanamide ;
N-[3-(3-méthoxy-4-nitrophényl)-11-oxo-10,11-dihydro-5H-dibenzo[b,e] [1,4]diazépin-8-yl]-2-{[(4-méthylphényl)sulfonyl]amino}acétamide ;
N-[3-(3-méthoxy-4-nitrophényl)-11-oxo-10,11-dihydro-5H-dibenzo[b,e] [1,4]diazépin-8-yl]pyridine-2-carboxamide ;
N-[3-(3-méthoxy-4-nitrophényl)-11-oxo-10,11-dihydro-5H-dibenzo[b,e] [1,4]diazépin-8-yl]nicotinamide ;
N-[3-(3-méthoxy-4-nitrophényl)-11-oxo-10,11-dihydro-5H-dibenzo[b,e] [1,4]diazépin-8-yl]-2-pyridin-3-ylacétamide ;
N-[3-(3-méthoxy-4-nitrophényl)-11-oxo-10,11-dihydro-5H-dibenzo[b,e] [1,4]diazépin-8-yl]-2-(4-méthylpipérazin-1-yl)acétamide ;
3-éthoxyN[3-(3-méthoxy-4-nitrophényl)-11-oxo-10,11-dihydro-5H-dibenzo[b,e][1,4]diazépin-8-yl]propanamide ;
(2R)N[3-(3-méthoxy-4-nitrophényl)-11-oxo-10,11-dihydro-5H-dibenzo[b,e] [1,4]diazépin-8-yl]-5-oxopyrrolidine-2-carboxamide ;
4-méthoxyN[3-(3-méthoxy-4-nitrophényl)-11-oxo-10,11-dihydro-5H-dibenzo[b,e][1,4]diazépin-8-yl]cyclohexanecarboxamide ;
(2R)-2-méthoxyN[3-(3-méthoxy-4-nitrophényl)-11-oxo-10,11-dihydro-5H-dibenzo[b,e][1,4]diazépin-8-yl]-2-phénylacétamide ;
(2S)-2-méthoxyN[3-(3-méthoxy-4-nitrophényl)-11-oxo-10,11-dihydro-5H-dibenzo[b,e][1,4]diazépin-8-yl]-2-phénylacétamide ;
N-(2-{[3-(3-méthoxy-4-nitrophényl)-11-oxo-10,11-dihydro-5H-dibenzo[b,e][1,4]diazépin-8-yl]amino)-2-oxoéthyl)-2-furamide ;
1-acétylN[3-(3-méthoxy-4-nitrophényl)-11-oxo-10,11-dihydro-5H-dibenzo[b,e][1,4]diazépin-8-yl]pipéridine-4-carboxamide ;
N-[3-(3-méthoxy-4-nitrophényl)-11-oxo-10,11-dihydro-5H-dibenzo[b,e] [1,4]diazépin-8-yl]-N'-phénylpentanediamide ;
N-[3-(3-méthoxy-4-nitrophényl)-11-oxo-10,11-dihydro-5H-dibenzo[b,e] [1,4]diazépin-8-yl]-2-[4-(méthylsulfonyl)phényl]acétamide ;
(2S)N[3-(3-méthoxy-4-nitrophényl)-11-oxo-10,11-dihydro-5H-dibenzo[b,e] [1,4]diazépin-8-yl]-5-oxopyrrolidine-2-carboxamide ;
4-(8-amino-11-oxo-10,11-dihydro-5H-dibenzo[b,e][1,4]diazépin-3-yl)-2-méthoxybenzonitrile ;
(2S)N[3-(4-cyano-3-méthoxyphényl)-11-oxo-10,11-dihydro-5H-dibenzo[b,e][1,4]diazépin-8-yl]-2-méthoxy-2-phénylacétamide ;
N-[3-(4-cyano-3-méthoxyphényl)-11-oxo-10,11-dihydro-5H-dibenzo[b,e] [1,4]diazépin-8-yl]-3-pipéridin-1-yl-propanamide ;
N-[3-(4-cyano-3-méthoxyphényl)-11-oxo-10,11-dihydro-5H-dibenzo[b,e] [1,4]diazépin-8-yl]méthanesulfonamide ;
8-amino-3-(4-chloro-3-méthoxyphényl)-5,10-dihydro-11H-dibenzo[b,e] [1,4]diazépin-11-one ;
N-[3-(4-chloro-3-méthoxyphényl)-11-oxo-10,11-dihydro-5H-dibenzo[b,e] [1,4]diazépin-8-yl]-3-pipéridin-1-ylpropanamide ;
3-(3-méthoxy-4-nitrophényl)-8-(2-oxopyrrolidin-1-yl)-5,10-dihydro-11H-dibenzo[b,e][1,4]diazépin-11-one ;
3-(3-méthoxy-4-nitrophényl)-8-(2-oxopipéridin-1-yl)-5,10-dihydro-11H-dibenzo[b,e][1,4]diazépin-11-one ;
3-(4-chloro-3-méthoxyphényl)-8-(2-oxopyrrolidin-1-yl)-5,10-dihydro-11H-dibenzo[b,e][1,4]diazépin-11-one ;
3-chloroN[3-(4-chloro-3-méthoxyphényl)-11-oxo-10,11-dihydro-5H-dibenzo[b,e][1,4]diazépin-8-yl]propane-1-sulfonamide ;
N-[3-(4-chloro-3-méthoxyphényl)-11-oxo-10,11-dihydro-5H-dibenzo[b,e] [1,4]diazépin-8-yl]-1-phénylméthanesulfonamide ;
3-(4-chloro-3-méthoxyphényl)-8-(1,1-dioxidoisothiazolidin-2-yl)-5,10-dihydro-11H-dibenzo[b,e][1,4]diazépin-11-one ;
2,2,2-trifluoroN[3-(3-méthoxy-4-nitrophényl)-11-oxo-10,11-dihydro-5H-dibenzo[b,e][1,4]diazépin-8-yl]éthanesulfonamide ;
N-[3-(3-méthoxy-4-nitrophényl)-11-oxo-10,11-dihydro-5H-dibenzo[b,e] [1,4]diazépin-8-yl]-1-méthyl-1H-imidazole-4-sulfonamide ;
N-[3-(3-méthoxy-4-nitrophényl)-11-oxo-10,11-dihydro-5H-dibenzo[b,e] [1,4]diazépin-8-yl]-1-phénylméthanesulfonamide ;
3-chloroN[3-(3-méthoxy-4-nitrophényl)-11-oxo-10,11-dihydro-5H-dibenzo[b,e][1,4]diazépin-8-yl]propane-1-sulfonamide ;
8-(1,1-dioxidoisothiazolidin-2-yl)-3-(3-méthoxy-4-nitrophényl)-5,10-dihydro-11H-dibenzo[b,e][1,4]diazépin-11-one
N-[3-(3-méthoxy-4-nitrophényl)-11-oxo-10,11-dihydro-5H-dibenzo[b,e] [1,4]diazépin-8-yl]-3-morpholin-4-ylpropane-1-sulfonamide ;
N-[3-(3-méthoxy-4-nitrophényl)-11-oxo-10,11-dihydro-5H-dibenzo[b,e] [1,4]diazépin-8-yl]-3-pipéridin-1-ylpropane-1-sulfonamide ;
3-(diéthylamino)N[3-(3-méthoxy-4-nitrophényl)-11-oxo-10,11-dihydro-5H-dibenzo[b,e][1,4]diazépin-8-yl]propane-1-sulfonamide ;
3-(diméthylamino)N[3-(3-méthoxy-4-nitrophényl)-11-oxo-10,11-dihydro-5H-dibenzo[b,e][1,4]diazépin-8-yl]propane-1-sulfonamide ;
1-chloroN[3-(3-méthoxy-4-nitrophényl)-11-oxo-10,11-dihydro-5H-dibenzo[b,e][1,4]diazépin-8-yl]méthanesulfonamide ;
N-[3-(3-méthoxy-4-nitrophényl)-11-oxo-10,11-dihydro-5H-dibenzo[b,e] [1,4]diazépin-8-yl]-4-morpholin-4-ylbenzamide ;
7-amino-3-(3-méthoxy-4-nitrophényl)-5,10-dihydro-11H-dibenzo[b,e] [1,4]diazépin-11-one ;
3-chloroN[3-(3-méthoxy-4-nitrophényl)-11-oxo-10,11-dihydro-5H-dibenzo[b,e][1,4]diazépin-7-yl]propane-1-sulfonamide ;
N-[3-(3-méthoxy-4-nitrophényl)-11-oxo-10,11-dihydro-5H-dibenzo[b,e] [1,4]diazépin-7-yl]-1-phénylméthanesulfonamide ;
1-(4-chlorophényl)N[3-(3-méthoxy-4-nitrophényl)-11-oxo-10,11-dihydro-5H-dibenzo[b,e][1,4]diazépin-7-yl]méthanesulfonamide ;
N-[3-(3-méthoxy-4-hitrophényl)-11-oxo-10,11-dihydro-5H-dibenzo[b,e] [1,4]diazépin-7-yl]-1-méthyl-1H-imidazole-4-sulfonamide ;
N-[3-(3-méthoxy-4-nitrophényl)-11-oxo-10,11-dihydro-5H-dibenzo[b,e] [1,4]diazépin-7-yl]méthanesulfonamide ;
N-[3-(3-méthoxy-4-nitrophényl)-11-oxo-10,11-dihydro-5H-dibenzo[b,e] [1,4]diazépin-7-yl]-3-morpholin-4-ylpropane-1-sulfonamide ;
N-[3-(3-méthoxy-4-nitrophényl)-11-oxo-10,11-dihydro-5H-dibenzo[b,e] [1,4]diazépin-7-yl]-3-pipéridin-1-ylpropane-1-sulfonamide ;
3-(diéthylamino)N[3-(3-méthoxy-4-nitrophényl)-11-oxo-10,11-dihydro-5H-dibenzo[b,e][1,4]diazépin-7-yl]propane-1-sulfonamide ;
3-(diméthylamino)N[3-(3-méthoxy-4-nitrophényl)-11-oxo-10,11-dihydro-5H-dibenzo[b,e][1,4]diazépin-7-yl]propane-1-sulfonamide ;
N-[3-(3-méthoxy-4-nitrophényl)-11-oxo-10,11-dihydro-5H-dibenzo[b,e] [1,4]diazépin-7-yl]tétrahydro-2H-pyrane-4-carboxamide ;
8-(1-hydroxy-1-méthyléthyl)-3-(3-méthoxy-4-nitrophényl)-5,10-dihydro-11H-dibenzo[b,e][1,4]diazépin-11-one ;
8-(1-éthyl-1-hydroxypropyl)-3-(3-méthoxy-4-nitrophényl)-5,10-dihydro-11H-dibenzo[b,e][1,4]diazépin-11-one ;
8-(1-hydroxy-1-méthyléthyl)-3-(pyridin-4-ylamino)-5,10-dihydro-11H-dibenzo[b,e][1,4]diazépin-11-one ;
3-[(2-chloropyridin-4-yl)amino]-8-(1-hydroxy-1-méthyléthyl)-5,10-dihydro-11H-dibenzo[b,e][1,4]diazépin-11-one ;
4-{[8-(1-hydroxy-1-méthyléthyl)-11-oxo-10,11-dihydro-5H-dibenzo[b,e] [1,4]diazépin-3-yl]amino}pyridine-2-carbonitrile ;
8-(1-hydroxy-1-méthyléthyl)-3-(pyrimidin-4-ylamino)-5,10-dihydro-11H-dibenzo[b,e][1,4]diazépin-11-one ;
8-(1-hydroxy-1-méthyléthyl)-3-[(2,3,5,6-tétrafluoropyridin-4-yl)amino]-5,10-dihydro-11H-dibenzo[b,e][1,4]diazépin-11-one ;
8-(1-éthyl-1-hydroxypropyl)-3-(pyridin-4-ylamino)-5,10-dihydro-11H-dibenzo[b,e][1,4]diazépin-11-one ;
3-[(2-aminopyrimidin-4-yl)amino]-8-(1-hydroxy-1-méthyléthyl)-5,10-dihydro-11H-dibenzo[b,e][1,4]diazépin-11-one ;
3-[(2-chloropyridin-4-yl)amino]-8-(1-éthyl-1-hydroxypropyl)-5,10-dihydro-11H-dibenzo[b,e][1,4]diazépin-11-one ;
8-(1-hydroxy-1-méthyléthyl)-3-[(2,3,6-trifluoropyridin-4-yl)amino]-5,10-dihydro-11H-dibenzo[b,e][1,4]diazépin-11-one ;
3-({2-[(2-chloropyridin-4-yl)amino]pyridin-4-yl}amino)-8-(1-hydroxy-1-méthyléthyl)-5,10-dihydro-11H-dibenzo[b,e][1,4]diazépin-11-one ;
2-méthyl-2-{11-oxo-3-[(2,3,6-trifluoropyridin-4-yl)amino]-10,11-dihydro-5H-dibenzo[b,e][1,4]diazépin-8-yl]propanoate de méthyle ;
2-méthylN(4-morpholin-4-ylphényl)-2-{11-oxo-3-[(2,3,6-trifluoxopyridin-4-yl)amino]-10,11-dihydro-5H-dibenzo[b,e][1,4]diazépin-8-yl}propanamide ;
2-{3-[(2,6-difluoropyridin-4-yl)amino]-11-oxo-10,11-dihydro-5H-dibenzo[b,e][1,4]diazépin-8-yl}-2-méthylN(4-morpholin-4-ylphényl)propanamide ;
3-[(2,6-difluoropyridin-4-yl)amino]-8-(2-hydroxyéthyl)-5,10-dihydro-11H-dibenzo[b,e][1,4]diazépin-11-one ;
acide 2-{3-[(2,6-difluoropyridin-4-yl)amino]-11-oxo-10,11-dihydro-5H-dibenzo[b,e][1,4]diazépin-8-yl}-2-méthylpropanoïque ;
3-[(2,6-difluoropyridin-4-yl)amino]-7-morpholin-4-yl-5,10-dihydro-11H-dibenzo[b,e][1,4]diazépin-11-one ;
7-morpholin-4-yl-3-[(2,3,6-trifluoropyridin-4-yl)amino]-5,10-dihydro-11H-dibenzo[b,e][1,4]diazépin-11-one ;
3-[(2,6-difluoropyridin-4-yl)amino]-8-(2-hydroxy-2-méthylpropyl)-5,10-dihydro-11H-dibenzo[b,e][1,4]diazépin-11-one ;
[11-oxo-3-(pyridin-4-ylamino)-10,11-dihydro-5H-dibenzo[b,e] [1,4]diazépin-8-yl]acétate de méthyle ;
{3-[(2-chloropyridin-4-yl)amino]-11-oxo-10,11-dihydro-5H-dibenzo[b,e] [1,4]diazépin-8-yl} acétate de méthyle ;
{3-[(2-méthylpyridin-4-yl)amino]-11-oxo-10,11-dihydro-5H-dibenzo[b,e] [1,4]diazépin-8-yl}acétate de méthyle ;
3-[(2-chloropyridin-4-yl)amino]-5,10-dihydro-11H-dibenzo[b,e] [1,4]diazépin-11-one ;
8-acétyl-3-[(2-chloropyridin-4-yl)amino]-5,10-dihydro-11H-dibenzo[b,e] [1,4]diazépin-11-one ;
acide {3-[(2-chloropyridin-4-yl)amino]-11-oxo-10,11-dihydro-5H-dibenzo[b,e][1,4]diazépin-8-yl}acétique ;
3-[(2-chloropyridin-4-yl)amino]-8-isopropényl-5,10-dihydro-11H-dibenzo[b,e][1,4]diazépin-11-one ;
2-{3-[(2-chloropyridin-4-yl)amino]-11-oxo-10,11-dihydro-5H-dibenzo[b,e] [1,4]diazépin-8-yl}N(4-morpholin-4-ylphényl)acétamide ;
3-[(2-chloropyridin-4-yl)amino]-8-(2-hydroxy-2-méthylpropyl)-5,10-dihydro-11H-dibenzo[b,e][1,4]diazépin-11-one ;
2-{3-[(2-chloropyridin-4-yl)amino]-11-oxo-10,11-dihydro-5H-dibenzo[b,e] [1,4]diazépin-8-yl}-2-méthylN(4-morpholin-4-ylphényl)propanamide ;
3-[(2-chloropyridin-4-yl)amino]-8-(2-oxopyrolidin-1-yl)-5,10-dihydro-11H-dibenzo[b,e][1,4]diazépin-11-one ;
{3-[(2-chloropyridin-4-yl)amino]-11-oxo-10,11-dihydro-5H-dibenzo[b,e] [1,4]diazépin-7-yl}acétate de méthyle ;
8-[2-(pyridin-2-yloxy)éthyl]-3-[(2,3,6-trifluoropyridin-4-yl)amino]-5,10-dihydro-11H-dibenzo[b,e][1,4]diazépin-11-one ;
8-(2-hydroxy-2-méthylpropyl)-3-[(2,3,5-trifluorophényl)amino]-5,10-dihydro-11H-dibenzo[b,e][1,4]diazépin-11-one ;
3-[(3,5-difluorophényl)amino]-7-(3-hydroxy-3-méthylbutyl)-8-méthoxy-5,10-dihydro-11H-dibenzo[b,e][1,4]diazépin-11-one ;
7-(3-hydroxy-3-méthylbutyl)-8-méthoxy-3-(3-méthoxy-4-nitrophényl)-5,10-dihydro-11H-dibenzo[b,e][1,4]diazépin-11-one ;
3-[(2,6-difluoropyridin-4-yl)amino]-7-(3-hydroxypropyl)-8-méthoxy-5,10-dihydro-11H-dibenzo[b,e][1,4]diazépin-11-one ;
7-(3-hydroxypropyl)-8-méthoxy-3-(3-méthoxy-4-nitrophényl)-5,10-dihydro-11H-dibenzo[b,e][1,4]diazépin-11-one ;
3-[(2,6-difluoropyridin-4-yl)amino]-8-(3-hydroxy-3-méthylbutyl)-5,10-dihydro-11H-dibenzo[b,e][1,4]diazépin-11-one ;
8-(3-hydroxy-3-méthylbutyl)-3-(3-méthoxy-4-nitrophényl)-5,10-dihydro-11H-dibenzo[b,e][1,4]diazépin-11-one
3-[3-(3-méthoxy-4-nitrophényl)-11-oxo-10,11-dihydro-5H-dibenzo[b,e] [1,4]diazépin-8-yl]propanoate de méthyle ;
acide 3-[3-(3-méthoxy-4-nitrophényl)-11-oxo-10,11-dihydro-5H-dibenzo[b,e][1,4]diazépin-8-yl]propanoïque ;
3-[3-(3-méthoxy-4-nitrophényl)-11-oxo-10,11-dihydro-5H-dibenzo[b,e] [1,4]diazépin-8-yl]-N,N-diméthylpropanamide ;
3-[3-(3-méthoxy-4-nitrophényl)-11-oxo-10,11-dihydro-5H-dibenzo[b,e] [1,4]diazépin-8-yl]N(4-morpholin-4-ylphényl)propanamide ;
8-(3-azétidin-1-yl-3-oxopropyl)-3-(3-méthoxy-4-nitrophényl)-5,10-dihydro-11H-dibenzo[b,e][1,4]diazépin-11-one ;
3-(3-méthoxy-4-nitrophényl)-8-(3-oxo-3-pyrrolidin-1-ylpropyl)-5,10-dihydro-11H-dibenzo[b,e][1,4]diazépin-11-one ;
3-(3-méthoxy-4-nitrophényl)-8-(3-morpholin-4-yl-3-oxopropyl)-5,10-dihydro-11H-dibenzo[b,e][1,4]diazépin-11-one ;
N,N-diéthyl]-3-[3-(3-méthoxy-4-nitrophényl)-11-oxo-10,11-dihydro-5H-dibenzo[b,e][1,4]diazépin-8-yl]propanamide ;
8-[3-(4-hydroxypipéridin-1-yl)-3-oxopropyl]-3-(3-méthoxy-4-nitrophényl)-5,10-dihydro-11H-dibenzo[b,e][1,4]diazépin-11-one ;
3-[3-(3-méthoxy-4-nitrophényl)-11-oxo-10,11-dihydro-5H-dibenzo[b,e] [1,4]diazépin-8-yl]N1,3-thiazol-2-ylpropanamide ;
8-(2-hydroxyéthyl)-3-(3-méthoxy-4-nitrophényl)-5,10-dihydro-11H-dibenzo[b,e][1,4]diazépin-11-one ;
8-(3-hydroxypropyl)-3-(3-méthoxy-4-nitrophényl)-5,10-dihydro-11H-dibenzo[b,e][1,4]diazépin-11-one ;
3-[3-(4-chloro-3-méthoxyphényl)-11-oxo-10,11-dihydro-5H-dibenzo[b,e] [1,4]diazépin-8-yl]propanoate de méthyle ;
3-[3-(4-chloro-3-méthoxyphényl)-11-oxo-10,11-dihydro-5H-dibenzo[b,e] [1,4]diazépin-8-yl]-N,N-diméthylpropanamide ;
3-(4-chloro-3-méthoxyphényl)-8-(3-hydroxy-3-méthylbutyl)-5,10-dihydro-11H-dibenzo[b,e][1,4]diazépin-11-one ;
3-(3-méthoxy-4-nitrophényl)-8-[2-(3-méthyl-1,2,4-oxadiazol-5-yl)éthyl]-5,10-dihydro-1H-dibenzo[b,e][1,4]diazépin-11-one ;
3-[8-méthoxy-3-(3-méthoxy-4-nitrophényl)-11-oxo-10,11-dihydro-5H-dibenzo[b,e][1,4]diazépin-7-yl]propanoate de méthyle ;
7-(2-hydroxy-2-méthylpropyl)-8-méthoxy-3-(3-méthoxy-4-nitrophényl)-5,10-dihydro-11H-dibenzo[b,e][1,4]diazépin-11-one.;
7-(2-hydroxyéthyl)-8-méthoxy-3-(3-méthoxy-4-nitrophényl)-5,10-dihydro-11H-dibenzo[b,e][1,4]diazépin-11-one ;
8-méthoxy-3-(3-méthoxy-4-nitrophényl)-7-(2-oxopropyl)-5,10-dihydro-11H-dibenzo[b,e][1,4]diazépin-11-one ;
7-(2-hydroxy-1,1-diméthyléthyl)-8-méthoxy-3-(3-méthoxy-4-nitrophényl)-5,10-dihydro-11H-dibenzo[b,e][1,4]diazépin-11-one ;
7-(3-hydroxypropyl)-3-(3-méthoxy-4-nitrophényl)-8-(trifluorométhoxy)-5,10-dihydro-11H-dibenzo[b,e][1,4]diazépin-11-one ;
7-(3-hydroxy-3-méthylbutyl)-3-(3-méthoxy-4-nitrophényl)-8-(trifluorométhoxy)-5,10-dihydro-11H-dibenzo[b,e][1,4]diazépin-11-one ;
7-(3-hydroxy-3-méthylbutyl)-3-(3-méthoxy-4-nitrophényl)-8-méthyl-5,10-dihydro-11H-dibenzo[b,e][1,4]diazépin-11-one ;
3-(3-méthoxy-4-nitrophényl)-8-(2-pyridin-4-yléthyl)-5,10-dihydro-11H-dibenzo[b,e][1,4]diazépin-11-one ;
3-(3-méthoxy-4-nitrophényl)-8-(2-pyridin-2-yléthyl)-5,10-dihydro-11H-dibenzo[b,e][1,4]diazépin-11-one ;
3-(3-méthoxy-4-nitrophényl)-8-[2-(2-oxopyridin-1(2H)-yl)éthyl]-5,10-dihydro-11H-dibenzo[b,e][1,4]diazépin-11-one ;
8-[2-(5-fluoro-2-oxopyridin-1(2H)-yl)éthyl]-3-(3-méthoxy-4-nitrophényl)-5,10-dihydro-11H-dibenzo[b,e][1,4]diazépin-11-one ;
3-(3-méthoxy-4-nitrophényl)-8-[2-(6-oxopyridazin-1(6H)-yl)éthyl]-5,10-dihydro-11H-dibenzo[b,e][1,4]diazépin-11-one ;
3-(3-méthoxy-4-nitrophényl)-8-[2-(pyridin-2-yloxy)éthyl]-5,10-dihydro-11H-dibenzo[b,e][1,4]diazépin-11-one ;
8-{2-[(5-chloropyridin-3-yl)oxy]éthyl}-3-(3-méthoxy-4-nitrophényl)-5,10-dihydro-11H-dibenzo[b,e][1,4]diazépin-11-one ;
8-méthoxy-3-(3-méthoxy-4-nitrophényl)-7-[2-(pyridin-3-yloxy)éthyl]-5,10-dihydro-11H-dibenzo[b,e][1,4]diazépin-11-one ;
(3-isoquinoléin-5-yl-11-oxo-10,11-dihydro-5H-dibenzo[b,e][1,4]diazépin-8-yl)acétate de méthyle ;
[3-(8-nitroisoquinoléin-5-yl)-11-oxo-10,11-dihydro-5H-dibenzo[b,e] [1,4]diazépin-8-yl]acétate de méthyle ;
3-[3-(4-formyl-3-méthoxyphényl)-11-oxo-10,11-dihydro-5H-dibenzo[b,e] [1,4]diazépin-8-yl]propanoate de méthyle ;
3-{3-[4-(hydroxyméthyl)-3-méthoxyphényl]-11-oxo-10,11-dihydro-5H-dibenzo[b,e][1,4]diazépin-8-yl}propanoate de méthyle;
3-[3-(3-méthoxy-4-propionylphényl)-11-oxo-10,11-dihydro-5H-dibenzo[b,e][1,4]diazépin-8-yl]propanoate de méthyle ;
2-[3-(3-méthoxy-4-nitrophényl)-11-oxo-10,11-dihydro-5H-dibenzo[b,e] [1,4]diazépin-8-yl]-2-méthylN(4-morpholin-4-ylphényl)propanamide ;
2-[(3-(4-cyano-3-méthoxyphényl)-11-oxo-10,11-dihydro-5H-dibenzo[b,e] [1,4]diazépin-8-yl]-2-méthylN(4-morpholin-4-ylphényl)propanamide ;
2-[3-(4-chloro-3-méthoxyphényl)-11-oxo-10,11-dihydro-5H-dibenzo[b,e] [1,4]diazépin-8-yl)-2-méthylN(4-morpholin-4-ylphényl)propanamide ;
2-[3-(3-méthoxy-4-nitrophényl)-11-oxo-10,11-dihydro-5H-dibenzo[b,e] [1,4]diazépin-8-yl]-2-méthylNpyridin-2-ylpropanamide ;
2-[3-(3-méthoxy-4-nitrophényl)-11-oxo-10,11-dihydro-5H-dibenzo[b,e] [1,4]diazépin-8-yl]-2-méthylNpyridin-3-ylpropanamide ;
2-[3-(3-méthoxy-4-nitrophényl)-11-oxo-10,11-dihydro-5H-dibenzo[b,e] [1,4]diazépin-8-yl]-2-méthylNpyridin-4-ylpropanamide ;
2-[3-(3-méthoxy-4-pitrophényl)-11-oxo-10,11-dihydro-5H-dibenzo[b,e] [1,4]diazépin-8-yl]-2-méthylN(pyridin-3-ylméthyl)propanamide ;
2-[3-(3-méthoxy-4-nitrophényl)-11-oxo-10,11-dihydro-5H-dibenzo[b,e] [1,4]diazépin-8-yl]-2-méthylN(pyridin-3-ylméthyl)propanamide ;
2-[3-(3-méthoxy-4-nitrophényl)-11-oxo-10,11-dihydro-5H-dibenzo[b,e] [1,4]diazépin-8-yl]-2-méthylN(tétrahydrofuran-3-ylméthyl)propanamide ;
2-[3-(3-méthoxy-4-nitrophényl)-11-oxo-10,11-dihydro-5H-dibénzo[b,e] [1,4]diazépin-8-yl]-2-méthylN1,3-thiazol-2-ylpropanamide ;
2-[3-(3-méthoxy-4-nitrophényl)-11-oxo-10,11-dihydro-5H-dibenzo[b,e] [1,4]diazépin-8-yl]-2-méthylN{[6-(trifluorométhyl)pyridin-3-yl]méthyl]propanamide ;
N-(4-fluorophényl)-2-[3-(3-méthoxy-4-nitrophényl)-11-oxo-10,11-dihydro-5H-dibenzo[b,e][1,4]diazépin-8-yl]-2-méthylpropanamide ;
N-(2,5-difluorobenzyl)-2-[3-(3-méthoxy-4-nitrophényl)-11-oxo-10,11-dihydro-5H-dibenzo[b,e][1,4]diazépin-8-yl]-2-méthylpropanamide ;
2-méthyl-2-[11-oxo-3-(pyrimidin-4-ylamino)-10,11-dihydro-5H-dibenzo[b,e][1,4]diazépin-8-yl]N[3-(trifluorométhyl)benzyl]propanamide ;
2-méthyl-2-[11-oxo-3-(pyrimidin-4-ylamino)-10,11-dihydro-5H-dibenzo[b,e][1,4]diazépin-8-yl]N[3-(trifluorométhoxy)benzyl]propanamide
1-[3-(3-méthoxy-4-nitrophényl)-11-oxo-10,11-dihydro-5H-dibenzo[b,e] [1,4]diazépin-8-yl]N(4-morpholin-4-ylphényl)cyclopropanecarboxamide ;
2-[3-(3-méthoxy-4-nitrophényl)-11-oxo-10,11-dihydro-5H-dibenzo[b,e] [1,4]diazépin-7-yl]-2-méthylN(4-morpholin-4-ylphényl)propanamide ;
2-[3-(3-méthoxy-4-nitrophényl}-11-oxo-10,11-dihydro-5H-dibenzo[b,e] [1,4]diazépin-7-yl]-2-méthylN(pyridin-2-ylméthyl)propanamide ;
2-[3-(3-méthoxy-4-nitrophényl)-11-oxo-10,11-dihydro-5H-dibenzo[b,e] [1,4]diazépin-7-yl]-2-méthylN(thién-2-ylméthyl)propanamide ;
N-(cyclopropylméthyl)-2-[3-(3-méthoxy-4-nitrophényl)-11-oxo-10,11-dihydro-5H-dibenzo[b,e][1,4]diazépin-7-yl]-2-méthylpropanamide ;
7-(3-hydroxypropyl)-3-(3-méthoxy-4-nitrophényl)-5,10-dihydro-11H-dibenzo[b,e][1,4]diazépin-11-one ;
7-(3-hydroxy-3-méthylbutyl)-3-(3-méthoxy-4-nitrophényl)-5,10-dihydro-11H-dibenzo[b,e][1,4]diazépin-11-one ;
8-(2-hydroxy-1,1-diméthyléthyl)-3-(3-méthoxy-4-nitrophényl)-5,10-dihydro-11H-dibenzo[b,e][1,4]diazépin-11-one ;
8-(2-hydroxy-1,1,2-triméthylpropyl)-3-(3-méthoxy-4-nitrophényl)-5,10-dihydro-11H-dibenzo[b,e][1,4]diazépin-11-one ;
8-(1,1-diméthyl-2-oxopropyl)-3-(3-méthoxy-4-nitrophényl)-5,10-dihydro-11H-dibenzo[b,e][1,4]diazépin-11-one ;
7-(2-hydroxy-1,1-diméthyléthyl)-3-(3-méthoxy-4-nitrophényl)-5,10-dihydro-11H-dibenzo[b,e][1,4]diazépin-11-one ;
8-[1-(hydroxyméthyl)cyclopropyl]-3-(3-methoxy-4-nitrophényl)-5,10-dihydro-11H-dibenzo[b,e][1,4]diazépin-11-one ;
3-[(2-chloropyridin-4-yl)amino]-8-(2-hydroxy-1,1-diméthyléthyl)-5,10-dihydro-11H-dibenzo[b,e][1,4]diazépin-11-one ;
3-[(2,6-difluoropyridin-4-yl)amino]-8-(2-hydroxy-1,1-diméthyléthyl)-5,10-dihydro-11H-dibenzo[b,e][1,4]diazépin-11-ohe ;
3-[(2,6-difluoropyridin-4-yl)amino]-8-(2-hydroxy-1,1,2-triméthylpropyl)-5,10-dihydro-11H-dibenzo[b,e][1,4]diazépin-11-one ;
3-(4-chloro-3-méthoxyphényl)-8-(2-hydroxy-1,1-diméthyléthyl)-5,10-dihydro-11H-dibenzo[b,e][1,4]diazépin-11-one ;
3-(3-méthoxy-4-nitrophényl)-8-[2-(4-morpholin-4-ylphénoxy)éthyl]-5,10-dihydro-11H-dibenzo[b,e][1,4]diazépin-11-one ;
3-(4-chloro-3-méthoxyphényl)-8-[2-(4-morpholin-4-ylphénoxy)éthyl]-5,10-dihydro-11H-dibenzo[b,e][1,4]diazépin-11-one ;
2-méthoxy-4-{8-[2-(4-morpholin-4-ylphéhoxy)éthyl]-11-oxo-10,11-dihydro-5H-dibenzo[b,e][1,4]diazépin-3-yl}benzonitrile ;
3-(3-méthoxy-4-nitrophényl)-8-{2-[(4-morpholin-4-ylphényl)amino]éthyl}-5,10-dihydro-11H-dibenzo[b,e][1,4]diazépin-11-one ;
3-(3-méthoxy-4-nitrophényl)-8-[2-(3-méthyl-2-oxopyridin-1(2H)-yl)éthyl]-5,10-dihydro-11H-dibenzo[b,e][1,4]diazépin-11-one ;
3-(3-méthoxy-4-nitrophényl)-8-{2-[(5-méthylpyridin-2-yl)oxy]éthyl}-5,10-dihydro-11H-dibenzo[b,e][1,4]diazépin-11-one ;
3-(3-méthoxy-4-nitrophényl)-8-[2-(4-méthyl-2-oxopyridin-1(2H)-yl)éthyl]-5,10-dihydro-11H-dibenzo[b,e][1,4]diazépin-11-one ;
8-[2-(isoquinoléin-3-yloxy)éthyl]-3-(3-méthoxy-4-nitrophényl)-5,10-dihydro-11H-dibenzo[b,e][1,4]diazépin-11-one ;
8-[2-(5-chloro-2-oxopyridin-1(2H)-yl)éthyl]-3-(3-méthoxy-4-nitrophényl)-5,10-dihydro-11H-dibenzo[b,e][1,4]diazépin-11-one ;
8-[1,1-diméthyl-2-(pyridin-2-yloxy)éthyl]-3-(3-méthoxy-4-nitrophényl)-5,10-dihydro-11H-dibenzo[b,e][1,4]diazépin-11-one ;
8-[1,1-diméthyl-2-(4-morpholin-4-ylphénoxy)éthyl]-3-(3-méthoxy-4-nitrophényl)-5,10-dihydro-11H-dibenzo[b,e][1,4]diazépin-11-one ;
2-[3-(3-méthoxy-4-nitrophényl)-11-oxo-10,11-dihydro-5H-dibenzo[b,e] [1,4]diazépin-8-yl]-2-méthylN(tétrahydrofuran-2-ylméthyl)propanamide ;
8-(2-hydroxy-1,1-diméthyléthyl)-3-(3-méthoxy-4-nitrophényl)-7-méthyl-5,10-dihydro-11H-dibenzo[b,e][1,4]diazépin-11-one ;
2-[3-(3-méthoxy-4-nitrophényl)-2-méthyl-11-oxo-10,11-dihydro-5H-dibenzo[b,e][1,4]diazépin-8-yl]N(4-morpholin-4-ylphényl)acétamide ;
{3-[4-(aminométhyl)-3-méthoxyphényl]-11-oxo-10,11-dihydro-5H-dibenzo[b,e][1,4]diazépin-8-yl}acétate de méthyle ;
2-[3-(2-méthoxy-5-méthylpyridin-4-yl)-11-oxo-10,11-dihydro-5H-dibènzo[b,e][1,4]diazépin-8-yl]-2-méthylN(4-morpholin-4-ylphényl)propanamide ;
8-(2-hydroxy-1,1-diméthyléthyl)-3-[(2-méthylpyridin-4-yl)amino]-5,10-dihydro-11H-dibenzo[b,e][1,4]diazépin-11-one ;
8-(2-hydroxy-1,1,2-triméthylpropyl)-3-(pyrimidin-4-ylamino)-5,10-dihydro-11H-dibenzo[b,e][1,4]diazépin-11-one ;
2-[3-(3-méthoxy-4-nitrophényl)-11-oxo-10,11-dihydro-5H-dibenzo[b,e] [1,4]diazépin-8-yl]NméthylN(3-pyrrolidin-1-ylpropyl)acétamide ;
2-[3-(3-méthoxy-4-nitrophényl)-11-oxo-10,11-dihydro-5H-dibenzo[b,e] [1,4]diazépin-8-yl]-2-méthylN(3-pyrrolidin-1-ylpropyl}propanamide ;
2-[3-(3-méthoxy-4-nitrophényl)-11-oxo-10,11-dihydro-5H-dibenzo[b,e] [1,4]diazépin-8-yl]-2-méthylNpyrimidin-4-ylpropanamide ;
2-[3-(3-méthoxy-4-nitrophényl)-11-oxo-10,11-dihydro-5H-dibenzo[b,e] [1,4]diazépin-8-yl]-2-méthylN(4-méthyl-1,3-thiazol-2-yl)propanamide ;
2-[3-(3-méthoxy-4-nitrophényl)-11-oxo-10,11-dihydro-5H-dibenzo[b,e] [1,4]diazépin-8-yl]-2-méthylN(2,2,2-trifluoroéthyl)propanamide ;
N-(3-fluorophényl)-2-[3-(3-méthoxy-4-nitrophényl)-11-oxo-10,11-dihydro-5H-dibenzo[b,e][1,4]diazépin-8-yl]-2-méthylpropanamide ;
2-[3-(3-méthoxy-4-nitrophényl)-11-oxo-10,11-dihydro-5H-dibenzo[b,e] [1,4]diazépin-8-yl]-2-méthylN[4-(trifluorométhoxy)phényl]propanamide ;
2-[3-(3-méthoxy-4-nitrophényl)-11-oxo-10,11-dihydro-5H-dibenzo[b,e] [1,4]diazépin-8-yl]-2-méthylN[3-(trifluorométhyl)phényl]propanamide ;
2-[3-(3-méthoxy-4-nitrophényl)-11-oxo-10,11-dihydro-5H-dibenzo[b,e] [1,4]diazépin-8-yl]-2-méthylN[3-(trifluorométhoxy)phényl]propanamide ;
N-[3-fluoro-5-(trifluorométhyl)benzyl]-2-[3-(3-méthoxy-4-nitrophényl)-11-oxo-10,11-dihydro-5H-dibenzo[b,e][1,4]diazépin-8-yl]-2-méthylpropanamide ;
N-(2-fluorobenzyl)-2-[3-(3-méthoxy-4-nitrophényl)-11-oxo-10,11-dihydro-5H-dibenzo[b,e][1,4]diazépin-8-yl]-2-méthylpropanamide ;
N-(3-fluorobenzyl)-2-[3(3-méthoxy-4-nitrophényl)-11-oxo-10,11-dihydro-5H-dibenzo[b,e][1,4]diazépin-8-yl]-2-méthylpropanamide ;
N-(4-fluorobenzyl)-2-[3-(3-méthoxy-4-nitrophényl)-11-oxo-10,11-dihydro-5H-dibenzo[b,e][1,4]diazépin-8-yl]-2-méthylpropanamide ;
2-[3-(3-méthoxy-4-nitrophényl)-11-oxo-10,11-dihydro-5H-dibenzo[b,e] [1,4]diazépin-8-yl]-2-méthylN[4-(trifluorométhoxy)benzyl]propanamide ;
2-[3-(3-méthoxy-4-nitrophényl)-11-oxo-10,11-dihydro-5H-dibenzo[b,e] [1,4]diazépin-8-yl]-2-méthylN[3-(trifluorométhyl)benzyl]propanamide ;
2-[3-(3-méthoxy-4-nitrophényl)-11-oxo-10,11-dihydro-5H-dibenzo[b,e] [1,4]diazépin-8-yl]-2-méthylN[4-(trifluorométhyl)benzyl]propanamide ;
2-[3-(3-méthoxy-4-nitrophényl)-11-oxo-10,11-dihydro-5H-dibenzo[b,e] [1,4]diazépin-8-yl]-2-méthylN[3-(trifluorométhoxy)benzyl]propanamide ;
N-[2-(2-fluorophényl)éthyl]-2-[3-(3-méthoxy-4-nitrophényl)-11-oxo-10,11-dihydro-5H-dibenzo[b,e][1,4]diazépin-8-yl]-2-méthylpropanamide ;
N-[2-(3-fluorophényl)éthyl]-2-[3-(3-méthoxy-4-nitrophényl)-11-oxo-10,11-dihydro-5H-dibenzo[b,e][1,4]diazépin-8-yl]-2-méthylpropanamide ;
N-(2,4-difluorobenzyl)-2-[3-(3-méthoxy-4-nitrophényl)-11-oxo-10,11-dihydro-5H-dibenzo[b,e][1,4]diazépin-8-yl]-2-méthylpropanamide ;
N-(2,6-difluorobenzyl)-2-[3-(3-méthoxy-4-nitrophényl)-11-oxo-10,11-dihydro-5H-dibenzo[b,e][1,4]diazépin-8-yl]-2-méthylpropanamide ;
N-(cyclopropylméthyl)-2-[3-(3-méthoxy-4-nitrophényl)-11-oxo-10,11-dihydro-5H-dibenzo[b,e][1,4]diazépin-8-yl]-2-méthylpropanamide ;
N-(3-éthoxypropyl)-2-méthyl-2-[11-oxo-3-(pyrimidin-4-ylamino)-10,11-dihydro-5H-dibenzo[b,e][1,4]diazépin-8-yl]propanamide ;
N-(3,4-difluorobenzyl)-2-méthyl-2-[11-oxo-3-(pyrimidin-4-ylamino)-10,11-dihydro-5H-dibenzo[b,e][1,4]diazépin-8-yl]propanamide ;
8-[1,1-diméthyl-2-oxo-2-(4-phénylpipérazin-1-yl)éthyl]-3-(pyrimidin-4-ylamino)-5,10-dihydro-11H-dibenzo[b,e][1,4]diazépin-11-one ;
N-cyclopentyl-2-méthyl-2-[11-oxo-3-(pyrimidin-4-ylamino)-10,11-dihydro-5H-dibenzo[b,e][1,4]diazépin-8-yl]propanamide ;
8-(1,1-diméthyl-2-morpholin-4-yl-2-oxoéthyl)-3-(pyrimidin-4-ylamino)-5,10-dihydro-11H-dibenzo[b,e][1,4]diazépin-11-one ;
2-méthyl-2-[11-oxo-3-(pyrimidin-4-ylamino)-10,11-dihydro-5H-dibenzo[b,e][1,4]diazépin-8-yl]N(tétrahydrofuran-3-ylméthyl)propanamide
N-(cyclopentylméthyl)-2-méthyl-2-[11-oxo-3-(pyrimidin-4-ylamino)-10,11-dihydro-5H-dibenzo[b,e][1,4]diazépin-8-yl]propanamide ;
N-(cyclopropylméthyl)-2-méthyl-2-[11-oxo-3-(pyrimidin-4-ylamino)-10,11-dihydro-5H-dibenzo[b,e][1,4]diazépin-8-yl]propanamide ;
2-méthyl-2-[11-oxo-3-(pyrimidin-4-ylamino)-10,11-dihydro-5H-dibenzo[b,e][1,4]diazépin-8-yl]N(tétrahydrofuran-2-ylméthyl)propanamide
2-[3-(3-méthoxy-4-nitrophényl)-11-oxo-10,11-dihydro-5H-dibenzo[b,e] [1,4]diazépin-7-yl]-2-méthylN1,3-thiazol-2-ylpropanamide ;
N-(2,5-difluorobenzyl)-2-[3-(3-méthoxy-4-nitrophényl)-11-oxo-10,11-dihydro-5H-dibenzo[b,e][1,4]diazépin-7-yl]-2-méthylpropanamide ;
N-(2-éthoxyéthyl)-2-[3-(3-méthoxy-4-nitrophényl)-11-oxo-10,11-dihydro-5H-dibenzo[b,e][1,4]diazépin-7-yl]-2-méthylpropanamide ;
N-(4-fluorophényl)-2-[3-(3-méthoxy-4-nitrophényl)-11-oxo-10,11-dihydro-5H-dibenzo[b,e][1,4]diazépin-7-yl]-2-méthylpropanamide ;
3-(3-méthoxy-4-nitrophényl)-8-[2-(quinoléin-2-yloxy)éthyl]-5,10-dihydro-11H-dibenzo[b,e][1,4]diazépin-11-one ;
3-(3-méthoxy-4-nitrophényl)-8-[2-(5-méthyl-2-oxopyridin-1(2H)-yl)éthyl]-5,10-dihydro-11H-dibenzo[b,e][1,4]diazépin-11-one ;
2-{2-[3-(3-méthoxy-4-nitrophényl)-11-oxo-10,11-dihydro-5H-dibenzo[b,e] [1,4]diazépin-8-yl]éthoxy}-6-méthylnicotinonitrile ;
3-(3-méthoxy-4-nitrophényl)-8-[2-(1-oxoisoquinoléin-2(1H)-yl)éthyl]-5,10-dihydro-11H-dibenzo[b,e][1,4]diazépin-11-one ;
3-(3-méthoxy-4-nitrophényl)-8-{2-[2-oxo-5-(trifluorométhyl)pyridin-1(2H)-yl]éthyl)-5,10-dihydro-11H-dibenzo[b,e][1,4]diazépin-11-one ;
3-(3-méthoxy-4-nitrophényl)-8-[2-(3-méthoxy-2-oxopyridin-1(2H)-yl)éthyl}-5,10-dihydro-11H-dibenzo[b,e][1,4]diazépin-11-one ;
3-(3-méthoxy-4-nitrophényl)-8-{2-[(4-méthylpyridin-2-yl)oxy]éthyl}-5,10-dihydro-11H-dibenzo[b,e][1,4]diazépin-11-one ;
3-(3-méthoxy-4-nitrophényl)-8-{2-[{3-méthoxypyridin-2-yl)oxy]éthyl}-5,10-dihydro-11H-dibenzo[b,e][1,4]diazépin-11-one ;
3-(3-méthoxy-4-nitrophényl)-8-[2-(3-oxoisoquinoléin-2(3H)-yl)éthyl]-5,10-dihydro-11H-dibenzo[b,e][1,4]diazépin-11-one ;
8-{2-[(6-chloropyridin-2-yl)oxy]éthyl}-3-(3-méthoxy-4-nitrophényl)-5,10-dihydro-11H-dibenzo[b,e][1,4]diazépin-11-one ;
8-{2-[(5-chloropyridin-2-yl)oxy]éthyl}-3-(3-méthoxy-4-nitrophényl)-5,10-dihydro-11H-dibenzo[b,e][1,4]diazépin-11-one ;
acide [[3-(4-chloro-3-méthoxyphényl)-11-oxo-10,11-dihydro-5H-dibenzo[b,e][1,4]diazépin-8-yl]acétique ;
[3-(4-acétyl-3-méthoxyphényl)-11-oxo-10,11-dihydro-5H-dibenzo[b,e] [1,4]diazépin-8-yl]acétate de méthyle ;
acide [3-(4-acétyl-3-méthoxyphényl)-11-oxo-10,11-dihydro-5H-dibenzo[b,e][1,4]diazépin-8-yl]acétique ;
2-[3-(4-chloro-3-méthoxyphényl)-11-oxo-10,11-dihydro-5H-dibenzo[b,e] [1,4]diazépin-8-yl]-N,N-diméthylacétamide ;
1-{[3-(3-méthoxy-4-nitrophényl)-11-oxo-10,11-dihydro-5H-dibenzo[b,e] [1,4]diazépin-8-yl]acétyl}pyrrolidin-3-ylcarbamate de tert-butyle ;
8-[2-(3-aminopyrrolidin-1-yl)-2-oxoéthyl]-3-(3-méthoxy-4-nitrophényl)-5,10-dihydro-11H-dibenzo[b,e][1,4]diazépin-11-one ;
acide [3-(2-méthoxy-5-méthylpyridin-4-yl)-11-oxo-10,11-dihydro-5H-dibenzo[b,e][1,4]diazépin-8-yl]acétique ;
2-[3-(2-méthoxy-5-méthylpyridin-4-yl)-11-oxo-10,11-dihydro-5H-dibenzo[b,e][1,4]diazépin-8-yl]-N,N-diméthylacétamide ;
8-{2-[(2S)-2-(hydroxyméthyl)pyrrolidin-1-yl]-2-oxoéthyl}-3-(2-méthoxy-5-méthylpyridin-4-yl)-5,10-dihydro-11H-dibenzo[b,e][1,4]diazépin-11-one ;
1-{[3-(2-méthoxy-5-méthylpyridin-4-yl)-11-oxo-10,11-dihydro-5H-dibenzo[b,e][1,4]diazépin-8-yl]acétyl}pyrrolidin-3-ylcarbamate de tert-butyle ;
N-{2-[4-(aminosulfonyl)phényl]éthyle}-2-[3-(4-chloro-3-méthoxyphényl)-11-oxo-10,11-dihydro-5H-dibenzo[b,e][1,4]diazépin-8-yl]acétamide ;
1-{[3-(4-chloro-3-méthoxyphényl)-11-oxo-10,11-dihydro-5H-dibenzo[b,e] [1,4]diazépin-8-yl]acétyl}pyrrolidin-3-ylcarbamate de tert-butyle ;
3-(4-chloro-3-méthoxyphényl)-8-[2-(3-hydroxypipéridin-1-yl)-2-oxoéthyl]-5,10-dihydro-11H-dibenzo[b,e][1,4]diazépin-11-one ;
3-(4-chloro-3-méthoxyphényl)-8-{2-[(2S)-2-(hydroxyméthyl)pyrrolidin-1-yl]-2-oxoéthyl]-5,10-dihydro-11H-dibenzo[b,e][1,4]diazépin-11-one ;
2-[3-(4-chloro-3-méthoxyphényl)-11-oxo-10,11-dihydro-5H-dibenzo[b,e] [1,4]diazépin-8-yl]N(4-morpholin-4-ylphényl)acétamide ;
4-{[3-(3-méthoxy-4-nitrophényl)-11-oxo-10,11-dihydro-5H-dibenzo[b,e] [1,4]diazépin-8-yl]acétyle}pipérazine-1-carboxylate de tert-butyle ;
3-(3-méthoxy-4-nitrophényl)-8-(2-oxo-2-pipérazin-1-yléthyl)-5,10-dihydro-11H-dibenzo[b,e][1,4]diazépin-11-one ;
acide [3-(4-cyano-3-méthoxyphényl)-11-oxo-10,11-dihydro-5H-dibenzo[b,e][1,4]diazépin-8-yl]acétique ;
2-[3-(4-cyano-3-méthoxyphényl)-11-oxo-10,11-dihydro-5H-dibenzo[b,e] [1,4]diazépin-8-yl]N(4-morpholin-4-ylphényl)acétamide ;
2-[3-(3-méthoxy-4-nitrophényl)-11-oxo-10,11-dihydro-5H-dibenzo[b,e] [1,4]diazépin-8-yl]Nméthylacétamide ;
3-(3-méthoxy-4-nitrophényl)-8-(2-morpholin-4-yl-2-oxoéthyl)-5,10-dihydro-11H-dibenzo[b,e][1,4]diazépin-11-one ;
3-(2-méthoxy-5-méthylpyridin-4-yl)-8-(2-morpholin-4-yl-2-oxoéthyl)-5,10-dihydro-11H-dibenzo[b,e][1,4]diazépin-11-one ;
acide [3-(2-fluoropyridin-4-yl)-11-oxo-10,11-dihydro-5H-dibenzo[b,e] [1,4]diazépin-8-yl]acétique ;
N,N-diméthyl-2-[11-oxo-3-(2-oxo-1,2-dihydropyridin-4-yl)-10,11-dihydro-5H-dibenzo[b,e][1,4]diazépin-8-yl]acétamide ;
8-(2-morpholin-4-yl-2-oxoéthyl)-3-(2-oxo-1,2-dihydropyridin-4-yl)-5,10-dihydro-11H-dibenzo[b,e][1,4]diazépin-11-one ;
N-(4-morpholin-4-ylphényl)-2-[11-oxo-3-(2-oxo-1,2-dihydropyridin-4-yl)-10,11-dihydro-5H-dibenzo[b,e][1,4]diazépin-8-yl]acétamide ;
[11-oxo-3-(2-oxo-1,2-dihydropyridin-4-yl)-10,11-dihydro-5H-dibenzo[b,e] [1,4]diazépin-8-yl]acétate de méthyle ;
[3-(3-méthoxy-4-nitrophényl)-11-oxo-10,11-dihydro-5H-dibenzo[b,e] [1,4]diazépin-7-yl]acétate de méthyle ;
acide [3-(3-méthoxy-4-nitrophényl)-11-oxo-10,11-dihydro-5H-dibenzo[b,e] [1,4]diazépin-7-yl]acétique ;
3-(3-méthoxy-4-nitrophényl)-7-(2-morpholin-4-yl-2-oxoéthyl)-5,10-dihydro-11H-dibenzo[b,e][1,4]diazépin-11-one ;
2-[3-(3-méthoxy-4-nitrophényl)-11-oxo-10,11-dihydro-5H-dibenzo[b,e] [1,4]diazépin-7-yl]Npyridin-2-ylacétamide ;
2-[3-(3-méthoxy-4-nitrophényl)-11-oxo-10,11-dihydro-5H-dibenzo[b,e] [1,4]diazépin-7-yl]Npyridin-3-ylacétamide ;
2-[3-(3-méthoxy-4-nitrophényl)-11-oxo-10,11-dihydro-5H-dibenzo[b,e] [1,4]diazépin-7-yl]Npyridin-4-ylacétamide ;
7-[2-(4-hydroxypipéridin-1-yl)-2-oxoéthyl]-3-(3-méthoxy-4-nitrophényl)-5,10-dihydro-11H-dibenzo[b,e][1,4]diazépin-11-one ;
7-[2-(3-hydroxypipéridin-1-yl)-2-oxoéthyl]-3-(3-méthoxy-4-nitrophényl)-5,10-dihydro-11H-dibenzo[b,e][1,4]diazépin-11-one ;
2-[3-(3-méthoxy-4-nitrophényl)-11-oxo-10,11-dihydro-5H-dibenzo[b,e] [1,4]diazépin-7-yl]N(pyridin-3-ylméthyl)acétamide ;
2-[3-(3-méthoxy-4-nitrophényl)-11-oxo-10,11-dihydro-5H-dibenzo[b,e] [1,4]diazépin-7-yl]N(pyridin-4-ylméthyl)acétamide ;
2-[3-(3-méthoxy-1-nitrophényl)-11-oxo-10,11-dihydro-5H-dibenzo[b,e] [1,4]diazépin-7-yl]N(pyridin-2-ylméthyl)acétamide ;
7-(2-azétidin-1-yl-2-oxoéthyl)-3-(3-méthoxy-4-nitrophényl)-5,10-dihydro-11H-dibenzo[b,e][1,4]diazépin-11-one ;
1-{[3-(3-méthoxy-4-nitrophényl)-11-oxo-10,11-dihydro-5H-dibenzo[b,e] [1,4]diazépin-7-yl]acétyl]pipéridine-3-carboxamide ;
1-{[3-(3-méthoxy-4-nitrophényl)-11-oxo-10,11-dihydro-5H-dibenzo[b,e] [1,4]diazépin-7-yl]acétyl}pipéridine-4-carboxamide ;
7-{2-[(2R)-2-(hydroxyméthyl)pyrrolidin-1-yl]-2-oxoéthyl}-3-(3-méthoxy-4-nitrophényl)-5,10-dihydro-11H-dibenzo[b,e][1,4]diazépin-11-one ;
2-[3-(3-méthoxy-4-nitrophényl)-11-oxo-10,11-dihydro-5H-dibenzo[b,e] [1,4]diazépin-7-yl]-N,N-diméthylacétamide ;
N,N-bis(2-méthoxyéthyl)-2-[3-(3-méthoxy-4-nitrophényl)-11-oxo-10,11-dihydro-5H-dibenzo[b,e][1,4]diazépin-7-yl]acétamide ;
2-[3-(3-méthoxy-4-nitrophényl)-11-oxo-10,11-dihydro-5H-dibenzo[b,e] [1,4]diazépin-7-yl]N[(2S)-tétrahydrofuran-2-ylméthyl]acétamide ;
2-[3-(3-méthoxy-4-nitrophényl)-11-oxo-10,11-dihydro-5H-dibenzo[b,e] [1,4]diazépin-7-yl]N(2-propoxyéthyl)acétamide ;
7-{2-[(2S)-2-(hydroxyméthyl)pyrrolidin-1-yl]-2-oxoéthyl}-3-(3-méthoxy-4-nitrophényl)-5,10-dihydro-11H-dibenzo[b,e][1,4]diazépin-11-one ;
7-[2-(3-aminopyrrolidin-1-yl)-2-oxoéthyl]-3-(3-méthoxy-4-nitrophényl)-5,10-dihydro-11H-dibenzo[b,e][1,4]diazépin-11-one ;
3-(3-méthoxy-4-nitrophényl)-7-(2-oxo-2-pipérazin-1-yléthyl)-5,10-dihydro-11H-dibenzo[b,e][1,4]diazépin-11-one ;
2-[3-(3-méthoxy-4-nitrophényl)-11-oxo-10,11-dihydro-5H-dibenzo[b,e] [1,4]diazépin-7-yl]N(3-méthoxypropyl)acétamide ;
N-(cyanométhyl)-2-[3-(3-méthoxy-4-nitrophényl)-11-oxo-10,11-dihydro-5H-dibenzo[b,e][1,4]diazépin-7-yl]acétamide ;
N-(cyclopropylméthyl)-2-[3-(3-méthoxy-4-nitrophényl)-11-oxo-10,11-dihydro-5H-dibenzo[b,e][1,4]diazépin-7-yl]acétamide ;
N-(1,3-dioxolan-2-ylméthyl)-2-[3-(3-méthoxy-4-nitrophényl)-11-oxo-10,11-dihydro-5H-dibenzo[b,e][1,4]diazépin-7-yl]Nméthylacétamide ;
3-(3-méthoxy-4-nitrophényl)-7-(2-oxo-2-thiomorpholin-4-yléthyl)-5,10-dihydro-11H-dibenzo[b,e][1,4]diazépin-11-one ;
2-[3-(3-méthoxy-4-nitrophényl)-11-oxo-10,11-dihydro-5H-dibenzo[b,e] [1,4]diazépin-7-yl]N(2-pyridin-3-yléthyl)acétamide ;
2-[3-(3-méthoxy-4-nitrophényl)-11-oxo-10,11-dihydro-5H-dibenzo[b,e] [1,4]diazépin-7-yl]N(2-pyridin-4-yléthyl)acétamide ;
N-[2-(2,3-diméthoxyphényl)éthyl]-2-[3-(3-méthoxy-4-nitrophényl)-11-oxo-10,11-dihydro-5H-dibenzo[b,e][1,4]diazépin-7-yl]acétamide ;
N-[2-(1,3-benzodioxol-5-yl)éthyl]-2-[3-(3-méthoxy-4-nitrophényl)-11-oxo-10,11-dihydro-5H-dibenzo[b,e][1,4]diazépin-7-yl]acétamide ;
2-[3-(3-méthoxy-4-nitrophényl)-11-oxo-10,11-dihydro-5H-dibenzo[b,e] [1,4]diazépin-7-yl]N(thién-2-ylméthyl)acétamide ;
2-[3-(3-méthoxy-4-nitrophényl)-11-oxo-10,11-dihydro-5H-dibenzo[b,e] [1,4]diazépin-7-yl]N(1,3-thiazol-5-ylméthyl)acétamide ;
N-[2-(1H-imidazol-4-yl)éthyl]-2-[3-(3-méthoxy-4-nitrophényl)-11-oxo-10,11-dihydro-5H-dibenzo[b,e][1,4]diazépin-7-yl]acétamide ;
7-[2-(1,4-dioxa-8-azaspiro[4,5]déc-8-yl)-2-oxoéthyl]-3-(3-méthoxy-4-nitrophényl)-5,10-dihydro-11H-dibenzo[b,e][1,4]diazépin=11-one ;
7-{2-[2,6-diméthylmorpholin-4-yl]-2-oxoéthyl}-3-(3-méthoxy-4-nitrophényl)-5,10-dihydro-11H-dibenzo[b,e][1,4]diazépin-11-one ;
7-[2-(4-acétylpipérazin-1-yl)-2-oxoéthyl]-3-(3-méthoxy-4-nitrophényl)-5,10-dihydro-11H-dibenzo[b,e][1,4]diazépin-11-one ;
3-(3-méthoxy-4-nitrophényl)-7-[2-oxo-2-(4-pyridin-2-ylpipérazin-1-yl)éthyl]-5,10-dihydro-11H-dibenzo[b,e][1,4]diazépin-11-one ;
3-({[3-(3-méthoxy-4-nitrophényl)-11-oxo-10,11-dihydro-5H-dibenzo[b,e] [1,4]diazépin-7-yl]acétyl}amino)benzamide ;
2-[3-(3-méthoxy-4-nitrophényl)-11-oxo-10,11-dihydro-5H-dibenzo[b,e] [1,4]diazépin-7-yl]N(4-morpholin-4-ylphényl)acétamide ;
2-[3-(3-méthoxy-4-nitrophényl)-11-oxo-10,11-dihydro-5H-dibenzo[b,e] [1,4]diazépin-7-yl]Nquinoléin-6-ylacétamide ;
N-[(1S)-1-(hydroxyméthyl)-2-méthylpropyl]-2-[3-(3-méthoxy-4-nitrophényl)-11-oxo-)-10,11-dihydro-5H-dibenzo[b,e][1,4]diazépin-7-yl]acétamide ;
(2S)-2-({[3-(3-méthoxy-4-nitrophényl)-11-oxo-10,11-dihydro-5H-dibenzo[b,e][1,4]diazépin-7-yl]acétyl}amino)-4-méthylpentanamide ;
N-[(1S)-2-hydroxy-1-phényléthyl]-2-[3-(3-méthoxy-4-nitrophényl)-11-oxo-10,11-dihydro-5H-dibenzo[b,e][1,4]diazépin-7-yl]acétamide ;
N-(2,3-dihydroxypropyl)-2-[3-(3-méthoxy-4-nitrophényl)-11-oxo-10,11-dihydro-5H-dibenzo[b,e][1,4]diazépin-7-yl]acétamide ;
N-[3-(1H-imidazol-1-yl)propyl]-2-[3-(3-méthoxy-4-nitrophényl)-11-oxo-10,11-dihydro-5H-dibenzo[b,e][1,4]diazépin-7-yl]acétamide ;
2-[3-(3-méthoxy-4-nitrophényl)-11-oxo-10,11-dihydro-5H-dibenzo[b,e] [1,4]diazépin-7-yl]]N[3-(2-oxopyrrolidin-1-yl)propyl]acétamide ;
N-(2,6-difluorobenzyl)-2-[3-(3-méthoxy-4-nitrophényl)-11-oxo-10,11-dihydro-5H-dibenzo[b,e][1,4]diazépin-7-yl]acétamide ;
N-{2-[4-(aminosulfonyl)phényl]éthyl}-2-[3-(3-méthoxy-4-nitrophényl)-11-oxo-10,11-dihydro-5H-dibenzo[b,e][1,4]diazépin-7-yl]acétamide ;
N-[(1R)-1-(hydroxyméthyl)-2-méthylpropyl]-2-[3-(3-méthoxy-4-nitrophényl)-11-oxo-10,11-dihydro-5H-dibenzo[b,e][1,4]diazépin-7-yl]acétamide ;
N-[(1R)-2-hydroxy-1-phényléthyl]-2-[3-(3-méthoxy-4-nitrophényl)-11-oxo-10,11-dihydro-5H-dibenzo[b,e][1,4]diazépin-7-yl]acétamide ;
2-[3-(3-méthoxy-4-nitrophényl)-11-oxo-10,11-dihydro-5H-dibenzo[b,e] [1,4]diazépin-7-yl]N(thién-3-ylméthyl)acétamide ;
{3-[4-(aminocarbonyl)-3-méthoxyphényl]-11-oxo-10,11-dihydro-5H-dibenzo[b,e][1,4]diazépin-8-yl}acétate de méthyle ;
8-hydroxy-3-(3-méthoxy-4-nitrophényl)-5,10-dihydro-11H-dibenzo[b,e] [1,4]diazépin-11-one ;
8-méthoxy-3-(3-méthoxy-4-nitrophényl)-5,10-dihydro-11H-dibenzo[b,e] [1,4]diazépin-11-one ;
8-éthoxy-3-(3-méthoxy-4-nitrophényl)-5,10-dihydro-11H-dibenzo[b,e] [1,4]diazépin-11-one ;
3-(3-méthoxy-4-nitrophényl)-8-[2-(4-méthyl-1,3-thiazol-5-yl)éthoxy]-5,10-dihydro-11H-dibenzo[b,e][1,4]diazépin-11-one ;
8-[3-(diméthylamino)propoxy]-3-(3-méthoxy-4-nitrophényl)-5,10-dihydro-11H-dibenzo[b,e][1,4]diazépin-11-one ;
3-(3-méthoxy-4-nitrophényl)-8-(2-morpholin-4-yléthoxy)-5,10-dihydro-11H-dibenzo[b,e][1,4]diazépin-11-one ;
3-(3-méthoxy-4-nitrophényl)-8-[2-(4-morpholin-4-ylphényl)éthoxy]-5,10-dihydro-11H-dibenzo[b,e][1,4]diazépin-11-one ;
3-(3-méthoxy-4-nitrophényl)-7-pipéridin-1-yl-5,10-dihydro-11H-dibenzo[b,e][1,4]diazépin-11-one ;
7-[(2S)-2-(hydroxyméthyl)pyrrolidin-1-yl]-3-(3-méthoxy-4-nitrophényl)-5,10-dihydro-11H-dibenzo[b,e][1,4]diazépin-11-one ;
3-(3-méthoxy-4-nitrophényl)-7-morpholin-4-yl-5,10-dihydro-11H-dibenzo[b,e][1,4]diazépin-11-one ;
7-(4-hydroxypipéridin-1-yl)-3-(3-méthoxy-4-nitrophényl)-5,10-dihydro-11H-dibenzo[b,e][1,4]diazépin-11-one ;
{3-[3-méthoxy-4-(3-méthyl-1,2,4-oxadiazol-5-yl)phényl]-11-oxo-10,11-dihydro-5H-dibenzo[b,e][1,4]diazépin-8-yl}acétate de méthyle ;
8-(2-éthyl-2-hydroxybutyl)-3-(3-méthoxy-4-nitrophényl)-5,10-dihydro-11H-dibenzo[b,e][1,4]diazépin-11-one ;
3-[(2-chloropyridin-4-yl)amino]-8-(2-éthyl-2-hydroxybutyl)-5,10-dihydro-11H-dibenzo[b,e][1,4]diazépin-11-one ;
N,N-diméthyl-2-[11-oxo-3-(pyridin-4-ylamino)-10,11-dihydro-5H-dibenzo[b,e][1,4]diazépin-8-yl]acétamide ;
N-(4-morpholin-4-ylphényl)-2-[11-oxo-3-(pyridin-4-ylamino)-10,11-dihydro-5H-dibenzo[b,e][1,4]diazépin-8-yl]acétamide ;
8-(2-hydroxy-2-méthylpropyl)-3-(pyrimidin-4-ylamino)-5,10-dihydro-11H-dibenzo[b,e][1,4]diazépin-11-one ;
8-(2-hydroxy-2-méthylpropyl)-3-[(2-méthylpyridin-4-yl)amino]-5,10-dihydro-11H-dibenzo[b,e][1,4]diazépin-11-one ;
3-(3-méthoxy-4-nitrophényl)-8-(2-oxopropyl)-5,10-dihydro-11H-dibenzo[b,e][1,4]diazépin-11-one ;
3-[(2-chloropyridin-4-yl)amino]-8-(2-oxopropyl)-5,10-dihydro-11H-dibenzo[b,e][1,4]diazépin-11-one ;
3-({2-[(2-chloropyridin-4-yl)amino]pyridin-4-yl}amino)-8-(2-oxopropyl)-5,10-dihydro-11H-dibenzo[b,e][1,4]diazépin-11-one ;
2-méthyl-2-[11-oxo-3-(pyrimidin-4-ylamino)-10,11-dihydro-5H-dibenzo[b,e][1,4]diazépin-8-yl]propanoate de méthyle ;
2-méthyl-2-{3-[(2-méthylpyridin-4-yl)amino]-11-oxo-10,11-dihydro-5H-dibenzo[b,e][1,4]diazépin-8-yl]propanoate de méthyle ;
2-méthylN(4-morpholin-4-ylphényl)-2-[11-oxo-3-(pyrimidin-4-ylamino)-10,11-dihydro-5H-dibenzo[b,e][1,4]diazépin-8-yl]propanamide ;
2-méthyl-2-{3-[(2-méthylpyridin-4-yl)amino]-11-oxo-10,11-dihydro-5H-dibenzo[b,e][1,4]diazépin-8-yl}N(4-morpholin-4-ylphényl)propanamide ;
3-{[3-(2-hydroxyéthyl)pyridin-4-yl]amino}-8-(1-hydroxy-1-méthyléthyl)-5,10-dihydro-11H-dibenzo[b,e][1,4]diazépin-11-one ;
8-(2-hydroxy-2-méthylpropyl)-3-[(2-méthoxypyridin-4-yl)amino]-5,10-dihydro-11H-dibenzo[b,e][1,4]diazépin-11-one ;
8-(2-hydroxy-2-méthylpropyl)-3-[(2-méthylpyridin-4-yl)amino]-5,10-dihydro-11H-dibenzo[b,e][1,4]diazépin-11-one ;
11-oxo-3-(pyrimidin-4-ylamino)-10,11-dihydro-5H-dibenzo[b,e] [1,4]diazépine-7-carboxylate de méthyle ;
7-(1-hydroxy-1-méthyléthyl)-3-(3-méthoxy-4-nitrophényl)-5,10-dihydro-11H-dibenzo[b,e][1,4]diazépin-11-one ;
7-(1-hydroxy-1-méthyléthyl)-3-(pyrimidin-4-ylamino)-5,10-dihydro-11H-dibenzo[b,e][1,4]diazépin-11-one ;
2-{3-[(6-méthoxypyrimidin-4-yl)amino]-11-oxo-10,11-dihydro-5H-dibenzo[b,e][1,4]diazépin-8-yl)-2-méthylN(4-morpholin-4-ylphényl)propanamide ;
3-(3-méthoxy-4-nitrophényl)-8-{2-[(6-morpholin-4-ylpyridin-3-yl)oxy]éthyl}-5,10-dihydro-11H-dibenzo[b,e][1,4]diazépin-11-one ;
3-(4-hydroxy-3-méthoxyphényl)-8-[2-(4-morpholin-4-ylphénoxy)éthyl]-5,10-dihydro-11H-dibenzo[b,e][1,4]diazépin-11-one ;
3-[(2,6-difluoropyridin-4-yl)amino]-8-[2-(4-morpholin-4-ylphénoxy)éthyl]-5,10-dihydro-11H-dibenzo[b,e][1,4]diazépin-11-one ;
7-hydroxy-8-méthoxy-3-(3-méthoxy-4-nitrophényl)-5,10-dihydro-11H-dibenzo[b,e][1,4]diazépin-11-one ;
8-hydroxy-7-méthoxy-3-(3-méthoxy-4-nitrophényl)-5,10-dihydro-11H-dibenzo[b,e][1,4]diazépin-11-one ;
8-méthoxy-3-(3-méthoxy-4-nitrophényl)-7-(tétrahydro-2H-pyran-2-ylméthoxy)-5,10-dihydro-11H-dibenzo[b,e][1,4]diazépin-11-one ;
8-méthoxy-3-(3-méthoxy-4-nitrophényl)-7-[(1-méthylpipéridin-3-yl)méthoxy]-5,10-dihydro-11H-dibenzo[b,e][1,4]diazépin-11-one ;
8-méthoxy-3-(3-méthoxy-4-nitrophényl)-7-(pyridin-2-ylméthoxy)-5,10-dihydro-11H-dibenzo[b,e][1,4]diazépin-11-one ;
8-méthoxy-3-(3-méthoxy-4-nitrophényl)-7-(pyridin-3-ylméthoxy)-5,10-dihydro-11H-dibenzo[b,e][1,4]diazépin-11-one ;
8-méthoxy-3-(3-méthoxy-4-nitrophényl)-7-(pyridin-4-ylméthoxy)-5,10-dihydro-11H-dibenzo[b,e][1,4]diazépin-11-one ;
8-méthoxy-3-(3-méthoxy-4-nitrophényl)-7-[(5-méthylisoxazol-3-yl)méthoxy]-5,10-dihydro-11H-dibenzo[b,e][1,4]diazépin-11-one ;
8-méthoxy-3-(3-méthoxy-4-nitrophényl)-7-[(1-méthyl-1H-imidazol-5-yl)méthoxy]-5,10-dihydro-11H-dibenzo[b,e][1,4]diazépin-11-one ;
8-méthoxy-3-(3-méthoxy-4-nitrophényl)-7-[(2-méthyl-1,3-thiazol-4-yl)méthoxy]-5,10-dihydro-11H-dibenzo[b,e][1,4]diazépin-11-one ;
8-méthoxy-3-(3-méthoxy-4-nitrophényl)-7-[(2-oxo-1,3-oxazolidin-5-yl)méthoxy]-5,10-dihydro-11H-dibenzo[b,e][1,4]diazépin-11-one ;
8-méthoxy-3-(3-méthoxy-4-nitrophényl)-7-(tétrahydrofuran-2-ylméthoxy)-5,10-dihydro-11H-dibenzo[b,e][1,4]diazépin-11-one ;
7-[(2,2-diméthyl-1,3-dioxolan-4-yl)méthoxy]-8-méthoxy-3-(3-méthoxy-4-nitrophényl)-5,10-dihydro-11H-dibenzo[b,e][1,4]diazépin-11-one ;
8-méthoxy-3-(3-méthoxy-4-nitrophényl)-7-[(2R)-pyrrolidin-2-ylméthoxy]-5,10-dihydro-11H-dibenzo[b,e][1,4]diazépin-11-one;
7,8-diméthoxy-3-(3-méthoxy-4-nitrophényl)-5,10-dihydro-11H-dibenzo[b,e][1,4]diazépin-11-one ;
8-méthoxy-7-[2-(2-méthoxyéthoxy)éthoxy]-3-(3-méthoxy-4-nitrophényl)-5,10-dihydro-11H-dibenzo[b,e][1,4]diazépin-11-one ;
7-(2,3-dihydroxypropoxy)-8-méthoxy-3-(3-méthoxy-4-nitrophényl)-5,10-dihydro-11H-dibenzo[b,e][1,4]diazépin-11-one;
7-[3-hydroxy-2,2-bis(hydroxyméthyl)propoxyl-8-méthoxy-3-(3-méthoxy-4-nitrophényl)-5,10-dihydro-11H-dibenzo[b,e][1,4]diazépin-11-one ;
acide 2-hydroxy-3-{[8-méthoxy-3-(3-méthoxy-4-nitrophényl)-11-oxo-10,11-dihydro-5H-dibenzo[b,e][1,4]diazépin-7-yl]oxy}propane-1-sulfonique ;
7-(3-aminopropoxy)-8-méthoxy-3-(3-méthoxy-4-nitrophényl)-5,10-dihydro-11H-dibenzo[b,e][1,4]diazépin-11-one ;
7-[2-(diméthylamino)éthoxy]-8-méthoxy-3-(3-méthoxy-4-nitrophényl)-5,10-dihydro-11H-dibenzo[b,e][1,4]diazépin-11-one ;
7-(2-chloroéthoxy)-8-méthoxy-3-(3-méthoxy-4-nitrophényl)-5,10-dihydro-11H-dibenzo[b,e][1,4]diazépin-11-one ;
8-méthoxy-3-(3-méthoxy-4-nitrophényl)-7-(2-pyrrolidin-1-yléthoxy)-5,10-dihydro-11H-dibenzo[b,e][1,4]diazépin-11-one ;
8-méthoxy-3-(3-méthoxy-4-nitrophényl)-7-(2-morpholin-4-yléthoxy)-5,10-dihydro-11H-dibenzo[b,e][1,4]diazépin-11-one ;
7-(4-hydroxybutoxy)-8-méthoxy-3-(3-méthoxy-4-nitrophényl)-5,10-dihydro-11H-dibenzo[b,e][1,4]diazépin-11-one ;
7-(4-hydroxybutoxy)-3-(4-hydroxy-3-méthoxyphényl)-8-méthoxy-5,10-dihydro-11H-dibenzo[b,e][1,4]diazépin-11-one ;
7-(4-hydroxybutoxy)-8-méthoxy-3-(pyrimidin-4-ylamino)-5,10-dihydro-11H-dibenzo[b,e][1,4]diazépin-11-one;
4-{[7-(4-hydroxybutoxy)-8-méthoxy-11-oxo-10,11-dihydro-5H-dibenzo[b,e][1,4]diazépin-3-yl]amino}pyridine-2-carbonitrile ;
3-[(2,6-difluoropyridin-4-yl)amino]-7-(4-hydroxybutoxy)-8-méthoxy-5,10-dihydro-11H-dibenzo[b,e][1,4]diazépin-11-one ;
7-(4-hydroxybutoxy)-8-méthoxy-3-[(2,3,6-trifluoropyridin-4-yl)amino]-5,10-dihydro-11H-dibenzo[b,e][1,4]diazépin-11-one ;
7-éthoxy-3-(3-méthoxy-4-nitrophényl)-5,10-dihydro-11H-dibenzo[b,e] [1,4]diazépin-11-one ;
7-(4-hydroxybutoxy)-3-(3-méthoxy-4-nitrophényl)-5,10-dihydro-11H-dibenzo[b,e][1,4]diazépin-11-one ;
7-(2-hydroxyéthoxy)-3-(3-méthoxy-4-nitrophényl)-5,10-dihydro-11H-dibenzo[b,e][1,4]diazépin-11-one ;
7-(2,3-dihydroxypropoxy)-3-(3-méthoxy-4-nitrophényl)-5,10-dihydro-11H-dibenzo[b,e][1,4]diazépin-11-one ;
7-[2-(2-méthoxyéthoxy)éthoxy]-3-(3-méthoxy-4-nitrophényl)-5,10-dihydro-11H-benzo[b,e][1,4]diazépin-11-one ;
7-(méthoxyméthyl)-3-(3-méthoxy-4-nitrophényl)-5,10-dihydro-11H-dibenzo[b,e][1,4]diazépin-11-one ;
7-(3-méthoxy-4-nitrobenzyl)-3-(3-méthoxy-4-nitrophényl)-5,10-dihydro-11H-dibenzo[b,e][1,4]diazépin-11-one ;
7-{[[2-(diméthylamino)éthyl](méthyl)amino]méthyl}-3-(3-méthoxy-4-nitrophényl)-5,10-dihydro-11H-dibenzo[b,e][1,4]diazépin-11-one ;
3-(3-méthoxy-4-nitrophényl)-7-{[(2-tétrahydro-2H-pyran-4-yléthyl)amino]méthyl}-5,10-dihydro-11H-dibenzo[b,e][1,4]diazépin-11-one ;
8-éthyl-7-méthoxy-3-(3-méthoxy-4-nitrophényl)-5,10-dihydro-11H-dibenzo[b,e][1,4]diazépin-11-one ;
8-méthoxy-3-(3-méthoxy-4-nitrophényl)-7-vinyl-5,10-dihydro-11H-dibenzo[b,e][1,4]diazépin-11-one ;
8-(3-hydroxypropyl)-7-méthoxy-3-(3-méthoxy-4-nitrophényl)-5,10-dihydro-11H-dibenzo[b,e][1,4]diazépin-11-one ;
7-méthoxy-3-(3-méthoxy-4-nitrophényl)-8-{3-[(2-méthylpyridin-3-yl)oxy]propyl)-5,10-dihydro-11H-dibenzo[b,e][1,4]diazépin-11-one ;
8-{3-[(2-chloropyridin-3-yl)oxy]propyl}-7-méthoxy-3-(3-méthoxy-4-nitrophényl)-5,10-dihydro-11H-dibenzo[b,e][1,4]diazépin-11-one ;
7-méthoxy-3-(3-méthoxy-4-nitrophényl)-8-[3-(4-morpholin-4-ylphénoxy)propyl]-5,10-dihydro-11H-dibenzo[b,e][1,4]diazépin-11-one ;
8-[3-(isoquinoléin-3-yloxy)propyl]-7-méthoxy-3-(3-méthoxy-4-nitrophényl)-5,10-dihydro-11H-dibenzo[b,e][1,4]diazépin-11-one ;
7-méthoxy-3-(3-méthoxy-4-nitrophényl)-8-[3-(3-oxoisoquinoléin-2(3H)-yl)propyl]-5,10-dihydro-11H-dibenzo[b,e][1,4]diazépin-11-one ;
7-méthoxy-3-(3-méthoxy-4-nitrophényl)-11-oxo-10,11-dihydro-5H-dibenzo[b,e][1,4]diazépine-8-carboxylate de méthyle ;
7-méthoxy-11-oxo-3-(pyrimidin-4-ylamino)-10,11-dihydro-5H-dibenzo[b,e] [1,4]diazépine-8-carboxylate de méthyle ;
3-[(2,6-difluoropyridin-4-yl)amino]-8-(1,1-diméthyl-2-morpholin-4-yl-2-oxoéthyl)-5,10-dihydro-11H-dibenzo[b,e][1,4]diazépin-11-one ;
2-{3-[(2,6-difluoropyridin-4-yl)amino]-11-oxo-10,11-dihydro-5H-dibenzo[b,e][1,4]diazépin-8-yl)-N,N,2-triméthylpropanamide ;
2-{3-[(2,6-difluoropyridin-4-yl)amino]-11-oxo-10,11-dihydro-5H-dibenzo[b,e][1,4]diazépin-8-yl}-2-méthylNpyridin-4-ylpropanamide ;
2-{3-[(2,6-difluoropyridin-4-yl)amino]-11-oxo-10,11-dihydro-5H-dibenzo[b,e][1,4]diazépin-8-yl}-2-méthylN1,3-thiazol-2-ylpropanamide ;
3-[(2,6-difluoropyridin-4-yl)amino]-8-{2-[(3R)-3-hydroxypipéridin-1-yl)-1,1-diméthyl-2-oxoéthyle}-5,10-dihydro-11H-dibenzo[b,e][1,4]diazépin-11-one ;
3-[(2,6-difluoropyridin-4-yl)amino]-8-[2-(4-hydroxypipéridin-1-yl)-1,1-diméthyl-2-oxoéthyl]-5,10-dihydro-11H-dibenzo[b,e][1,4]diazépin-11-one ;
3-[(2,6-difluoropyridin-4-yl)amino]-8-{2-[(2S)-2-(hydroxyméthyl)pyrrolidin-1-yl]-1,1-diméthyl-2-oxoéthyl}-5,10-dihydro-11H-dibenzo[b,e] [1,4]diazépin-11-one ;
3-[(2,6-difluoropyridin-4-yl)amino]-8-(1,1-diméthyl-2-oxo-2-pyrrolidin-1-yléthyl)-5,10-dihydro-11H-dibenzo[b,e][1,4]diazépin-11-one ;
2-{3-[(2,6-difluoropyridin-4-yl)amino]-11-oxo-10,11-dihydro-5H-dibenzo[b,e][1,4]diazépin-8-yl]-2-méthylNpyridin-2-ylpropanamide ;
2-{3-[(2,6-difluoropyridin-4-yl)amino]-11-oxo-10,11-dihydro-5H-dibenzo[b,e][1,4]diazépin-8-yl}-2-méthylNpyridin-3-ylpropanamide ;
2-{3-[(2,6-difluoropyridin-4-yl)amino]-11-oxo-10,11-dihydro-5H-dibenzo[b,e][1,4]diazépin-8-yl}N(4-fluorophényl)-2-méthylpropanamide ;
3-[(2,6-difluoropyridin-4-yl)amino]-8-{2-[(2R)-2-(hydroxyméthyl)pyrrolidin-1-yl]-1,1-diméthyl-2-oxoéthyl}-5,10-dihydro-11H-dibenzo[b,e][1,4]diazépin-11-one ;
8-méthoxy-3-(3-méthoxy-4-nitrophényl)-7-(3-morpholin-4-yl-3-oxopropyl)-5,10-dihydro-11H-dibenzo[b,e][1,4]diazépin-11-one ;
3-[3-(3-méthoxy-4-nitrophényl)-11-oxo-10,11-dihydro-5H-dibenzo[b,e] [1,4]diazépin-8-yl]Npyridin-3-ylpropanamide ;
3-[3-(3-méthoxy-4-nitrophényl)-11-oxo-10,11-dihydro-5H-dibenzo[b,e] [1,4]diazépin-8-yl]Npyridin-4-ylpropanamide ;
8-[3-(3-hydroxypipéridin-1-yl)-3-oxopropyl]-3-(3-méthoxy-4-nitrophényl)-5,10-dihydro-11H-dibenzo[b,e][1,4]diazépin-11-one ;
N-(4-fluorophényl)-3-[3-(3-méthoxy-4-nitrophényl)-11-oxo-10,11-dihydro-5H-dibenzo[b,e][1,4]diazépin-8-yl]propanamide ;
3-[3-(3-méthoxy-4-nitrophényl)-11-oxo-10,11-dihydro-5H-dibenzo[b,e] [1,4]diazépin-8-yl]N[4-(trifluorométhyl)phényl]propanamide ;
3-[3-(3-méthoxy-4-nitrophényl)-11-oxo-10,11-dihydro-5H-dibenzo[b,e] [1,4]diazépin-8-yl]Nméthylpropanamide ;
3-[8-méthoxy-3-(3-methoxy-4-nitrophényl)-11-oxo-10,11-dihydro-5H-dibenzo[b,e][1,4]diazépin-7-yl]-N,N-diméthylpropanamide ;
8-{2-[(6-chloropyridin-3-yl)oxy]éthyl}-3-(3-méthoxy-4-nitrophényl)-5,10-dihydro-11H-dibenzo[b,e][1,4]diazépin-11-one ;
8-{2-[(2-chloropyridin-3-yl)oxy]éthyl}-3-(3-méthoxy-4-nitrophényl)-5,10-dihydro-11H-dibenzo[b,e][1,4]diazépin-11-one ;
3-(3-méthoxy-4-nitrophényl)-8-{2-[(6-méthylpyridin-3-yl)oxy]éthyl}-5,10-dihydro-11H-dibenzo[b,e][1,4]diazépin-11-one;
3-(3-méthoxy-4-nitrophényl)-8-{2-[(2-méthylpyridin-3-yl)oxy]éthyl}-5,10-dihydro-11H-dibenzo[b,e][1,4]diazépin-11-one ;
3-(3-méthoxy-4-nitrophényl)-8-[2-(pyridin-3-yloxy)éthyl]-5,10-dihydro-11H-dibenzo[b,e][1,4]diazépin-11-one ;
8-{2-[(2,6-diméthylpyridin-3-yl)oxy]éthyl}-3-(3-méthoxy-4-nitrophényl)-5,10-dihydro-11H-dibenzo[b,e][1,4]diazépin-11-one ;
8-[2-({2-[(diméthylamino)méthyl]pyridin-3-yl)oxy)éthyl]-3-(3-méthoxy-4-nitrophényl)-5,10-dihydro-11H-dibenzo[b,e][1,4]diazépin-11-one ;
8-[2-(isoquinoléin-7-yloxy)éthyl]-3-(3-méthoxy-4-nitrophényl)-5,10-dihydro-11H-dibenzo[b,e][1,4]diazépin-11-one ;
7-méthoxy-3-(3-méthoxy-4-nitrophényl)-N,N-diméthyl-11-oxo-10,11-dihydro-5H-dibenzo[b,e][1,4]diazépine-8-carboxamide ;
acide 7-méthoxy-3-(3-méthoxy-4-nitrophényl)-11-oxo-10,11-dihydro-5H-dibenzo[b,e][1,4]diazépine-8-carboxylique ;
7-{2-[(2-chloropyridin-3-yl)oxy]éthyl}-8-méthoxy-3-(3-méthoxy-4-nitrophényl)-5,10-dihydro-11H-dibenzo[b,e][1,4]diazépin-11-one ;
8-[2-(isoquinoléin-5-yloxy)éthyl]-3-(3-méthoxy-4-nitrophényl)-5,10-dihydro-11H-dibenzo[b,e][1,4]diazépin-11-one ;
3-(3-méthoxy-4-nitrophényl)-8-[2-(quinoléin-5-yloxy)éthyl]-5,10-dihydro-11H-dibenzo[b,e][1,4]diazépin-11-one ;
3-(3-méthoxy-4-nitrophényl)-8-[2-(4-méthoxyphénoxy)éthyl]-5,10-dihydro-11H-dibenzo[b,e][1,4]diazépin-11-one ;
3-(3-méthoxy-4-nitrophényl)-8-[2-(3-méthoxyphénoxy)éthyl]-5,10-dihydro-11H-dibenzo[b,e][1,4]diazépin-11-one ;
3-{2-[3-(3-méthoxy-4-nitrophényl)-11-oxo-10,11-dihydro-5H-dibenzo[b,e] [1,4]diazépin-8-yl]éthoxy}pyridine-2-carboxamide ;
3-(3-méthoxy-4-nitrophényl)-8-[3-(4-morpholin-4-ylphénoxy)propyl]-5,10-dihydro-11H-dibenzo[b,e][1,4]diazépin-11-one ;
3-(3-méthoxy-4-nitrophényl)-8-[3-(pyridin-3-yloxy)propyl]-5,10-dihydro-11H-dibenzo[b,e][1,4]diazépin-11-one ;
8-[2-(3-aminophénoxy)éthyl]-3-(3-méthoxy-4-nitrophényl)-5,10-dihydro-11H-dibenzo[b,e][1,4]diazépin-11-one ;
3-(3-méthoxy-4-nitrophényl)-8-{2-[(2-méthyl-1,3-benzothiazol-7-yl)oxy]éthyl}-5,10-dihydro-11H-dibenzo[b,e][1,4]diazépin-11-one ;
2-[3-(3-méthoxy-4-nitrophényl)-11-oxo-10,11-dihydro-5H-dibenzo[b,e] [1,4]diazépin-8-yl]-2-méthylN(pyridin-2-ylméthyl)propanamide ;
8-(2-hydroxy-2-méthylpropyl)-3-(3-méthoxy-4-nitrophényl)-5,10-dihydro-11H-dibenzo[b,e][1,4]diazépin-11-one ;
3-(3-méthoxy-4-nitrophényl)-8-[(4-méthylpipérazin-1-yl)méthyl]-5,10-dihydro-11H-dibenzo[b,e][1,4]diazépin-11-one ;
3-(4-chloro-3-méthoxyphényl)-8-[(4-méthylpipérazin-1-yl)méthyl]-5,10-dihydro-11H-dibenzo[b,e][1,4]diazépin-11-one ;
2-méthoxy-4-{8-[(4-méthylpipérazin-1-yl)méthyl]-11-oxo-10,11-dihydro-5H-dibenzo[b,e][1,4]diazépin-3-yl}benzonitrile ;
3-(4-hydroxy-3-méthoxyphényl)-8-(hydroxyméthyl)-5,10-dihydro-11H-dibenzo[b,e][1,4]diazépin-11-one ;
3-(3-méthoxy-4-nitrophényl)-8-(morpholin-4-ylméthyl)-5,10-dihydro-11H-dibenzo[b,e][1,4]diazépin-11-one ;
8-{[(2R)-2-(hydroxyméthyl)pyrrolidin-1-yl]méthyl}-3-(3-méthoxy-4-nitrophényl)-5,10-dihydro-11H-dibenzo[b,e][1,4]diazépin-11-one ;
7-(2-hydroxyéthoxy)-8-méthoxy-3-(3-méthoxy-4-nitrophényl)-5,10-dihydro-11H-dibenzo[b,e][1,4]diazépin-1-1-one ;
8-{3-[2-(hydroxyméthyl)pyrrolidin-1-yl]-3-oxopropyl}-3-(3-méthoxy-4-nitrophényl)-5,10-dihydro-11H-dibenzo[b,e][1,4]diazépin-11-one ;
(cis)-4-hydroxyN[3-(3-méthoxy-4-nitrophényl)-11-oxo-10,11-dihydro-5H-dibenzo[b,e][1,4]diazépin-7-yl]cyclohexanecarboxamide.

17. Composition pharmaceutique comprenant un composé de formule (I) 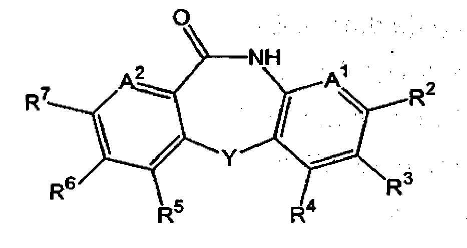 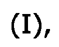 ou un sel thérapeutiquement acceptable de celui-ci, en combinaison avec un véhicule thérapeutiquement acceptable, dans lequel
(a)
A¹ est CH ;
A² est CH ;
R² est choisi dans le groupe constitué d'hydrogène, alcényle, alcoxy, alcoxyalcoxy, alcoxyalcoxyalcoxy, alcoxyalkyle, alcoxycarbonyle, alcoxycarbonylalkyle, alkyle, alkylcarbonyle, alkylcarbonylalkyle, amino, aminoalcoxy, aminoalkyle, aminocarbonyle, aminocarbonylalkyle, aminosulfonyle, aryle, arylalcoxy, arylalkyle, aryloxyalkyle, carboxy, carboxyalkyle, cyano, cyanoalkyle, cycloalkyle, cycloalkylalkyle, halogéno, halogénoalcoxy, halogénoalkyle, hétérocyclyle, hétérocyclylalcoxy, hétérocyclylalkyle, hétérocyclylcarbonylalkyle, hétérocyclyloxyalkyle, hydroxy, hydroxyalcoxy, hydroxyalkyle, nitro, nitroalkyle, et - (CR⁹R¹⁰)ₘC(O)NR¹¹R¹² ;
R³, R⁴ et R⁵ sont chacun hydrogène ;
R⁶ est choisi dans le groupe constitué d'aryle, cycloalkyle, halogéno, hétérocyclyle, et -XR¹³ ;
R⁷ est hydrogène ;
R⁹ et R¹⁰ sont indépendamment choisis dans le groupe constitué d'hydrogène, alcényle, alkyle, aminoalkyle, et hydroxyalkyle ; ou
R⁹ et R¹⁰, conjointement avec l'atome de carbone auquel ils sont liés, forment un groupe cycloalkyle ;
R¹¹ et R¹² sont chacun indépendamment choisis dans le groupe constitué d'hydrogène, alcényle, alcoxyalkyle, alkyle, amino, aminoalkyle, aryle, arylalkyle, cyanoalkyle, cycloalkyle, cycloalkylalkyle, halogénoalkyle, hétérocyclyle, hétérocyclylalkyle, hydroxyalkyle, -NR^{c}R^{d}, (NR^{c}R^{d})alkyle, et (NR^{c}R^{d})carbonylalkyle ; ou
R¹¹ et R¹², conjointement avec l'atome d'azote auquel ils sont liés, forment un cycle hétérocyclyle choisi dans le groupe constitué d'azétidinyle, diazépanyle, imidazolidinyle, morpholinyle, pipérazinyle, pipéridinyle, pyrrolidinyle et thiomorpholinyle, qui peuvent chacun être facultativement substitués par un, deux ou trois substituants indépendamment choisis dans le groupe constitué d'alcoxy, alcoxycarbonyle, alcényle, alkyle, alkylcarbonyle, aryle, carboxy, carboxyalkyle, hétérocyclyle, hétérocyclylalkyle, hydroxy, hydroxyalkyle, -NR^{c}R^{d}, (NR^{c}R^{d})alkyle, et (NR^{c}R^{d})carbonyle ;
R¹³ est choisi dans le groupe constitué d'aryle, cycloalkyle, et hétérocyclyle ;
X est choisi dans le groupe constitué de -O-, -NR¹⁴-, -C(O)-, -S-, -SO₂-, -(CH₂)ₙ-, -C(O)NR¹⁴-, -NR¹⁴C(O)-, -SO₂NR¹⁴-, -NR¹⁴SO₂, -O(CH₂)ₘ-, - (CH₂)ₘO-, -CH=CH-, et C=C ; où R¹⁴ est choisi dans le groupe constitué d'hydrogène, alcényle, alkyle, aminoalkyle, et hydroxyalkyle ;
Y est NH ;
m est 0 à 3; et
n est 1 à 3;
ou dans lequel
(b)
A¹ est CH ;
A² est CH;
Y est NH;
R⁴, R⁵, et R⁷ sont chacun hydrogène ;
R⁶ est phényle substitué à la position trois par méthoxy et substitué à la position quatre par alcoxycarbonyle, alcoxycarbonylalcényle, alkylcarbonyle, alkylsulfonyle, cyano, halogéno, halogénoalcényle, hydroxy, nitro, NH₂SO₂-, ou NH₂CO- ; et soit
(i) R² est hydrogène et R³ est -(CR⁹R¹⁰)C(O)NHR¹² où R⁹ et R¹⁰ sont indépendamment choisis dans le groupe constitué d'hydrogène, alcényle, alkyle, aminoalkyle, et hydroxyalkyle, ou R⁹ et R¹⁰, conjointement avec l'atome de carbone auquel ils sont liés, forment un groupe cycloalkyle, et R¹² est phényle facultativement substitué par 1 groupe morpholinyle ;
(ii) R² est hydrogène et R³ est hydroxyalkyle ;
(iii) R² est hydrogène et R³ est alcoxy ;
(iv) R² et R³ sont indépendamment -(CR⁹R¹⁰)C(O)NHR¹² où R⁹ et R¹⁰ sont indépendamment choisis dans le groupe constitué d'hydrogène, alcényle, alkyle, aminoalkyle, et hydroxyalkyle, ou R⁹ et R¹⁰, conjointement avec l'atome de carbone auquel ils sont liés, forment un groupe cycloalkyle, et R¹² est phényle facultativement substitué par 1 groupe morpholinyle ;
(v) R² et R³ sont indépendamment hydroxalkyle ; ou
(vi) R² et R³ sont indépendamment alcoxy ;
dans lequel
alkyle est un alkyle en C₁-C₁₀ ;
alcényle est un alcényle en C₂-C₆ ;
alcoxy est un alcoxy en C₁-C₁₀ ;
aryle est un groupe phényle, ou un système cyclique condensé bicyclique ou tricyclique dans lequel un ou plusieurs des cycles condensés est un groupe phényle, où l'aryle peut être facultativement substitué par un, deux, trois, quatre, ou cinq substituants indépendamment choisis dans le groupe constitué d'alcoxy, alcoxyalkyle, alcoxycarbonyle, alcoxycarbonylalcényle, alcoxycarbonylalkyle, alcoxysulfonyle, alkyle, alkylcarbonyle, alkylsulfonyle, amino, aminocarbonyle, aminosulfonyle, un deuxième groupe aryle, arylalkyle, arylsulfonyle, carboxy, cyano, formyle, halogéno, halogénoalcoxy, halogénoalcényle, halogénoalkyle, hétérocyclyle, hétérocyclylalkyle, hydroxy, hydroxyalkyle, nitro, (NR^{c}R^{d})alkyle, et oxo, où le deuxième groupe aryle, la partie aryle de l'arylalkyle et de l'arylsulfonyle, l'hétérocyclyle, et la partie hétérocyclyle de l'hétérocyclylalkyle peuvent être facultativement substitués par un, deux, trois, quatre, ou cinq substituants indépendamment choisis dans le groupe constitué d'alcoxy, alcoxyalkyle, alcoxycarbonyle, alkyle, alkylcarbonyle, carboxy, cyano, halogéno, halogénoalcoxy, halogénoalkyle, hydroxy, nitro, et oxo ;
hétérocyclyle est choisi parmi un cycle de cinq, six, ou sept chaînons contenant un, deux, ou trois hétéroatomes indépendamment choisis dans le groupe constitué de l'azote, l'oxygène et le soufre, le cycle de cinq chaînons ayant zéro à deux doubles liaisons et les cycles de six et sept chaînons ayant zéro à trois doubles liaisons ; des groupes bicycliques dans lesquels le cycle hétérocyclyle est condensé avec un groupe phényle, un groupe cycloalcényle monocyclique, un groupe cycloalkyle monocyclique, ou un autre groupe hétérocyclyle monocyclique ; et des groupes tricycliques dans lesquels un système bicyclique est condensé avec un groupe phényle, un groupe cycloalcényle monocyclique, un groupe cycloalkyle monocyclique, ou un autre groupe hétérocyclyle monocyclique ; où un hétérocyclyle peut être facultativement substitué par un, deux, trois, quatre, ou cinq substituants indépendamment choisis dans le groupe constitué d'alcoxy, alcoxyalkyle, alcoxycarbonyle, alkyle, alkylcarbonyle, alkylsulfonyle, amino, aminocarbonyle, aminosulfonyle, aryle, arylalkyle, arylsulfonyle, carboxy, cyano, halogéno, un deuxième groupe hétérocyclyle, hétérocyclylalkyle, hydroxy, hydroxyalkyle, nitro, -NR^{c}R^{d}, (NR^{c}R^{d})alkyle, et oxo, où l'aryle, la partie aryle de l'arylalkyle et l'arylsulfonyle, le deuxième groupe hétérocyclyle, et la partie hétérocyclyle de l'hétérocyclylalkyle peuvent être facultativement substitués par un, deux, trois, quatre, ou cinq substituants indépendamment choisis dans le groupe constitué d'alcoxy, alcoxyalkyle, alcoxycarbonyle, alkyle, alkylcarbonyle, carboxy, cyano, halogéno, halogénoalcoxy, halogénoalkyle, hydroxy, méthylènedioxy, nitro, et oxo ;
cycloalkyle est un système cyclique hydrocarboné monocyclique, bicyclique, ou tricyclique saturé ayant trois à douze atomes de carbone, un cycloalkyle pouvant être facultativement substitué par un, deux, trois, ou quatre substituants indépendamment choisis dans le groupe constitué d'alcoxy, alcoxyalkyle, alcoxycarbonyle, alkyle, alkylcarbonyle, alkylsulfonyle, -NR^{c}R^{d}, (NR^{c}R^{d})alkyle, (NR^{c}R^{d})carbonyle, un deuxième groupe aryle, arylalkyle, arylsulfonyle, carboxy, cyano, halogéno, hétérocyclyle, hétérocyclylalkyle, hydroxy, hydroxyalkyle, nitro, et oxo, où le deuxième groupe aryle, la partie aryle de l'arylalkyle et de l'arylsulfonyle, l'hétérocyclyle, et la partie hétérocyclyle de l'hétérocyclylalkyle peuvent être facultativement substitués par un, deux, trois, quatre, ou cinq substituants indépendamment choisis dans le groupe constitué d'alcoxy, alcoxyalkyle, alcoxycarbonyle, alkyle, alkylcarbonyle, carboxy, cyano, halogéno, halogénoalcoxy, halogénoalkyle, hydroxy, nitro, et oxo ;
cycloalcényle est un système cyclique ou bicyclique non aromatique ayant trois à dix atomes de carbone et un à trois cycles, dans lequel chaque cycle de cinq chaînons a une double liaison, chaque cycle de six chaînons a une ou deux doubles liaisons, chaque cycle de sept et huit chaînons a une à trois doubles liaisons, et chaque cycle de neuf à dix chaînons a une à quatre doubles liaisons ;
amino désigne -NR^{a}R^{b}, où R^{a} et R^{b} sont indépendamment choisis dans le groupe constitué d'hydrogène, alcoxyalkylcarbonyle, alcoxycarbonyle, alkyle, alkylcarbonyle, alkylsulfonyle, aryle, arylalkyle, arylalkylcarbonyle, arylalkylsulfonyle, arylsulfonyle, arylsulfonylalkyle, arylsulfonylalkylcarbonyle, cycloalkyle, cycloalkylalkyle, cycloalkylcarbonyle, halogénoalkylsulfonyle, hétérocyclyle, hétérocyclylalkylcarbonyle, hétérocyclylalkylsulfonyle, hétérocyclylcarbonyle, hétérocyclylsulfonyle, -NR^{c}R^{d}, (NR^{c}R^{d})alkyle, (NR^{c}R^{d})alkylcarbonyle, (NR^{c}R^{d})alkylsullfonyle, (NR^{c}R^{d})carbonyle, (NR^{c}R^{d})carbonylalkyle, et (NR^{c}R^{d})carbonylalkylcarbonyle, où aryle peut être facultativement substitué par un, deux, trois, quatre, ou cinq substituants indépendamment choisis dans le groupe constitué d'alcoxy, alcoxyalkyle, alcoxycarbonyle, alkyle, alkylcarbonyle, alkylsulfonyle, un deuxième groupe aryle, arylalkyle, arylsulfonyle, carboxy, cyano, halogéno, hétérocyclyle, hétérocyclylalkyle, hydroxy, nitro, et oxo, où le deuxième groupe aryle, la partie aryle de l'arylalkyle et de l'arylsulfonyle, l'hétérocyclyle, et la partie hétérocyclyle de l'hétérocyclylalkyle peuvent être facultativement substitués par un, deux, trois, quatre, ou cinq substituants indépendamment choisis dans le groupe constitué d'alcoxy, alcoxyalkyle, alcoxycarbonyle, alkyle, alkylcarbonyle, carboxy, cyano, halogéno, halogénoalcoxy, halogénoalkyle, hydroxy, nitro, et oxo ; et
R^{c} et R^{d} sont chacun indépendamment hydrogène, alcoxycarbonyle, alkyle, alkylcarbonyle, aryle, arylsulfonyle, hétérocyclcarbonyle, ou formyle.

18. Composition pharmaceutique telle que définie dans la revendication 17, ou sel thérapeutiquement acceptable de celle-ci, pour utilisation dans le traitement de tumeurs solides primitives et métastatiques.

19. Composition pharmaceutique de la revendication 18 pour utilisation dans le traitement de carcinomes du sein, du côlon, du rectum, du poumon, de l'oropharynx, de l'hypopharynx, de l'oesophage, de l'estomac, du pancréas, du foie, de la vésicule biliaire et des voies biliaires, de l'intestin grêle, des voies urinaires, de l'appareil génital féminin, de l'appareil génital masculin, des glandes endocrines et de la peau ; des hémangiomes, des mélanomes, des sarcomes, comprenant ceux provenant de tissus osseux et mous ainsi que le sarcome de Kaposi ; des tumeurs du cerveau, des nerfs, des yeux et des méninges, comprenant les astrocytomes, les gliomes, les glioblastomes, les rétinoblastomes, les neuromes, les neuroblastomes, les schwannomes et les méningiomes ; des tumeurs solides provenant de malignités hématopoïétiques telles que des leucémies ; ou des lymphomes ; ou pour prévenir les métastases des tumeurs ci-dessus lorsqu'elle est utilisée seule ou en combinaison avec une radiothérapie et/ou d'autres agents chimiothérapeutiques.

20. Composition pharmaceutique telle que définie dans la revendication 17, ou un sel thérapeutiquement acceptable de celle-ci, pour utilisation dans le traitement du cancer chez un patient ayant un besoin reconnu d'un tel traitement.

21. Composition pharmaceutique telle que définie dans la revendication 17 pour utilisation en tant qu'agent pharmaceutique.
